(19) Europäisches Patentamt
European Patent Office
Office européen des brevets

(11) EP 3 976 186 B1

(12) # EUROPEAN PATENT SPECIFICATION

(45) Date of publication and mention of the grant of the patent:
**24.06.2026 Bulletin 2026/26**

(21) Application number: **20732078.9**

(22) Date of filing: **24.05.2020**

(51) International Patent Classification (IPC):
***A61P 25/00*** *(2006.01)* ***C07D 401/14*** *(2006.01)*
***C07D 405/14*** *(2006.01)* ***A61K 31/4545*** *(2006.01)*

(52) Cooperative Patent Classification (CPC):
**C07D 401/14; A61P 25/00; C07D 405/14**

(86) International application number:
**PCT/US2020/034434**

(87) International publication number:
**WO 2020/243027 (03.12.2020 Gazette 2020/49)**

(54) **COMPOUNDS, COMPOSITIONS, AND METHODS OF USE**

VERBINDUNGEN, ZUSAMMENSETZUNGEN UND VERFAHREN ZUR VERWENDUNG

COMPOSÉS, COMPOSITIONS ET PROCÉDÉS D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.05.2019 US 201962852565 P**

(43) Date of publication of application:
**06.04.2022 Bulletin 2022/14**

(73) Proprietor: **Sage Therapeutics, LLC**
**Rockville, MD 20850 (US)**

(72) Inventors:
- **HOPPER, Allen, Taylor**
**Lexington, MA 02420 (US)**
- **MISCHKE, Steven**
**Waltham, MA 02453 (US)**
- **LA, Daniel**
**Chestnut Hill, MA 02467 (US)**

(74) Representative: **Abthorpe, Mark et al**
**Kilburn & Strode LLP**
**Lacon London**
**84 Theobalds Road**
**London WC1X 8NL (GB)**

(56) References cited:
**EP-A1- 2 933 247** **EP-A1- 2 982 666**
**WO-A1-2011/033255** **WO-A1-2011/135276**
**WO-A1-2013/054822** **WO-A1-2021/062472**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 976 186 B1

## Description

### Background of the Invention

CYP46A1 is a gene expressed in the brain that encodes the enzyme cholesterol 24-hydroxylase (also known as CYP46A1 and CH24H), which converts cholesterol into 24S-hydroxycholesterol (24-HC), a positive allosteric modulator of N-methyl-D-aspartate (NMDA) receptors. Inhibition of 24-HC production in the brain by CYP46A1 inhibitors can negatively modulate glutamatergic over-activation in neurological diseases associated with NMDA hyperfunction such as epilepsy and autism spectrum disorder (ASD); or diseases associated with elevated 24-HC levels such as multiple sclerosis (MS). Findings have suggested that CYP46A1 inhibitors may also be promising therapeutics for neurogenerative diseases such as Alzheimer's disease, Huntington's disease, Parkinson's disease, cerebral infarction, traumatic brain injury, glaucoma, and amyotrophic lateral sclerosis.
EP2933247 A1 and EP2982666 A1 disclose compounds and salts thereof that are said to be useful as agents for the prevention or treatment of epilepsy and neurodegenerative diseases.
WO 2013/054822 A1 discloses compounds and salts thereof that are said to be useful as agents for the prophylaxis or treatment of neurodegenerative diseases.
The present disclosure provides compounds capable of modulating (e.g., inhibiting) CYP46A1. Any reference in this specification to a method of treatment is to be interpreted as referring to the compounds, salts, or compositions for use in the recited method of treatment.

### Summary of the Invention

Provided herein, in part, are compounds designed, for example, to act as CYP46A1 inhibitors. In some embodiments, such compounds are useful as therapeutic agents for treating diseases associated with the inhibition of CYP46A1, for example, a neurodegenerative disease (e.g. Alzheimer's disease, mild cognitive impairment, Huntington's disease, Parkinson's disease, amyotrophic lateral sclerosis, traumatic brain injury, cerebral infarction, glaucoma, and multiple sclerosis), epilepsy, developmental and epileptic encephalopathies, psychiatric disorders (e.g. schizophrenia and autism spectrum disorder (ASD)), and spasm.

Accordingly, in one aspect, provided herein are compounds of Formula I:

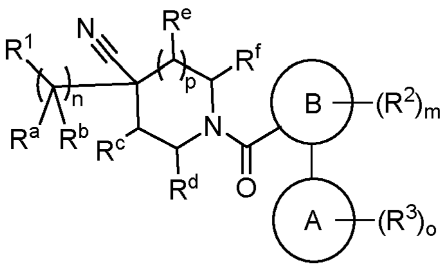

(I),

or pharmaceutically acceptable salts thereof; wherein: $R^1$ is selected from the group consisting of $C_6$-$C_{10}$ aryl, $C_3$-$C_7$ cycloalkyl, 3-7 membered heterocyclyl, and 5-10 membered heteroaryl, wherein $R^1$ is optionally substituted with one, two, three, or four instances of $R^4$; each of $R^a$ and $R^b$ is independently selected from the group consisting of H, halo, -CN, -OH, -$NO_2$, -$N(R^5)_2$, $C_1$-$C_6$ alkyl, $C_1$-$C_6$ haloalkyl, $C_1$-$C_6$ alkoxy, and $C_1$-$C_6$ haloalkoxy; each of $R^c$, $R^d$, $R^e$, and $R^f$ is independently selected from the group consisting of H, $C_1$-$C_6$ alkyl, $C_1$-$C_6$ haloalkyl, $C_1$-$C_6$ alkoxy, and $C_1$-$C_6$ haloalkoxy; or $R^c$ and $R^e$ may form, together with the carbons to which they are attached, a $C_1$-$C_3$ alkylene bridge; or $R^d$ and $R^f$ may form, together with the carbons to which they are attached, a $C_1$-$C_3$ alkylene bridge; each $R^4$ is independently selected from the group consisting of halo, -CN, -OH, -$NO_2$, -$N(R^5)_2$, - $S(O)_2R^5$, $C_1$-$C_6$ alkyl, $C_1$-$C_6$ haloalkyl, $C_1$-$C_6$ alkoxy, $C_1$-$C_6$ haloalkoxy, $C_6$-$C_{10}$ aryl, $C_3$-$C_7$ cycloalkyl, and 3-7 membered heterocyclyl; each $R^5$ is independently selected from H and $C_1$-$C_6$ alkyl; each $R^2$ is independently selected from the group consisting of halo, -CN, -OH, - $NO_2$, -$N(R^5)_2$, $C_1$-$C_6$ alkyl, $C_3$-$C_7$ cycloalkyl, $C_1$-$C_6$ haloalkyl, $C_1$-$C_6$ alkoxy, and $C_1$-$C_6$ - haloalkoxy; each $R^3$ is independently selected from the group consisting of halo, -CN, -OH, - $NO_2$, -$N(R^5)_2$, $C_1$-$C_6$ alkyl, $C_1$-$C_6$ haloalkyl, $C_1$-$C_6$ alkoxy, $C_3$-$C_7$ cycloalkyl, and $C_1$-$C_6$ - haloalkoxy; A is a 5-6 membered nitrogen-containing heteroaryl; B is selected from $C_6$-$C_{10}$ aryl and 5-6 membered heteroaryl; m is 0, 1, 2, or 3; n is 1, 2, 3, or 4; o is 0, 1, 2, or 3; and p is 0, 1, or 2.
Described herein, but not presently claimed, are compounds of Formula I in which $R^a$ and $R^b$ may form, together with the carbon to which they are attached, a $C_3$-$C_7$ cycloalkyl. Also described herein, but not presently claimed, are compounds of Formula I in which n is 0.

In some embodiments, the compound of Formula I is a compound of Formula I-a:

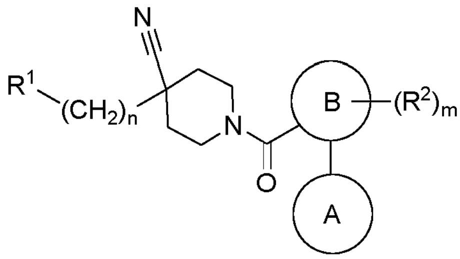

(I-a),

or a pharmaceutically acceptable salt thereof.

In some embodiments, the compound of Formula I is a compound of Formula I-b:

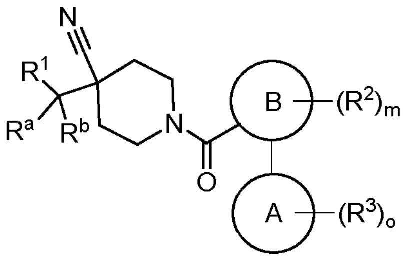

(I-b),

or a pharmaceutically acceptable salt thereof.

In some embodiments, the compound of Formula I is a compound of Formula I-c:

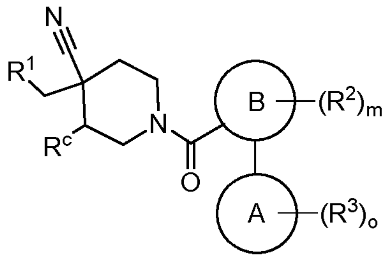

(I-c),

or a pharmaceutically acceptable salt thereof.

In some embodiments, the compound of Formula I is a compound of Formula I-d:

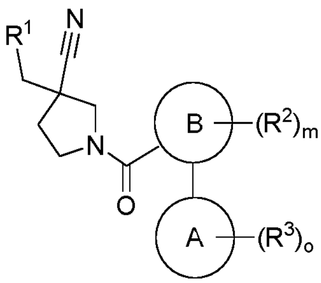

(I-d),

or a pharmaceutically acceptable salt thereof.

In some embodiments, the compound of Formula I is a compound of Formula I-e:

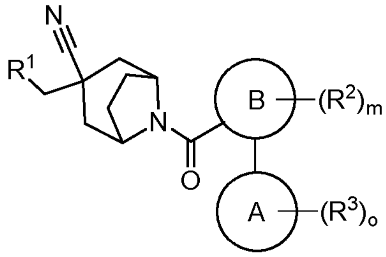

(I-e),

or a pharmaceutically acceptable salt thereof.

In some embodiments, the compound of Formula I is a compound of Formula I-f:

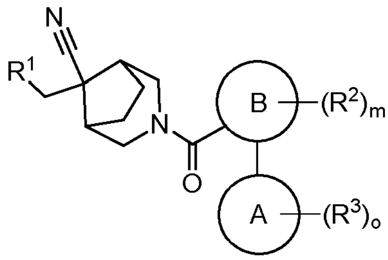

(I-f),

or a pharmaceutically acceptable salt thereof.

Also provided herein are pharmaceutical compositions comprising compounds described herein and pharmaceutically acceptable excipients. In an embodiment, provided herein is a method of treating a disorder described herein in a subject in need thereof, the method comprising administering to the subject a therapeutically effective amount of the compound. In some embodiments, the disorder is neurodegenerative disease (e.g. Alzheimer's disease, mild cognitive impairment, Huntington's disease, Parkinson's disease, amyotrophic lateral sclerosis, traumatic brain injury, cerebral infarction, glaucoma, and multiple sclerosis), epilepsy, developmental and epileptic encephalopathies, psychiatric disorders (e.g. schizophrenia and autism spectrum disorder), and spasm.

## Brief Description of the Drawings

**FIG. 1** shows a dose response curve for inhibition of human CYP46A1 by 4-(4-fluorobenzyl)-1-(2-(pyrimidin-4-yl) nicotinoyl)piperidine-4-carbonitrile (A6).

## Detailed Description of Certain Embodiments of the Invention

As generally described herein, the present invention provides compounds designed, for example, to act as CYP46A1 inhibitors. In certain embodiments, such compounds are useful as therapeutic agents for treating neurodegenerative disease (e.g. Alzheimer's disease, mild cognitive impairment, Huntington's disease, Parkinson's disease, amyotrophic lateral sclerosis, traumatic brain injury, cerebral infarction, glaucoma, and multiple sclerosis), epilepsy, developmental and epileptic encephalopathies, psychiatric disorders (e.g. schizophrenia and autism spectrum disorder), and spasm.

### *Compounds*

Compounds of Formula I are described herein. In embodiments, the compounds inhibit CYP46A1 and can be used in the treatment of neurodegenerative diseases, epilepsy, developmental and epileptic encephalopathies, psychiatric disorders, and spasm. For example, in an aspect, described herein are compounds of Formula I:

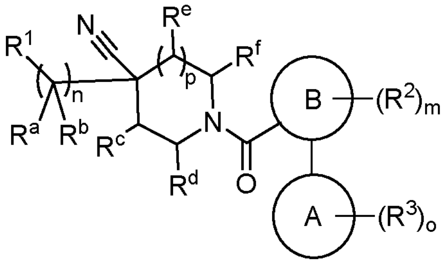

(I),

or pharmaceutically acceptable salts thereof; wherein: $R^1$ is selected from the group consisting of $C_6$-$C_{10}$ aryl, $C_3$-$C_7$ cycloalkyl, 3-7 membered heterocyclyl, and 5-10 membered heteroaryl, wherein $R^1$ is optionally substituted with one, two, three, or four instances of $R^4$; each of $R^a$ and $R^b$ is independently selected from the group consisting of H, halo, -CN, -OH, -$NO_2$, -$N(R^5)_2$, $C_1$-$C_6$ alkyl, $C_1$-$C_6$ haloalkyl, $C_1$-$C_6$ alkoxy, and $C_1$-$C_6$ haloalkoxy; each of $R^c$, $R^d$, $R^e$, and $R^f$ is independently selected from the group consisting of H, $C_1$-$C_6$ alkyl, $C_1$-$C_6$ haloalkyl, $C_1$-$C_6$ alkoxy, and $C_1$-$C_6$ haloalkoxy; or $R^c$ and $R^e$ may form, together with the carbons to which they are attached, a $C_1$-$C_3$ alkylene bridge; or $R^d$ and $R^f$ may form, together with the carbons to which they are attached, a $C_1$-$C_3$ alkylene bridge; each $R^4$ is independently selected from the group consisting of halo, -CN, -OH, -$NO_2$, -$N(R^5)_2$, - $S(O)_2R^5$, $C_1$-$C_6$ alkyl, $C_1$-$C_6$ haloalkyl, $C_1$-$C_6$ alkoxy, $C_1$-$C_6$ haloalkoxy, $C_6$-$C_{10}$ aryl, $C_3$-$C_7$ cycloalkyl, and 3-7 membered heterocyclyl; each $R^5$ is independently selected from H and $C_1$-$C_6$ alkyl; each $R^2$ is independently selected from the group consisting of halo, -CN, -OH, - $NO_2$, -$N(R^5)_2$, $C_1$-$C_6$ alkyl, $C_3$-$C_7$ cycloalkyl, $C_1$-$C_6$ haloalkyl, $C_1$-$C_6$ alkoxy, and $C_1$-$C_6$ - haloalkoxy; each $R^3$ is independently selected from the group consisting of halo, -CN, -OH, - $NO_2$, -$N(R^5)_2$, $C_1$-$C_6$ alkyl, $C_1$-$C_6$ haloalkyl, $C_1$-$C_6$ alkoxy, $C_3$-$C_7$ cycloalkyl, and $C_1$-$C_6$ - haloalkoxy; A is a 5-6 membered nitrogen-containing heteroaryl; B is selected from $C_6$-$C_{10}$ aryl and 5-6 membered heteroaryl; m is 0, 1, 2, or 3; n is 1, 2, 3, or 4; o is 0, 1, 2, or 3; and p is 0, 1, or 2.

Described herein, but not presently claimed, are compounds of Formula I in which $R^a$ and $R^b$ may form, together with the carbon to which they are attached, a $C_3$-$C_7$ cycloalkyl. Also described herein, but not presently claimed, are compounds of Formula I in which n is 0.

*Group 1*

In some embodiments, $R^1$ is substituted $C_6$-$C_{10}$ aryl. In some embodiments, $R^1$ is unsubstituted $C_6$-$C_{10}$ aryl.

In some embodiments, $R^1$ is

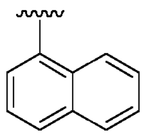

or

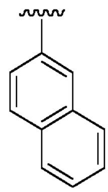

.

In some embodiments, $R^1$ is

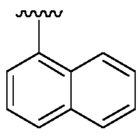

.

In some embodiments, $R^1$ is

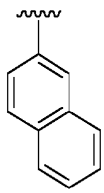

.

In some embodiments, $R^1$ is

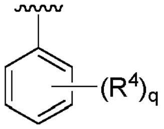

,

wherein each $R^4$ is independently halo, -CN, -OH, $-NO_2$, $-N(R^5)_2$, $-S(O)_2R^5$, $C_1$-$C_6$ alkyl, $C_1$-$C_6$ haloalkyl, $C_1$-$C_6$ alkoxy, $C_1$-$C_6$ - haloalkoxy, $C_6$-$C_{10}$ aryl, $C_3$-$C_7$ cycloalkyl, or 3-7 membered heterocyclyl; wherein each $R^5$ is independently H or $C_1$-$C_6$ alkyl; and q is 0, 1, 2, or 3.

In embodiments, $R^1$ is

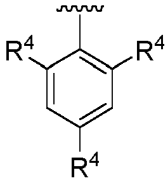

.

In certain embodiments, $R^1$ is

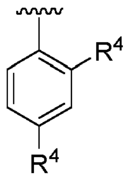

,

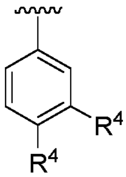

,

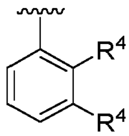

,

or

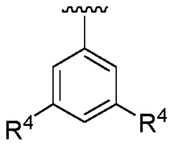

.

In embodiments, $R^1$ is

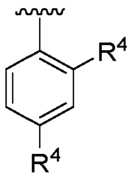

.

In some embodiments, $R^1$ is

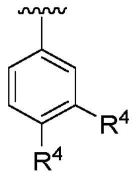
.

In some embodiments, $R^1$ is

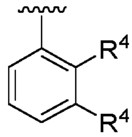
.

In some embodiments, $R^1$ is

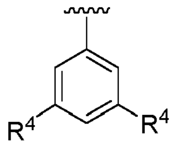
.

In some embodiments, $R^1$ is

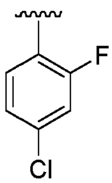
,
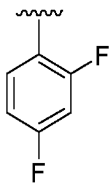
,
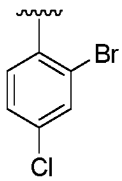
,
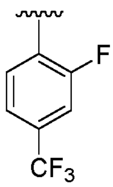
,
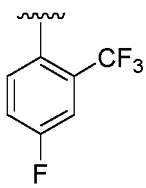
,
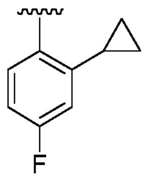
,

or

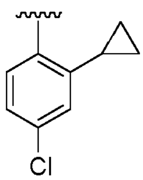
.

In an embodiment, $R^1$ is

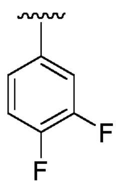
,

,

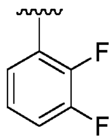, 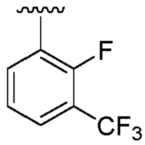,

or

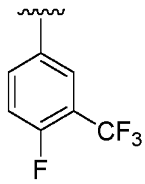.

In an embodiment, $R^1$ is

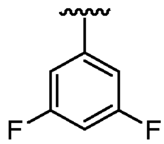

or

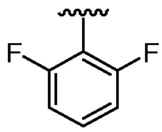.

In an embodiment, $R^1$ is

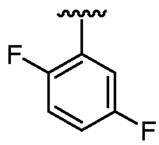

or

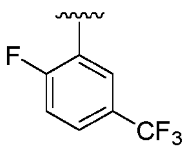.

In some embodiments, $R^1$ is

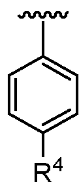.

In embodiments, $R^1$ is

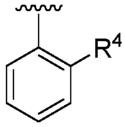.

In embodiments, $R^1$ is

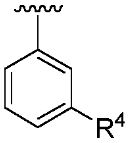.

In embodiments, $R^1$ is:

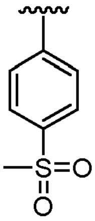, 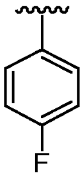, 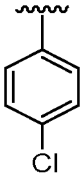, 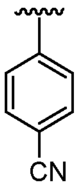, 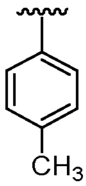, 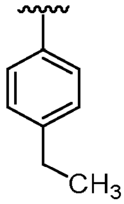, 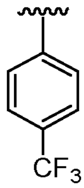, 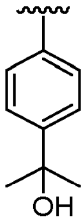, 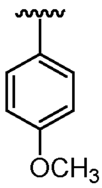, 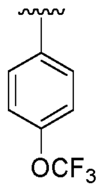, 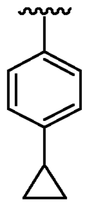,

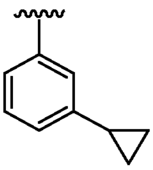, 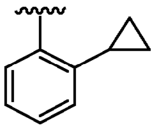, 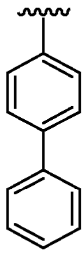, 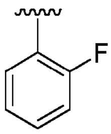, 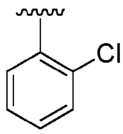, 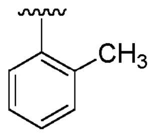, 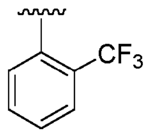, 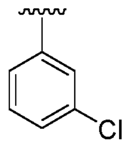,

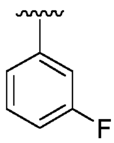,

or

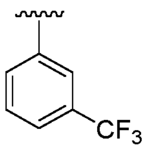.

In embodiments, $R^1$ is substituted 5-10 membered heteroaryl. In embodiments, $R^1$ is unsubstituted 5-10 membered heteroaryl. In some embodiments, $R^1$ is pyrrolyl, furanyl, thiophenyl, imidazolyl, pyrazolyl, oxazolyl, isoxazolyl, thiazolyl, isothiazolyl, triazolyl, oxadiazolyl, thiadiazolyl, pyridyl, 2-pyridyl, 3-pyridyl, 4-pyridyl, pyridinyl, pyridazinyl, pyrimidinyl, 2-pyrimidinyl, 4-pyrimidinyl, pyrazinyl, triazinyl, tetrazinyl, tetrazolyl, azocinyl, dithiazinyl, or oxazinyl. In some embodiments, $R^1$ is pyridyl, 2-pyridyl, 3-pyridyl, 4-pyridyl, pyridinyl, pyridazinyl, pyrimidinyl, 2-pyrimidinyl, 4-pyrimidinyl, pyridazinyl, or pyrazinyl.

In embodiments, $R^1$ is

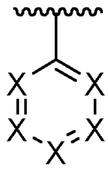,

wherein each X is independently CH or N, wherein the H of CH may be substituted with one or more instances of $R^4$; wherein each $R^4$ is independently halo, -CN, -OH, $-NO_2$, $-N(R^5)_2$, $C_1$-$C_6$ alkyl, $C_1$-$C_6$ haloalkyl, $C_1$-$C_6$ alkoxy, $C_1$-$C_6$ haloalkoxy, $C_6$-$C_{10}$ aryl, $C_3$-$C_7$ cycloalkyl, or 3-7 membered heterocyclyl; and each $R^5$ is independently H or $C_1$-$C_6$ alkyl. In certain embodiments, $R^1$ is

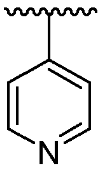, 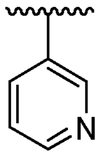,

or

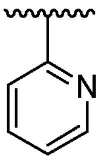.

In some embodiments, $R^1$ is:

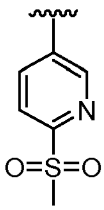, 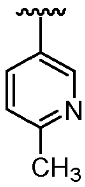, 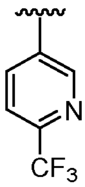, 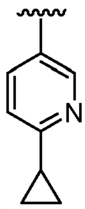, 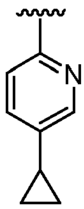, 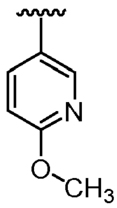, 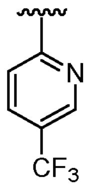, 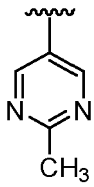, or 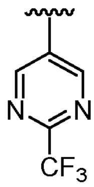.

In some embodiments, $R^1$ is substituted $C_3$-$C_7$ cycloalkyl. In some embodiments, $R^1$ is unsubstituted $C_3$-$C_7$ cycloalkyl. In certain embodiments, $R^1$ is cyclopropyl or cyclobutyl. In certain embodiments, $R^1$ is

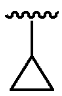.

In some embodiments, $R^1$ is

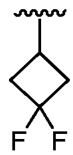.

In some embodiments, $R^1$ is substituted 3-7 membered heterocyclyl. In some embodiments, $R^1$ is unsubstituted 3-7 membered heterocyclyl. In embodiments, $R^1$ is tetrahydrofuran, tetrahydropyran, pyrrolidine, piperidine, piperazine, dioxolane, dioxane, thiomorpholine, or dithiane. In embodiments, $R^1$ is tetrahydrofuran or tetrahydropyran. In certain embodiments, $R^1$ is

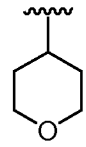

.

*Group $R^4$*

In some embodiments, $R^4$ is independently substituted $C_1$-$C_6$ alkyl, substituted $C_1$-$C_6$ alkoxy, or substituted $C_3$-$C_7$ cycloalkyl. In embodiments, $R^4$ is independently unsubstituted $C_1$-$C_6$ alkyl, unsubstituted $C_1$-$C_6$ alkoxy, or unsubstituted $C_3$-$C_7$ cycloalkyl. In embodiments, $R^4$ is independently halo, $-NH_2$, $-NH(C_1$-$C_6$ alkyl), $-N(C_1$-$C_6$ alkyl$)_2$, $C_1$-$C_6$ alkyl, $C_1$-$C_6$ haloalkyl, $C_1$-$C_6$ alkoxy, $C_1$-$C_6$ haloalkoxy, or $C_6$-$C_{10}$ aryl. In some embodiments, $R^4$ is independently halo, $C_1$-$C_6$ alkyl, $C_1$-$C_6$ haloalkyl, $C_1$-$C_6$ alkoxy, $C_1$-$C_6$ haloalkoxy, or $C_6$-$C_{10}$ aryl. In certain embodiments, $R^4$ is independently halo, -CN, $-CH_3$, $-CH_2CH_3$, $-CF_3$, $-OCH_3$, - $OCF_3$, $-C(CH_3)_2OH$, or $-C_6H_5$. In embodiments, $R^4$ is independently Cl, F, Br, or I.

*Group B*

In certain embodiments, B is

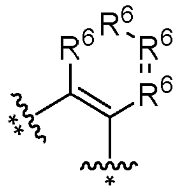

;

wherein each $R^6$ is independently N or $CR^{6a}$, wherein $R^{6a}$ is H or $R^2$; and ** is the point of attachment to a carbonyl, and * is the point of attachment to A.

In some embodiments, $R^2$ is halo, -CN, -OH, $-NO_2$, $-CH_3$, $-CH_2CH_3$, $-CHF_2$, $-CF_3$, $-OCF_3$, $-OCH_3$, $-OCH_2CH_3$, or $-OCH_2CF_3$. In embodiments, up to two $R^6$ may be N and the other occurences of $R^6$ are CH. In some embodiments, B is:

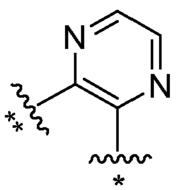
,
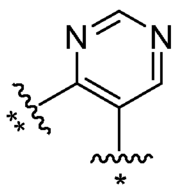
,
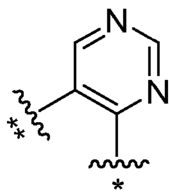
,
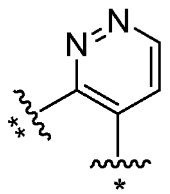
,
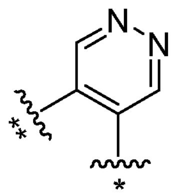
,
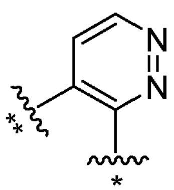
,
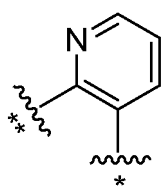
,
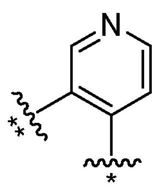
,
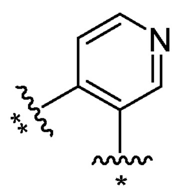
,
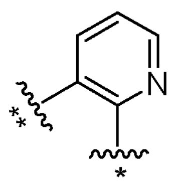
,

or

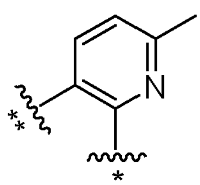

;

wherein ** is the point of attachment to a carbonyl, and * is the point of attachment to A.

In certain embodiments, B is

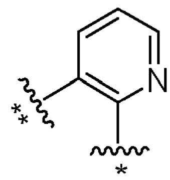

;

wherein ** is the point of attachment to a carbonyl, and * is the point of attachment to A. In embodiments, B is

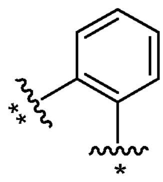

,

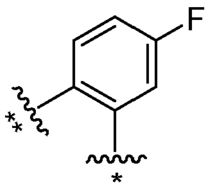

,

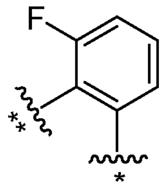

,

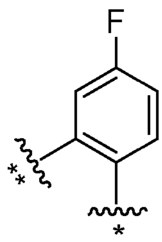

,

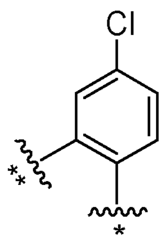

,

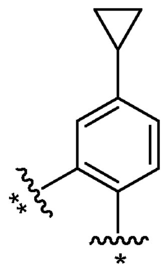

,

or

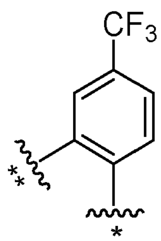

;

wherein ** is the point of attachment to a carbonyl, and * is the point of attachment to A.

*Group $R^2$*

In some embodiments, $R^2$ is halo, -CN, -OH, $-NO_2$, $-CH_3$, $-CH_2CH_3$, cyclopropyl, $-CHF_2$, $-CF_3$, $-OCF_3$, $-OCH_3$, $-OCH_2CH_3$, or $-OCH_2CF_3$.

*Group A*

In certain embodiments, A is pyridinyl, pyrrolyl, imidazolyl, pyrazolyl, pyridazinyl, pyrimidinyl, pyrazinyl, triazolyl, triazinyl, tetrazinyl, tetrazolyl, oxazolyl, isoxazolyl, or thiozolyl. In embodiments, A is pyridinyl oxazolyl, imidazolyl, triazolyl, pyridinyl, pyridazinyl, pyrimidinyl, or triazinyl. In embodiments, A is

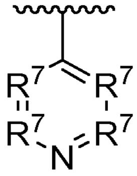
;

wherein each $R^7$ is independently N or CH, wherein up to two $R^7$ may be N and the other occurences of $R^7$ are CH. In some embodiments, A is

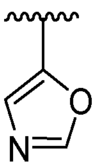 , 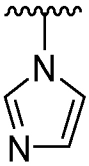 , 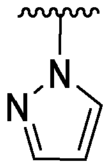 , 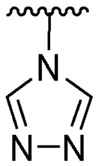 , 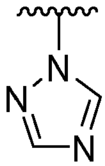 , 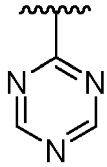 , 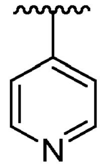 ,

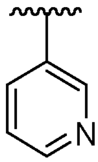 , 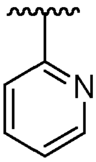 , 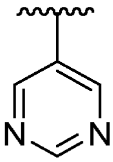 , 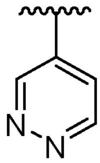 , 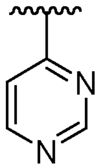 ,

or

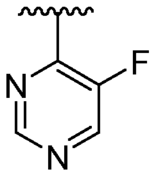
.

In some embodiments, A is

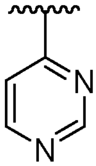 , 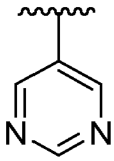 , 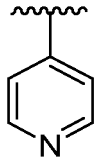 , 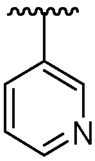 , 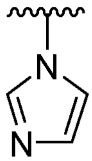 , 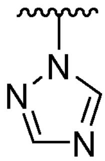 , 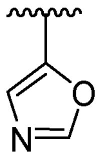 ,

or

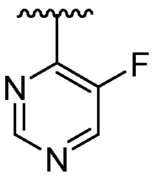
.

*n*

In some embodiments, n is 4. In some embodiments, n is 3. In some embodiments, n is 2. In some embodiments, n is 1. In some examples described herein but not presently claimed, n is 0.

In some embodiments, n is 1, and $R^a$ is $C_1$-$C_6$ alkyl and $R^b$ is H. In embodiments, n is 1, and $R^a$ is ethyl and $R^b$ is H. In embodiments, n is 1, and $R^a$ is methyl and $R^b$ is H. In some examples described herein but not presently claimed, n is 1, and $R^a$ and $R^b$ form together with the carbon to which they are attached, a cyclopropyl.

m

In some embodiments, m is 3. In some embodiments, m is 2. In some embodiments, m is 1. In some

embodiments, m is 0.

*o*

In some embodiments, o is 3. In some embodiments, o is 2. In some embodiments, o is 1. In some embodiments, o is 0.

*p*

In some embodiments, p is 2. In some embodiments, p is 1. In some embodiments, p is 0.

In some embodiments, p is 1, and $R^c$, $R^d$, $R^e$, and $R^f$ are H. In some embodiments, p is 1, $R^c$ is methyl, and $R^d$, $R^e$, and $R^f$ are H. In embodiments, p is 1, $R^c$ and $R^e$ are H, and $R^d$ and $R^f$ form together with the carbon to which they are attached, an $C_1$-$C_3$ alkylene bridge. In some embodiments, p is 1, $R^d$ and $R^f$ are H, and $R^c$ and $R^e$ form together with the carbon to which they are attached, an $C_1$-$C_3$ alkylene bridge. In some embodiments, p is 0, and $R^c$, $R^d$, and $R^f$ are H.

*q*

In some embodiments, q is 3. In some embodiments, q is 2. In some embodiments, q is 1. In some embodiments, q is 0.

In some embodiments, the compound is selected from the group consisting of the compounds identified in **Table 1** below, with the proviso that the compound is not A256, which is described but not claimed:

**Table 1.** Exemplary compounds and reference example A256.

| Compound No. | Structure |
|---|---|
| A6 | 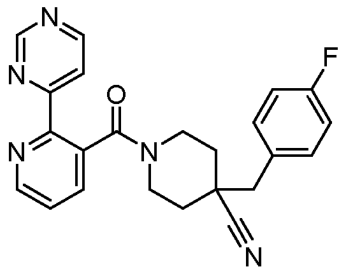 |
| A10 | 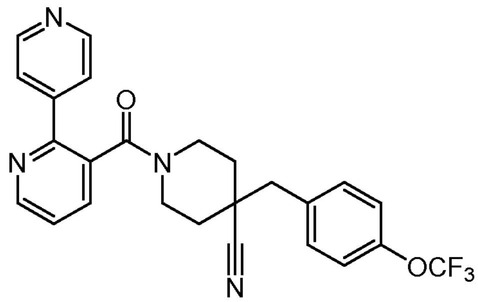 |
| A11 | 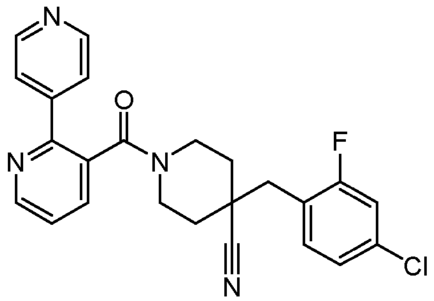 |

(continued)

| Compound No. | Structure |
|---|---|
| A12 | 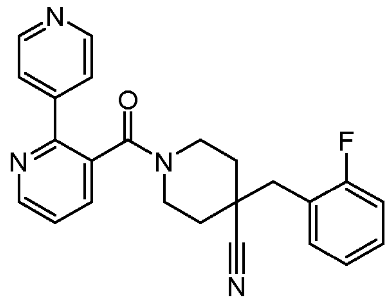 |
| A13 | 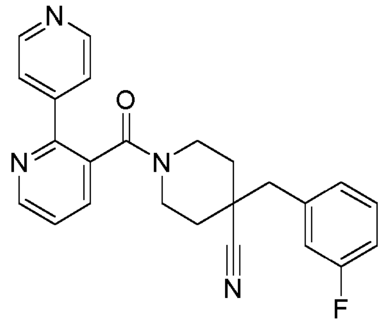 |
| A14 | 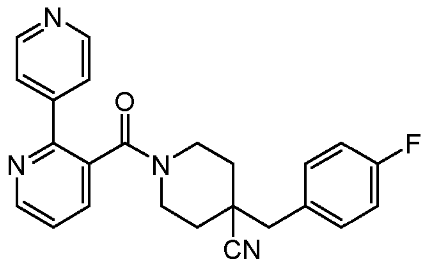 |
| A16 | 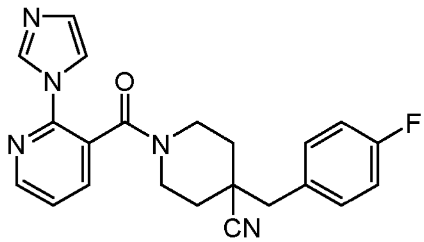 |
| A18 | 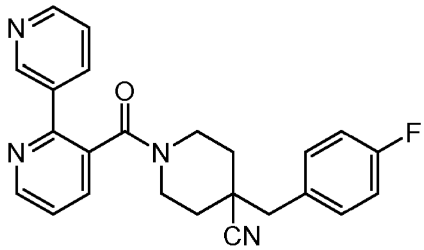 |

(continued)

| Compound No. | Structure |
|---|---|
| A19 | 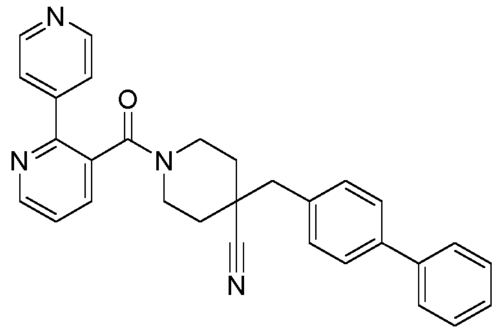 |
| A21 | 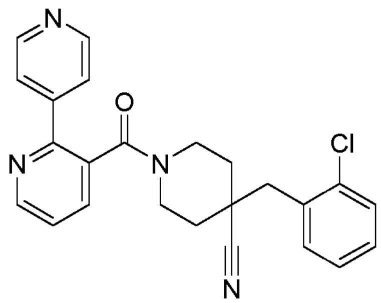 |
| A22 | 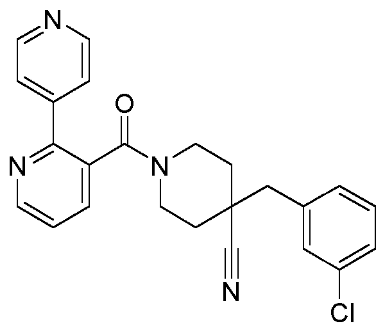 |
| A25 | 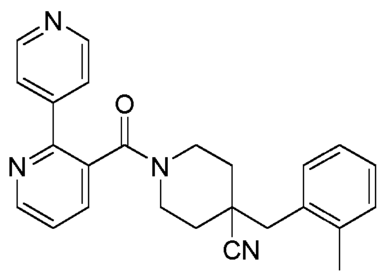 |
| A27 | 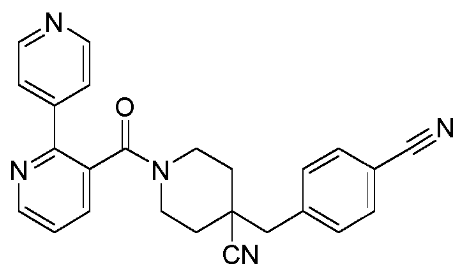 |

(continued)

| Compound No. | Structure |
|---|---|
| A31 | 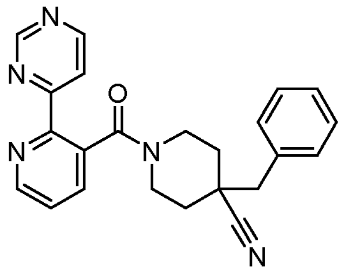 |
| A32 | 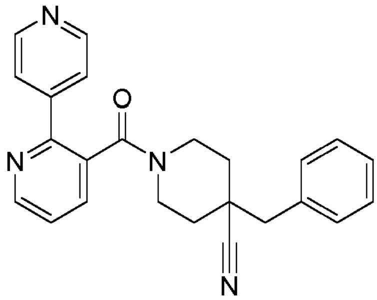 |
| A35 | 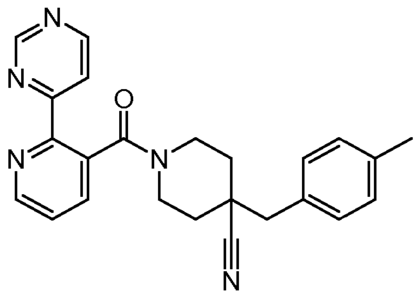 |
| A37 | 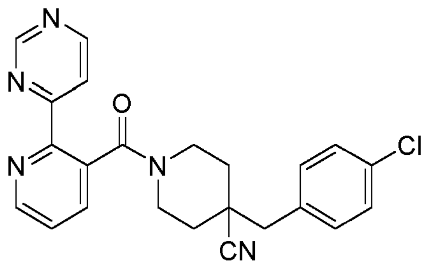 |
| A40 | 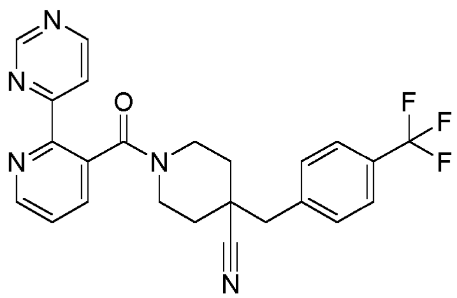 |

(continued)

| Compound No. | Structure |
|---|---|
| A43 | 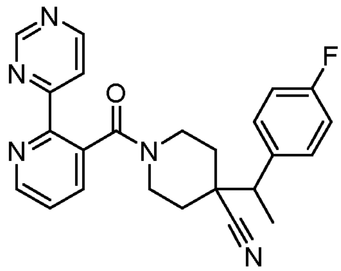 |
| A44 | 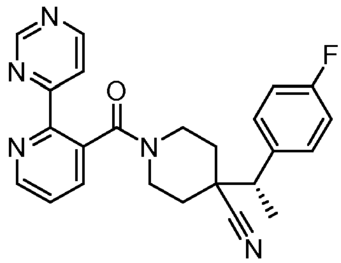 |
| A45 | 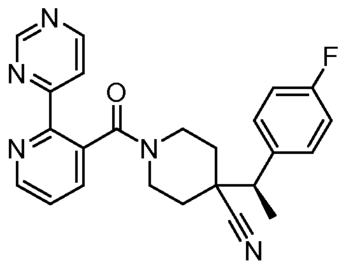 |
| A48 | 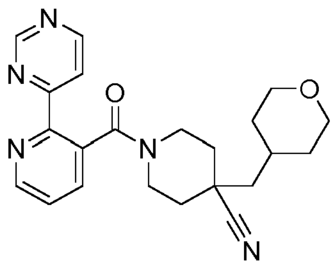 |
| A51 | 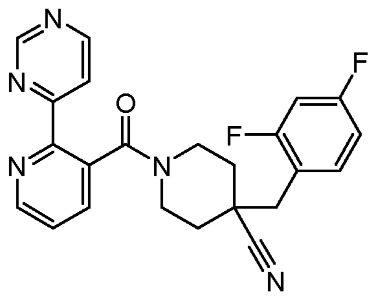 |
| A54 | 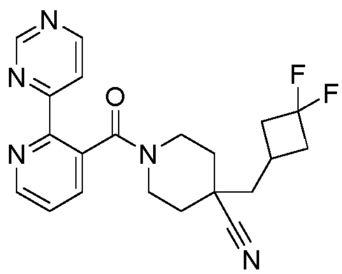 |

(continued)

| Compound No. | Structure |
|---|---|
| A56 | 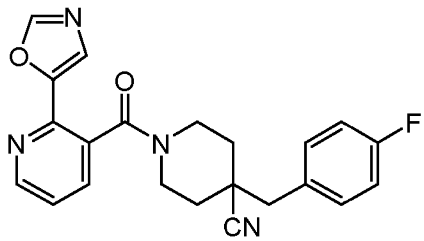 |
| A58 | 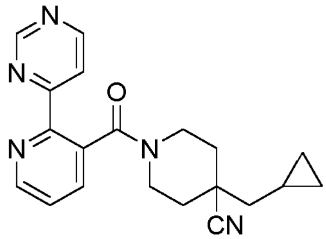 |
| A63 | 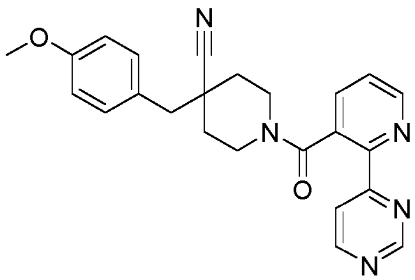 |
| A65 | 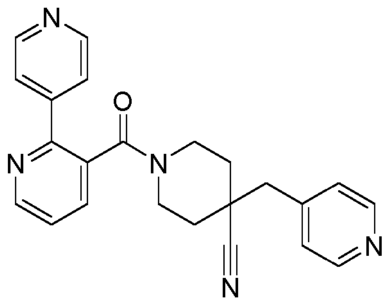 |
| A66 | 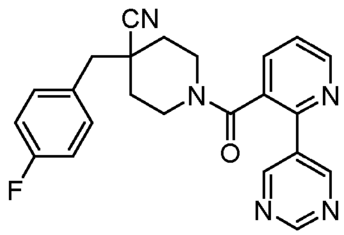 |
| A67 | 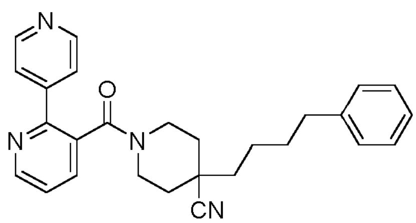 |

(continued)

| Compound No. | Structure |
|---|---|
| A68 | 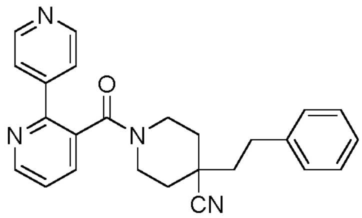 |
| A69 | 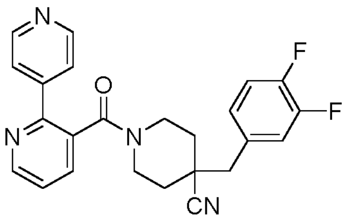 |
| A70 | 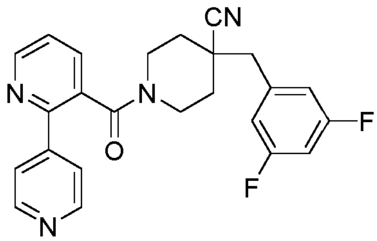 |
| A71 | 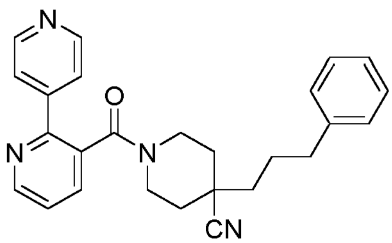 |
| A72 | 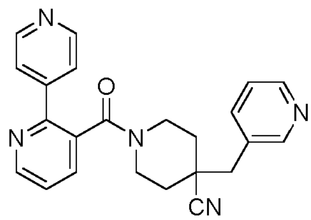 |
| A73 | 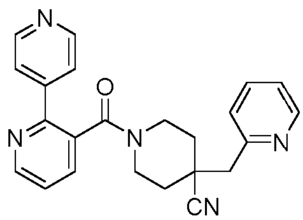 |
| A74 | 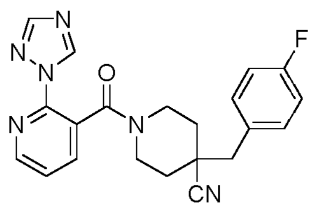 |

(continued)

| Compound No. | Structure |
|---|---|
| A200 | 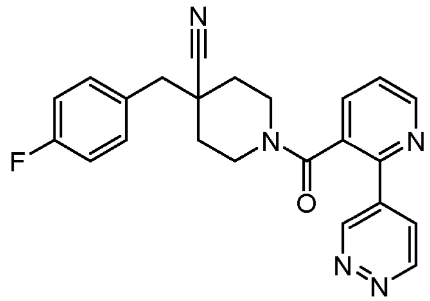 |
| A201 | 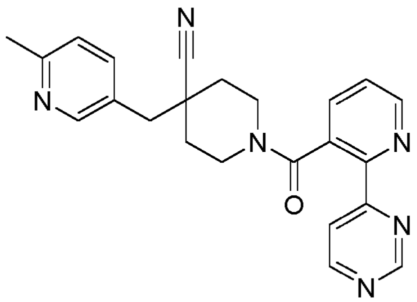 |
| A202 | 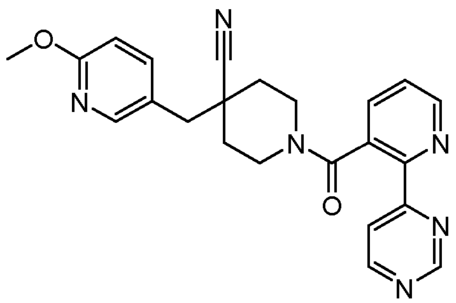 |
| A203 | 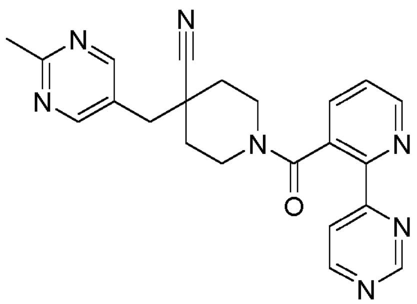 |
| A204 | 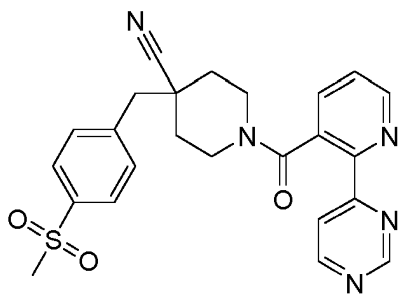 |

(continued)

| Compound No. | Structure |
|---|---|
| A205 | 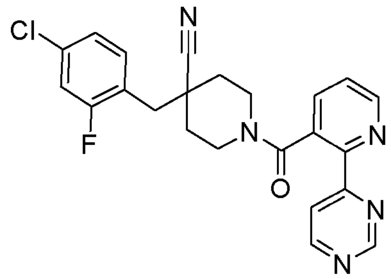 |
| A206 | 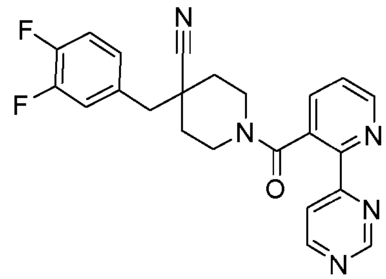 |
| A207 | 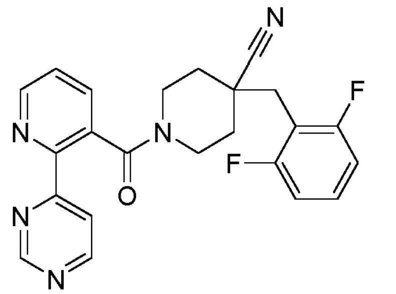 |
| A208 | 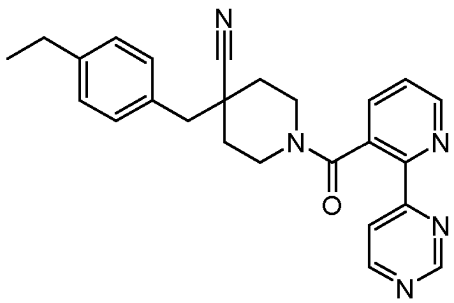 |
| A209 | 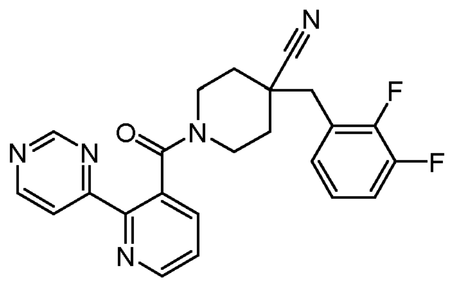 |

(continued)

| Compound No. | Structure |
|---|---|
| A210 | 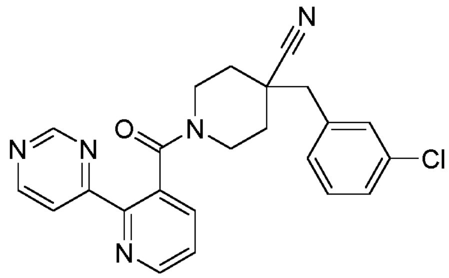 |
| A211 | 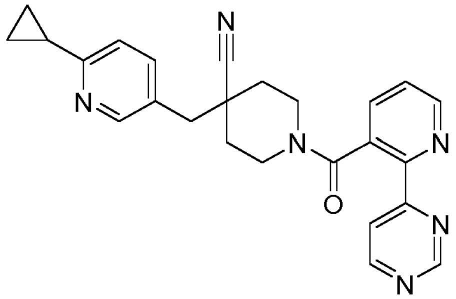 |
| A212 | 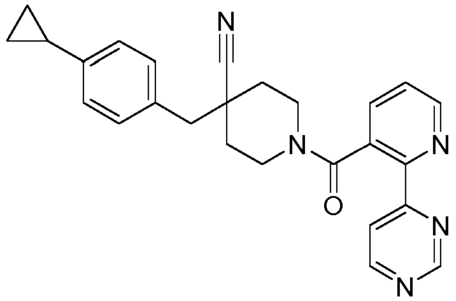 |
| A213 | 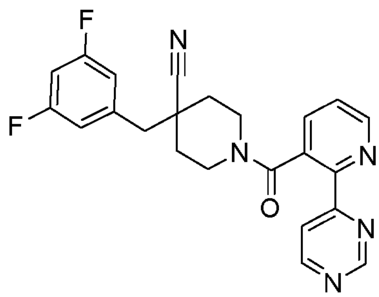 |
| A214 | 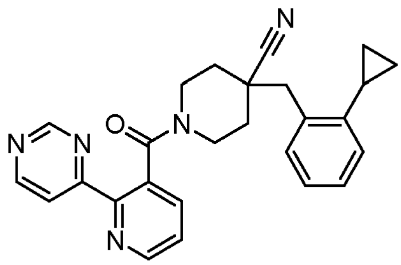 |

(continued)

| Compound No. | Structure |
|---|---|
| A215 | 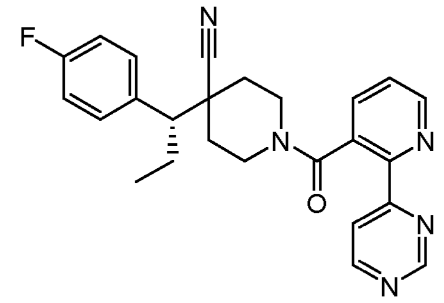 |
| A216 | 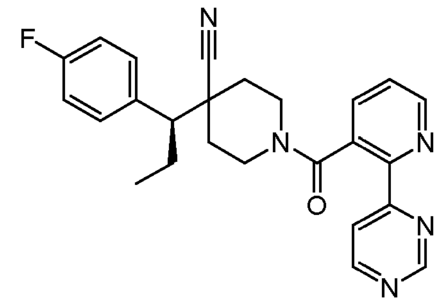 |
| A217 | 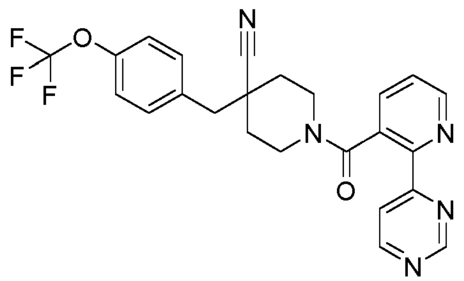 |
| A218 | 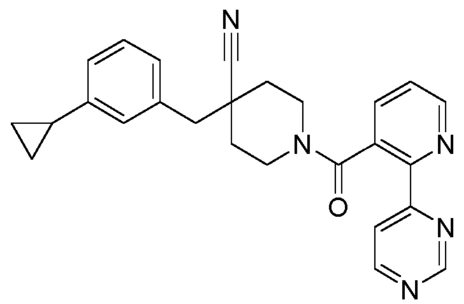 |
| A219 | 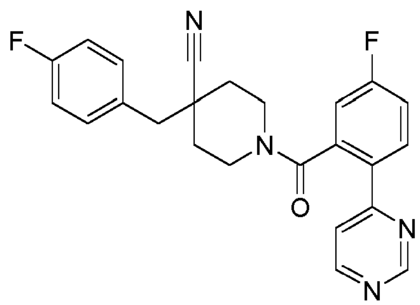 |

(continued)

| Compound No. | Structure |
|---|---|
| A220 | 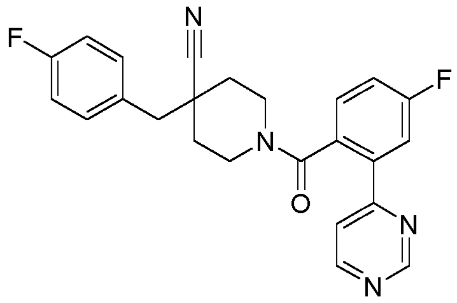 |
| A221 | 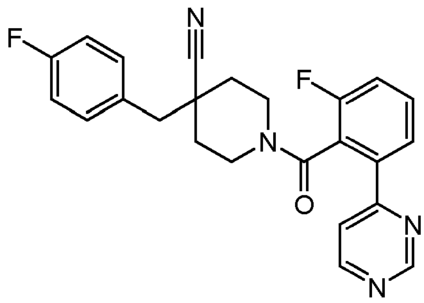 |
| A222 | 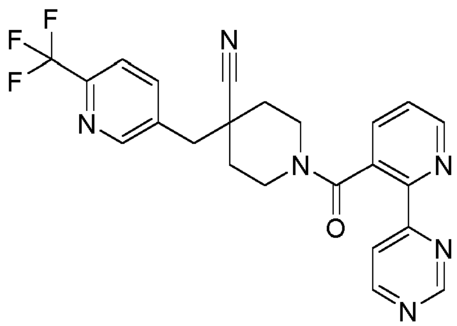 |
| A223 | 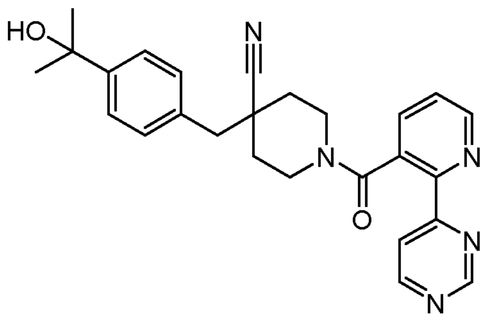 |
| A224 | 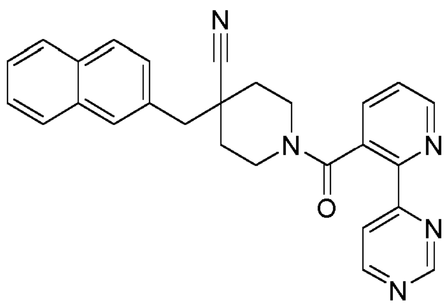 |

(continued)

| Compound No. | Structure |
|---|---|
| A225 | 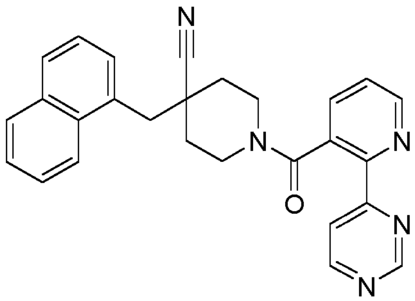 |
| A226 | 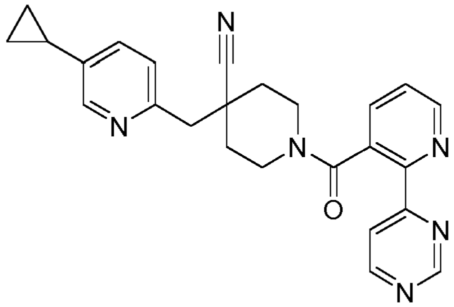 |
| A227 | 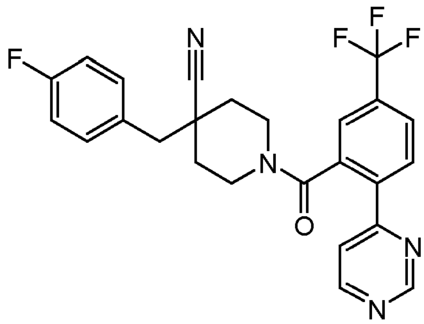 |
| A228 | 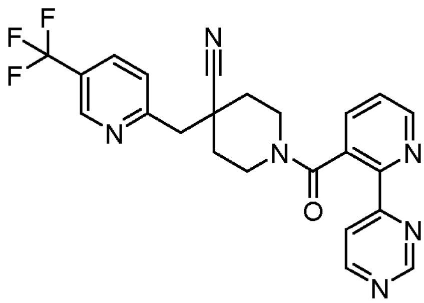 |
| A229 | 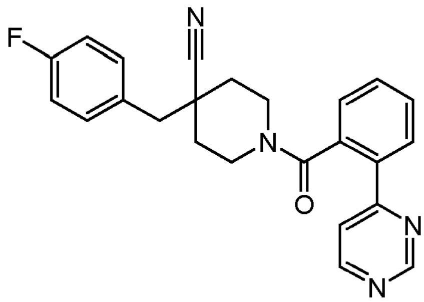 |

(continued)

| Compound No. | Structure |
|---|---|
| A230 | 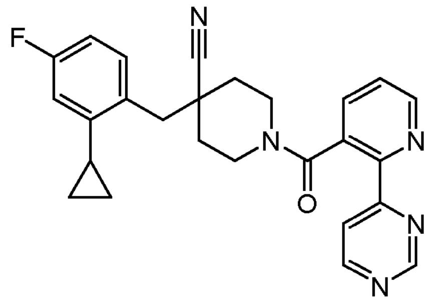 |
| A231 | 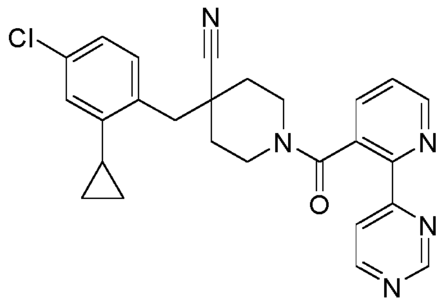 |
| A232 | 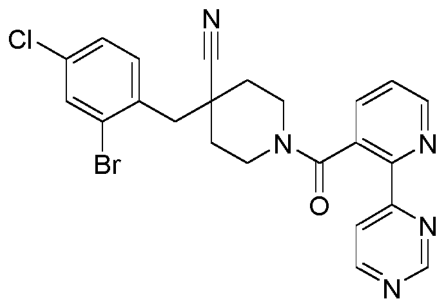 |
| A233 | 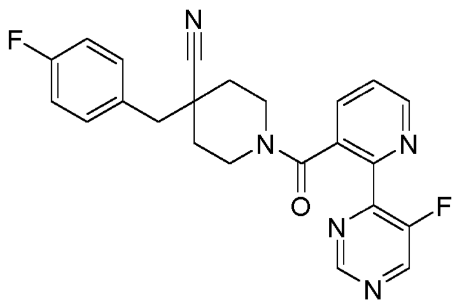 |
| A234 | 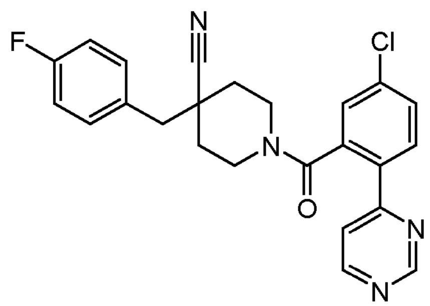 |

(continued)

| Compound No. | Structure |
|---|---|
| A235 | 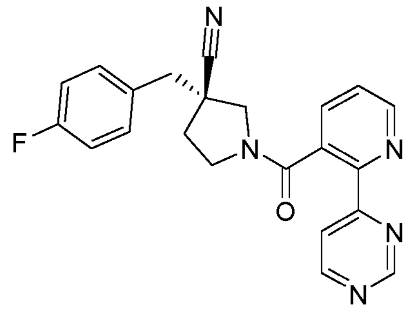 |
| A236 | 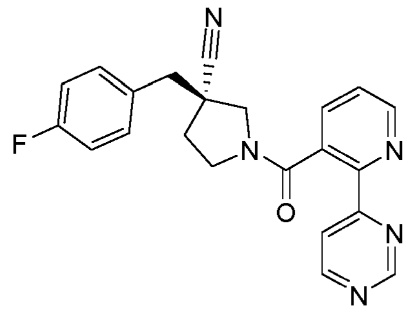 |
| A237 | 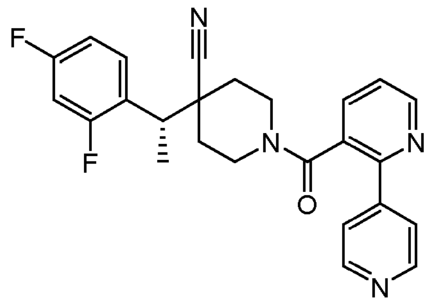 |
| A238 | 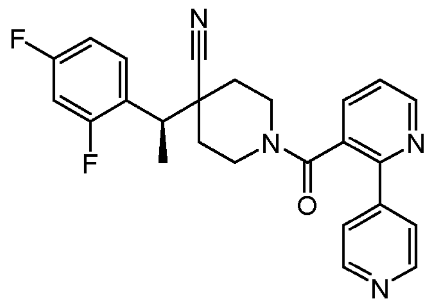 |
| A239 | 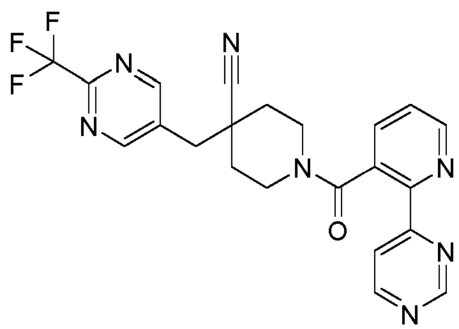 |

(continued)

| Compound No. | Structure |
|---|---|
| A240 | 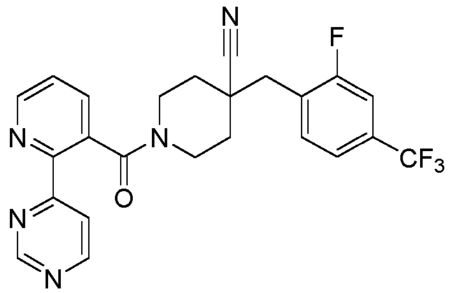 |
| A241 | 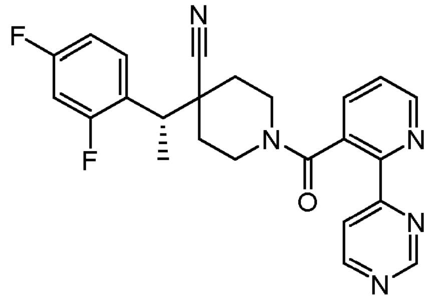 |
| A242 | 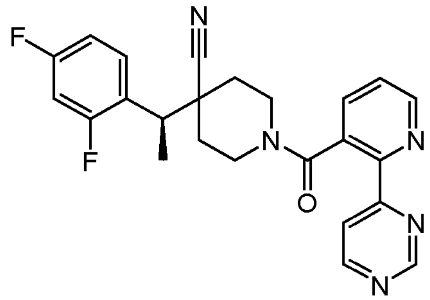 |
| A243 | 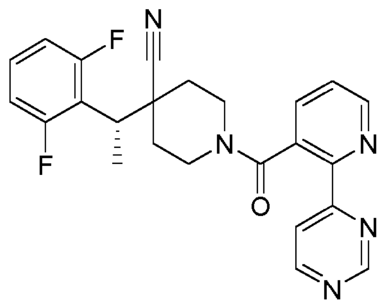 |
| A244 | 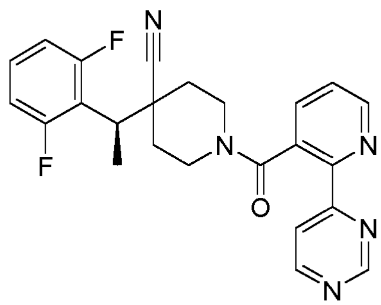 |

(continued)

| Compound No. | Structure |
|---|---|
| A245 | 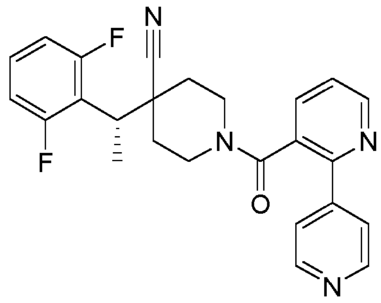 |
| A246 | 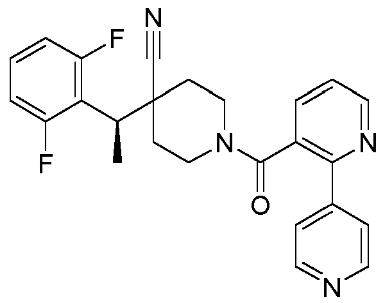 |
| A247 | 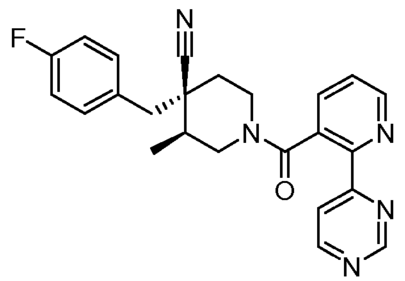 |
| A248 | 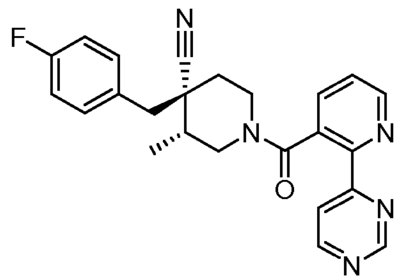 |
| A249 | 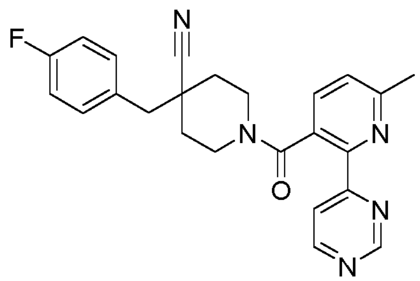 |

(continued)

| Compound No. | Structure |
|---|---|
| A250 | 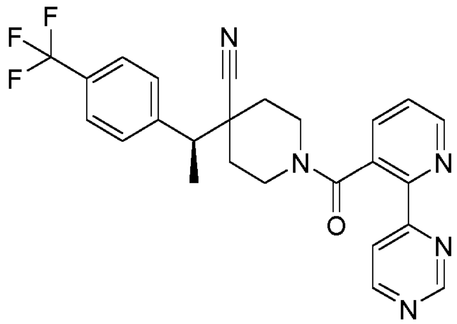 |
| A251 | 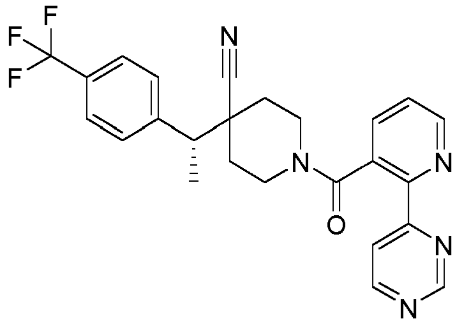 |
| A252 | 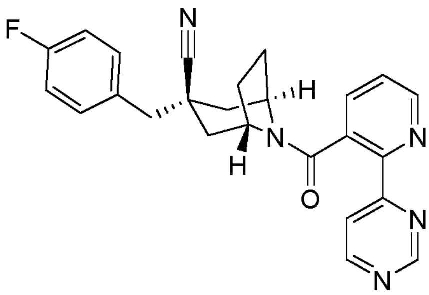 |
| A253 | 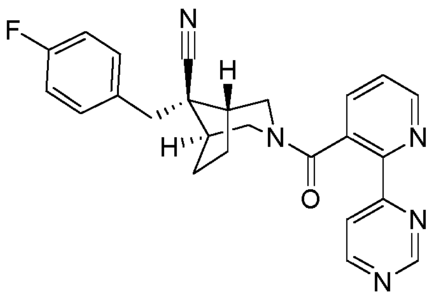 |
| A254 | 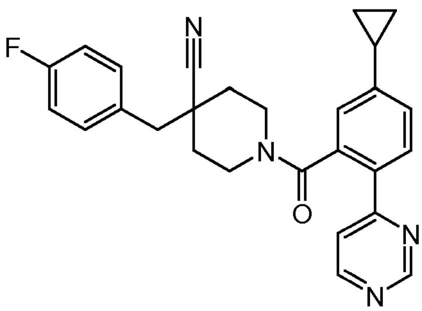 |

(continued)

| Compound No. | Structure |
|---|---|
| A255 | 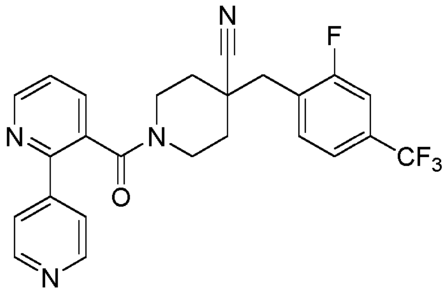 |
| A256* | 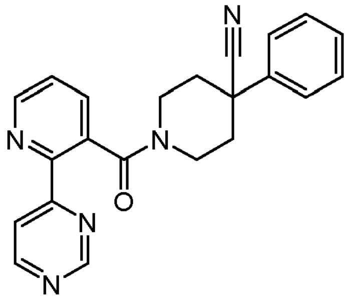 |
| A257 | 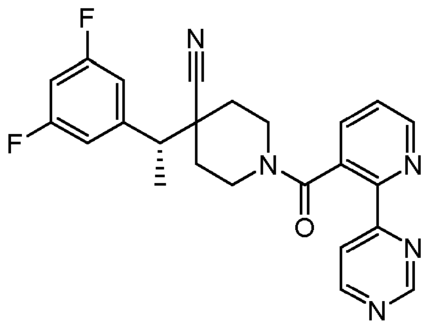 |
| A258 | 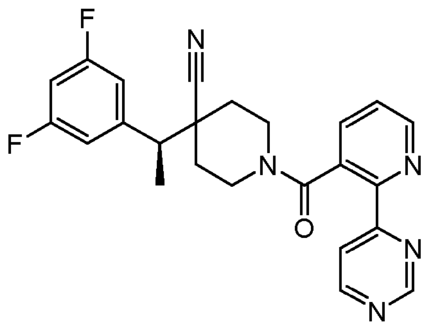 |
| A259 | 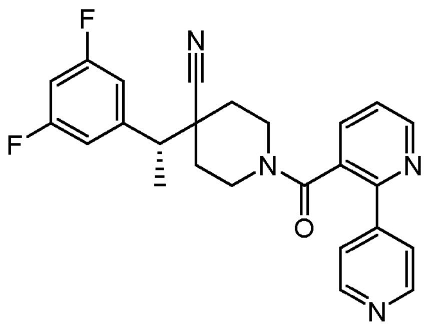 |

(continued)

| Compound No. | Structure |
|---|---|
| A260 | 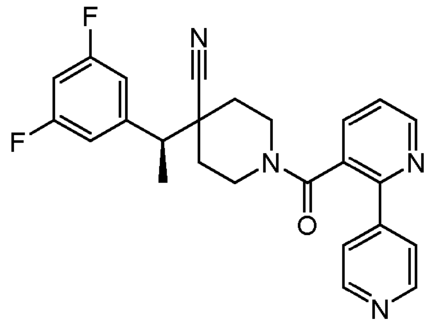 |
| A261 | 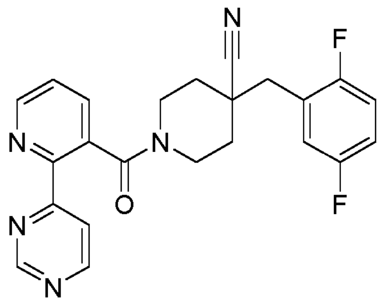 |
| A262 | 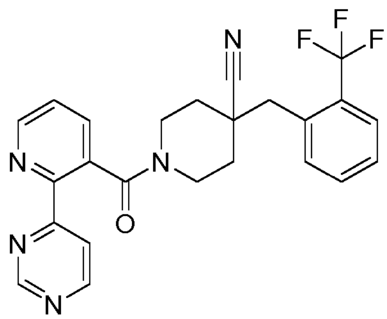 |
| A263 | 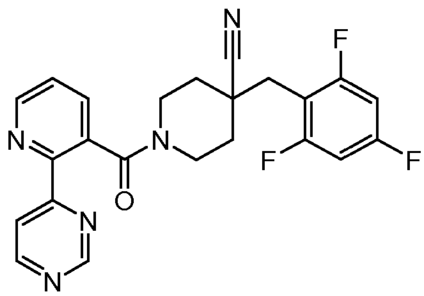 |
| A264 | 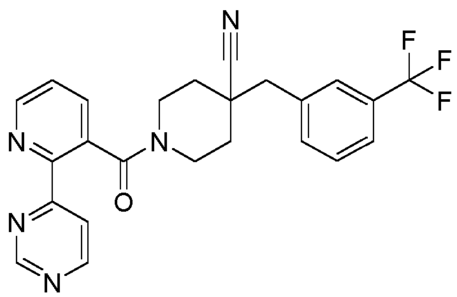 |

(continued)

| Compound No. | Structure |
|---|---|
| A265 | 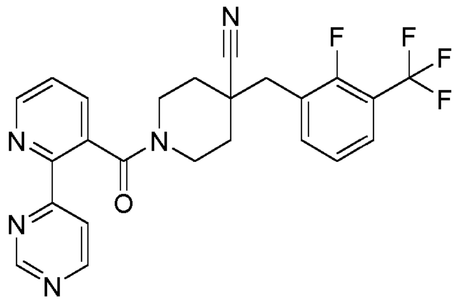 |
| A266 | 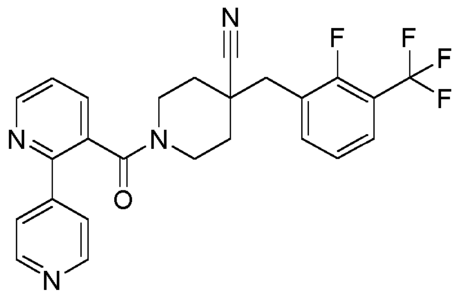 |
| A267 | 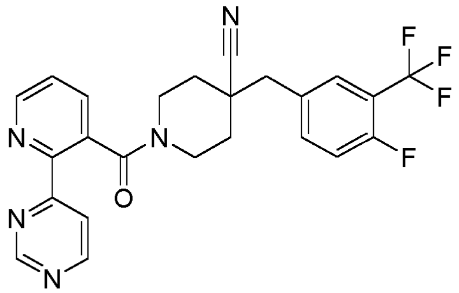 |
| A268 | 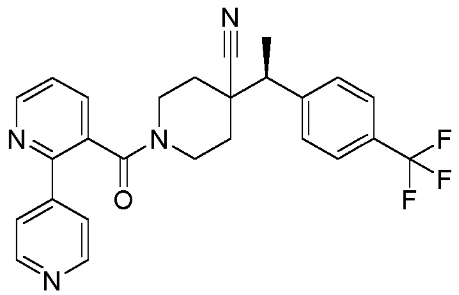 |
| A269 | 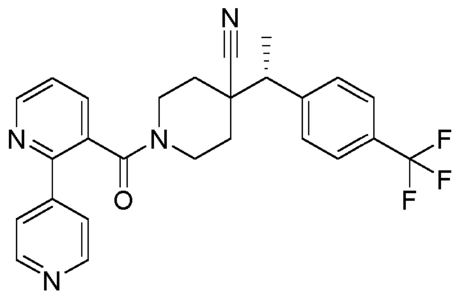 |

(continued)

| Compound No. | Structure |
|---|---|
| A270 | 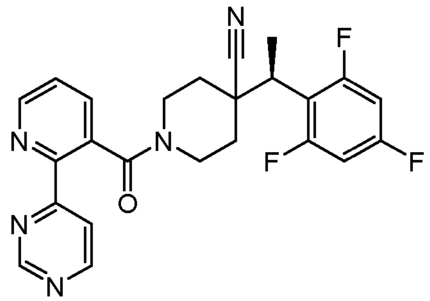 |
| A271 | 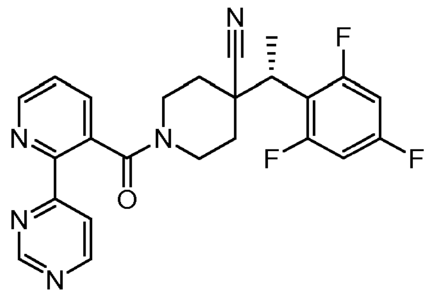 |
| A272 | 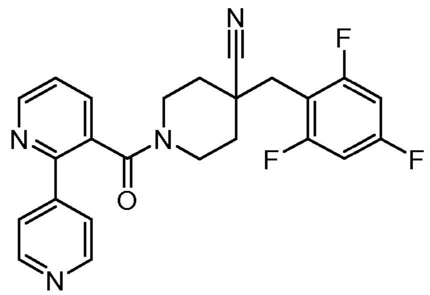 |
| A273 | 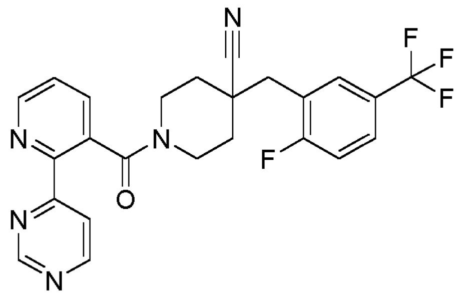 |
| A274 | 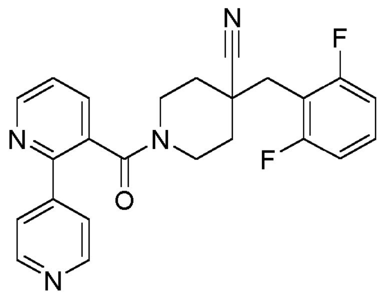 |

(continued)

| Compound No. | Structure |
|---|---|
| A275 | 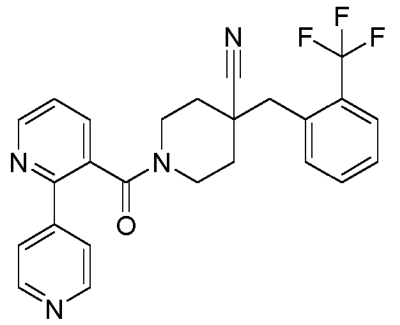 |
| A276 | 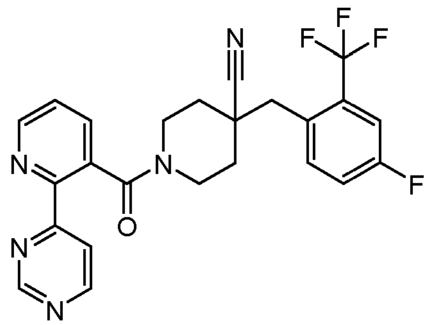 |
| A277 | 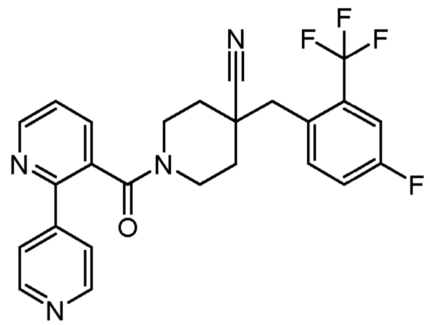 |
| A278 | 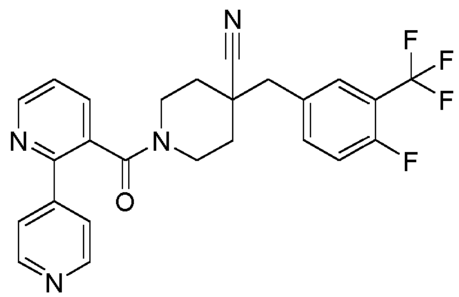 |
| A279 | 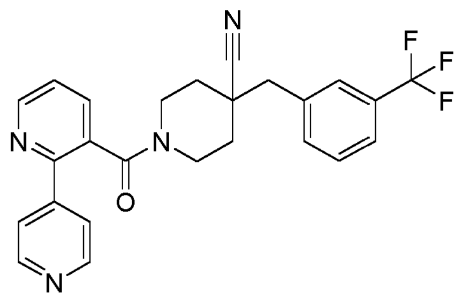 |

(continued)

| Compound No. | Structure |
|---|---|
| A280 | 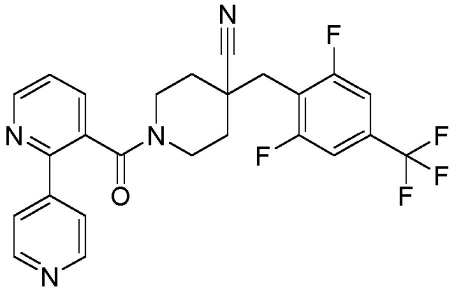 |
| A281 | 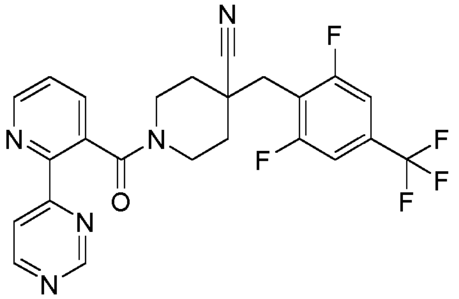 |
| A282 | 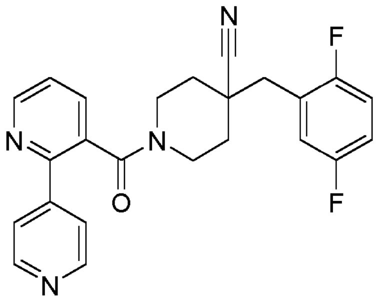 |
| A283 | 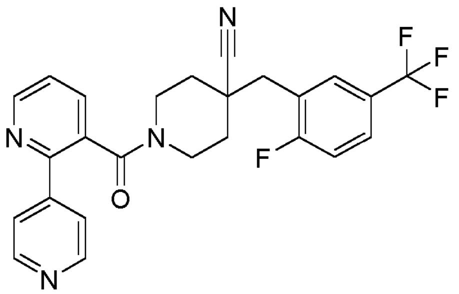 |
| A284 | 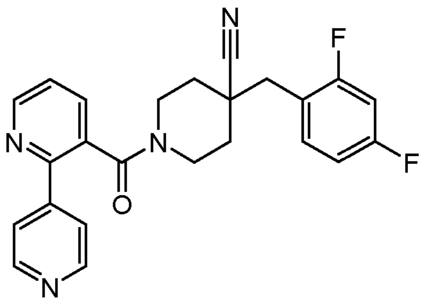 |

(continued)

| Compound No. | Structure |
|---|---|
| A285 | 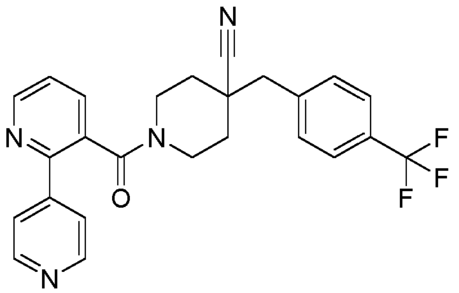 |
| A286 | 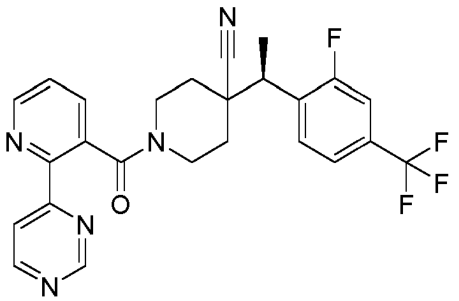 |
| A287 | 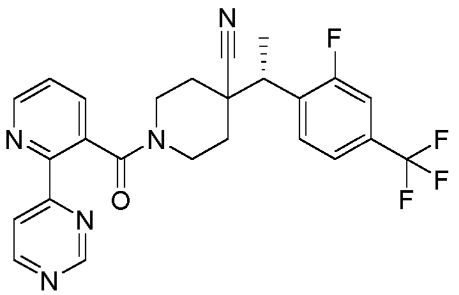 |
| *Reference example not falling within the scope of the present invention. | |

*Alternative Embodiments*

In an alternative embodiment, compounds of Formula I may also comprise one or more isotopic substitutions. For example, hydrogen may be $^{2}H$ (D or deuterium) or $^{3}H$ (T or tritium); carbon may be, for example, $^{13}C$ or $^{14}C$; oxygen may be, for example, $^{18}O$; nitrogen may be, for example, $^{15}N$, and the like. In other embodiments, a particular isotope (e.g., $^{3}H$, $^{13}C$, $^{14}C$, $^{18}O$, or $^{15}N$) can represent at least 1%, at least 5%, at least 10%, at least 15%, at least 20%, at least 25%, at least 30%, at least 35%, at least 40%, at least 45%, at least 50%, at least 60%, at least 65%, at least 70%, at least 75%, at least 80%, at least 85%, at least 90%, at least 95%, at least 99%, or at least 99.9% of the total isotopic abundance of an element that occupies a specific site of the compound.

*Pharmaceutical Compositions*

In another aspect, the invention provides a pharmaceutical composition comprising a compound of the present invention (e.g., a compound of Formula I or a pharmaceutically acceptable salt thereof, and a pharmaceutically acceptable excipient). The compounds of Formula I can be used in the treatment of certain disorders as described herein.

In certain embodiments, the compound of the present invention is provided in an effective amount in the pharmaceutical composition. In certain embodiments, the compound of the present invention is provided in a therapeutically effective amount. In certain embodiments, the compound of the present invention is provided in a prophylactically effective amount.

In certain embodiments, the pharmaceutical composition comprises an effective amount of the active ingredient. In certain embodiments, the pharmaceutical composition comprises a therapeutically effective amount of the active ingredient. In certain embodiments, the pharmaceutical composition comprises a prophylactically effective amount of the

active ingredient.

The pharmaceutical compositions provided herein can be administered by a variety of routes including, but not limited to, oral (enteral) administration, parenteral (by injection) administration, rectal administration, transdermal administration, intradermal administration, intrathecal administration, subcutaneous (SC) administration, intravenous (IV) administration, intramuscular (IM) administration, and intranasal administration.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

The pharmaceutical compositions provided herein can also be administered chronically ("chronic administration"). Chronic administration refers to administration of a compound or pharmaceutical composition thereof over an extended period of time, *e.g.,* for example, over 3 months, 6 months, 1 year, 2 years, 3 years, 5 years, *etc,* or may be continued indefinitely, for example, for the rest of the subject's life. In certain embodiments, the chronic administration is intended to provide a constant level of the compound in the blood, e.g., within the therapeutic window over the extended period of time.

The pharmaceutical compositions of the present invention may be further delivered using a variety of dosing methods. For example, in certain embodiments, the pharmaceutical composition may be given as a bolus, *e.g.,* in order to raise the concentration of the compound in the blood to an effective level. The placement of the bolus dose depends on the systemic levels of the active ingredient desired throughout the body, *e.g.,* an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, e.g., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or preferably from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with preferred doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, preferably from about 0.1 to about 20% by weight, preferably from about 0.1 to about 10% by weight, and more preferably from about 0.5 to about 15% by weight.

Injection dose levels range from about 0.1 mg/kg/hour to at least 20 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 5 g/day for a 40 to 80 kg human patient.

Liquid forms suitable for oral administration may include a suitable aqueous or nonaqueous vehicle with buffers, suspending and dispensing agents, colorants, flavours and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavouring agent such as peppermint, methyl salicylate, or orange flavouring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional

ingredients to enhance the dermal penetration of stability of the active ingredients or formulation. All such known transdermal formulations and ingredients are included within the scope provided herein.

The compounds provided herein can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania.

The compounds of the present invention can also be administered in sustained release forms or from sustained release drug delivery systems. A description of representative sustained release materials can be found in *Remington's Pharmaceutical Sciences.*

The present invention also relates to the pharmaceutically acceptable acid addition salt of a compound of the present invention. The acid which may be used to prepare the pharmaceutically acceptable salt is that which forms a non-toxic acid addition salt, *i.e.,* a salt containing pharmacologically acceptable anions such as the hydrochloride, hydroiodide, hydrobromide, nitrate, sulfate, bisulfate, phosphate, acetate, lactate, citrate, tartrate, succinate, maleate, fumarate, benzoate, para-toluenesulfonate, and the like.

In another aspect, the invention provides a pharmaceutical composition comprising a compound of the present invention and a pharmaceutically acceptable excipient, *e.g.,* a composition suitable for injection, such as for intravenous (IV) administration.

Pharmaceutically acceptable excipients include any and all diluents or other liquid vehicles, dispersion or suspension aids, surface active agents, isotonic agents, preservatives, lubricants and the like, as suited to the particular dosage form desired, e.g., injection. General considerations in the formulation and/or manufacture of pharmaceutical compositions agents can be found, for example, in Remington's Pharmaceutical Sciences, Sixteenth Edition, E. W. Martin (Mack Publishing Co., Easton, Pa., 1980), and Remington: The Science and Practice of Pharmacy, 21st Edition (Lippincott Williams & Wilkins, 2005).

For example, injectable preparations, such as sterile injectable aqueous suspensions, can be formulated according to the known art using suitable dispersing or wetting agents and suspending agents. Exemplary excipients that can be employed include, but are not limited to, water, sterile saline or phosphate-buffered saline, or Ringer's solution.

The injectable composition can be sterilized, for example, by filtration through a bacterial-retaining filter, or by incorporating sterilizing agents in the form of sterile solid compositions which can be dissolved or dispersed in sterile water or other sterile injectable medium prior to use.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, response of the individual patient, the severity of the patient's symptoms, and the like.

The compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include pre-filled, premeasured ampules or syringes of the liquid compositions. In such compositions, the compound is usually a minor component (from about 0.1% to about 50% by weight or preferably from about 1% to about 40% by weight) with the remainder being various vehicles or carriers and processing aids helpful for forming the desired dosing form.

The compounds provided herein can be administered as the sole active agent, or they can be administered in combination with other active agents. In one aspect, the present invention provides a combination of a compound of the present invention and another pharmacologically active agent. Administration in combination can proceed by any technique apparent to those of skill in the art including, for example, separate, sequential, concurrent, and alternating administration.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with ordinary experimentation. General considerations in the formulation and/or manufacture of pharmaceutical compositions can be found, for example, in Remington: The Science and Practice of Pharmacy 21st ed., Lippincott Williams & Wilkins, 2005.

In one aspect, provided is a kit comprising a composition (e.g., a solid composition) comprising a compound of Formula I.

*Methods of Use and Treatment*

One feature of the present disclosure relates to compounds that can be useful as therapeutic agetns for the treatment of diseases associated with the inhibition of CYP46A1 (e.g., spasm, neurodegenerative disease, epilepsy, schizophrenia, and autism spectrum disorder). For example, in an aspect of the disclosure, provided herein is method of treating a disorder in a subject in need thereof, the method comprising administering to the subject a therapeutically effective amount of a compound or composition described herein to the subject, including the compound of Formula I as defined herein. Example of disorders that can be treated by the compounds include, but are not limited to, diseases associated with the inhibition of CYP46A1 (e.g., spasm, neurodegenerative disease, epilepsy, and schizophrenia), neurodegenerative disease, epilepsy, psychiatric disorders (e.g. schizophrenia, and autism spectrum disorder), spasm, and developmental and epileptic encephalopathies.

In some embodiments, the diseases or disorder involving the inhibition of CYP46A1 is a neurodegenerative disorder. In some embodiments, the neurodegenerative disease is selected from the group consisting of Alzheimer's disease, mild cognitive impairment, Huntington's disease, Parkinson's disease, amyotrophic lateral sclerosis, traumatic brain injury, cerebral infarction, glaucoma, and multiple sclerosis. In certain embodiments, the disease or disorder involving the inhibition of CYP46A1 is epilepsy. In certain embodiments, the disease or disorder involving the inhibition of CYP46A1 is developmental and epileptic encephalopathies. In embodiments, the psychiatric disorder is selected from the group consisting of schizophrenia, delusional disorder, schizoaffective disorder, depression, and autism spectrum disorder. In embodiments, the disease or disorder involving the inhibition of CYP46A1 is spasm.

In certain embodiments, the compound is administered to the subject chronically. In certain embodiments, the compound is administered to the subject orally, subcutaneously, intramuscularly, or intravenously. In some embodiments, the compound is administered by a route of oral administration.

**Neurodegenerative Diseases and Disorders**

A compound of Formula I, or pharmaceutically acceptable salt thereof, or a pharmaceutically acceptable composition comprising of a compound of Formula I, or a pharmaceutically acceptable salt thereof, can be used in a method described herein, for example the treatment of neurodegenerative diseases and disorders.

The term "neurodegenerative disease" includes diseases and disorders that are associated with the progressive loss of structure or function of neurons, or death of neurons. Neurodegenerative diseases and disorders include, but are not limited to, Alzheimer's disease (including the associated symptoms of mild, moderate, or severe cognitive impairment); amyotrophic lateral sclerosis (ALS); anoxic and ischemic injuries; ataxia and convulsion (including for the treatment and prevention and prevention of seizures that are caused by schizoaffective disorder or by drugs used to treat schizophrenia); benign forgetfulness; brain edema; cerebellar ataxia including McLeod neuroacanthocytosis syndrome (MLS); closed head injury; coma; contusive injuries (e.g., spinal cord injury and head injury); dementias including multi-infarct dementia and senile dementia; disturbances of consciousness; Down syndrome; drug-induced or medication-induced Parkinsonism (such as neuroleptic-induced acute akathisia, acute dystonia, Parkinsonism, or tardive dyskinesia, neuroleptic malignant syndrome, or medication-induced postural tremor); epilepsy; fragile X syndrome; Gilles de la Tourette's syndrome; head trauma; hearing impairment and loss; Huntington's disease; Lennox syndrome; levodopa-induced dyskinesia; mental retardation; movement disorders including akinesias and akinetic (rigid) syndromes (including basal ganglia calcification, corticobasal degeneration, multiple system atrophy, Parkinsonism-ALS dementia complex, Parkinson's disease, postencephalitic parkinsonism, and progressively supranuclear palsy); muscular spasms and disorders associated with muscular spasticity or weakness including chorea (such as benign hereditary chorea, drug-induced chorea, hemiballism, Huntington's disease, neuroacanthocytosis, Sydenham's chorea, and symptomatic chorea), dyskinesia (including tics such as complex tics, simple tics, and symptomatic tics), myoclonus (including generalized myoclonus and focal cycloclonus), tremor (such as rest tremor, postural tremor, and intention tremor) and dystonia (including axial dystonia, dystonic writer's cramp, hemiplegic dystonia, paroxysmal dystonia, and focal dystonia such as blepharospasm, oromandibular dystonia, and spasmodic dysphonia and torticollis); neuronal damage including ocular damage, retinopathy or macular degeneration of the eye; neurotoxic injury which follows cerebral stroke, thromboembolic stroke, hemorrhagic stroke, cerebral ischemia, cerebral vasospasm, hypoglycemia, amnesia, hypoxia, anoxia, perinatal asphyxia and cardiac arrest; glaucoma (including the associated symptoms of blindness, normal intraocular pressure type field stenosis); Parkinson's disease; seizure; status epilecticus; stroke; tinnitus; tubular sclerosis, and viral infection induced neurodegeneration (e.g., caused by acquired immunodeficiency syndrome (AIDS) and encephalopathies). Neurodegenerative diseases also include, but are not limited to, neurotoxic injury which follows cerebral stroke, thromboembolic stroke, hemorrhagic stroke, cerebral ischemia, cerebral vasospasm, hypoglycemia, amnesia, hypoxia, anoxia, perinatal asphyxia and cardiac arrest. Methods of treating or preventing a neurodegenerative disease also include treating or preventing loss of neuronal function characteristic of neurodegenerative disorder.

## Psychiatric Disorders

A compound of Formula I, or pharmaceutically acceptable salt thereof, or a pharmaceutically acceptable composition comprising of a compound of Formula I, or a pharmaceutically acceptable salt thereof, can be used in a method described herein, for example the treatment of psychiatric disorders.

The term "psychiatric disorders" includes diseases and disorders that are associated with the clinically significant disturbance in an individual's cognition, emotion regulation, or behavior that reflects a dysfunction in the psychological, biological, or developmental processes underlying mental function. Psychiatric disorders include, but are not limited to, schizophrenia (including the associated symptoms of hallucinations, delusions, disorganized thinking, avolition, and diminished emotional expression); delusional disorder; schizoaffective disorder; dissociative identity disorder; depression, also known as depressive disorder (including the associated symptoms of persistent anxiety, feelings of helplessness, hopelessness, pessimism, worthlessness, low energy, restlessness, difficulty sleeping, sleeplessness, irritability, fatigue, motor challenges, loss of interest in pleasurable activities or hobbies, loss of concentration, loss of energy, poor self-esteem, absence of positive thoughts or plans, excessive sleeping, overeating, appetite loss, insomnia, self-harm, thoughts of suicide, and suicide attempts); psychotic major depression (PMD); autism spectrum disorder; autism (including the associated symptoms of impaired social interaction, and impaired verbal and non-verbal communication); bipolar disorder (including the associated symptoms of anxiety, and mood fluctuations); and attention-deficit/hyperactivity disorder (including the associated symptoms of attention deficits, hyperactivity, and impulsiveness).

## Epilepsy

A compound of Formula I, or pharmaceutically acceptable salt thereof, or a pharmaceutically acceptable composition comprising of a compound of Formula I, or a pharmaceutically acceptable salt thereof, can be used in a method described herein, for example the treatment of a disorder described herein such as epilepsy, developmental and epileptic encephalopathies, status epilepticus, or seizure.

Epilepsy is a syndrome of episodic brain dysfunction characterized by recurrent unpredictable, spontaneous seizures. Cerebellar dysfunction is a recognized complication of temporal lobe epilepsy and it is associated with seizure generation, motor deficits and memory impairment. Types of epilepsy can include, but are not limited to generalized epilepsy, e.g., childhood absence epilepsy, juvenile nyoclonic epilepsy, epilepsy with grand-mal seizures on awakening, West syndrome, Lennox-Gastaut syndrome, partial epilepsy, *e.g.*, temporal lobe epilepsy, frontal lobe epilepsy, benign focal epilepsy of childhood.

## Epileptic Encephalopathies

A compound of Formula I, or pharmaceutically acceptable salt thereof, or a pharmaceutically acceptable composition comprising of a compound of Formula I, or a pharmaceutically acceptable salt thereof, can be used in a method described herein, for example the treatment of developmental and epileptic encephalopathies.

Epileptic encephalopathies are conditions in which neurologic deterioration is attributable entirely or partly to epileptic activity. It can be due to very frequent or severe seizures and/or to sub-continuous paroxysmal interictal activity. Developmental and epileptic encephalopathies represent a group of epileptic disorders that appear early in life and are characterized by pharmacoresistant generalized or focal seizures, persistent severe electroencephalography (EEG) abnormalities, and cognitive dysfunction or decline.

## Epileptogenesis

A compound of Formula I, or pharmaceutically acceptable salt thereof, or a pharmaceutically acceptable composition comprising of a compound of Formula I, or a pharmaceutically acceptable salt thereof, can be used in a method described herein, for example the treatment of epileptogenesis.

Epileptogenesis is a gradual process by which a normal brain develops epilepsy (a chronic condition in which seizures occur). Epileptogenesis results from neuronal damage precipitated by the initial insult (e.g., status epilepticus).

## Status epilepticus (SE)

A compound of Formula I, or pharmaceutically acceptable salt thereof, or a pharmaceutically acceptable composition comprising of a compound of Formula I, or a pharmaceutically acceptable salt thereof, can be used in a method described herein, for example the treatment of status epilepticus (SE).

Status epilepticus (SE) can include, *e.g.,* convulsive status epilepticus, *e.g.,* early status epilepticus, established status epilepticus, refractory status epilepticus, super-refractory status epilepticus; non-convulsive status epilepticus,

e.g., generalized status epilepticus, complex partial status epilepticus; generalized periodic epileptiform discharges; and periodic lateralized epileptiform discharges. Convulsive status epilepticus is characterized by the presence of convulsive status epileptic seizures, and can include early status epilepticus, established status epilepticus, refractory status epilepticus, super-refractory status epilepticus. Early status epilepticus is treated with a first line therapy. Established status epilepticus is characterized by status epileptic seizures which persist despite treatment with a first line therapy, and a second line therapy is administered. Refractory status epilepticus is characterized by status epileptic seizures which persist despite treatment with a first line and a second line therapy, and a general anesthetic is generally administered. Super refractory status epilepticus is characterized by status epileptic seizures which persist despite treatment with a first line therapy, a second line therapy, and a general anesthetic for 24 hours or more.

Non-convulsive status epilepticus can include, *e.g.,* focal non-convulsive status epilepticus, e.g., complex partial non-convulsive status epilepticus, simple partial non-convulsive status epilepticus, subtle non-convulsive status epilepticus; generalized non-convulsive status epilepticus, e.g., late onset absence non-convulsive status epilepticus, atypical absence non-convulsive status epilepticus, or typical absence non-convulsive status epilepticus.

**Spasm**

A compound of Formula I, or pharmaceutically acceptable salt thereof, or a pharmaceutically acceptable composition comprising of a compound of Formula I, or a pharmaceutically acceptable salt thereof, can be used in a method described herein, for example the treatment of spasm.

Spasm is a disease that occurs in fits along with abnormal electric excitement of intracerebral nerve cells which includes the associated symptoms of muscle cramps, change in level of consciousness or lethargy, nausea, severe headache, sudden numbness, and vomiting. Spasm is one of the characteristic clinical findings in Alzheimer's disease.

*Combination Therapies and Treatments*

When the compound of the present invention is applied to each of the above-mentioned diseases, it can be administered in combination with a medicament or a treatment method generally employed for the disease. Administration in combination can proceed by any technique apparent to those of skill in the art including, for example, separate, sequential, concurrent and alternating administration.

Examples of the medicament (hereinafter to be abbreviated as "concomitant drug") to be used in combination with the compound of the present invention include acetylcholine esterase inhibitors (e.g., donepezil, rivastigmine, galanthamine, zanapezil etc.), antidementian agents (e.g., memantine), inhibitors of β amyloid protein production, secretion, accumulation, coagulation and/or deposition, β secretase inhibitors (e.g., 6-(4-biphenylyl)methoxy-2-[2-(N,N-dimethylamino) ethyl]tetralin, 6-(4-biphenylyl)methoxy-2-(N,N-dimethylamino)methyltetralin, 6-(4-biphenylyl)methoxy-2-(N,N-dipropylamino)methyltetralin, 2-(N,N-dimethylamino)methyl-6-(4'-methoxybiphenyl-4-yl)methoxytetralin, and 6-(4-biphenylyl)methoxy-2-[2-(N,N-diethylamino)ethyl]tetralin), γ secretase inhibitory agent, β amyloid protein coagulation inhibitory agent (e.g., PTI-00703, ALZHEMED (NC-53 1), PPI-368 (JP-A-11-514333), and PPI-558 (JP-A-2001-500852)), β amyloid vaccine, β amyloid degrading enzyme and the like, cerebral function activators (e.g., aniracetam, nicergoline), other therapeutic drug for Parkinson's disease (e.g., dopamine receptor agonists), a monoamine oxidase (MAO) inhibitors (e.g., deprenyl, Selgiline (selegiline), remacemide, riluzole), anticholinergic agents (e.g., trihexyphenidyl, biperiden), COMT inhibitors (e.g., entacapone)], therapeutic drug for amyotropic lateral sclerosis (e.g., riluzole etc., neurotrophic factor), therapeutic drug for abnormal behavior, wandering and the like due to the progress of dementia (e.g., sedative drug, antianxiety drug), apoptosis inhibitors (e.g., CPI-1189, IDN-6556, CEP-1347), neuronal differentiation or regeneration promoters (e.g., leteprinim, xaliproden (SR-57746-A), SB-216763, Y-128, VX-853, prosaptide, 5,6-dimethoxy-2-[2,2,4,6,7-pentamethyl-3-(4-methylphenyl)-2,3-dihydro-1-benzofuran-5-yl]isoindoline and optically active forms, salts and hydrates), antidepressants (e.g., desipramine, amitriptyline, imipramine, tramadol), antiepilepsy drug (e.g., lamotrigine), antianxiety drugs (e.g., benzodiazepine), non-steroidal anti-inflammatory drugs (e.g., meloxicam, tenoxicam, indomethacin, ibuprofen, celecoxib, rofecoxib, aspirin, indomethacin), disease-modifying antirheumatic drugs (DMARDs), anti-cytokine drugs (e.g., TNF inhibitor, MAP kinase inhibitor), steroidal drugs (e.g., dexamethasone, hexestrol, cortisone acetate), therapeutic agents for incontinence or frequent urination (e.g., flavoxate hydrochloride, oxybutynin hydrochloride, propiverine hydrochloride), phosphodiesterase inhibitors (e.g., sildenafil (citrate)), dopamine agonists (e.g., apomorphine etc.), antiarrhythmics (e.g., mexiletine), sex hormones or derivatives thereof (e.g., progesterone, estradiol, estradiol benzoate), therapeutic agents for osteoporosis (e.g., alfacalcidol, calcitriol, elcatonin, calcitonin salmon, estriol, ipriflavone, disodium pamidronate, sodium alendronate hydrate, disodium incadronate), parathyroid hormone (PTH), calcium receptor antagonists, therapeutic drugs for insomnia (e.g., benzodiazepine medicament, non-benzodiazepine medicament, melatonin agonist), and therapeutic drugs for schizophrenia (e.g., typical antipsychotic agents such as haloperidol and the like; atypical antipsychotic agents such as clozapine, olanzapine, risperidone, aripiprazole and the like; medicament acted on metabotropic glutamate receptor or ionic channel-conjugated glutamate receptor; phosphodiesterase inhibitor).

In addition, a combined use with a transplantation method of neural stem cell or neural precursor cell prepared from embryonic stem cell or nervous tissue, or fetal neural tissue, and a combined use with a pharmaceutical agent such as an immunosuppressant after the transplantation and the like.

Furthermore, the compound of the present invention may be used in combination with the following concomitant drugs.

1. Therapeutic Agents for Diabetic Complications

For example, aldose reductase inhibitors (e.g., tolrestat, epalrestat, zenarestat, zopolrestat, minalrestat, fidarestat, CT-112), neurotrophic factor and an increasing agent thereof (e.g., NGF, NT-3, BDNF, neurotrophic factors and increasing drugs described in WO01/14372 (e.g., 4-(4-chlorophenyl)-2-(2-methyl-1-imidazolyl)-5-[3-(2-methylphenoxy)propyl- ]oxazole)), nerve regeneration promoting agent (e.g., Y-128), PKC inhibitor (e.g., ruboxistaurin mesylate), AGE inhibitor (e.g., ALT946, pimagedine, pyratoxanthine, N-phenacylthiazolium bromide (ALT766), ALT-711, EXO-226, Pyridorin, pyridoxamine), active oxygen scavengers (e.g., thioctic acid), cerebral vasodilator (e.g., tiapuride, mexiletine), somatostatin receptor agonists (e.g., BIM23190), apoptosis signal regulating kinase-1(ASK-1) inhibitor and the like can be mentioned.

2. Therapeutic Agent for Hyperlipidemia

For example, statin compound (e.g., pravastatin, simvastatin, lovastatin, atorvastatin, fluvastatin, rosuvastatin, pitavastatin, or a salt thereof (e.g., sodium salt, calcium salt)), squalene synthase inhibitors (e.g., lapaquistat acetate or a salt thereof), fibrate compound (e.g., bezafibrate, clofibrate, simfibrate, clinofibrate), ACAT inhibitor (e.g., Avasimibe, Eflucimibe), anion exchange resin (e.g., colestyramine), probucol, nicotinic acid drug (e.g., nicomol, niceritrol), ethyl icosapentate, phytosterol (e.g., soysterol, gamma oryzanol) and the like.

3. Diuretic

For example, xanthine derivative (e.g., theobromine sodium salicylate, theobromine calcium salicylate), thiazide preparation (e.g., ethiazide, cyclopenthiazide, trichloromethyazide, hydrochlorothiazide, hydroflumethiazide, benzylhydrochlorothiazide, penflutizide, polythiazide, methyclothiazide), antialdosterone preparation (e.g., spironolactone, triamterene), carbonic anhydrase inhibitors (e.g., acetazolamide), chlorobenzenesulfonamide agent (e.g., chlortalidone, mefruside, indapamide), azosemide, isosorbide, ethacrynic acid, piretanide, bumetanide, furosemide and the like.

4. Chemotherapeutic Agent

For example, alkylating agents (e.g., cyclophosphamide, ifosfamide), metabolic antagonists (e.g., methotrexate, 5-fluorouracil or derivative thereof), antitumor antibiotics (e.g., mitomycin, adriamycin), plant-derived antitumor agents (e.g., vincristine, vindesine, Taxol), cisplatin, carboplatin, etoposide and the like. Of these, Furtulon and NeoFurtulon, which are 5-fluorouracil derivatives, and the like are preferable.

5. Immunotherapeutic Agent

For example, microorganism or bacterial components (e.g., muramyl dipeptide derivative, Picibanil), polysaccharides having immunity potentiating activity (e.g., lentinan, schizophyllan, krestin), cytokines obtained by genetic engineering techniques (e.g., interferon, interleukin (IL)), colony stimulating factors (e.g., granulocyte colony stimulating factor, erythropoietin) and the like, with preference given to interleukins such as IL-1, IL-2, IL-12 and the like.

6. Antithrombotic Agent

For example, heparin (e.g., heparin sodium, heparin calcium, dalteparin sodium), warfarin (e.g., warfarin potassium), anti-thrombin drug (e.g., argatroban), thrombolytic agent (e.g., urokinase, tisokinase, alteplase, nateplase, monteplase, pamiteplase), platelet aggregation inhibitor (e.g., ticlopidine hydrochloride, cilostazol, ethyl icosapentate, beraprost sodium, sarpogrelate hydrochloride) and the like.

7. Cachexia Improving Medicament

For example, cyclooxygenase inhibitors (e.g., indomethacin etc.) [Cancer Research, Vol. 49, pages 5935-5939,

1989], progesterone derivatives (e.g., megestrol acetate) [Journal of Clinical Oncology, Vol. 12, pages 213-225, 1994], glucosteroids (e.g., dexamethasone etc.), metoclopramide agents, tetrahydrocannabinol agents (publications are all as mentioned above), fat metabolism improving agents (e.g., eicosapentanoic acid etc.) [British Journal of Cancer, Vol. 68, pages 314-318, 1993], growth hormones, IGF-1, or antibodies to a cachexia-inducing factor such as TNF-.alpha., LIF, IL-6, oncostatin M and the like.

Two or more kinds of the above-mentioned concomitant drugs may be used in combination at an appropriate ratio.

It is also possible to apply compound of the present invention to each of the above-mentioned diseases in combination with a biologic (e.g., antibody, vaccine preparation and the like), or as a combination therapy in combination with gene therapy method and the like.

Examples of the antibody and vaccine preparation include vaccine preparation to angiotensin II, vaccine preparation to CETP, CETP antibody, TNF.alpha. antibody and antibody to other cytokine, amyloid β vaccine preparation, type 1 diabetes vaccine (e.g., DIAPEP-277 manufactured by Peptor Ltd.), anti-HIV antibody, HIV vaccine preparation and the like, antibody or vaccine preparation to cytokine, renin-angiotensin enzyme and a product thereof, antibody or vaccine preparation to enzyme or protein involved in blood lipid metabolism, antibody or vaccine to enzyme or protein involved in blood coagulation or fibrinolytic system, antibody or vaccine preparation to protein involved in saccharometabolism or insulin resistance and the like.

In addition, a combined use with a biological preparation involved in a growth factor such as GH, IGF and the like is possible.

Examples of the gene therapy method include a treatment method using a gene relating to cytokine, renin-angiotensin enzyme and a product thereof, G protein, G protein conjugated receptor and its phosphorylation enzyme, a treatment method using a DNA decoy such as NF.kappa.B decoy and the like, a treatment method using an antisense, a treatment method using a gene relating to an enzyme or protein involved in blood lipid metabolism (e.g., gene relating to metabolism, excretion or absorption of cholesterol or triglyceride or HDL-cholesterol or blood phospholipid), a treatment method using a gene relating to an enzyme or protein involved in angiogenesis therapy targeting obstruction of peripheral vessel and the like (e.g., growth factors such as HGF, VEGF etc.), a treatment method using a gene relating to a protein involved in saccharometabolism or insulin resistance, an antisense to cytokine such as TNF and the like, and the like.

In addition, it is possible to use in combination with various organ regeneration methods such as heart regeneration, kidney regeneration, pancreas regeneration, blood vessel regeneration and the like or cell transplantation therapy utilizing bone marrow cell (myelomonocytic cell, myeloid stem cell) or an artificial organ utilizing tissue engineering (e.g., artificial blood vessel and cardiac muscle cell sheet).

The time of administration of the compound of the present invention and that of the concomitant drug are not limited, and they may be administered simultaneously or in a staggered manner to the administration subject. Furthermore, the compound of the present invention and the concomitant drug may be administered as two kinds of preparations containing each active ingredient, or a single preparation containing both active ingredients.

The dose of the concomitant drug can be appropriately determined based on the dose employed in clinical situations. The mixing ratio of the compound of the present invention and a concomitant drug can be appropriately determined depending on the administration subject, administration route, target disease, symptom, combination and the like. When the subject of administration is human, for example, a concomitant drug can be used in 0.01-100 parts by weight relative to 1 part by weight of the compound of the present invention.

*Chemical Definitions*

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Isomers, *e.g.*, stereoisomers, can be isolated from mixtures by methods known to those skilled in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The invention additionally encompasses compounds described herein as individual isomers substantially free of other isomers, and alternatively, as mixtures of various

isomers.

Compounds that have the same molecular formula but differ in the nature or sequence of bonding of their atoms or the arrangement of their atoms in space are termed "isomers." Isomers that differ in the arrangement of their atoms in space are termed "stereoisomers." Stereoisomers that are not mirror images of one another are termed "diastereomers" and those that are non-superimposable mirror images of each other are termed "enantiomers." When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (*i.e.,* as (+) or (-)-isomers respectively). A chiral compound can exist as either individual enantiomer or as a mixture thereof. A mixture containing equal proportions of the enantiomers is called a "racemic mixture".

As used herein, a pure enantiomeric compound is substantially free from other enantiomers or stereoisomers of the compound (i.e., in enantiomeric excess). In other words, an "S" form of the compound is substantially free from the "R" form of the compound and is, thus, in enantiomeric excess of the "R" form. The term "enantiomerically pure" or "pure enantiomer" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of the enantiomer. In certain embodiments, the weights are based upon total weight of all enantiomers or stereoisomers of the compound. As used herein, the term "diastereomeric purity" refers to the amount of a compound having the depicted absolute stereochemistry, expressed as a percentage of the total amount of the depicted compound and its diastereomers. The term "diastereomierically pure" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of the diastereomer. Methods for determining diastereomeric and enantiomeric purity are well-known in the art. Diastereomeric purity can be determined by any analytical method capable of quantitatively distinguishing between a compound and its diastereomers, such as high performance liquid chromatography (HPLC).

In the compositions provided herein, an enantiomerically pure compound can be present with other active or inactive ingredients. For example, a pharmaceutical composition comprising enantiomerically pure R-position/center/ carbon compound can comprise, for example, about 90% excipient and about 10% enantiomerically pure R- compound. In certain embodiments, the enantiomerically pure R-compound in such compositions can, for example, comprise, at least about 95% by weight R-compound and at most about 5% by weight S-compound, by total weight of the compound. For example, a pharmaceutical composition comprising enantiomerically pure S-compound can comprise, for example, about 90% excipient and about 10% enantiomerically pure S-compound. In certain embodiments, the enantiomerically pure S-compound in such compositions can, for example, comprise, at least about 95% by weight S-compound and at most about 5% by weight R-compound, by total weight of the compound. In certain embodiments, the active ingredient can be formulated with little or no excipient or carrier.

As used herein, the term "diastereomeric purity" refers to the amount of a compound having the depicted absolute stereochemistry, expressed as a percentage of the total amount of the depicted compound and its diastereomers. The term "diastereomierically pure" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of the diastereomer. Methods for determining diastereomeric and enantiomeric purity are well-known in the art. Diastereomeric purity can be determined by any analytical method capable of quantitatively distinguishing between a compound and its diastereomers, such as high performance liquid chromatography (HPLC).

The articles "a" and "an" may be used herein to refer to one or to more than one (*i.e.* at least one) of the grammatical objects of the article. By way of example "an analogue" means one analogue or more than one analogue.

When a range of values is listed, it is intended to encompass each value and sub-range within the range. For example "$C_{1-6}$ alkyl" is intended to encompass, $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$, and $C_{5-6}$ alkyl.

The following terms are intended to have the meanings presented therewith below and are useful in understanding the description and intended scope of the present invention.

"Alkyl" refers to a radical of a straight-chain or branched saturated hydrocarbon group having from 1 to 20 carbon

atoms ("$C_{1-20}$ alkyl"). In some embodiments, an alkyl group has 1 to 6 carbon atoms ("$C_{1-6}$ alkyl"). In some embodiments, an alkyl group has 1 to 5 carbon atoms ("$C_{1-5}$ alkyl"). In some embodiments, an alkyl group has 1 to 4 carbon atoms ("$C_{1-4}$ alkyl"). In some embodiments, an alkyl group has 1 to 3 carbon atoms ("$C_{1-3}$ alkyl"). In some embodiments, an alkyl group has 1 to 2 carbon atoms ("$C_{1-2}$ alkyl"). In some embodiments, an alkyl group has 1 carbon atom ("$C_1$ alkyl"). Examples of $C_{1-6}$ alkyl groups include methyl ($C_1$), ethyl ($C_2$), n-propyl ($C_3$), isopropyl ($C_3$), n-butyl ($C_4$), tert-butyl ($C_4$), sec-butyl ($C_4$), iso-butyl ($C_4$), n-pentyl ($C_5$), 3-pentanyl ($C_5$), amyl ($C_5$), neopentyl ($C_5$), 3-methyl-2-butanyl ($C_5$), tertiary amyl ($C_5$), and n-hexyl ($C_6$). Unless otherwise specified, each instance of an alkyl group is independently optionally substituted, *i.e.,* unsubstituted (an "unsubstituted alkyl") or substituted (a "substituted alkyl") with one or more substituents; *e.g.,* for instance from 1 to 4 substituents, 1 to 3 substituents, or 1 substituent. Common alkyl abbreviations include Me ($-CH_3$), Et ($-CH_2CH_3$), iPr ($-CH(CH_3)_2$), nPr ($-CH_2CH_2CH_3$), n-Bu ($-CH_2CH_2CH_2CH_3$), or i-Bu ($-CH_2CH(CH_3)_2$).

"Alkylene" refers to a bivalent saturated hydrocarbon. Alkylenes can be represented by $-(CH_2)_n-$, $-(CH_2)-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, or $-(CH_2)_{10}-$. In some embodiments, alkylenes can be an indicated number of carbon atoms, for example, $C_1$-$C_4$ alkylene, $C_1$-$C_3$ alkylene, or $C_1$-$C_2$ alkylene. Unless otherwise specified, each instance of an alkylene group is independently optionally substituted, *i.e.,* unsubstituted (an "unsubstituted alkylene") or substituted (a "substituted alkylene") with one or more substituents (for instance from 1 to 4 substituents, 1 to 3 substituents, or 1 substituent) which may be halo, $-NO_2$, -OH, $C_1$-$C_6$ alkoxy, $C_1$-$C_6$ alkyl, or $C_1$-$C_6$ cycloalkyl. Alkylene abbreviations include $-(CH(CH_3))-$, $-(CH(CH_2CH_3))-$, $-(CH(CH_2CH_2CH_3))-$, $-(CH(CH_2CH_2CH_2CH_3))-$, $-(CH_2CH(CH_2CH_2CH_2CH_3))-$, $-(CH_2CH_2CH(CH_2CH_2CH_2CH_3))-$, $-(CH(CH_3)CH_2)-$, $-(CH(CH_3)CH_2CH_2)-$, $-(CH(CH_3)CH_2CH_2CH_2)-$, $-(CH_2CH(CH_3)CH_2)-$, $-(CH_2CH(CH_3)CH_2CH_2)-$, and $-(CH_2CH_2CH(CH_3)CH_2CH_2)-$,

"Aryl" refers to a radical of a monocyclic or polycyclic (*e.g.,* bicyclic or tricyclic) 4n+2 aromatic ring system (*e.g.,* having 6, 10, or 14 $\pi$ electrons shared in a cyclic array) having 6-14 ring carbon atoms and zero heteroatoms provided in the aromatic ring system ("$C_{6-14}$ aryl"). In some embodiments, an aryl group has six ring carbon atoms ("$C_6$ aryl"; *e.g.,* phenyl). Aryl" also includes ring systems wherein the aryl ring, as defined herein, is fused with one or more carbocyclyl or heterocyclyl groups wherein the radical or point of attachment is on the aryl ring, and in such instances, the number of carbon atoms continue to designate the number of carbon atoms in the aryl ring system. Typical aryl groups include, but are not limited to, groups derived from benzene. Particularly aryl groups include phenyl, and indenyl. Unless otherwise specified, each instance of an aryl group is independently optionally substituted, *i.e.,* unsubstituted (an "unsubstituted aryl") or substituted (a "substituted aryl") with one or more substituents.

"Heteroaryl" refers to a radical of a 5-10 membered monocyclic or bicyclic 4n+2 aromatic ring system (*e.g.,* having 6 or 10 $\pi$ electrons shared in a cyclic array) having ring carbon atoms and 1-4 ring heteroatoms provided in the aromatic ring system, wherein each heteroatom is independently selected from nitrogen, oxygen and sulfur ("5-10 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system.

In some embodiments, a heteroaryl group is a 1-4 ring heteroatoms provided in the aromatic ring system, wherein each heteroatom is independently selected from nitrogen, oxygen, and sulfur. In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur. In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Unless otherwise specified, each instance of a heteroaryl group is independently optionally substituted, *i.e.,* unsubstituted (an "unsubstituted heteroaryl") or substituted (a "substituted heteroaryl") with one or more substituents.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively.

Examples of representative heteroaryls include the following:

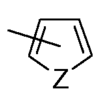

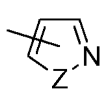

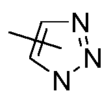

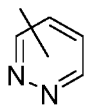 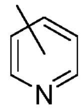 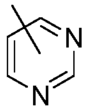 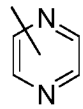

wherein each Z is selected from carbonyl, N, $NR^{65}$, O, and S; and $R^{65}$ is independently hydrogen, $C_1$-$C_8$ alkyl, $C_3$-$C_{10}$ cycloalkyl, 4-10 membered heterocyclyl, $C_6$-$C_{10}$ aryl, and 5-10 membered heteroaryl.

"Alkylene bridge" refers to a straight or branched divalent hydrocarbon bridge, linking two different carbons of the same ring structure. The alkylene bridge may link any two carbons within the ring structure. In some embodiments, alkylene bridges can be an indicated number of carbon atoms, for example, $C_1$-$C_6$ alkylene bridge, $C_1$-$C_5$ alkylene bridge, $C_1$-$C_4$ alkylene bridge, $C_1$-$C_3$ alkylene bridge, or $C_1$-$C_2$ alkylene bridge. Unless otherwise specified, each instance of an alkylene bridge is independently optionally substituted, *i.e.,* unsubstituted (an "unsubstituted alkylene bridge") or substituted (a "substituted alkylene bridge") with one or more substituents (for instance from 1 to 4 substituents, 1 to 3 substituents, or 1 substituent) which may be halo, $-NO_2$, -OH, $C_1$-$C_6$ alkoxy, $C_1$-$C_6$ alkyl, or $C_1$-$C_6$ cycloalkyl. Examples of alkylene bridge include, but are not limited to, methylene, ethylene, propylene, tetramethylene, and n-butylene.

"Nitrogen-containing heteroaryl" refers to a monocyclic aromatic heterocyclic group containing at least one nitrogen atom. Examplery nitrogen-containing heteroaryl groups include, but without limitation, pyrrolyl, thiazolyl, isoxazolyl, pyrazinyl, imidazolyl, oxazolyl, pyridyl (e.g. 2-pyridyl, 3-pyridyl, 4-pyridyl), pyrimidinyl (e.g. 2-pyrimidinyl, 4-pyrimidinyl), pyridazinyl, triazolyl, triazinyl, tetrazolyl, azepinyl, azocinyl, dithiazinyl, and oxazinyl.

"Hetero" when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, *e.g.,* heteroalkyl, cycloalkyl, *e.g.,* heterocyclyl, aryl, *e.g,.* heteroaryl, cycloalkenyl, *e.g,.* cycloheteroalkenyl, and the like having from 1 to 5, and particularly from 1 to 3 heteroatoms.

"Carbocyclyl" or "carbocyclic" refers to a radical of a non-aromatic cyclic hydrocarbon group having from 3 to 10 ring carbon atoms ("$C_{3-10}$ carbocyclyl") and zero heteroatoms in the non-aromatic ring system. In some embodiments, a carbocyclyl group has 3 to 8 ring carbon atoms ("$C_{3-8}$ carbocyclyl"). In some embodiments, a carbocyclyl group has 3 to 6 ring carbon atoms ("$C_{3-6}$ carbocyclyl"). In some embodiments, a carbocyclyl group has 3 to 6 ring carbon atoms ("$C_{3-6}$ carbocyclyl"). In some embodiments, a carbocyclyl group has 5 to 10 ring carbon atoms ("$C_{5-10}$ carbocyclyl"). Exemplary $C_{3-6}$ carbocyclyl groups include, without limitation, cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), and the like. Exemplary $C_{3-8}$ carbocyclyl groups include, without limitation, the aforementioned $C_{3-6}$ carbocyclyl groups as well as cycloheptyl ($C_7$), cycloheptenyl ($C_7$), cycloheptadienyl ($C_7$), cycloheptatrienyl ($C_7$), cyclooctyl ($C_8$), cyclooctenyl ($C_8$), bicyclo[2.2.1]heptanyl ($C_7$), bicyclo[2.2.2]octanyl ($C_8$), and the like. Exemplary $C_{3-10}$ carbocyclyl groups include, without limitation, the aforementioned $C_{3-8}$ carbocyclyl groups as well as cyclononyl ($C_9$), cyclononenyl ($C_9$), cyclodecyl ($C_{10}$), cyclodecenyl ($C_{10}$), octahydro-1*H*-indenyl ($C_9$), decahydronaphthalenyl ($C_{10}$), spiro[4.5]decanyl ($C_{10}$), and the like. As the foregoing examples illustrate, in certain embodiments, the carbocyclyl group is either monocyclic ("monocyclic carbocyclyl") or contain a fused, bridged or spiro ring system such as a bicyclic system ("bicyclic carbocyclyl") and can be saturated or can be partially unsaturated. "Carbocyclyl" also includes ring systems wherein the carbocyclyl ring, as defined above, is fused with one or more aryl or heteroaryl groups wherein the point of attachment is on the carbocyclyl ring, and in such instances, the number of carbons continue to designate the number of carbons in the carbocyclic ring system. Unless otherwise specified, each instance of a carbocyclyl group is independently optionally substituted, *i.e.,* unsubstituted (an "unsubstituted carbocyclyl") or substituted (a "substituted carbocyclyl") with one or more substituents. In certain embodiments, the carbocyclyl group is unsubstituted $C_{3-10}$ carbocyclyl. In certain embodiments, the carbocyclyl group is a substituted $C_{3-10}$ carbocyclyl.

In some embodiments, "carbocyclyl" is a monocyclic, saturated carbocyclyl group having from 3 to 10 ring carbon atoms ("$C_{3-10}$ cycloalkyl"). In some embodiments, a cycloalkyl group has 3 to 8 ring carbon atoms ("$C_{3-8}$ cycloalkyl"). In some embodiments, a cycloalkyl group has 3 to 6 ring carbon atoms ("$C_{3-6}$ cycloalkyl"). In some embodiments, a cycloalkyl group has 5 to 6 ring carbon atoms ("$C_{5-6}$ cycloalkyl"). In some embodiments, a cycloalkyl group has 5 to 10 ring carbon atoms ("$C_{5-10}$ cycloalkyl"). Examples of $C_{5-6}$ cycloalkyl groups include cyclopentyl ($C_5$) and cyclohexyl ($C_5$). Examples of $C_{3-6}$ cycloalkyl groups include the aforementioned $C_{5-6}$ cycloalkyl groups as well as cyclopropyl ($C_3$) and cyclobutyl ($C_4$). Examples of $C_{3-8}$ cycloalkyl groups include the aforementioned $C_{3-6}$ cycloalkyl groups as well as cycloheptyl ($C_7$) and cyclooctyl ($C_8$). Unless otherwise specified, each instance of a cycloalkyl group is independently unsubstituted (an "unsubstituted cycloalkyl") or substituted (a "substituted cycloalkyl") with one or more substituents. In certain embodiments, the cycloalkyl group is unsubstituted $C_{3-10}$ cycloalkyl. In certain embodiments, the cycloalkyl group is substituted $C_{3-10}$ cycloalkyl.

"Heterocyclyl" or "heterocyclic" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon ("3-10 membered heterocyclyl"). In heterocyclyl groups that contain one or more

nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"), and can be saturated or can be partially unsaturated. Heterocyclyl bicyclic ring systems can include one or more heteroatoms in one or both rings. "Heterocyclyl" also includes ring systems wherein the heterocyclyl ring, as defined above, is fused with one or more carbocyclyl groups wherein the point of attachment is either on the carbocyclyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system. Unless otherwise specified, each instance of heterocyclyl is independently optionally substituted, *i.e.,* unsubstituted (an "unsubstituted heterocyclyl") or substituted (a "substituted heterocyclyl") with one or more substituents. In certain embodiments, the heterocyclyl group is unsubstituted 3-10 membered heterocyclyl. In certain embodiments, the heterocyclyl group is substituted 3-10 membered heterocyclyl.

In some embodiments, a heterocyclyl group is a 5-10 membered non-aromatic ring system having ring carbon atoms and 1-4 ring heteroatoms, wherein each heteroatom is independently selected from nitrogen, oxygen, sulfur, boron, phosphorus, and silicon ("5-10 membered heterocyclyl"). In some embodiments, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has one ring heteroatom selected from nitrogen, oxygen, and sulfur.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing two heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a $C_6$ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

"Alkoxy" refers to the group $-OR^{29}$ where $R^{29}$ is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl. Particular alkoxy groups are methoxy, ethoxy, n-propoxy, isopropoxy, n-butoxy, tert-butoxy, sec-butoxy, n-pentoxy, n-hexoxy, and 1,2-dimethylbutoxy. Particular alkoxy groups are lower alkoxy, *i.e.* with between 1 and 6 carbon atoms. Further particular alkoxy groups have between 1 and 4 carbon atoms.

In certain embodiments, $R^{29}$ is a group that has 1 or more substituents, for instance from 1 to 5 substituents, and particularly from 1 to 3 substituents, in particular 1 substituent, selected from the group consisting of amino, substituted amino, $C_6$-$C_{10}$ aryl, aryloxy, carboxyl, cyano, $C_3$-$C_{10}$ cycloalkyl, 4-10 membered heterocyclyl, halogen, 5-10 membered heteroaryl, hydroxyl, nitro, thioalkoxy, thioaryloxy, thiol, alkyl-S(O)-, aryl-S(O)-, alkyl$S(O)_2$- and aryl-$S(O)_2$-. Exemplary 'substituted alkoxy' groups include, but are not limited to, $-O-(CH_2)_t(C_6-C_{10}$ aryl), $-O-(CH_2)_t$(5-10 membered heteroaryl), $-O-(CH_2)_t(C_3-C_{10}$ cycloalkyl), and $-O-(CH_2)_t$(4-10 membered heterocyclyl), wherein t is an integer from 0 to 4 and any aryl, heteroaryl, cycloalkyl or heterocyclyl groups present, may themselves be substituted by unsubstituted $C_1$-$C_4$ alkyl, halo, unsubstituted $C_1$-$C_4$ alkoxy, unsubstituted $C_1$-$C_4$ haloalkyl, unsubstituted $C_1$-$C_4$ hydroxyalkyl, or unsubstituted $C_1$-$C_4$ haloalkoxy or hydroxy. Particular exemplary 'substituted alkoxy' groups are $-OCF_3$, $-OCH_2CF_3$, $-OCH_2Ph$, $-OCH_2$-cyclopropyl, $-OCH_2CH_2OH$, and $-OCH_2CH_2NMe_2$.

"Haloalkoxy" refers to a haloalkyl group as defined herein attached through an oxygen bridge (oxygen of an alcohol radical).

"Halo" or "halogen" refers to fluoro (F), chloro (Cl), bromo (Br), and iodo (I). In certain embodiments, the halo

group is either fluoro or chloro.

"Haloalkyl" refers to an alkyl radical in which the alkyl group is substituted with one or more halogens. Typical haloalkyl groups include, but are not limited to, trifluoromethyl, difluoromethyl, fluoromethyl, chloromethyl, dichloromethyl, dibromoethyl, tribromomethyl, tetrafluoroethyl, and the like.

Alkyl, heterocyclyl, aryl, and heteroaryl groups, as defined herein, are optionally substituted (*e.g.,* "substituted" or "unsubstituted" alkyl, "substituted" or "unsubstituted" heterocyclyl, "substituted" or "unsubstituted" aryl or "substituted" or "unsubstituted" heteroaryl group). In general, the term "substituted", whether preceded by the term "optionally" or not, means that at least one hydrogen present on a group (*e.g.,* a carbon or nitrogen atom) is replaced with a permissible substituent, *e.g.*, a substituent which upon substitution results in a stable compound, *e.g.,* a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. The present invention contemplates any and all such combinations in order to arrive at a stable compound. For purposes of this invention, heteroatoms such as nitrogen may have hydrogen substituents and/or any suitable substituent as described herein which satisfy the valencies of the heteroatoms and results in the formation of a stable moiety.

Exemplary carbon atom substituents include, but are not limited to, halogen, -CN, $-NO_2$, $-N_3$, $-SO_2H$, $-SO_3H$, -OH, $-OR^{aa}$, $-ON(R^{bb})_2$, $-N(R^{bb})_2$, $-N(R^{bb})_3{}^+X^-$ , $-N(OR^{cc})R^{bb}$, - SH, $-SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, - $OCO_2R^{aa}$, $-C(=O)N(R^{bb})_2$, $-OC(=O)N(R^{bb})_2$, $-NR^{bb}C(=O)R^{aa}$, $-NR^{bb}CO_2R^{aa}$, - $NR^{bb}C(=O)N(R^{bb})_2$, $-C(=NR^{bb})R^{aa}$, $-C(=NR^{bb})OR^{aa}$, $-OC(=NR^{bb})R^{aa}$, $-OC(=NR^{bb})OR^{aa}$,$-C(=NR^{bb})N(R^{bb})_2$, $-OC(=NR^{bb})N(R^{bb})_2$, $-NR^{bb}C(=NR^{bb})N(R^{bb})_2$, $-C(=O)NR^{bb}SO_2R^{aa}$,$-NR^{bb}SO_2R^{aa}$, $-SO_2N(R^{bb})_2$, $-SO_2R^{aa}$, $-SO_2OR^{aa}$, $-OSO_2R^{aa}$, $-S(=O)R^{aa}$, $-OS(=O)R^{aa}$, - $Si(R^{aa})_3$, $-OSi(R^{aa})_3$ $-C(=S)N(R^{bb})_2$, $-C(=O)SR^{aa}$, $-C(=S)SR^{aa}$, $-SC(=S)SR^{aa}$, $-SC(=O)SR^{aa}$, $-OC(=O)SR^{aa}$, $-SC(=O)OR^{aa}$, $-SC(=O)R^{aa}$, $-P(=O)_2R^{aa}$, $-OP(=O)_2R^{aa}$, $-P(=O)(R^{aa})_2$, - $OP(=O)(R^{aa})_2$, $-OP(=O)(OR^{cc})_2$, $-P(=O)_2N(R^{bb})_2$, $-OP(=O)_2N(R^{bb})_2$, $-P(=O)(NR^{bb})_2$, - $OP(=O)(NR^{bb})_2$, $-NR^{bb}P(=O)(OR^{cc})_2$, $-NR^{bb}P(=O)(NR^{bb})_2$, $-P(R^{cc})_2$, $-P(R^{cc})_3$, $-OP(R^{cc})_2$,$-OP(R^{cc})_3$, $-B(R^{aa})_2$, $-B(OR^{cc})_2$, $-BR^{aa}(OR^{cc})$, $C_{1-10}$ alkyl, $C_{1-10}$ haloalkyl, $C_{2-10}$ alkenyl, $C_{2-10}$ alkynyl, $C_{3-10}$ carbocyclyl, 3-14 membered heterocyclyl, $C_{6-14}$ aryl, and 5-14 membered heteroaryl, wherein each alkyl, alkenyl, alkynyl, carbocyclyl, heterocyclyl, aryl, and heteroaryl is independently substituted with 0, 1, 2, 3, 4, or 5 $R^{dd}$ groups; or two geminal hydrogens on a carbon atom are replaced with the group =O, =S, $=NN(R^{bb})_2$, $=NNR^{bb}C(=O)R^{aa}$, $=NNR^{bb}C(=O)OR^{aa}$, $=NNR^{bb}S(=O)_2R^{aa}$, $=NR^{bb}$, or $=NOR^{cc}$;

each instance of $R^{aa}$ is, independently, selected from $C_{1-10}$ alkyl, $C_{1-10}$ haloalkyl, $C_{2-10}$ alkenyl, $C_{2-10}$ alkynyl, $C_{3-10}$ carbocyclyl, 3-14 membered heterocyclyl, $C_{6-14}$ aryl, and 5-14 membered heteroaryl, or two $R^{aa}$ groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 $R^{dd}$ groups;

each instance of $R^{bb}$ is, independently, selected from hydrogen, -OH, $-OR^{aa}$, - $N(R^{cc})_2$, -CN, $-C(=O)R^{aa}$, $-C(=O)N(R^{cc})_2$, $-CO_2R^{aa}$, $-SO_2R^{aa}$, $-C(=NR^{cc})OR^{aa}$, - $C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, - $C(=S)SR^{cc}$, $-P(=O)_2R^{aa}$, $-P(=O)(R^{aa})_2$, $-P(=O)_2N(R^{cc})_2$, $-P(=O)(NR^{cc})_2$, $C_{1-10}$ alkyl, $C_{1-10}$ haloalkyl, $C_{2-10}$ alkenyl, $C_{2-10}$ alkynyl, $C_{3-10}$ carbocyclyl, 3-14 membered heterocyclyl, $C_{6-14}$ aryl, and 5-14 membered heteroaryl, or two $R^{bb}$ groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 $R^{dd}$ groups;

each instance of $R^{cc}$ is, independently, selected from hydrogen, $C_{1-10}$ alkyl, $C_{1-10}$ haloalkyl, $C_{2-10}$ alkenyl, $C_{2-10}$ alkynyl, $C_{3-10}$ carbocyclyl, 3-14 membered heterocyclyl, $C_{6-14}$ aryl, and 5-14 membered heteroaryl, or two $R^{cc}$ groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 $R^{dd}$ groups;

each instance of $R^{dd}$ is, independently, selected from halogen, -CN, $-NO_2$, $-N_3$, - $SO_2H$, $-SO_3H$, -OH, $-OR^{ee}$, $-ON(R^{ff})_2$, $-N(R^{ff})_2$, $-N(R^{ff})_3{}^+X^-$, $-N(OR^{ee})R^{ff}$, -SH, $-SR^{ee}$, - $SSR^{ee}$, $-C(=O)R^{ee}$, $-CO_2H$, $-CO_2R^{ee}$, $-OC(=O)R^{ee}$, $-OCO_2R^{ee}$, $-C(=O)N(R^{ff})_2$, - $OC(=O)N(R^{ff})_2$, $-NR^{ff}C(=O)R^{ee}$, $-NR^{ff}CO_2R^{ee}$, $-NR^{ff}C(=O)N(R^{ff})_2$, $-C(=NR^{ff})OR^{ee}$, - $OC(=NR^{ff})R^{ee}$, $-OC(=NR^{ff})OR^{ee}$, $-C(=NR^{ff})N(R^{ff})_2$, $-OC(=NR^{ff})N(R^{ff})_2$, - $NR^{ff}C(=NR^{ff})N(R^{ff})_2$,$-NR^{ff}SO_2R^{ee}$, $-SO_2N(R^{ff})_2$, $-SO_2R^{ee}$, $-SO_2OR^{ee}$, $-OSO_2R^{ee}$, $-S(=O)R^{ee}$, $-Si(R^{ee})_3$, $-OSi(R^{ee})_3$, $-C(=S)N(R^{ff})_2$, $-C(=O)SR^{ee}$, $-C(=S)SR^{ee}$, $-SC(=S)SR^{ee}$, $-P(=O)_2R^{ee}$, - $P(=O)(R^{ee})_2$, $-OP(=O)(R^{ee})_2$, $-OP(=O)(OR^{ee})_2$, $C_{1-6}$ alkyl, $C_{1-6}$ haloalkyl, $C_{2-6}$ alkenyl, $C_{2-6}$ alkynyl, $C_{3-10}$ carbocyclyl, 3-10 membered heterocyclyl, $C_{6-10}$ aryl, 5-10 membered heteroaryl, wherein each alkyl, alkenyl, alkynyl, carbocyclyl, heterocyclyl, aryl, and heteroaryl is independently substituted with 0, 1, 2, 3, 4, or 5 $R^{gg}$ groups, or two geminal $R^{dd}$ substituents can be joined to form =O or =S;

each instance of $R^{ee}$ is, independently, selected from $C_{1-6}$ alkyl, $C_{1-6}$ haloalkyl, $C_{2-6}$ alkenyl, $C_{2-6}$ alkynyl, $C_{3-10}$ carbocyclyl, $C_{6-10}$ aryl, 3-10 membered heterocyclyl, and 3-10 membered heteroaryl, wherein each alkyl, alkenyl, alkynyl, carbocyclyl, heterocyclyl, aryl, and heteroaryl is independently substituted with 0, 1, 2, 3, 4, or 5 $R^{gg}$ groups;

each instance of $R^{ff}$ is, independently, selected from hydrogen, $C_{1-6}$ alkyl, $C_{1-6}$ haloalkyl, $C_{2-6}$ alkenyl, $C_{2-6}$ alkynyl, $C_{3-10}$ carbocyclyl, 3-10 membered heterocyclyl, $C_{6-10}$ aryl and 5-10 membered heteroaryl, or two $R^{ff}$ groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 $R^{gg}$ groups; and

each instance of $R^{gg}$ is, independently, halogen, -CN, $-NO_2$, $-N_3$, $-SO_2H$, $-SO_3H$, - OH, $-OC_{1-6}$ alkyl, $-ON(C_{1-6}$ alkyl$)_2$, $-N(C_{1-6}$ alkyl$)_2$, $-N(C_{1-6}$ alkyl$)_3{}^+X^-$, $-NH(C_{1-6}$ alkyl$)_2{}^+X^-$, $-NH_2(C_{1-6}$ alkyl) $^+X^-$, $-NH_3{}^+X^-$, $-N(OC_{1-6}$ alkyl)($C_{1-6}$ alkyl), -N(OH)($C_{1-6}$ alkyl), -NH(OH), -SH, $-SC_{1-6}$ alkyl, -SS($C_{1-6}$ alkyl), -C(=O)($C_{1-6}$ alkyl), $-CO_2H$, $-CO_2(C_{1-6}$ alkyl), -OC(=O)($C_{1-6}$ alkyl), $-OCO_2(C_{1-6}$ alkyl), $-C(=O)NH_2$, $-C(=O)N(C_{1-6}$ alkyl$)_2$, - OC(=O)NH($C_{1-6}$ alkyl), -NHC(=O)( $C_{1-6}$ alkyl), $-N(C_{1-6}$ alkyl)C(=O)( $C_{1-6}$ alkyl), - $NHCO_2(C_{1-6}$ alkyl), $-NHC(=O)N(C_{1-6}$ alkyl$)_2$, -NHC(=O)NH($C_{1-6}$ alkyl), $-NHC(=O)NH_2$, -C(=NH)O($C_{1-6}$ alkyl),-OC(=NH)($C_{1-6}$ alkyl), $-OC(=NH)OC_{1-6}$ alkyl, $-C(=NH)N(C_{1-6}$ alkyl$)_2$, -C(=NH)NH($C_{1-6}$ alkyl), $-C(=NH)NH_2$, $-OC(=NH)N(C_{1-6}$ alkyl$)_2$, - OC(NH)NH($C_{1-6}$ alkyl), $-OC(NH)NH_2$, $-NHC(NH)N(C_{1-6}$ alkyl$)_2$, $-NHC(=NH)NH_2$, - $NHSO_2(C_{1-6}$ alkyl), $-SO_2N(C_{1-6}$ alkyl$)_2$, $-SO_2NH(C_{1-6}$ alkyl), $-SO_2NH_2$,$-SO_2C_{1-6}$ alkyl, - $SO_2OC_{1-6}$ alkyl, $-OSO_2C_{1-6}$ alkyl, $-SOC_{1-6}$ alkyl, $-Si(C_{1-6}$ alkyl$)_3$, $-OSi(C_{1-6}$ alkyl$)_3$ - $C(=S)N(C_{1-6}$ alkyl$)_2$, C(=S)NH($C_{1-6}$ alkyl), $C(=S)NH_2$, -C(=O)S($C_{1-6}$ alkyl), $-C(=S)SC_{1-6}$ alkyl, $-SC(=S)SC_{1-6}$ alkyl, $-P(=O)_2(C_{1-6}$ alkyl), $-P(=O)(C_{1-6}$ alkyl$)_2$, $-OP(=O)(C_{1-6}$ alkyl$)_2$, - $OP(=O)(OC_{1-6}$ alkyl$)_2$, $C_{1-6}$ alkyl, $C_{1-6}$ haloalkyl, $C_{2-6}$ alkenyl, $C_{2-6}$ alkynyl, $C_{3-10}$ carbocyclyl, $C_{6-10}$ aryl, 3-10 membered heterocyclyl, 5-10 membered heteroaryl; or two geminal $R^{gg}$ substituents can be joined to form =O or =S; wherein $X^-$ is a counterion.

*Other Definitions*

"Pharmaceutically acceptable" means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

"Pharmaceutically acceptable salt" refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. The term "pharmaceutically acceptable cation" refers to an acceptable cationic counterion of an acidic functional group. Such cations are exemplified by sodium, potassium, calcium, magnesium, ammonium, tetraalkylammonium cations, and the like. See, *e.g.,* Berge, et al., J. Pharm. Sci. (1977) 66(1): 1-79.

"Pharmaceutically acceptable carrier" refers to compositions, carriers, diluents, and reagents which are pharmaceutically acceptable materials that are capable of administration to or upon a subject. A pharmaceutically acceptable carrier can be involved with carrying or transporting the subject agents from one organ, or portion of the body, to another organ, or portion of the body. The carrier can be in the form of a solid, semi-solid or liquid diluent, cream or a capsule. The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof.

A "subject" to which administration is contemplated includes, but is not limited to, human subject (*i.e.,* a male or female of any age group, *e.g.,* a pediatric subject (*e.g.,* infant, child, adolescent) or adult subject (*e.g.,* young adult, middle-aged adult or senior adult)) and/or a non-human animal, *e.g.,* a mammal such as primates (*e.g.,* cynomolgus monkeys, rhesus monkeys), cattle, pigs, horses, sheep, goats, rodents, cats, and/or dogs. In certain embodiments, the subject is a

human. In certain embodiments, the subject is a non-human animal.

In certain embodiments, the substituent present on an oxygen atom is an oxygen protecting group (also referred to as a hydroxyl protecting group). Oxygen protecting groups include, but are not limited to, $-R^{aa}$, $-N(R^{bb})_2$, $-C(=O)SR^{aa}$, $-C(=O)R^{aa}$, $-CO_2R^{aa}$, - $C(=O)N(R^{bb})_2$, $-C(=NR^{bb})R^{aa}$, $-C(=NR^{bb})OR^{aa}$, $-C(=NR^{bb})N(R^{bb})_2$, $-S(=O)R^{aa}$, $-SO_2R^{aa}$,$-Si(R^{aa})_3$, $-P(R^{cc})_2$, $-P(R^{cc})_3$, $-P(=O)_2R^{aa}$, $-P(=O)(R^{aa})_2$, $-P(=O)(OR^{cc})_2$, $-P(=O)_2N(R^{bb})_2$, and - $P(=O)(NR^{bb})_2$, wherein $R^{aa}$, $R^{bb}$, and $R^{cc}$ are as defined herein. Oxygen protecting groups are well known in the art and include those described in detail in Protecting Groups in Organic Synthesis, T. W. Greene and P. G. M. Wuts, 3rd edition, John Wiley & Sons, 1999.

Exemplary oxygen protecting groups include, but are not limited to, methyl, methoxylmethyl (MOM), 2-methoxyethoxymethyl (MEM), benzyl (Bn), triisopropylsilyl (TIPS), t-butyldimethylsilyl (TBDMS), t-butylmethoxyphenylsilyl (TBMPS), methanesulfonate (mesylate), and tosylate (Ts).

In certain embodiments, the substituent present on a nitrogen atom is an amino protecting group (also referred to herein as a nitrogen protecting group). Amino protecting groups include, but are not limited to, -OH, $-OR^{aa}$, $-N(R^{cc})_2$, $-C(=O)R^{aa}$, $-C(=O)OR^{aa}$, - $C(=O)N(R^{cc})_2$, $-S(=O)_2R^{aa}$, $-C(=NR^{cc})R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $C_{1-10}$ alkyl, $C_{2-10}$ alkenyl, $C_{2-10}$ alkynyl, $C_{3-10}$ carbocyclyl, 3-14-membered heterocyclyl, $C_{6-14}$ aryl, and 5-14-membered heteroaryl groups, wherein each alkyl, alkenyl, alkynyl, carbocyclyl, heterocyclyl, aryl, and heteroaryl is independently substituted with 0, 1, 2, 3, 4, or 5 $R^{dd}$ groups, and wherein $R^{aa}$, $R^{bb}$, $R^{cc}$ and $R^{dd}$ are as defined herein. Amino protecting groups are well known in the art and include those described in detail in Protecting Groups in Organic Synthesis, T. W. Greene and P. G. M. Wuts, 3rd edition, John Wiley & Sons, 1999.

Exemplary amino protecting groups include, but are not limited to amide groups (*e.g.,* $-C(=O)R^{aa}$), which include, but are not limited to, formamide and acetamide; carbamate groups (*e.g.,* $-C(=O)OR^{aa}$), which include, but are not limited to, 9-fluorenylmethyl carbamate (Fmoc), t-butyl carbamate (BOC), and benzyl carbamate (Cbz); sulfonamide groups (*e.g.,* $-S(=O)_2R^{aa}$), which include, but are not limited to, *p*-toluenesulfonamide (Ts), methanesulfonamide (Ms), and N-[2-(trimethylsilyl)ethoxy]methylamine (SEM).

Disease, disorder, and condition are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition. In an alternate embodiment, the present invention contemplates administration of the compounds of the present invention as a prophylactic before a subject begins to suffer from the specified disease, disorder or condition.

In general, the "effective amount" of a compound refers to an amount sufficient to elicit the desired biological response. As will be appreciated by those of ordinary skill in this art, the effective amount of a compound of the invention may vary depending on such factors as the desired biological endpoint, the pharmacokinetics of the compound, the disease being treated, the mode of administration, and the age, weight, health, and condition of the subject. An effective amount encompasses therapeutic and prophylactic treatment.

As used herein, and unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

As used herein, and unless otherwise specified, a "prophylactically effective amount" of a compound is an amount sufficient to prevent a disease, disorder or condition, or one or more symptoms associated with the disease, disorder or condition, or prevent its recurrence. A prophylactically effective amount of a compound means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

As used herein, and unless otherwise specified, "pharmacokinetics" can be defined as the study of bodily absorption, distribution, metabolism, and excretion of drugs. "Pharmocokinetics" can also be defined as the characteristic interactions of a drug and a body in terms of its absorption, distribution, metabolism, and excretion; or a branch of pharmacology concerned with the way drugs are taken into, move around, and are eliminated from, a body.

## EXAMPLES

In order that the invention described herein may be more fully understood, the following examples are set forth. The synthetic and biological examples described in this application are offered to illustrate the compounds, pharmaceutical compositions, and methods provided herein and are not to be construed in any way as limiting their scope.

*Materials and Methods*

The compounds provided herein can be prepared from readily available starting materials using the following general methods and procedures. It will be appreciated that where typical or preferred process conditions (*i.e.,* reaction temperatures, times, mole ratios of reactants, solvents, pressures, *etc.*) are given, other process conditions can also be used unless otherwise stated. Optimum reaction conditions may vary with the particular reactants or solvent used, but such conditions can be determined by one skilled in the art by routine optimization.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art. For example, numerous protecting groups, and their introduction and removal, are described in T. W. Greene and P. G. M. Wuts, Protecting Groups in Organic Synthesis, Second Edition, Wiley, New York, 1991, and references cited therein.

The compounds provided herein may be isolated and purified by known standard procedures. Such procedures include (but are not limited to) recrystallization, column chromatography, HPLC, or supercritical fluid chromatography (SFC). The following schemes are presented with details as to the preparation of representative piperidines that have been listed herein. The compounds provided herein may be prepared from known or commercially available starting materials and reagents by one skilled in the art of organic synthesis. Exemplary chiral columns available for use in the separation/purification of the enantiomers/diastereomers provided herein include, but are not limited to, CHIRALPAK® AD-10, CHIRALCEL® OB, CHIRALCEL® OB-H, CHIRALCEL® OD, CHIRALCEL® OD-H, CHIRALCEL® OF, CHIRALCEL® OG, CHIRALCEL® OJ and CHIRALCEL® OK.

$^1$H-NMR reported herein (e.g., for the region between $\delta$ (ppm) of about 0.5 to about 10 ppm) will be understood to be an exemplary interpretation of the NMR spectrum (e.g., exemplary peak integratations) of a compound.

Exemplary general method for LCMS/LC ELSD: 30-90AB_2 min. Lcm. (Mobile Phase: 1.5mL/4L TFA in water (solvent A) and 0.75mL/4L TFA in acetonitrile (solvent B), using the elution gradient 30%-90% (solvent B) over 0.9 minutes and holding at 90% for 0.6 minutes at a flow rate of 1.2 mL/min; Column: Xtimate C18 2.1*30mm, 3$\mu$m; Wavelength: UV 220 nm; Column temperature: 50°C; MS ionization: ESI; Detector: PDA&ELSD)

*Abbreviations*

ACN: acetonitrile; AcOK or KOAc: potassium acetate; AUC: area under the curve; sec-BuLi: sec-butyllithium; BSA: bis(trimethylsilyl)acetamide; BuOH: butanol; BPO: benzoyl peroxide; n-BuLi: n-butyllithium; CAN: ceric ammonium nitrate; CYP46A1: cholesterol 24-hydroxylase; DIPEA or DIEA: diisopropylethylamine; DEA: diethanolamine; DME: dimethoxyethane; DMF: dimethylformamide; DCM: dichloromethane; DMA: dimethylacetamide; DIPA: diisopropylamine; DMSO: dimethyl sulfoxide; EDCI: 1-Ethyl-3-(3-dimethylaminopropyl)carbodiimide; EtOH: ethanol; EtOAc: ethyl acetate; HATU: 1-[bis(dimethylamino)methylene]-1H-1,2,3-triazolo[4,5-b]pyridinium 3-oxid hexafluorophosphate; HBSS: hank's balanced salt solution; HOBt: hydroxybenzotriazole; HSS: high strength silica; IPA: isopropyl alcohol; LC: liquid chromatography; LDA: lithium diisopropylamide; MeOD: deuterated methanol; MeCN: acetonitrile; MS: mass spectrometry; MDCK: madin-darby canine kidney cells; MDR1: multidrug resistance mutation; MeOH: methanol; NADPH: dihydronicotinamide-adenine dinucleotide phosphate; NBS: N-Bromosuccinimide; NMR: nuclear magnetic resonsnace; *i*-$Pr_2O$: diisopropyl ether; $Pd_2(dba)_3$: tris(dibenzylideneacetone)dipalladium(0); $Pd(OAc)_2$: palladium(II) Acetate; $Pd(dppf)Cl_2$: (1,1'-Bis(diphenylphosphino)ferrocene)palladium(II) dichloride; PE: petroleum ether; PET: polyethylene membrane; PK: pharmacokinetics; PO: per os; RFU: relative fluorescence unit; TEA: triethylamine; TFA: trifluoroacetic acid; THF: tetrahydrofuran; TQ: triple quadrupole; UPLC: ultra performace liquid chromatography.

*Example 1. General synthetic scheme*

The compounds of the present invention can be prepared according to the following methods outlined in Schemes 1 and 2. As illustrated in Scheme 1, protected 4-cyanopiperidines **A** can be treated with sterically hindered bases such as LDA followed by addition of alkyl halides or benzyl halides (or tosylates) to give 4,4-disubstituted protected piperidines **B.** Deprotection to give piperidines **C** and subsequent amide coupling with acids **D** provides target compounds **E** of this invention.

Scheme 1

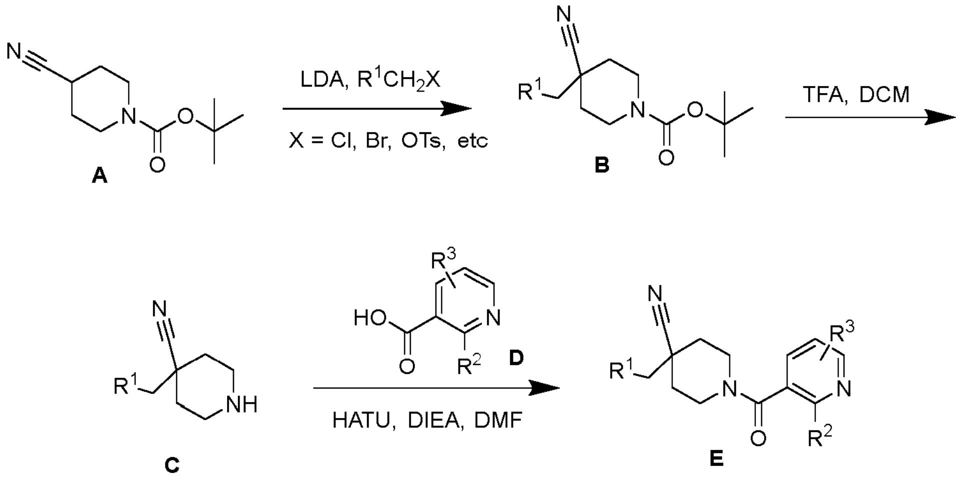


Alternatively, as shown in Scheme 2, amide coupling of 4-cyanopiperidine **F** with acids **D** gives amides **G.** Alkylation reaction by treatment of cyanopiperidine **G** with a sterically hindered base such as LDA followed by addition of alkyl halides or benzyl halides (or tosylates) provides target compounds **E.**

Scheme 2

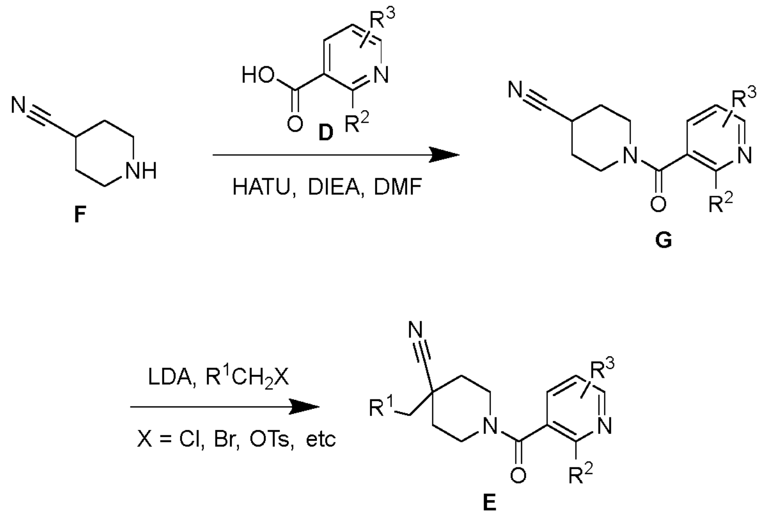


*Example 2. Synthesis of 4-(4-fluorobenzyl)-1-(2-(pyrimidin-4-yl)nicotinoyl)piperidine-4-carbonitrile (A6)*

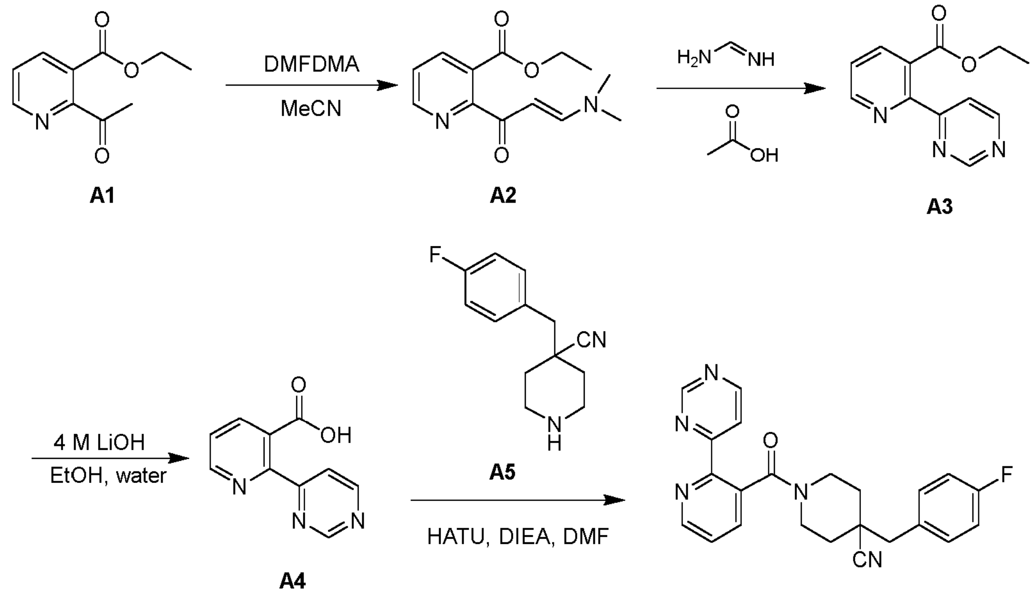


A1 A2 A3 A4 A5 A6

Synthesis of A2

To a solution of ethyl 2-acetylnicotinate (2.7 g, 13.9 mmol) in MeCN (27 mL) was added DMF-DMA (27 mL) in one portion at 25 °C. The mixture was stirred at 85 °C for 2 h to give a solution. The reaction mixture was concentrated to give ethyl (E)-2-(3-(dimethylamino)acryloyl)nicotinate (3.3 g) as an oil and used directly for the next step.

Synthesis of A3

To a solution of ethyl (E)-2-(3-(dimethylamino)acryloyl)nicotinate (3.3 g, 13.3 mmol) and acetic acid (9.96 g, 166 mmol) in n-BuOH (30 mL) was added DIPEA (30 mL) in one portion at 25 °C. The mixture was stirred at 120 °C for 40 h giving a solution. The residue was poured into water (50 mL) and saturated $NaHCO_3$ (18 mL). The aqueous phase was extracted with EtOAc (3 x 20 mL) and the combined organic extracts were washed with brine (2 x 20 mL), dried over anhydrous $Na_2SO_4$, filtered and concentrated. The residue was purified by flash column chromatography (0~30% EtOAc in PE) to afford ethyl 2-(pyrimidin-4-yl)nicotinate (2.3 g) as a red oil. $^1H$ NMR (400 MHz, $CDCl_3$) $\delta_H$ 9.29-9.12 (m, 1H), 9.01-8.86 (m, 1H), 8.83-8.68 (m, 1H), 8.17-7.94 (m, 2H), 7.58-7.38 (m, 1H), 4.34-4.20 (m, 2H), 1.64 (s, 3H).

Syntheis of A4

A suspension of ethyl 2-(pyrimidin-4-yl)nicotinate (2.3 g 10.0 mmol) and $LiOH \cdot H_2O$ (629 mg, 15.0 mmol) in THF (10 mL) and MeOH (10 mL) was stirred at 25 °C for 2 h to give a suspension. The mixture was concentrated, and the residue dried in a vacuum drying oven at 70 °C for 2 h and then at 100 °C for 10 min to give 2-(pyrimidin-4-yl)nicotinic acid (2.48 g) as a solid. $^1H$ NMR (400 MHz, $CDCl_3$) $\delta_H$ 9.16-9.03 (m, 1H), 8.84-8.72 (m, 1H), 8.55-8.41 (m, 1H), 7.97-7.84 (m, 1H), 7.76-7.67 (m, 1H), 7.44-7.32 (m, 1H).

Synthesis of A5

Step 1. To a solution of DIPA (642 mg, 6.3 mmol) in THF (4 mL) was added n-BuLi (2.53 mL, 2.5 M in hexane, 6.3 mmol) at -70 °C. The mixture was warmed to 0 °C and stirred for 1 h. To a cold (0 °C) of this LDA solution was added tert-butyl 4-cyanopiperidine-1-carboxylate (1.33 g, 6.3 mmol) in THF (10 mL). The mixture was stirred at 0 °C for 0.5 h. Then 1-(bromomethyl)-4-fluorobenzene (1 g, 5.3 mmol) was added and the mixture was stirred at 75 °C for 5h, poured into water (20 mL), and extracted with EtOAc (3 x 30 mL). The combined organic extracts were washed with brine (2 x 50 mL), dried over anhydrous $Na_2SO_4$, filtered, and concentrated to give 2 g of the product as a solid. The residue was purified by silica gel flash chromatography using a gradient elution from 0 to 30% EtOAc and PE to give 4-(4-fluorobenzyl)-1-(2-(pyrimidin-4-yl)nicotinoyl)piperidine-4-carbonitrile (1.6 g, 80% yield) as a solid. $^1H$ NMR (400 MHz, $CDCl_3$) $\delta$ 7.26-7.19 (m, 2H), 7.03 (t), 4.25-4.05 (m, 2H), 3.10-2.90 (m, 2H), 2.83 (s, 3H), 1.87-1.77 (m, 2H), 1.55-1.42 (m, 11H).

Step 2. To a solution of *tert*-butyl 4-cyano-4-(4-fluorobenzyl)piperidine-1-carboxylate (300 mg, 0.9 mmol) in DCM (10 mL) was added TFA (527 mg, 4.7 mmol). The mixture was stirred at 10°C for 3 h. Saturated $NaHCO_3$ solution (10 mL)

was added to adjust the pH to 8 and the aqueous layer was extracted with DCM (3 x 10 mL). The combined organic extracts were washed with brine (10 mL), dried over anhydrous $Na_2SO_4$, filtered and concentrated under reduced pressure to afford 4-(4-fluorobenzyl)piperidine-4-carbonitrile (200 mg, 99%) as an oil.

Synthesis of A6

To a solution of 2-(pyrimidin-4-yl)nicotinic acid (138 mg, 0.7 mmol) in DMF (5 mL) at 0 °C was added HATU (208 mg, 0.5 mmol) and DIPEA (176 mg, 1.4 mmol). The mixture was stirred at 0 °C for 10 min and then a solution of 4-(4-fluorobenzyl)piperidine-4-carbonitrile (100 mg, 0.5 mmol) in DMF (3 mL) was added. The reaction stirred at 10 °C for 2h and then combined with the duplicate reaction. Water (30 mL) was added and the aqueous layer was extracted with EtOAc (3 x 30 mL). The combined organic extracts were washed with brine (30 mL), dried over anhydrous $Na_2SO_4$, filtered and concentrated under reduced pressure. Purification by column chromatography ($CH_2Cl_2$/MeOH 10/1) afforded 160 mg of an oil, which was further purified by prep-HPLC (column: Waters Xbridge 150*25 5μm. condiion; water (10 mM $NH_4HCO_3$)-ACN; Begin B: 25; End B: 55; Flow Rate of 25 mL/min to afford 4-(4-fluorobenzyl)-1-(2-(pyrimidin-4-yl) nicotinoyl)piperidine-4-carbonitrile (118.8 mg, 32% yield) as a solid. $^1H$ NMR (400MHz, DMSO-d6, t = 80°C) $\delta_H$ 9.10 (s, 1H), 8.97 (d), 8.79 (t), 8.20-8.18 (m, 1H), 7.86 (dd, 1H), 7.68-7.60 (m, 1H), 7.40-7.30 (m, 2H), 7.16 (t, 2H), 4.57 (d, 1H), 3.55-3.40 (m, 1H), 3.30-2.90 (m, 4H), 2.00-1.92 (m, 1H), 1.80-1.60 (m, 3H). LC-ELSD/MS purity>=99%, MS ESI calcd. for $C_{23}H_{21}FN_5O$ $[M+H]^+$ 402, found 402.

*Example 3. Synthesis of 1-([2,4'-bipyridine]-3-carbonyl)-4-(4-(trifluoromethoxy)benzyl)piperidine-4-carbonitrile (A10)*

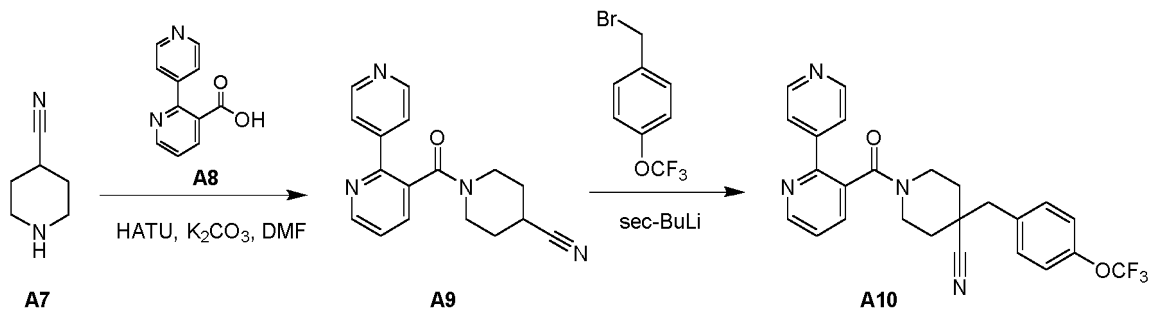


A7 A9 A10

Synthesis of A9

To a suspension of piperidine-4-carbonitrile trifluoroacetic acid (2.37 g, 10.6 mmol) and lithio[2,4'-bipyridine]-3-carboxylate (2.2 g, 10.6 mmol) in a mixture of THF (10.6 mL) and DMF (106 mL) was added anhydrous potassium carbonate (4.39 g, 31.8 mmol) in one portion. The mixture stirred for 1 h at rt and then HATU (4.22 g, 11.1 mmol) was added and stirring continued for 2 h at rt. The mixture was diluted with EtOAc (250 mL), filtered through glass wool and the organic phase was concentrated under reduced pressure. The residue was purified by reverse phase chromatography, ISCO 2x60 g C-18 silica, solvents: solvent A: 0.1% ammonium carbonate in water, solvent B (acetonitrile), 10% MeCN (3 CV), 10% to 50 % MeCN (5 CV), 50 % MeCN (3 CV). The fractions containing the product were combined and lyophilized affording 1-{[2,4'-bipyridine]-3-carbonyl}piperidine-4-carbonitrile (2.33 g, 7.97 mmol, 75% yield) as a foam. LCMS (ESI): $(M+H)^+$ calcd. 293.2, found 293.1. $^1H$ NMR (400 MHz, DMSO-d6): δ 8.78 (d, 1 H); 8.69 (m; 2 H); 7.93-7.87 (m; 1 H); 7.54-7.60 (m; 3 H); 3.88 (s; 1 H); 3.2 (m; 1 H); 3.12 (m; 0.5 H); 2.79 (s; 2 H); 2.8 (s; 0.5); 1.9-1.5 (m; 3 H); 1.3 (m; 1 H); 0.66 (s; 0.4 H).

Synthesis of A10

To a solution of 1-{[2,4'-bipyridine]-3-carbonyl}piperidine-4-carbonitrile (116.2 mg, 396 μmol) in THF (1.97 mL) was added sec-BuLi (1.4 M in cyclohexanes,367 μL,514 μmol) at -78 °C and the reaction mixture stirred 30 min. Solid 1-(bromomethyl)-4-(trifluoromethoxy)benzene (131 mg, 514 μmol) was added in one portion and the reaction mixture stirred 2 h at -78 °C and then warmed to room temperature and stirred overnight. Water was added and the mixture extracted with EtOAc twice. The combined organic extracts were washed with brine, dried over anhydrous sodium sulfate and concentrated under reduced pressure. The residue was dissolved in DMSO and purified over a 12 g C18 reverse-phase column using a gradient elution. Solvent A was 0.1% ammonium carbonate in water; solvent B was acetonitrile; and the gradient was 20% solvent B (5 min), 20% to 100% solvent B (15 min) and 100% solvent B (5 min) to provide the product (30 mg, 77 μmol, 17.4%, 90%) as a solid. The material was further purified by HPLC to afford 1-([2,4'-bipyridine]-3-carbonyl)-4-(4-(trifluoromethoxy)benzyl)piperidine-4-carbonitrile (11.2 mg, 32 μmol, 7.2% yield) as a solid. LCMS (ESI): $(M+H)^+$ calcd. 467.2, found 467.1. $^1H$ NMR (400 MHz, $CD_3OD$): δ 8.79 (m; 2 H); 8.66 (s; 1 H); 7.92 (s; 1 H); 7.79 (s; 1 H); 7.66 (s; 1 H); 7.60 (s; 1 H); 7.38 (s; 1 H); 7.25 (m; 3 H); 4.69 (s; 1.2 H); 3.4 (m, 0.4 H); 3.11 (s; 0.8 H); 2.94 (s; 1.7 H); 2.72 (m; 1.7 H); 2.57 (m; 0.6 H); 1.91 (d; 1 H); 1.63 (s; 1 H); 1.45 (s; 0.5 H); 1.29 (s; 0.6 H); 0.10 (s; 0.5 H).

*Example 4. Synthesis of 1-{[2,4'-bipyridine]-3-carbonyl}-4-[(4-chloro-2-fluorophenyl)methyl]piperidine-4-carbonitrile (A11)*

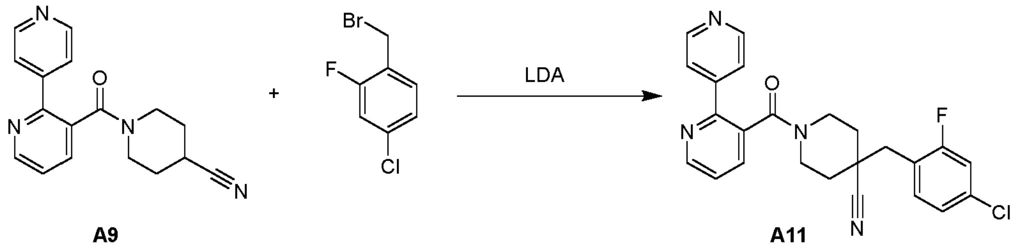


A9

A11

To a solution of 1-{[2,4'-bipyridine]-3-carbonyl}piperidine-4-carbonitrile (116 mg, 396 μmol) in THF (1.32 mL) was added 2 M LDA in THF (217 μL, 435 μmol) at -78 °C and the reaction mixture stirred 30 min. 1-(Bromomethyl)-4-chloro-2-fluorobenzene (56.6 μL, 407 μmol) was added in one portion and the reaction mixture stirred for 2 h at -78 °C and then warmed to room temperature and stirred overnight. The mixture was diluted with EtOAc, filtered and the organic phase concentrated under reduced pressure. The residue was dissolved in DMSO and purified over a 12 g C18 reverse-phase column using a gradient elution. Solvent A was 0.1% ammonium carbonate in water; solvent B was acetonitrile; and the gradient was 20% solvent B (5 min), 20% to 100% solvent B (15 min) and 100% solvent B (5 min) to provide the product (55 mg, 90% purity) as a solid. The material was further purified by HPLC to afford 1-{[2,4'-bipyridine]-3-carbonyl}-4-[(4-chloro-2-fluorophenyl)methyl]piperidine-4-carbonitrile (35 mg, 80.4 μmol, 20% yield, 99% purity) as a solid. LCMS (ESI): $(M+H)^+$ calcd. 435.1, 437.1, found 435.1, 437.1. $^1H$ NMR (400 MHz, $CD_3OD$): δ 8.80 (dd; 1 H); 8.73 (m; 2 H); 7.91 (m; 1 H); 7.77 (s; 1 H); 7.66 (s; 1 H); 7.60 (t; 1 H); 7.30 (m; 1 H); 7.17-7.22 (m; 2 H); 4.70 (s; 1.2 H); 3.39 (s; 0.5 H); 3.11 (m; 0.6 H); 2.96 (s; 1.5 H); 2.60-2.76 (m; 2.2 H); 1.93 (s; 1 H); 1.66 (m; 1.2 H); 1.47 (m; 0.6 H); 1.27 (s; 0.6 H); 0.18 (s; 0.5 H).

*Example 5. Synthesis of 1-{[2,4'-bipyridine]-3-carbonyl}-4-[(2-fluorophenyl)methyl]piperidine-4-carbonitrile (A12)*

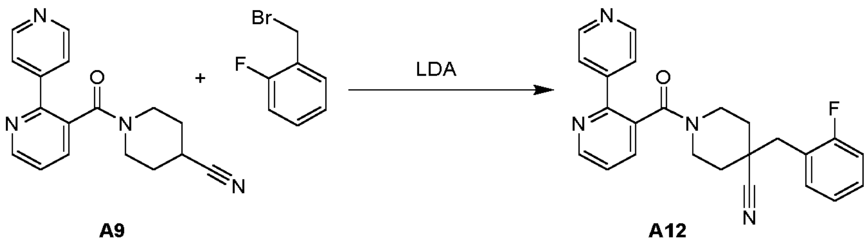


A9

A12

To a solution of 1-{[2,4'-bipyridine]-3-carbonyl}piperidine-4-carbonitrile (100 mg, 342 μmol) in THF (1.14 mL) was added LDA (2 M in THF, 188 μL, 376μmol) at -78 °C. The reaction mixture stirred for 30 min and then 1-(bromomethyl)-2-fluorobenzene (66.5 mg, 352 μmol) was added in one portion and stirring continued for 2 h at -78 °C and then warmed to room temperature and stirred overnight. The mixture was diluted with EtOAc and solids were removed by filtration and the organic phase concentrated under reduced pressure. The residue was dissolved in DMSO and purified over a 12 g C18 reverse-phase column using a gradient elution. Solvent A was 0.1% ammonium carbonate in water; solvent B was acetonitrile; and the gradient was 20% solvent B (5 min), 20% to 100% solvent B (15 min) and 100% solvent B (5 min) to provide the product (50 mg, 90% purity) as a solid. The material was further purified by HPLC to afford 1-{[2,4'-bipyridine]-3-carbonyl}-4-[(2-fluorophenyl)methyl]piperidine-4-carbonitrile (20 mg, 49.9 μmol, 14.7% yield, 99% purity) as a solid. LCMS (ESI): $(M+H)^+$ calcd. 401.2, found 401.2. $^1H$ NMR (400 MHz, $CD_3OD$): δ 8.80 (d; 1 H); 8.69 (m; 2 H); 7.91 (m; 1 H); 7.77 (s; 1 H); 7.66 (s; 1 H); 7.60 (s; 1 H); 7.30-7.33 (br; 2 H); 7.07-7.16 (m; 2 H); 4.70 (br; 1.2 H); 3.10 (m; 0.7 H); 2.97 (s; 1.6 H); 2.73 (m; 1.6 H); 2.62-2.65 (m; 0.8 H); 1.93 (s; 1.2 H); 1.76 (s; 0.4 H); 1.66 (s; 0.7 H); 1.48 (d; 0.6 H); 1.29 (s; 0.6 H); 0.22 (s; 0.5 H).

*Example 6. Synthesis of 1-{[2,4'-bipyridine]-3-carbonyl}-4-[(3-fluorophenyl)methyl]piperidine-4-carbonitrile (A13)*

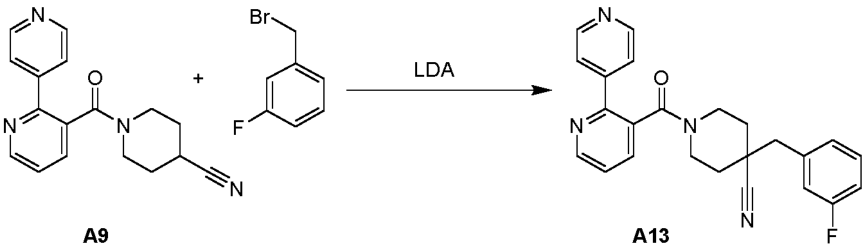


A9 A13

To a solution of 1-{[2,4'-bipyridine]-3-carbonyl}piperidine-4-carbonitrile (100 mg, 342 μmol) in THF (1.14 mL) was added LDA (2 M in THF, 188 μL, 376μmol) at -78 °C and the reaction mixture stirred for 30 min. 1-(Bromomethyl)-3-fluorobenzene (43.1 μL, 352μmol) was added in one portion and the reaction mixture stirred 2 h at -78 °C and then was warmed to room temperature and stirred overnight. The mixture was diluted with EtOAc, solids were removed by filtration and the organic phase concentrated under reduced pressure. The residue was dissolved in DMSO and purified over a 12 g C18 reverse-phase column using a gradient elution. Solvent A was 0.1% ammonium carbonate in water; solvent B was acetonitrile; and the gradient was 20% solvent B (5 min), 20% to 100% solvent B (15 min) and 100% solvent B (5 min) to provide the product (50 mg, 90% purity) as a solid. Further purification by HPLC provided 1-{[2,4'-bipyridine]-3-carbonyl}-4-[(3-fluorophenyl)methyl]piperidine-4-carbonitrile (15 mg, 37.4 μmol, 11% yield, 99% purity) as a solid. LCMS (ESI): $(M+H)^+$ calcd. 401.1, found 401.2. $^1H$ NMR (400 MHz, $CD_3OD$): δ 8.80 (m; 1 H); 8.75-8.66 (m; 2 H); 7.93 (s; 1 H); 7.77 (s; 1 H); 7.66 (s; 1 H); 7.60 (s; 1 H); 7.33 (m; 1 H); 7.03 (s; 3 H); 4.69 (s; 1.2 H); 3.4 (s; 0.5 H); 3.1 (m; 0.6 H); 2.92 (m; 1.5 H); 2.73 (s; 1.5 H); 2.68 (m; 0.6 H); 1.91 (d; 1 H); 1.62 (m; 1.3 H); 1.41 (m; 0.5 H); 1.28 (m; 0.5 H); 0.01 (m; 0.5 H).

*Example 7. Synthesis of 1-([2,4'-bipyridine]-3-carbonyl)-4-(4-fluorobenzyl)piperidine-4-carbonitrile (A14)*

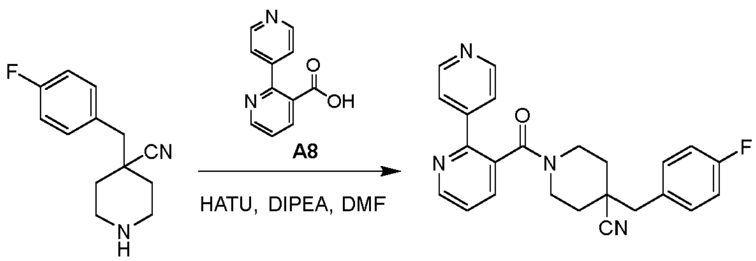


A5 A14

4-[(4-Fluorophenyl)methyl]piperidine-4-carbonitrile hydrochloride (0.39 mmol), [2,4'-bipyridine]-3-carboxylic acid dihydrochloride (0.47 mmol), and HATU (0.51 mmol) were dissolved in DMF (3 ml) under nitrogen and DIPEA (3.13 mmol) was added and stirred at rt for 2 h. The mixture was taken up in EtOAc and washed with $NH_4Cl$ sat, $NaHCO_3$ sat and brine, dried over $MgSO_4$ and concentrated under reduced pressure. The residue was purified by silica gel chromatography using a gradient elution (2% to 10% MeOH/DCM) to give a solid. The solid was taken up in 30% acetonitrile/water, frozen and lyophilized to give 1-([2,4'-bipyridine]-3-carbonyl)-4-(4-fluorobenzyl)piperidine-4-carbonitrile as a solid (45 mg, 28% yield). LCMS (ESI): $(M+H)^+$ calcd. 401.2, found 401.2. $^1H$ NMR, (400 MHz, DMSO-d6): mixture of rotamers: 8.79 (d; 1 H); 8.75 (s; 1 H); 8.66 (s; 1 H); 7.90 (s; 1 H); 7.60 (d, 2 H); 7.55 (s; 2 H); 7.27 (s; 1 H); 7.16 (t; 3 H); 4.51 (t; 1 H); 3.34 (s; 0.5 H); 3.18 (d; 0.5 H); 2.98 (t; 0.5 H); 2.88 (s; 1 H); 2.79 (t; 0.5 H); 2.66 (t; 1 H); 2.55 (s; 0.5 H); 1.81-1.82 (m; 0.5 H); 1.64 (m; 1 H); 1.52 (d, 0.5 H); 1.40 (d; 0.5 H); 1.23 (t; 0.5 H); 0.14 (t; 0.5 H).

*Example 8. Synthesis of 1-(2-(1H-imidazol-1-yl)nicotinoyl)-4-(4-fluorobenzyl)piperidine-4-carbonitrile (A16)*

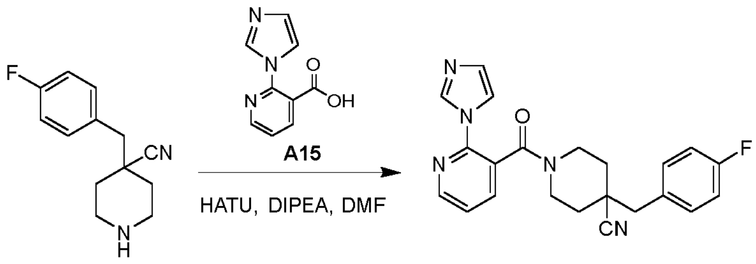


A5 A16

4-[(4-Fluorophenyl)methyl]piperidine-4-carbonitrile hydrochloride (0.39 mmol), 2-(1H-imidazol-1-yl)nicotinic acid (0.47 mmol), and HATU (0.51 mmol) were dissolved in DMF (3 ml) under nitrogen and DIPEA (3.13 mmol) was added and stirred at rt for 2 h. The mixture was taken up in EtOAc and washed with saturated $NH_4Cl$, saturated $NaHCO_3$ and brine, dried over $MgSO_4$ and concentrated under reduced pressure. The residue was purified by silica gel chromatography using a gradient elution (2% to 10% MeOH/DCM) to give a solid. The solid was taken up in 30% acetonitrile/water, frozen and lyophilized to give 1-(2-(1H-imidazol-1-yl)nicotinoyl)-4-(4-fluorobenzyl)piperidine-4-carbonitrile as a solid (106 mg, 70% yield). LCMS (ESI): $(M+H)^+$ calcd. 390.2, found 390.2. $^1H$ NMR, (400 MHz, DMSO-*d6*): mixture of rotamers: 8.61 (dd, 1 H); 8.21 (s; 1 H); 7.84 (d, 1 H); 7.61 (s; 1 H); 7.44 (dd, 1 H); 7.18 (dd, 2 H); 7.02 (t, 2 H); 4.83 (d, 1 H); 3.12 (d, 1.5 H); 2.95 (t, 1 H); 2.70 (d, 0.5 H); 2.58 (d, 0.5 H); 1.90 (d, 0.5 H); 1.46 (m; 1.5 H); 0.09 (m; 0.5 H).

*Example 9. Synthesis of 1-([2,3'-bipyridine]-3-carbonyl)-4-(4-fluorobenzyl)piperidine-4-carbonitrile (A18)*

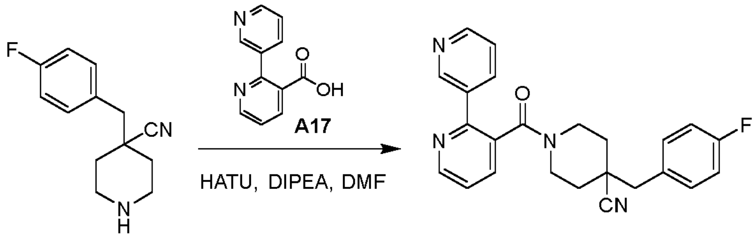


A5 A18

4-[(4-Fluorophenyl)methyl]piperidine-4-carbonitrile hydrochloride (0.39 mmol), [2,3'-bipyridine]-3-carboxylic acid dihydrochloride (0.47 mmol), and HATU (0.51 mmol) were dissolved in DMF (3 ml) under nitrogen and DIPEA (3.13 mmol) was added and stirred at rt for 2 h. The mixture was taken up in EtOAc and washed with saturated $NH_4Cl$, saturated $NaHCO_3$ and brine, dried over $MgSO_4$ and concentrated under reduced pressure. The residue was purified by silica gel chromatography using a gradient elution (2% to 10% MeOH/DCM) to give a solid. The solid was taken up in 30% acetonitrile/water, frozen and lyophilized to give 1-([2,3'-bipyridine]-3-carbonyl)-4-(4-fluorobenzyl)piperidine-4-carbonitrile as a solid (125 mg, 80% yield). LCMS (ESI): $(M+H)^+$ calcd. 401.2, found 401.2. $^1H$ NMR, (400 MHz, DMSO-*d6*): mixture of rotamers: 8.74-8.82 (m; 2 H); 8.68 (m; 1 H); 8.62 (m; 1 H); 7.95 (m; 2 H); 7.54 (m; 2 H); 7.27 (m; 1 H); 7.16 (d, 3 H); 4.49 (m; 1 H); 3.31 (m; 0.5 H); 3.17 (d, 0.5 H); 2.87 (m; 1.5 H); 2.76 (t, 0.5 H); 2.64 (m; 1 H); 2.56 (d, 0.5 H); 1.81 (t, 1 H); 1.62 (m; 1 H); 1.49 (d, 0.5 H); 1.39 (d, 0.5 H); 1.07 (m; 0.5 H); -0.01 (t, 0.5 H).

*Example 10. Synthesis of 4-([1,1'-biphenyl]-4-ylmethyl)-1-([2,4'-bipyridine]-3-carbonyl)piperidine-4-carbonitrile (A19)*

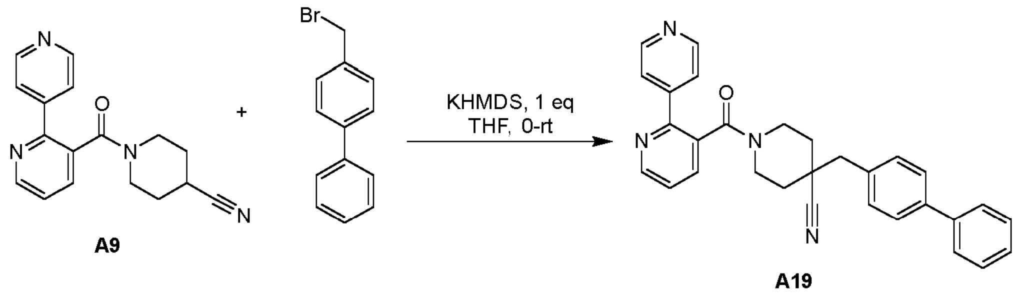


A9 A19

1-([2,4'-Bipyridine]-3-carbonyl)piperidine-4-carbonitrile (0.34 mmol) was dissolved in THF (10 ml), cooled to 0 °C and potassium hexamethyldisilizane (0.37 mmol) added. The mixture stirred for 1 h at 0 °C and then 4-(bromomethyl)-1,1'-biphenyl (0.41 mmol) was added and stirred at 0 °C for 1 h. Water and EtOAc were added and the organic layer washed with saturated $NaHCO_3$ and brine, dried over $MgSO_4$ and concentrated under reduced pressure. The residue was purified by silica gel chromatography using a gradient elution (2% to 10% MeOH/DCM) to give a solid. The solid was taken up in 30% acetonitrile/water, frozen and lyophilized to give 4-([1,1'-biphenyl]-4-ylmethyl)-1-([2,4'-bipyridine]-3-carbonyl)piperidine-4-carbonitrile as a solid (73 mg, 46% yield). LCMS (ESI): $(M+H)^+$ calcd. 459.2, found 459.3. $^1H$ NMR (400 MHz, $CD_3OD$): mixture of rotamers: 8.76-8.79 (m, 2 H); 8.66 (s, 1 H); 7.97 (s, 0.5 H); 7.90 (s, 0.5 H); 7.78 (s, 1 H); 7.66 (s, 1 H); 7.57 (d, 5 H); 7.42 (t, 2 H); 7.24-7.34 (m, 3 H); 4.69 (m, 2 H); 3.38 (d, 0.5H); 3.25 (d, 0.5 H); 3.10 (t, 0.5 H); 2.94 (s, 1.5 H); 2.73 (d, 1 H); 2.56 (d, 0.5 H); 1.94 (d, 1 H); 1.65 (m, 2 H); 1.45 (d, 0.5 H); 1.29 (t, 0.5 H); 0.12 (t, 0.5 H).

*Example 11. Synthesis of 1-([2,4'-bipyridine]-3-carbonyl)-4-(2-chlorobenzyl)piperidine-4-carbonitrile (A21)*

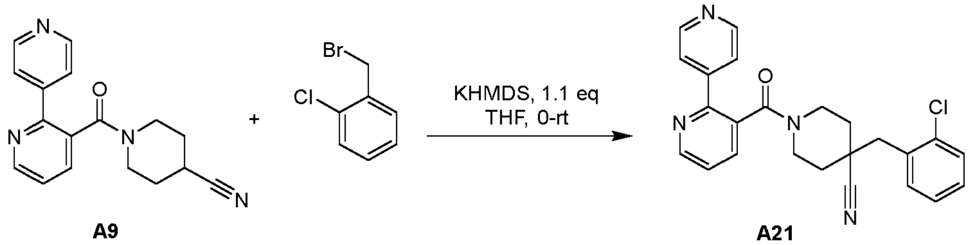


A9 A21

1-([2,4'-Bipyridine]-3-carbonyl)piperidine-4-carbonitrile (0.34 mmol) was dissolved in THF (10 ml), cooled to 0 °C and potassium hexamethyldisilizane (0.37 mmol) was added. The mixture stirred 1 h at 0 °C and 1-(bromomethyl)-2-chlorobenzene (0.32 mmol) was added and stirred at 0 °C for 1 h. Water and EtOAc were added and the organic layer washed with saturated $NaHCO_3$ and brine, dried over $MgSO_4$ and concentrated under reduced pressure. The residue was purified by silica gel chromatography using a gradient elution (2% to 10% MeOH/DCM) to give a solid. The solid was taken up in 30% acetonitrile/water, frozen and lyophilized to give 1-([2,4'-bipyridine]-3-carbonyl)-4-(2-chlorobenzyl)piperidine-4-carbonitrile as a solid (68 mg, 48% yield). LCMS (ESI): $(M+H)^+$ calcd. 417.1, found 417.2. $^1H$ NMR (400 MHz, $CD_3OD$): mixture of rotamers: 8.80 (dd, 1 H); 8.70 (s, 2 H); 7.98 (s, 0.5 H); 7.90 (s, 0.5 H); 7.78 (s, 1 H); 7.66 (s, 0.5 H); 7.60 (s, 1 H); 7.40-7.42 (m, 1.5 H); 7.27-7.29 (m, 2 H); 4.71 (m, 1 H); 3.39 (d, 0.5 H);3.24 (m, 0.5 H); 3.12 (m, 1 H); 2.94 (m, 0.5 H); 2.85 (s; 1 H); 2.81 (s, 0.5 H); 2.73 (m, 0.5 H); 1.91 (m, 1 H); 1.71 (s; 0.5 H); 1.50 (d, 0.5 H); 1.35 (t, 0.5 H); 0.32 (t, 0.5 H).

*Example 12. 1-([2,4'-bipyridine]-3-carbonyl)-4-(2-chlorobenzyl)piperidine-4-carbonitrile (A22)*

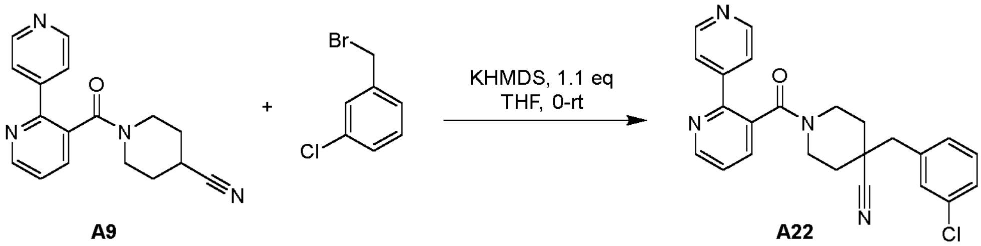


A9 A22

1-([2,4'-Bipyridine]-3-carbonyl)piperidine-4-carbonitrile (0.34 mmol) was dissolved in THF (10 ml), cooled to 0 °C and potassium hexamethyldisilizane (0.37 mmol) was added. The mixture stirred for 1 h at 0 °C and 1-(bromomethyl)-3-chlorobenzene (0.32 mmol) was added and stirred at 0 °C for 1 h. Water and EtOAc were added and the organic layer was washed with saturated $NaHCO_3$ and brine, dried over $MgSO_4$ and concentrated under reduced pressure. The residue was purified by silica gel chromatography using a gradient elution (2% to 10% MeOH/DCM) to give a solid. The solid was taken up in 30% acetonitrile/water, frozen and lyophilized to give a solid (32 mg, 22% yield). LCMS (ESI): $(M+H)^+$ calcd. 417.1, found 417.2. $^1H$ NMR (400 MHz, $CD_3OD$): mixture of rotamers: 8.80 (dd; 1 H); 8.71 (d; 2 H); 7.97 (m; 1 H); 7.91 (m; 1 H); 7.77 (s; 1 H); 7.66 (br s; 1 H); 7.60 (m; 1 H); 7.31 (d; 2 H); 7.21 (s; 1 H); 7.11 (s; 1 H); 4.69 (m; 1 H); 3.39 (d; 0.5 H); 3.25 (d; 0.5 H); 3.10 (t; 0.5 H); 2.90-2.98 (m; 1 H); 2.69-2.75 (m; 1 H); 2.54 (d; 0.5 H); 1.91 (d; 1 H); 1.71 (m; 0.5 H); 1.62 (s; 0.5 H); 1.40 (d; 0.5 H); 1.28 (t; 0.5 H); 0.07 (t; 0.5 H).

*Example 13. Synthesis of 1-([2,4'-bipyridine]-3-carbonyl)-4-(2-methylbenzyl)piperidine-4-carbonitrile (A25)*

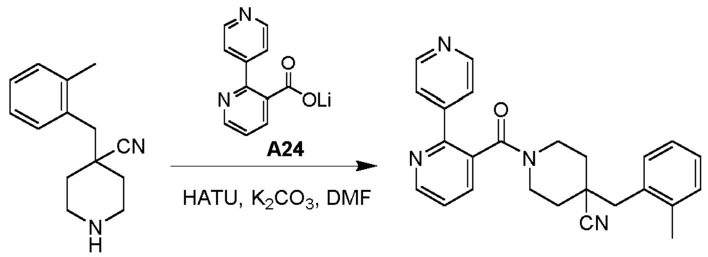


A23 A25

To a suspension of 4-(2-methylbenzyl)piperidine-4-carbonitrile trifluoroacetate (100 mg, 0.30 mmol) and lithio [2,4'-bipyridine]-3-carboxylate (65.8 mg, 0.32 mmol) in a mixture of THF (0.3 mL) and DMF (2.7 mL) was added anhydrous potassium carbonate (126 mg, 0.91 mmol) and the mixture stirred for 1 h at rt. HATU (121 mg, 0.32 mmol) was added and the reaction mixture stirred 16 h at rt. The mixture was diluted with EtOAc (15 mL), filtered through glass wool and the organic phase was concentrated under reduced pressure. The residue was purified by flash chromatography using a gradient elution (0% to 100% MeOH with 10% $NH_4OH$/DCM) to give 1-([2,4'-bipyridine]-3-carbonyl)-4-(2-methylbenzyl) piperidine-4-carbonitrile (101 mg, 0.25 mmol, 83% yield). LCMS (ESI):$(M+H)^+$calcd. 397.2, found 397.2. $^1H$ NMR (400 MHz, DMSO-$d_6$): Mixture of rotamers; 8.79 (d; 1 H); 8.70 (d; 2 H); 7.88-7.96 (m; 1 H); 7.64 (s; 1 H); 7.56 (d; 2 H); 7.11-7.17 (m; 3 H); 4.51-4.60 (m; 1 H); 3.3 1 (br s; 0.7 H);3.17 (d; 0.4 H); 2.87-3.00 (m; 1.2 H); 2.60-2.80 (m; 2 H); 2.33 (s; 1.2 H); 2.24 (s; 1.5 H); 1.89 (d; 0.8 H); 1.67-1.79 (m; 1.1 H); 1.49 (d; 0.4 H); 1.19-1.26 (m; 0.6 H); 0.20 (t; 0.3 H).

*Example 14. Synthesis of 1-([2,4'-bipyridine]-3-carbonyl)-4-(4-cyanobenzyl)piperidine-4-carbonitrile (A27)*

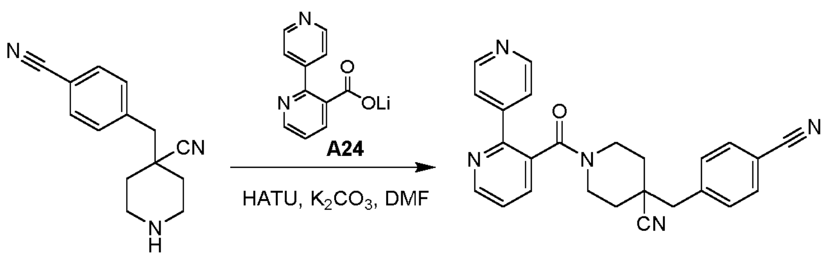


A26 A27

To a suspension of 4-(4-cyanobenzyl)piperidine-4-carbonitrile trifluoroacetate (104 mg, 0.31 mmol) and lithio [2,4'-bipyridine]-3-carboxylate (66.3 mg, 0.32 mmol) in a mixture of THF (0.3 mL) and DMF (2.7 mL) was added anhydrous potassium carbonate (127 mg, 0.92 mmol) and the mixture stirred 1 h at rt. HATU (122 mg, 0.32 mmol) was added and the reaction mixture stirred 16 h at room temperature. The mixture was diluted with EtOAc (15 mL), filtered through glass wool and the organic phase was concentrated under reduced pressure. The residue was purified by flash chromatography using a gradient elution (0% to 100% MeOH with 10% $NH_4OH$/DCM) to afford 1-([2,4'-bipyridine]-3-carbonyl)-4-(4-cyanobenzyl)piperidine-4-carbonitrile (89 mg, 0.21 mmol, 70% yield). LCMS (ESI): $(M+H)^+$ calcd. 408.2, found 408.2. $^1H$

NMR (400 MHz, DMSO-$d_6$): Mixture of rotamers; 8.79 (s; 1 H); 8.70 (d; 2 H); 7.90 (t; 1 H); 7.82 (d; 2 H); 7.53-7.61 (m; 4 H); 7.40 (dd; 2 H); 4.51 (t; 1 H); 3.17-3.28 (m; 1.2 H); 3.00 (s; 1.6 H); 2.62-2.82 (m; 2.8 H); 1.82-1.85 (m; 1.1 H); 1.62-1.73 (m; 1 H); 1.41-1.54 (m; 1 H); 1.22-1.29 (m; 0.5 H); 0.19 (t; 0.3 H).

*Example 15. Synthesis of 4-benzyl-1-(2-(pyrimidin-4-yl)nicotinoyl)piperidine-4-carbonitrile (A31)*

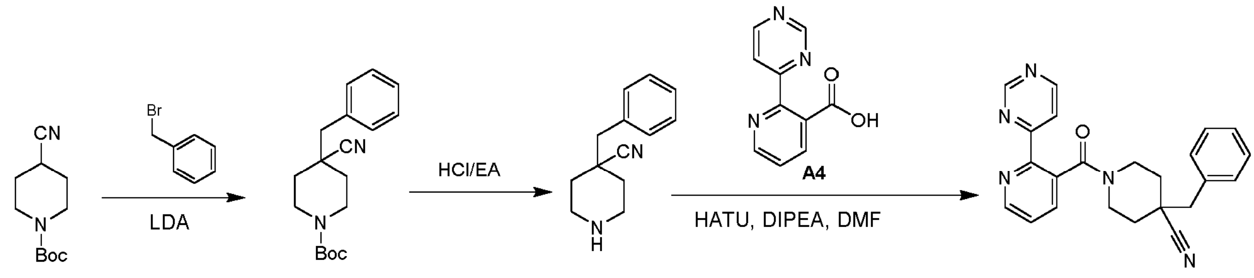


A28 A29 A30 A31

Synthesis of A29

To a solution of DIPA (5.75 g, 56.9 mmol) in THF (40 mL) was added BuLi (22.7 mL, 2.5 M in hexane, 56.9 mmol) at -70 °C. The mixture was warmed to 0 °C and stirred for 30 min to give an LDA solution. To the cold (0°C) LDA solution was added a solution of tert-butyl 4-cyanopiperidine-1-carboxylate (10 g, 47.5 mmol) in THF (20 mL). After stirring at 0 °C for 0.5 h, (bromomethyl)benzene (9.73 g, 56.9 mmol) was added. The mixture was stirred at 75 °C for 5 h, poured into water (100 mL) and extracted with EtOAc (3 x 100 mL). The combined organic phase was washed with brine (2 x 100 mL), dried over $Na_2SO_4$ and concentrated. The residue was purified by flash chromatography using a gradient elution (0~30% EtOAc in PE) to give tert-butyl 4-benzyl-4-cyanopiperidine-1-carboxylate (10g, 70.4% yield) as a solid and used directly for the next step.

Synthesis of A30

To a solution of 4-benzyl-4-cyanopiperidine-1-carboxylate (10 g, 33.2 mmol) in EtOAc (40 mL) was added 40 mL of 4 M HCl in EtOAc. The mixture was stirred at 25 °C for 16 h and concentrated under reduced pressure to give 4-benzylpiperidine-4-carbonitrile hydrogen chloride salt (5.5g) as a solid. $^1$H NMR (400 MHz, $CDCl_3$) $\delta_H$ 10.20-9.66 (m, 2H), 7.27-7.40 (m, 5H), 3.55 (d, 2H) 3.28 - 3.07 (m, 2H), 2.93 (s, 2H), 2.25-1.97 (m, 4H).

Synthesis of A31

To a solution of 2-(pyrimidin-4-yl)nicotinic acid (203 mg, 1.00 mmol) and HATU (384 mg, 1.00 mmol) in DMF (10 mL) was added DIPEA (435 mg, 3.36 mmol) and 4-benzylpiperidine-4-carbonitrile-HCl (200 mg, 0.844 mmol) at 10-15 °C. The mixture was stirred at 10-15 °C for 12 h, poured into ice-water (60 mL) and stirred for 1 minute. The aqueous phase was extracted with ethyl acetate (3 x 60 mL) and the combined organic phase was washed with brine (3 x 60 mL), dried over anhydrous $Na_2SO_4$ and concentrated. The residue was purified by preparative HPLC (prep-HPLC method, Instrument: Gilson GX-281 Liquid Handler, Gilson 322 Pump, Gilson 156 UV Detector; Column: Phenomenex Gemini-NX 150*30mm*5um, Mobile phase A: water (0.04%$NH_3.H_2O$+10mM $NH_4HCO_3$), Mobile phase B: MeCN; Gradient: B from 29% to 59% in 10 min, then hold 100% B for 2.5 min; Flow Rate: 25 mL/min; Column Temperature: 30 °C; Wavelength: 220 nm, 254 nm) to afford 4-benzyl-1-(2-(pyrimidin-4-yl)nicotinoyl)piperidine-4-carbonitrile (115.1 mg, 0.3 mmol) as a solid. LC-MS purity 99%, MS ESI calcd. for $C_{23}H_{22}N_5O$ [M+H]$^+$ 384.2, found 384.2. $^1$H NMR (400 MHz,DMSO-$d_6$) $\delta_H$ 9.17 (s, 0.5H), 8.98-8.95 (m, 1.5H), 8.79 (s, 1H), 8.24-8.19 (m, 1H), 7.87 (d, 1H), 7.66 (s, 1H), 7.37-7.32 (m, 5H), 4.62-4.56 (m, 1H), 3.52-3.49 (m, 0.5H), 3.29 (s, 1.5H), 3.00-2.88 (m, 3H), 1.95 (d, 1H), 1.75-1.67 (m, 3H).

*Example 16. Synthesis of 1-([2,4'-bipyridine]-3-carbonyl)-4-benzylpiperidine-4-carbonitrile (A32)*

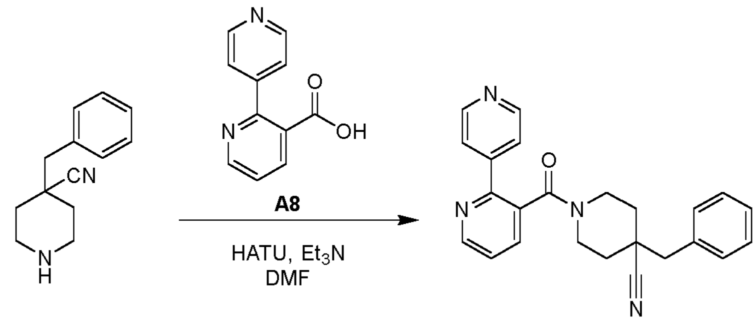


A30 A32

To a solution of [2,4'-bipyridine]-3-carboxylic acid (0.3 g, 1.49 mmol), HATU(1.13 g, 2.98 mmol), triethylamine (452 mg, 4.47 mmol) in DMF (5 mL) was added 4-benzylpiperidine-4-carbonitrile hydrogen chloride (421 mg, 1.78 mmol). The mixture was stirred at 15 °C for 16 h and poured into ice-water (60 mL) and stirred for 1 minute. The aqueous phase was extracted with ethyl acetate (3 x 60 mL) and the combined organic phase was washed with brine (3 x 60 mL), dried over anhydrous $Na_2SO_4$ and concentrated. The residue was purified by prep-HPLC (Column: YMC Triart C18 150*25mm*5um) using a gradient elution (condition A: 10 mM aqueous $NH_4HCO_3$ and condition B: acetonitrile) going from 41% to 71% B over 9.5 min, then 100% B for 2 min to afford 1-([2,4'-bipyridine]-3-carbonyl)-4-benzylpiperidine-4-carbonitrile (120 mg, 21.0% yield) as a solid. $^1H$ NMR (400 MHz, $CDCl_3$) $\delta_H$ 8.84 - 8.76 (m, 2.4H), 8.75 - 8.67 (m, 0.6H), 7.80 - 7.72 (m, 2.4H), 7.63 - 7.54 (m, 0.6H), 7.48 - 7.40 (m, 1H), 7.35 - 7.27 (m, 3H), 7.23 - 7.17 (m, 0.5H), 7.16 - 7.07 (m, 1.5H), 4.88 - 4.67 (m, 1H), 3.26 - 2.16 (m, 0.2H), 3.14 - 2.98 (m, 1.5H), 2.97 - 2.63 (m, 2H), 2.63 - 2.55 (m, 0.8H), 2.50 - 2.38 (m, 0.8H), 1.99-1.91 (m, 0.2H), 1.91 - 1.80 (m, 0.8H), 1.66 (br s, 0.2H), 1.35 - 1.23 (m, 1H), 1.22 - 1.09 (m, 0.8H), -0.07 - -0.20 (m, 0.7H). LCMS Purity 99%, MS ESI calcd. For $C_{24}H_{23}N_4O$ $[M+H]^+$ 383.2, found 383.2.

*Example 17. Synthesis of 4-(4-methylbenzyl)-1-(2-(pyrimidin-4-yl)nicotinoyl)piperidine-4-carbonitrile (A35)*

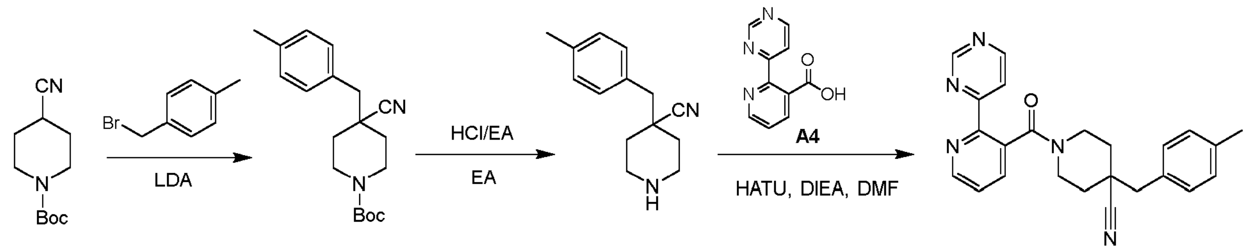


A28 A33 A34 A35

Synthesis of A33

To a solution of DIPA (2.87g, 28.4mmol) in THF (20 mL) was added BuLi (11.3 mL, 2.5 M in hexane, 28.4mmol) at -70 °C. The mixture was warmed to 0 °C and for 30 min to give an LDA solution. To the cold (0 °C) LDA solution (28.4 mmol) was added tert-butyl 4-cyanopiperidine-1-carboxylate (5 g, 23.7 mmol) as a solution in THF (10 mL) at 0 °C and stirred for 0.5 h. Then 1-(bromomethyl)-4-methylbenzene (5.25 g, 28.4 mmol) was added and the mixture was stirred at 75 °C for 5 h, poured into water (20 mL) and extracted with EtOAc (3 x 30 mL). The combined organic extracts were washed with brine (2 x 50 mL), dried over $Na_2SO_4$ and concentrated under reduced pressure. The residue was purified by silica gel chromatography using a gradient elution (0 to 15% EtOAc in PE) to give tert-butyl 4-cyano-4-(4-methylbenzyl)piperidine-1-carboxylate (6.1 g, 81.8%) as a solid. $^1H$ NMR (400 MHz, $CDCl_3$) $\delta_H$ 7.15 (s, 4H), 4.29-4.08 (m, 2H), 3.08-2.91 (m, 2H), 2.82 (s, 2H), 2.34 (s, 3H), 1.83 (d, 2H), 1.45 (s, 9H), 0.89-0.85 (m, 2H).

Synthesis of A34

To a solution of tert-butyl 4-cyano-4-(4-methylbenzyl)piperidine-1-carboxylate (0.5 g, 1.59 mmol) in 2 mL of EtOAc was added 2 mL of 4 M HCl in EtOAc and the mixture was stirred at 25 °C for 16 h. The reaction mixture was concentrated under reduced pressure to give 4-(4-methylbenzyl)piperidine-4-carbonitrile HCl salt (0.3g) as a solid. $^1H$ NMR (400 MHz, $CDCl_3$) $\delta_H$ 7.17 (s, 4H), 3.53 (d, 2H), 3.15 (d, 2H), 2.89 (s, 2H), 2.34 (s, 3H), 2.18-1.99 (m, 4H), 1.35-1.17 (m, 1H).

Synthesis of A35

To a solution of 2-(pyrimidin-4-yl)nicotinic acid (200 mg, 0.994 mmol) in DMF (5 mL) at 15 °C was added HATU (452 mg, 1.19 mmol) and DIEA (384 mg, 2.98 mmol). After stirring at 15°C for 10 min a solution of 4-(4-methylbenzyl) piperidine-4-carbonitrile (255 mg, 1.19 mmol) in DMF (3 mL) was added and stirred at 15°C for 2 h, treated with water (30 mL) and extracted with EtOAc (3 x 30 mL). The combined organic extracts were washed with brine (30 mL), dried over anhydrous $Na_2SO_4$ and concentrated under reduced pressure to give 200 mg product as an oil. The residue was purified by HPLC (Column: Waters Xbridge BEH C18 150*25mm*5um) using a gradient elution (condition A 10mM aqueous $NH_4HCO_3$ and condition B acetonitrile) going from 36% to 46% B over 9.5 min) to afford 4-(4-methylbenzyl)-1-(2-(pyrimidin-4-yl)nicotinoyl)piperidine-4-carbonitrile (72.4 mg, 36.3%) as a solid. $^1H$ NMR (400 MHz, $CDCl_3$) $\delta_H$ 9.21 (s, 0.3H), 8.88 (d, 1H), 8.75 (d, 1H), 8.73-8.70 (m, 1H), 8.72 (s, 0.7H), 8.30-8.18 (m, 1H), 7.71-7.60 (m, 1H), 7.46 (dd, 1H), 7.17-7.09 (m, 4H), 4.98-4.63 (m, 1H), 3.72-3.02 (m, 3H), 2.99-2.75 (m, 2H), 2.39-2.32 (m, 3H), 2.17-1.96 (m, 1H), 1.91-1.57 (m, 3H). LCMS purity>99%, MS ESI calcd. for $C_{24}H_{24}N_5O$ $[M-H]^+$ 398.1, found 398.1.

*Example 18. Synthesis of 4-(4-chlorobenzyl)-1-(2-(pyrimidin-4-yl)nicotinoyl)piperidine-4-carbonitrile (A37)*

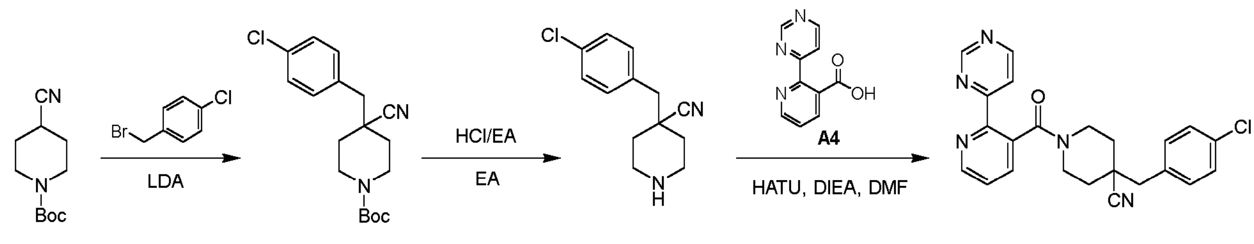


**A28** **A59** **A36** **A37**

Synthesis of A59

To a solution of DIPA (2.87g, 28.4mmol) in THF (20 mL) was added BuLi (11.3 mL, 2.5 M in hexane, 28.4 mmol) at -70 °C. The mixture was warmed to 0° C and stirred for 30min to give an LDA solution. To the cold (0 °C) LDA solution (28.4 mmol) was added tert-butyl 4-cyanopiperidine-1-carboxylate (5 g, 23.7 mmol) in THF (10 mL) at 0 °C over 0.5 h. Then 1-chloro-4-(chloromethyl)benzene (4.57 g,28.4 mmol) was added and the mixture stirred at 75 °C for 5 h, poured into water (20 mL) and extracted with EtOAc (3 x 30 mL). The combined organic extracts were washed with brine (2 x 50 mL), dried over $Na_2SO_4$ and concentrated under reduced pressure. The residue was purified by silica gel chromatography using a gradient elution (0-10% EtOAc in PE) to give tert-butyl 4-(4-chlorobenzyl)-4-cyanopiperidine-1-carboxylate (6.7 g, 84.4%) as a solid. $^1H$ NMR (400 MHz, $CDCl_3$) $\delta_H$ 7.32 (d, 2H), 7.21 (d, 2H), 4.30-3.94 (m, 2H), 3.11-2.90 (m, 2H), 2.83 (s, 2H), 1.83 (d, 2H), 1.56-1.46 (m, 2H), 1.45 (s, 9H).

Synthesis of A36

To tert-butyl 4-(4-chlorobenzyl)-4-cyanopiperidine-1-carboxylate (0.5 g, 1.49 mmol) in 2 mL of EtOAc was added 2 mL of 4 M HCl in EtOAc. The mixture was stirred at 25 °C for 16 h and then concentrated under reduced pressure to give 4-(4-chlorobenzyl)piperidine-4-carbonitrile (0.3 g) as a solid. $^1H$ NMR (400 MHz, $CDCl_3$) $\delta_H$ 7.34 (d, 2H), 7.25-7.21 (m, 2H), 3.60-3.45 (m, 2H), 3.23-3.10 (m, 2H), 2.91 (s, 2H), 2.21-2.01 (m, 4H).

Synthesis of A37

To a solution of 2-(pyrimidin-4-yl)nicotinic acid (200 mg, 0.9941 mmol) in DMF (5 mL) at 15 °C was added HATU (452 mg, 1.19 mmol) and DIEA (384 mg, 2.98 mmol) and stirred for 10 min at 15 °C. A solution of 4-(4-chlorobenzyl) piperidine-4-carbonitrile (279 mg, 1.19 mmol) in DMF (3 mL) was added to the reaction mixture and stirred at 15°C for 2 h, then quenched with water (30 mL) and extracted with EtOAc (3 x 30 mL). The combined organic layers were washed with brine (30 mL), dried over anhydrous $Na_2SO_4$ and concentrated under reduced pressure to give the product (200 mg,) as an oil, which was purified by HPLC (Column: Waters Xbridge BEH C18 150*25mm*5um; Condition: water (10mM $NH_4HCO_3$)-ACN; Begin B:37; End B :47 ) to afford 4-(4-chlorobenzyl)-1-(2-(pyrimidin-4-yl)nicotinoyl)piperidine-4-carbonitrile (61.9 mg, 31.1% yield) as a solid. $^1H$ NMR (400 MHz, $CDCl_3$) $\delta_H$ 9.21 (s, 0.4H), 8.96 (s, 0.6H), 8.91 (d, 0.6H), 8.87 (d, 0.4H), 8.76 (dd, 1H), 8.32-8.20 (m, 1H), 7.72-7.63 (m, 1H), 7.47 (dd, 1H), 7.33 (d, 2H), 7.24-7.16 (m, 2H), 4.99-4.70 (m,

1H), 3.69-3.01 (m, 3H), 2.98-2.81 (m, 2H), 2.16-1.97 (m, 1H), 1.88-1.66 (m, 2H), 1.56-1.39 (m, 1 H). LCMS purity 99.1%, MS ESI calcd. for $C_{23}H_{21}ClN_5O$ $[M+H]^+$ 418.0, found 418.0.

*Example 19. Synthesis of 1-(2-(pyrimidin-4yl)nicotinoyl)-4-(4-(trifluoromethyl)benzyl)piperidine-4-carbonitrile (A40)*

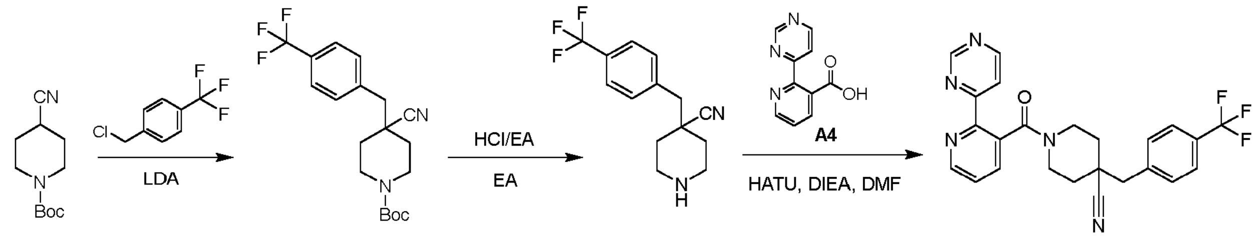


A28 A38 A39 A40

Synthesis of A38

To a solution of DIPA (2.87 g, 28.4 mmol) in THF (20mL) was added BuLi (11.3 mL, 2.5 M in hexane, 28.4 mmol) at -70 °C. The mixture was warmed to 0 °C and stirred for 30 min to give an LDA solution. To the cold (0 °C) LDA (28.4 mmol) was added tert-butyl 4-cyanopiperidine-1-carboxylate (5 g, 23.7 mmol) in THF (10 mL) at 0 °C over 0.5 h. Then 1-(chloromethyl)-4-(trifluoromethyl)benzene (5.52 g,28.4 mmol) was added and the mixture was stirred at 75 °C for 5 h, poured into water (20 mL) and extracted with EtOAc (3 x 30 mL). The combined organic extracts were washed with brine (2 x 50 mL), dried over $Na_2SO_4$ and concentrated under reduced pressure. The residue was purified by silica gel chromatography using a gradient elution (0-20% EtOAc in PE) to give tert-butyl 4-cyano-4-(4-(trifluoromethyl)benzyl) piperidine-1-carboxylate (5 g, 57.2% yield) as a solid. $^1H$ NMR (400 MHz, $CDCl_3$) $\delta_H$ 7.61 (d, 2H), 7.41 (d, 2H), 4.30-4.03 (m, 2H), 3.11-2.94 (m, 2H), 2.92 (s, 2H), 1.84 (d, 2H), 1.57-1.45 (m, 2H), 1.46 (s, 9H).

Synthesis of A39

To tert-butyl 4-cyano-4-(4-(trifluoromethyl)benzyl)piperidine-1-carboxylate (0.5 g, 1.35 mmol) was added 2 mL of 4 M HCl in EtOAc and the mixture was stirred at 25 °C for 16 h. The reaction mixture was concentrated under reduced pressure to give 4-(4-(trifluoromethyl)benzyl)piperidine-4-carbonitrile (0.3 g) as a solid. $^1H$ NMR (400 MHz, $CDCl_3$) $\delta_H$ 9.8 (s, 1H), 7.64 (d, 2H), 7.43 (d, 2H), 3.57 (d, 2H), 3.26-3.10 (m, 2H), 3.00 (s, 2H), 2.27 - 2.02(m, 4H), 1.38- 1.13 (m, 1H).

Synthesis of A40

To a solution of 2-(pyrimidin-4-yl)nicotinic acid (185 mg, 0.9195 mmol) in DMF (5 mL) at 15 °C was added HATU (418 mg, 1.10 mmol) and DIEA (354 mg, 2.75 mmol) and stirred at 15 °C for 10 min. A solution of 4-(4-(trifluoromethyl) benzyl)piperidine-4-carbonitrile (295 mg, 1.10 mmol) in DMF (3 mL) was added and the reaction mixture was stirred at 15 °C for 2 h, quenched with water (30 mL) and extracted with EtOAc (3 x 30 mL). The combined organic layers were washed with brine (30 mL), dried over anhydrous $Na_2SO_4$ and concentrated under reduced pressure to give the product (200 mg) as an oil, which was purified by HPLC (Column: Waters Xbridge BEH C18 150*25mm*5um) condition A 10 mM aqueous $NH_4HCO_3$, condition B acetonitrile; using a gradient elution from 40% B to 50% Bover 9.5 min to afford 1-(2-(pyrimidin-4-yl) nicotinoyl)-4-(4-(trifluoromethyl)benzyl)piperidine-4-carbonitrile (96.4 mg, 48.4%) as a solid. $^1H$ NMR (400 MHz, $CDCl_3$) $\delta_H$ 9.21 (s, 0.4H), 9.05 (s, 0.6H), 8.96 - 8.82 (m, 1H), 8.77 (dd, 1H), 8.30-8.25 (m, 1H), 7.74-7.59 (m, 3H), 7.50-7.36 (m, 3H), 5.04-4.72 (m, 1H), 3.70-3.18 (m, 2H), 3.17-2.91 (m, 3H), 2.09-1.99 (m, 1H), 1.96-1.56 (m, 3H). LCMS purity>99%, MS ESI calcd. for $C_{24}H_{21}F_3N_5O$ $[M+H]^+$ 452.1, found 452.1.

*Example 20. Syntheses of (S)-4-(1-(4-fluorophenyl)ethyl)-]-(2-(pyrimidin-4-yl)nicotinoyl)piperidine-4-carbonitrile (A44) and (R)-4-(1-(4-fluorophenyl)ethyl)-1-(2-(pyrimidin-4-yl)nicotinoyl)piperidine-4-carbonitrile (A45)*

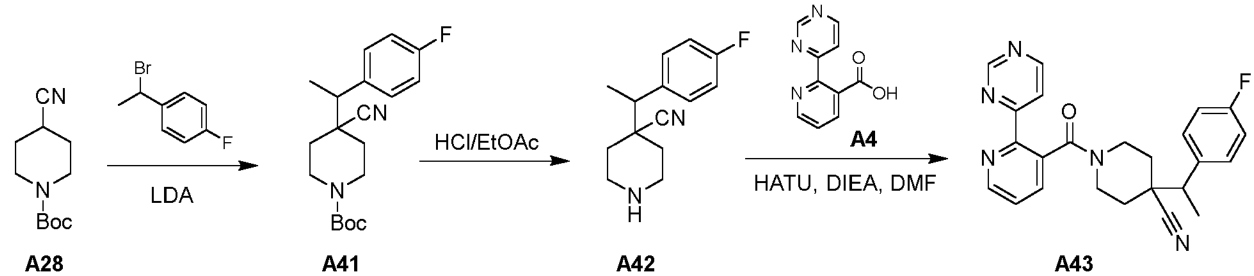


A28 A41 A42 A43

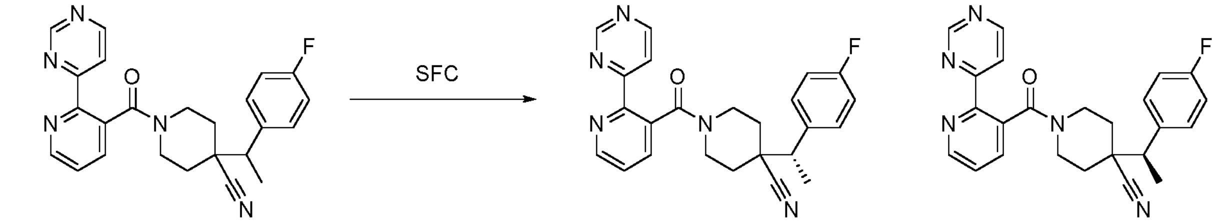


A43 A44 A45

Synthesis of A41

To a solution of DIPA (574 mg, 5.68 mmol) in THF (10 mL) was added n-BuLi (2.27 mL, 2.5 M in hexane,5.68 mmol) at -70 °C and stirred at 0 °C for 30 min. A solution of tert-butyl 4-cyanopiperidine-1-carboxylate (500 mg, 2.37 mmol) in THF (5 mL) was added and stirred at 0 °C for 0.5 h followed by addition of 1-(1-bromoethyl)-4-fluorobenzene (576 mg, 2.84 mmol) and the mixture was stirred at 75 °C for 5 h, poured into water (10 mL), and extracted with EtOAc (3 x 10 mL). The combined organic extracts were washed with brine (2 x 10 mL), dried over $Na_2SO_4$ and concentrated. The residue was purified by flash chromatography using a gradient elution (0~30% of EtOAc in PE) to give tert-butyl 4-cyano-4-(1-(4-fluorophenyl)ethyl)piperidine-1-carboxylate (250 mg, 31.7% yield) as a solid. $^1H$ NMR (400 MHz, $CDCl_3$) $\delta_H$ ppm 7.26 (m, 2 H), 7.03 (m, 2 H), 3.34-3.92 (m, 2 H), 3.12-2.78 (m, 2 H), 2.66 (m, 1 H), 2.12 (m, 1 H), 1.49-1.47 (m, 3 H), 1.44 (s, 9 H), 1.46 (m, 1 H), 1.39 (m, 1 H), 1.37-1.27 (m, 1 H).

Synthesis of A42

To a solution of tert-butyl 4-cyano-4-(1-(4-fluorophenyl)ethyl)piperidine-1-carboxylate (250 mg, 0.752 mmol) in EtOAc (10 mL) was added 10 mL of 4 M HCl in EtOAc. The mixture was stirred at 25 °C for 16 h and concentrated under reduced pressure to give 4-(1-(4-fluorophenyl)ethyl)piperidine-4-carbonitrile (200 mg) as a solid. $^1H$ NMR (400 MHz, methanol-d4) $\delta_H$ ppm 7.45-7.36 (m, 2 H), 7.11 (m, 2 H), 3.54 (m, 1 H), 3.42 (m, 1 H), 3.24-3.13 (m, 1 H), 3.06 (m, 1 H), 2.96 (q, 1 H), 2.44 ( dd, 1 H), 1.94-1.82 (m, 1 H), 1.78 (m, 1 H), 1.74-1.65 (m, 1 H), 1.51 (d, 3 H).

Synthesis of A43

To a solution of 2-(pyrimidin-4-yl)nicotinic acid (100 mg, 0.497 mmol) in DMF (5 mL) at 15 °C was added HATU (226 mg, 0.596 mmol) and DIEA (192 mg, 1.49 mmol) and stirred at 15 °C for 10 min. A solution of 4-(1-(4-fluorophenyl) ethyl)piperidine-4-carbonitrile (160 mg, 0.596 mmol) in DMF (3 mL) was added and the reaction was stirred at 15 °C for 2 h, treated with water (30 mL) and extracted with EtOAc (3 x 30 mL). The combined organic extracts were washed with brine (30 mL), dried over anhydrous $Na_2SO_4$ and concentrated under reduced pressure to give the product (200 mg) as a solid.

Synthesis of A44 and A45

Racemic 4-(1-(4-fluorophenyl)ethyl)-1-(2-(pyrimidin-4-yl)nicotinoyl)piperidine-4-carbonitrile (200 mg,) was purified by SFC chromatography using a DAICEL CHIRALCEL OD-H (250 mm*30 mm,5 μm column and eluting with 25:75 0.1% $NH_3H_2O$ : EtOH; flow rate 60 mL/min to give (Peak 1, Rt = 2.82 min, 71.3 mg) as a solid and (Peak 2, Rt = 3.13 min, 74.5 mg) as a solid. The products (54.5 mg, 0.1311 mmol) were diluted with EtOAc (30 mL), washed with saturated $Na_2CO_3$ (30 mL), dried over anhydrous $Na_2SO_4$ and evaporated under reduced pressure to give the enantiomerically pure compounds as solids. (*S*)-4-(1-(4-Fluorophenyl)ethyl)-1-(2-(pyrimidin-4-yl)nicotinoyl)piperidine-4-carbonitrile $^1H$ NMR (400 MHz, $CDCl_3$) $\delta_H$ 9.21 (s, 0.3 H), 8.94 (s, 0.3 H), 8.92-8.83 (m, 1.4 H), 8.74 (m, 1 H), 8.30-8.19 (m, 1 H), 7.69-7.61 (m, 1 H), 7.49-7.40 (m, 1 H), 7.26 (m, 1.4 H), 7.19 (m, 0.6 H), 7.12-7.03 (m, 1 H), 6.99 (t, 1 H), 5.00-4.64 (m, 1 H), 3.74-3.39 (m, 1 H), 3.38-2.91 (m, 2 H), 2.77-2.66 (m, 1 H), 2.43-2.23 (m, 0.5 H), 2.10-1.86 (m, 0.5 H), 1.85-1.57 (m, 2 H), 1.56-1.42 (m, 3 H),

1.38-1.19 (m, 1 H). $^{19}$F NMR (376.5 MHz, $CDCl_3$) $\delta_F$ -114.485. LC-ELSD/MS purity 99%, MS ESI calcd. for C24H22FN5O [M+H]+ 416.2 found 416.3. analytic SFC 100% de.

(*R*)-4-(1-(4-fluorophenyl)ethyl)-1-(2-(pyrimidin-4-yl)nicotinoyl)piperidine-4-carbonitrile $^1$H NMR (400 MHz, $CDCl_3$) $\delta_H$ 9.21 ( s, 0.3 H), 8.95 (s, 0.3 H), 8.92-8.84 (m, 1.4 H), 8.75 (m, 1 H), 8.24 (m, 1 H), 7.65 (m, 1 H), 7.50-7.41 (m, 1 H), 7.32-7.26 (m, 1.4 H), 7.22-7.15 (m, 0.6 H), 7.12-7.03 (m, 1 H), 6.99 (m, 1 H), 4.82 (m, 1 H), 4.99 -4.63 (m, 1 H), 3.71 -3.39 (m, 1 H), 3.37-2.93 (m, 2 H), 2.81-2.62 (m, 1 H), 2.44-2.19 (m, 0.5 H), 2.11-1.85 (m, 0.5 H), 1.84-1.72 (m, 0.7 H), 1.71-1.67 (m, 0.3 H), 1.60-1.55 (m, 1 H), 1.54-1.43 (m, 3 H), 1.41 -1.22 (m, 1 H). $^{19}$F NMR (376.5 MHz, $CDCl_3$) $\delta_F$ -114.485. LC-ELSD/MS purity 99%, MS ESI calcd. for C24H22FN5O [M+H]+ 416.2 found 416.1. analytic SFC 100%de.

*Example 21. Synthesis of 1-(2-(pyrimidin-4-yl)nicotinoyl)-4-((tetrahydro-2H-pyran-4-yl)methyl)piperidine-4-carbonitrile (A48)*

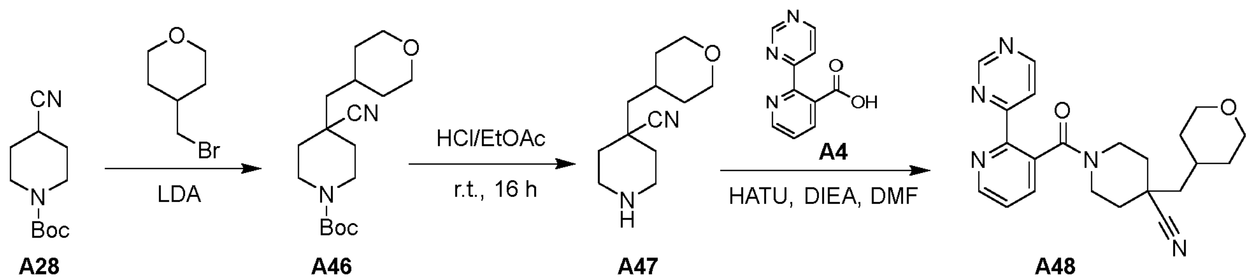


A28 A46 A47 A48

Synthesis of A46

To a solution of DIPA (574 mg, 5.68 mmol) in THF (10 mL) was added n-BuLi (2.27 mL, 2.5 M in hexane, 5.68 mmol) at -70 °C. The mixture was warmed to 0 °C and stirred for 30 min. To the LDA solution (2.84 mmol) was added tert-butyl 4-cyanopiperidine-1-carboxylate (500 mg, 2.37 mmol) in THF (5 mL) at 0 °C over 0.5 h. Then 4-(bromomethyl) tetrahydro-2H-pyran (508 mg, 2.84 mmol) was added and the mixture was stirred at 75 °C for 5 h, poured into water (10 mL), and extracted with EtOAc (3 x 10 mL). The combined organic extracts were washed with brine (2 x 10 mL), dried over $Na_2SO_4$ and concentrated. The residue was purified by flash chromatography using a gradient elution (0~30% of EtOAc in PE) to give tert-butyl 4-cyano-4-((tetrahydro-2H-pyran-4-yl)methyl)piperidine-1-carboxylate (300 mg, 41%) as a solid. $^1$H NMR (400 MHz,$CDCl_3$) $\delta_H$ 4.28-4.00 (m, 2H), 3.94 (dd, 2H), 3.40 (td, 2H), 3.04 (s, 2H), 1.94 (d, 2H), 1.88-1.78 (m, 1H), 1.74 (d, 2H), 1.53 (d, 2 H), 1.45 (s, 9 H), 1.44-1.36 (m, 4 H).

Synthesis of A47

To a solution of tert-butyl 4-cyano-4-((tetrahydro-2H-pyran-4-yl)methyl)piperidine-1-carboxylate (300 mg, 0.973 mmol) in EtOAc (10 mL) was added 10 mL of 4 M HCl in EtOAc and the mixture was stirred at 25 °C for 16 h. The reaction mixture was concentrated under reduced pressure to give 4-((tetrahydro-2H-pyran-4-yl)methyl)piperidine-4-carbonitrile (218 mg) as a solid. $^1$H NMR (400 MHz, MeOD-*d₄*) $\delta_H$ 3.92 ( dd, 2H), 3.52-3.40 (m, 4H), 3.20 (td, 2H), 2.27 ( d, 2H), 1.96-1.87 (m, 1H), 1.87-1.74(m, 4H), 1.66 (d, 2H), 1.34-1.34 (m, 2H).

Synthesis of A48

To a solution of 2-(pyrimidin-4-yl)nicotinic acid (150 mg, 0.7455 mmol) in DMF (5 mL) at 15 °C was added HATU (339 mg, 0.895 mmol) and DIEA (287 mg, 2.23 mmol) and stirred for 10 min. A solution of 4-((tetrahydro-2H-pyran-4-yl) methyl)piperidine-4-carbonitrile (218 mg, 0.8946 mmol) in DMF (3 mL) was added and the reaction was stirred at 15 °C for 2 h, treated with water (30 mL) and extracted with EtOAc (3 x 30 mL). The combined organic extracts were washed with brine (30 mL), dried over anhydrous $Na_2SO_4$ and concentrated. The residue was purified by HPLC (Column: Phenomenex Gemini-NX 150*30mm*5um; Condition: water (0.04% $NH_3H_2O$+10mM $NH_4HCO_3$)-ACN; Begin B: 17%; End B :47% ) to give 1-(2-(pyrimidin-4-yl)nicotinoyl)-4-((tetrahydro-2H-pyran-4-yl)methyl)piperidine-4-carbonitrile (46.0 mg,15.8% yield) as a solid. $^1$H NMR (400 MHz,$CDCl_3$) $\delta_H$ 9.23 (s, 0.4H), 9.10 (s, 0.6H), 8.89 (d, 1H), 8.76 (dd, 1H), 8.32-8.17 (m, 1H), 7.67 ( d,1H), 7.52-7.42 (m, 1H), 4.87 ( d, 0.6H), 4.75 ( d, 0.4H), 4.02-3.89 (m, 2H), 3.66-3.55 (m, 0.5H), 3.51-3.35 (m, 3H), 3.34-3.23 (m, 0.5H), 3.21-3.10 (m, 1H), 2.19 (d, 0.4H), 2.12 (d, 0.6H), 1.92-1.61 (m, 5H), 1.60-1.30 (m, 5H). LC-MS purity 99%, MS ESI calcd. for $C_{22}H_{26}N_5O_2$ [M+H]+ 392.2 found 392.3.

*Example 22. Synthesis of 4-(2,4-difluorobenzyl)-1-(2-(pyrimidin-4-yl)nicotinoyl)piperidine-4-carbonitrile (A51)*

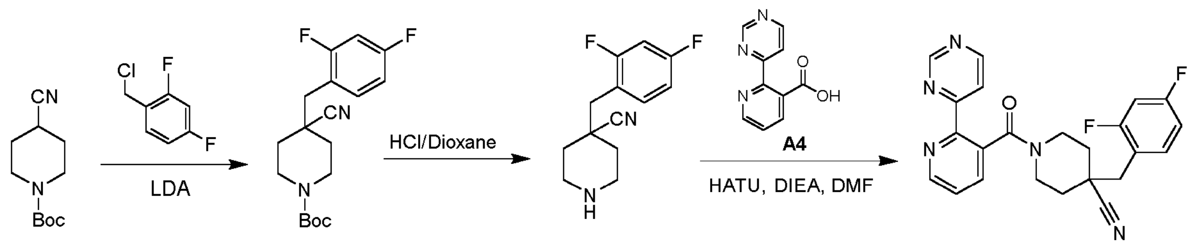


A28 A49 A50 A51

Synthesis of A49

To a cold (0 °C) LDA solution (11.4 mmol) was added tert-butyl 4-cyanopiperidine-1-carboxylate (2 g, 9.51 mmol) in THF (5 mL) at 0 °C over 0.5 h. 1-(Bromomethyl)-2,4-difluorobenzene (2.36 g, 11.4 mmol) was added and the mixture was stirred at 25 °C for 16 h, poured into water (10 mL) and extracted with EtOAc (3 x 10 mL). The combined organic extracts were washed with brine (2 x 10 mL), dried over $Na_2SO_4$ and concentrated. The residue was purified by flash column using a gradient elution (0~30% EtOAc in PE) to give tert-butyl 4-cyano-4-(2,4-difluorobenzyl)piperidine-1-carboxylate (2 g, 62.6%) as a solid. $^1H$ NMR (400 MHz, $CDCl_3$) $\delta_H$ 7.29 (dt, 1H), 6.87-6.73 (m, 2H), 4.21-3.97 (m, 2H), 3.0-2.85 (m, 2H), 2.82 (s, 2H), 1.84-1.71 (m, 2H), 1.39 (s, 9H).

Synthesis of A50

To a solution of tert-butyl 4-cyano-4-(2,4-difluorobenzyl)piperidine-1-carboxylate (2 g) in dioxane (15 mL) was added HCl/dioxane (20 mL), stirred at 25 °C for 16 h and concentrated to give the 4-(2,4-difluorobenzyl)piperidine-4-carbonitrile (1.7 g). $^1H$ NMR (400 MHz, $CDCl_3$) $\delta_H$ 9.5-9.0 (m, 2H), 7.53-7.43 (m, 1H), 7.31 (dt, 1H), 7.19-7.10 (m, 1H), 3.35 (s, 2H), 3.01 (s, 2H), 2.92-2.80 (m, 2H), 2.10-2.00 (m, 2H), 1.98-1.87 (m, 2H).

Synthesis of A51

To a solution of 2-(pyrimidin-4-yl)nicotinic acid (221 mg) in DMF (5 mL) was added HATU (627 mg, 1.65 mmol) and DIPEA (0.575 mL) at 20 °C, stirred for 30 min, and 4-(2,4-difluorobenzyl)piperidine-4-carbonitrile was added. The mixture was stirred for 1 h at 20 °C, treated with water (30 mL) and extracted with EtOAc (3 x 30 mL). The combined organic extracts were washed with brine (30 mL), dried over anhydrous $Na_2SO_4$ and concentrated. The residue was purified by prep-HPLC (Column (Phenomenex Gemini-NX 150*30mm*5um), Condition: water (0.04%$NH_3H_2O$+10mM $NH_4HCO_3$)-CAN, Begin B: 30%, End B:60%, Flow Rate 30 mL/min) to afford 4-(2,4-difluorobenzyl)-1-(2-(pyrimidin-4-yl)nicotinoyl)piperidine-4-carbonitrile (146.3 mg, 31.6%) as a solid. $^1H$ NMR (400MHz, $CDCl_3$) $\delta_H$ 9.24-8.94 (m, 1H), 8.92-8.85 (m, 1H), 8.76 (dd, 1H), 8.30-8.22 (m, 1H), 7.72-7.63 (m, 1H), 7.47 (dd, 1H), 7.41-7.37 (m, 1H), 6.97-6.80 (m, 2H), 5.00-4.72 (m, 1H), 3.68-3.20 (m, 2H), 3.11 (t, 1H), 2.99-2.91 (m, 2H), 2.15-1.98 (m, 1H), 1.97-1.61 (m, 3H). LC-MS purity 99%, MS for $C_{23}H_{19}F_2N_5O$ [M+H]+ 420.2 found 420.2.

*Example 23. Synthesis of 4-((3, 3-difluorocyclobutyl)methyl)-1-(2-(pyrimidin-4-yl)nicotinoyl)piperidine-4-carbonitrile (A54)*

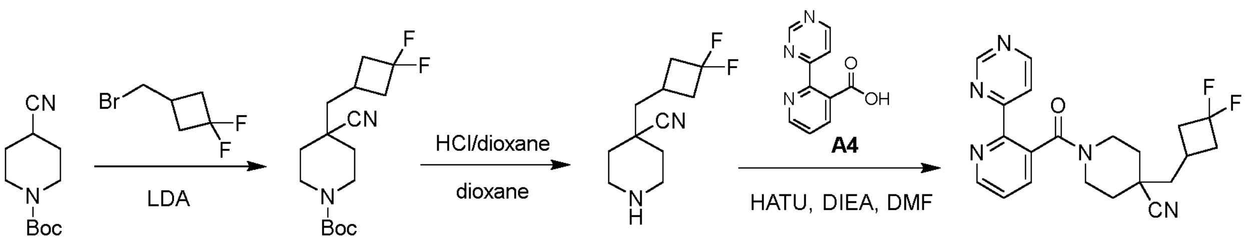


A28 A52 A53 A54

Synthesis of A52

To a solution of DIPA (1.72 g, 17.09 mmol) in THF (17 mL) was added BuLi (6.8 mL, 2.5 M in hexane, 17 mmol) at -70 °C. The mixture was warmed to 0 °C and stirred for 30 min to give an LDA solution. To the cold (0 °C) LDA solution was added tert-butyl 4-cyanopiperidine-1-carboxylate (1 g, 4.75 mmol) in THF (5 mL) over 0.5 h. 3-(Bromomethyl)-1,1-difluorocyclobutane (1.05 g, 5.69 mmol) was added and the mixture was stirred at 25 °C for 16 h, quenched with sat. $NH_4Cl$ (20 mL) and extracted with EtOAc (2 x 20 mL). The combined organic extracts were washed with brine (5 mL), dried over $Na_2SO_4$, concentrated and purified by flash chromatography using a gradient elution (0~30% EtOAc in PE) to give tert-butyl 4-cyano-4-((3,3-difluorocyclobutyl)methyl)piperidine-1-carboxylate (900 mg, 60.8% yield) as a solid. $^1H$ NMR (400 MHz, $CDCl_3$) $\delta_H$ = 4.27-4.00 (m, 2H), 3.13-2.92 (m, 2H), 2.89-2.74 (m, 2H), 2.47-2.22 (m, 3H), 1.88 (d, 2H), 1.81 (d, 2H), 1.46 (s, 9H), 1.44-1.36 (m, 2H). $^{19}F$ NMR (376.5 MHz, $CDCl_3$) $\delta_F$ -81.612, -82.127, -97.509, -98.025.

Synthesis of A53

To a solution of tert-butyl 4-cyano-4-((3,3-difluorocyclobutyl)methyl)piperidine-1-carboxylate (900 mg, 2.86 mmol) in dioxane (3 mL) was added dioxane/HCl (10 mL) at 25 °C and stirred for 16 h. The reaction was concentrated under reduced pressure to give 4-((3,3-difluorocyclobutyl)methyl)piperidine-4-carbonitrile (620 mg, 86.4% yield) and used directly in next step. $^1H$ NMR (400 MHz, DMSO-$d_6$) $\delta_H$ = 8.96 (br s, 2H), 2.97-2.94 (m, 2H), 2.81-2.69 (m, 2H), 2.55-2.51 (m, 2H), 2.44-2.26 (m, 3H), 2.10 (d, 2H), 1.90 (d, 2H), 1.83-1.69 (m, 2H). $^{19}F$ NMR (376.5 MHz, DMSO-$d_6$) $\delta_F$ -79.392, -79.898, -96.561, -97.067.

Synthesis of A54

To a solution of 2-(pyrimidin-4-yl)nicotinic acid (250 mg, 1.24 mmol) and HATU (707 mg, 1.86 mmol) in DMF (6 mL) was added DIPEA (0.65 mL, 3.72 mmol) at 25 °C and stirred for 30 min. 4-((3,3-Difluorocyclobutyl)methyl) piperidine-4-carbonitrile (310 mg, 1.24 mmol) was added and the mixture was stirred at 25°C for 1 h, quenched with water (30 mL) and extracted with EtOAc (3 x 30 mL). The combined organic extracts were washed with brine (30 mL), dried over anhydrous $Na_2SO_4$ and concentrated. The residue was purified by prep-HPLC (Column: XBridge Shield RP18 2.1×50mm×5μm, condition: water (0.04% $NH_3H_2O$+10 mM $NH_4HCO_3$)-ACN; Begin B: 32%; End B: 52%; Gradient Time: 8 min ) to give the product (87.6 mg, 17.8%) as a solid. The solid was washed by water (5 mL x 2), filtered and the filter cake was triturated from *i*-$Pr_2O$ (2 mL) to give 4-((3,3-difluorocyclobutyl)methyl)-1-(2-(pyrimidin-4-yl)nicotinoyl)piperidine-4-carbonitrile (19.2 mg, 22.2% yield) as a solid. $^1H$ NMR (400 MHz, $CDCl_3$) $\delta_H$ 9.32-9.06 (m, 1H), 8.94-8.86 (m, 1H), 8.77 (dd, 1H), 8.33-8.22 (m, 1H), 7.68 (d, 1H), 7.52-7.42 (m, 1H), 4.96-4.71 (m, 1H), 3.68-3.06 (m, 3H), 2.91-2.74 (m, 2H), 2.48-2.24 (m, 3H), 2.18-2.03 (m, 1H), 1.92-1.61 (m, 4.6H), 1.48-1.39 (m, 0.4H). $^{19}F$ NMR (400 MHz, $CDCl_3$) $\delta_{F1}$ -81.575, -81.731, -82.090, - 82.256, -97.270, -97.654, -97.786, -98.172. LC-MS purity 98%, MS ESI calcd. for $C_{21}H_{22}F_2N_5O$ [M+H]+ 398.4 found 398.4.

*Example 24. Synthesis of 4-(4-fluorobenzyl)-1-(2-(oxazol-5-yl)nicotinoyl)piperidine-4-carbonitrile (A56)*

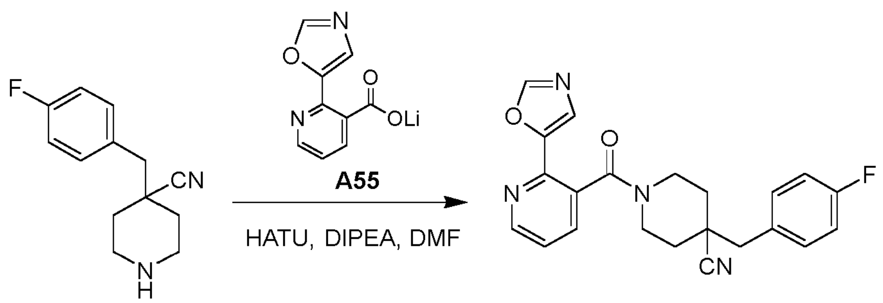


A5 A56

4-[(4-Fluorophenyl)methyl]piperidine-4-carbonitrile hydrochloride (0.39 mmol), lithium 2-(oxazol-5-yl)nicotinate (0.47 mmol), and HATU (0.51 mmol) were dissolved in DMF (4 ml) under nitrogen and DIPEA (3.13 mmol) was added and the mixture was stirred at rt for 2 h. The mixture was taken up in EtOAc and washed with saturated $NH_4Cl$, saturated $NaHCO_3$ and brine, dried over $MgSO_4$ and concentrated under reduced pressure. The residue was purified by silica gel chromatography using a gradient elution (2 to 10% MeOH/DCM) to give a solid. The solid was taken up in 30% AcN/water,

frozen and lyophilized to give a solid (61 mg, 40% yield). LCMS (ESI): $(M+H)^+$ calcd. 391.2, found 391.2. $^1H$ NMR, (400 MHz, $CD_3OD$): mixture of rotamers: 9.50 (t; 1 H); 9.38 (d; 1 H); 8.65 (t; 1 H); 8.47 (d; 1 H); 8.26-8.31 (m; 1 H); 8.11 (m; 2 H); 7.98 (t; 2 H); 5.43 (t; 1 H); 4.16 (t;1 H); 3.94 (t; 0.5 H); 3.71 (m; 4 H); 2.74 (t; 1 H); 2.50-2.57 (m; 0.5 H); 2.45 (d; 2 H); 1.84 (t; 0.5 H).

*Example 25. Synthesis of 4-(cyclopropylmethyl)-1-(2-(pyrimidin-4-yl)nicotinoyl)piperidine-4-carbonitrile (A58)*

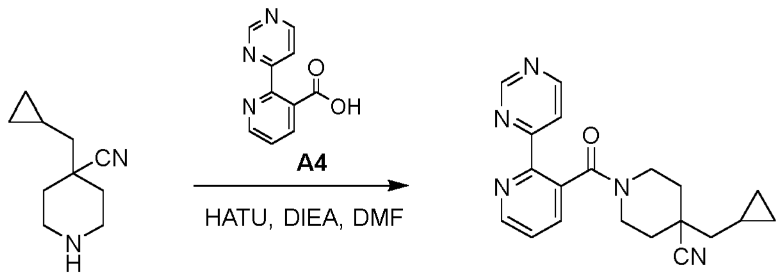


**A57** **A58**

To a solution of 4-(cyclopropylmethyl)piperidine-4-carbonitrile (300 mg, 1.82 mmol) in DMF (5 mL) at 25 °C were added HATU (828 mg, 2.18 mmol) and DIEA (703 mg, 5.45 mmol). After stirring at 25 °C for 10 min a solution of 2-(pyrimidin-4-yl)nicotinic acid (366 mg, 1.82 mmol) in DMF (3 mL) was added and the reaction stirred at 25 °C for 12 h, then was quenched with water (30 mL) and extracted with EtOAc (3 x 30 mL). The combined organic extracts were washed with brine (30 mL), dried over anhydrous $Na_2SO_4$, filtered and concentrated under reduced pressure to give the product (200 mg) as an oil, which was purified by HPLC (Column: YMC Triart C18 150*25mm*5um; elution condition: water (10 mM $NH_4HCO_3$)-ACN; Begin B:43%; End B :53%) to afford 4-(cyclopropylmethyl)-1-(2-(pyrimidin-4-yl)nicotinoyl)piperidine-4-carbonitrile (94.4 mg, 47.4% yield) as a solid. $^1H$ NMR (400 MHz, $CDCl_3$) $\delta_H$ 9.25-9.24 (s, 0.4H), 9.08-9.05 (s, 0.6H), 8.88 (d, 1 H), 8.75 (dd, 1H), 8.30-8.20 (m, 1H), 7.73-7.65 (m, 1H), 7.50-7.42 (m, 1H), 4.95-4.85 (m, 0.6H), 4.85-4.70 (m, 0.4H), 3.64-3.55 (m, 0.6H), 3.50-3.37 (m, 1H), 3.35-3.25 (m, 0.4H), 3.20-3.10 (m, 1H), 2.30-2.15 (m, 1H), 2.03-1.95 (m, 0.4H), 1.83-1.68 (m, 2H), 1.65-1.50 (m, 2.6H), 0.95-0.85 (m, 1H), 0.65-0.55 (m, 2H), 0.25-0.12 (m, 2H). LC-MS purity 99%, MS for $C_{20}H_{22}N_5O$ [M+H]+ 348.2 found 348.1.

*Example 26. Synthesis of 4-(4-methoxybenzyl)-]-(2-(pyrimidin-4-yl)nicotinoyl)piperidine-4-carbonitrile (A63)*

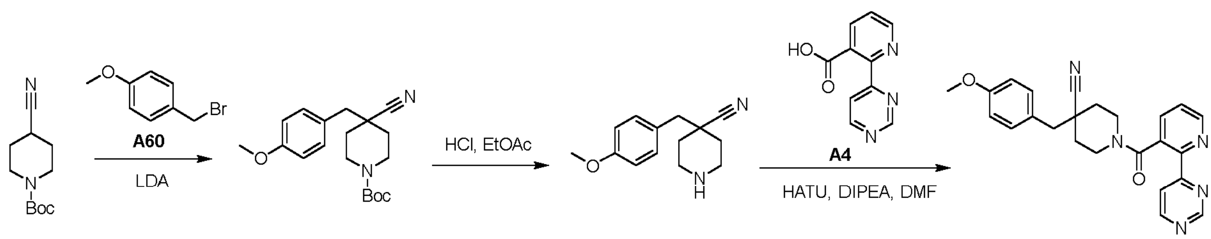


**A28** **A61** **A62** **A63**

Synthesis of A61

To a solution of DIPA (2.87 g, 28.4 mmol) in THF (20 mL) was added n-BuLi (11.3 mL, 2.5 M in hexane, 28.4 mmol) at -70°C. The mixture was warmed to 0°C and stirred at 0°C for 30 min to give a LDA solution. To the LDA (28.4 mmol) solution was added tert-butyl 4-cyanopiperidine-1-carboxylate (5 g, 23.7 mmol) in THF (10 mL) at 0°C, and the mixture was stirred for 0.5 h. Then 1-(chloromethyl)-4-methoxybenzene (3.71 g, 23.7 mmol) was added and the mixture was stirred at 75 °C for 5 h, cooled to r.t., poured into water (50 mL) and extracted with EtOAc (3 x 50 mL). The combined organic extracts were washed with brine (2 x 50 mL), dried over $Na_2SO_4$, filtered and concentrated in vacuo. The residue was purified by silica gel chromatography using a gradient elution (0-30% EtOAc in PE) to give tert-butyl 4-cyano-4-(4-methoxybenzyl) piperidine-1-carboxylate (7.5 g, 95.7%) as a solid. $^1H$ NMR (400 MHz, $CDCl_3$) $\delta_H$ 7.19 (d, 2H), 6.87 (d, 2H), 4.12 (m, 2H), 3.80 (s, 3H), 2.98 (s, 2H), 2.80 (s, 2H), 1.83 (d, 2H), 1.51-1.42 (m, 11H).

Synthesis of A62

A mixture of tert-butyl 4-cyano-4-(4-methoxybenzyl)piperidine-1-carboxylate (0.5 g, 1.59 mmol) and 4 M HCl in EtOAc (2 mL) was stirred at 25 °C for 16 h. The reaction mixture was concentrated under reduced pressure to give 4-(4-methoxybenzyl)piperidine-4-carbonitrile hydrogen chloride salt (0.3 g,) as a solid. The residue was used directly for the next step.

Synthesis of A63

To a solution of 2-(pyrimidin-4-yl)nicotinic acid (261 mg, 1.3 mmol) in DMF (5 mL) at 25 °C was added HATU (592 mg, 1.56 mmol) and DIEA (503 mg, 3.9 mmol). The mixture was stirred at 15 °C for 10 min and a solution of 4-(4-methoxybenzyl)piperidine-4-carbonitrile hydrogen chloride salt (300 mg, 1.3 mmol) in DMF (3 mL) was added. The reaction stirred at 25 °C for 12 h, treated with water (30 mL) and extracted with EtOAc (3 x 30 mL). The combined organic fractions were washed with brine (30 mL), dried over anhydrous $Na_2SO_4$, filtered and concentrated under reduced pressure to give 200 mg of an oil. Purification by HPLC (Column: YMC Triart C18 150*25mm*5μm; Condition: water (10mM $NH_4HCO_3$)-CAN; Begin B:42; End B :52) gave 4-(4-methoxybenzyl)-1-(2-(pyrimidin-4-yl)nicotinoyl)piperidine-4-carbonitrile (87 mg, 43.7%) as a solid. $^1H$ NMR (400 MHz, $CDCl_3$) $\delta_H$ 9.21 (s, 0.3H), 8.89 (d), 8.80 (s, 0.7H), 8.75 (d), 8.08-8.40 (m, 1H), 7.82-7.58 (m, 1H), 7.49-7.43 (m, 1H), 7.22-7.12 (m, 2H), 6.88 (d, 2H), 4.96-4.70 (m, 1H), 3.88-3.74 (m, 3H), 3.61 (d), 3.47-3.35 (m, 1H), 3.26-3.03 (m, 1.4H), 2.96-2.80 (m, 2H), 2.14-1.96 (m, 1H), 1.64 (d), 1.54-1.39 (m, 1H), 1.25 (s, 1H). LC-MS purity>97%, MS ESI calcd. For $C_{24}H_{24}NsO_2$ $[M+H]^+$ 414.1, found 414.1.

*Example 27. 1-([2,4'-Bipyridine]-3-carbonyl)-4-(pyridin-4-ylmethyl)piperidine-4-carbonitrile (A65)*

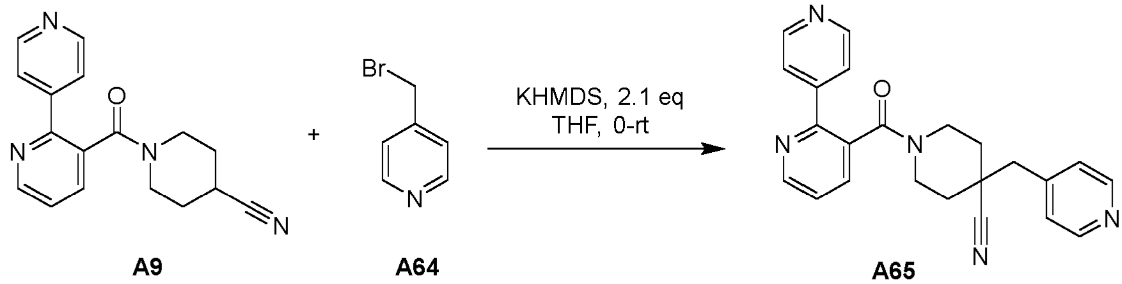


A9 A64 A65

1-([2,4'-Bipyridine]-3-carbonyl)piperidine-4-carbonitrile (0.34 mmol) was dissolved in THF (10 ml), cooled to 0 °C and potassium hexamethyldisilizane (0.71 mmol) was added. The mixture stirred for 1 h at 0 °C and 4-(bromomethyl) pyridine hydrobromide (0.32 mmol) was added and stirred at 0 °C for 1 h. Water and EtOAc were added and the organic layer washed with saturated $NaHCO_3$ and brine, dried over $MgSO_4$ and concentrated under reduced pressure. The residue was purified by silica gel chromatography using a gradient elution (2% to 10% MeOH/DCM). The residue was then further purified by reverse phase chromatography. The residue was taken up in 30% acetonitrile/water, frozen and lyophilized to give 1-([2,4'-bipyridine]-3-carbonyl)-4-(pyridin-4-ylmethyl)piperidine-4-carbonitrile as a solid (2 mg, 2% yield). LCMS (ESI): $(M+H)^+$ calcd. 384.2, found 384.2. $^1H$ NMR (400 MHz, $CD_3OD$): mixture of rotamers: 8.80 (dd; 1 H); 8.77 (s; 1 H); 8.67 (s; 1 H); 8.50 (d; 2 H); 7.94 (br s; 2 H); 7.72 (d; 2 H); 7.60 (s; 1 H); 7.34 (d; 2 H); 4.69 (m; 1 H); 3.39 (br s; 0.5 H); 3.12 (m; 1 H); 2.97 (br s; 1 H); 2.75 (m; 2 H); 2.63 (m; 0.5 H); 1.93 (d; 1 H); 1.64 (s; 1 H); 1.46 (d; 0.5 H); 1.31 (m; 0.5 H); 0.12 (m; 0.5 H).

*Example 28. Synthesis of 4-(4-fluorobenzyl)-1-(2-(pyridazin-4-yl)nicotinoyl)piperidine-4-carbonitrile (A200)*

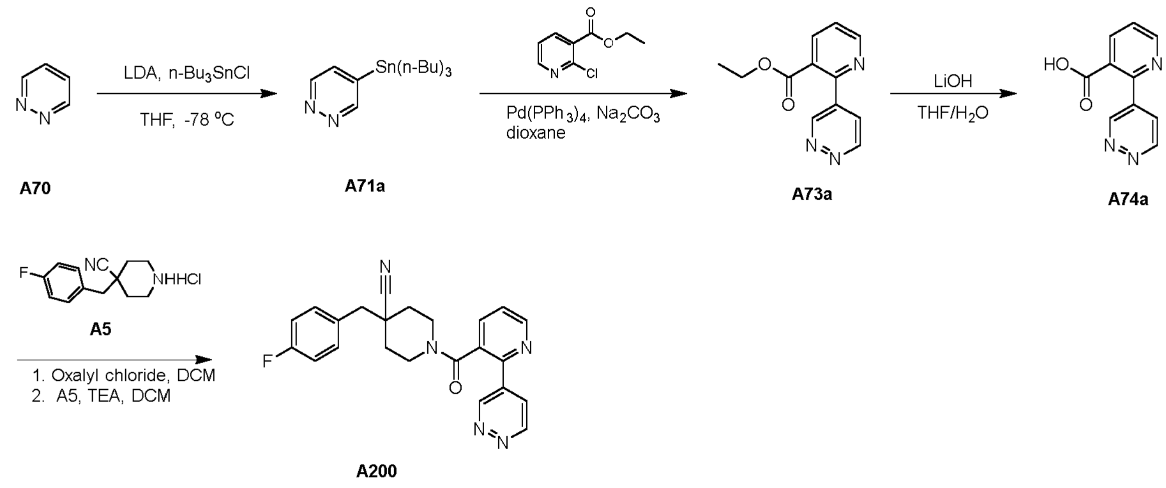

A70 A71a A73a A74a A5 A200

Synthesis of A71a

To a solution of pyridazine (5 g, 62.4 mmol) and n-$Bu_3$SnCl (22.3 g, 68.6 mmol) in THF (50 mL) was added LDA (31.2 mL, 62.4 mmol) at -70°C, and the mixture was stirred at -70°C for 2 hours. To the mixture was added sat. $NH_4Cl$ (20 mL), and the mixture was warmed to 15°C and stirred for 30 minutes. The mixture was diluted with water (50 mL) and extracted with EtOAc (2 x 100 mL). The combined organic layers were washed with brine (30 mL), dried over $Na_2SO_4$, filtered and concentrated to give the product, which was purified by flash chromatography (10%~30% of EtOAc in PE) to give 4-(tributylstannyl)pyridazine (5 g, 21.7%).

Synthesis of A73a

4-(Tributylstannyl)pyridazine (2.38 g, 6.44 mmol), Pd($PPh_3$)$_4$ (563 mg, 0.54 mmol) and ethyl 2-chloropyridine-3-carboxylate (1 g, 5.38 mmol) in DMF (10 mL) was stirred under $N_2$ at 120°C for 16 hours. After cooling to 25°C, the mixture was diluted with water (100 mL) and extracted with EtOAc (2 x 100 mL). The combined organic layers were washed with brine (100 mL), dried over $Na_2SO_4$, filtered and concentrated to give product, which was purified by flash chromatography (10~100% of EtOAc in PE) to give ethyl 2-(pyridazin-4-yl)nicotinate (550 mg, 44.7%). $^1$H NMR (400 MHz, $CDCl_3$) $\delta_H$ 9.40-9.17 (m, 2H), 8.86 (d, 1H), 8.33 (d, 1H), 7.72-7.59 (m, 1H), 7.51 (dd, 1H), 4.24 (q, 2H), 1.15 (t, 3H).

Synthesis of A74a

Ethyl 2-(pyridazin-4-yl)nicotinate (300 mg, 1.3 mmol) and LiOH·$H_2O$ (163 mg, 3.9 mmol) were combined in THF (2 mL) and water (0.5 mL). The mixture was stirred at 15°C for 2 hours. The mixture was concentrated and EtOH (10 mL) was added. The suspension was stirred at 15°C for 30 minutes. The mixture was filtered, and the filtrate was concentrated under reduced pressure to afford 2-(pyridazin-4-yl)nicotinic acid (200 mg), which was used directly in the next step.

Synthesis of A200

Step 1: To a solution of 2-(pyridazin-4-yl)nicotinic acid (100 mg, 0.50 mmol) and DMF (18.1 mg, 0.248 mmol) in DCM (5 mL) was added oxalic dichloride (127 mg, 1.0 mmol) at 0°C. After stirring at 20°C for 1 h, the resulting acyl chloride was concentrated and used directly for the next step.

Step 2: To the freshly prepared acyl chloride in DCM (5 mL) was added 4-[(4-fluorophenyl) methyl] piperidine-4-carbonitrile hydrochloride (126 mg, 0.50 mmol) and TEA (222 mg, 0.19 mL, 1.5 mmol) at 25°C. After stirring at 25°C for 16 hours, the reaction mixture was filtrated and concentrated to give the product (180 mg), which was purified by HPLC (Column: Welch Xtimate C18 150*25mm*5μm; Condition:water (0.225%FA)-ACN; Begin B:25; End B:55) to afford 80 mg. The residue was dissolved in EtOAc (10 mL), and water (10 mL) was added in one portion. The pH of the mixture was adjusted to 7~8 with aqueous $NaHCO_3$ (10 ml). The aqueous phase was extracted with EtOAc (3 x 20 mL). The combined organic layers were washed with brine (2 x 20 mL), dried over anhydrous $Na_2SO_4$, filtered and concentrated to give 4-(4-fluorobenzyl)-1-(2-(pyridazin-4-yl)nicotinoyl)piperidine-4-carbonitrile (29.2 mg, 14.6%). $^1$H NMR (400 MHz, $CDCl_3$) $\delta_H$ 9.63 (s, 1H), 9.36 (s, 1H), 8.85 (dd, 1H), 8.10-7.960 (m, 1H), 7.79 (dd, 1H), 7.51 (dd, 1H), 7.21-7.55 (m, 2H), 7.05-6.95 (m, 2H), 4.82 (d, 1H), 3.26-2.42 (m, 5H), 1.93 (s, 1H), 1.73-1.59 (m, 1H), 1.46-1.19 (m, 2H), 0.14-0.16 (m, 1H). LC-MS purity>=99%, MS ESI calcd. for $C_{23}H_{20}FN_5O$ $[M+H]^+$ 402.2, found 402.1.

*Example 29. Synthesis of 4-((6-methylpyridin-3-yl)methyl)-1-(2-(pyrimidin-4-yl)nicotinoyl)piperidine-4-carbonitrile (A201)*

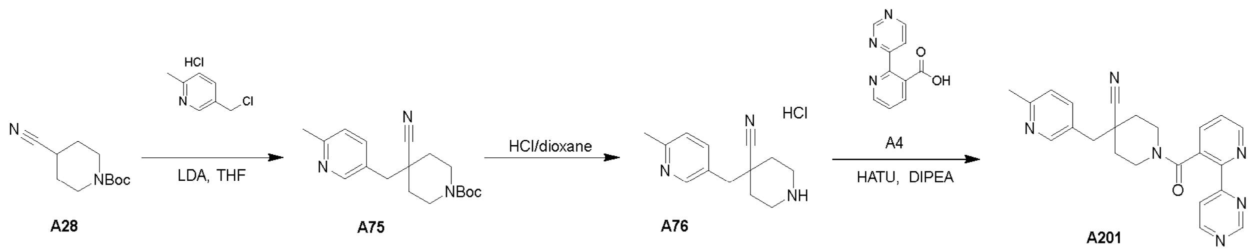


A28 A75 A76 A201

Synthesis of A75

To a mixture of tert-butyl 4-cyanopiperidine-1-carboxylate (706 mg, 3.36 mmol) in THF (10 mL) was added dropwise LDA (3.36 mL, 2 M) at -78oC. After stirring at -78oC for 1 hour, 5-(chloromethyl)-2-methylpyridine hydrochloride (300 mg, 1.68 mmol) was added at -78oC and then warmed to 20oC and stirred for 16 hours. Water (20 mL) was added, and mixture was extracted with EtOAc (2 x 10 mL). The combined organic layers were washed with brine (2 x 10 mL), dried, filtered and concentrated to give residue, which was purified by column (of 0-10% MeOH in DCM) to give tert-butyl 4-cyano-4-((6-methylpyridin-3-yl)methyl)piperidine-1-carboxylate (250 mg). 1H NMR (400 MHz, CDCl3) δH 8.36-8.33 (m, 1H), 8.70-8.55 (m, 1H), 7.18-7.13 (m, 1H), 3.10-2.91 (m, 1H), 2.83 (s, 2H), 2.55 (m, 3H), 1.88-1.80 (m, 2H), 1.70-1.47 (m, 3H), 1.48-1.44 (m, 11H). LC-ELSD/MS purity 99%, MS ESI calcd. for C18H26N3O2 [M+H]+ 316.0, found 316.0.

Synthesis of A76

The mixture of tert-butyl 4-cyano-4-((6-methylpyridin-3-yl)methyl)piperidine-1-carboxylate (300 mg, 0.951 mmol) in HCl/dioxane (5 mL) was stirred at 25°C for 1h. The reaction mixture was concentrated to give 4-((6-methylpyridin-3-yl)methyl)piperidine-4-carbonitrile hydrochloride (300 mg).

Synthesis of A201

To a solution of 2-(pyrimidin-4-yl)pyridine-3-carboxylic acid (49.9 mg, 0.248 mmol), 4-[(4-fluorophenyl)methyl] piperidine-4-carbonitrile hydrochloride (50 mg, 0.20 mmol) and HATU (141 mg, 0.372 mmol) in DMF (2 mL) was added DIPEA (159 mg, 1.24 mmol). The mixture was stirred at 20°C for 12 hours. The reaction mixture poured into water (10 mL), the aqueous layer was extracted with EtOAc (2 x 10 mL). The combined organic phase was washed with brine (20 mL), dried over anhydrous $Na_2SO_4$ and evaporated under reduced pressure to give 40 mg, which was purified by prep-HPLC (Column: YMC Triart C18 150*25mm*5um; Condition: water (10mM $NH_4HCO_3$)-ACN; Begin B: 22%; End 52%) to give 4-((6-methylpyridin-3-yl)methyl)-1-(2-(pyrimidin-4-yl)nicotinoyl)piperidine-4-carbonitrile (7.0 mg, 0.017 mmol, 8%). $^1H$ NMR (400 MHz, $CDCl_3$) $\delta_H$, 7.25-8.84(m, 2H), 8.79-8.73(m, 1H), 8.34(s, 1H), 8.30-8.22(m, 1H), 7.72-7.64(m, 1H), 7.64-7.50(m, 1H), 7.50-7.44(m, 1H), 7.20-7.15(m, 1H), 4.97-4.72(m, 1H), 3.67-3.00(m, 3H), 2.96-2.81(m, 2H), 2.60-2.51(m, 3H), 2.14-1.97(m, 1H), 1.87-1.44(m, 4H). LC-ELSD/MS purity 99%;; MS ESI calcd. for $C_{23}H_{23}N_6O$ [M +H]$^+$ 399.2, found 399.2

*Example 30. Synthesis of 4-((6-methoxypyridin-3-yl)methyl)-1-(2-(pyrimidin-4-yl)nicotinoyl)piperidine-4-carbonitrile (A202)*

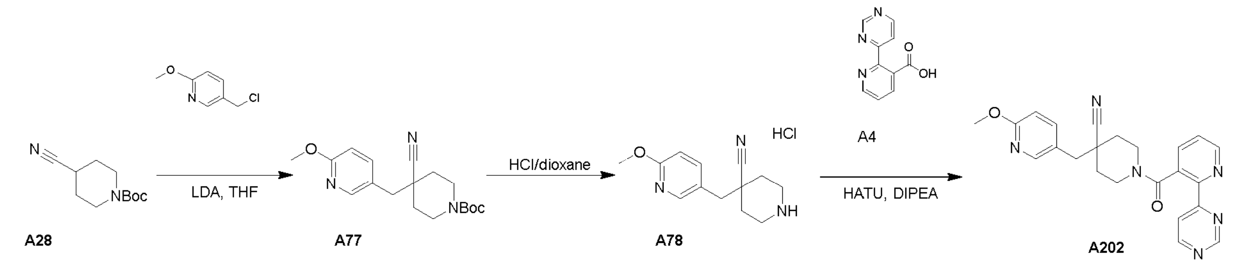


Synthesis of A77

To tert-butyl 4-cyanopiperidine-1-carboxylate (529 mg, 2.52 mmol) in THF (6 mL) was added dropwise LDA (2.52 mL, 2 M) at -78°C. After stirring at -78°C for 1 hour, 5-(chloromethyl)-2-methoxypyridine (200 mg, 1.26 mmol) was added at -78°C. The mixture was warmed to 20°C and stirred for 16 hours. The mixture was quenched with water (20 mL), extracted with EtOAc (2 x 10 mL). The combined organic layers were washed with sodium chloride solution (2 x 10 mL), dried, filtered and concentrated to give residue, which was purified by column chromatography (of 0~50% EtOAc in PE) to give tert-butyl 4-cyano-4-((6-methoxypyridin-3-yl)methyl)piperidine-1-carboxylate (100 mg). $^1$H NMR (400 MHz, $CDCl_3$) $\delta_H$, 8.05-7.99 (m, 1H), 7.59-7.53 (m, 1H), 6.77-6.72 (m, 1H), 4.25-4.03 (m, 2H), 3.93 (s, 3H), 3.06-2.90 (m, 2H), 2.79 (s, 2H), 1.89-1.80 (m, 2H), 1.49-1.43 (m, 11H). LC-ELSD/MS purity 99%, MS ESI calcd. for $C_{14}H_{17}N_3O_3$ $[M-C_4H_9+H]^+$ 275.9, found 275.9.

Synthesis A78

The mixture of tert-butyl 4-cyano-4-((6-methoxypyridin-3-yl)methyl)piperidine-1-carboxylate (100 mg, 0.302 mmol) in HCl/dioxane (5 mL) was stirred at 25°C for 1 hour. The mixture was concentrated to give 4-((6-methoxypyridin-3-yl)methyl)piperidine-4-carbonitrile hydrochloride (100 mg), which was used directly in the next reaction.

Synthesis A202

To a solution of 2-(pyrimidin-4-yl)pyridine-3-carboxylic acid (93.8 mg, 0.467 mmol) , 4-[(4-fluorophenyl)methyl] piperidine-4-carbonitrile hydrochloride (100 mg, 0.373 mmol) and HATU (266 mg, 0.700 mmol) in DMF (2 mL) was added DIPEA (300 mg, 2.33 mmol). The mixture was stirred at 20°C for 12 hours. The reaction mixture poured into water (10 mL), the aqueous layer was extracted with EtOAc (2 x 10 mL). The combined organic layers were washed with brine (10 mL), dried over anhydrous $Na_2SO_4$ and evaporated under reduced pressure to give 100 mg , which was purified by HPLC (Column: YMC Triart C18 150*25mm*5um, gradient: 28-58% and B: Condition: water (10mM $NH_4HCO_3$)-ACN; flow rate: 30 mL/min) to give 4-((6-methoxypyridin-3-yl)methyl)-1-(2-(pyrimidin-4-yl)nicotinoyl)piperidine-4-carbonitrile (20.8 mg, 20.8%). $^1$H NMR (400 MHz, $CDCl_3$) $\delta_H$ 9.24-8.93 (m, 1H), 8.93-8.84 (m, 1H), 8.79-8.74 (m, 1H), 8.81-8.23 (m, 1H), 8.01 (s, 1H), 7.73-7.64 (m, 1H), 7.60-7.42 (m, 2H), 6.78-6.73 (m, 1H), 4.99-4.72 (m, 1H), 3.94 (m, 3H), 3.67-3.56 (m, 1H), 3.47-3.35 (m, 1H), 3.29-3.03 (m, 2H), 2.93-2.79 (m, 2H), 2.16-1.99 (m, 1H), 1.86-1.75 (m, 1H), 1.54-1.43 (m, 1H). LC-ELSD/MS purity 99%;; MS ESI calcd. for $C_{23}H_{23}N_6O_2$ $[M+H]^+$ 415.2, found 415.2.

*Example 31. Synthesis of 4-((2-methylpyrimidin-5-yl)methyl)-1-(2-(pyrimidin-4-yl)nicotinoyl)piperidine-4-carbonitrile (A203)*

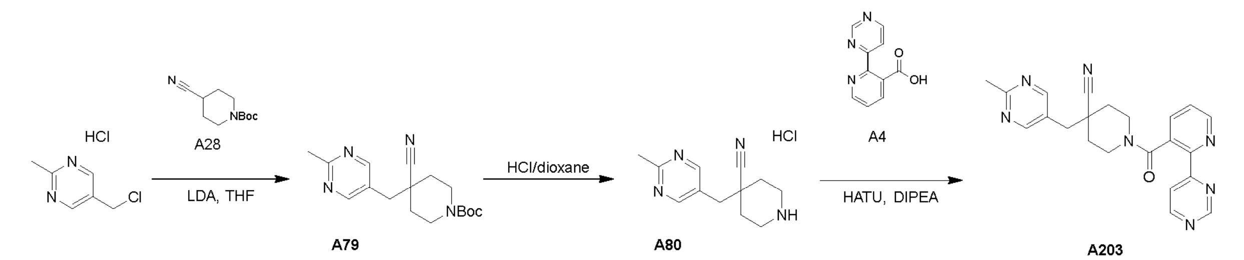


Synthesis A79

To a mixture of tert-butyl 4-cyanopiperidine-1-carboxylate (1.17 g, 5.60 mmol) in THF (10 mL) was added

dropwise LDA (5.60 mL, 2 M) at -78°C. After stirring at -78°C for 1 hour, 5-(chloromethyl)-2-methylpyridine hydrochloride (400 mg, 2.80 mmol) was added at - 78°C. The mixture was warmed to 20°C and stirred for 16 hours. The mixture was quenched with water (20 mL) and extracted with EtOAc (2 x 10 mL). The organic layers were washed with sodium chloride solution (2 x 10 mL), dried, filtered and concentrated to give the product, which was purified by column chromatography (0~50% EtOAc in PE) to give tert-butyl 4-cyano-4-((2-methylpyrimidin-5-yl)methyl)piperidine-1-carboxylate (150 mg). LC-ELSD/MS purity 99%; MS ESI calcd. for $C_{13}H_{16}N_4O_2$ $[M-C_4H_9+H]^+$ 260.9, found 260.9.

Synthesis A80

A mixture of tert-butyl 4-cyano-4-((2-methylpyrimidin-5-yl)methyl)piperidine-1-carboxylate (150 mg, 0.474 mmol) in HCl/dioxane (5 mL) was stirred at 25°C for 1 hour. The mixture was concentrated to give 4-((2-methylpyrimidin-5-yl)methyl)piperidine-4-carbonitrile hydrochloride (100 mg).

Synthesis A203

To a solution of 2-(pyrimidin-4-yl)pyridine-3-carboxylic acid (99.4 mg, 0.495 mmol), 4-[(4-fluorophenyl)methyl] piperidine-4-carbonitrile hydrochloride (100 mg, 0.396 mmol) and HATU (255 mg, 0.593 mmol) in DMF (2 mL) was added DIPEA (254 mg, 1.97 mmol). The mixture was stirred at 20°C for 12 hours. The reaction mixture poured into water(10 mL), and the aqueous layer was extracted with EtOAc (2 x 10 mL). The combined organic layers were washed with brine (10 mL), dried over anhydrous $Na_2SO_4$ and evaporated under reduced pressure to give 50 mg, which was purified by HPLC (column: Welch Xtimate C18 150*25mm*5um, gradient: 12-42%B; Condition: water (0.04%NH3H2O)-ACN; flow rate: 25 mL/min) to give 4-((2-methylpyrimidin-5-yl)methyl)-1-(2-(pyrimidin-4-yl)nicotinoyl)piperidine-4-carbonitrile (27.6 mg, 0.069 mmol, 17%). $^1H$ NMR (400 MHz, $CDCl_3$) $\delta_H$, 9.23-9.00(m, 1H), 8.93-8.85(m, 1H), 8.80-8.74(m, 1H), 8.61-8.54(m, 2H), 8.30-8.25(m, 1H), 7.71-7.64(m, 1H), 7.51-7.44(m, 1H), 4.98-4.77(m, 1H), 3.70-3.40(m, 2H), 3.40-3.04(m, 2H), 3.01-2.82(m, 2H), 2.82-2.70(m, 3H), 2.15-2.00(m, 1H), 1.90-1.70(m, 2H). LC-ELSD/MS purity 99%; MS ESI calcd. for $C_{22}H_{22}N_7O$ $[M +H]^+$ 400.2, found 400.2.

*Example 32. Synthesis of 4-(4-(methylsulfonyl)benzyl)-1-(2-(pyrimidin-4-yl)nicotinoyl)piperidine-4-carbonitrile (A204)*

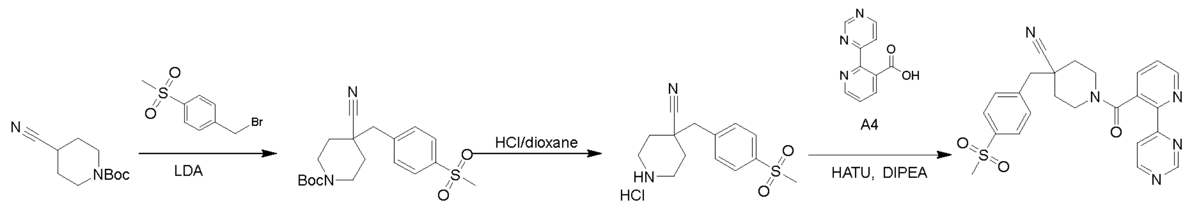


A28 A81 A82 A204

Synthesis A81

To a mixture of tert-butyl 4-cyanopiperidine-1-carboxylate (600 mg, 2.85 mmol) in THF (10 mL) was added drop wise LDA (2.13 mL, 4.27 mmol, 2 M in THF/n-heptane) at - 78°C. After stirring under nitrogen at -78°C for 1 hour, 1-(bromomethyl)-4-methanesulfonylbenzene (851 mg, 3.42 mmol) was added, and the reaction was warmed to 20°C and stirred for 16 hours. The reaction mixture was poured into saturated aqueous $NH_4Cl$ solution (100 mL) and extracted with ethyl acetate (2 x 100 mL). The combined organic layers were washed with brine (100 mL) and dried over $Na_2SO_4$. The mixture was filtered, concentrated and purified by flash chromatography (30~50% EtOAc in PE) to give tert-butyl 4-cyano-4-(4-(methylsulfonyl)benzyl)piperidine-1-carboxylate (500 mg, 46.7%). $^1H$ NMR (400 MHz, $CDCl_3$) $\delta_H$ 7.95 (d, 2H), 7.51 (d, 2H), 4.26-4.05 (m, 2H), 3.08 (s, 3H), 2.96 (s, 2H), 1.93-1.78 (m, 2H), 1.64-1.48 (m, 4H), 1.47 (s, 9H).

Synthesis A82

To a mixture of tert-butyl 4-cyano-4-(4-(methylsulfonyl)benzyl)piperidine-1-carboxylate (550 mg, 1.45 mmol) in dioxane (10 mL) was added HCl/dioxane (4 M, 3.60 mL, 14.4 mmol ). After stirring at 20°C for 16 hours, the reaction mixture was filtered and the filter cake was washed with 50 mL of EtOAc, and dried to give 4-(4-(methylsulfonyl)benzyl) piperidine-4-carbonitrile hydrochloride (400 mg, 99.2%). $^1H$ NMR (400 MHz, $CDCl_3$) $\delta_H$ 8.07-7.91 (m, 2H), 7.71-7.59 (m, 2H), 3.59-3.43 (m, 2H), 3.22-3.09 (m, 7H), 2.20-2.10 (m, 2H), 2.03-1.92 (m, 2H).

Synthesis A204

To a solution of 2-(pyrimidin-4-yl)pyridine-3-carboxylic acid (72.2 mg, 0.359 mmol), 4-(4-(methylsulfonyl)benzyl) piperidine-4-carbonitrile hydrochloride (100 mg, 0.359 mmol) and HATU (204 mg, 0.538 mmol) in DMF (5 mL) was added DIPEA (360 mg, 1.79 mmol). After stirring at 20°C for 3 hours, the reaction mixture poured into water (50 mL) and extracted with EtOAc (2 x 50 mL). The combined organic layers were washed with brine (100 mL), dried over anhydrous $Na_2SO_4$, and evaporated under reduced pressure to give the product, which was purified by HPLC (Welch Xtimate C18 150 x 25mm, 5um; Condition: water (0.225% FA)-ACN; Gradient: from 52% to 82% of B in 8.5 min and hold 100% for 2 min; Flow rate: 30 mL/min; Injections: 8) to afford 4-(4-(methylsulfonyl)benzyl)-1-(2-(pyrimidin-4-yl)nicotinoyl)piperidine-4-carbonitrile (69.7 mg, 42.2%). $^1$H NMR (400 MHz, $CDCl_3$) $\delta_H$ 9.25-9.19 (m, 1H), 8.92-8.87 (m, 1H), 8.87-8.83 (m, 1H), 8.80-8.75 (m, 1H), 8.32-8.24 (m, 1H), 7.96 (d, 2H), 7.73-7.65 (m, 1H), 7.53-7.46 (m, 3H), 5.00-4.88 (m, 1H), 4.86-4.76 (m, 1H), 3.70-3.59 (m, 1H), 3.49-3.39 (m, 1H), 3.30-3.20 (m, 1H), 3.19-3.10 (m, 3H), 3.09-3.01 (m, 3H), 2.16-2.01 (m, 1H), 1.96-1.74 (m, 2H), 1.72-1.63 (m, 1H). LC-ELSD/MS purity 98.19%, MS ESI calcd. for $C_{24}H_{23}N_5O_3S$ [M+H]$^+$ 462.2 found 462.3.

*Example 33. Synthesis of 4-(4-chloro-2-fluorobenzyl)-1-(2-(pyrimidin-4-yl)nicotinoyl)piperidine-4-carbonitrile (A205)*

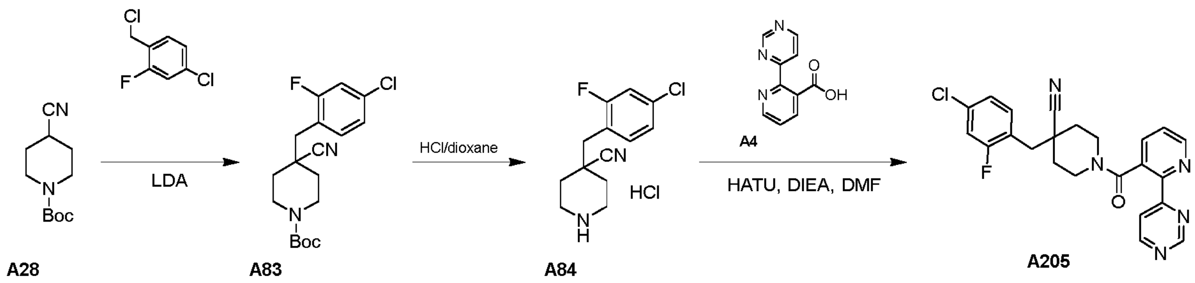


Synthesis of A83

To a mixture of tert-butyl 4-cyanopiperidine-1-carboxylate (1 g, 4.75 mmol) in THF (10 mL) was added dropwise LDA (3.56 mL, 7.12 mmol, 2 M in THF/n-hexane) at -78°C. After stirring under nitrogen at -78°C for 1 hour, 4-chloro-1-(chloromethyl)-2-fluorobenzene (1.01 g, 5.69 mmol) was added. The mixture was warmed to 20°C and stirred for 16 hours. The reaction mixture was poured into saturated $NH_4Cl$ solution (30 mL), and the aqueous layer was extracted with ethyl acetate (2 x 30 mL). The combined organic layers were washed with brine (20 mL) and dried over $Na_2SO_4$. The product was purified by silica gel chromatography (PE: EtOAc from 5:1 to 4:1) to give tert-butyl 4-(4-chloro-2-fluorobenzyl)-4-cyanopiperidine-1-carboxylate (1.3 g, 77.8 %). $^1$H NMR $\delta_H$ 7.36 - 7.29 (m, 1H), 7.19 - 7.08 (m, 2H), 4.25 - 4.06 (m, 2H), 3.08 - 2.92 (m, 2H), 2.89 (s, 2H), 1.90 - 1.78 (m, 2H), 1.58 - 1.49 (m, 2H), 1.45 (s, 9H).

Synthesis of A84

The mixture of tert-butyl 4-(4-chloro-2-fluorobenzyl)-4-cyanopiperidine-1-carboxylate (1.3 g, 3.68 mmol) in HCl/dioxane(4M, 10 mL) was stirred at 15°C for 16 hours and concentrated to afford 4-(4-chloro-2-fluorobenzyl) piperidine-4-carbonitrile hydrochloride (1.5 g). $^1$H NMR $\delta_H$ 7.34 - 7.27 (m, 1H), 7.21 - 7.12 (m, 2H), 3.61 - 3.52 (m, 2H), 3.25 - 3.09 (m, 2H), 2.97 (s, 2H), 2.30 - 2.00 (m, 4H).

Synthesis of A205

To a solution of 2-(pyrimidin-4-yl)nicotinic acid (100 mg, 0.497 mmol) in DMF (3 mL) was added HATU (283 mg, 0.7455 mmol) and DIPEA (0.259 mL, 1.49 mmol) at 25°C. After stirring at 25°C for 30 minutes, 4-(4-chloro-2-fluorobenzyl) piperidine-4-carbonitrile hydrochloride (125 mg, 0.4970 mmol) was added to the solution, and the mixture was stirred for 2 hours at 25°C. The reaction mixture poured into water (50 mL) and extracted with EtOAc (2 x 50 mL). The combined organic layers were washed with brine (100 mL), dried over anhydrous $Na_2SO_4$, and evaporated under reduced pressure to give the product, which was purified by prep-HPLC (Welch Xtimate C18 150*25mm*5um; Condition: water (water (0.04%$NH_3H_2O$)-ACN; Begin B: 33, End B: 63; Gradient Time (min):8.5; 100%B Hold Time (min):2) to afford the 4-(4-chloro-2-fluorobenzyl)-1-(2-(pyrimidin-4-yl)nicotinoyl)piperidine-4-carbonitrile (23.7 mg, 10.9%). $^1$H NMR $\delta_H$ 9.21 (s, 0.3H), 8.98 (s, 0.6H), 8.93 - 8.83 (m, 1H), 8.76 (dd, 1H), 8.36 - 8.18 (m, 1H), 7.75 - 7.60 (m, 1H), 7.50 - 7.43 (m, 1H), 7.37 - 7.29 (m, 1H), 7.19 - 7.10 (m, 2H), 4.95 - 4.85 (m, 0.6H), 4.83 - 4.72 (m, 0.4H), 3.68 - 3.58 (m, 0.6H), 3.48 - 3.34 (m, 1H), 3.22 -

3.07 (m, 2H), 3.01 - 2.89 (m, 3.4H), 2.16 - 1.80 (m, 3H). LC-ELSD/MS purity 100%, MS ESI calcd. For $C_{23}H_{19}ClFN_5O$ $[M+H]^+$ 436, found 436.

*Example 34. Synthesis of 4-(3, 4-difluorobenzyl)-1-(2-(pyrimidin-4-yl)nicotinoyl)piperidine-4-carbonitrile (A206)*

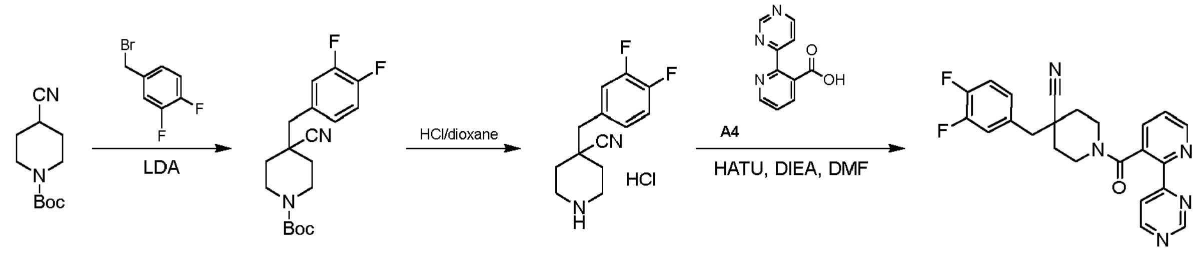


A28 A85 A86 A206

Synthesis of A85

To a mixture of tert-butyl 4-cyanopiperidine-1-carboxylate (1 g, 4.75 mmol) in THF (10 mL) was added LDA (3.56 mL, 7.12 mmol, 2 M in THF/n-heptane) dropwise at -78°C. After stirring under nitrogen at -78°C for 1 hour, 4-(bromomethyl)-1,2-difluorobenzene (1.17 g, 5.69 mmol) was added. The mixture was warmed to 20°C and stirred for 16 hours. The reaction mixture was poured into saturated $NH_4Cl$ solution (30 mL) and extracted with ethyl acetate (2 x 30 mL). The combined organic layers were washed with brine (20 mL), dried over $Na_2SO_4$, filtered and concentrated. The product was purified by silica gel chromatography (PE: EtOAc from 5:1 to 3:1) to give tert-butyl 4-cyano-4-(3,4-difluorobenzyl) piperidine-1-carboxylate (1.3 g, 82 %). $^1H$ NMR $\delta_H$ 7.20 - 7.07 (m, 2H), 7.03 - 6.98 (m, 1H), 4.28 - 4.11 (m, 2H), 3.11 - 2.89 (m, 2H), 2.81 (s, 2H), 1.92 - 1.75 (m, 2H), 1.52 - 1.43 (m, 11H).

Synthesis of A86

A mixture of tert-butyl 4-cyano-4-(3,4-difluorobenzyl)piperidine-1-carboxylate (1.3 g, 3.86 mmol) in HCl/dioxane (4 M, 10 mL) was stirred at 15°C for 16 hours. The mixture was concentrated to afford 4-(3,4-difluorobenzyl)piperidine-4-carbonitrile hydrochloride (1.5 g). $^1H$ NMR $\delta_H$ 7.22 - 7.10 (m, 2H), 7.06 - 6.99 (m, 1H), 3.63 - 3.51 (m, 2H), 3.26 - 3.09 (m, 2H), 2.90 (s, 2H), 2.23 - 1.99 (m,4H).

Synthesis of A206

To a solution of 2-(pyrimidin-4-yl)nicotinic acid (100 mg, 0.497 mmol) in DMF (3 mL) was added HATU (283 mg, 0.745 mmol) and DIPEA (0.259 mL, 1.49 mmol) at 15°C. After stirring at 25°C for 30 minutes, 4-(3,4-difluorobenzyl) piperidine-4-carbonitrile hydrochloride (150 mg, 0.572 mmol) was added. The mixture was stirred for 10 h at 25°C and then directly purified by prep-HPLC (Column: Welch Xtimate C18 150*25 mm*5 um; Condition: water(0.04%$NH_3H_2O$)-ACN; Begin B:33, End B:63; Gradient Time(min):8.5;100%B Hold Time(min):2; FlowRate (ml/min): 30; Injections:9), lyophilized and triturated from DCM/n-hexane (1:2, 5 mL) to give 4-(3,4-difluorobenzyl)-1-(2-(pyrimidin-4-yl)nicotinoyl)piperidine-4-carbonitrile (13.2 mg, 6%). $^1H$ NMR $\delta_H$ 9.15 (s, 0.4H), 8.95 (s, 0.6H), 8.89 - 8.77 (m, 1H), 8.70 (dd, 1H), 8.24 - 8.17 (m, 1H), 7.64 - 7.57 (m, 1H), 7.43 - 7.36 (m, 1H), 7.13 - 6.99 (m, 2H), 6.98 - 6.88 (m, 1H), 4.89 - 4.80 (m, 0.6H), 4.78 - 4.68 (m, 0.4H), 3.62 - 3.51 (m, 0.5H), 3.43 - 3.26 (m, 1H), 3.22 - 2.96 (m, 1.5H), 2.89 - 2.73 (m, 2H), 2.08 - 1.90 (m, 1H), 1.82 - 1.66 (m, 1.5H), 1.63 - 1.53 (m, 1H), 0.84 - 0.72 (m, 0.5H). LC-ELSD/MS purity 100%, MS ESI calcd. For $C_{23}H_{20}F_2N_5O$ $[M+H]^+$ 420, found 420.

*Example 35. Synthesis of 4-(2, 6-difluorobenzyl)-1-(2-(pyrimidin-4-yl)nicotinoyl)piperidine-4-carbonitrile (A207)*

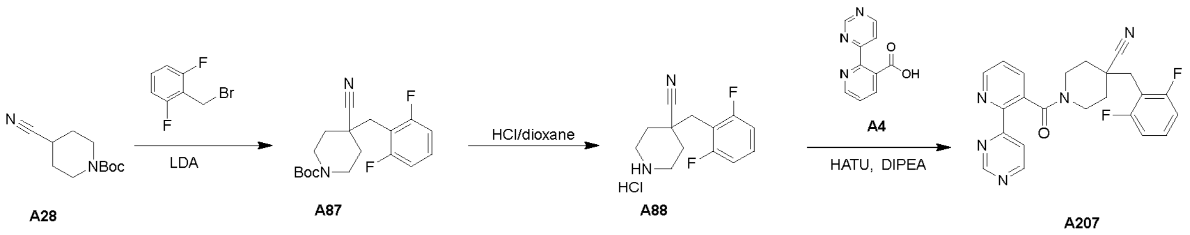


Synthesis of A87

To a mixture of tert-butyl 4-cyanopiperidine-1-carboxylate (800 mg, 3.80 mmol) in THF (10 mL) was added drop wise LDA (2.85 mL, 5.70 mmol, 2 M in THF/n-heptane) at - 78°C. After stirring at -78°C under nitrogen for 1 hour, 2-(bromomethyl)-1,3-difluorobenzene (944 mg, 4.56 mmol) was added. The mixture was warmed to 20°C and stirred for 16 hours. The reaction mixture was poured into saturated $NH_4Cl$ solution (100 mL), and the aqueous layer was extracted with ethyl acetate (2 x 100 mL). The combined organic layers were washed with brine (100 mL) and dried over $Na_2SO_4$, filtered, concentrated and purified by flash chromatography (30~50% EtOAc in PE) to give tert-butyl 4-cyano-4-(2,6-difluorobenzyl)piperidine-1-carboxylate (1.2 g, 94%). $^1$H NMR (400 MHz, $CDCl_3$) $\delta_H$ 7.33-7.27 (m, 1H), 6.99-6.90 (m, 2H), 4.31-4.02 (m, 2H), 3.02 (s, 4H), 1.99-1.88 (m, 2H), 1.62-1.59 (m, 1H), 1.58-1.54 (m, 1H), 1.46 (s, 9H).

Synthesis of A88

To a mixture of tert-butyl 4-cyano-4-(2,6-difluorobenzyl)piperidine-1-carboxylate (1.2 g, 3.56 mmol) in dioxane (12 mL) was added HCl/dioxane (4 M, 8.90 mL, 35.6 mmol) and mixture was stirred at 20°C for 16 hours. The reaction mixture was filtered, and the filter cake was washed with EtOAc (50 mL) and dried to give 4-[(2,6-difluorophenyl)methyl] piperidine-4-carbonitrile hydrochloride (800 mg, 95.1%). $^1$H NMR (400 MHz, $CDCl_3$) $\delta_H$ 7.51-7.35 (m, 1H), 7.12-7.00 (m, 2H), 3.58-3.45 (m, 2H), 3.24-3.12 (m, 4H), 2.31-2.15 (m, 2H), 2.08-1.91 (m, 2H).

Synthesis of A207

To a solution of 2-(pyrimidin-4-yl)pyridine-3-carboxylic acid (127 mg, 0.634 mmol), 4-[(2,6-difluorophenyl) methyl]piperidine-4-carbonitrile hydrochloride (150 mg, 0.634 mmol) and HATU (361 mg, 0.951 mmol) in DMF (5 mL) was added DIPEA (637 mg, 3.17 mmol). After stirring at 20°C for 3 hours, the reaction mixture poured into water (150 mL). The aqueous layer was extracted with EtOAc (2 x 100 mL), and the combined organic layers were washed with brine (100 mL), dried over anhydrous $Na_2SO_4$, and concentrated under reduced pressure to give the product, which was purified by HPLC (Phenomenex Gemini-NX 150 x 30mm, 5um; Condition: water (0.04%$NH_3H_2O$+10mM $NH_4HCO_3$)-ACN; Gradient: from 33% to 57% of B in 8 min and hold 100% for 2 min; Flow rate: 30mL/min; Injections: 7) to afford 4-(2,6-difluorobenzyl)-1-(2-(pyrimidin-4-yl)nicotinoyl)piperidine-4-carbonitrile (79.1 mg, 27.0%). $^1$H NMR (400 MHz, $CDCl_3$) $\delta_H$ 9.26-9.20 (m, 1H), 8.90-8.84 (m, 1H), 8.79-8.73 (m, 1H), 8.54-8.49 (m, 1H), 8.30-8.20 (m, 1H), 7.71-7.63 (m, 1H), 7.50-7.44 (m, 1H), 7.43-7.30 (m, 1H), 7.03-6.91 (m, 2H), 4.97-4.87 (m, 1H), 4.84-4.75 (m, 1H), 3.69-3.60 (m, 1H), 3.52-3.39 (m, 1H), 3.32-3.21 (m, 1H), 3.20-3.04 (m, 3H), 2.23-2.06 (m, 1H), 2.01-1.81 (m, 2H), 1.80-1.72 (m, 1H). LC-ELSD/MS purity 100%, MS ESI calcd. for $C_{23}H_{19}F_2N_5O$ $[M+H]^+$ 420.2 found 420.3.

*Example 36. Synthesis of 4-(4-ethylbenzyl)-1-(2-(pyrimidin-4-yl)nicotinoyl)piperidine-4-carbonitrile (A208)*

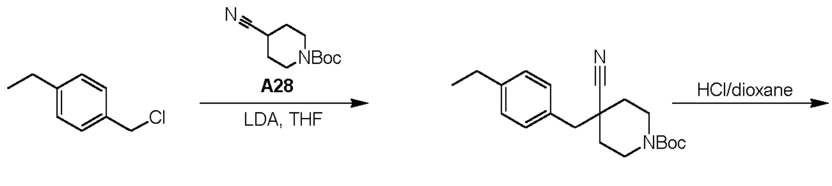


A89

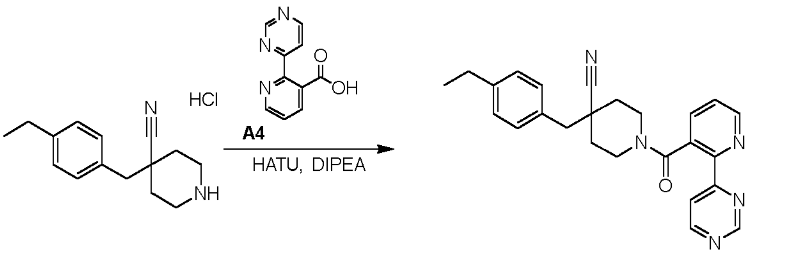


A90 A208

Synthesis of A89

To a mixture of tert-butyl 4-cyanopiperidine-1-carboxylate (1 g, 4.75 mmol) in THF (10 mL) was added dropwise LDA (3.56 mL, 7.12 mmol, 2 M in THF/n-heptane) at -70°C. After stirring under nitrogen at -70°C for 1 hour, 1-(chloromethyl)-4-ethylbenzene (734 mg, 4.75 mmol) was added and kept at 25°C for 16 hrs. The reaction mixture was poured into saturated $NH_4Cl$ solution (20 mL) and extracted with ethyl acetate (3 x 20 mL). The combined organic layers were washed with brine (20 mL) and dried over $Na_2SO_4$. The residue was purified by flash chromatography (0~30% of EtOAc in PE) to give 1.5 , which was further purified by chromatography (0-30% EA in PE) to give tert-butyl 4-cyano-4-(4-ethylbenzyl)piperidine-1-carboxylate (1.16 g, 77.8%). $^1$H NMR (400 MHz, $CDCl_3$) $\delta_H$ 7.17 (s, 4H), 4.25-3.90 (m, 2H), 2.99 (s, 2H), 2.83 (s, 2H), 2.64 (d, 2H), 1.84 (d, 2H), 1.45 (s, 11H), 1.23 (t, 3H).

Synthesis of A90

To a mixture of tert-butyl 4-cyano-4-(4-ethylbenzyl)piperidine-1-carboxylate (1.16 g, 3.53 mmol) in dioxane (12 mL) was added HCl/dioxane (4 M, 8.82 mL, 35.3 mmol), the mixture was stirred at 20°C for 16 hrs. The reaction mixture was concentrated to give 4-(4-ethylbenzyl)piperidine-4-carbonitrile hydrochloride (895.8 mg, 95.8%). $^1$H NMR (400 MHz, $CD_3OD$) $\delta_H$ 6.94 (d, 4H), 3.20 (d, 2H), 3.03-3.01 (m, 1H), 3.01 (s, 1H), 2.89-2.78 (m, 2H), 2.69 (s, 2H), 2.40-2.30 (m, 2H), 1.84 (d, 2H), 1.6 -1.55 (m, 2H), 0.93(t, 3H).

Synthesis of A208

To a solution of 2-(pyrimidin-4-yl) pyridine-3-carboxylic acid (143 mg, 0.71 mmol), 4-(4-ethylbenzyl)piperidine-4-carbonitrile hydrochloride (189.4 mg, 0.71 mmol) and HATU (406 mg, 1.07 mmol) in DMF (5 mL) was added DIEA (459 mg, 3.56 mmol). The mixture was stirred at 20°C for 16 hours. The mixture was concentrated and purified by HPLC (column: Phenomenex Gemini-NX 150*30mm*5um, condition: water (0.04%$NH_3H_2O$+10mM NH4HCO3)-ACN, begin B: 45, end B: 61, gradient time: 8 min, 100%B Hold Time: 3.5 min, flow rate: 30 mL/min) to give 4-(4-ethylbenzyl)-1-(2-(pyrimidin-4-yl) nicotinoyl)piperidine-4-carbonitrile (89.3 mg, 32%). $^1$H NMR (400 MHz, $CDCl_3$) $\delta_H$ 9.21 (s, 1H), 8.88 (d, 1H), 8.75 (s, 1H), 8.30-8.19 (m, 1H), 7.71-7.62 (m, 1H), 7.45 (d, 1H), 7.23-7.10 (m, 4H), 4.91-4.73 (m, 1H), 3.61 (d, 1H), 3.47-3.36 (m, 1H), 3.29-3.01 (m, 2H), 2.95-2.85 (m, 2H), 2.66 (d, 2H), 2.12-1.99 (m, 1H), 1.92-1.73 (m, 2H), 1.29-1.20 (m, 3H). LC-ELSD/MS purity 98%, MS ESI calcd. for $C_{25}H_{26}N_5O$ [M+H] 412, found 412.

*Example 37. Synthesis of 4-(2, 3-difluorobenzyl)-1-(2-(pyrimidin-4-yl)nicotinoyl)piperidine-4-carbonitrile (A209)*

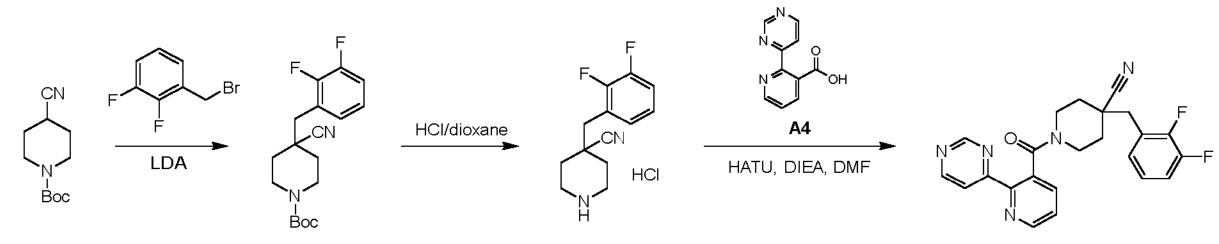


A28 A91 A92 A209

Synthesis of A91

To a mixture of tert-butyl 4-cyanopiperidine-1-carboxylate (1 g, 4.75 mmol) in THF (15 mL) was added dropwise LDA (4.01 mL, 2 M, 8.02 mmol) at -78°C. After stirring at - 78°C for 1 h, 1-(bromomethyl)-2,3-difluorobenzene (1.17 g, 5.69 mmol) was added. The mixture was warmed to 20°C and stirred for 16 hours. The mixture was poured into water (25 mL) and extracted with EtOAc (3 x 20 mL). The combined organic layers were washed with brine (2 x 50 mL), dried over $Na_2SO_4$, filtered and concentrated to give the product, which was purified by flash chromatography (10~30% of EtOAc in PE) to give tert-butyl 4-cyano-4-(2,3-difluorobenzyl)piperidine-1-carboxylate (1.1 g, 69.1 %). $^1H$ NMR(400 MHz, $CDCl_3$) $\delta_H$ 7.22-7.04 (m, 3H), 4.16 (br s, 2H), 3.09-2.91 (m, 4H), 1.87 (br d, 2H), 1.62 (br s, 1H), 1.55 (br d, 1H), 1.49-1.44 (m, 1H), 1.46 (s, 8H).

Synthesis of A92

To a solution of tert-butyl 4-cyano-4-(2,3-difluorobenzyl)piperidine-1-carboxylate (1.1 g, 3.27 mmol) in MeOH (10 mL) was added HCl/Dioxane (0.817 mL, 3.27 mmol, 4M). The mixture was stirred at 25°C under nitrogen for 12 hours and concentrated to give 4-(2,3-difluorobenzyl)piperidine-4-carbonitrile hydrochloride (800mg). $^1H$ NMR (400 MHz, $CDCl_3$) $\delta_H$ 7.39-7.14 (m, 3H), 3.58-3.48 (m, 2H), 3.24-3.10 (m, 4H), 2.21 (br d, 2H), 2.06-1.94 (m, 2H).

Synthesis of A209

To a solution of 4-(2,3-difluorobenzyl)piperidine-4-carbonitrile hydrochloride (150 mg, 0.550 mmol) in DMF (1 mL) was added HATU (313 mg, 0.825 mmol) and DIPEA (0.287 mL, 1.65 mmol) at 25°C. After stirring for 30 minutes at 25°C, 2-(pyrimidin-4-yl)nicotinic acid (110 mg, 0.55 mmol) was added. The mixture was stirred for 10 hours at 25°C, concentrated and directly purified by prep-HPLC (Column:Phenomenex Gemini -NX 150*30mm*5um; Condition:water (0.04%$NH_3H_2O$+ 10mM $NH_4HCO_3$)-ACN; Begin B:22, End B:52; Gradient Time(min):3; 100%B Hold Time(min): 2; FlowRate (ml/ min) :30; Injections:7) to afford 4-(2,3-difluorobenzyl)-1-(2-(pyrimidin-4-yl)nicotinoyl)piperidine-4-carbonitrile (20.7 mg, 9.0%). $^1H$ NMR(400 MHz, $CDCl_3$) $\delta_H$ 9.26-8.74 (m, 2H), 8.73-8.12 (m, 2H), 7.68-7.35 (m, 2H), 7.14-7.00 (m, 3H), 4.95-4.65 (m, 1H), 3.63-3.12 (m, 2H), 3.09-2.90 (m, 3H), 2.11-1.93 (m, 1H), 1.91-1.82 (m, 1H), 1.81-1.55 (m, 2H). LC-MS: purity>99%, MS ESI calcd. for $C_{23}H_{19}F_2N_5O$ $[M+H]^+$420.3, found 420.3

*Example 38. Synthesis of 4-(3-chlorobenzyl)-1-(2-(pyrimidin-4-yl)nicotinoyl)piperidine-4-carbonitrile (A210)*

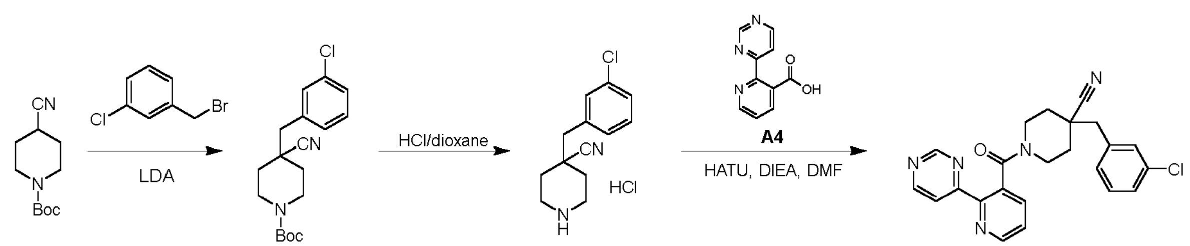


A28 A93 A94 A210

Synthesis of A93

To a mixture of tert-butyl 4-cyanopiperidine-1-carboxylate (1 g, 4.75 mmol) in THF (15 mL) was added dropwise LDA (4.01 mL, 2 M, 8.02 mmol) at -78°C. After stirring at - 78°C for 1 h, 1-(bromomethyl)-3-chlorobenzene (1.16 g, 5.69

mmol) was added. The mixture was warmed to 20°C and stirred for 16 hours. The mixture was poured into water (25 mL) and extracted with EtOAc (3 x 20 mL). The combined organic layers were washed with brine (2 x 50 mL), dried over $Na_2SO_4$, filtered and concentrated. The product was purified by flash chromatography (10~30% of EtOAc in PE) to give tert-butyl 4-(3-chlorobenzyl)-4-cyanopiperidine-1-carboxylate (1.0 g, 62.8 %). $^1$H NMR(400 MHz, $CDCl_3$) $\delta_H$ 7.32-7.28 (m, 2H), 7.26-7.17 (m, 2H), 4.31-4.04 (m, 2H), 3.15-2.92 (m, 2H), 2.84 (s, 2H), 1.85 (br d, 2H), 1.55-1.40 (m, 11H).

Synthesis of A94

To a solution of tert-butyl 4-(3-chlorobenzyl)-4-cyanopiperidine-1-carboxylate (1.0 g, 2.98 mmol) in MeOH (10 mL) was added HCl/Dioxane (5.95 mL, 23.8 mmol, 4M). The mixture was stirred under nitrogen at 25°C for 12 hours and concentrated to give 4-(3-chlorobenzyl)piperidine-4-carbonitrile hydrochloride (800 mg). $^1$H NMR (400 MHz, $CDCl_3$) $\delta_H$ 7.49-7.26 (m, 4H), 3.52 (br d, 2H), 3.18 (br t, 2H), 3.04 (s, 2H), 2.16 (br d, 2H), 2.01-1.83 (m, 2H).

Synthesis of A210

To a solution of 4-(3-chlorobenzyl)piperidine-4-carbonitrile hydrochloride (150 mg, 0.553 mmol) in DMF (1 mL) was added HATU (315 mg, 0.827 mmol) and DIPEA (0.287 mL, 1.65 mmol) at 25°C. After stirring for 30 minutes at 25°C, 2-(pyrimidin-4-yl)nicotinic acid (111 mg, 0.553 mmol) was added to the solution. The mixture was stirred for 10 hours at 25°C, concentrated, and directly purified by prep-HPLC (Column:Phenomenex Gemini-NX 150*30mm*5um;Condition:water (0.04%$NH_3H_2O$+10mM $NH_4HCO_3$)-ACN; Begin B:22, End B:52; Gradient Time(min):3; 100%B Hold Time(min): 2; FlowRate (ml/ min):30; Injections:7) to afford the product 4-(3-chlorobenzyl)-1-(2-(pyrimidin-4-yl)nicotinoyl)piperidine-4-carbonitrile (30.4 mg, 13.1%). $^1$H NMR: (400 MHz, $CDCl_3$) $\delta_H$ 9.27-8.74 (m, 3H), 8.31-8.21 (m, 1H), 7.73-7.60 (m, 1H), 7.48 (dd, 1H), 7.38-7.28 (m, 2H), 7.26-7.13 (m, 2H), 4.97-4.74 (m, 1H), 3.72-3.07 (m, 3H), 3.00-2.84 (m, 2H), 2.21-1.96 (m, 1H), 1.91-1.61 (m, 3H). LC-MS: purity>99%, MS for $C_{23}H_{20}ClN_5O$ [M +H]$^+$418.3, found 418.3.

*Example 39. Synthesis of 4-((6-cyclopropylpyridin-3-yl)methyl)-1-(2-(pyrimidin-4-yl)nicotinoyl)piperidine-4-carbonitrile (A211)*

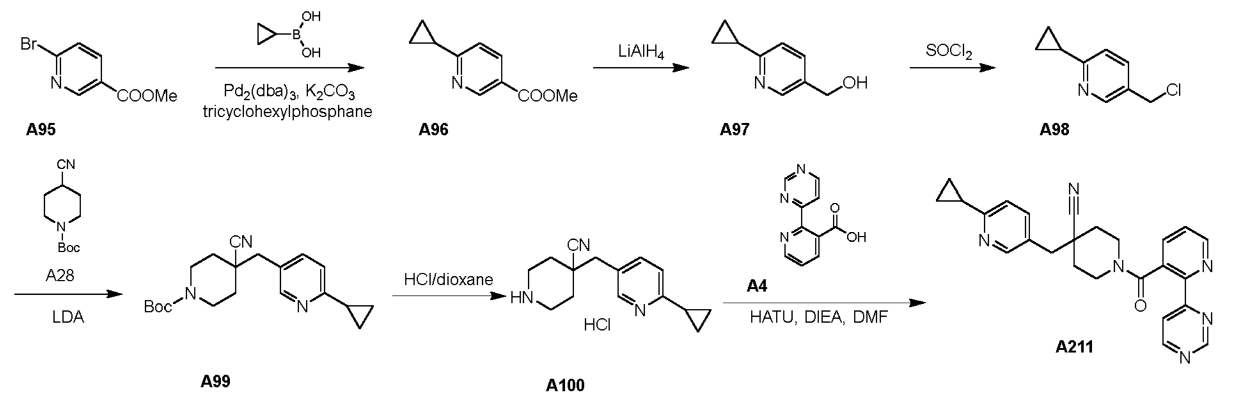

A95 A96 A97 A98 A99 A100 A211

Synthesis of A96

A mixture of methyl 6-bromonicotinate (2 g, 9.25 mmol), cyclopropylboronic acid (1 g, 11.6 mmol), tricyclohexylphosphane (776 mg, 2.77 mmol) $Pd_2(dba)_3$ (847 mg, 0.925 mmol) and $K_2CO_3$ (3.82 g, 27.7 mmol) in toluene (40 mL)/water (4 mL) was stirred under nitrogen at 100°C for 16 hours. The reaction mixture poured into water (50 mL) and the aqueous layer was extracted with EtOAc (2 x 20 mL). The combined organic layers were washed with brine (30 mL), dried over anhydrous $Na_2SO_4$, and concentrated to give the product, which was purified by flash chromatography (0~10% of EtOAc in PE) to give methyl 6-cyclopropylnicotinate (2 g). $^1$H NMR (400 MHz, $CDCl_3$) $\delta_H$ 9.91-9.89 (m, 1H), 8.23-8.03 (m, 1H), 7.85-7.75 (m, 1H), 7.65-7.55 (m, 1H), 7.43-7.32 (m, 1H), 7.20-7.15 (m, 1H), 7.12-7.03 (m, 1H), 3.91 (s, 3H), 2.11-2.02 (m, 1H), 1.15-1.01 (m, 1H).

Synthesis of A97

To the mixture of methyl 6-cyclopropylnicotinate (2 g, 11.2 mmol) in THF (30 mL) at 0°C was added $LiAlH_4$ (1.27 g,

33.5 mmol) in one portion. After stirring at 0°C for 1 hour, the reaction mixture was diluted with THF (10 mL) and water (1.3 g) was added. After stirring at 15°C for 5 minutes, 15% NaOH (1.3 g) was added. After stirring at 15°C for 5 minutes, water (3.9 g) was added. Anhydrous sodium sulfate was added, and the mixture was filtered and concentrated to give (6-cyclopropylpyridin-3-yl)methanol (1.5 g). $^1$H NMR (400 MHz, $CDCl_3$) $\delta_H$ 8.45-8.35 (m, 1H), 7.53-7.58 (m, 1H), 7.09-7.01 (m, 1H), 4.60-4.53 (m, 2H), 2.05-1.96 (m 1H), 1.03-0.90 (m, 4H).

Synthesis of A98

A mixture of (6-cyclopropylpyridin-3-yl)methanol (1.5 g, 10.0 mmol) in $SOCl_2$ (5.95 g, 50.0 mmol) was stirred 12 hours at 70°C. The reaction was cooled and poured into $NaHCO_3$ solution (50 mL). The aqueous layer was extracted with EtOAc (2 x 20 mL), and the combined organic layers were washed with brine (30 mL), dried over anhydrous $Na_2SO_4$, and concentrated to give the product. The product was purified by flash chromatography (0~10% of EA in PE) to give 5-(chloromethyl)-2-cyclopropylpyridine (1.1 g, 65.8%). $^1$H NMR (400 MHz, $CDCl_3$) $\delta_H$ 8.42-8.41 (m, 1H), 7.68-7.58 (m, 1H), 7.09-7.01 (m, 1H), 4.60-4.53 (m, 2H), 2.05-1.96 (m, 1H), 1.09-0.93 (m, 4H).

Synthesis of A99

To a mixture of tert-butyl 4-cyanopiperidine-1-carboxylate (626 mg, 2.98 mmol) in THF (10 mL) was added dropwise LDA (5 mL, 4.47 mmol) at -78°C, the mixture was stirred under nitrogen at -78°C for 1 hour. 5-(Chloromethyl)-2-cyclopropylpyridine (500 mg, 2.98 mmol) was added, and the mixture was warmed to 20°C for 16 hours. The reaction mixture was poured into saturated $NH_4Cl$ solution (30 mL). The aqueous layer was extracted with ethyl acetate (2 x 30 mL), and the combined organic layers were washed with brine (20 mL) and dried over $Na_2SO_4$. The product was purified by silica gel chromatography (PE: EtOAc from 5:1 to 4:1) to give tert-butyl 4-cyano-4-((6-cyclopropylpyridin-3-yl)methyl) piperidine-1-carboxylate (460 mg, 45.5%). $^1$H NMR (400 MHz, $CDCl_3$) $\delta_H$ 8.35-8.25 (m, 1H), 7.59-7.48 (m, 1H), 7.09-7.01 (m, 1H), 4.25-4.05 (m, 2H), 3.05-2.95 (m, 2H), 2.85-2.75 (m, 2H), 2.09-1.85 (m, 1H), 1.82-1.75 (m, 2H), 1.72-1.55 (m, 2H), 1.55-1.45 (m, 9H), 1.09-0.93 (m, 4H).

Synthesis of A100

To a mixture of tert-butyl 4-cyano-4-((6-cyclopropylpyridin-3-yl)methyl)piperidine-1-carboxylate (200 mg, 0.5857 mmol) was added HCl/dioxane (4 M, 10 mL, 40 mmol), the mixture was stirred at 20°C for 16 hours. The reaction mixture was concentrated to give 4-((6-cyclopropylpyridin-3-yl)methyl)piperidine-4-carbonitrile hydrochloride (170 mg), which was used directly in the next step.

Synthesis of A211

To a solution of 2-(pyrimidin-4-yl)pyridine-3-carboxylic acid (142 mg, 0.7073 mmol) in DMF (3 mL) was added HATU (403 mg, 1.06 mmol) and DIPEA (273 mg, 2.12 mmol) at 25°C. After stirring at 25°C for 30 minutes, 4-((6-cyclopropylpyridin-3-yl)methyl)piperidine-4-carbonitrile hydrochloride (170 mg, 0.7073 mmol) was added, and the reaction was stirred for 10 hours at 25°C. The mixture was concentrated and purified by prep.HPLC (Phenomenex Gemini-NX 150*30mm*5um; Condition: water (0.04%$NH_3H_2O$+10mM $NH_4HCO_3$)-ACN; Begin B:30, End B:50; Gradient Time(min):8; 100%B Hold Time(min):3.5; FlowRate(ml/min):30; Injections:6) to afford 4-((6-cyclopropylpyridin-3-yl) methyl)-1-(2-(pyrimidin-4-yl)nicotinoyl)piperidine-4-carbonitrile (52.5 mg, 17.5%). $^1$H NMR (400 MHz, $CDCl_3$) $\delta_H$ 9.25-9.15 (m, 1H), 8.93-7.83 (m, 1H), 8.76-8.66 (m, 1H), 8.30-8.20 (m, 2H), 7.70-7.60 (m, 1H), 7.55-7.43 (m, 2H), 7.18-7.08 (m, 1H), 4.95-4.75 (m, 2H), 3.65-3.35 (m, 2H), 3.25-3.05 (m, 1H), 2.85-2.75 (m, 2H), 2.22-1.95 (m, 2H), 1.75-1.65 (m, 3H), 1.09-0.95 (m, 4H). LC-ELSD/MS purity 97%, MS ESI calcd. for $C_{25}H_{25}N_6O$ $[M+H]^+$ 425.2, found 425.2.

*Example 40. Synthesis of 4-(4-cyclopropylbenzyl)-1-(2-(pyrimidin-4-yl)nicotinoyl)piperidine-4-carbonitrile (A212)*

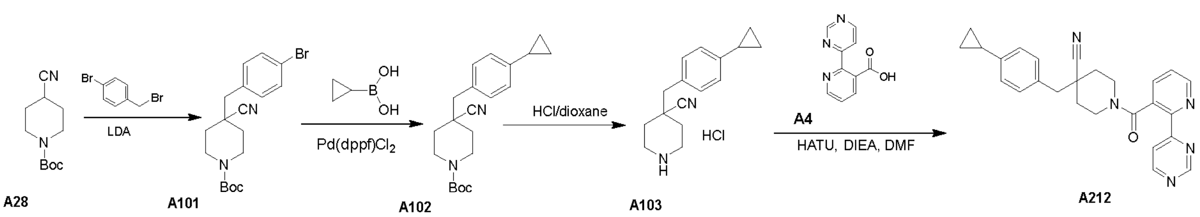


A28 A101 A102 A103 A212

Synthesis of A101

To a mixture of tert-butyl 4-cyanopiperidine-1-carboxylate (5 g, 23.7 mmol) in THF (100 mL) was added dropwise LDA (17.7 mL, 35.5 mmol, 2 M in THF/n-heptane) at -78°C, the mixture was stirred under nitrogen at -78°C for 1 hour. 1-(Bromomethyl)-4-fluorobenzene (7.09 g, 28.4 mmol) was added, and the mixture was warmed to 20°C and stirred for 16 hours. The reaction mixture was poured into saturated $NH_4Cl$ solution (100 mL), and the aqueous layer was extracted with ethyl acetate (2 x 100 mL). The combined organic layers were washed with brine (100 mL) and dried over $Na_2SO_4$. The product was purified by silica gel chromatography (PE: EtOAc from 50:1 to 3:1) to give tert-butyl 4-(4-bromobenzyl)-4-cyanopiperidine-1-carboxylate (5.2 g, 57.9% yield). $^1H$ NMR (400 MHz, $CDCl_3$) $\delta_H$ ppm 7.49-7.47 (d, 2 H), 7.16-7.14 (d, 2 H), 4.15-4.10 (m, 2 H), 3.10- 2.85(m, 2 H), 2.80 (s, 2 H), 1.85-1.75 (d, 2 H), 1.50-1.46 (m, 2 H), 1.44 (s, 9 H).

Synthesis of A102

A mixture of tert-butyl 4-(4-bromobenzyl)-4-cyanopiperidine-1-carboxylate (2 g, 5.27 mmol), cyclopropylboronic acid (1.35 g, 15.8 mmol), Pd(dppf)$Cl_2$ (430 mg, 0.527 mmol) and DIPEA (3.39 g, 26.3 mmol) in toluene (30 mL)/$H_2O$ (5 mL) was stirred at 100°C under nitrogen for 16 hours. The reaction mixture poured into water (50 mL) and the aqueous layer was extracted with EtOAc (2 x 20 mL). The combined organic layers were washed with brine (30 mL), dried over anhydrous $Na_2SO_4$, and concentrated under reduced pressure. The product was purified by flash chromatography (0~10% of EA in PE) to give tert-butyl 4-cyano-4-(4-cyclopropylbenzyl)piperidine-1-carboxylate (1.2 g). $^1H$ NMR (400 MHz, METHANOL-d4) $\delta_H$ ppm 7.15-7.13 (d, 2 H), 7.04-7.02 (d, 2 H), 4.22-4.00 (m, 2 H), 3.05-2.90(m, 2 H), 2.81 (s, 2 H), 1.91-1.86 (m, 1 H), 1.85-1.78 (m, 2 H), 1.50-1.46 (m, 2 H), 1.45 (s, 9 H), 0.98-0.93 (m, 2 H), 0.70-0.66 (m, 2 H).

Synthesis of A103

To a mixture of tert-butyl 4-cyano-4-(4-cyclopropylbenzyl)piperidine-1-carboxylate (1.2 g, 3.52 mmol) in dioxane (15 mL) was added HCl/dioxane (4 M, 17.6 mL, 70.4 mmol ), the mixture was stirred at 20°C for 16 hours. The reaction mixture was filtered, and the filter cake was washed with 5 mL of EtOAc, and dried to give 4-(4-cyclopropylbenzyl) piperidine-4-carbonitrile hydrochloride (800 mg, 82.1% yield). $^1H$ NMR (400 MHz, $CD_3OD$) $\delta_H$ ppm 7.23-7.19 (d, 2 H), 7.09-7.04 (d, 2 H), 3.52-3.45 (m, 2 H), 3.18- 3.08 (td, 2 H), 2.95 (s, 2 H), 2.17-2.04 (m, 2 H), 1.96-1.80 (m, 3 H), 0.98-0.93 (m, 2 H), 0.67-0.63 (m, 2 H).

Synthesis of A212

To a solution of 2-(pyrimidin-4-yl)pyridine-3-carboxylic acid (159 mg, 0.7947 mmol) , 4-(4-cyclopropylbenzyl) piperidine-4-carbonitrile hydrochloride (200 mg, 0.7225 mmol) and HATU (410 mg, 1.08 mmol) in DMF (5 mL) was added DIPEA (465 mg, 3.61 mmol). After stirring at 20°C for 2 hours, the reaction mixture poured into $H_2O$ (50 mL), the aqueous layer was extracted with EtOAc (2 x 100 mL). The combined organic layers were washed with brine (100 mL), dried over anhydrous $Na_2SO_4$, and concentrated to give product, which was purified by Prep-HPLC (Column: Phenomenex Gemini-NX 150*30mm*5um; Condition:water(0.04%$NH_3H_2O$+10mM NH4HCO3)-ACN; Begin B: 42%; End 64%) to give 4-(4-cyclopropylbenzyl)-1-(2-(pyrimidin-4-yl)nicotinoyl)piperidine-4-carbonitrile (60.6 mg, 19.8% yield ). $^1H$ NMR (400 MHz, $CDCl_3$) $\delta_H$ 9.21 (s, 0.4 H), 8.89-8.88 (m, 1 H), 8.81 (s, 0.6 H), 8.75-8.74 (m, 1 H), 8.26-8.21 (m, 1 H), 7.66-7.64 (m, 1 H), 7.47-7.45 (m, 1 H), 7.13-7.11 (m, 2 H), 7.05-7.03 (m, 2 H), 4.88-4.85 (m, 0.7 H), 4.77-4.73 (m, 0.3 H), 3.62-3.58 (m, 0.7 H), 3.44-3.37 (m, 1 H), 3.25-3.21 (m, 0.3 H), 3.14-3.11 (m, 1 H), 2.89-2.87 (m, 2 H), 2.10-2.06 (m, 1 H), 1.85-1.75 (m, 2 H), 1.74-1.65 (m, 0.5 H), 1.64-1.60 (m, 1 H), 1.50-1.40 (m, 0.5 H), 0.97-0.95 (m, 2 H), 0.69-0.65 (m, 2 H). LC-ELSD/MS purity 100%, MS ESI calcd. For C26H25N5O [M+H]+ 424.2 found 424.3.

*Example 41. Synthesis of 4-(3,5-difluorobenzyl)-1-(2-(pyrimidin-4-yl)nicotinoyl)piperidine-4-carbonitrile (A213)*

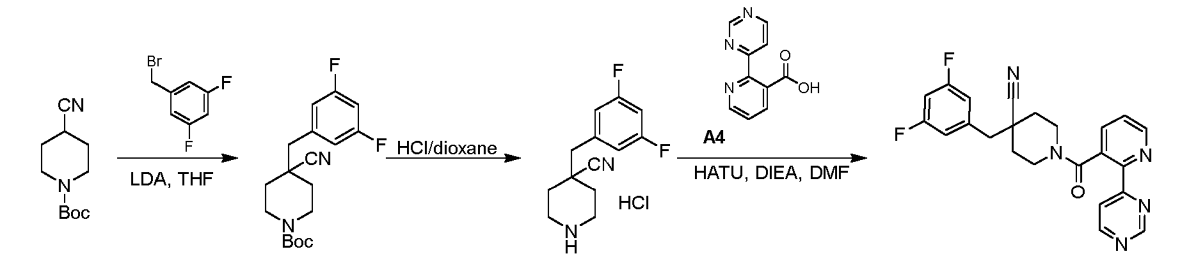


A28 A104 A105 A213

Synthesis of A104

To a mixture of tert-butyl 4-cyanopiperidine-1-carboxylate (2 g, 9.51 mmol) in THF (40 mL) was added dropwise LDA (7.10 mL, 14.2 mmol, 2 M in THF/n-heptane) at -78°C. After stirring at -78°C under nitrogen for 1 hour, 1-(bromomethyl)-3,5-difluorobenzene (2.36 g, 11.4 mmol) was added. The mixture was warmed to 20°C and stirred for 16 hours. The reaction mixture was poured into saturated $NH_4Cl$ solution (100 mL). The aqueous layer was extracted with ethyl acetate (2 x 100 mL). The combined organic layers were washed with brine (100 mL) and dried over $Na_2SO_4$. The product was purified by flash chromatography (0~10% of EA in PE) to give tert-butyl 4-cyano-4-[(3,5-difluorophenyl)methyl] piperidine-1-carboxylate (2.4 g, 75%). $^1$H NMR (400 MHz, $CDCl_3$) $\delta_H$ 6.88-6.68 (m, 4H), 4.26-4.11 (m, 2H), 3.10-2.90 (m, 2H), 2.85-2.75 (m, 2H), 1.95-1.85 (m, 2H), 1.56-1.43 (m, 13H).

Synthesis of A105

To a mixture of tert-butyl 4-cyano-4-[(3,5-difluorophenyl)methyl]piperidine-1-carboxylate (2.4 g, 7.13 mmol) in dioxane (30 mL) was added HCl/dioxane (4 M, 35.5 mL, 142 mmol ), the mixture was stirred at 20°C for 16 hours. The reaction mixture was filtered, and the filter cake was washed with 10 mL of EtOAc and dried to give 4-[(3,5-difluorophenyl) methyl]piperidine-4-carbonitrile hydrochloride (600 mg). $^1$H NMR (400 MHz, $CDCl_3$) $\delta_H$ 7.13-7.01 (m, 2H), 7.95-7.85 (m, 1H), 3.58-3.42 (m, 2H), 3.28-3.18 (m, 2H), 3.08-3.01 (m, 2H), 2.18-2.08 (m, 2H), 2.05-1.95 (m, 2H).

Synthesis of A213

To a solution of 2-(pyrimidin-4-yl)pyridine-3-carboxylic acid (200 mg, 0.9941 mmol), 4-[(3,5-difluorophenyl) methyl]piperidine-4-carbonitrile hydrochloride (243 mg, 0.8946 mmol) and HATU (566 mg, 1.49 mmol) in DMF (5 mL) was added DIPEA (641 mg, 4.97 mmol). After stirring at 20°C for 2 hours, the reaction mixture poured into water (50 mL). The aqueous layer was extracted with EtOAc (2 x 100 mL), and the combined organic layers were washed with brine (100 mL), dried over anhydrous $Na_2SO_4$, and concentrated to give the product, which was purified by Prep-HPLC (Column:PhenomenexGemini-NX150*30mm*5um; Condition:water(0.04%$NH_3H_2O$+10mM $NH_4HCO_3$)-ACN;BeginB:38%;End58%) togive4-[(3,5-difluorophenyl)methyl]-1-[2-(pyrimidin-4-yl)pyridine-3-carbonyl]piperidine-4-carbonitrile (54.1 mg, 15%). $^1$H NMR (400 MHz, $CDCl_3$) $\delta_H$ 9.21 (s, 0.34H), 8.97 (s, 0.56H), 8.96-8.86 (m, 1H), 8.78-8.68 (m, 1H), 8.30-8.20 (m, 1H), 7.70-7.60 (m, 1H), 7.49-7.39 (m, 1H), 6.80-6.68 (m, 3H), 4.95-4.85 (m, 0.60H), 3.65-3.55 (m, 0.61H), 3.50-3.35 (m, 1H), 3.32-3.21 (m, 0.39H), 3.20-3.05 (m, 1H), 2.95-2.75 (m, 2H), 2.21-2.01 (m, 1H), 1.85-1.75 (m, 1.6H), 1.71-1.59 (m, 1H), 1.55-1.45 (m, 0.43H). $^{19}$F NMR (376.5 MHz, $CDCl_3$) $\delta_F$ -108.921, $\delta_F$ -108.986. LC-ELSD/MS purity 99%, MS ESI calcd. for $C_{23}H_{20}F_2N_5O$ $[M+H]^+$ 420.3, found 420.3

*Example 42. Synthesis of 4-(2-cyclopropylbenzyl)-1-(2-(pyrimidin-4-yl)nicotinoyl)piperidine-4-carbonitrile (A214)*

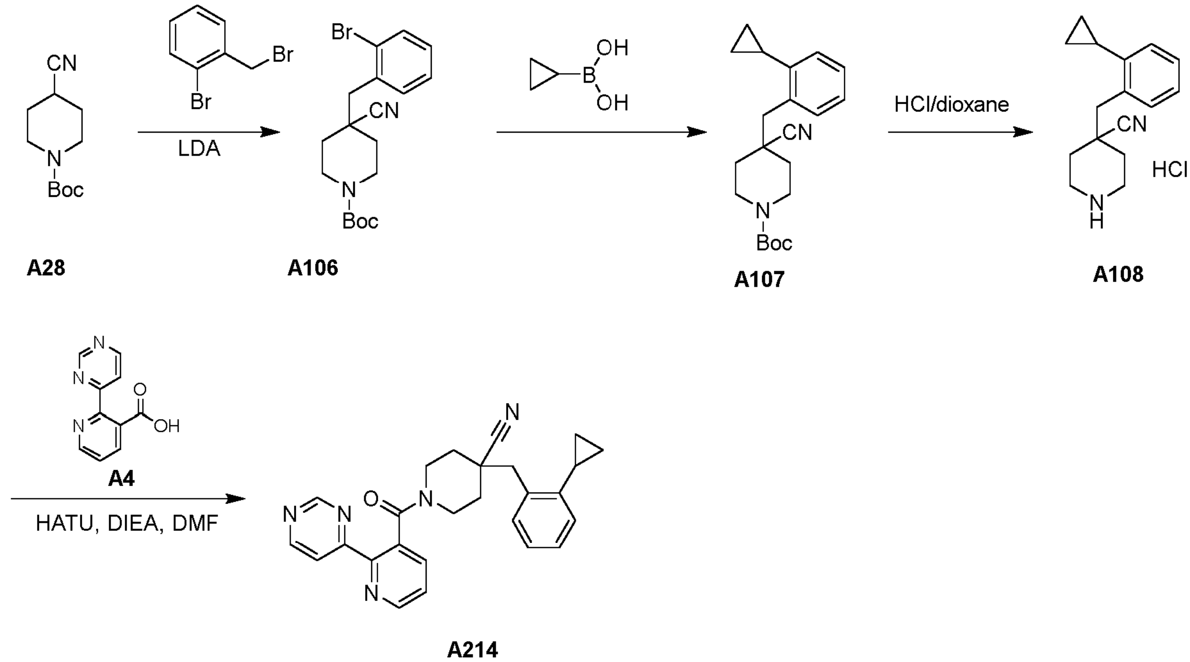


A28 A106 A107 A108 A4 A214

Synthesis of A106

To a mixture of tert-butyl 4-cyanopiperidine-1-carboxylate (2 g, 9.51 mmol) in THF (30 mL) was added dropwise LDA (8.00 mL, 2 M, 16.0 mmol) at -78°C. After stirring at - 78°C for 1 hour, 1-bromo-2-(bromomethyl)benzene (2.84 g, 11.4 mmol) was added. The mixture was warmed to 20°C and stirred for 16 h. The mixture was poured into water (25 mL) and extracted with EtOAc (3 x 20 mL). The combined organic layers were washed with brine (2 x 50 mL), dried over $Na_2SO_4$, filtered and concentrated to give the product, which was purified by flash chromatography (10~30% of EtOAc in PE) to give tert-butyl 4-(2-bromobenzyl)-4-cyanopiperidine-1-carboxylate (2.6 g, 74.4%). $^1H$ NMR(400 MHz, $CDCl_3$) $\delta_H$ 7.59 (dd, 1H), 7.50 (dd, 1H), 7.32 (dt, 1H), 7.16 (dt, 1H), 4.25-4.06 (m, 2H), 3.13 (s, 2H), 2.99 (br s, 2H), 1.87 (br d, 2H), 1.68 (dt, 2H), 1.46 (s, 9H).

Synthesis of A107

A mixture of tert-butyl 4-(2-bromobenzyl)-4-cyanopiperidine-1-carboxylate (1 g, 2.63 mmol), cyclopropylboronic acid (677 mg, 7.89 mmol), Pd(dppf)$Cl_2$ (214 mg, 0.263 mmol) and DIPEA (2.27 mL, 13.1 mmol) in toluene (15 mL)/$H_2O$ (2.5 mL) was stirred at 100°C under nitrogren for 16 hours. The reaction mixture poured into $H_2O$ (50 mL), and the aqueous layer was extracted with EtOAc (2 x 20 mL). The combined organic layers were washed with brine (30 mL), dried over anhydrous $Na_2SO_4$, and concentrated to give the product, which was purified by flash chromatography (0~10% of EtOAc in PE) to give tert-butyl 4-cyano-4-(2-cyclopropylbenzyl)piperidine-1-carboxylate (1 g). $^1H$ NMR(400 MHz, $CDCl_3$) $\delta_H$ 7.30 (dd, 1H), 7.25-7.12 (m, 2H), 7.04-6.98 (m, 1H), 4.33-4.01 (m, 2H), 3.16 (s, 2H), 3.02 (br s, 2H), 2.09-1.99 (m, 1H), 1.94 (br d, 2H), 1.63 (br d, 1H), 1.56 (br d, 1H), 1.46 (s, 8H), 1.04-0.94 (m, 2H), 0.73-0.65 (m, 2H).

Synthesis of A108

To a solution of tert-butyl 4-cyano-4-(2-cyclopropylbenzyl)piperidine-1-carboxylate (1 g, 2.93 mmol) in MeOH (10 mL) was added HCl/Dioxane (5.85 mL, 4M, 23.4 mmol) at 25°C. The mixture was stirred at 25°C under nitrogen for 12 hours. The mixture was concentrated to give 4-(2-cyclopropylbenzyl)piperidine-4-carbonitrile hydrochloride (900 mg). $^1H$ NMR(400 MHz, $CDCl_3$) $\delta_H$ 7.37-7.30 (m, 1H), 7.27-7.15 (m, 2H), 7.03 (d, 1H), 3.52 (br d, 2H), 3.30 (s, 2H), 3.19 (br t, 2H), 2.25 (br d, 2H), 2.18-2.10 (m, 1H), 2.09-1.94 (m, 3H), 1.12-0.99 (m, 2H), 0.76-0.63 (m, 2H).

Synthesis of A214

To a solution of 2-(pyrimidin-4-yl)nicotinic acid (100 mg, 0.3612 mmol) in DMF (1 mL) was added HATU (206 mg, 0.5418 mmol) and DIPEA (0.187 mL, 1.08 mmol) at 25°C. After stirring for 30 min at 25°C, 4-(2-cyclopropylbenzyl) piperidine-4-carbonitrile hydrochloride (72.6 mg, 0.3612 mmol) was added to the solution. The mixture was stirred for 10

hours at 25°C, concentrated, and directly purified by prep-HPLC (Column:Phenomenex Gemini-NX 150*30mm*5um; Condition:water (0.04% $NH_3H_2O$ + 10mM $NH_4HCO_3$) -ACN; Begin B:37, End B:67; Gradient Time(min):3; 100%B Hold Time(min):2; FlowRate(ml/min):30; Injections:7) to afford 4-(2-cyclopropylbenzyl)-1-(2-(pyrimidin-4-yl)nicotinoyl)piperidine-4-carbonitrile (30.2 mg, 19.8%). $^1$H NMR(400 MHz, $CDCl_3$) $\delta_H$ 9.22 (s, 1H), 8.89-8.82 (m, 1H), 8.75 (br d, 1H), 8.50 (s, 1H), 8.27 (br d, 1H), 8.21 (d, 1H), 7.71-7.62 (m, 1H), 7.46 (dd, 1H), 7.32-7.26 (m, 1H), 7.25-7.20 (m, 1H), 7.19-7.11 (m, 1H), 7.01 (br d, 1H), 4.91 (br d, 1H), 4.79 (br d, 1H), 3.67-3.55 (m, 1H), 3.52-3.36 (m, 1H), 3.32-3.04 (m, 3H), 2.23-2.07 (m, 1H), 2.05-1.83 (m, 3H), 1.80-1.68 (m, 1H), 1.05-0.88 (m, 2H), 0.73-0.58 (m, 2H). LC-MS: purity 100%, MS ESI calcd. for $C_{26}H_{25}N_5O$ $[M+H]^+$ 424.3, found 424.3.

*Example 43. Synthesis of (R)-4-(1-(4-fluorophenyl)propyl)-1-(2-(pyrimidin-4-yl)nicotinoyl)piperidine-4-carbonitrile (A215) and (S)-4-(1-(4-fluorophenyl)propyl)-1-(2-(pyrimidin-4-yl)nicotinoyl)piperidine-4-carbonitrile (A216)*

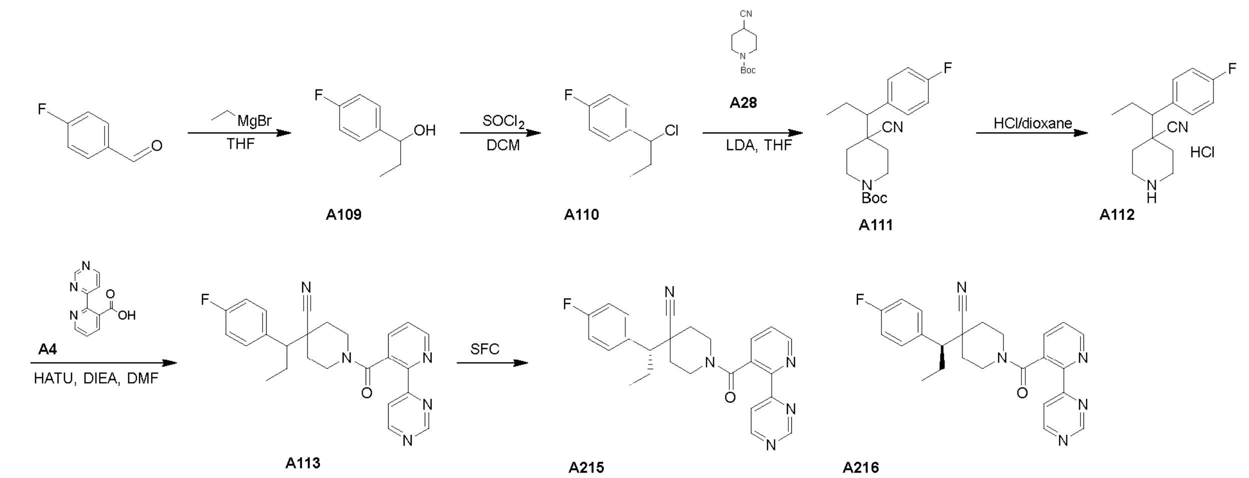

A109 A110 A111 A112 A113 A215 A216

Synthesis of A109

To a mixture of 4-fluorobenzaldehyde (5 g, 40.2 mmol) in THF (100 mL) was added dropwise bromo(ethyl) magnesium in $Et_2O$ (26.8 mL, 80.4 mmol, 3 M in $Et_2O$) at 0°C. The mixture was warmed to 20°C and stirred for 16 hours. The reaction mixture was poured into saturated $NH_4Cl$ solution (200 mL) and extracted with ethyl acetate (2 x 100 mL). The combined organic layers were washed with brine (200 mL), dried over $Na_2SO_4$, and concentrated to give 1-(4-fluorophenyl)propan-1-ol (6.5 g). $^1$H NMR (400 MHz, $CDCl_3$) $\delta_H$ ppm 7.32-7.28 (dd, 2 H), 7.04-7.01 (td, 2 H), 4.60-4.57 (td, 1H), 1.88-1.87 (dd, 1 H), 1.84-1.68 (m, 2 H), 0.92-0.88 (t, 3 H).

Synthesis of A110

To a mixture of 1-(4-fluorophenyl)propan-1-ol (1 g, 6.48 mmol) in DCM (15 mL) was added thionyl chloride (1.03 mL, 14.2 mmol) at 0°C. The mixture warmed to 20°C and was stirred for 3 hours. The reaction mixture was concentrated to give 1-(1-chloropropyl)-4-fluorobenzene (1 g). $^1$H NMR (400 MHz, $CDCl_3$) $\delta_H$ ppm 7.36-7.32 (dd, 2 H), 7.05-7.01 (td, 2 H), 4.79-4.75 (t, 1H), 2.15-2.02 (m, 2 H), 1.01-0.97 (t, 3 H).

Synthesis of A111

To a mixture of tert-butyl 4-cyanopiperidine-1-carboxylate (1 g, 4.75 mmol) in THF (20 mL) was added dropwise LDA (3.56 mL, 7.12 mmol, 2 M in THF/n-heptane) at -78°C, the mixture was stirred under nitrogen at -78°C for 1 hour, and 1-(1-chloropropyl)-4-fluorobenzene (982 mg, 5.69 mmol) was added. The mixture was warmed to 20°C and stirred for 16 hours. The reaction mixture was poured into saturated $NH_4Cl$ solution (100 mL), the aqueous layer was extracted with ethyl acetate (2 x 100 mL). The combined organic layers were washed with brine (100 mL) and dried over $Na_2SO_4$. The product was purified by flash chromatography (0~10% of EA in PE) to give tert-butyl 4-cyano-4-(1-(4-fluorophenyl)propyl) piperidine-1-carboxylate (700 mg, 42.6% yield). $^1$H NMR (400 MHz, $CDCl_3$) $\delta_H$ ppm 7.25-7.21 (dd, 2 H), 7.05-7.01 (t, 2 H), 4.25-3.90 (m, 2 H), 3.10-2.75 (td, 2 H), 2.38-2.34 (dd, 2 H), 2.05-1.97 (m, 2 H), 1.91-1.81 (m, 1 H), 1.43 (s, 9 H), 1.39-1.30 (m, 2 H), 0.74-0.70 (t, 3 H). $^{19}$F NMR (376.5 MHz, $CDCl_3$) $\delta_F$ -114.911.

Synthesis of A112

To a mixture of tert-butyl 4-cyano-4-(1-(4-fluorophenyl)propyl)piperidine-1-carboxylate (700 mg, 2.02 mmol) in dioxane (10 mL) was added HCl/dioxane (4 M, 10.1 mL, 40.4 mmol ), the mixture was stirred at 20°C for 16 hours. The reaction mixture was concentrated to give 4-(1-(4-fluorophenyl)propyl)piperidine-4-carbonitrile hydrochloride (550 mg). $^1$H NMR (400 MHz, $CDCl_3$) $\delta_H$ ppm 7.40-7.36 (dd, 2 H), 7.15-7.10 (t, 2 H), 3.59 (s, 1 H), 3.54-3.50 (m, 1 H), 3.41-3.37 (m, 1 H), 3.21-3.15 (td, 1 H), 3.10-3.02 (td, 1 H), 2.70-2.66 (dd, 1 H), 2.51-2.46 (m, 1 H), 2.13-2.03 (m, 1 H), 1.96-1.62 (m, 4 H), 0.78-0.74 (t, 3 H).

Synthesis of A113 and SFC Separation

To a solution of 2-(pyrimidin-4-yl)pyridine-3-carboxylic acid (500 mg, 2.48 mmol) , 4-(1-(4-fluorophenyl)propyl) piperidine-4-carbonitrile hydrochloride (525 mg, 1.86 mmol) and HATU (1.41 g, 3.72 mmol) in DMF (15 mL) was added DIPEA (1.58 g, 12.3 mmol). The mixture was stirred at 20°C for 2 hours. The reaction mixture poured into water (50 mL), and the aqueous layer was extracted with EtOAc (2 x 100 mL). The combined organic layers were washed with brine (100 mL), dried over anhydrous $Na_2SO_4$, and concentrated to give the product. The product was purified by prep-HPLC (Column:Phenomenex Gemini-NX 150*30mm*5um; Condition:water(0.04%NH3H2O+10mM NH4HCO3)-ACN; Begin B: 38%; End 68%) to give racemic-4-(1-(4-fluorophenyl)propyl)-1-(2-(pyrimidin-4-yl)nicotinoyl)piperidine-4-carbonitrile (300 mg, 28.3 % yield). $^1$H NMR (400 MHz, $CDCl_3$) $\delta_H$ 9.21 (s, 0.4 H), 8.93-8.85 (m, 1.6 H), 8.76-8.72 (m, 1 H), 8.27-8.22 (m, 1 H), 7.66-7.62 (m, 1 H), 7.47-7.41 (m, 1 H), 7.29-7.19 (m, 1.4 H), 7.19-7.14 (m, 0.6 H), 7.11-7.07 (m, 1 H), 7.02-6.98 (m, 1 H), 4.93-4.65 (m, 1 H), 4.05-3.95 (m, 0.1 H), 3.75-3.65 (m, 0.3 H), 3.50-3.25 (m, 1.6 H), 3.20-2.80 (m, 1.4 H), 2.50-2.25 (m, 1.6 H), 2.20-1.75 (m, 3 H), 1.55-1.45 (m, 1 H), 1.35-1.15 (m, 2 H), 0.80-0.70 (m, 3 H), which was further purified by SFC (Column:DAICEL CHIRALCEL OD(250mm*30mm,10um); Condition:0.1%NH3H2O ETOH; Begin B: 25%) to give:

(R)-4-(1-(4-fluorophenyl)propyl)-1-(2-(pyrimidin-4-yl)nicotinoyl)piperidine-4-carbonitrile (87.0 mg, 29%). $^1$H NMR (400 MHz, $CDCl_3$) $\delta_H$ 9.21 (s, 0.4 H), 8.93-8.85 (m, 1.6 H), 8.76-8.72 (m, 1 H), 8.27-8.22 (m, 1 H), 7.66-7.62 (m, 1 H), 7.47-7.41 (m, 1 H), 7.29-7.19 (m, 1.4 H), 7.19-7.14 (m, 0.6 H), 7.11-7.07 (m, 1 H), 7.02-6.98 (m, 1 H), 4.93-4.65 (m, 1 H), 4.05-3.95 (m, 0.1 H), 3.75-3.65 (m, 0.3 H), 3.50-3.25 (m, 1.6 H), 3.20-2.80 (m, 1.4 H), 2.50-2.25 (m, 1.6 H), 2.20-1.75 (m, 3 H), 1.55-1.45 (m, 1 H), 1.35-1.15 (m, 2 H), 0.80-0.70 (m, 3 H). $^{19}$F NMR (376.5 MHz, $CDCl_3$) $\delta_F$ -114.475. LC-ELSD/MS purity 100%, MS ESI calcd. for $C_{25}H_{24}FN_5O$ [M+H]+ 430.2 found 430.3. analytic SFC 98%de.

(S)-4-(1-(4-fluorophenyl)propyl)-1-(2-(pyrimidin-4-yl)nicotinoyl)piperidine-4-carbonitrile (102.5 mg, 34%). $^1$H NMR (400 MHz, $CDCl_3$) $\delta_H$ 9.21 (s, 0.4 H), 8.93-8.85 (m, 1.6 H), 8.76-8.72 (m, 1 H), 8.27-8.22 (m, 1 H), 7.66-7.62 (m, 1 H), 7.47-7.41 (m, 1 H), 7.29-7.19 (m, 1.4 H), 7.19-7.14 (m, 0.6 H), 7.11-7.07 (m, 1 H), 7.02-6.98 (m, 1 H), 4.93-4.65 (m, 1 H), 4.05-3.95 (m, 0.1 H), 3.75-3.65 (m, 0.3 H), 3.50-3.25 (m, 1.6 H), 3.20-2.80 (m, 1.4 H), 2.50-2.25 (m, 1.6 H), 2.20-1.75 (m, 3 H), 1.55-1.45 (m, 1 H), 1.35-1.15 (m, 2 H), 0.80-0.70 (m, 3 H). $^{19}$F NMR (376.5 MHz, $CDCl_3$) $\delta_F$ -114.466. LC-ELSD/MS purity 98%, MS ESI calcd. for $C_{25}H_{24}FN_5O$ [M+H]+ 430.2 found 430.3. analytic SFC 99%de.

*Example 44. Synthesis of 1-(2-(pyrimidin-4-yl)nicotinoyl)-4-(4-(trifluoromethoxy)benzyl)piperidine-4-carbonitrile (A217)*

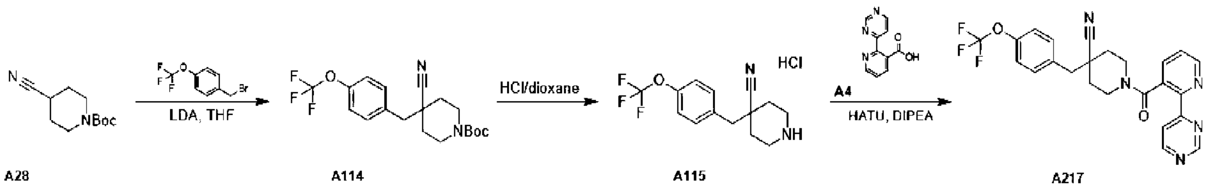


A28 A114 A115 A217

Synthesis of A114

To a mixture of tert-butyl 4-cyanopiperidine-1-carboxylate (5 g, 23.7 mmol) in THF (100 mL) was added dropwise LDA (17.7 mL, 35.5 mmol, 2 M in THF/n-heptane) at -78°C under nitrogen. After stirring at -78°C for 1 hour, the mixture was warmed to 20°C and 1-(bromomethyl)-4-(trifluoromethoxy) benzene (7.27 g, 28.5 mmol) was added and stirred for 16 hours. The reaction mixture was poured into $NH_4Cl$ solution (100 mL, sat.) and extracted with ethyl acetate (2 x 100 mL). The combined organic layers were washed with brine (100 mL), dried over $Na_2SO_4$, filtered and concentrated. The residue was purified by flash chromatography (0-30% of EtOAc in PE) to give tert-butyl 4-cyano-4-(4-(trifluoromethoxy)benzyl) piperidine-1-carboxylate (6 g, 65.8%). $^1$H NMR (400 MHz, $CDCl_3$) $\delta_H$ 7.29-7.23 (m, 2H), 7.16 (d, 2H), 4.08 (q, 2H), 3.01-2.90 (m, 2H), 2.82 (s, 2H), 2.05-1.92 (m, 1H), 1.86-1.74 (m, 2H), 1.65 (s, 1H), 1.43-1.40 (m, 9H).

Synthesis of A115

To a solution of tert-butyl 4-cyano-4-(4-(trifluoromethoxy)benzyl)piperidine-1-carboxylate (6 g, 15.8 mmol) in dioxane (63 mL) was added HCl/dioxane (4 M, 50 mL, 200 mmol) at 20°C. After stirring at 20°C for 16 hours, the reaction was concentrated to give 4-(4-(trifluoromethoxy)benzyl)piperidine-4-carbonitrile hydrochloride (3.28 g, 64.8 %). $^1$H NMR (400 MHz, $CDCl_3$) $\delta_H$ 10.04-9.60 (m, 2H), 7.33 (d, 2H), 7.22 (d, 2H), 3.61-3.52 (m, 2H), 3.21-3.09 (m, 3H), 2.95 (s, 2H), 2.22-2.12 (m, 2H), 2.10-2.02 (m, 2H). $^{19}$F NMR (377MHz, $CDCl_3$) $\delta_F$ -57.86.

Synthesis of A217

To a solution of 2-(pyrimidin-4-yl)nicotinic acid (200 mg, 0.994 mmol) , 4-(4-(trifluoromethoxy)benzyl)piperidine-4-carbonitrile hydrochloride (287 mg, 0.894 mmol) and HATU (566 mg, 1.49 mmol) in DMF (5 mL) was added DIPEA (641 mg, 4.97 mmol) . After stirring at 20°C for 2 hours, the reaction poured into water (50 mL) and extracted with EtOAc (2 x 100 mL). The combined organic layers were washed with brine (100 mL), dried over anhydrous $Na_2SO_4$, filtered and concentrated. The residue was purified by prep-HPLC (Column: Phenomenex Gemini-NX 150*30mm*5um; Condition: water (0.04%$NH_3H_2O$+10mM $NH_4HCO_3$)-ACN; Begin B: 40%; End 60%) to give 1-(2-(pyrimidin-4-yl)nicotinoyl)-4-(4-(trifluoromethoxy)benzyl)piperidine-4-carbonitrile (95 mg, 20%). $^1$H NMR (400 MHz, $CDCl_3$) $\delta_H$ 9.25-9.17 (m, 1H), 9.04 (s, 1H), 8.91 (br d, 1H), 8.76 (dd, 1H), 8.26 (br d, 1H), 7.72-7.62 (m, 1H), 7.47 (dd, 1H), 7.34-7.28 (m, 2H), 7.21 (s, 2H), 4.94-4.86 (m, 1H), 4.83-4.74 (m, 1H), 3.68-3.56 (m, 1H), 3.49-3.35 (m, 1H), 3.30-3.18 (m, 1H), 3.10 (br d, 1H), 2.92 (br d, 2H), 2.01 (br d, 1H), 1.83 (br d, 2H), 1.66 (br s, 1H), 1.49 (br d, 1H). $^{19}$F NMR (377 MHz, $CDCl_3$) $\delta_F$ -57.84. LC-ELSD/MS purity 100%, MS ESI calcd. for $C_{24}H_{20}F_3N_5O_2$ [M +H] 467.4 found 467.4.

*Example 45. Synthesis of 4-(3-cyclopropylbenzyl)-1-(2-(pyrimidin-4-yl)nicotinoyl)piperidine-4-carbonitrile (A218)*

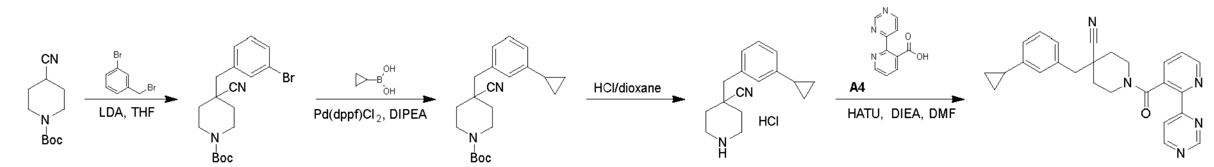


A28 A116 A117 A118 A218

Synthesis of A116

To a mixture of tert-butyl 4-cyanopiperidine-1-carboxylate (5 g, 23.7 mmol) in THF (100 mL) under nitrogen was added dropwise LDA (17.7 mL, 35.5 mmol, 2 M in THF/n-heptane) at -78°C. After stirring at -78°C for 1 hour, the mixture was warmed to 20°C and 1-bromo-3-(bromomethyl) benzene (7.09 g, 28.4 mmol) was added. After stirring at 20°C for 16 hours, the reaction mixture was poured into saturated $NH_4Cl$ solution (100 mL) and extracted with ethyl acetate (2 x 100 mL). The combined organic layers were washed with brine (100 mL), dried over $Na_2SO_4$, filtered and concentrated. The residue was purified by flash chromatography (0-30% of EtOAc in PE) to give tert-butyl 4-(3-bromobenzyl)-4-cyanopiperidine-1-carboxylate (5 g, 55.6%). $^1$H NMR (400 MHz, $CDCl_3$) $\delta_H$ 7.47-7.43 (m, 1H), 7.40 (s, 1H), 7.24 (s, 2H), 3.07-2.92 (m, 2H), 2.82 (s, 2H), 1.91-1.78 (m, 2H), 1.53-1.47 (m, 2H), 1.45 (s, 9H).

Synthesis of A117

A mixture of tert-butyl 4-(3-bromobenzyl)-4-cyanopiperidine-1-carboxylate (2 g, 5.27 mmol), cyclopropylboronic acid (1.35 g, 15.8 mmol), Pd (dppf) $Cl_2$ (430 mg, 0.527 mmol) and DIPEA (3.39 g, 26.3 mmol) in toluene (30 mL)/water (5 mL) was stirred under nitrogen at 100°C for 16 hours. The reaction mixture poured into water (50 mL) and extracted with EtOAc (2 x 20 mL). The combined organic layers were washed with brine (30 mL), dried over anhydrous $Na_2SO_4$, filtered and concentrated. The residue was purified by flash chromatography (0~10% of EA in PE) to give tert-butyl 4-cyano-4-(3-cyclopropylbenzyl)piperidine-1-carboxylate (1.21 g, 67.5 %). $^1$H NMR (400 MHz, $CDCl_3$) $\delta_H$ 7.24-7.18 (m, 1H), 6.97 (br s, 3H), 4.23-4.02 (m, 2H), 3.05-2.91 (m, 2H), 2.81 (s, 2H), 1.81 (br s, 1H), 1.65 (s, 2H), 1.45 (s, 12H), 1.01-0.92 (m, 2H), 0.74-0.66 (m, 2H).

Synthesis of A118

To a mixture of tert-butyl 4-cyano-4-(3-cyclopropylbenzyl)piperidine-1-carboxylate (1.19 g, 3.52 mmol) in

dioxane (15 mL) was added HCl/dioxane (4 M, 17.6 mL, 70.4 mmol ) at 20°C. After stirring at 20 °C for 16 hours, the reaction mixture was filtered. The filter cake was washed with of EtOAc (5 mL) and dried to give 4-(3-cyclopropylbenzyl) piperidine-4-carbonitrile hydrochloride (1.2 g, 77.4%). $^1$H NMR (400 MHz, $CDCl_3$) $\delta_H$ 9.81 (br s, 2H), 7.25-7.20 (m, 1H), 7.08-6.97 (m, 3H), 3.55 (br d, 2H), 3.16 (br d, 2H), 2.89 (s, 2H), 2.19-1.95 (m, 4H), 1.87 (dt, 1H), 1.01-0.91 (m, 2H), 0.76-0.63 (m, 2H).

Synthesis of A218

To a solution of 2-(pyrimidin-4-yl)nicotinic acid (200 mg, 0.994 mmol), 4-(3-cyclopropylbenzyl)piperidine-4-carbonitrile hydrochloride (247 mg, 0.892 mmol) and HATU (566 mg, 1.49 mmol) in DMF (5 mL) was added DIPEA (641 mg, 4.97 mmol) . After stirring at 20 °C for 2 hours, the reaction mixture poured into water (5 mL). The aqueous layer was extracted with EtOAc (2 x 10 mL) and the combined organic layers were washed with brine (10 mL), dried over anhydrous $Na_2SO_4$, filtered and concentrated. The residue was purified by prep-HPLC (Column: Phenomenex Gemini-NX 150*30mm*5um; Condition: water (0.04%$NH_3H_2O$+10mM NH4HCO3)-ACN; Begin B: 37%; End 67%) to give 4-(3-cyclopropylbenzyl)-1-(2-(pyrimidin-4-yl)nicotinoyl)piperidine-4-carbonitrile (20 mg, 4.76 %). $^1$H NMR (400 MHz, $CDCl_3$) $\delta_H$ 8.87 (d, 1H), 8.75 (br d, 1H), 8.60 (s, 1H), 8.22 (br s, 1H), 7.64 (s, 1H), 7.46 (dd, 1H), 7.22 (br d, 1H), 7.07-6.91 (m, 3H), 4.93-4.73 (m, 1H), 3.69-3.56 (m, 1H), 3.49-3.35 (m, 1H), 3.11 (br d, 1H), 2.95-2.84 (m, 2H), 2.00 (br d, 1H), 1.86 (br d, 3H), 1.66 (br s, 1H), 0.95 (br d, 2H), 0.73-0.65 (m, 2H). LC-ELSD/MS purity 100%, MS ESI calcd. for C26H25N5O [M +Na]+ 446.3found 446.3.

*Example 46. Synthesis of 1-(4-fluoro-2-(pyrimidin-4-yl)benzoyl)-4-(4-fluorobenzyl)piperidine-4-carbonitrile (A220)*

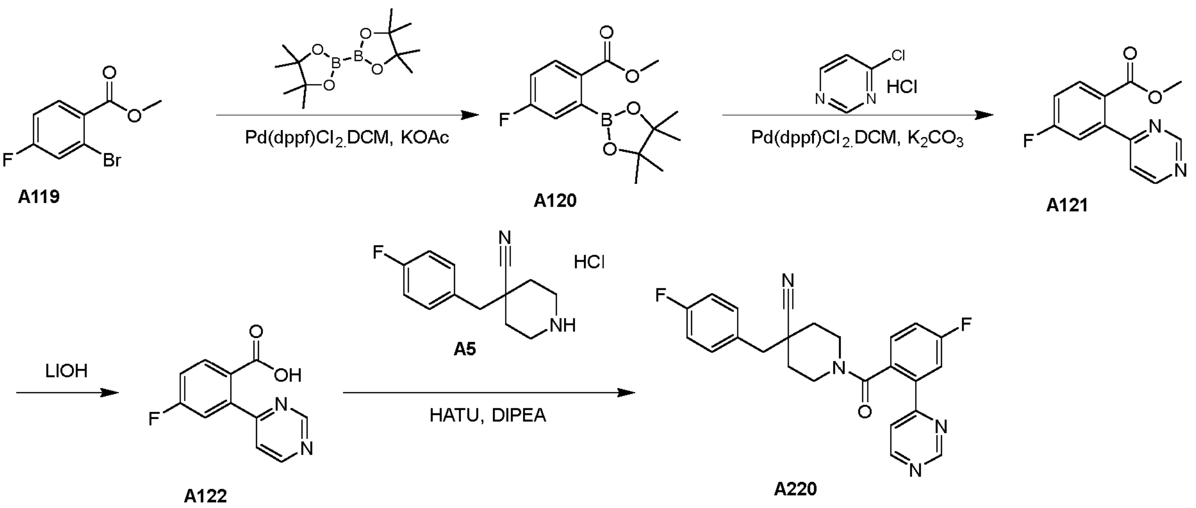

A119 A120 A121 A5 A122 A220

Synthesis of A120

A mixture of methyl 2-bromo-4-fluorobenzoate (2 g, 8.58 mmol), 4,4,5,5-tetramethyl-2-(4,4,5,5-tetramethyl-1,3,2-dioxaborolan-2-yl)-1,3,2-dioxaborolane (2.59 g, 10.2 mmol), Pd(dppf)$Cl_2$ (350 mg, 0.4290 mmol) and AcOK (849 mg, 8.58 mmol) in dioxane (30 mL) was stirred under nitrogen at 100°C for 16 hours. The reaction mixture poured into water (50 mL), and the aqueous layer was extracted with EtOAc (2 x 20 mL). The combined organic layers were washed with brine (30 mL), dried over anhydrous $Na_2SO_4$, and concentrated to give the product. The product was purified by flash chromatography (0~10% of EA in PE) to give methyl 4-fluoro-2-(4,4,5,5-tetramethyl-1,3,2-dioxaborolan-2-yl)benzoate (1.7 g, 70.8 %). $^1$H NMR (400 MHz, $CDCl_3$) $\delta_H$ 7.93-7.85 (m, 1H), 7.08-7.05 (m, 1H), 7.04-6.96 (m, 1H), 3.89-3.79 (m, 3H), 1.55-1.50 (m, 4H), 1.41-1.31 (m, 13H).

Synthesis of A121

A mixture of methyl 4-fluoro-2-(4,4,5,5-tetramethyl-1,3,2-dioxaborolan-2-yl)benzoate (500 mg, 1.78 mmol),4-chloropyrimidine hydrochloride (241 mg, 1.60 mmol), Pd(dppf)$Cl_2$.DCM (205 mg, 0.1780mmol) and $K_2CO_3$ (1.22 g, 8.90 mmol) in dioxane (10 mL)/water (2.5 mL) was stirred under nitrogen at 100°C for 3 hours. The reaction mixture poured into water (50 mL), and the aqueous layer was extracted with EtOAc (2 x 20 mL). The combined organic layers were washed

with brine (30 mL), dried over anhydrous $Na_2SO_4$, and concentrated. The product was purified by flash chromatography (0~10% of EA in PE) to give methyl 4-fluoro-2-(pyrimidin-4-yl)benzoate (330 mg, 82.7 %). $^1H$ NMR (400 MHz, $CDCl_3$) $\delta_H$ 10.42-10.33 (m, 1H), 8.93-8.65 (m, 1H), 8.42 - 7.82 (m, 1H), 7.37-7.27 (m, 1H), 7.24-7.06 (m, 2H), 3.78-3.52 (m, 3H).

Synthesis of A122

To a solution of methyl 4-fluoro-2-(pyrimidin-4-yl)benzoate (330 mg, 1.42 mmol) in MeOH (10 mL) was added LiOH·$H_2O$ (65.5 mg, 1.56 mmol) at 25°C. The mixture was warmed to 60°C and stirred under nitrogen for 12 hours. The reaction mixture was concentrated to give 4-fluoro-2-(pyrimidin-4-yl)benzoic acid (310 mg). $^1H$ NMR (400 MHz, $CDCl_3$) $\delta_H$ 9.19-9.16 (m, 1H), 8.75-8.72 (m, 1H), 7.82-7.76 (m, 1H), 7.74-7.68 (m, 1H), 7.45-7.39 (m, 1H), 7.26-7.20 (m, 2H).

Synthesis of A220

To a solution of 4-fluoro-2-(pyrimidin-4-yl)benzoic acid (100 mg, 0.458 mmol) in DMF (1 mL) was added HATU (261 mg, 0.687 mmol) and DIPEA (0.239 mL, 1.37mmol) at 25°C, After stirring for 30 minutes at 25°C, 4-[(4-fluorophenyl) methyl]piperidine-4-carbonitrile hydrochloride (116 mg, 0.458 mmol) was added, and the mixture was stirred for 10 hours at 25°C. The mixture was concentrated, and the residue was purified by prep.HPLC (Column:Phenomenex Gemini-NX 150*30mm*5um; Condition:water(0.225%FA)-ACN; Begin B:35, End B:65; Gradient Time(min):3; 100%B Hold Time(min):2; FlowRate(ml/min):30; Injections:7) and lyophilized to give 1-(4-fluoro-2-(pyrimidin-4-yl)benzoyl)-4-(4-fluorobenzyl)piperidine-4-carbonitrile (20.3 mg, 10%). $^1H$ NMR (400 MHz, $CDCl_3$) $\delta_H$ 9.23 (s, 0.34H), 9.01 (s, 0.58H), 8.87-8.72 (m, 1H), 7.62-7.51 (m, 1H), 7.50-7.40 (m, 1H), 7.39-7.30 (m, 1H), 7.26-7.13 (m, 3H), 7.05-6.95 (m, 2H), 4.80-4.60 (m, 1H), 3.66-3.60 (m, 0.63H), 3.55-3.45 (m, 0.38H), 3.45-3.25 (m, 0.52H), 3.13-2.95 (m, 1H), 2.90-2.72 (m, 2H), 2.05-1.95 (m, 1H), 1.75-1.65 (m, 1H), 1.50-1.43 (m, 3H). $^{19}F$ NMR (376.5 MHz, $CDCl_3$) $\delta_F$ -110.204, $\delta_F$ -114.432. LC-ELSD/MS purity 99%, MS ESI calcd. for $C_{24}H_{21}F_2N_4O$ $[M+H]^+$ 419.3, found 419.3.

*Example 47. Synthesis of 1-(2-fluoro-6-(pyrimidin-4-yl)benzoyl)-4-(4-fluorobenzyl)piperidine-4-carbonitrile (A221)*

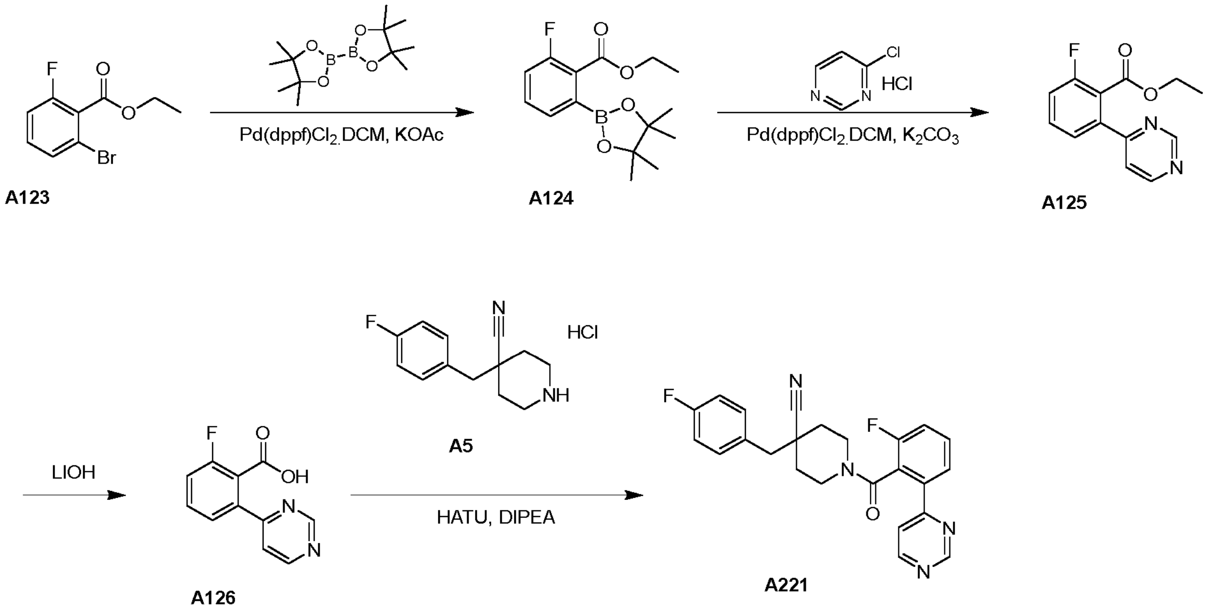


A123 A124 A125 A5 A126 A221

Synthesis of A124

A mixture of ethyl 2-bromo-6-fluorobenzoate (2 g, 8.09 mmol), 4,4,5,5-tetramethyl-2-(4,4,5,5-tetramethyl-1,3,2-dioxaborolan-2-yl)-1,3,2-dioxaborolane (2.46 g, 9.70 mmol), Pd(dppf)$Cl_2$ (329 mg, 0.404 mmol) and AcOK (2.39 g, 24.2 mmol) in dioxane (30 mL) was stirred under nitrogen at 100°C for 16 hours. The reaction mixture poured into water (50 mL) and extracted with EtOAc (2 x 20 mL). The combined organic layers were washed with brine (30 mL), dried over anhydrous $Na_2SO_4$, filtered and concentrated. The residue was purified by flash chromatography (0~10% of EA in PE) to give ethyl 2-fluoro-6-(4,4,5,5-tetramethyl-1,3,2-dioxaborolan-2-yl)benzoate (1.41 g, 59.4 %). $^1H$ NMR (400 MHz, $CDCl_3$) $\delta_H$ 7.43-7.39

(m, 2H), 7.12 (ddd, 1H), 4.44-4.33 (m, 2H), 1.38 (t, 3H), 1.35 (s, 12H).

Synthesis of A125

A mixture of ethyl 2-fluoro-6-(4,4,5,5-tetramethyl-1,3,2-dioxaborolan-2-yl)benzoate (500 mg, 1.69 mmol), 4-chloropyrimidine hydrochloride (229 mg, 1.52 mmol), Pd (dppf) $Cl_2$ (124 mg, 0.169 mmol) and $K_2CO_3$ (1.16 g, 8.45 mmol) in dioxane (10 mL)/water (2.5 mL) was stirred under nitrogen at 100°C for 3 hours. The reaction was poured into water(50 mL) and extracted with EtOAc (2 x 20 mL). The combined organic layers were washed with brine (30 mL), dried over anhydrous $Na_2SO_4$, filtered and concentrated. The residue was purified by flash chromatography (0~10% of EA in PE) to give ethyl 2-fluoro-6-(pyrimidin-4-yl)benzoate (170 mg, 40.8 %). $^1$H NMR (400 MHz, $CDCl_3$) $\delta_H$ 9.21 (s, 1H), 8.80 (d, 1H), 7.59 (d, 1H), 7.48 (s, 2H), 7.26 (s, 1H), 4.30 (d, 2H), 1.24 (d, 2H), 1.20 (s, 1H).

Synthesis of A126

To a mixture of ethyl 2-fluoro-6-(pyrimidin-4-yl)benzoate (170 mg, 0.69 mmol) in MeOH (5 mL)/ water (0.5 mL) was added LiOH·$H_2O$ (31.8 mg, 0.759 mmol). After stirring at 60°C for 16 hours, the reaction was concentrated to give 2-fluoro-6-(pyrimidin-4-yl)benzoic acid (140 mg, 93.3 %). $^1$H NMR (400 MHz, $CDCl_3$) $\delta_H$ 13.13 (s, 1H), 12.71 (d, 1H), 12.01-11.85 (m, 1H), 11.57 (s, 1H), 11.66-11.52 (m, 1H), 11.43-11.31 (m, 1H), 11.21 (s, 1H).

Synthesis of A221

To a solution of 2-fluoro-6-(pyrimidin-4-yl)benzoic acid (130 mg, 0.595 mmol), 4-[(4-fluorophenyl)methyl]piperidine-4-carbonitrile hydrochloride (136 mg, 0.535 mmol) and HATU (338 mg, 0.892 mmol) in DMF (5 mL) was added DIPEA (383 mg, 2.97 mmol) . After stirring at 20°C for 2 hours, the reaction mixture was poured into water (50 mL) and extracted with EtOAc (2 x 100 mL). The combined organic layers were washed with brine (100 mL), dried over anhydrous $Na_2SO_4$, filtered and concentrated. The residue was purified by Prep-HPLC (Column: Venusil ASB Phenyl 150*30mm*5um; Condition: water (0.05%HCl) -ACN; Begin B: 50%; End 80%) to give 1-(2-fluoro-6-(pyrimidin-4-yl) benzoyl)-4-(4-fluorobenzyl)piperidine-4-carbonitrile (9.7 mg, 3.9%). $^1$H NMR (400 MHz, $CDCl_3$) $\delta_H$ 9.33-9.20 (m, 1H), 9.08 (br s, 1H), 8.88 (br s, 1H), 7.76 (br s, 1H), 7.52 (br s, 2H), 7.50-7.26 (m, 2H), 7.05 (br t, 2H), 4.88-4.65 (m, 1H), 3.67 (s, 1H), 3.52-3.27 (m, 1H), 3.19-2.95 (m, 1H), 2.95-2.80 (m, 2H), 1.86 (br d, 2H), 1.78-1.54 (m, 2H). LC-ELSD/MS purity 100%, MS ESI calcd. for C24H20F2N4O [M+H]+ 419.0 found 419.0. $^{19}$F NMR (376.5 MHz, $CDCl_3$) $\delta_F$ -113.89.

*Example 48. Synthesis of 1-(2-(pyrimidin-4-yl)nicotinoyl)-4-((6-(trifluoromethyl)pyridin-3-yl)methyl)piperidine-4-carbonitrile (A222)*

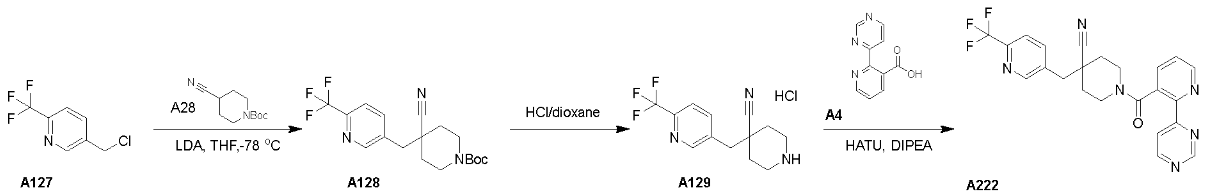

A127 A128 A129 A222

Synthesis of A128

To a mixture of tert-butyl 4-cyanopiperidine-1-carboxylate (300 mg, 1.42 mmol) in THF (10 mL) was added dropwise LDA (1.18 mL, 2 M, 2.36 mmol) at -78°C. After stirring at -78°C for 1 hour, 5-(chloromethyl)-2-(trifluoromethyl) pyridine (307 mg, 1.57 mmol) was added. The mixture was warmed to 20°C and stirred for 16 hours. The mixture was poured into water (25 mL) and extracted with EtOAc (3 x 20 mL). The combined organic layers were washed with brine (2 x 50 mL), dried over $Na_2SO_4$, filtered and concentrated. The residue was purified by flash chromatography (10~30% of EtOAc in PE) to give tert-butyl 4-cyano-4-((6-(trifluoromethyl)pyridin-3-yl)methyl)piperidine-1-carboxylate (200 mg, 34.5%). $^1$H NMR (400 MHz, $CDCl_3$) $\delta_H$ 8.60 (d, 1H), 7.90 (dd, 1H), 7.71 (d, 1H), 4.28-4.06 (m, 2H), 2.95 (s, 4H), 1.85 (d, 2H), 1.60-1.53 (m, 5H), 1.49-1.43 (m, 9H).

Synthesis of A129

To a solution of tert-butyl 4-cyano-4-((6-(trifluoromethyl)pyridin-3-yl)methyl)piperidine-1-carboxylate (200 mg,0.54 mmol) in dioxane (2 mL) was added HCl/dioxane (3 mL, 4M, 12.0 mmol ) at 25°C, and the mixture was stirred under nitrogen for 12 hours. The mixture was concentrated to give 4-((6-(trifluoromethyl)pyridin-3-yl)methyl)piperidine-4-carbonitrile hydrochloride (200 mg), which was used without further purification.

Synthesis of A222

To a solution of 2-(pyrimidin-4-yl)nicotinic acid (78.9 mg, 0.39 mmol) in DMF (2 mL) was added HATU (186 mg, 0.49 mmol) and DIPEA (0.17 mL, 0.981 mmol) at 25°C. After stirring for 30 minutes at 25°C, 4-((6-(trifluoromethyl)pyridin-3-yl)methyl)piperidine-4-carbonitrile hydrochloride (100 mg, 0.33 mmol) was added to the solution. The mixture was stirred for 2 hours at 25°C, poured into water (20 mL), and extracted with EtOAc (3 x 10 mL). The combined organic layers were washed with brine (2 x 10 mL), dried over $Na_2SO_4$, filtered and concentrated. The residue was purified by prep-HPLC (Column Welch Xtimate C18 150*25 mm*5 um, Condition water (0.05%$NH_3H_2O$)-ACN, Begin B: 30, End B: 60, Gradient Time (min) 8, 100%B Hold Time (min):2) and lyophilized to give 1-(2-(pyrimidin-4-yl)nicotinoyl)-4-((6-(trifluoromethyl)pyridin-3-yl)methyl)piperidine-4-carbonitrile (21.1 mg, 14%). $^1$H NMR (400MHz, $CDCl_3$) $\delta_H$ 9.21 (s, 0.4H), 9.12 (s, 0.6H), 8.97-8.85 (m, 1H), 8.80-8.73 (m, 1H), 8.60 (s, 1H), 8.32-8.24 (m, 1H), 7.98-7.82 (m, 1H), 7.76-7.63 (m, 2H), 7.52-7.43 (m, 1H), 4.99-4.89 (m, 0.5H), 4.87-4.76 (m, 0.5H), 3.69-3.57 (m, 0.5H), 3.53-3.32 (m, 1H), 3.29-3.18 (m, 0.5H), 3.17-2.88 (m, 3H), 2.18-1.99 (m, 1H), 1.92-1.61 (m, 3H).LC-ELSD/MS purity 100%, MS ESI calcd. For $C_{23}H_{20}F_3N_6O$ $[M+H]^+$ 453, found 453.

*Example 49. Synthesis of 4-(4-(2-hydroxypropan-2-yl)benzyl)-1-(2-(pyrimidin-4-yl)nicotinoyl)piperidine-4-carbonitrile (A223)*

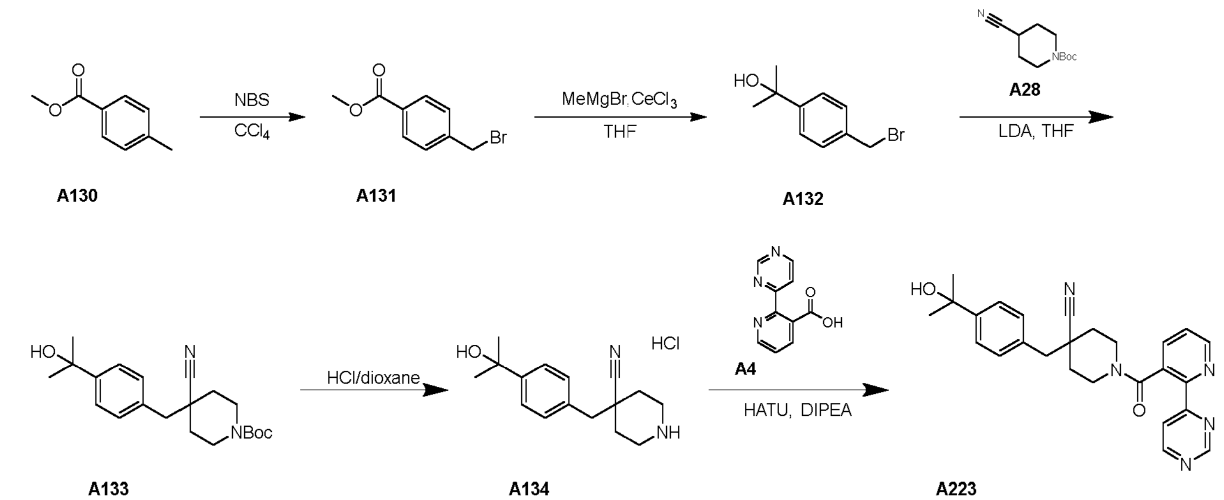

A130 A131 A132 A133 A134 A223

Synthesis of A131

To a solution of methyl 4-methylbenzoate (5 g, 33.2 mmol) in MeOH (80 ml) was added NBS (11.8 g, 66.4 mmol) and BPO (1.6 g, 6.64 mmol) at 25°C. The mixture was stirred at 25°C for 2 hours. The mixture was quenched by sat.aq $NaHCO_3$ (200 mL), treated with water (100 mL), and extracted with EtOAc (2 x 200 mL). The combined organic layers were washed with brine (100 mL), dried over anhydrous $Na_2SO_4$, filtered, concentrated to afford methyl 4-(bromomethyl)benzoate (7 g).

Synthesis of A132

To a solution of bromo(methyl)magnesium (30.5 mL, 3M in diethyl ether, 91.5 mmol) was added methyl 4-(bromomethyl)benzoate (7 g, 30.5 mmol) in THF (100 mL) in one portion at 25°C. The mixture was stirred at 25°C for 2 hours. The mixture was poured into $NH_4Cl$ solution (200 mL) and extracted with EtOAc (3 x 200 mL). The combined organic layers were washed with brine (2 x 100 mL), dried over anhydrous $Na_2SO_4$, filtered and concentrated to give

2-(4-(bromomethyl)phenyl)propan-2-ol (6.9 g).

Synthesis of A133

To a mixture of tert-butyl 4-cyanopiperidine-1-carboxylate (2.73 g, 13 mmol) in THF (30 mL) was added dropwise LDA (6.5 mL, 2 M, 13 mmol) at -78°C, and the mixture was stirred at -78°C for 1 hour. Then 2-(4-(bromomethyl)phenyl) propan-2-ol (2 g, 8.72 mmol) was added and the mixture was warmed to 20°C and stirred for 16 hours. The mixture was poured into water (100 mL), extracted with EtOAc (3 x 100 mL). The combined organic layers were washed with brine (2 x 100 mL), dried over $Na_2SO_4$, filtered and concentrated to give tert-butyl 4-cyano-4-(4-(2-hydroxypropan-2-yl)benzyl) piperidine-1-carboxylate (1.5 g).

Synthesis of A134

To a solution of tert-butyl 4-cyano-4-(4-(2-hydroxypropan-2-yl)benzyl)piperidine-1-carboxylate1.5 g, 4.18 mmol) in dioxane (30 mL) was added HCl (4 M in dioxane, 2.09 mL). The mixture was stirred at 25°C for 16 hrs. The reaction mixture was concentrated to give 4-(4-(2-hydroxypropan-2-yl)benzyl)piperidine-4-carbonitrile hydrochloride (1.2 g).

Synthesis of A223

To a solution of 2-(pyrimidin-4-yl)pyridine-3-carboxylic acid (508 mg, 2.53 mmol) in DMF (15 mL) at 15°C were added HATU (962 mg, 2.53 mmol) and DIPEA (653 mg, 5.06 mmol). After stirring at 15°C for 10 minutes, 4-(4-(2-hydroxypropan-2-yl)benzyl)piperidine-4-carbonitrile hydrochloride (500 mg, 1.69 mmol) was added to the above reaction mixture. The mixture was stirred at 15°C for 2 hrs. Water (100 mL) was added, and the aqueous layer was extracted with EtOAc (3 x 100 mL). The combined organic layers were washed with water (3 x 100 mL) and brine (100 mL), dried over anhydrous $Na_2SO_4$, filtered and concentrated to afford a product (300 mg). The residue was purified by HPLC (Column: Phenomenex Gemini-NX 80*30mm*3μm); Condition: water (10 mM $NH_4HCO_3$)-ACN; Begin B: 21%; End B: 51%) to afford 4-(4-(2-hydroxypropan-2-yl)benzyl)-1-(2-(pyrimidin-4-yl)nicotinoyl)piperidine-4-carbonitrile (92.5 mg, 31%). $^1H$ NMR (400 MHz, $CDCl_3$) $\delta_H$ 8.65-8.74 (m, 2 H), 8.22 (d, 1 H), 7.61 (d, 1 H), 7.55 (d, 2 H), 7.46-7.41 (m, 1 H), 7.03-7.12 (m, 3 H), 4.97-4.90 (m, 1 H), 4.84 (s, 1 H), 3.42-3.57 (m, 2 H), 3.06-3.21 (m, 3 H), 2.05-1.95 (m, 1 H), 1.79-1.94 (m, 2H), 1.70 (s, 3 H), 1.59-1.63 (m, 1 H), 1.54 (s, 3 H). LCMS purity 99%, MS ESI calcd. for C26H25N5O [M-$H_2O$+H]$^+$ 424.2, found 424.2.

*Example 50. Synthesis of 4-(naphthalen-2-ylmethyl)-1-(2-(pyrimidin-4-yl)nicotinoyl)piperidine-4-carbonitrile (A224)*

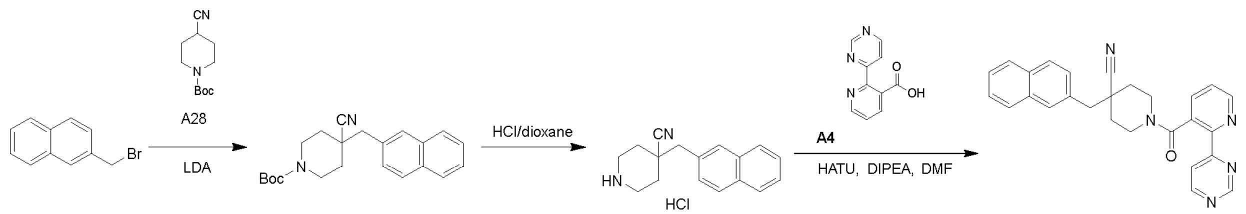


A135 A136 A137 A224

Synthesis of A136

To a mixture of tert-butyl 4-cyanopiperidine-1-carboxylate (500 mg, 2.37 mmol) in THF (20 mL) was added dropwise LDA (1.77 mL, 3.55 mmol, 2 M in THF/n-heptane) at - 78°C, the mixture was stirred under nitrogen at -78°C for 1 hour, and then 2-(bromomethyl)naphthalene (627 mg, 2.84 mmol) was added at -78°C. The mixture was allowed to warm to 20°C and stirred under nitrogen for 16 hours. The reaction mixture was poured into saturated $NH_4Cl$ solution (50 mL) and extracted with ethyl acetate (2 x 50 mL). The combined organic layers were washed with brine (50 mL) and dried over $Na_2SO_4$. The product was purified by column chromatography (0-5% EtOAc in PE) to give tert-butyl 4-cyano-4-(naphthalen-2-ylmethyl)piperidine-1-carboxylate (630 mg). $^1H$ NMR (400MHz, $CDCl_3$) $\delta_H$ 7.86 - 7.80 (m, 3H), 7.72 (s, 1H), 7.52 - 7.46 (m, 2H), 7.45 - 7.40 (m, 2H), 4.18 - 4.06 (m, 4H), 3.04 (s, 4H), 1.88 (br d, 2H), 1.48 - 1.39 (m, 11H).

Synthesis of A137

To a mixture of tert-butyl 4-carboximidoyl-4-[(naphthalen-2-yl)methyl]piperidine-1-carboxylate (630 mg, 1.78 mmol) in dioxane (20 mL) was added HCl/dioxane (4 M, 4.45 mL, 17.8 mmol ), the mixture was stirred at 20°C. The mixture was stirred for 16 hours at 20°C. The reaction mixture was filtered, and the filter cake was washed with 50 mL of EtOAc and dried to give 4-(naphthalen-2-ylmethyl)piperidine-4-carbonitrile hydrochloride (247 mg, 48%). $^1$H NMR (400MHz, $CD_3OD$) $\delta_H$ 7.94 - 7.80 (m, 4H), 7.55 - 7.45 (m, 3H), 3.49 (br d, 2H), 3.23 - 3.09 (m, 4H), 2.18 (br d, 1H), 2.23 - 2.12 (m, 1H), 1.98 (br dd, 2H).

Synthesis of A224

To a solution of 2-(pyrimidin-4-yl)pyridine-3-carboxylic acid (247 mg, 1.23 mmol) and HATU (703 mg, 1.85 mmol) in DMF (50 mL) was added DIPEA (478 mg, 3.71 mmol) . The mixture was stirred at 15 °C for 10 minutes and then 4-(naphthalen-2-ylmethyl)piperidine-4-carbonitrile hydrochloride (247 mg, 0.8673 mmol) was added. The mixture was stirred at 15°C for 16 hours. The reaction mixture poured into water (100 mL), and the aqueous layer was extracted with EtOAc (3 x 100 mL). The combined organic layers were washed with brine (2 x 100 mL), dried over anhydrous $Na_2SO_4$, and concentrated. The residue was purified by HPLC to give 4-(naphthalen-2-ylmethyl)-1-(2-(pyrimidin-4-yl)nicotinoyl)piperidine-4-carbonitrile (160.6 mg, 30%). $^1$H NMR (400MHz, $CDCl_3$) $\delta_H$ 8.93 (br d, 2H), 8.80 (br d, 1H), 8.41 (br s, 1H), 7.93 - 7.79 (m, 3H), 7.78 - 7.69 (m, 2H), 7.58 - 7.53 (m, 1H), 7.50 (dd, 2H), 7.41 (br d, 1H), 4.84 (br s, 1H), 3.68 - 3.54 (m, 1H), 3.43 (br s, 1H), 3.21 - 3.03 (m, 3H), 2.10 (br s, 1H), 1.96 - 1.69 (m, 3H), 1.54 - 1.44 (m, 1H). LC-ELSD/MS: purity 95%; MS ESI calcd. for $C_{27}H_{23}N_5O$ $[M+H]^+$ 434.2, found 434.0.

*Example 51. Synthesis of 4-(naphthalen-1-ylmethyl)-1-(2-(pyrimidin-4-yl)nicotinoyl)piperidine-4-carbonitrile (A225)*

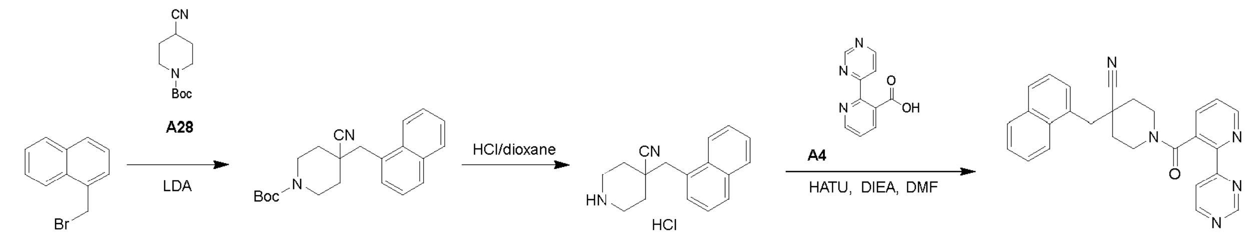

A138 A139 A140 A225

Synthesis of A139

To a mixture of tert-butyl 4-cyanopiperidine-1-carboxylate (2 g, 9.51mmol) in THF (40 mL) was added dropwise LDA (7.10 mL, 14.2 mmol, 2 M in THF/n-heptane) at -78°C, and the mixture was stirred under nitrogen at -78°C for 1 hour. Then 1-(bromomethyl)naphthalene (2.52 g, 11.4 mmol) was added at -78°C, and the mixture was warmed to 20°C and stirred under nitrogen for 16 hours. The reaction mixture was poured into saturated $NH_4Cl$ solution (50 mL), The aqueous layer was extracted with ethyl acetate (2 x 50 mL). The combined organic layers were washed with brine (50 mL x 2) and dried over $Na_2SO_4$. The product was purified by column (0-10% EtOAc in PE) to give tert-butyl 4-cyano-4-(naphthalen-1-ylmethyl)piperidine-1-carboxylate (1.89 g, 5.39 mmol, 56.7%). $^1$H NMR (400MHz, $CDCl_3$) $\delta_H$ 8.05 (d, 1H), 7.91-7.79 (m, 2H), 7.57-7.44 (m, 4H), 4.12 (q, 3H), 3.39 (s, 2H), 2.98 (br s, 2H), 1.86 (br d, 2H), 1.62 (br d, 2H), 1.45 (s, 9H).

Synthesis of A140

To a mixture of tert-butyl 4-cyano-4-[(naphthalen-1-yl)methyl]piperidine-1-carboxylate (1.89 g, 5.39 mmol) in dioxane (40 mL) was added HCl/dioxane (4 M, 13.4 mL, 53.9 mmol ), and the mixture was stirred at 20°C for 16 hours. The reaction mixture was filtered, and the filter cake was washed with EtOAc (50 mL) and dried to give 4-(naphthalen-1-ylmethyl)piperidine-4-carbonitrile hydrochloride (660 mg, 49.2%). $^1$H NMR (400MHz, $CD_3OD$) $\delta_H$ (d, 1H), 7.90 (m, 2H), 7.61-7.46 (m, 4H), 3.58 (s, 2H), 3.47 (d, 2H), 3.21-3.09 (m, 2H), 2.24-2.14 (m, 2H), 2.07-1.96 (m, 2H).

Synthesis of A225

To a solution of 2-(pyrimidin-4-yl)pyridine-3-carboxylic acid ( 301 mg, 1.50 mmol) and HATU (855 mg, 2.25 mmol)

in DMF (50 mL) was added DIPEA (580 mg, 4.50 mmol). The mixture was stirred at 15 °C for 10 minutes and then 4-(naphthalen-1-ylmethyl)piperidine-4-carbonitrile hydrochloride (300 mg, 1.05 mmol) was added. The mixture was stirred at 15 °C for 16 hours. The reaction mixture poured into water(100 mL), and the aqueous layer was extracted with EtOAc (3 x 100 mL). The combined organic layers were washed with brine (2 x 100 mL), dried over anhydrous $Na_2SO_4$, and concentrated. The residue was purified by HPLC (Column: Phenomenex Gemini-NX 80*40mm*3um,Condition: water(0.05%NH3H2O+10mM $NH_4HCO_3$)-ACN,Begin: B 33,End: B 57 ,Gradient Time(min): 9, 100%B Hold Time(min): 4, FlowRate(ml/min): 30, Injections: 7.) to afford 4-(naphthalen-1-ylmethyl)-1-(2-(pyrimidin-4-yl)nicotinoyl)piperidine-4-carbonitrile (84.7 mg, 13 %). $^1$H NMR (400MHz, $CDCl_3$) $\delta_H$ 9.20 (s, 1H), 8.86 (d, 1H), 8.73 (d, 1H), 8.47 (s, 1H), 8.30-8.14 (m, 1H), 8.09-7.96 (m, 1H), 7.94-7.79 (m, 2H), 7.71-7.59 (m, 1H), 7.57-7.38 (m, 5H), 4.94-4.67 (m, 1H), 3.63-3.31 (m, 4H), 3.27-2.95 (m, 1H), 2.07 (d, 1H), 1.96-1.84 (m, 1H), 1.79 (d, 1H), 1.67 (d, 1H) LCMS: purity 99%; MS ESI calcd. for $C_{27}H_{23}N_5O$ $[M+Na]^+$ 456.2, found 456.0 and MS ESI calcd. for $C_{27}H_{23}N_5O$ $[M+H]^+$ 434.2, found 434.0.

*Example 52. Synthesis of 4-((5-cyclopropylpyridin-2-yl)methyl)-1-(2-(pyrimidin-4-yl)nicotinoyl)piperidine-4-carbonitrile (A226)*

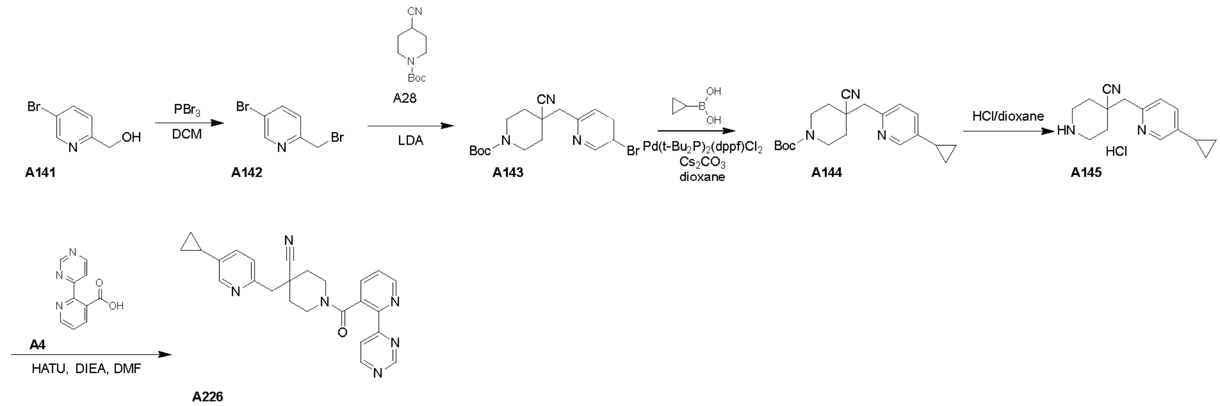


Synthesis of A142

To a solution of (5-bromopyridin-2-yl)methanol (3 g, 15.9 mmol) in DCM under nitrogen was added $PBr_3$ (10.7 g, 39.7 mmol) at 0°C. After stirring at 25°C for 2 hours, saturated $NH_4Cl$ (20 mL) was added. The mixture was extracted with DCM (2 x 50 mL). The combined organic layers were dried over $Na_2SO_4$, filtered and concentrated to give 5-bromo-2-(bromomethyl)pyridine (1.6 g, 40%). $^1$H NMR (400 MHz, $CDCl_3$) $\delta_H$ 8.55 (d, 1H), 7.74 (dd, 1H), 7.27 (d, 1H), 4.43 (s, 2H).

Synthesis of A143

To a solution of tert-butyl 4-cyanopiperidine-1-carboxylate (1.11 g, 5.30 mmol) in THF (10 mL) under nitrogen was added dropwise LDA (3.98 mL, 2 M, 7.96 mmol) at -78°C. After stirring at -78°C for 1 hour, 5-bromo-2-(bromomethyl) pyridine (1.6 g, 6.37 mmol) in THF (30 mL) was added. After stirring at 25°C for 16 hours, the mixture was poured into water (25 mL) and extracted with EtOAc (3 x 20 mL). The combined organic layers were washed with brine (2 x 50 mL), dried over $Na_2SO_4$, filtered and concentrated. The residue was purified by flash chromatography (10~30% of EtOAc in PE) to give tert-butyl 4-((5-bromopyridin-2-yl)methyl)-4-cyanopiperidine-1-carboxylate (1.1 g, 55%). $^1$H NMR (400 MHz, $CDCl_3$) $\delta_H$ 8.64-8.62 (m, 1H), 7.83-7.79 (m, 1H), 7.28-7.25 (m, 1H), 4.25-4.00 (m, 3H), 3.10-2.92 (m, 3H), 1.91-1.86 (m, 2H), 1.65-1.56 (m, 2H), 1.45 (s, 9H).

Synthesis of A144

A mixture of tert-butyl 4-((5-bromopyridin-2-yl)methyl)-4-cyanopiperidine-1-carboxylate (1.1 g, 2.89 mmol), cyclopropyl boronic acid (744 mg, 8.67 mmol), $Pd(t\text{-}Bu_2P)_2(dppf)Cl_2$ (188 mg, 0.289 mmol) and $Cs_2CO_3$ (1.19 g, 8.67 mmol) in dioxane (30 mL) was stirred under nitrogen at 100°C for 16 hours. The reaction mixture was filtered, and the filtrate was extracted with EtOAc (2 x 50 mL). The combined organic layers were concentrated, and the residue was purified by flash chromatography (0~15% of EtOAc in PE) to give tert-butyl 4-cyano-4-((5-cyclopropylpyridin-2-yl)methyl) piperidine-1-carboxylate (780 mg, 79%). $^1$H NMR (400 MHz, $CDCl_3$) $\delta_H$ 8.37 (s, 1H), 7.32-7.23 (m, 2H), 4.20-4.00 (m, 2H),

3.13-2.90 (m, 4H), 1.90-1.84 (m, 3H), 1.69-1.60 (m, 1H), 1.58-1.52 (m, 1H), 1.45 (s, 9H), 1.03 (d, 2H), 0.73-0.70 (m, 2H).

Synthesis of A145

To a mixture of tert-butyl 4-cyano-4-((5-cyclopropylpyridin-2-yl)methyl)piperidine-1-carboxylate (300 mg, 0.879 mmol) in dioxane (5 mL) was added HCl/dioxane (4 M, 4.37 mL, 17.5 mmol) at 25°C. After stirring for 1 hour, the reaction mixture was filtered. The filter cake was washed with EtOAc (5 mL) and dried to give 4-((5-cyclopropylpyridin-2-yl)methyl) piperidine-4-carbonitrile hydrochloride (200 mg, 82%) which was used directly for next step. $^1$H NMR (400 MHz, $CD_3OD$) $\delta_H$ 8.72 (s, 1H), 8.37-8.18 (m, 1H), 8.09-7.90 (m, 1H), 4.20 (d, 1H), 3.58 (d, 1H), 3.48 (s, 1H), 3.36 (s, 1H), 3.27-3.13 (m, 1H), 2.99 (br s, 1H), 2.32-2.09 (m, 3H), 1.96-1.90 (m, 2H), 1.78-1.71 (m, 1H), 1.30 (d, 2H), 1.08-0.94 (m, 2H).

Synthesis of A226

To a solution of 2-(pyrimidin-4-yl) pyridine-3-carboxylic acid (200 mg, 0.994 mmol), HATU (566 mg, 1.49 mmol) in DMF (5 mL) was added DIPEA (641 mg, 4.97 mmol) at 25°C. After stirring for 30 minutes, 4-((5-cyclopropylpyridin-2-yl) methyl)piperidine-4-carbonitrile hydrochloride (193 mg, 0.696 mmol) was added. The mixture was stirred at 25°C for 16 hrs. The reaction mixture poured into water(30 mL), and the aqueous layer was extracted with EtOAc (2 x 30 mL). The combined organic layers were washed with brine (50 mL), dried over anhydrous $Na_2SO_4$, filtered and concentrated. The residue was purified by prep-HPLC (Column: Phenomenex Gemini-NX 80*40mm*3um; Condition: water (0.05% $NH_3H_2O$+10 mM $NH_4HCO_3$)-ACN; Begin B: 22%; End 48%) to give 4-((5-cyclopropylpyridin-2-yl)methyl)-1-(2-(pyrimidin-4-yl)nicotinoyl)piperidine-4-carbonitrile (31 mg, 7%). $^1$H NMR (400 MHz, $CDCl_3$) $\delta_H$ 9.22-8.93 (m, 1H), 8.87 (s, 1H), 8.77-8.84 (m, 1H), 8.36 (s, 1H), 8.25-8.21 (m, 1H), 7.65-7.63 (m, 1H), 7.48-7.42 (m, 1H), 7.33-7.27 (m, 1H), 7.25-7.19 (m, 1H), 4.96-4.65 (m, 1H), 3.63-3.61 (m, 1H), 3.50-3.33 (m, 1H), 3.50-3.33 (m, 1H), 3.31-2.94 (m, 4H), 2.22-2.02 (m, 1H), 1.92-1.75 (m, 2H), 1.03 (d, 2H), 0.73 (s, 2H). LCMS purity 97.8%, MS ESI calcd. for $C_{25}H_{24}N_6O$ $[M+H]^+$ 425, found 425.

*Example 53. Synthesis of 4-(4-fluorobenzyl)-1-(2-(pyrimidin-4-yl)-5-(trifluoromethyl)benzoyl)piperidine-4-carbonitrile (A227)*

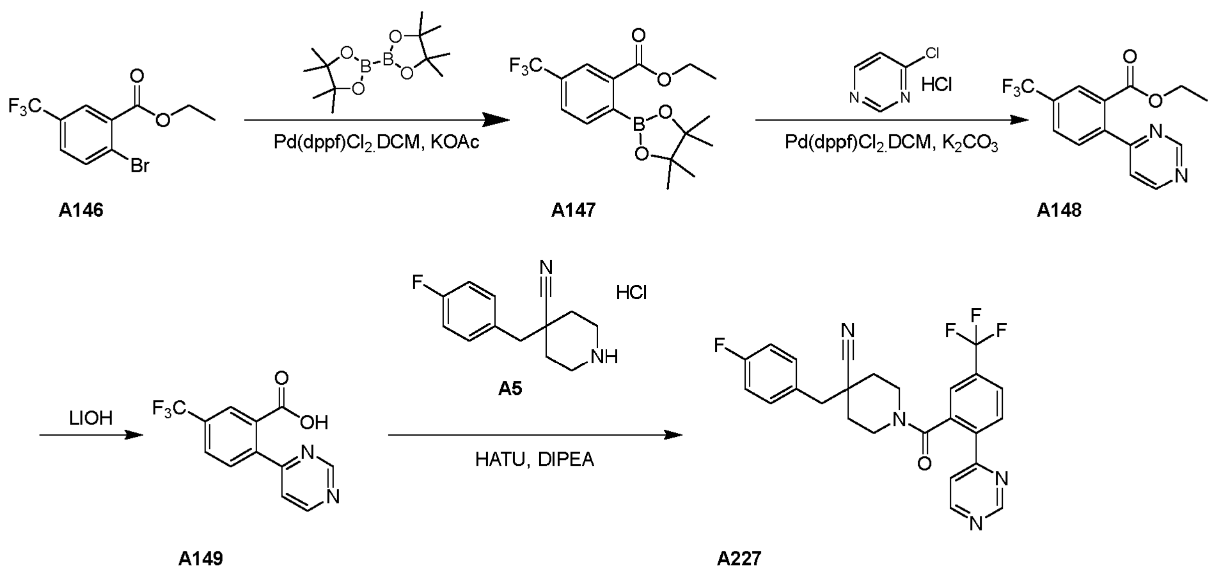


A146 A147 A148 A5 A149 A227

Synthesis of A147

A mixture of ethyl 2-bromo-5-(trifluoromethyl)benzoate (3 g, 10.0 mmol), 4,4,5,5-tetramethyl-2-(4,4,5,5-tetramethyl-1,3,2-dioxaborolan-2-yl)-1,3,2-dioxaborolane (3.80 g, 15.0 mmol), $Pd(dppf)Cl_2$ (408 mg, 0.5 mmol) and AcOK (4.14 g, 30.0 mmol) in dioxane (30 mL) was stirred under nitrogen at 100 °C for 16 hours. The reaction mixture poured into water (50 mL), and the aqueous layer was extracted with EtOAc (2 x 20 mL). The combined organic layers were washed with brine (30 mL), dried over anhydrous $Na_2SO_4$, and evaporated under reduced pressure and purified by flash chromatography (0~10% of EA in PE) to give ethyl 2-(4,4,5,5-tetramethyl-1,3,2-dioxaborolan-2-yl)-5-(trifluoromethyl) benzoate (1.2 g, 34.8 % yield).. $^1$H NMR (400 MHz, $CDCl_3$) $\delta_H$ 8.18 (s, 1H), 7.77-7.73 (m, 1H), 7.61 (d, 1H), 4.42 (d, 2H),

1.43 (s, 15H). $^{19}F$ NMR (376.5 MHz, $CDCl_3$) $\delta_F$ -62.92.

Synthesis of A148

A mixture of ethyl 2-(4,4,5,5-tetramethyl-1,3,2-dioxaborolan-2-yl)-5-(trifluoromethyl)benzoate (600 mg, 1.74 mmol), 4-chloropyrimidine hydrochloride (235 mg, 1.56 mmol), Pd(dppf)$Cl_2$ (128 mg, 0.174 mmol) and $K_2CO_3$ (1.2 g, 8.70 mmol) in dioxane (20 mL)/$H_2O$ (2 mL) was stirred under nitrogen at 100°C for 3 hours. The reaction mixture was poured into water (50 mL), and the aqueous layer was extracted with EtOAc (2 x 50 mL). The combined organic layers were washed with brine (40 mL), dried over anhydrous $Na_2SO_4$, and concentrated. The residue was purified by flash chromatography (20~25% of EA in PE) to give ethyl 2-(pyrimidin-4-yl)-5-(trifluoromethyl)benzoate (330 mg, 64.0 %). $^1H$ NMR (400 MHz, $CDCl_3$) $6_H$ 9.27 (s, 1H), 8.83 (d, 1H), 8.17 (s, 1H), 7.89-7.83 (m, 1H), 7.67 (d, 1H), 7.49 (dd, 1H), 4.22 (d, 2H), 1.15 (t, 3H). $^{19}F$ NMR (376.5 MHz, $CDCl_3$) $\delta_F$ -62.92.

Synthesis of A149

To a mixture of ethyl 2-(pyrimidin-4-yl)-5-(trifluoromethyl)benzoate (340 mg, 1.14 mmol) in MeOH /water (5/1, 6 mL) was added LiOH $H_2O$ (52.5 mg, 1.25 mmol), and the mixture was stirred at 60°C for 16 hours. The reaction mixture was concentrated to give 2-(pyrimidin-4-yl)-5-(trifluoromethyl)benzoic acid (340 mg). $^1H$ NMR (400 MHz, $CD_3OD$) $\delta_H$ 9.19 (d, 1H), 8.76 (d, 1H), 7.93 (s, 1H), 7.85-7.72 (m, 3H). $^{19}F$ NMR (376.5 MHz, $CD_3OD$) $\delta_F$ -64.35.

Synthesis of A227

To a solution of 2-(pyrimidin-4-yl)-5-(trifluoromethyl)benzoic acid (190 mg, 0.708 mmol) , 4-[(4-fluorophenyl) methyl]piperidine-4-carbonitrile hydrochloride (180 mg, 0.708 mmol) and HATU (402 mg, 1.05 mmol) in DMF (3 mL) was added DIPEA (456 mg, 3.54 mmol) . The mixture was stirred at 20°C for 2 hours. The reaction mixture was poured into water (50 mL), and the aqueous layer was extracted with EtOAc (2 x 10 mL). The combined organic layers were washed with brine (20 mL), dried over anhydrous $Na_2SO_4$, and concentrated. The product was purified by prep-HPLC (Column:Phenomenex Gemini-NX 80*30mm*3um; Condition:water(10mM $NH_4HCO_3$)-ACN; Begin B: 38%; End 68%) to give 4-(4-fluorobenzyl)-1-(2-(pyrimidin-4-yl)-5-(trifluoromethyl)benzoyl)piperidine-4-carbonitrile (35.9 mg, 10.8% yield). $^1H$ NMR (400 MHz, $CDCl_3$) $\delta_H$ 9.42-8.96 (m, 1H), 8.86 (s, 1H), 7.93-7.76 (m, 2H), 7.72-7.59 (m, 2H), 7.22 (d, 2H), 7.05 (m, 2H), 4.91-4.59 (m, 1H), 3.66-3.33 (m, 2H), 3.17-2.99 (m, 1H), 2.92-2.80 (m, 2H), 2.06-1.87 (m, 1H), 1.70-1.58 (m, 2H), 1.52-1.44 (m, 1H). LC/MS purity 99%, MS ESI calcd. for $C_{25}H_{20}F_4N_4O$ [M+H]+ 469.0 found 469.0. $^{19}F$ NMR (376.5 MHz, $CDCl_3$) $\delta_F$ -62.86, -62.95, -114.37.

*Example 54. Synthesis of 1-(2-(pyrimidin-4-yl)nicotinoyl)-4-((5-(trifluoromethyl)pyridin-2-yl)methyl)piperidine-4-carbonitrile (A228)*

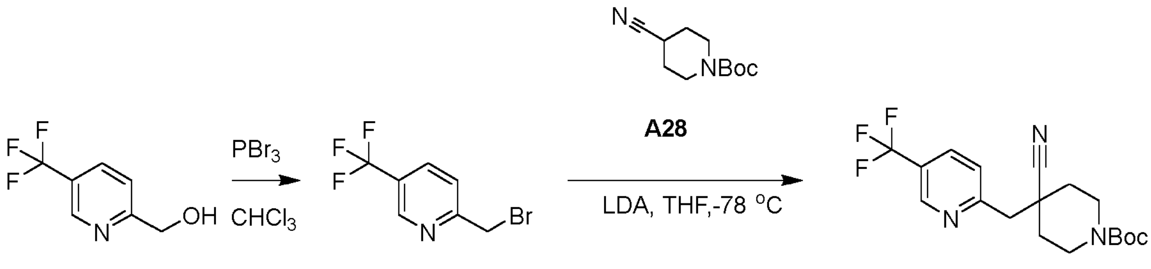


A150 A151 A152

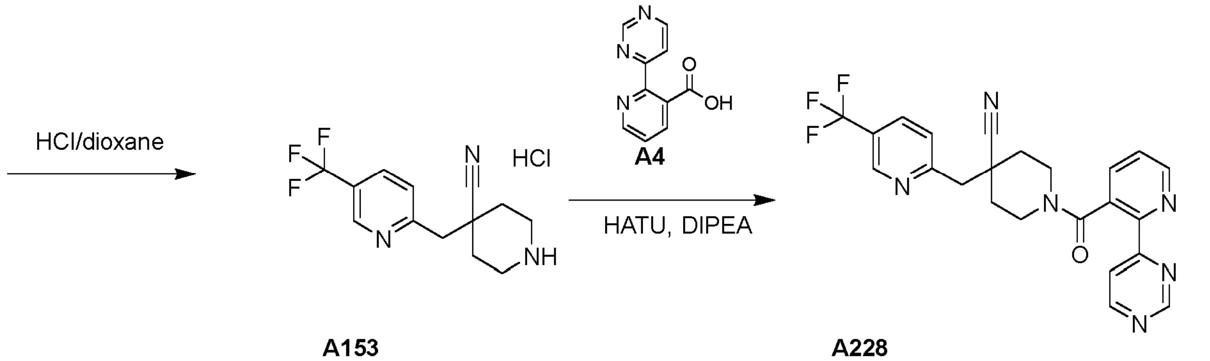


A153 A228

Synthesis of A151

To a solution of (5-(trifluoromethyl)pyridin-2-yl)methanol (1 g, 5.64 mmol) in $CHCl_3$ (20 mL) under nitrogen was added tribromophosphane (4.57 g, 16.9 mmol) in one portion at 25°C. The mixture was stirred at 25°C for 2 hours. The mixture was poured slowly into ice-water (50 mL) and extracted with EtOAc (3 x 50 mL). The combined organic layers were washed with brine (2 x 50 mL), dried over anhydrous $Na_2SO_4$, filtered and concentrated to give 2-(bromomethyl)-5-(trifluoromethyl)pyridine (670 mg). $^1H$ NMR (400 MHz, $CDCl_3$) $\delta_H$ 8.84 (s, 1 H), 7.97-7.90 (m, 1 H), 7.61-7.54 (m, 1 H), 4.59 (s, 2 H), 1.70 (s, 3 H).

Synthesis of A152

To a mixture of tert-butyl 4-cyanopiperidine-1-carboxylate (656 mg, 3.12 mmol) in THF (10 mL) was added dropwise LDA (1.56 mL, 2 M, 3.12 mmol) at -78°C, the mixture was stirred at -78°C for 1 hour, and then 2-(bromomethyl)-5-(trifluoromethyl)pyridine (500 mg, 2.08 mmol) was added. The mixture was warmed to 20°C and stirred for 16 hours. The mixture was poured into water (100 mL) and extracted with EtOAc (3 x 100 mL). The combined organic layers were washed with brine (2x100 mL), dried over $Na_2SO_4$, filtered, and concentrated. The residue was purified by flash chromatography (0~30% of EtOAc in PE) to give tert-butyl 4-cyano-4-((5-(trifluoromethyl)pyridin-2-yl)methyl)piperidine-1-carboxylate (0.3 g, 39%). $^1H$ NMR (400 MHz, $CDCl_3$) $\delta_H$ 8.85 (s, 1 H), 7.93 (d, 1 H), 7.50 (d, 1 H), 4.23-4.06 (m, 2 H), 3.15-3.10 (m, 2 H), 3.07-2.97 (m, 2 H), 1.95-1.86 (m, 2 H), 1.68-1.59 (m, 2 H), 1.4-1.45 (m, 9H).

Synthesis of A153

To a solution of tert-butyl 4-cyano-4-((5-(trifluoromethyl)pyridin-2-yl)methyl)piperidine-1-carboxylate (0.18 g, 0.49 mmol) in dioxane (5 mL) under nitrogen was added HCl/dioxane (0.12 mL, 4M HCl in dioxane) at 25°C. The mixture was stirred at 25°C for 16 hours. The reaction mixture was concentrated to give 4-((5-(trifluoromethyl)pyridin-2-yl)methyl) piperidine-4-carbonitrile hydrochloride (140 mg).

Synthesis of A228

To a solution of 2-(pyrimidin-4-yl)pyridine-3-carboxylic acid (128 mg, 0.64 mmol), HATU (242 mg, 0.64 mmol), DIPEA (0.37 mL, 2.12 mmol) and 4-((5-(trifluoromethyl)pyridin-2-yl)methyl)piperidine-4-carbonitrile hydrochloride (130 mg, 0.43 mmol) in DMF (10 mL) was stirred at 0°C for 2 hours. The mixture was poured into saturated $NH_4Cl$ solution (50 mL) and extracted with EtOAc (3 x 50 mL). The combined organic layers were washed with brine (2 x 50 mL), dried over

anhydrous $Na_2SO_4$, filtered and concentrated to give 295 mg, which was purified by prep-HPLC (Column:Phenomenex Gemini-NX 80*30mm*3μm ); Condition:water(10mM $NH_4HCO_3$)-ACN; Begin B: 28%; End B:58 %) to afford 1-(2-(pyrimidin-4-yl)nicotinoyl)-4-((5-(trifluoromethyl)pyridin-2-yl)methyl)piperidine-4-carbonitrile (166.5 mg, 56%). $^1$H NMR (400 MHz, $CDCl_3$) $\delta_H$ 9.32-9.02 (m, 1 H), 8.94-8.85 (m, 1 H), 8.85-8.80 (m, 1 H), 8.79-8.73 (m, 1 H), 8.30-8.24 (m, 1 H), 7.98-7.91 (m, 1 H), 7.71-7.64 (m, 1 H), 7.55-7.40 (m, 2 H), 4.97-4.69 (m, 1 H), 3.68-3.59 (m, 1 H), 3.48-3.34 (m, 0.5 H), 3.26-3.12 (m, 3 H), 2.25-2.06 (m, 1 H), 2.02-1.93 (m, 1 H), 1.90-1.73 (m, 1.5 H), 1.71-1.61 (m, 0.5 H), 1.39-1.20 (m, 0.5 H). $^{19}$F NMR (376.5 MHz, $CDCl_3$) $\delta_F$ -62.430. LCMS purity 99%, calcd. for $C_{23}H_{19}F_3N_6O$ $[M+H]^+$ 453.2, found 453.2

*Example 55. Synthesis of 4-(4-fluorobenzyl)-1-(2-(pyrimidin-4-yl)benzoyl)piperidine-4-carbonitrile (A229)*

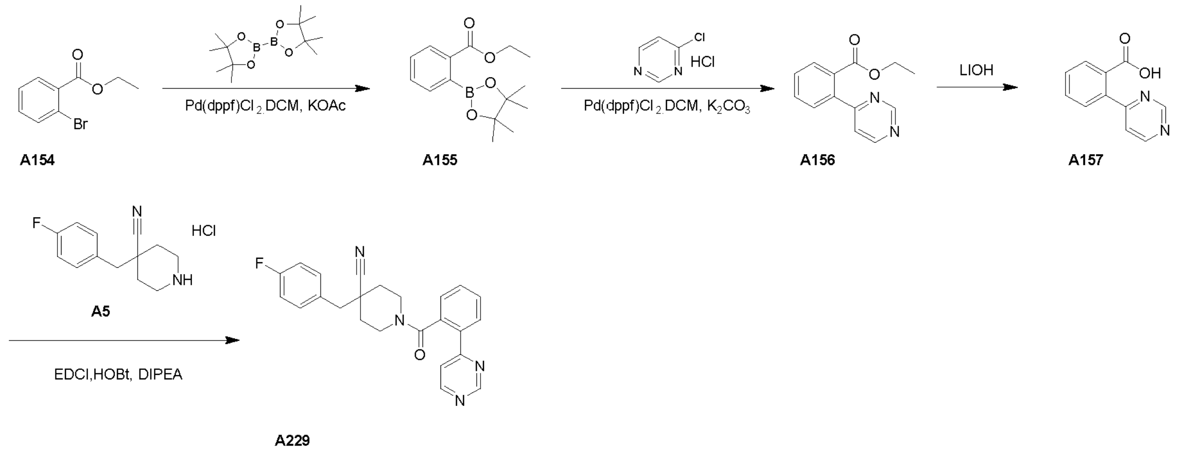


Synthesis of A155

To a solution of ethyl 2-bromobenzoate (5 g, 21.8 mmol ) in dry toluene (50 mL) were added under nitrogen 4,4,5,5-tetramethyl-2-(4,4,5,5-tetramethyl-1,3,2-dioxaborolan-2-yl)-1,3,2-dioxaborolane (6.62 g, 26.1 mmol), KOAc (4.27 g, 43.6 mmol) and $Pd(pddf)Cl_2$ (1.59 g, 2.18 mmol) at 20°C. After heating at 100°C for 16 hours, water (50 mL) was added and the product was extracted with EtOAc (3 x 50 mL). The combined organic layers were washed with brine (50 mL), dried over $Na_2SO_4$, and concentrated to afford the ethyl 2-(4,4,5,5-tetramethyl-1,3,2-dioxaborolan-2-yl)benzoate (5 g). $^1$H NMR (400 MHz, $CDCl_3$) $\delta_H$ 7.78-7.74 (m, 1H), 7.68-7.62 (m, 1H), 7.48 (s, 1H), 7.44-7.28 (m, 3H), 4.50-4.32 (M, 3H), 1.44-1.36 (s, 9H), 1.26 (s, 3H).

Synthesis of A156

A mixture of ethyl 2-(4,4,5,5-tetramethyl-1,3,2-dioxaborolan-2-yl)benzoatebenzoate (1 g, 3.62 mmol), 4-chloropyrimidine hydrochloride (490 mg, 3.25 mmol), $Pd(dppf)Cl_2$ (267 mg, 0.3620 mmol) and $K_2CO_3$ (2.50 g, 18.1 mmol) in dioxane (15 mL)/water (2 mL) was stirred at 100°C for 3 hours under nitrogen. The reaction mixture was poured into water (50 mL). The aqueous layer was extracted with EtOAc (2 x 50 mL). The combined organic layers were washed with brine (100 mL), dried over anhydrous $Na_2SO_4$, and concentrated. The residue was purified by flash chromatography (0~30% of EA in PE) to give ethyl 2-(pyrimidin-4-yl)benzoate (800 mg). $^1$H NMR (400 MHz, $CDCl_3$) $\delta_H$ 9.21 (S, 1H), 8.80-8.72 (m, 1H), 7.90-7.84 (m, 1H), 7.60-7.56 (m, 1H), 7.54-7.52 (m, 2H), 7.46-7.44 (m, 1H), 4.20-4.10 (m, 2H), 2.00 (s, 2H), 1.26-1.20 (m, 3H).

Synthesis of A157

To a mixture of ethyl 5-fluoro-2-(pyrimidin-4-yl)benzoate (400 mg, 1.75 mmol) in MeOH (10 mL)/water (1 mL) was added $LiIOH \cdot H_2O$ (80.6 mg, 1.92 mmol). The mixture was stirred at 60°C for 16 hours. The reaction mixture was concentrated to dryness to give 5-fluoro-2-(pyrimidin-4-yl)benzoic acid (300 mg). $^1$H NMR (400 MHz, $CD_3OD$) $\delta_H$ 9.13 (s, 1H), 8.72-8.66 (m, 1H), 7.80-7.74 (m, 1H), 7.66-7.60 (m, 2H), 7.50-7.46 (m, 2H).

Synthesis of A229

A solution of ethyl 2-(pyrimidin-4-yl)benzoate (350 mg, 1.74 mmol), HOBt (469 mg, 3.48 mmol), EDCI (664 mg, 3.48 mmol), DIPEA (0.908 mL, 5.22 mmol) and 4-[(4-fluorophenyl)methyl] piperidine-4-carbonitrile hydrochloride (443 mg, 1.74 mmol) in DMF (5 mL) was stirred at 0°C for 2 hours. The mixture was filtered, concentrated, and purified by prep-HPLC (Column: Xtimate C18 150*40mm*5um; Condition: water(10mM $NH_4HCO_3$)-ACN; Begin B: 25; End B: 35; Gradient Time (min):8; 100%B Hold Time (min):2) to afford 4-(4-fluorobenzyl)-1-(2-(pyrimidin-4-yl)benzoyl)piperidine-4-carbonitrile (49.1 mg, 7%). $^1H$ NMR (400 MHz, $CDCl_3$) $\delta_H$ 9.40-9.02 (br s, 1H), 8.80 (s, 1H), 7.80-7.58 (m, 2H), 7.58-7.50 (m, 2H), 7.38-7.32 (m, 1H), 7.24-7.18 (m, 2H), 7.04 (s, 2H), 4.86-4.66 (m, 1H), 3.68-3.24 (m, 2H), 3.10-2.74 (m, 4H), 2.05-1.88 (m, 1H), 1.86-1.58 (m, 2H). $^{19}F$ NMR (376.5 MHz, $CDCl_3$) $\delta_F$ -114.52. LC-ELSD/MS purity 98%, MS ESI calcd. for $C_{24}H_{21}FN_4O$ $[M+H]^+$ 401, found 401.

*Example 56. Synthesis of 4-(2-cyclopropyl-4-fluorobenzyl)-1-(2-(pyrimidin-4-yl)nicotinoyl)piperidine-4-carbonitrile (A230)*

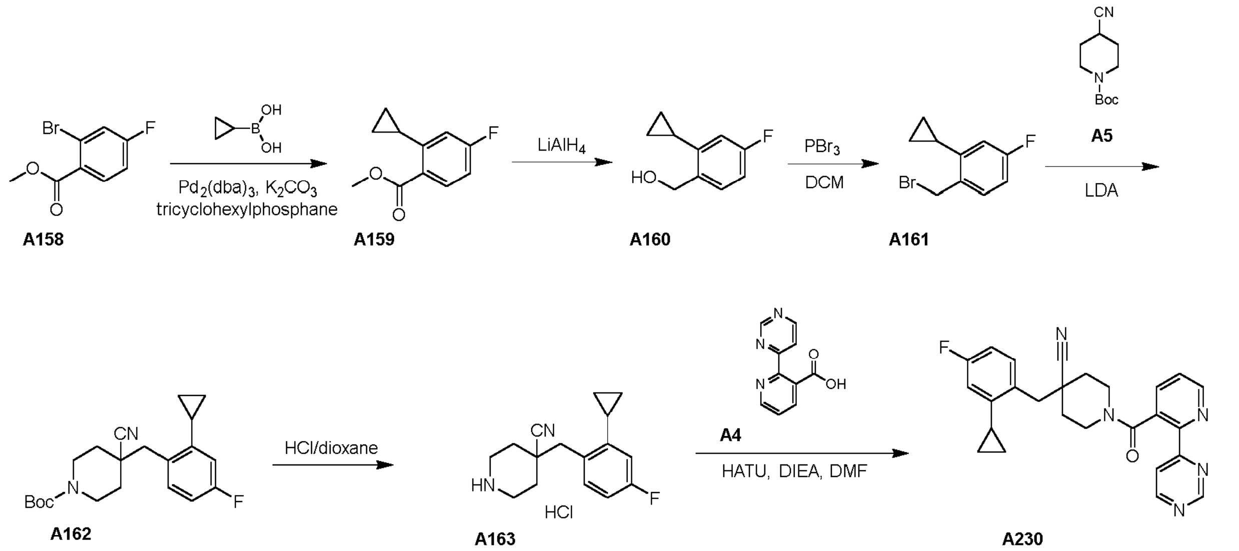

A158 A159 A160 A161 A162 A163 A230

Synthesis of A159

A mixture of methyl 2-bromo-4-fluorobenzoate (3 g, 12.8 mmol), cyclopropylboronic acid (1.53 g, 17.9 mmol), palladium(2+) bis(di-tert-butyl(cyclopenta-1,3-dien-1-yl)phosphane) iron dichloride (834 mg, 1.28 mmol) and $Cs_2CO_3$ (2.64 g, 19.2 mmol) in dioxane (40 mL) was stirred at 100°C for 16 hours under nitrogen. The reaction mixture poured into water (50 mL), and the aqueous layer was extracted with EtOAc (2 x 20 mL). The combined organic layers were washed with brine (30 mL), dried over anhydrous $Na_2SO_4$, and concentrated. The residue was purified by flash chromatography (0~30% of EtOAc in PE) to give methyl 2-cyclopropyl-4-fluorobenzoate (1.2 g, 48.3 % yield). $^1H$ NMR (400 MHz, $CDCl_3$) $\delta_H$ 7.85 (m, 1H), 6.86 (m, 1H), 6.66 (m, 1H), 3.90 (s, 3H), 2.80-2.71 (m, 1H), 1.09-0.98 (m, 2H), 0.72-0.65 (m, 2H). $^{19}F$ NMR (376.5 MHz, $CDCl_3$) $\delta_F$ -107.49.

Synthesis of A160

To a solution of methyl 2-cyclopropyl-4-fluorobenzoate (500 mg, 2.57 mmol) in THF (10 mL) was added $LiAlH_4$ (195 mg, 5.14 mmol) in one portion. After stirring at 0°C for 1 hour. The reaction mixture poured into water (20 mL), and the aqueous layer was extracted with EtOAc (2 x 10 mL). The combined organic layers were washed with brine (30 mL), dried over anhydrous $Na_2SO_4$, and concentrated to give (2-cyclopropyl-4-fluorophenyl)methanol (330 mg). $^1H$ NMR (400 MHz, $CDCl_3$) $\delta_H$ 7.30 (m, 1H), 6.85 (m, 1H), 6.67 (m, 1H), 4.82 (s, 2H), 2.09-1.98 (m, 1H), 1.83 (ms, 1H), 1.06-0.91 (m, 2H), 0.74-0.59 (m, 2H). $^{19}F$ NMR (376.5 MHz, $CDCl_3$) $\delta_F$ -114.77.

Synthesis of A161

To a solution of (2-cyclopropyl-4-fluorophenyl)methanol (100 mg, 0.601 mmol) was stirred in DCM (20 mL) was added $PBr_3$ (243 mg, 0.901 mmol) at 25°C under nitrogen. The reaction was stirred at 25°C for 2 hours. The mixture was cooled in an ice-bath and saturated $NH_4Cl$ solution (30 mL) was added. The mixture was extracted with DCM (2 x 30 mL) and the combined organic layers were dried over $Na_2SO_4$, filtered, and concentrated. The residue was purified by flash chromatography (0~5% of EA in PE) to give 1-(bromomethyl)-2-cyclopropyl-4-fluorobenzene (110 mg, 80.2 % yield). $^1H$ NMR (400MHz, CHLOROFORM-d) $\delta_H$ 7.29 (m, 1H), 6.89-6.81 (m, 1H), 6.70 (m, 1H), 4.74-4.66 (m, 2H), 1.54 (s, 1H), 1.11-1.02 (m, 2H), 0.78-0.69 (m, 2H).

Synthesis of A162

To a tert-butyl 4-cyanopiperidine-1-carboxylate (175 mg, 0.833mmol) in THF (30 mL) was added dropwise LDA (0.63 mL, 1.25 mmol, 2 M in THF/n-heptane) at -78°C, and the mixture was stirred at -78°C for 1 hour under nitrogen. Then 1-(bromomethyl)-2-cyclopropyl-4-fluorobenzene (230 mg, 1.0 mmol) was added at -78°C, and the mixture was allowed to warm to 20°C and stirred under nitrogen for 16 hours. The reaction mixture was poured into saturated $NH_4Cl$ solution (50 mL), and the aqueous layer was extracted with ethyl acetate (2 x 50 mL). The combined organic layers were washed with brine (2 x 50 mL), dried over $Na_2SO_4$, filtered, and concentrated. The product was purified by column chromatography (0-10% EtOAc in PE) to give tert-butyl 4-cyano-4-(2-cyclopropyl-4-fluorobenzyl)piperidine-1-carboxylate (110 mg, yield 36.9%). $^1H$ NMR (400MHz, $CDCl_3$) $\delta_H$ 7.24 (d, 1H), 6.86 (m, 1H), 6.67 (m 1H), 4.14 (m, 2H), 3.09 (s, 2H), 3.05-2.90 (m, 2H), 2.06-1.97 (m, 1H), 1.91 (m, 2H), 1.46 (s, 10H), 1.07-0.99 (m, 2H), 0.67 (q, 2H).

Synthesis of A163

To a mixture of tert-butyl 4-cyano-4-(2-cyclopropyl-4-fluorobenzyl)piperidine-1-carboxylate (110 mg, 0.306 mmol) in dioxane (20 mL) was added HCl/dioxane (4 M, 0.77 mL, 3.06mmol ), the mixture was stirred at 20°C for 16 hours. The reaction mixture was concentrated to give 4-(2-cyclopropyl-4-fluorobenzyl)piperidine-4-carbonitrile hydrochloride (120 mg), which was used without further purification.

Synthesis of A230

To a solution of 2-(pyrimidin-4-yl)pyridine-3-carboxylic acid ( 117 mg, 0.584 mmol) and HATU (332 mg, 0.876 mmol) in DMF (20 mL) was added DIEA (225 mg, 1.75 mmol) . The mixture was stirred at 25°C for 10 minutes, and then 4-(2-cyclopropyl-4-fluorobenzyl)piperidine-4-carbonitrile hydrochloride (120 mg, 0.409 mmol) was added. The mixture was stirred for 16 hours at 25 °C. The reaction mixture poured into water (50 mL), and the aqueous layer was extracted with EtOAc (3 x 50 mL). The combined organic layers were washed with brine (2 x 50 mL), dried over anhydrous $Na_2SO_4$, and concentrated. The product was purified by HPLC (Column: Phenomenex Gemini-NX 80*40mm*3um,Condition: water(0.05%$NH_3H_2O$+10mM $NH_4HCO_3$)-ACN,Begin: B 40,End: B 56 ,Gradient Time(min): 8, 100%B Hold Time(min): 2.6, FlowRate(ml/min): 30, Injections: 5.) to afford 4-(2-cyclopropyl-4-fluorobenzyl)-1-(2-(pyrimidin-4-yl)nicotinoyl)piperidine-4-carbonitrile (9.3 mg, 3.61%). $^1H$ NMR (400MHz, $CDCl_3$) $\delta_H$ 9.22 (s, 1H), 8.88 (d, 1H), 8.83-8.72 (m, 2H), 8.24 (d, 1H), 7.66 (d, 1H), 7.47 (m, 1H), 7.23 (d, 1H), 6.91-6.80 (m, 1H), 6.68 (d, 1H), 4.92 (d, 1H), 4.80 (d, 1H), 3.62 (d, 1H), 3.51-3.36 (m, 1H), 3.17 (d, 3H), 2.20-2.05 (m, 1H), 2.04-1.81 (m, 3H), 1.70 (d, 1H), 1.57 (s, 3H), 1.25 (s, 1H), 1.08-0.92 (m, 2H), 0.67 (br s, 2H). LCMS: purity 100%; MS ESI calcd. for $C_{26}H_{24}FN_5O$ $[M+Na]^+$ 464.2, found 464.2.

*Example 57. Synthesis of 4-(4-chloro-2-cyclopropylbenzyl)-1-(2-(pyrimidin-4-yl)nicotinoyl)piperidine-4-carbonitrile (A231) and 4-(2-bromo-4-chlorobenzyl)-1-(2-(pyrimidin-4-yl)nicotinoyl)piperidine-4-carbonitrile (A232)*

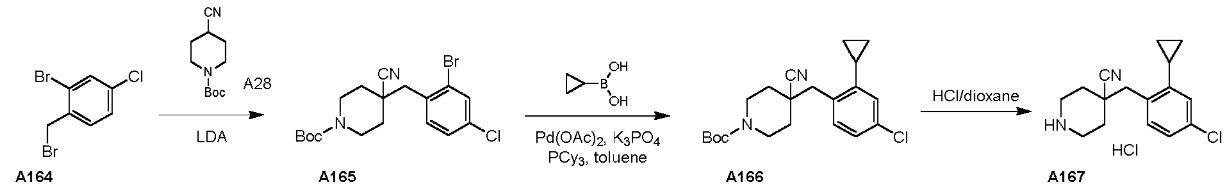


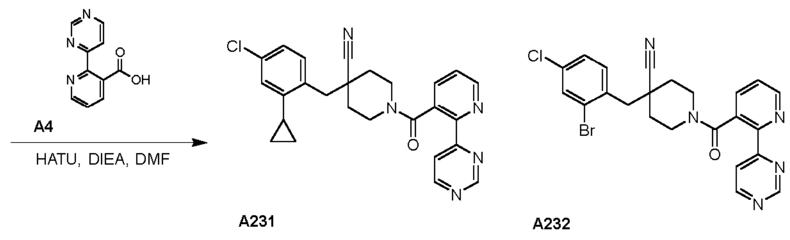


Synthesis of A165

To a solution of tert-butyl 4-cyanopiperidine-1-carboxylate (403 mg, 1.92 mmol) in THF (10 mL) at -70°C was slowly added a solution of LDA (1.75 mL, 2 M in hexane, 3.50 mmol). After 30 minutes, 2-bromo-1-(bromomethyl)-4-chlorobenzene (500 mg, 1.75 mmol) was added. The reaction mixture was stirred at -70°C for 1 hour and then warmed to 25°C and stirred for 16 hours. Saturated aqueous $NH_4Cl$ solution (50 mL) was added, and the mixture was extracted with EtOAc (2 x 30 mL). The combined organic layers were dried over $Na_2SO_4$, filtered and concentrated. The residue was purified by silica gel chromatography (PE/EtOAc = 10/1 to 5/1) to give tert-butyl 4-(2-bromo-4-chlorobenzyl)-4-cyanopiperidine-1-carboxylate (400 mg, 55.2%). $^1H$ NMR (400MHz, $CDCl_3$) $\delta_H$ 7.62 (d, 1H), 7.44 (d, 1H), 7.32 (dd, 1H), 4.29-4.00 (m, 2H), 3.09 (s, 2H), 3.06-2.88 (m, 2H), 1.90-1.80 (m, 2H), 1.72-1.60 (m, 2H), 1.46 (s, 9H).

Synthesis of A166

tert-Butyl 4-(2-bromo-4-chlorobenzyl)-4-cyanopiperidine-1-carboxylate (400 mg, 0.97 mmol), cyclopropylboronic acid (165 mg, 1.93 mmol), Pd(OAc)$_2$ (125 mg, 0.19 mmol) and $K_3PO_4$ (331 g, 1.93 mmol) were combined in toluene (10 mL) and water (1 mL) and stirred at 100°C for 16 hours under nitrogen. Ice-water (10 mL) was added and the mixture was extracted with EtOAc (15 mL x 2). The combined organic layers were dried over $Na_2SO_4$, concentrated and purified twice by flash chromatography (0~90% of EtOAc in PE) to give tert-butyl 4-(4-chloro-2-cyclopropylbenzyl)-4-cyanopiperidine-1-carboxylate (150 mg). $^1H$ NMR (400MHz, $CDCl_3$) $\delta_H$ 7.62 (d, 0.5H), 7.47-7.42 (m, 0.5H), 7.34-7.29 (m, 0.5H), 7.24-7.20 (m, 0.5H), 7.16-7.11 (m, 1H), 6.96 (d, 1H), 4.14 (s, 2H), 3.13-2.87 (m, 4H), 2.02-1.82 (m, 2H), 1.71-1.57 (m, 1H), 1.46 (s, 9H), 1.31-1.24 (m, 1H), 1.06-0.93 (m, 2H), 0.91-0.81 (m, 2H).

Synthesis of A167

To a solution of tert-butyl 4-(4-chloro-2-cyclopropylbenzyl)-4-cyanopiperidine-1-carboxylate (150 mg, 0.4 mmol) was added HCl/Dioxane (10 mL, 4M, 40.0 mmol) at 25°C. The mixture was stirred at 25°C for 16 hours under nitrogen. The mixture was concentrated to give 4-(4-chloro-2-cyclopropylbenzyl)piperidine-4-carbonitrile hydrochloride (100 mg). The product was used directly in the next step.

Synthesis of A231 and A232

To a solution of 2-(pyrimidin-4-yl)nicotinic acid (87.8 mg, 0.45 mmol), HOBt (98.2 mg, 0.73 mmol), EDCI (139 mg, 0.73 mmol), DIPEA (0.314 mL, 1.81 mmol) and 4-(4-chloro-2-cyclopropylbenzyl)piperidine-4-carbonitrile hydrochloride (100 mg, 0.36 mmol) in DMF (2 mL) was stirred at 15°C for 16 hours. The mixture was filtered, concentrated, and purified by prep-HPLC (Column: Xtimate C18 150 * 40 mm * 5 um; Condition: water(0.05%$NH_3H_2O$)-ACN; Begin B: 50; End B: 80; Gradient Time (min):8.5; 100%B Hold Time (min): 2) to afford 4-(4-chloro-2-cyclopropylbenzyl)-1-(2-(pyrimidin-4-yl) nicotinoyl)piperidine-4-carbonitrile (30 mg, 12.0%) and 4-(2-bromo-4-chlorobenzyl)-1-(2-(pyrimidin-4-yl)nicotinoyl)piperidine-4-carbonitrile (30 mg, 16.6%).

SFC purification

4-(4-Chloro-2-cyclopropylbenzyl)-1-(2-(pyrimidin-4-yl)nicotinoyl)piperidine-4-carbonitrile (20 mg, 0.045 mmol) was further purified by SFC (Column DAICEL CHIRALPAK AD-H (250mm * 30mm, 5um), Condition 0.1%$NH_3H_2O$ IPA, Begin B 40, End B 40) to give 4-(4-chloro-2-cyclopropylbenzyl)-1-(2-(pyrimidin-4-yl)nicotinoyl)piperidine-4-carbonitrile (12.1 mg, 60.8%). $^1H$ NMR (400MHz, $CDCl_3$) $\delta_H$ 9.22 (s,0.3H), 8.91-8.82 (m, 1.7H), 8.79-8.74 (m, 1H), 8.33-8.19 (m, 1H), 7.74-7.61 (m, 1H), 7.51-7.40 (m, 1H), 7.24-7.18 (m, 1H), 7.17-7.11 (m, 1H), 7.02-6.94 (m, 1H), 5.01-4.72 (m, 1H), 3.67-3.58 (m, 0.5H), 3.53-3.35 (m, 1H), 3.32-3.00 (m, 3.5H), 2.21-2.04 (m, 1H), 2.01-1.82 (m, 3H), 1.79-1.65 (m, 1H), 1.08-0.92 (m, 2H), 0.77-0.60 (m, 2H). LCMS purity 100%, MS ESI calcd. For $C_{26}H_{24}ClN_5ONa$ $[M+Na]^+$ 480. found 480.

4-(2-Bromo-4-chlorobenzyl)-1-(2-(pyrimidin-4-yl)nicotinoyl)piperidine-4-carbonitrile (30 mg, 0.06 mmol) was further purified by SFC (Column: DAICEL CHIRALPAK AD-H (250mm * 30mm, 5um), Condition 0.1%$NH_3H_2O$ IPA, Begin B 40, End B 40) to give 4-(2-bromo-4-chlorobenzyl)-1-(2-(pyrimidin-4-yl)nicotinoyl)piperidine-4-carbonitrile (20.6 mg, 68.8%). $^1H$ NMR (400MHz, $CDCl_3$) $\delta_H$ 9.21 (s, 0.3H), 9.09 (s, 0.7H), 8.90 (d, 1H), 8.76 (d, 1H), 8.26 (d, 1H), 7.74-7.58 (m, 2H), 7.46 (d, 2H), 7.33 (d, 1H), 5.05-4.62 (m, 1H), 3.63 (d, 1H), 3.50-3.33 (m, 1H), 3.29-3.01 (m, 3H), 2.19-1.95 (m, 3H), 1.88-1.76 (m, 1H), 1.64 (d, 1H). LCMS purity 98%, MS ESI calcd. For $C_{23}H_{20}BrClN_5ONa$ $[M+H+Na]^+$ 520. found 520.

*Example 58. Synthesis of 4-(4-fluorobenzyl)-1-(2-(5-fluoropyrimidin-4-yl)nicotinoyl)piperidine-4-carbonitrile (A233)*

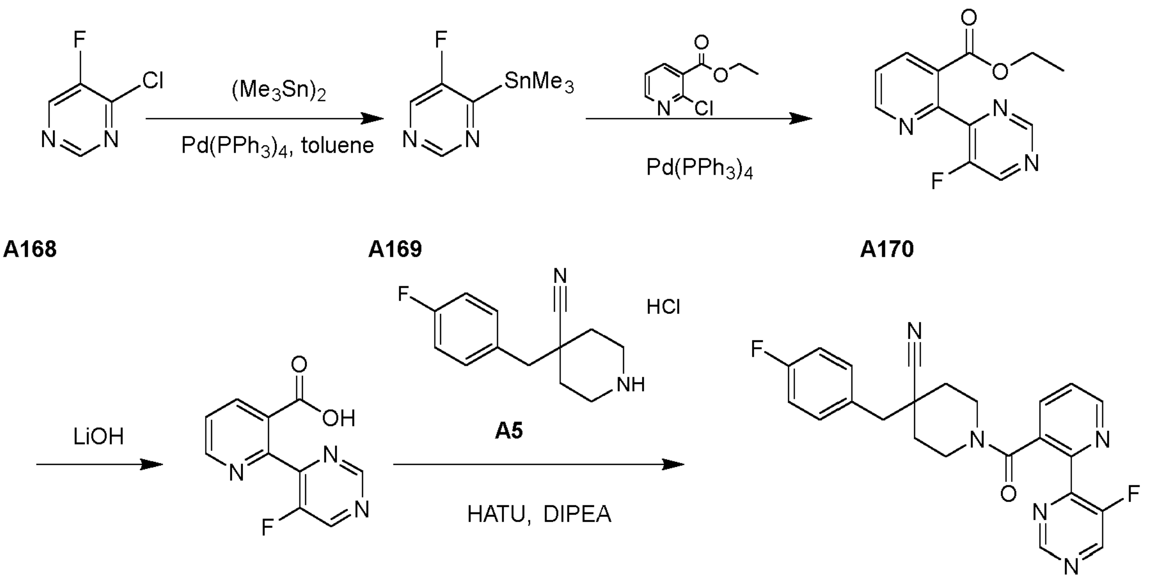


A168 A169 A170 A5 A171 A233

Synthesis of A169

To a mixture of 4-chloro-5-fluoropyrimidine (2 g, 15 mmol), hexakis($\lambda^3$-methyl) ditin (7.37 g, 22.5 mmol) in toluene (10 mL) under nitrogen, $Pd(PPh_3)_4$ (866 mg, 0.75 mmol) was added at 25°C. The mixture was stirred at 110°C under nitrogen for 2 hours. The residue was poured into a KF solution (200 mL). The aqueous layer was extracted with EtOAc (3 x 100 mL). The combined organic layers were washed with brine (2 x 100 mL), dried over anhydrous $Na_2SO_4$, filtered and concentrated to afford 5-fluoro-4-(trimethylstannyl)pyrimidine (1 g). $^1H$ NMR (400 MHz, $CDCl_3$) $\delta_H$ 9.07-9.02 (m, 1 H), 8.30 (s, 1 H), 0.51-0.24 (m, 9 H).

Synthesis of A170

To a solution of 5-fluoro-4-(trimethylstannyl)pyrimidine (500 mg, 1.91 mmol) in DMF (20 mL) was added ethyl 2-chloropyridine-3-carboxylate (0.53 g, 2.86 mmol), $\lambda^2$-iron(2+)bis(cyclopenta-2,4-diyn-1-yl)diphenyl-$\lambda^4$-phosphane) palladium dichloride (138 mg, 0.19 mmol) and CuI (36.3 mg, 0.19 mmol) at 25°C under nitrogen. The mixture was stirred at 110°C for 2 hours. The mixture was poured into saturated $NH_4Cl$ solution (100 mL) and extracted with EtOAc (3 x 100 mL). The combined organic layers were washed with brine (100 mL), dried over anhydrous $Na_2SO_4$, filtered and concentrated to give ethyl 2-(5-fluoropyrimidin-4-yl)nicotinate (0.3 g), which was used directly in the next step.

Synthesis of A171

To a solution of ethyl 2-(5-fluoropyrimidin-4-yl)nicotinate (0.3 g, 1.21 mmol) in THF (3 mL), MeOH (1 mL) and water (0.2 mL) was added $LiOH \cdot H_2O$ (91.9 mg, 2.42 mmol) in one portion at 25°C under nitrogen. The mixture was stirred at 25°C for 30 minutes. The mixture was concentrated to afford 2-(5-fluoropyrimidin-4-yl)nicotinic acid (180 mg), which was used directly in the next step.

Synthesis of A233

To a solution of 2-(5-fluoropyrimidin-4-yl)nicotinic acid (150 mg, 0.68 mmol) in DMF (5 mL) at 0°C were added HATU (387 mg, 1.02 mmol) and DIPEA (442 mg, 3.42 mmol). After stirring at 0°C for 10 mins, 4-[(4-fluorophenyl)methyl] piperidine-4-carbonitrile hydrochloride (226 mg, 0.89 mmol) was added. The mixture was stirred at 15°C for 2 hours. Water (20 mL) was added, and the aqueous layer was extracted with EtOAc (3 x 20 mL). The combined organic layers were washed with water (3 x 20 mL), brine (20 mL), dried over anhydrous $Na_2SO_4$, filtered and concentrated. The residue was purified by HPLC (Column: Phenomenex Gemini-NX 80*40mm*3μm);Condition:water(0.05%$NH_3H_2O$+10mM $NH_4HCO_3$)-ACN; Begin B:34%; End B:54%) to afford 4-(4-fluorobenzyl)-1-(2-(5-fluoropyrimidin-4-yl)nicotinoyl)piperidine-4-carbonitrile (10.2 mg, 10%). $^1H$ NMR (400 MHz, $CDCl_3$) $\delta_H$ 8.82-8.78 (m, 1 H), 8.75-8.73 (m, 1 H), 7.75-7.70 (m, 1 H), 7.51-7.46 (m, 1 H), 7.29-7.26 (m, 1 H), 7.26-7.20 (m, 2 H), 7.09-7.02 (m, 2 H), 4.77-4.64 (m, 1 H), 3.77-3.59 (m, 1 H), 3.53-3.33 (m, 1 H), 3.13-2.98 (m, 1 H), 2.98-2.83 (m, 2 H), 2.13-2.10 (m, 0.5 H), 2.03-1.95 (m, 1 H), 1.84-1.76 (m, 1 H), 1.67-1.59 (m, 1.5 H). $^{19}F$ NMR (376.5 MHz, $CDCl_3$) δF -114.337. LCMS purity 99%, 5-95 AB_1.5min_220&254 calcd. for $C_{23}H_{19}F_2N_5O$ $[M+H]^+$ 420.2, found 420.2.

*Example 59. Synthesis of 1-(5-chloro-2-(pyrimidin-4-yl)benzoyl)-4-(4-fluorobenzyl)piperidine-4-carbonitrile (A234)*

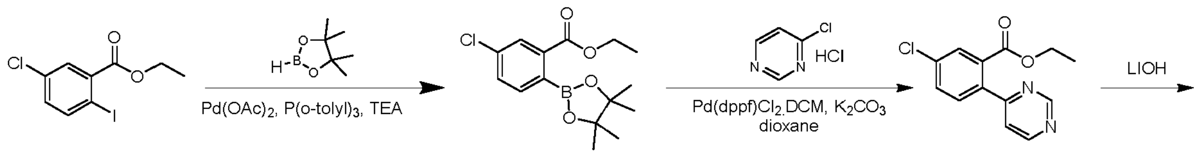


A172 A173 A174

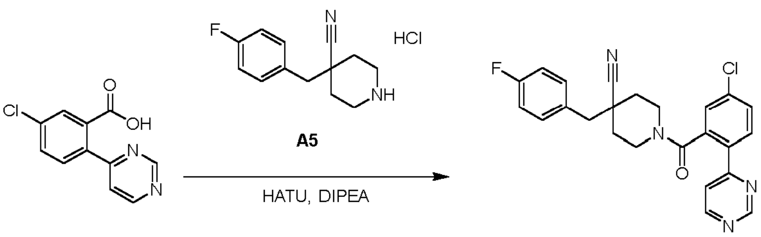


A5

A175 A234

Synthesis of A173

A mixture of ethyl 5-chloro-2-iodobenzoate (3 g, 9.66 mmol) in THF (30 mL) was added TEA (4.02 mL, 28.9 mmol). The mixture was stirred for 5 minutes and then 4,4,5,5-tetramethyl-1,3,2-dioxaborolane (2.09 mL, 14.4 mmol) was added. After stirring for an additional 5 minutes, (acetyloxy)palladio acetate (108 mg, 0.483 mmol) and tri-(o-tolyl) phosphine (294 mg, 0.966 mmol) were added. The reaction mixture was stirred at 65°C for 1 hour. The reaction mixture poured into water (30 mL), and the aqueous layer was extracted with EtOAc (2 x 30 mL). The combined organic layers were washed with brine (40 mL), dried over anhydrous $Na_2SO_4$, and concentrated. The product was purified by flash chromatograph (0~10% of EtOAc in PE) to give ethyl 5-chloro-2-(4,4,5,5-tetramethyl-1,3,2-dioxaborolan-2-yl)benzoate (1.2 g, 39.9 % yield). $^1H$ NMR (400 MHz, $CDCl_3$) $\delta_H$ 7.91 (d, 1H), 7.47 (d, 1H), 7.43 (s, 1H), 4.38 (m, 2H), 1.41 (s, 12H), 1.40-1.34 (m, 3H).

Synthesis of A174

A mixture of ethyl 5-chloro-2-(4,4,5,5-tetramethyl-1,3,2-dioxaborolan-2-yl)benzoate (1.2 g, 3.86 mmol), 4-chloropyrimidine hydrochloride (523 mg, 3.47 mmol), Pd(dppf)$Cl_2$ (284 mg, 0.3860 mmol) and $K_2CO_3$ (2.66 g, 19.3 mmol) in dioxane (15 mL)/water (2 mL) was stirred at 100°C for 3 hours under nitrogen. The reaction mixture was poured into water (20 mL), and the aqueous layer was extracted with EtOAc (2 x 20 mL). The combined organic layers were washed with brine (40 mL), dried over anhydrous $Na_2SO_4$, and concentrated. The product was purified by flash chromatography (0~25% of EtOAc in PE) to give ethyl 5-chloro-2-(pyrimidin-4-yl)benzoate (740 mg, 73.2 %, yield). $^1$H NMR (400 MHz, $CDCl_3$) $\delta_H$ 9.23 (d, 1H), 8.78 (d, 1H), 7.85 (d, 1H), 7.59-7.54 (m, 1H), 7.51-7.47 (m, 1H), 7.45 (m, 1H), 4.19 (d, 2H), 1.13 (t, 2H), 1.10-1.10 (m, 1H).

Synthesis of A175

To ethyl 5-chloro-2-(pyrimidin-4-yl)benzoate (740 mg, 2.81 mmol) in MeOH (5 mL)/water (1 mL) was added LiOH·$H_2$O(176 mg, 4.21 mmol), and the mixture was stirred at 60°C for 3 hours. The reaction mixture was concentrated to give 5-chloro-2-(pyrimidin-4-yl)benzoic acid (1 g). $^1$H NMR (400 MHz, $CD_3OD$) $\delta_H$ 9.14 (s, 1H), 8.72 (d, 1H), 7.76 (d, 1H), 7.67 (d, 1H), 7.61 (d, 1H), 7.46 (m, 1H).

Synthesis of A234

5-Chloro-2-(pyrimidin-4-yl)benzoic acid (100 mg, 0.4261 mmol), HOBt (190 mg, 0.8509 mmol), EDCI (171 mg, 0.8522 mmol), DIPEA (274 mg, 2.13 mmol) and 4-[(4-fluorophenyl)methyl]piperidine-4-carbonitrile hydrochloride (130 mg, 0.5113 mmol) were combined in DMF (5 mL) and stirred at 20°C for 2 hours. The mixture was filtered, concentrated, and purified by Prep-HPLC (Column: Phenomenex Gemini-NX 80*30mm*3um; Condition: water(10mM $NH_4HCO_3$)-ACN; Begin B: 42; End B: 72) to afford 1-(5-chloro-2-(pyrimidin-4-yl)benzoyl)-4-(4-fluorobenzyl)piperidine-4-carbonitrile (20 mg, 10.8%). $^1$H NMR (400 MHz, $CDCl_3$) $\delta_H$ 9.24-8.75 (m, 2H), 7.75-7.48 (m, 3H), 7.35 (d, 1H), 7.26-7.17 (m, 2H), 7.04 (s, 2H), 4.82-4.63 (m, 1H), 3.69-3.57 (m, 1H), 3.52-3.28 (m, 1H), 3.14-2.98 (m, 1H), 2.87 (s, 2H), 2.21 (s, 1H), 2.13-1.73 (m, 2H), 1.61 (s, 1H), 1.54-1.45 (m, 1H). LC-ELSD/MS purity 99%, MS ESI calcd. for $C_{24}H_{20}ClFN_4O$ [M+H]+ 435.2 found 435.2. $^{19}$F NMR (376.5 MHz, $CDCl_3$) $\delta_F$ -62.86, -62.95, -114.40.

*Example 60. Synthesis of (S)-4-(1-(2, 4-difluorophenyl)ethyl)-1-(2-(pyrimidin-4-yl)nicotinoyl)piperidine-4-carbonitrile (A241) and (R)-4-(1-(2, 4-difluorophenyl)ethyl)-1-(2-(pyrimidin-4-yl)nicotinoyl)piperidine-4-carbonitrile (A242)*

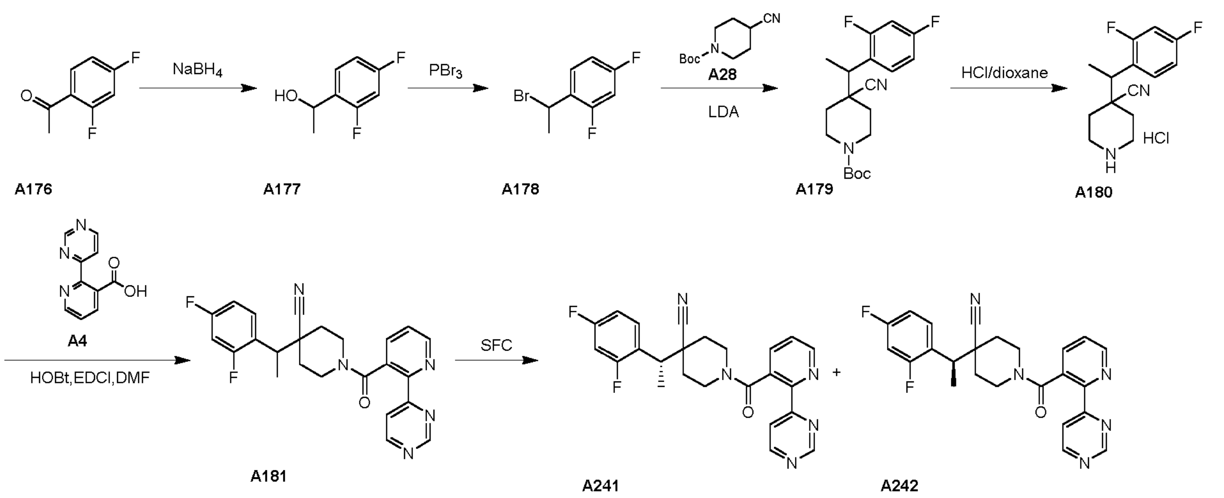


Synthesis of A177

To a solution of 1-(2,4-difluorophenyl)ethan-1-one (10 g,64 mmol) in THF (100 mL) was slowly added $NaBH_4$ (3.63 g, 96 mmol) in portions at 25°C. The mixture was stirred at 25°C for 1 hour. Saturated $Na_2S_2O_3$ solution (100 mL) was added, and the mixture was stirred for 30 minutes. The mixture was extracted with EtOAc (2 x 100 mL). The combined organic layers were dried over $Na_2SO_4$, filtered and concentrated to give 1-(2,4-difluorophenyl)ethan-1-ol (10 g). $^1$H NMR

(400MHz, $CDCl_3$) $\delta_H$ 7.54-7.44 (m, 1H), 6.96-6.86 (m, 1H), 6.86-6.74 (m, 1H), 5.20-5.12 (mz, 1H), 1.50-1.40 (m, 3H).

Synthesis of A178

To a solution of 1-(2,4-difluorophenyl)ethan-1-ol (5 g,31.6 mmol) in $CHCl_3$ (80 mL) was added tribromophosphane (8.98 ml,94.8 mmol)in one portion at 25°C under nitrogen. The mixture was stirred at 25°C for 2 hours. The mixture was slowly poured into ice-water (100 mL) and extracted with EtOAc (3 x 50 mL). The combined organic layers were washed with brine (2 x 50 mL), dried over anhydrous $Na_2SO_4$, filtered and concentrated to give 1-(1-bromoethyl)-2,4-difluorobenzene (5.5 g). $^1H$ NMR (400 MHz, $CDCl_3$) $\delta_H$ 7.60-7.40 (m, 1H), 6.94-9.86 (m=2.0 Hz, 1H), 6.86-6.74 (s, 1H), 5.48-5.36 (m, 1H), 2.10-1.98 (m, 3H).

Synthesis of A179

To a mixture of tert-butyl 4-cyanopiperidine-1-carboxylate (7.12 g, 33.9 mmol) in THF (50 mL) was added dropwise LDA (16.9 mL, 2 M, 33.9 mmol) at -78°C, and the mixture was stirred at -78°C for 1 hour. Then 1-(1-bromoethyl)-2,4-difluorobenzene (5 g, 22.6 mmol) was added and stirring was continued at -78°C for 2 hours. The mixture was poured into water (200 mL) and extracted with EtOAc (3 x 100 mL). The combined organic layers were washed with brine (2 x 100 mL), dried over $Na_2SO_4$, filtered, and concentrated. The residue was purified by flash chromatography (0~30% of EtOAc in PE) to give tert-butyl 4-cyano-4-(1-(2,4-difluorophenyl)ethyl)piperidine-1-carboxylate (5 g, 63.2%). $^1H$ NMR (400 MHz, $CDCl_3$) $\delta_H$ 7.56-7.46 (m, 1H), 6.98-6.88 (m, 1H), 6.84-6.76 (m, 1H), 4.30 - 4.14 (m, 1H), 4.08 - 3.88 (m, 1H), 3.20-3.10 (m, 1H), 3.06 - 2.80 (m, 2H), 2.22 - 2.10 (m, 1H), 1.48 (s, 3H), 1.46 - 1.44 (m, 2H), 1.44 (s, 9H).

Synthesis of A180 (including SFC separation)

To tert-butyl 4-cyano-4-(1-(2,4-difluorophenyl)ethyl)piperidine-1-carboxylate (3 g, 8.56 mmol) in dioxane (5 mL) was added HCl/dioxane (4 M, 21.4 mL, 85.6 mmol), and the mixture was stirred at 20°C for 16 hours. The mixture was concentrated to give 4-(1-(2,4-difluorophenyl)ethyl)piperidine-4-carbonitrile hydrochloride (2 g). The residue was used directly in the next step. $^1H$ NMR (400 MHz, $CDCl_3$)δH 7.54 - 7.42 (m, 1H), 7.02 - 6.88 (m, 1H), 6.86 - 6.76 (m, 1H), 3.66 - 3.42 (m, 2H), 3.30 - 2.96 (m, 3H), 2.44 - 2.30 (m, 1H), 2.26 - 2.04 (m, 2H), 1.76 - 1.64 (m, 1H), 1.50 - 1.46 (m, 3H), 1.44 - 1.42 (m, 1H).

Synthesis of A181

2-(Pyrimidin-4-yl)pyridine-3-carboxylic acid (400 mg, 1.99 mmol), HOBt (888 mg, 3.98 mmol), EDCI (800 mg, 3.98 mmol), DIPEA (760 mg, 3.98 mmol) and 4-(1-(2,4-difluorophenyl)ethyl)piperidine-4-carbonitrile hydrochloride (500 mg, 1.99 mmol) were combined in DMF (10 mL). After stirring at 20°C for 2 hours, the mixture was filtered, concentrated, and purified by prep-HPLC (Column: Xtimate C18 150*40mm*5um; Condition: water(10mM $NH_4HCO_3$)-ACN; Begin B: 25; End B: 35; Gradient Time (min):8; 100%B Hold Time (min): 2) to afford racemic-4-(1-(2,4-difluorophenyl) ethyl)-1-(2-(pyrimidin-4-yl)nicotinoyl)piperidine-4-carbonitrile (300 mg, 0.692 mmol), which was further purified by SFC (Column: Chiralcel OD-3 150×4.6mm I.D., 3um Mobile phase: A: $CO_2$ B:ethanol (0.05% DEA) Gradient: from 5% to 40% of B in 5 min and hold 40% for 2.5 min, then 5% of B for 2.5 min Flow rate: 2.5mL/min Column temp.: 35°C ABPR: 1500psi) to give:

(S)-4-(1-(2,4-difluorophenyl)ethyl)-1-(2-(pyrimidin-4-yl)nicotinoyl)piperidine-4-carbonitrile (104.2 mg, 34%). $^1H$ NMR (400 MHz, $CDCl_3$) $\delta_H$ 9.30-9.00 (m, 1H), 8.95-8.71 (m, 2H), 8.34-8.18 (m, 1H), 7.77-7.42 (m, 3H), 7.07-6.66 (m, 2H), 5.02-4.77 (m, 1H), 3.77-2.89 (m, 5H), 2.49-1.63 (m, 3H), 1.54 ( s, 1H), 1.48 (d, 2H). $^{19}F$ NMR (376.5 MHz, $CDCl_3$) $\delta_F$ -110.440, -110.463, -110.575, -110.680. LCMS purity 99%, de 100%, MS ESI calcd. for $C_{24}H_{21}F_2N_5O$ [M+H] 434.2 found 434.2.

(R)-4-(1-(2,4-difluorophenyl)ethyl)-1-(2-(pyrimidin-4-yl)nicotinoyl)piperidine-4-carbonitrile (85.2 mg, 27%). $^1H$ NMR ( (400 MHz, $CDCl_3$) $\delta_H$ 9.31-9.00 (m, 1H), 8.98-8.70 (m, 2H), 8.26 (m, 1H), 7.74-7.61 (m, 1H), 7.59-7.39 (m, 2H), 7.05-6.70 (m, 2H), 5.01-4.71 (m, 1H), 3.68-2.97 (m, 4H), 2.51-1.96 (m, 1H), 1.94-1.66 (m, 2H), 1.55 (m, 2H), 1.47 (d, 2H) $^{19}F$ NMR (376.5 MHz, $CDCl_3$) $\delta_F$ -110.440,-110.463,-110.575, -110.680. LCMS purity 97%, de 99%, MS ESI calcd. for $C_{24}H_{21}F_2N_5O$ [M+H] 434.2 found 434.2.

*Example 61. Synthesis of (S)-1-([2,4'-bipyridine]-3-carbonyl)-4-(1-(2, 4-difluorophenyl)ethyl)piperidine-4-carbonitrile (A237) and (R)-1-([2,4'-bipyridine]-3-carbonyl)-4-(1-(2,4-difluorophenyl)ethyl)piperidine-4-carbonitrile (A238)*

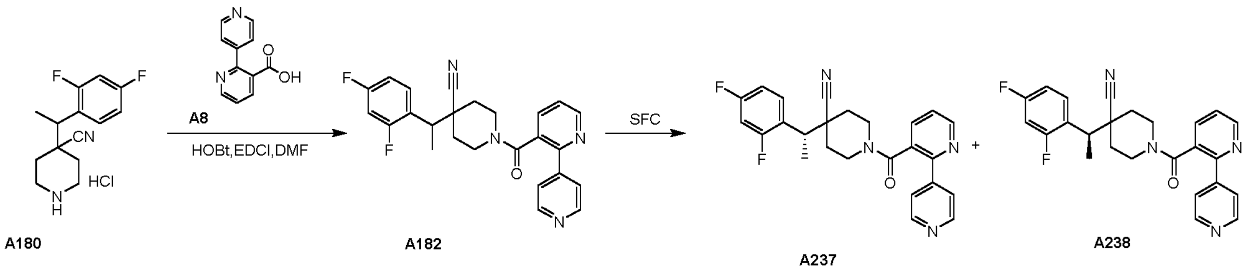


A180 A182 A237 A238

Synthesis of A182 and SFC Separation

2-(Pyrimidin-4-yl)pyridine-3-carboxylic acid (400 mg, 1.99 mmol), HATU (1.13 g, 2.98 mmol), DIPEA (760 mg, 3.98 mmol) and 4-(1-(2,4-difluorophenyl)ethyl)piperidine-4-carbonitrile hydrochloride (500 mg, 1.99 mmol) were combined in DMF (10 mL) and stirred at 20°C for 2 hours. The mixture was filtered, concentrated and purified by prep-HPLC (Column: Phenomenex Genimi NX C18 150*40 mm*5 um; Condition: water (10mM $NH_4HCO_3$)-ACN; Begin B: 30; End B: 60; Gradient Time (min): 10; 100%B Hold Time (min): 2) to afford racemic-1-([2,4'-bipyridine]-3-carbonyl)-4-(1-(2,4-difluorophenyl)ethyl)piperidine-4-carbonitrile (500 mg, 58%), which was twice purified by SFC (Column: DAICEL CHIRALPAK AD-H (250 mm*30 mm, 5 um)); Condition: 0.1% $NH_3H_2O$ ETOH; Begin B: 25%; End B: 25%) to afford: (S)-1-([2,4'-bipyridine]-3-carbonyl)-4-(1-(2,4-difluorophenyl)ethyl)piperidine-4-carbonitrile (182 mg, 36.6%). $^1H$ NMR (400 MHz, $CDCl_3$) $\delta_H$ 9.00-8.60 (m, 3H), 7.80-7.60 (m, 3H), 7.50-7.2 (m, 2H), 7.00-6.60 (m, 2H), 4.90-4.60 (m, 1H), 3.25-1.90 (m, 5H), 1.50-0.95 (m, 5H), 0.25-0 (m, 1H). $^{19}F$ NMR (376.5 MHz, $CDCl_3$) δF -110.552, -110.727. LC-ELSD/MS purity 99%, de 100%, MS ESI calcd. for $C_{25}H_{22}F_2N_4O$ $[M+H]^+$ 433.3 found 433.3. (R)-1-([2,4'-bipyridine]-3-carbonyl)-4-(1-(2,4-difluorophenyl)ethyl)piperidine-4-carbonitrile (136.6 mg, 27.3%). $^1H$ NMR (400 MHz, $CDCl_3$) $\delta_H$ 9.00-8.60 (m, 3H), 7.80-7.60 (m, 3H), 7.50-7.2 (m, 2H), 7.00-6.60 (m, 2H), 4.90-4.60 (m, 1H), 3.25-1.90 (m, 5H), 1.50-0.95 (m, 5H), 0.25-0 (m, 1H). $^{19}F$ NMR (376.5 MHz, $CDCl_3$) $\delta_F$ -110.523, -110.718. LC-ELSD/MS purity 99%, de 96%, MS ESI calcd. for $C_{25}H_{22}F_2N_4O$ $[M+H]^+$ 433.3 found 433.3.

*Example 62. Synthesis of 1-(2-(pyrimidin-4-yl)nicotinoyl)-4-((2-(trifluoromethyl)pyrimidin-5-yl)methyl)piperidine-4-carbonitrile (A239)*

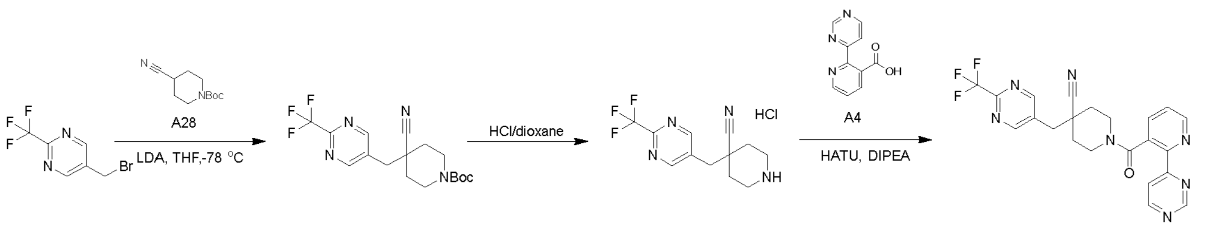


A183 A184 A185 A239

Synthesis of A184

To tert-butyl 4-cyanopiperidine-1-carboxylate (130 mg, 0.622 mmol) in THF (10 mL) was added dropwise LDA (0.46 mL, 2 M, 0.933 mmol) at -78°C. After stirring at -78°C for 1 hours, 5-(bromomethyl)-2-(trifluoromethyl)pyrimidine (150 mg, 0.622 mmol) was added. The mixture was warmed to 20°C and stirred for 16 hours. The mixture was poured into water (25 mL) and extracted with EtOAc (3 x 20 mL). The combined organic layers were washed with brine (2 x 50 mL), dried over $Na_2SO_4$, filtered and concentrated. The residue was purified by flash chromatography (10~30% of EtOAc in PE) to give tert-butyl 4-cyano-4-((2-(trifluoromethyl)pyrimidin-5-yl)methyl)piperidine-1-carboxylate (200 mg). $^1H$ NMR (400 MHz, $CDCl_3$) $\delta_H$ 8.87 (s, 2H), 3.69-3.62 (m, 2H), 3.38-3.31 (m, 2H), 2.83-2.62 (m, 3H), 1.86 (s, 3H), 1.58 (br s, 3H), 1.47 (s, 9H).

Synthesis of A185

To a solution of tert-butyl 4-cyano-4-((2-(trifluoromethyl)pyrimidin-5-yl)methyl)piperidine-1-carboxylate (200 mg,0.5414 mmol) in dioxane (2 mL) was added HCl/Dioxane (3 mL, 4M, 12.0 mmol ) at 25°C. The mixture was stirred at 25°C for 12 hours under nitrogen. The mixture was concentrated to give 4-((2-(trifluoromethyl)pyrimidin-5-yl)methyl) piperidine-4-carbonitrile hydrochloride (150 mg). $^1H$ NMR (400 MHz, $CDCl_3$) $\delta_H$ 8.92 (s, 2H), 3.57 (d, 2H), 3.32-3.31 (m,

1H), 3.28 (m, 3H), 3.05 (m, 2H), 2.42-2.29 (m, 3H).

Synthesis of A239

To a solution of 2-(pyrimidin-4-yl)pyridine-3-carboxylic acid (78.9 mg, 0.3924 mmol) in DMF (2 mL) was added HATU(186 mg, 0.4905 mmol) and DIPEA(0.170 mL, 0.9810 mmol) at 25°C, and the mixture was stirred for 30 minutes at 25°C. 4-((2-(Trifluoromethyl)pyrimidin-5-yl)methyl)piperidine-4-carbonitrile hydrochloride (100 mg, 0.3270 mmol) was added to the mixture. After stirring for 2 hours at 25°C, the mixture was poured into water (20 mL) and extracted with EtOAc (3 x 10 mL). The combined organic layers were washed with brine (2 x 10 mL), dried over $Na_2SO_4$, filtered and concentrated. The residue was purified by prep-HPLC (Column Welch Xtimate C18 150*25mm*5um, Condition water(0.05%$NH_3H_2O$)-ACN, Begin B: 30, End B :60, Gradient Time(min) 8, 100%B Hold Time(min) :2) to product (50 mg, 0.11 mmol), which was further purified by prep-HPLC (Column Phenomenex Gemini-NX 80*40mm*3um Condition water (0.05% $NH_3H_2O$+10mM $NH_4HCO_3$)-ACN Begin B 40 End B 40 Gradient Time(min) 8 100%B Hold Time(min) 2.5 FlowRate(ml/min) 30 Injections 4) to give 1-(2-(pyrimidin-4-yl)nicotinoyl)-4-((2-(trifluoromethyl)pyrimidin-5-yl)methyl) piperidine-4-carbonitrile (9.3 mg,18.6%). $^1H$ NMR (400 MHz, $CDCl_3$) $\delta_H$ 9.28-9.10 (m, 1H), 8.99-8.75 (m, 4H), 8.30 (m, 1H), 7.69 (d, 1H), 7.50 (m, 1H), 5.06-4.79 (m, 1H), 3.75-3.24 (m, 2H), 3.14-2.88 (m, 3H), 2.23-1.65 (m, 4H). $^{19}F$ NMR (376.5 MHz, $CDCl_3$) $\delta F$ -70.275. LCMS purity 98%, MS ESI calcd. for $C_{22}H_{18}F_3N_7O$ [M+H] 454.2 found 454.2.

*Example 63. Synthesis of 4-(2-fluoro-4-(trifluoromethyl)benzyl)-1-(2-(pyrimidin-4-yl)nicotinoyl)piperidine-4-carbonitrile (A240)*

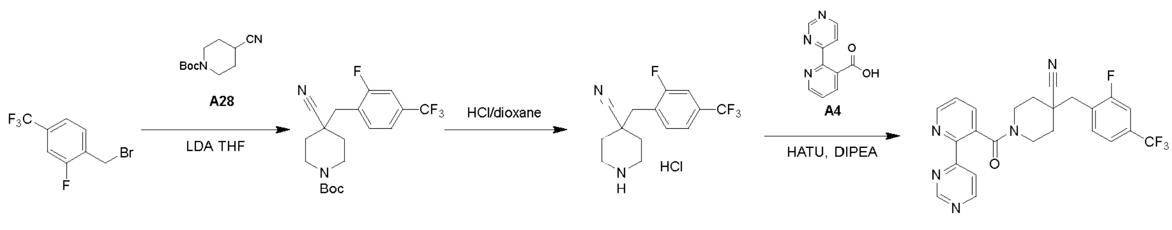


A186 A187 A188 A240

Synthesis of A187

An LDA solution (1.45 mL 2M in heptane 2.90 mmol) was added dropwise to tert-butyl 4-cyanopiperidine-1-carboxylate(487 mg, 2.32 mmol) in THF (5 mL) at -75°C during 30 minutes. Then 1-(bromomethyl)-2-fluoro-4-(trifluoromethyl)benzene (0.5 g,1.94 mmol) was added the mixture and stirring was continued at -75°C for 5 hours. The mixture was poured into water (20 mL) and extracted with EtOAc (3 x 30 mL). The combined organic layers were washed with brine (2 x 50 mL), dried over $Na_2SO_4$, filtered, and concentrated. The residue was purified by silica gel chromatography (10-50% EtOAc in PE) to give tert-butyl 4-cyano-4-(2-fluoro-4-(trifluoromethyl)benzyl)piperidine-1-carboxylate (750 mg). $^1H$ NMR (400 MHz, $CDCl_3$) $\delta_H$ 7.27 (s, 2H), 7.25-7.15 (m, 1H), 3.71 (s, 1H), 3.10-2.93 (m, 2H), 2.66 (m, 1H), 1.92-1.57 (m, 5H), 1.47 (s, 9H).

Synthesis of A188

To tert-butyl 4-cyano-4-(2-fluoro-4-(trifluoromethyl)benzyl)piperidine-1-carboxylate (750 mg, 1.94 mmol) was added HCl/dioxane (4 M, 10 mL), and the mixture was stirred at 25°C for 16 hours. The reaction mixture was concentrated to give 4-(2-fluoro-4-(trifluoromethyl)benzyl)piperidine-4-carbonitrile hydrochloride (0.5 g). $^1H$ NMR (400 MHz, $CDCl_3$) $\delta_H$ 7.57-7.36 (m, 2H), 7.24-7.18 (m, 1H), 3.58 (d, 3H), 3.19 (d, 2H), 2.34-2.06 (m, 4H), 1.81 (s, 2H), 1.26 (s, 2H).

Synthesis of A240

To a solution of 2-(pyrimidin-4-yl)nicotinic acid (372 mg, 1.85 mmol) in DMF (2 mL) were added HATU (1.05 g, 2.77 mmol), DIPEA (0.968 mL, 5.55 mmol), and 4-(2-fluoro-4-(trifluoromethyl)benzyl)piperidine-4-carbonitrile hydrochloride (600 mg, 1.85 mmol). After stirring at 25°C for 30 minutes, the mixture was poured into water (20 mL) and extracted with EtOAc (3 x 10 mL). The combined organic layers were washed with brine (2 x 10 mL), dried over $Na_2SO_4$, filtered and concentrated. The residue was purified by prep-HPLC(Column Welch Xtimate C18 150*25mm*5um, Condition

water(0.05%$NH_3H_2O$)-ACN, Begin B: 30, End B :60, Gradient Time(min) 8, 100%B Hold Time(min) :2) to give product (100 mg) which was further purified by SFC (Column: Chiralpak AD(250 mm*30 mm, 10 um), Mobile phase: A: $CO_2$ B:ethanol (0.05% DEA) Gradient: from 5% to 40% of B in 2 min and hold 40% for 1.2 min, then 5% of B for 0.8 min Flow rate: 4mL/min Column temp.: 35 °C ABPR: 1500psi) to give 4-(2-fluoro-4-(trifluoromethyl)benzyl)-1-(2-(pyrimidin-4-yl)nicotinoyl)piperidine-4-carbonitrile (56.5 mg, 56.5%). $^1H$ NMR (400 MHz, $CDCl_3$) $\delta_H$ 9.28-9.03 (m, 1H), 8.96-8.86 (m, 1H), 8.78 (m, 1H), 8.27 (m, 1H), 7.74-7.65 (m, 1H), 7.60-7.53 (m, 1H), 7.51-7.44 (m, 2H), 7.37 (m, 1H), 4.99-4.74 (m, 1H), 3.72-3.35 (m, 2H), 3.32-2.99 (m, 4H), 2.14-1.62 (m, 4H). $^{19}F$ NMR (376.5 MHz, $CDCl_3$) $\delta_F$ -62.858, -113.187, - 113.322. LCMS purity 99%, MS ESI calcd. for $C_{24}H_{19}F_4N_5O$ [M+H] 470.1 found 470.1.

*Example 64. Synthesis of (S)-4-(1-(2, 6-difluorophenyl)ethyl)-1-(2-(pyrimidin-4-yl)nicotinoyl)piperidine-4-carbonitrile (A243) and (R)-4-(1-(2, 6-difluorophenyl)ethyl)-1-(2-(pyrimidin-4-yl)nicotinoyl)piperidine-4-carbonitrile (A244)*

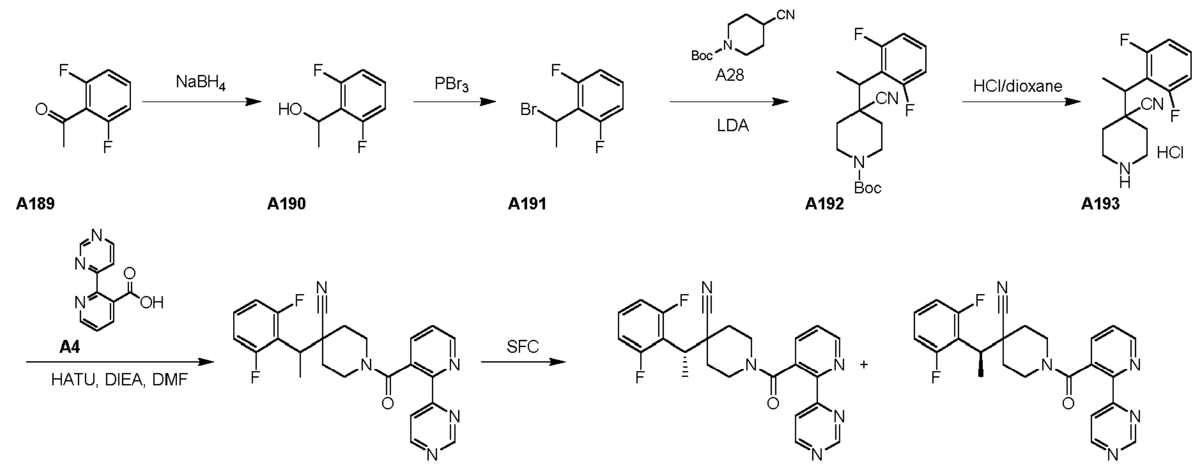


A189 A190 A191 A192 A193

A194 A243 A244

Synthesis of A190

To $NaBH_4$ (1.81 g, 48.0 mmol) in THF (50 mL) was added 1-(2,6-difluorophenyl)ethan-1-one (5 g, 32.0 mmol), and the mixture was stirred at 25°C for 1 hour. Water (20 mL) was added, and the mixture was stirred for 5 minutes and then extracted with EtOAc (2 x 20 mL). The combined organic layers were dried over $Na_2SO_4$, filtered, and concentrated to give 1-(2,6-difluorophenyl)ethan-1-ol (4.9 g, 27.0 mmol). $^1H$ NMR (400 MHz, $CDCl_3$) $\delta_H$ 7.16-7.25 (m, 1 H) 6.82-6.92 (m, 2 H) 5.19-5.31 (m, 1 H) 1.63 (d, 3 H). $^{19}H$ NMR (376.5 MHz, $CDCl_3$) $\delta_F$ -115.2.

Synthesis of A191

To a solution of 1-(2,6-difluorophenyl)ethan-1-ol (2.4 g, 15.1 mmol) in $CDCl_3$ (20 mL) was added $PBr_3$ (8.06 g, 30.6 mmol) at 0°C. The mixture was stirred at 0°C for 30 minutes. Ice-water (40 mL) was added, and the mixture was extracted with DCM (40 mL x 2). The combined organic layers were dried over $Na_2SO_4$ and concentrated to give 2-(1-bromoethyl)-1,3-difluorobenzene (1.7 g, 51.0%). $^1H$ NMR (400MHz, DMSO-$d_6$) $\delta_H$ 7.52-7.38 (m, 1H), 7.14 (t, 2H), 5.63 (q, 1H), 2.05 (d, 3H). $^{19}H$ NMR (376.5 MHz, $CDCl_3$) $\delta_F$ -112.54.

Synthesis of A192

To a solution of tert-butyl 4-cyanopiperidine-1-carboxylate (1.13g, 5.42 mmol) in THF (20 mL) was added a solution of LDA (4.52 mL, 2 M in hexane, 9.04 mmol) at -70°C. After stirring for 30 minutes, 2-(1-bromoethyl)-1,3-difluorobenzene (1 g, 4.52 mmol) was added at -70°C. The mixture was stirred at -70°C for 1 hour. The mixture was warmed to 25°C and stirred for 16 hours. Saturated aqueous $NH_4Cl$ solution (50 mL) was added, and the mixture was extracted with EtOAc (2 x 30 mL). The combined organic layers were dried over $Na_2SO_4$, filtered and concentrated. The residue was purified by silica gel chromatography (PE/EtOAc = 10/1 to 5/1) to give tert-butyl 4-cyano-4-(1-(2,6-difluorophenyl)ethyl)piperidine-1-carboxylate (1.2 g). $^1H$ NMR (400MHz, $CDCl_3$) $\delta_H$ 7.25-7.20 (m, 1H), 6.96-6.84 (m, 2H), 4.26-4.03 (m, 2H), 3.40-3.31 (m, 1H), 3.13-2.88 (m, 2H), 2.12-2.01 (m, 1H), 1.71 (d, 1H), 1.61 (d, 3H), 1.44 (s, 11H). $^{19}H$ NMR (376.5 MHz, $CDCl_3$) $\delta_F$ -104.4, -111.3.

Synthesis of A193

To tert-butyl 4-cyano-4-(1-(2,6-difluorophenyl)ethyl)piperidine-1-carboxylate (600 mg, 1.71 mmol) was added HCl/Dioxane (10 mL, 4 M, 40.0 mmol) at 25°C. The mixture was stirred at 25°C for 16 hours under nitrogen. The mixture was concentrated to give 4-(1-(2,6-difluorophenyl)ethyl)piperidine-4-carbonitrile hydrochloride (500 mg), which was used directly in the next step.

Synthesis of A194

A mixture of 2-(pyrimidin-4-yl)nicotinic acid (239 mg, 1.19 mmol), HATU (756 mg, 1.99 mmol), DIPEA (0.87 mL, 4.99 mmol) and 4-(1-(2,6-difluorophenyl)ethyl)piperidine-4-carbonitrile hydrochloride (250 mg, 0.99 mmol) in DMF (2 mL) was stirred at 15°C for 2 hours. The mixture was filtered, concentrated, and purified by prep-HPLC (Phenomenex Gemini-NX 80*30 mm*3 um; Condition: water (10mM $NH_4HCO_3$)-ACN; Begin B: 30; End B: 60; Gradient Time (min):9; 100%B Hold Time (min): 2) to afford racemic-4-(1-(2,6-difluorophenyl)ethyl)-1-(2-(pyrimidin-4-yl)nicotinoyl)piperidine-4-carbonitrile (150 mg, 34.7%) LCMS purity 91%, MS ESI calcd. For $C_{24}H_{22}F_2N_5O$ $[M+H]^+$ 434. found 433, which was further purified by SFC (Column: DAICEL CHIRALCEL OD-H(250 mm*30 mm, 5 um), Condition 0.1%$NH_3H_2O$ IPA, Begin B 30, End B 30) to give:

(S)-4-(1-(2,6-difluorophenyl)ethyl)-1-(2-(pyrimidin-4-yl)nicotinoyl)piperidine-4-carbonitrile (20.6 mg, 68.8%). $^1H$ NMR (400MHz, $CDCl_3$) $\delta_H$ 9.23 (s, 0.3H), 8.90-8.80 (m, 1H), 8.78-8.71 (m, 1H), 8.57-8.47 (m, 0.7H), 8.33-8.13 (m, 1H), 7.71-7.60 (m, 1H), 7.49-7.41 (m, 1H), 7.41-7.28 (m, 1H), 7.08-6.85 (m, 2H), 4.98-4.65 (m, 1H), 3.70-2.97 (m, 4H), 2.42-2.18 (m, 0.5H), 2.03-1.73 (m, 2.5H), 1.69-1.59 (m, 4H). LCMS purity 99%, MS ESI calcd. For $C_{24}H_{22}F_2N_5O$ $[M+H]^+$ 434. found 434.

(R)-4-(1-(2,6-difluorophenyl)ethyl)-1-(2-(pyrimidin-4-yl)nicotinoyl)piperidine-4-carbonitrile (20.6 mg, 68.8%). $^1H$ NMR (400MHz, $CDCl_3$) $\delta_H$ 9.23 (s, 0.3H), 8.90-8.81 (m, 1H), 8.78-8.71 (m, 1H), 8.51 (d, 0.7H), 8.30-8.16 (m, 1H), 7.68-7.60 (m, 1H), 7.50-7.42 (m, 1H), 7.41-7.27 (m, 1H), 7.07-6.85 (m, 2H), 4.98-4.71 (m, 1H), 3.71-2.97 (m, 3H), 2.39-2.20 (m, 0.2H), 2.04-1.71 (m, 2.5H), 1.69-1.57 (m, 4H). LCMS purity 199%, MS ESI calcd. For $C_{24}H_{22}F_2N_5O$ $[M+H]^+$ 434. found 434.

*Example 65. Synthesis of (S)-1-([2,4'-bipyridine]-3-carbonyl)-4-(1-(2, 6-difluorophenyl)ethyl)piperidine-4-carbonitrile (A245) and (R)-1-([2,4'-bipyridine]-3-carbonyl)-4-(1-(2,6-difluorophenyl)ethyl)piperidine-4-carbonitrile (A246)*

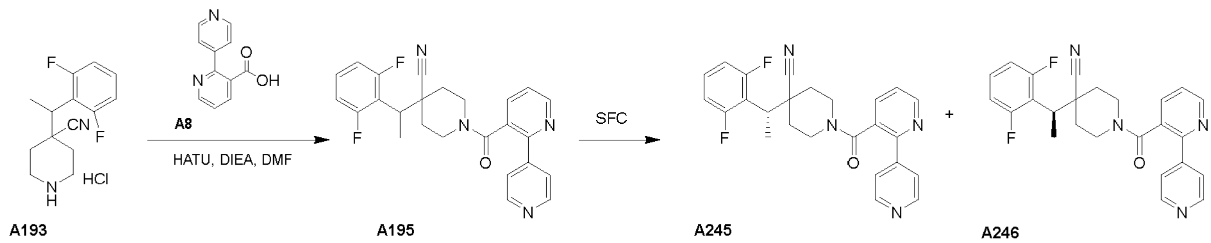


Synthesis of A195

A solution of [2,4'-bipyridine]-3-carboxylic acid (238 mg,1.19 mmol), HATU (756 mg,1.99 mmol), DIPEA (0.867 mL, 4.99 mmol) and 4-(1-(2,6-difluorophenyl)ethyl)piperidine-4-carbonitrile hydrochloride (250 mg, 0.99 mmol) in DMF (2 mL) was stirred at 15°C for 2 hours. The mixture was filtered, concentrated, and purified by prep-HPLC (Column: Phenomenex Gemini-NX 80*30 mm*3 um; Condition: water (10mM $NH_4HCO_3$)-ACN; Begin B: 28; End B: 58; Gradient Time (min):9; 100%B Hold Time (min): 2) to afford racemic-1-([2,4'-bipyridine]-3-carbonyl)-4-(1-(2,6-difluorophenyl)ethyl) piperidine-4-carbonitrile (150 mg, 34.8%).

SFC purification of A195

Racemic-1-([2,4'-bipyridine]-3-carbonyl)-4-(1-(2,6-difluorophenyl)ethyl)piperidine-4-carbonitrile (150 mg, 0.35 mmol) was further purified by SFC (Column: DAICEL CHIRALPAK IG (250 mm*30 mm, 10 um), Condition 0.1%$NH_3H_2O$ IPA, Begin B 50, End B 50) to give:
(S)-1-([2,4'-bipyridine]-3-carbonyl)-4-(1-(2,6-difluorophenyl)ethyl)piperidine-4-carbonitrile (60.2 mg, 40.4%). $^1H$ NMR (400MHz, $CDCl_3$) $\delta_H$ 8.83-8.76 (m, 1H), 8.75-8.64 (m, 2H), 7.79-7.65 (m, 2.4H), 7.63-7.54 (m, 0.6H), 7.48-7.37 (m,

1H), 7.34-7.27 (m, 0.5H), 7.25-7.19 (m, 0.5H), 6.96-6.82 (m, 2H), 4.90-4.68 (m, 1H), 3.34-2.60 (m, 4H), 2.23-2.06 (m, 0.5H), 1.94-1.61 (m, 1H), 1.55-1.33 (m, 3H), 1.32-1.01 (m, 2H), 0.25-0.09 (m, 0.5H). LCMS purity 96%, MS ESI calcd. For $C_{25}H_{23}F_2N_4O$ $[M+H]^+$ 433. found 433.

(R)-1-([2,4'-bipyridine]-3-carbonyl)-4-(1-(2,6-difluorophenyl)ethyl)piperidine-4-carbonitrile (60.2 mg, 40.4%). $^1$H NMR (400MHz, $CDCl_3$) $\delta_H$ 8.82-8.76 (m, 1H), 8.75-8.64 (m, 2H), 7.79-7.64 (m, 2.4H), 7.63-7.54 (m, 0.6H), 7.49-7.38 (m, 1H), 7.35-7.27 (m, 0.5H), 7.25-7.20 (m, 0.5H), 6.97-6.82 (m, 2H), 4.88-4.61 (m, 1H), 3.36-2.66 (m, 4H), 2.22-2.09 (m, 0.5H), 1.94-1.64 (m, 1H), 1.49-1.35 (m, 3H), 1.33-1.03 (m, 2H), 0.22--0.07 (m, 0.5H). LCMS purity 100%, MS ESI calcd. For $C_{25}H_{23}F_2N_4O$ $[M+H]^+$ 433. found 433.

*Example 66. Synthesis of (3S, 4S)-4-(4-fluorobenzyl)-3-methyl-1-(2-(pyrimidin-4-yl)nicotinoyl)piperidine-4-carbonitrile (A247) and (3R, 4R)-4-(4-fluorobenzyl)-3-methyl-1-(2-(pyrimidin-4-yl)nicotinoyl)piperidine-4-carbonitrile (A248)*

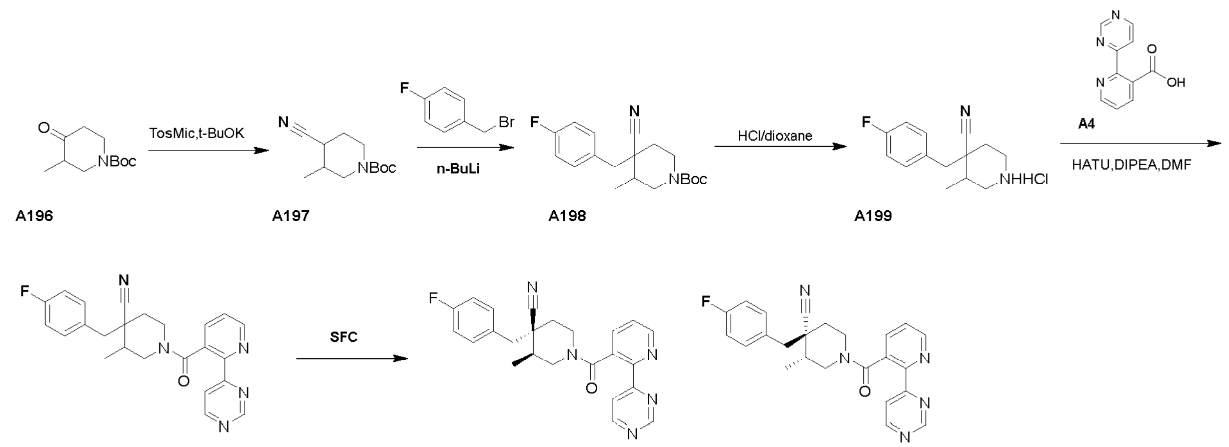


A196 A197 A198 A199

A300 A247 A248

Synthesis of A197

To tert-butyl 3-methyl-4-oxopiperidine-1-carboxylate (2 g, 9.37 mmol) and TosMic (2.35 g, 12.1 mmol) in DME (20 mL) was added t-BuOK (2.59 g, 23.2 mmol) under nitrogen at 0°C. The resulting mixture was warmed to 25°C and stirred for 16 hours. Water (50mL) was added and aqueous layer was extracted with EtOAc (50 mL). The combined organic layers were washed with brine (2 x 50 mL), dried over anhydrous $Na_2SO_4$, filtered and concentrated. The residue was purified by silica gel chromatography (PE/EtOAc = 5/1 to 3/1) to give tert-butyl 4-cyano-3-methylpiperidine-1-carboxylate (600 mg, 50.4%). $^1$H NMR (400 MHz, $CDCl_3$) $\delta_H$ 4.12-4.02 (m, 1.3H), 3.98-3.86 (m, 0.7H), 3.21-3.08 (m, 0.5H), 2.99-2.88 (m, 0.5H), 2.85-2.70 (m, 1H), 2.29-2.18 (m, 1H), 2.03-1.66 (m, 3H), 1.46 (s, 9H), 1.14-1.07 (m, 3H).

Synthesis of A198

A solution of tert-butyl 4-cyano-3-methylpiperidine-1-carboxylate (600 mg, 2.67 mmol) in THF (10 mL) was added LDA (2.67 mL, 2 M in hexane, 5.34 mmol) at -70°C. After 30 minutes, 1-(bromomethyl)-4-fluorobenzene (655 mg, 3.47 mmol) in THF (10 mL) was added at -70°C and the mixture was stirred for 2 hours. Saturated aqueous $NH_4Cl$ solution (50 mL) and the mixture was extracted with EtOAc (2 x 30 mL). The combined organic layers were dried over $Na_2SO_4$, filtered, and concentrated. The residue was purified by silica gel chromatography (PE/EtOAc = 5/1 to 3/1) to give tert-butyl 4-cyano-4-(4-fluorobenzyl)-3-methylpiperidine-1-carboxylate (300 mg, 33.8%). $^1$H NMR (400MHz, $CDCl_3$) $\delta_H$ 7.25-7.19 (m, 2H), 7.07-6.98 (m, 2H), 4.22-3.84 (m, 2H), 3.27 (d, 1H), 3.01-2.57 (m, 2H), 2.52 (d, 1H), 1.68-1.57 (m, 2H), 1.47-1.26 (m, 10H), 1.19 (d, 3H).

Synthesis of A199

To tert-butyl 4-cyano-4-(4-fluorobenzyl)-3-methylpiperidine-1-carboxylate (300 mg, 0.90 mmol) was added HCl/Dioxane (10 mL, 4M, 40.0 mmol) at 25°C, and the mixture was stirred for 16 h under nitrogen. The mixture was concentrated to give 4-(4-fluorobenzyl)-3-methylpiperidine-4-carbonitrile hydrochloride (250 mg).

Synthesis of A300

2-(Pyrimidin-4-yl)nicotinic acid (187 mg, 0.93 mmol), HATU (706 mg, 1.86 mmol), DIPEA (0.80 mL, 0.93 mmol) and 4-(4-fluorobenzyl)-3-methylpiperidine-4-carbonitrile hydrochloride (250 mg, 0.93 mmol) were combined in DMF (2 mL) and stirred at 15°C for 16 hours. The mixture was filtered, concentrated, and purified by prep-HPLC (Column: Phenomenex Gemini-NX 80*40 mm*3 um; Condition: water (0.05%$NH_3H_2O$+10mM $NH_4HCO_3$)-ACN; Begin B: 28; End B: 58; Gradient Time (min):8; 100%B Hold Time (min): 2) to afford 4-(4-fluorobenzyl)-3-methyl-1-(2-(pyrimidin-4-yl) nicotinoyl)piperidine-4-carbonitrile (150 mg, 38.8%). $^1H$ NMR (400MHz, $CDCl_3$) $\delta_H$ 8.88 (d, 1H), 8.80-8.66 (m, 2H), 8.32-8.17 (m, 1H), 7.69-7.61 (m, 1H), 7.44 (dd, 1H), 7.24-7.14 (m, 2H), 7.11-6.97 (m, 2H), 4.92-4.63 (m, 1H), 3.62-2.74 (m, 5H), 2.71-2.52 (m, 1H), 2.03-1.69 (m, 2H), 1.34 (d, 2H), 1.07-1.00 (m, 1H), which further was purified by SFC (Column: DAICEL CHIRALPAK AD(250 mm*30 mm, 10 um), Condition 0.1%$NH_3H_2O$ IPA, Begin B 30, End B 30, FlowRate (ml/min) 70) to give:
(3S,4S)-4-(4-fluorobenzyl)-3-methyl-1-(2-(pyrimidin-4-yl)nicotinoyl)piperidine-4-carbonitrile (28.6 mg, 24.0%). $^1H$ NMR (400MHz, $CDCl_3$) $\delta_H$ 9.14 (s, 0.3H), 8.86-8.78 (m, 1H), 8.74-8.62 (m, 1.7H), 8.26-8.12 (m, 1H), 7.62-7.54 (m, 1H), 7.44-7.34 (m, 1H), 7.19-7.07 (m, 2H), 7.05-6.90 (m, 2H), 4.83-4.58 (m, 1H), 3.55-2.44 (m, 5H), 1.98-1.66 (m, 2H), 1.46-1.36 (m, 0.7H), 1.27 (d, 2H), 1.01-0.93 (m, 1.3H). LCMS purity 100%, MS ESI calcd. For $C_{24}H_{23}FN_5O$ $[M+H]^+$ 416. found 416.

(3R,4R)-4-(4-fluorobenzyl)-3-methyl-1-(2-(pyrimidin-4-yl)nicotinoyl)piperidine-4-carbonitrile ( 53 mg, 44.5%). $^1H$ NMR (400MHz, $CDCl_3$) $\delta_H$ 9.21 (s, 0.3H), 8.93-8.85 (m, 1H), 8.80-8.69 (m, 1.7H), 8.32-8.15 (m, 1H), 7.70-7.60 (m, 1H), 7.50-7.42 (m, 1H), 7.26-7.13 (m, 2H), 7.12-6.96 (m, 2H), 4.90-4.64 (m, 1H), 3.60-2.50 (m, 5H), 2.04-1.71 (m, 0.7H), 1.34 (d, 2H), 1.08-0.98 (m, 1.3H). LCMS purity 100%, MS ESI calcd. For $C_{24}H_{23}FN_5O$ $[M+H]^+$ 416. found 416.

*Example 67. Synthesis of 4-(4-fluorobenzyl)-1-(6-methyl-2-(pyrimidin-4-yl)nicotinoyl)piperidine-4-carbonitrile (A249)*

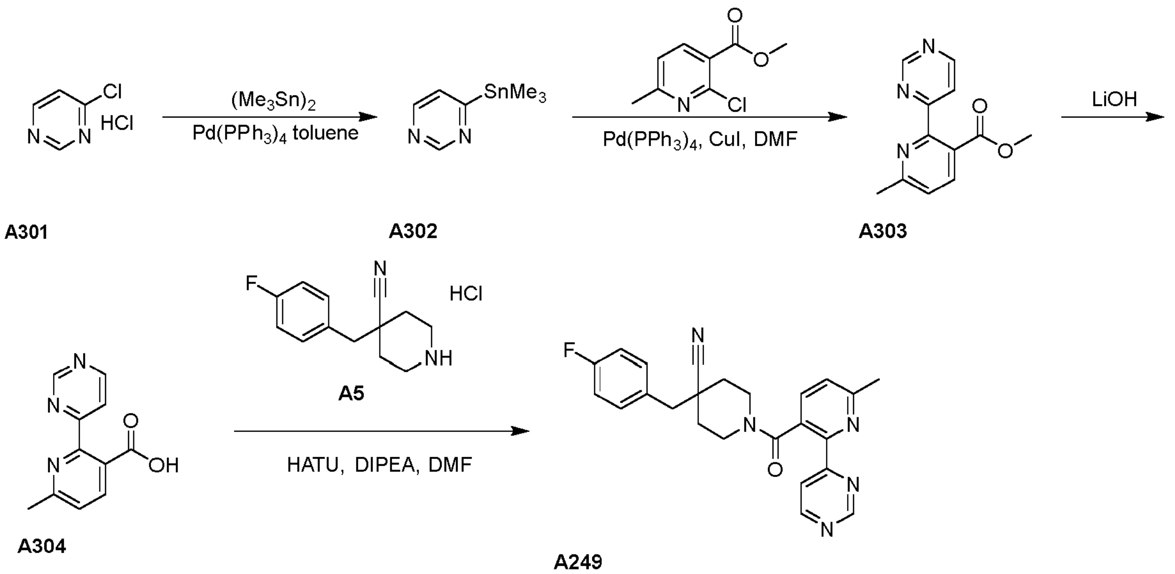


A301 A302 A303 A304 A5 A249

Synthesis of A302

To a mixture of 4-chloropyrimidine hydrochloride (2 g, 13.2 mmol) and hexamethyl ditin (6.45 g, 19.7 mmol) in toluene (40 mL) was added $Pd(PPh_3)_4$ (0.762 g, 0.66 mmol) under nitrogen at 25°C. The mixture was stirred at 110°C under nitrogen for 2 hours. The residue was poured into aq. KF (200 mL). The aqueous phase was extracted with EtOAc (3 x 100 mL). The combined organic layers were washed with brine (2 x 100 mL), dried over anhydrous $Na_2SO_4$, filtered and concentrated to afford 4-(trimethylstannyl)pyrimidine (1.8 g).

Synthesis of A303

To a solution of 4-(trimethylstannyl)pyrimidine (0.8 g, 3.29 mmol) in toluene (15 mL) was added $Pd(PPh_3)_4$ (239 mg, 0.329 mmol) and methyl 2-chloro-6-methylpyridine-3-carboxylate (792 mg, 4.27 mmol) in one portion at 25°C under

nitrogen. The reaction mixture was stirred at 110°C for 12 hours. The mixture was poured into water (30 mL) and extracted with EtOAc (3 x 50 mL). The combined organic layers were washed with brine (50 mL), dried over anhydrous $Na_2SO_4$, filtered, and concentrated. The residue was purified by flash chromatography (0~30% of EtOAc in PE) to give (0.3 g), which was re-purified by HPLC (Column: Phenomenex Gemini-NX 80*30 mm*3 um); Condition: water (water (10mM NH4HCO3)-ACN; Begin B: 20%; End B: 50%) to afford methyl 6-methyl-2-(pyrimidin-4-yl)nicotinate (120 mg, 15.9 %). $^1$H NMR (400 MHz, $CDCl_3$) $\delta_H$ 9.22 (s, 1H), 8.88 (d, 1H), 8.04 (d, 1H), 7.95 (d, 1H), 7.29 (t, 1H), 3.79 (s, 3H), 2.67 (s, 3H).

Synthesis of A304

To a solution of methyl 6-methyl-2-(pyrimidin-4-yl)nicotinate (0.12 g, 0.523 mmol) in THF (3 mL), MeOH (1 mL) and water (0.2 mL) was added $LiOH{\cdot}H_2O$ (24.9 mg, 1.04 mmol) in one portion at 25°C under nitrogen. The mixture was stirred at 25°C for 30 minutes. The mixture was concentrated under reduced pressure to afford 6-methyl-2-(pyrimidin-4-yl) nicotinic acid (110 mg).

Synthesis of A249

A solution of 6-methyl-2-(pyrimidin-4-yl)nicotinic acid (60 mg, 0.2787 mmol), HATU (158 mg, 0.4180 mmol), DIPEA (179 mg, 1.39 mmol) and 4-(4-fluorobenzyl)piperidine-4-carbonitrile hydrochloride (85.1 mg, 0.3344 mmol) in DMF (5 mL) was stirred at 25°C for 2 hours. The mixture was filtered, concentrated, and the residue was purified by prep-HPLC (Column: Phenomenex Gemini-NX 80*30 mm*3 um); Condition: water (10mM NH4HCO3)-ACN; Begin B: 32%; End B: 26%) to afford 4-(4-fluorobenzyl)-1-(6-methyl-2-(pyrimidin-4-yl)nicotinoyl)piperidine-4-carbonitrile (16.1 mg, 14.7%). $^1$H NMR (400 MHz, $CDCl_3$) $\delta_H$ 9.20-9.8.80 (m, 2H), 8.26(s, 1H), 7.60-7.52(d, 1H), 7.34-7.10 (m, 3H), 7.08-7.00 (t, 2H), 4.90-4.75 (m, 1H), 3.70-3.00 (m, 3H), 2.95-2.75 (m, 2H), 2.66 (s, 3H), 2.30-1.25 (m, 4H). $^{19}$F NMR (400 MHz, $CDCl_3$) $\delta_F$ -114.439, 114.549. LCMS purity 99%, MS ESI calcd. for $C_{24}H_{22}FN_5O$ $[M+H]^+$ 416.3, found 416.3.

*Example 68. Synthesis of (S)-1-(2-(pyrimidin-4-yl)nicotinoyl)-4-(1-(4-(trifluoromethyl)phenyl)ethyl)piperidine-4-carbonitrile (A250) and (R)-1-(2-(pyrimidin-4-yl)nicotinoyl)-4-(1-(4-(trifluoromethyl)phenyl)ethyl)piperidine-4-carbonitrile (A251)*

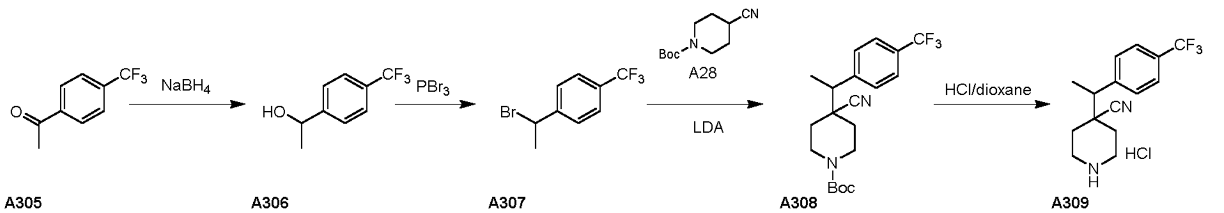


A305 A306 A307 A308 A309

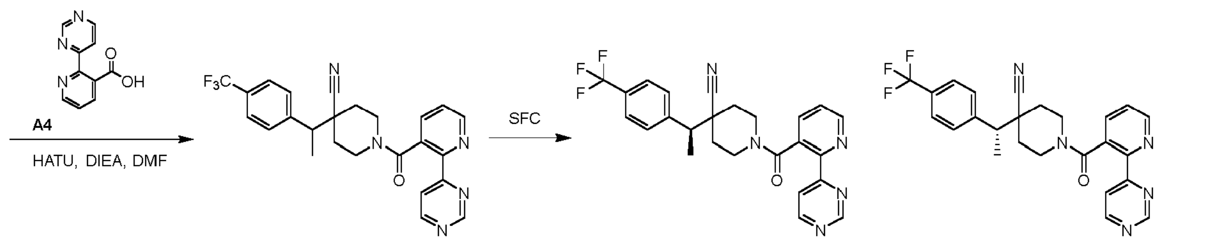


A310 A250 A251

Synthesis of A306

To $NaBH_4$ (1.65 g, 43.7 mmol) in THF (50 mL) was added 1-(4-(trifluoromethyl)phenyl)ethan-1-one (5 g, 26.5 mmol) and the mixture was stirred at 25°C for 1 hour. Ice-water was added and the mixture was stirred for 5 minutes. The mixture was extracted with EtOAc (2 x 100 mL), filtered and concentrated to give 1-(4-(trifluoromethyl)phenyl)ethan-1-ol (4.85 g).

Synthesis of A307

To a solution of 1-(4-(trifluoromethyl)phenyl)ethan-1-ol (2.4 g, 15.1 mmol) in $CHCl_3$ (20 mL) was added $PBr_3$ (6.72 g, 25.2 mmol) at 0°C. The mixture was stirred at 0°C for 30 minutes. Ice-water (40 mL) was added, and the organic phase was extracted with $NaHCO_3$ (30 mL), and then the mixture was extracted with DCM (40 mL x 2). The combined organic layers were dried over $Na_2SO_4$ and concentrated to give 1-(1-bromoethyl)-4-(trifluoromethyl)benzene (1.7 g).

Synthesis of A308

To a solution of tert-butyl 4-cyanopiperidine-1-carboxylate (529 mg, 2.52 mol) in THF (30 mL) was added LDA (3.16 mL, 2 M in hexane, 6.32 mmol) at -70°C. After stirring for 30 minutes, 1-(1-bromoethyl)-4-(trifluoromethyl)benzene (800 mg, 3.16 mmol) was added at -70°C, and mixture was stirred at -70°C for 1 hour. The mixture was warmed to 25°C and stirred for 16 hours. The mixture was poured into ice-water and stirred. Saturated $NH_4Cl$ solution (50 mL) was added, and the mixture was extracted with EtOAc (2 x 50 mL). The combined organic layers were dried over Na2SO4, filtered, and concentrated. The residue was purified by silica gel chromatography (PE/EtOAc = 10/1 to 3/1) to give tert-butyl 4-cyano-4-(1-(4-(trifluoromethyl)phenyl)ethyl)piperidine-1-carboxylate (560 mg, 46.6%). $^1H$ NMR (400 MHz, $CDCl_3$) $\delta_H$ 7.60 (d, 2 H), 7.43 (d, 2 H), 4.15-4.43 (m, 1 H), 3.92-4.09 (m, 1 H), 2.80-3.12 (m, 2 H), 2.09-2.23 (m, 1 H), 1.48-1.54 (m, 4 H), 1.44 (s, 9 H), 1.25 (t, 3 H).

Synthesis of A309

To a solution of tert-butyl 4-cyano-4-(1-(4-(trifluoromethyl)phenyl)ethyl)piperidine-1-carboxylate (240 mg, 627 mmol) in dioxane (2 mL) was added HCl/Dioxane (4 mL, 4M, 16.0 mmol ) at 25°C. The mixture was stirred at 25°C for 12 hours under nitrogen. The mixture was concentrated to give 4-(1-(4-(trifluoromethyl)phenyl)ethyl)piperidine-4-carbonitrile hydrochloride (170 mg).

Synthesis of A310 and SFC Separation

To a solution of 2-(pyrimidin-4-yl)pyridine-3-carboxylic acid (100mg, 0.501 mmol), HATU (285 mg, 0.752 mmol) in DMF (3 mL) was added DIPEA (0.8 mL) and 4-(1-(4-(trifluoromethyl)phenyl)ethyl)piperidine-4-carbonitrile hydrochloride (170 mg, 602 mmol). The mixture was stirred at 20°C for 2 hours. The mixture solution was concentrated to afford 250 mg. The product was purified by SFC (Column: DAICEL CHIRALCEL OD-H(250mm*30mm,5um), Condition 0.1%$NH_3H_2O$ IPA, Begin B 35, End B 35) to give:
(S)-1-(2-(pyrimidin-4-yl)nicotinoyl)-4-(1-(4-(trifluoromethyl)phenyl)ethyl)piperidine-4-carbonitrile ( 40.0 mg, 16.0%). $^1H$ NMR (400 MHz, $CDCl_3$) $\delta_H$ 9.21 (s, 0.3 H), 9.05 (d, 0.7 H), 8.85-8.95 (m, 1 H), 8.72-8.79 (m, 1 H), 8.25 (s, 1 H), 7.62-7.70 (m, 2 H), 7.56 (d, 1 H), 7.36-7.49 (m, 3 H), 4.66-5.00 (m, 1 H), 2.99-3.71 (m, 3 H), 2.69-2.84 (m, 1 H), 2.29 - 2.45 (m, 0.5 H), 1.68-2.14 (m, 1.5 H), 1.59 (d, 2 H), 1.52 (d, 2 H), 1.22-1.35 (m, 1 H). LC-ELSD/MS purity 97%, MS ESI calcd. For $C_{25}H_{22}F_3N_5O$ [M+H]+ 466.1 found 466.3.

(R)-1-(2-(pyrimidin-4-yl)nicotinoyl)-4-(1-(4-(trifluoromethyl)phenyl)ethyl)piperidine-4-carbonitrile ( 47.4 mg, 19.0%). $^1H$ NMR (400 MHz, $CDCl_3$) $\delta_H$ 9.21 (s, 0.3 H), 9.05 (d, 0.7 H), 8.84-8.94 (m, 1 H), 8.71-8.79 (m, 1 H), 8.22-8.30 (m, 1 H), 7.62-7.69 (m, 2 H), 7.56 (d, 1 H), 7.34-7.49 (m, 3 H), 4.65-5.02 (m, 1 H), 3.68 (d, 0.2 H), 3.41-3.56 (m, 0.8 H), 2.94-3.38 (m, 2 H), 2.77 (t, 1 H), 2.28-2.47 (m, 0.5 H), 1.91-2.14 (m, 0.5 H), 1.68-1.88 (m, 1 H), 1.59-1.67 (m, 2 H), 1.51 (d, 2 H), 1.17-1.38 (m, 1 H). LCMS purity 98%, MS ESI calcd. For $C_{25}H_{22}F_3N_5O$ $[M+H]^+$ 466.1 found 466.3.

*Example 69. Synthesis of 1-(5-cyclopropyl-2-(pyrimidin-4-yl)benzoyl)-4-(4-fluorobenzyl)piperidine-4-carbonitrile (A254)*

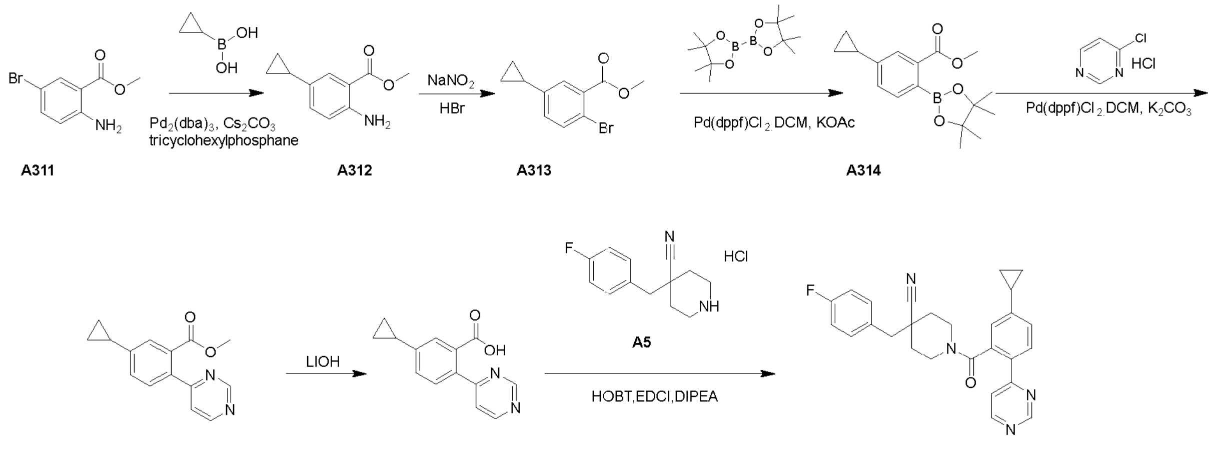


A311 A312 A313 A314

A315 A316 A254

Synthesis of A312

A mixture of methyl 2-amino-5-bromobenzoate (4 g, 17.3 mmol), cyclopropylboronic acid (2.07 g, 24.2 mmol), Pd(dppf)$Cl_2$ (1.12 g, 1.73 mmol) and $Cs_2CO_3$ (3.57g, 25.9 mmol) in dioxane (50 mL) was stirred at 100°C for 16 hours under nitrogen. The reaction mixture was poured into water (50 mL), and the aqueous layer was extracted with EtOAc (2 x 50 mL). The combined organic layers were washed with brine (2 x 50 mL), dried over anhydrous $Na_2SO_4$, filtered, concentrated. The residue was purified by flash chromatography (0~15% of EtOAc in PE) to give methyl 2-amino-5-cyclopropylbenzoate (3.26 g, 98.7%). $^1$HNMR (400MHz, $CDCl_3$) $\delta_H$ 7.59 (d, 1H), 7.03 (dd, 1H), 6.59 (d, 1H), 5.55 (br s, 2H), 3.86 (s, 3H), 1.87-1.74 (m, 1H), 0.92-0.79 (m, 2H), 0.66-0.52 (m, 2H).

Synthesis of A313

To methyl 2-amino-5-cyclopropylbenzoate (1 g, 5.22 mmol) in HBr solution (20 mL, 40% aq.) was slowly added sodium $NaNO_2$ (396 mg, 5.74 mmol) in water (10 mL) at -5°C. The mixture was stirred at -5°C for 1 hour. The reaction mixture was poured rapidly into a stirred suspension of CuBr (1.12 g, 7.83 mmol) in water (20 mL) at 70°C. Then HBr solution (10 mL) was added in one portion and stirring was continued at 70°C for 30 minutes. The reaction mixture poured into water (50 mL), and the aqueous layer was extracted with EtOAc (2 x 50 mL). The combined organic layers were washed with brine (50 mL), dried over anhydrous $Na_2SO_4$, filtered, and concentrated. The residue was purified by flash chromatography (0~5% of EtOAc in PE) to give methyl 2-bromo-5-cyclopropylbenzoate (0.7 g, 52.5%). $^1$H NMR (400MHz, $CDCl_3$) $\delta_H$ 7.55-7.44 (m, 2H), 7.02 (dd, 1H), 1.95-1.81 (m, 1H), 1.06-0.95 (m, 2H), 0.76-0.65 (m, 2H).

Synthesis of A314

A mixture of methyl 2-bromo-5-cyclopropylbenzoate (700 mg, 2.74 mmol), 4,4,5,5-tetramethyl-2-(4,4,5,5-tetramethyl-1,3,2-dioxaborolan-2-yl)-1,3,2-dioxaborolane (1.04 g, 4.11 mmol), Pd(dppf)$Cl_2$.$CH_2Cl_2$ ( 111 mg, 0.137 mmol) and KOAc ( 1.13 g, 8.22 mmol) were combined in dioxane (20 mL) and stirred at 100°C for 16 hours under nitrogen. The reaction mixture was poured into water (30 mL), and the aqueous layer was extracted with EtOAc (2 x 30 mL). The combined organic layers were washed with brine (50 mL), dried over anhydrous $Na_2SO_4$, filtered, concentrated. The residue was purified by flash chromatography (0~10% of EtOAc in PE) to give methyl 5-cyclopropyl-2-(4,4,5,5-tetramethyl-1,3,2-dioxaborolan-2-yl)benzoate (0.9 g). $^1$H NMR (400MHz, $CDCl_3$) $\delta_H$ 7.61 (s, 1H), 7.38 (d, 1H), 7.20 (br d, 1H), 1.92 (dt, 1H), 1.30-1.21 (m, 14H), 0.98 (br d, 2H), 0.78-0.67 (m, 2H).

Synthesis of A315

A mixture of methyl 5-cyclopropyl-2-(4,4,5,5-tetramethyl-1,3,2-dioxaborolan-2-yl)benzoate (900 mg, 2.97 mmol), 4-chloropyrimidine hydrochloride (403 mg, 2.67 mmol), Pd(dppf)$Cl_2$ (219 mg, 0.297 mmol) and $K_2CO_3$ (2.04 g, 14.8 mmol) was combined in dioxane (15 mL)/water (2 mL) and stirred at 100°C for 3 hours under nitrogen. The reaction mixture was poured into water (20 mL), and the aqueous layer was extracted with EtOAc (2 x 20 mL). The combined organic layers were washed with brine (40 mL), dried over anhydrous $Na_2SO_4$, and concentrated. The residue was purified by flash chromatography (0~25% of EtOAc in PE) to give methyl 5-cyclopropyl-2-(pyrimidin-4-yl)benzoate (640 mg). $^1$H NMR (400MHz, $CDCl_3$) $\delta_H$ 9.21 (s, 1H), 8.75 (d, 1H), 7.51 (s, 1H), 7.48-7.43 (m, 2H), 7.29 (br d, 1H), 3.71 (s, 3H), 2.02-1.96

(m, 1H), 1.10-1.03 (m, 2H), 0.82-0.76 (m, 2H).

Synthesis of A316

To a solution of methyl 5-cyclopropyl-2-(pyrimidin-4-yl)benzoate ( 0.64 g, 2.51 mmol) in THF (15 mL) and water(3 mL) was added LiOH·$H_2O$ (478 mg, 20.0 mmol) at 25°C .The mixture was stirred at 25°C for 16 hours. The reaction mixture was concentrated to give 5-cyclopropyl-2-(pyrimidin-4-yl)benzoic acid (600 mg). $^1H$ NMR (400MHz, $CH_3OD$) $\delta_H$ 9.11 (d, 1H), 8.76 (d, 1H), 7.64 (dd, 1H), 7.59 (d, 1H), 7.49 (d, 1H), 7.35 (dd, 1H), 2.05 (s, 1H), 1.12-1.05 (m, 2H), 0.83-0.78 (m, 2H).

Synthesis of A254

A mixture of 5-cyclopropyl-2-(pyrimidin-4-yl)benzoic acid (60 mg, 0.249 mmol), HOBt (166 mg, 0.747 mmol), EDCI (150 mg, 0.747 mmol), DIPEA (159 mg, 1.24 mmol) and 4-[(4-fluorophenyl)methyl]piperidine-4-carbonitrile (108 mg, 0.498 mmol) in DMF (6 mL) was stirred at 20°C for 16 h. The reaction mixture poured into water (20 mL), and the aqueous layer was extracted with EtOAc (2 x 25 mL). The combined organic layers were washed with brine (2 x 50 mL), dried over anhydrous $Na_2SO_4$, filtered, and concentrated. The residue was purified by prep-HPLC (Column: Phenomenex Gemini-NX 80*40mm*3um; Condition: water (0.05%$NH_3H_2O$+10mM $NH_4HCO_3$)-ACN; Begin B: 38; End B 62; Gradient Time(min): 8; 100%B Hold Time(min): 3.5 FlowRate (ml/min): 30; Injections: 6; HPLC) to give 1-(5-cyclopropyl-2-(pyrimidin-4-yl)benzoyl)-4-(4-fluorobenzyl)piperidine-4-carbonitrile (4.6 mg, 4.22%). $^1H$ NMR (400MHz, $CDCl_3$) $\delta_H$ 9.19 (s, 1H), 9.00 (s, 1H), 8.79-8.69 (m, 1H), 7.69-7.51 (m, 2H), 7.25-7.14 (m, 3H), 7.09-6.98 (m, 3H), 4.86-4.68 (m, 1H), 3.61 (br d, 1H), 3.50-3.36 (m, 1H), 3.34-3.23 (m, 1H), 3.02 (br t, 1H), 2.92-2.71 (m, 2H), 2.06-1.88 (m, 2H), 1.58 (br s, 2H), 1.45-1.35 (m, 1H), 1.08 (br s, 2H), 0.77 (br s, 2H). LC/MS: purity 98%; MS ESI calcd. for $C_{27}H_{23}N_5O$ $[M+H]^+$ 441.2, found 441.2.

*Example 70. Synthesis of 1-([2, 4'-bipyridine]-3-carbonyl)-4-(2-fluoro-4-(trifluoromethyl)benzyl)piperidine-4-carbonitrile (A255)*

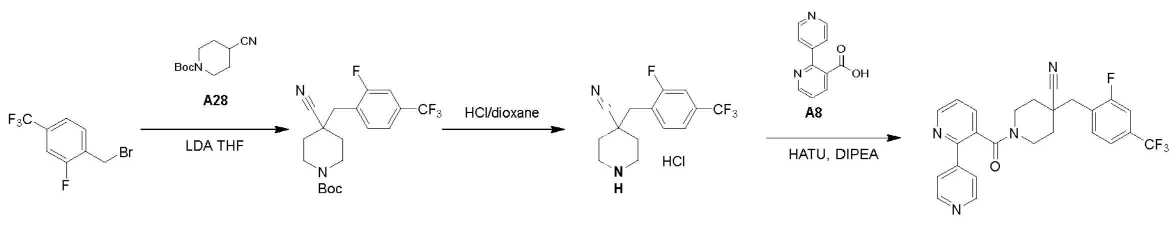


A317 A318 A319 A255

Synthesis of A318

LDA solution (1.45 mL 2M in heptane 2.90 mmol) was slowly added to a solution of tert-butyl 4-cyanopiperidine-1-carboxylate (487 mg, 2.32 mmol) in THF (5 mL) at -75°C during 30 minutes. Then 1-(bromomethyl)-2-fluoro-4-(trifluoromethyl)benzene (0.5 g,1.94 mmol) was added the mixture and stirring was continued at -75°C for 5 hours. The mixture was poured into water (20 mL) and extracted with EtOAc (3 x 30 mL). The combined organic layers were washed with brine (2 x 50 mL), dried over $Na_2SO_4$, filtered, and concentrated. The residue was purified by silica gel chromatography (10-50% EtOAc in PE) to give tert-butyl 4-cyano-4-(2-fluoro-4-(trifluoromethyl)benzyl)piperidine-1-carboxylate (750 mg). $^1H$ NMR (400 MHz, $CDCl_3$) $\delta_H$ 7.27 (s, 2H), 7.25-7.15 (m, 1H), 3.71 (s, 1H), 3.10-2.93 (m, 2H), 2.66 (m, 1H), 1.92-1.57 (m, 5H), 1.47 (s, 9H).

Synthesis of A319

To tert-butyl 4-cyano-4-(2-fluoro-4-(trifluoromethyl)benzyl)piperidine-1-carboxylate (750 mg, 1.94 mmol) was added HCl/dioxane (4 M, 10 mL), and the mixture was stirred at 25°C for 16 hours. The reaction mixture was concentrated to give 4-(2-fluoro-4-(trifluoromethyl)benzyl)piperidine-4-carbonitrile hydrochloride (0.5 g). $^1H$ NMR (400 MHz, $CDCl_3$) $\delta_H$ 7.57-7.36 (m, 2H), 7.24-7.18 (m, 1H), 3.58 (d, 3H), 3.19 (d, 2H), 2.34-2.06 (m, 4H), 1.81 (s, 2H), 1.26 (s, 2H).

Synthesis of A255

To a solution of [2,4'-bipyridine]-3-carboxylic acid (186 mg, 0.9295 mmol) in DMF (2 mL) was added HATU (528 mg, 1.39 mmol) and DIPEA (0.48 mL, 2.78 mmol) was added 4-(2-Fluoro-4-(trifluoromethyl)benzyl)piperidine-4-carbonitrile hydrochloride (300 mg, 0.9295 mmol) at 25°C and stirring was continued at 25°C for 2 hours. The mixture was poured into water (20 mL) and extracted with EtOAc (3 x 10 mL). The combined organic layers were washed with brine (2 x 10 mL), dried over $Na_2SO_4$, filtered and concentrated. The residue was purified by prep-HPLC (Column Welch Xtimate C18 150*25mm*5um, Condition water (0.05%$NH_3H_2O$)-ACN, Begin B: 30, End B:60, Gradient Time(min) 8, 100%B Hold Time(min) :2) to give 1-([2,4'-bipyridine]-3-carbonyl)-4-(2-fluoro-4-(trifluoromethyl)benzyl)piperidine-4-carbonitrile (200 mg). A portion of the product (120 mg) was further purified by prep-HPLC (Column Phenomenex Gemini-NX 80*40mm*3um Condition water(0.05%$NH_3H_2O$+10mM NH4HCO3)-ACN Begin B 29 End B 59 Gradient Time(min) 8 100%B Hold Time(min) 3 FlowRate(ml/min) 30 Injections 7) to give 1-([2,4'-bipyridine]-3-carbonyl)-4-(2-fluoro-4-(trifluoromethyl)benzyl)piperidine-4-carbonitrile (39.5 mg, 33.1%). $^1$H NMR (400 MHz, $CDCl_3$) $\delta_H$ 8.85-8.69 (m, 3H), 7.60 (s, 1H), 7.83-7.55 (m, 2H), 7.50-7.29 (m, 4H), 4.93-4.74 (m, 1H), 3.29-2.85 (m, 3H), 2.74-2.53 (m, 2H), 2.03-1.80 (m, 1H), 1.65 (d, 1H), 1.39-1.21 (m, 2H), 0.08--0.07 (m, 1H) $^{19}$F NMR (376.5 MHz, $CDCl_3$) $\delta_F$ -62.877,-62.905,-12.688. LCMS purity 99%, MS ESI calcd. for $C_{25}H_{20}F_4N_4O$ [M+H] 469.2 found 469.2.

*Example 71. Synthesis of (S)-4-(1-(3,5-difluorophenyl)ethyl)-1-(2-(pyrimidin-4-yl)nicotinoyl)piperidine-4-carbonitrile (A257) and (R)-4-(1-(3,5-difluorophenyl)ethyl)-1-(2-(pyrimidin-4-yl)nicotinoyl)piperidine-4-carbonitrile (A258)*

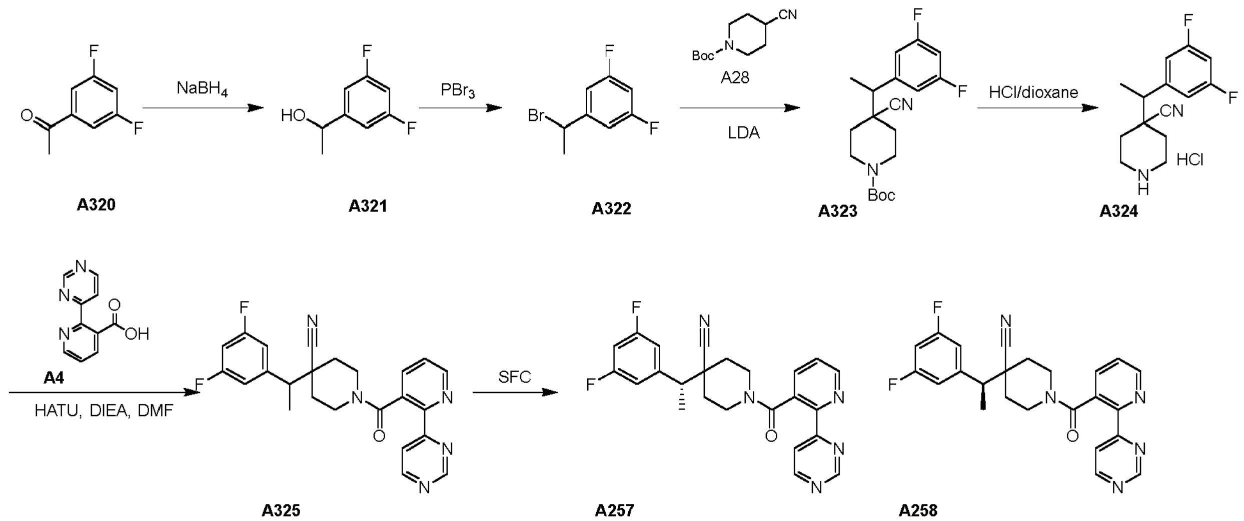


A320 A321 A322 A323 A324

A325 A257 A258

Synthesis of A321

To $NaBH_4$ (1.81 g, 48.0 mmol) in THF (50 mL) was added 1-(3,5-difluorophenyl)ethan-1-one (5 g, 32.0 mmol) and the mixture was stirred at 25°C for 1 hour. Saturated aqueous $Na_2S_2O_3$ solution (50 mL) and stirred was continued for 30 minutes. The mixture was extracted with EtOAc (2 x 50 mL). The combined organic layers were dried over $Na_2SO_4$, filtered and concentrated to give 1-(3,5-difluorophenyl)ethan-1-ol (4.9 g). $^1$H NMR (400 MHz, $CDCl_3$) $\delta_H$ 6.95-6.80 (m, 2H), 6.75-6.65 (m, 1H), 4.90-4.80 (m, 1H), 1.50-1.42 (d, 3H). $^{19}$F NMR (376.5 MHz, $CDCl_3$) $\delta_F$ -109.60.

Synthesis of A322

To a solution of 1-(3,5-difluorophenyl)ethan-1-ol (1 g, 6.32 mmol) in $CHCl_3$ (20 mL) was added tribromophosphane (8.52 g, 2.98 mL, 31.5 mmol) in one portion at 25°C under nitrogen. The mixture was stirred at 25°C for 2 hours. The mixture was slowly poured into ice-water (50 mL) and extracted with EtOAc (3 x 20 mL). The combined organic layers were washed with brine (2 x 50 mL), dried over anhydrous $Na_2SO_4$, filtered and concentrated to give 1-(1-bromoethyl)-3,5-difluorobenzene (430 mg). $^1$H NMR (400 MHz, $CDCl_3$) $\delta_H$ 7.05-6.94 (m, 2H), 6.72-6.70 (m, 1H), 5.09-5.08 (q, 1H), 2.05-1.95 (d, 3H).

Synthesis of A323

To a solution of tert-butyl 4-cyanopiperidine-1-carboxylate (487 mg, 2.32 mmol) in THF (5 mL) was added LDA (1.94 mL, 2 M in hexane, 3.88 mmol) at -70°C during 30 minutes. 1-(1-Bromoethyl)-3,5-difluorobenzene (430 mg, 1.94 mmol) in THF (5 mL) was added at -70°C and the mixture was stirred at -70°C for 1 hour. Saturated aqueous $NH_4Cl$ solution (50 mL) was added, and the mixture was extracted with EtOAc (2 x 30 mL). The combined organic layers were dried over $Na_2SO_4$, filtered, and concentrated. The residue was purified by flash chromatography (0~15% of EtOAc in PE) to give tert-butyl 4-cyano-4-(1-(3,5-difluorophenyl)ethyl)piperidine-1-carboxylate (430 mg, 63.3%). $^1H$ NMR (400 MHz, $CDCl_3$) $\delta_H$ 6.85-6.83 (m, 2H), 6.80-6.74 (m, 1H), 3.10-2.80 (m, 2H), 2.69-2.58 (m, 1H), 2.15-2.07 (m, 1H), 1.52-1.40 (m, 13H), 1.39-1.25 (d, 3H). $^{19}F$ NMR (376.5 MHz, $CDCl_3$) $\delta_F$ - 108.95.

Synthesis of A324

To a solution of tert-butyl 4-cyano-4-(1-(3,5-difluorophenyl)ethyl)piperidine-1-carboxylate (430 mg, 1.22 mmol) in dioxane (10 mL) was added HCl/dioxane (0.61 mL, 4M HCl in dioxane) at 25°C under nitrogen. The mixture was stirred at 25°C for 16 hours. The reaction mixture was concentrated to give 4-(1-(3,5-difluorophenyl)ethyl)piperidine-4-carbonitrile hydrochloride (300 mg). LC-ELSD/MS purity 66%, MS ESI calcd. for $C_{14}H_{16}F_2N_2$ $[M+H]^+$ 250.0, found 250.0.

Synthesis of A325 and SFC Separation

A mixture of 4-[(4-fluorophenyl)methyl]piperidine-4-carbonitrile hydrochloride (120 mg, 0.419 mmol), HATU (341 mg, 0.8988 mmol), DIPEA (386 mg, 2.99 mmol) and 2-(pyrimidin-4-yl)nicotinic acid (150 mg, 0.746 mmol) in DMF (5 mL) was stirred at 0°C for 2 hours. The mixture was poured into water (20 mL) and extracted with EtOAc (3 x 10 mL). The combined organic layers were washed with brine (2 x 20 mL), dried over anhydrous $Na_2SO_4$, filtered, concentrated. The residue was purified by HPLC (Column: Phenomenex Gemini-NX 80*30 mm*3 um);Condition: water(10mM $NH_4HCO_3$)-ACN; Begin B: 33%; End B: 63%) to afford racemic-4-(1-(3,5-difluorophenyl)ethyl)-1-(2-(pyrimidin-4-yl) nicotinoyl)piperidine-4-carbonitrile (160 mg). LC-ELSD/MS purity 90%, MS ESI calcd. for $C_{24}H_{21}F_2N_5O$ $[M+H]^+$ 434.1, found 434.1. The residue was further purified by SFC (Column: DAICEL CHIRALCEL OD-H (250 mm*30 mm, 5 um); Condition: 0.1% $NH_3H_2O$ ETOH; Begin B: 30%; End B: 30%) to give:
(S)-4-(1-(3,5-difluorophenyl)ethyl)-1-(2-(pyrimidin-4-yl)nicotinoyl)piperidine-4-carbonitrile (53.7 mg, 33.7%). $^1H$ NMR (400 MHz, $CDCl_3$) $\delta_H$ 9.25-8.85 (m, 2H), 8.80-8.70 (m, 1H), 8.32-8.19 (s, 1H), 7.72-7.60 (m, 1H), 7.31-7.20 (m, 1H), 6.93-6.67 (m, 3H), 4.98-4.69 (m, 1H), 3.77-2.90 (m, 3H), 2.76-2.58 (m, 1H), 2.43-1.99 (m, 1H), 1.98-1.66 (m, 1H), 1.65-1.21 (m, 5H).$^{19}F$ NMR (376.5 MHz, $CDCl_3$) $\delta_F$ -108.50. LC-ELSD/MS purity 99%, MS ESI calcd. for $C_{24}H_{21}F_2N_5O$ $[M+H]^+$ 434.2, found 434.2.

(R)-4-(1-(3,5-difluorophenyl)ethyl)-1-(2-(pyrimidin-4-yl)nicotinoyl)piperidine-4-carbonitrile (44.9 mg, 28.2%). $^1H$ NMR (400 MHz, $CDCl_3$) $\delta_H$ 9.26-8.84 (m, 2H), 8.80-8.70 (m, 1H), 8.32-8.19 (s, 1H), 7.72-7.60 (m, 1H), 7.31-7.20 (m, 1H), 6.93-6.67 (m, 3H), 4.98-4.69 (m, 1H), 3.77-2.90 (m, 3H), 2.76-2.58 (m, 1H), 2.43-1.99 (m, 1H), 1.98-1.66 (m, 1H), 1.65-1.21 (m, 5H).$^{19}F$ NMR (376.5 MHz, $CDCl_3$) $\delta_F$ -108.50. LC-ELSD/MS purity 99%, MS ESI calcd. for $C_{24}H_{21}F_2N_5O$ $[M+H]^+$ 434.2, found 434.2.

*Example 72. Synthesis of (S)-1-([2,4'-bipyridine]-3-carbonyl)-4-(1-(3,5-difluorophenyl)ethyl)piperidine-4-carbonitrile (A259) and (R)-1-([2,4'-bipyridine]-3-carbonyl)-4-(1-(3,5-difluorophenyl)ethyl)piperidine-4-carbonitrile (A260)*

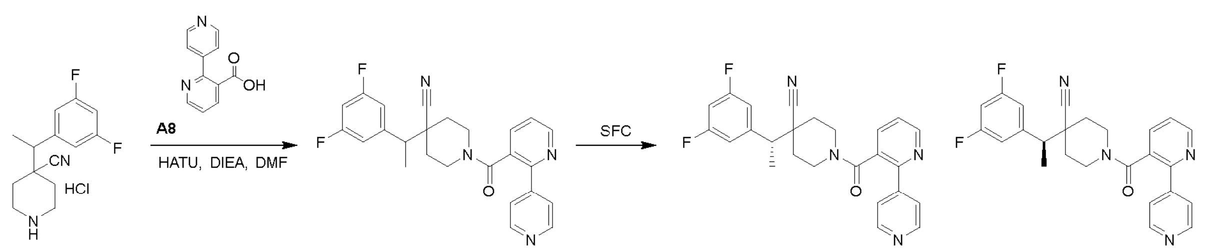


A324 A326 A259 A260

Synthesis of A326

A mixture of [2,4'-bipyridine]-3-carboxylic acid (119 mg, 0.5992 mmol), HATU (341 mg, 0.8988 mmol), DIPEA

(386 mg, 2.99 mmol) and 4-(1-(3,5-difluorophenyl)ethyl)piperidine-4-carbonitrile hydrochloride (150 mg, 0.5992 mmol) in DMF (5 mL) was stirred at 0°C for 2 hours. The mixture was poured into water (20 mL) and extracted with EtOAc (3 x 10 mL). The combined organic layers were washed with brine (2 x 20 mL), dried over anhydrous $Na_2SO_4$, filtered, and concentrated. The residue was purified by HPLC (Column: Phenomenex Gemini-NX 80*30 mm*3 um); Condition: water (10mM $NH_4HCO_3$)-ACN; Begin B: 31%; End B: 61%) to afford racemic-1-([2,4'-bipyridine]-3-carbonyl)-4-(1-(3,5-difluorophenyl)ethyl)piperidine-4-carbonitrile (180 mg). LCMS purity 98%, MS ESI calcd. for $C_{25}H_{22}F_2N_4O$ $[M+H]^+$ 433.1, found 433.1. The residue was further purified by SFC (Column: DAICEL CHIRALCEL OD-H (250 mm*30 mm, 5 um)); Condition: 0.1% $NH_3H_2O$ ETOH; Begin B: 25%; End B: 25%) to give:

(S)-1-([2,4'-bipyridine]-3-carbonyl)-4-(1-(3,5-difluorophenyl)ethyl)piperidine-4-carbonitrile (60.1 mg, 33.7 %). $^1$H NMR (400 MHz, $CDCl_3$) $\delta_H$ 9.25-8.85 (m, 2H), 8.80-8.70 (m, 1H), 8.32-8.19 (s, 1H), 7.72-7.60 (m, 1H), 7.31-7.20 (m, 1H), 6.93-6.67 (m, 3H), 4.98-4.69 (m, 1H), 3.77-2.90 (m, 3H), 2.76-2.58 (m, 1H), 2.43-1.99 (m, 1H), 1.98-1.66 (m, 1H), 1.65-1.21 (m, 5H). $^{19}$F NMR (376.5 MHz, $CDCl_3$) $\delta_F$ -108.72. LCMS purity 99%, MS ESI calcd. for $C_{25}H_{22}F_2N_4O$ $[M+H]^+$ 433.1, found 433.1.

(R)-1-([2,4'-bipyridine]-3-carbonyl)-4-(1-(3,5-difluorophenyl)ethyl)piperidine-4-carbonitrile (52.1 mg, 28.2 %). $^1$H NMR (400 MHz, $CDCl_3$) $\delta_H$ 9.25-8.85 (m, 2H), 8.80-8.70 (m, 1H), 8.32-8.19 (s, 1H), 7.72-7.60 (m, 1H), 7.31-7.20 (m, 1H), 6.93-6.67 (m, 3H), 4.98-4.69 (m, 1H), 3.77-2.90 (m, 3H), 2.76-2.58 (m, 1H), 2.43-1.99 (m, 1H), 1.98-1.66 (m, 1H), 1.65-1.21 (m, 5H). $^{19}$F NMR (376.5 MHz, $CDCl_3$) $\delta_F$ -108.72. LCMS purity 99%, MS ESI calcd. for $C_{25}H_{22}F_2N_4O$ $[M+H]^+$ 433.1, found 433.1.

*Example 73. Synthesis of 4-(2,5-difluorobenzyl)-1-(2-(pyrimidin-4-yl)nicotinoyl)piperidine-4-carbonitrile (A261)*

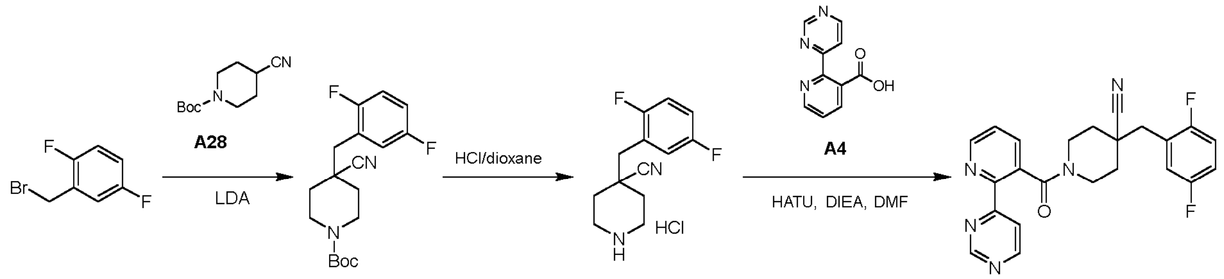

A327 A328 A329 A261

Synthesis of A328

LDA solution (5.70 mL, 11.4 mmol) was added to tert-butyl 4-cyanopiperidine-1-carboxylate (2 g, 9.51 mmol) in THF (10 mL) at 0°C during 30 minutes. Then 2-(bromomethyl)-1,4-difluorobenzene (2.36 g, 11.4 mmol) was added the mixture, and the mixture was stirred at 25°C for 16 hours. The reaction mixture was poured into water (10 mL) and extracted with EtOAc (10 mL x 3). The combined organic layers were washed with brine (2 x 10 mL), dried over $Na_2SO_4$, filtered, and concentrated to give tert-butyl 4-cyano-4-(2,5-difluorobenzyl)piperidine-1-carboxylate (3.5 g). $^1$H NMR (400 MHz, $CDCl_3$) $\delta_H$ 7.26 - 6.94 (m, 4H), 2.70-2.63 (m,4H), 1.875-1.842 (m,2H), 1.596-1.554 (m,2H),1.454 (s,9H). $^{19}$F NMR (376.5 MHz, $CDCl_3$) $\delta_F$ -118.215, -121.614.

Synthesis of A329

To a solution of tert-butyl 4-cyano-4-(2,5-difluorobenzyl)piperidine-1-carboxylate (300 mg, 0.892 mmol) in dioxane (2 mL) was added the dioxane/HCl (4.0 mL) at 25°C and mixture was stirred for 2 hours. The reaction solution was concentrated to give 4-(2,5-difluorobenzyl)piperidine-4-carbonitrile hydrochloride (200 mg). $^1$H NMR (400 MHz, $CDCl_3$) $\delta_H$ 7.089-7.016 (m,3 H),3.74 (s, 1H), 3.605-3.573 (m, 1H), 3.190 - 3.162 (m, 2H), 2.980 (s, 2H), 2.273-2.209 (m, 2H), 2.172-2.076 (s, 1H), 1.870-1.770(m,2H), 1.260-1.245(m, 1H) $^{19}$F NMR (376.5 MHz, $CDCl_3$) $\delta_F$ -117.865, -121.083.

Synthesis of A261

A mixture of 4-[(4-fluorophenyl)methyl]piperidine-4-carbonitrile hydrochloride (203 mg, 1.01 mmol), HATU (479 mg,1.26 mmol), DIPEA (546 mg, 4.23 mmol) and 2-(pyrimidin-4-yl)nicotinic acid (200 mg, 0.846 mmol) in DMF (5 mL) was stirred at 0°C for 2 hours. The mixture was poured into water (10 mL) and extracted with EtOAc (10 mL x 3). The combined organic layers were washed with brine (2 x 10 mL), dried over $Na_2SO_4$, filtered, and concentrated. The residue (300 mg)

was purified by prep-HPLC (Column: Phenomenex Gemini-NX 80*40mm*3um; Condition: water(0.05%$NH_3H_2O$+10mM $NH_4HCO_3$)-ACN; Begin B: 31; End B: 51) to afford 4-(2,5-difluorobenzyl)-1-(2-(pyrimidin-4-yl)nicotinoyl)piperidine-4-carbonitrile (106.2 mg, 29.8%). $^1$H NMR (400 MHz, $CDCl_3$) $\delta_H$ 9.218-8.753 (m, 3H), 8.280-8.234 (m, 1H), 7.698-7.855 (m, 1H), 7.480-7.449 (m,1H), 7.102-6.997 (m, 3H), 4.920-4.887 (m, 2H), 3.654-3.402 (m, 1H), 3.154-2.960(m, 3H),2.048-1.654 (m, 4H) $^{19}$F NMR (376.5 MHz, $CDCl_3$) $\delta_F$ -117.822, -117.868, -121.423, -112.469, -112.709, -112.799. LC-ELSD/MS purity >99%, MS ESI calcd. for $C_{23}H_{20}F_2N_5O$ [M+H]$^+$ 420.2, found 420.2.

*Example 74.* Synthesis of *1-(2-(pyrimidin-4-yl)nicotinoyl)-4-(2-(trifluoromethyl)benzyl)piperidine-4-carbonitrile (A262)*

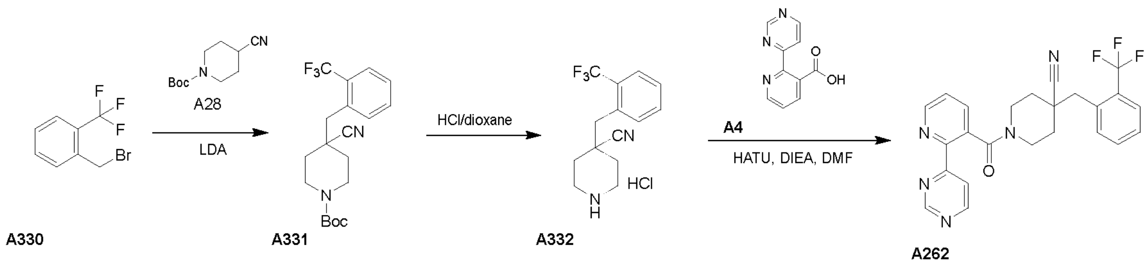


A330 A331 A332 A262

Synthesis of A331

To a solution of tert-butyl 4-cyanopiperidine-1-carboxylate (1.40 g, 6.68 mol) in THF (30 mL) was added LDA solution (8.35 mL, 2 M in hexane, 16.7 mmol) at -70°C. After stirring 30 minutes, 1-(bromomethyl)-2-(trifluoromethyl) benzene (2 g, 8.36 mmol) in THF (10 mL) was added. The mixture was stirred at -70°C for 1 hour. The reaction mixture was poured into ice-water and extracted with EtOAc (2 x 50 mL). The combined organic layers were dried over $Na_2SO_4$, filtered, and concentrated. The residue was purified by silica gel chromatography (PE/EtOAc = 10/1 to 3/1) to give tert-butyl 4-cyano-4-(2-(trifluoromethyl)benzyl)piperidine-1-carboxylate (1.7 g, 55.3%). $^1$H NMR (400 MHz, $CDCl_3$) $\delta_H$ 7.77 (d, 1 H), 7.69 (d, 1 H), 7.57 (t, 1 H), 7.38-7.45 (m, 1 H), 4.00-4.27 (m, 2 H), 3.10 (s, 2 H), 3.00 (s, 2 H), 1.82 (d, 2 H), 1.58-1.62 (m, 1 H), 1.50-1.57 (m, 2 H), 1.45 (s, 9 H).

Synthesis of A332

To a solution of tert-butyl 4-cyano-4-(2-(trifluoromethyl)benzyl)piperidine-1-carboxylate (1.7 g, 54.1mmol) in dioxane (60 mL) was added HCl/dioxane (40 mL, 4M, 160 mmol ) at 25°C. The mixture was concentrated to give 4-(2-(trifluoromethyl)benzyl)piperidine-4-carbonitrile hydrochloride (1.4 g), which was used directly in the next reaction.

Synthesis of A262

To a mixture of 2-(pyrimidin-4-yl)pyridine-3-carboxylic acid (93.5 mg, 0.465 mmol), HATU (265 mg, 0.698 mmol) in DMF (2 mL) was added DIPEA (0.8 mL) and 4-(2-(trifluoromethyl)benzyl)piperidine-4-carbonitrile hydrochloride (150 mg, 0.559 mmol). The mixture was stirred at 20°C for 2 hours. The mixture was concentrate, and the residue was purified by HPLC (Column: Phenomenex Gemini-NX 80*30mm*3um water (10mM $NH_4HCO_3$)-ACN Begin B 32 End B 62) to afford 1-(2-(pyrimidin-4-yl)nicotinoyl)-4-(2-(trifluoromethyl)benzyl)piperidine-4-carbonitrile (99.2 mg, 47.4%). $^1$H NMR (400 MHz, $CDCl_3$) $\delta_H$ 9.22 (s, 0.4 H), 9.01 (s, 0.6 H), 8.84-8.93 (m, 1 H), 8.75 (dd, 1 H), 8.19-8.30 (m, 1 H), 7.75-7.82 (m, 1 H), 7.63-7.73 (m, 2 H), 7.58 (t, 1 H), 7.39-7.50 (m, 2 H), 4.74-4.96 (m, 1 H), 3.64 (d, 0.6 H), 3.35-3.54 (m, 1 H), 3.04-3.31 (m, 3.4 H), 1.83-2.11 (m, 2.4 H), 1.62-1.78 (m, 1.6 H). $^{19}$F NMR (376.5 MHz, $CDCl_3$) $\delta_F$ -56.680. LCMS purity 99%, MS ESI calcd. For $C_{24}H_{20}F_3N_5O$ [M+H]+ 452.1 found 452.2.

*Example 75. Synthesis of 1-(2-(pyrimidin-4-yl)nicotinoyl)-4-(2,4,6-trifluorobenzyl)piperidine-4-carbonitrile (A263)*

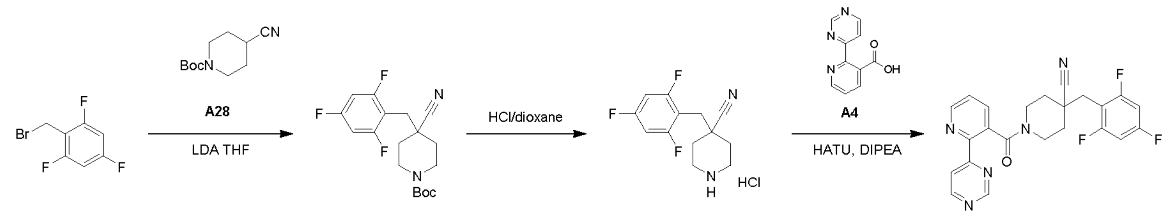


A333 A334 A335 A263

Synthesis of A334

To a mixture of tert-butyl 4-cyanopiperidine-1-carboxylate (500 mg, 2.22 mmol) in THF (10 mL) was added dropwise LDA solution (1.66 mL, 3.33 mmol, 2 M in THF) at -78°C and stirring was continued at -78°C for 1 hour under nitrogen. Then 2-(bromomethyl)-1,3,5-trifluorobenzene (605 mg, 2.88 mmol) was added, and the mixture was warmed to 25°C stirred for 16 hrs. Water (30mL) was added, and the mixture was extracted with EtOAc (3 x 40 mL). The combined organic layers were washed with brine (2 x 20 mL), dried over anhydrous $Na_2SO_4$, filtered and concentrated to give tert-butyl 4-cyano-4-(2,4,6-trifluorobenzyl)piperidine-1-carboxylate (850 mg). $^1H$ NMR (400 MHz, $CDCl_3$) $\delta_H$ 7.24-7.05 (m, 1H), 6.76-6.67 (m, 1H), 2.95 (s, 2H), 1.90 (d, 2H), 1.63-1.50 (m, 2H), 1.45 (s, 9H), 1.25 (d, 2H) $^{19}F$ NMR (376.5MHz, $CDCl_3$) $\delta_F$ -107.14- -112.31.

Synthesis of A335

To a solution of tert-butyl 4-cyano-4-(2,4,6-trifluorobenzyl)piperidine-1-carboxylate (850 mg, 2.39 mmol) in DMF (10 mL) was added HCl/dioxane (10 mL, 20.0 mmol, 2 M). The mixture was stirred at 20°C for 1 hour. The reaction mixture concentrated to give 4-(2,4,6-trifluorobenzyl)piperidine-4-carbonitrile hydrochloride (800 mg). LCMS purity 96.5%; MS ESI calcd. for $C_{18}H_{27}F_3N_3O_3$ $[M + H]^+$ 255.0, found 255.0.

Synthesis of A263

To a mixture of 2-(pyrimidin-4-yl)nicotinic acid (172 mg, 0.859 mmol) , 4-(2,4,6-trifluorobenzyl)piperidine-4-carbonitrile hydrochloride (250 mg, 0.859 mmol) and HATU (486 mg, 1.28 mmol) in DMF (5 mL) was added DIPEA (0.448 mL, 2.57 mmol) . The mixture was stirred at 25°C for 12 hours. The reaction mixture poured into water (50 mL) and stirred for 20 minutes, and then the mixture was extracted with EtOAc (3 x 20 mL). The combined organic layers were washed with brine (2 x 20 mL), dried over anhydrous $Na_2SO_4$, filtered and concentrated to afford the product (250 mg) The product was purified by prep-HPLC (column: Xbridge 150*30mm*10um), gradient: 75-95% B (A= water(10mM $NH_4HCO_3$, B= MeCN), flow rate: 25 mL/min) to give 1-(2-(pyrimidin-4-yl)nicotinoyl)-4-(2,4,6-trifluorobenzyl)piperidine-4-carbonitrile (21 mg, 5.6%). $^1H$ NMR (400MHz, $CDCl_3$) $\delta_H$ 9.25-9.19 (m, 1H), 8.92-8.81 (m, 1H), 8.76 (s, 1H), 8.31-8.21 (m, 1H), 7.66 (s, 1H), 7.47 (s, 1H), 6.74 (s, 2H), 4.98-4.73 (m, 1H), 3.68-3.06 (m, 3H), 3.05-2.95 (m, 2H), 2.20-1.67 (m, 4H) $^{19}F$ NMR (376.5 MHz, $CDCl_3$) $\delta_F$ -100.85, -115.21 LCMS purity 99%; MS ESI calcd. for $C_{23}H_{18}F_3N_5O$ $[M + H]^+$ 438.2, found 438.2.

*Example 76. Synthesis of 1-(2-(pyrimidin-4-yl)nicotinoyl)-4-(3-(trifluoromethyl)benzyl)piperidine-4-carbonitrile (A264)*

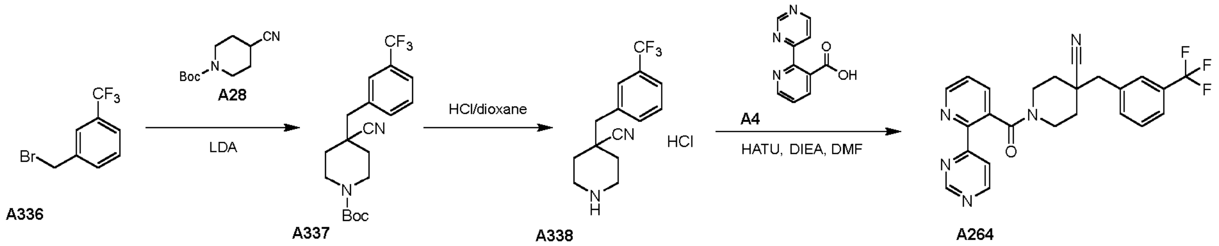


A336 A337 A338 A264

Synthesis of A337

To tert-butyl 4-cyanopiperidine-1-carboxylate (1.31 g, 6.27 mmol) in THF (10 mL) was added dropwise LDA solution (3.13 mL, 2 M, 6.27 mmol) at -78°C. Stirring was continued at -78°C for 1 hour, and then 1-(bromomethyl)-3-(trifluoromethyl)benzene (1 g, 4.18 mmol) was added. The mixture was warmed to 20°C stirred for 16 hours. The mixture was

poured into water (50 mL) and extracted with EtOAc (3 x 50 mL). The combined organic layers were washed with brine (2 x 50 mL), dried over $Na_2SO_4$, filtered, concentrated to give tert-butyl 4-cyano-4-(3-(trifluoromethyl)benzyl)piperidine-1-carboxylate (1 g). The product was used directly in the next step.

Synthesis of A338

To tert-butyl 4-cyano-4-(3-(trifluoromethyl)benzyl)piperidine-1-carboxylate (0.5 g, 1.35 mmol) in dioxane (5 mL) was added HCl/dioxane (1.68 mL, 4M in dioxane, 6.75 mmol), and the mixture was stirred at 25°C for 4 hours. The mixture was cooled and concentrated to give 4-(3-(trifluoromethyl)benzyl)piperidine-4-carbonitrile hydrochloride (360 mg). The product was used directly in the next step.

Synthesis of A264

A mixture of 2-(pyrimidin-4-yl)pyridine-3-carboxylic acid (142 mg, 0.709 mmol), HATU (336 mg, 0.886 mmol), DIPEA (381 mg, 2.95 mmol) and 4-(3-(trifluoromethyl)benzyl)piperidine-4-carbonitrile hydrochloride (180 mg, 0.591 mmol) in DMF (5 mL) was stirred at 0°C for 2 hours. The mixture was concentrated, and the residue was purified by prep-HPLC (Column: Phenomenex Gemini-NX 80*30 mm*3 um); Condition: water (10 mM $NH_4HCO_3$)-ACN; Begin B: 33%; End B: 63%) to afford 1-(2-(pyrimidin-4-yl)nicotinoyl)-4-(3-(trifluoromethyl)benzyl)piperidine-4-carbonitrile (82.8 mg, 31%). $^1H$ NMR (400 MHz, $CDCl_3$) $\delta_H$ 9.26-8.63 (m, 2H), 8.80-8.70 (m, 1H), 8.32-8.19 (m, 1H), 7.73-7.56 (m, 2H), 7.55-7.39 (m, 4H), 4.96-4.71 (m, 1H), 3.69-3.57 (m, 1H), 3.50-3.35 (m, 1H), 3.27-2.91 (m, 3H), 2.16-1.97 (m, 1H), 1.91-1.45 (m, 3H). $^{19}F$ NMR (376.5 MHz, $CDCl_3$) $\delta_F$ -62.65. LC-ELSD/MS purity 99%, MS ESI calcd. for $C_{24}H_{20}F_3N_5O$ $[M+H]^+$ 452.2, found 452.2.

*Example 77. Synthesis of 4-(2-fluoro-3-(trifluoromethyl)benzyl)-1-(2-(pyrimidin-4-yl)nicotinoyl)piperidine-4-carbonitrile (A265)*

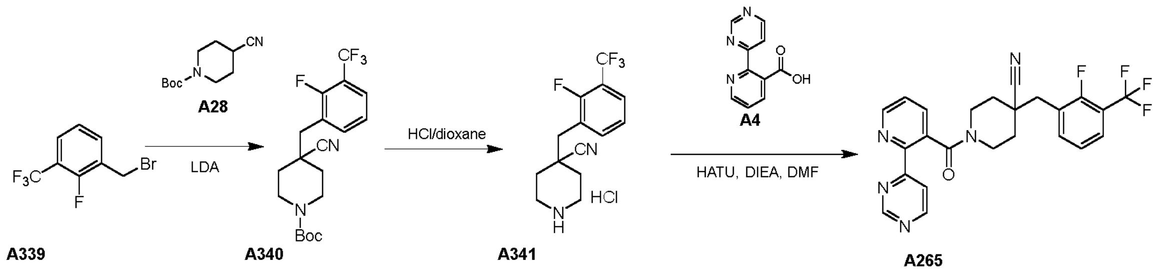


A339 A340 A341 A265

Synthesis of A340

To a mixture of tert-butyl 4-cyanopiperidine-1-carboxylate (487 mg, 2.32 mmol) in THF (10 mL) was added dropwise LDA solution (1.45 mL, 2 M, 2.90 mmol) at -78°C. After stirring at -78°C for 1 hour, 1-(bromomethyl)-2-fluoro-3-(trifluoromethyl)benzene (500 mg, 1.94 mmol) was added. The reaction mixture was warmed to 20°C and stirred for 3 hours. The mixture was poured into water (25 mL) and extracted with EtOAc (3 x 20 mL). The combined organic layers were washed with brine (2 x 50 mL), dried over $Na_2SO_4$, filtered and concentrated to give tert-butyl 4-cyano-4-(2-fluoro-3-(trifluoromethyl)benzyl)piperidine-1-carboxylate (600 mg). $^1H$ NMR (400 MHz, $CDCl_3$) $\delta_H$ 7.74-7.52 (m, 2H), 7.34-7.28 (m, 1H), 7.31-7.27 (m, 1H), 4.31-4.06 (m, 2H), 3.46-3.27 (m, 1H), 2.99 (s, 2H), 2.45 (m, 2H), 1.85 s, 1H), 1.72-1.57 (m, 1H), 1.47 (s, 9H).

Synthesis of A341

To a mixture of tert-butyl 4-cyano-4-(2-fluoro-3-(trifluoromethyl)benzyl)piperidine-1-carboxylate (680 mg, 1.75 mmol) in dioxane (5 mL) was added HCl/dioxane (4.37 mL, 4M in dioxane, 17.5 mmol). The mixture was stirred at 25°C for 4 hours. The mixture was concentrated to give 4-(2-fluoro-3-(trifluoromethyl)benzyl)piperidine-4-carbonitrile hydrochloride (500 mg).$^1H$ NMR (400 MHz, $CDCl_3$) $\delta_H$ 7.57-7.36 (m, 2H), 7.24-7.18 (m, 1H), 3.58 (m, 3H), 3.19 (m, 2H), 2.34-2.06 (m, 4H), 1.81 (s, 2H), 1.26 (s, 2H).

Synthesis of A265

To a mixture of 2-(pyrimidin-4-yl)pyridine-3-carboxylic acid (155 mg, 0.775 mmol), 4-{[2-fluoro-3-(trifluoromethyl) phenyl]methyl}piperidine-4-carbonitrile hydrochloride (250 mg, 0.775 mmol) and HATU (441 mg, 1.16 mmol) in DMF (5 mL) was added DIPEA (0.404 mL, 2.32 mmol). After stirring at 20 °C for 12 hours, the reaction mixture poured into water (50 mL) and extracted with EtOAc (3 x 20 mL). The combined organic layers were washed with brine (2 x 20 mL), dried over anhydrous $Na_2SO_4$, filtered and concentrated to afford a the product (70 mg), which was purified by HPLC (Phenomenex Gemini-NX 80*40mm*3um Condition water(0.05%$NH_3H_2O$+10mM $NH_4HCO_3$)-ACN Begin B 34 End B 60 Gradient Time(min) 8 100%B Hold Time(min) 3.5 FlowRate(ml/min) 30 Injections 5) to afford 4-(2-fluoro-3-(trifluoromethyl) benzyl)-1-(2-(pyrimidin-4-yl)nicotinoyl)piperidine-4-carbonitrile (12.5 mg, 3.44%). $^1$H NMR (400 MHz, $CDCl_3$) $\delta_H$ 9.22 (s, 1H), 8.96-8.87 (m, 2H), 8.78 (m, 1H), 8.32-8.21 (m, 1H), 7.76-7.59 (m, 3H), 7.54-7.43 (m, 1H), 7.31 (m, 1H), 4.98-4.76 (m, 1H), 3.66 (m, 1H), 3.53-3.37 (m, 1H), 3.20-3.03 (m, 3H), 2.15-1.67 (m, 3H) $^{19}$F NMR (376.5 MHz, $CDCl_3$) $\delta_F$ -61.294, -61.423, -61.488, -117.361, -117.398. LC-ELSD/MS purity 99%, MS ESI calcd. for $C_{24}H_{19}F_4N_5O$ [M+H] 470.2 found 470.2.

*Example 78. Synthesis of 1-([2, 4'-bipyridine]-3-carbonyl)-4-(2-fluoro-3-(trifluoromethyl)benzyl)piperidine-4-carbonitrile (A266)*

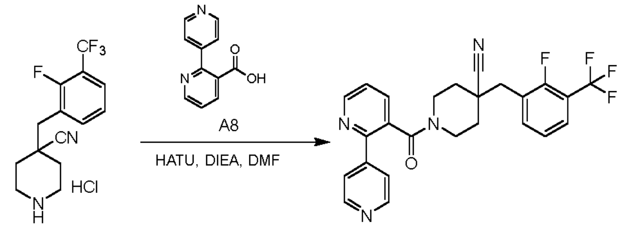


A341 A266

To a mixture of [2,4'-bipyridine]-3-carboxylic acid (155 mg, 0.7746 mmol) , 4-{[2-fluoro-3-(trifluoromethyl)phenyl] methyl }piperidine-4-carbonitrile hydrochloride (250 mg, 0.7746 mmol) and HATU (441 mg, 1.16 mmol) in DMF (5 mL) was added DIPEA (0.404 mL, 2.32 mmol). After stirring at 20°C for 12 hours, the reaction mixture was poured into water (50 mL) and extracted with EtOAc (3 x 20 mL). The combined organic layers were washed with brine (2 x 20 mL), dried over anhydrous $Na_2SO_4$, filtered and concentrated to afford the product (70 mg), which was purified by prep-HPLC (Phenomenex Gemini-NX 80*40mm*3um Condition water(0.05%$NH_3H_2O$+10mM $NH_4HCO_3$)-ACN Begin B 34 End B 60 Gradient Time(min) 8 100%B Hold Time(min) 3.5 FlowRate(ml/min) 30 Injections 5) to afford 1-([2,4'-bipyridine]-3-carbonyl)-4-(2-fluoro-3-(trifluoromethyl)benzyl)piperidine-4-carbonitrile (19.6 mg, 5.41%). $^1$H NMR (400 MHz, $CDCl_3$) $\delta_H$ 8.78-8.62 (m, 3H), 7.74-7.65 (m, 2H), 7.60 (m, 1H), 7.55-7.47 (m, 2H), 7.43-7.37 (m, 2H), 4.78 (m, 1H), 3.12-2.76 (m, 3H), 2.56 (m, 2H), 1.80 (br d, 1H), 1.36-1.13 (m, 2H), -0.01--0.13 (m, 1H) $^{19}$F NMR (376.5 MHz, $CDCl_3$) $\delta_F$ -61.432, -116.882, -116.919. LCMS purity 99%, MS ESI calcd. for $C_{25}H_{20}F_4N_4O$ [M+H] 469.1 found 469.1.

*Example 79. Synthesis of 4-(4-fluoro-3-(trifluoromethyl)benzyl)-1-(2-(pyrimidin-4-yl)nicotinoyl)piperidine-4-carbonitrile (A267)*

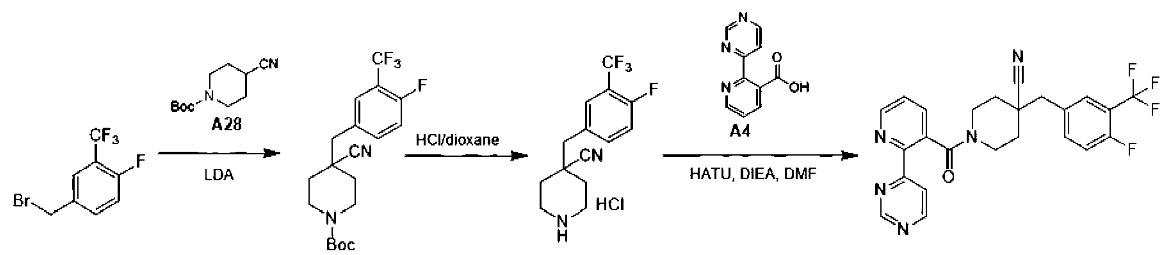


A342 A343 A344 A267

Synthesis of A343

To a mixture of tert-butyl 4-cyanopiperidine-1-carboxylate (979 mg, 4.66 mmol) in THF (10 mL) was added dropwise LDA solution (2.91 mL, 2 M, 5.83 mmol) at -78°C. After stirring at -78°C for 1 hour, tert-butyl 4-cyanopiperidine-1-carboxylate (1.0 g, 3.89 mmol) was added and the mixture was warmed to 20°C and stirred for 3 hours. The mixture was poured into water (25 mL) and extracted with EtOAc (3 x 20 mL). The combined organic layers were washed with brine (2 x 50 mL), dried over $Na_2SO_4$, filtered and concentrated to give tert-butyl 4-cyano-4-(4-fluoro-3-(trifluoromethyl)benzyl) piperidine-1-carboxylate (1.4 g). $^1$H NMR (400 MHz, $CDCl_3$) $\delta_H$ 7.57-7.44 (m, 1H), 7.32-7.25 (m, 1H), 7.25-7.15 (m, 1H), 4.13 (m, 2H), 2.99 (s, 2H), 2.89 (s, 1H), 1.84 (d, 2H), 1.64-1.53 (m, 2H), 1.50 (d, 1H), 1.47 (s, 9H), 1.40 (s, 1H).

Synthesis of A344

To a mixture of tert-butyl 4-cyano-4-(4-fluoro-3-(trifluoromethyl)benzyl)piperidine-1-carboxylate (1.4 g, 3.62 mmol) in dioxane (5 mL) was added HCl/dioxane (4.52 mL, 4M in dioxane, 18.1mmol), and the mixture was stirred at 25°C for 4 hours. The mixture concentrated to give 4-(4-fluoro-3-(trifluoromethyl)benzyl)piperidine-4-carbonitrile hydrochloride (1.0 g). $^1$H NMR (400 MHz, $CDCl_3$) $\delta_H$ 7.59-7.46 (m, 2H), 7.26-7.20 (m, 1H), 3.75 (s, 1H), 3.64-3.53 (m, 2H), 3.19 (d, 2H), 2.98 (s, 2H), 2.28-2.15 (m, 2H), 2.06 (m, 2H).

Synthesis of A267

To a mixture of 2-(pyrimidin-4-yl)pyridine-3-carboxylic acid (140 mg, 0.699 mmol), 4-(4-fluoro-3-(trifluoromethyl) benzyl)piperidine-4-carbonitrile hydrochloride (200 mg, 0.699 mmol) and HATU (395 mg, 1.04 mmol) in DMF (5 mL) was added DIPEA (0.364 mL, 2.09 mmol). The mixture was stirred at 20°C for 12 hours. The reaction mixture poured into water (50 mL) and stirring was continued for 20 minutes. The aqueous layer was extracted with EtOAc (3 x 20 mL). The combined organic layers were washed with brine (2 x 20 mL), dried over anhydrous $Na_2SO_4$, filtered and concentrated to give the product (200 mg). The product was purified by prep-HPLC (Column Phenomenex Gemini-NX 80*30mm*3um Condition water(10mM $NH_4HCO_3$)-ACN Begin B 30 End B 90 Gradient Time(min) 9 100%B Hold Time(min) 1.5 FlowRate(ml/min) 30 Injections 6) to afford 4-(4-fluoro-3-(trifluoromethyl)benzyl)-1-(2-(pyrimidin-4-yl)nicotinoyl)piperidine-4-carbonitrile (49.5 mg, 15.1%). $^1$H NMR (400 MHz, $CDCl_3$) $\delta_H$ 9.22 (s, 1H), 9.06 (s, 1H), 8.96-8.87 (m, 1H), 8.78 (m, 1H), 8.28 (d, 1H), 7.68 (d, 1H), 7.56-7.44 (m, 3H), 7.26-7.18 (m, 1H), 5.03-4.76 (m, 1H), 3.65 (m, 1H), 3.50-3.38 (m, 1H), 3.30-3.04 (m, 2H), 3.03-2.85 (m, 2H), 2.16-1.98 (m, 1H), 1.86-1.64 (m, 2H) $^{19}$F NMR (376.5 MHz, $CDCl_3$) $\delta_F$ -61.414, -61.451,-115.399, -115.436. LC-ELSD/MS purity 99%, MS ESI calcd. for $C_{24}H_{19}P_4N_5O$ [M+H] 470.2 found 470.2.

*Example 80. Synthesis of (R)-1-((2,4'-bipyridine)-3-carbonyl)-4-(1-(4-(trifluoromethyl)phenyl)ethyl)piperidine-4-carbonitrile (A268) and (S)-1-([2,4'-bipyridine]-3-carbonyl)-4-(1-(4-(trifluoromethyl)phenyl)ethyl)piperidine-4-carbonitrile (A269)*

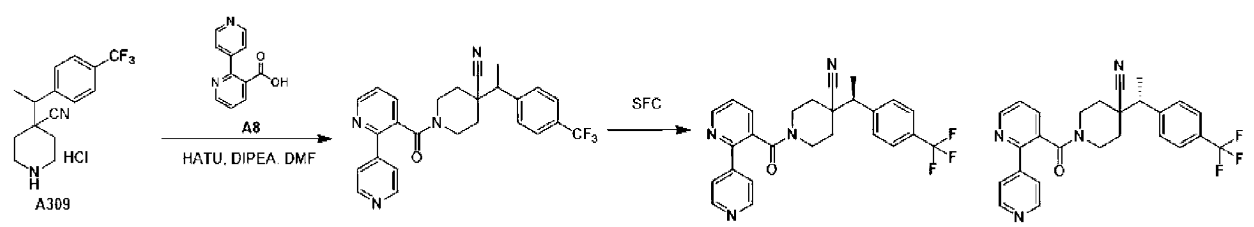

A309 A345 A268 A269

Synthesis of A345 and SFC Separation

To a mixture of 2-(pyrimidin-4-yl)pyridine-3-carboxylic acid (100mg, 0.501 mmol), HATU (285 mg, 0.752 mmol) in DMF (3 mL) was added DIPEA (0.8 mL) and 4-(1-(4-(trifluoromethyl)phenyl)ethyl)piperidine-4-carbonitrile hydrochloride (170 mg, 0.602 mmol). The mixture was stirred at 20°C for 2 hours. The mixture solution was concentrated, and the residue was purified by HPLC (water (10mM $NH_4HCO_3$)-ACN Begin B 29 End B 59) to afford racemic-1-([2,4'-bipyridine]-3-carbonyl)-4-(1-(4-(trifluoromethyl)phenyl)ethyl)piperidine-4-carbonitrile (465 mg), which was purified by SFC(Column: REGIS (s,s) WHELK-O1 (250mm*30mm,5um), Condition 0.1%$NH_3H_2O$ IPA, Begin B 45, End B 45) to give:
(R)-1-([2,4'-bipyridine]-3-carbonyl)-4-(1-(4-(trifluoromethyl)phenyl)ethyl)piperidine-4-carbonitrile ( 18.7 mg, 4.03%). $^1$H NMR (400 MHz, $CDCl_3$) $\delta_H$ 8.71-8.84 (m, 3 H), 7.75-7.78 (m, 2 H), 7.52-7.66 (m, 3 H), 7.40-7.51 (m, 1.5 H), 7.31-7.40 (m, 1

H), 7.24 (s, 0.5 H), 4.66-4.95 (m, 1 H), 2.70-3.18 (m, 4 H), 2.17-2.23 (m, 1 H), 1.52 (m, 1.5 H), 1.40 (d, 1.5 H), 1.29 (d, 1 H), 1.08-1.21 (m, 1 H), 0.78 (d, 0.4 H), -0.11 (s, 0.2 H) -0.49--0.39 (m, 0.4 H). LCMS purity 98%, MS ESI calcd. For $C_{26}H_{23}F_3N_4O$ [M+H]+ 465.1 found 465.3.

(S)-1-([2,4'-bipyridine]-3-carbonyl)-4-(1-(4-(trifluoromethyl)phenyl)ethyl)piperidine-4-carbonitrile (44.2 mg, 9.52%). $^1H$ NMR (400 MHz, $CDCl_3$) $\delta_H$ 8.68-8.85 (m, 3 H), 7.76 (m, 2.5 H), 7.53-7.64 (m, 2.5 H), 7.28-7.52 (m, 2.5 H), 7.24 (s, 0.5 H), 4.66-4.94 (m, 1 H), 2.64-3.32 (m, 4 H), 2.17-2.22 (m, 1 H), 1.49-1.56 (m, 1 H), 1.36-1.44 (m, 2 H), 1.29 (d, 1 H), 1.10-1.20 (m, 0.7 H), 0.94-1.04 (m, 0.3 H), 0.78 (d, 0.4 H), -0.18--0.09 (m, 0.2 H), -0.45 (m, 0.4H). LCMS purity 99%, MS ESI calcd. For $C_{26}H_{23}F_3N_4O$ [M+H]+ 465.1 found 465.2.

*Example 81. Synthesis of 1-([2, 4'-bipyridine]-3-carbonyl)-4-(2-(trifluoromethyl)benzyl)piperidine-4-carbonitrile (A275)*

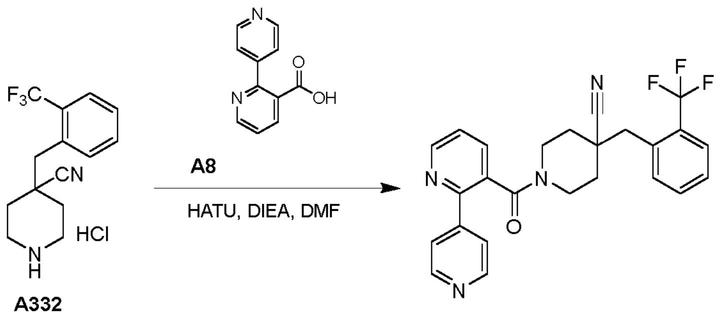


A332

A275

To a mixture of [2,4'-bipyridine]-3-carboxylic acid (93.0 mg, 465 μmol), HATU (265 mg, 0.698 mmol) in DMF (2 mL) was added DIPEA (0.8 mL) and 4-(2-(trifluoromethyl)benzyl)piperidine-4-carbonitrile hydrochloride (150 mg, 0.559 mmol). The mixture was stirred at 20oC for 2 hours. The mixture solution was concentrated and purified by HPLC: Phenomenex Gemini-NX 80*30mm*3um (water (10mM NH4HCO3)-ACN Begin B 33 End B 63) to afford 1-([2,4'-bipyridine]-3-carbonyl)-4-(2-(trifluoromethyl)benzyl)piperidine-4-carbonitrile (11.2 mg, 5.35%). 1H NMR (400 MHz, CDCl3) δH 8.68-8.83 (m, 3 H), 7.64-7.82 (m, 3.7 H), 7.50-7.63 (m, 2.3 H), 7.38-7.47 (m, 2 H), 4.81 (d, 1 H), 3.02-3.17 (m, 2 H), 2.91-2.97 (m, 0.4 H), 2.61-2.84 (m, 1.6 H), 1.76-2.00 (m, 0.7 H), 1.51-1.75 (m, 3 H), 1.45 (d, 0.5 H), 1.18-1.26 (m, 0.4 H), 0.15 (s, 0.5 H). 19F NMR (376.5 MHz, CDCl3) δF -56.742, -56.877. LCMS purity 97%, MS ESI calcd. For C25H21F3N4O [M+H]+ 451.1 found 451.3.

*Example 82. Synthesis of 1-([2, 4'-bipyridine]-3-carbonyl)-4-(4-fluoro-3-(trifluoromethyl)benzyl)piperidine-4-carbonitrile (A278)*

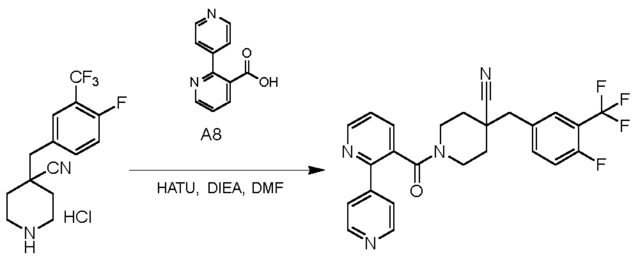


A344

A278

To a solution of [2,4'-bipyridine]-3-carboxylic acid (139 mg, 0.699 mmol) , 4-(4-fluoro-3-(trifluoromethyl)benzyl) piperidine-4-carbonitrile hydrochloride (200 mg, 0.699 mmol) and HATU (395 mg, 1.04 mmol) in DMF (5 mL) was added DIPEA (0.364 mL, 2.09 mmol) . The mixture was stirred at 20oC for 12 hours. The reaction mixture poured into H2O (50 mL) and stirred for 20 minutes. The mixture was extracted with EtOAc (3 x 20 mL). The combined organic layers were washed with brine (2 x 20 mL), dried over anhydrous Na2SO4, filtered and concentrated to afford a product (70 mg). The residue was purified by HPLC (Column Phenomenex Gemini-NX 80*30mm*3um Condition water(10mM NH4HCO3)-ACN Begin B 34 End B 64 Gradient Time(min) 9 100%B Hold Time(min) 1.5 FlowRate(ml/min) 30 Injections 6) to afford 1-([2,4'-bipyridine]-3-carbonyl)-4-(4-fluoro-3-(trifluoromethyl)benzyl)piperidine-4-carbonitrile (57.4 mg, 17.5%). 1H NMR (400

MHz, CDCl3) δH 8.87-8.69 (m, 3H), 7.79 (m, 2H), 7.60 (br s, 1H), 7.50-7.43 (m, 2H), 7.50-7.29 (m, 1H), 7.19 (m, 1H), 4.94-4.78 (m, 1H), 3.11-2.82 (m, 3H), 2.66 (d, 1H), 2.41 (d, 1H), 1.89 (m, 1H), 1.20 (m, 2H), -0.17--0.41 (m, 1H) 19F NMR (376.5 MHz, CDCl3) δF -61.414,-61.451,-115.473. LC-ELSD/MS purity 99%, MS ESI calcd. for C25H20F4N4O [M+H] 469.2 found 469.2.

*Example 83. Synthesis of 1-([2,4'-bipyridine]-3-carbonyl)-4-(2,5-difluorobenzyl)piperidine-4-carbonitrile (A282)*

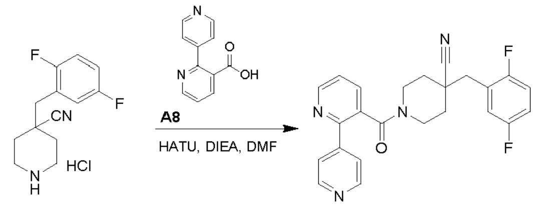


A329 A282

A mixture of [2,4'-bipyridine]-3-carboxylic acid (202 mg, 1.01 mmol) added HATU (479 mg,1.26 mmol), DIPEA (546 mg, 4.23 mmol) and 4-(2,5-difluorobenzyl)piperidine-4-carbonitrile hydrochloride (200 mg, 0.846 mmol) in DMF (5 mL) was stirred at 25oC for 2 hours. The mixture was poured into water (10 mL) and extracted with EtOAc (3 x 10 mL). The combined organic layers were washed with brine (2 x 10 mL), dried over Na2SO4, filtered, and concentrated. The residue was purified by prep-HPLC (Column: Phenomenex Gemini-NX 80*30mm*3um; Condition: water (10mM NH4HCO3)-ACN; Begin B: 33; End B: 63) to afford 1-([2,4'-bipyridine]-3-carbonyl)-4-(2,5-difluorobenzyl)piperidine-4-carbonitrile (72.3 mg, 20.4%). $^{1}$H NMR (400 MHz, CDCl3) δH 8.82-8.68 (m, 3H), 7.82-7.53 (m, 3H), 7.47-7.42 (m, 1H), 7.08-6.89 (m,3H), 4.91-4.89 (m, 1H), 3.40-2.84 (m, 3H), 2.65-2.46 (m, 2H), 2.02-1.83(m, 1H), 1.44-1.15 (m, 2H), 0.10-0 (m, 1H). 19F NMR (376.5 MHz, CDCl3) δF -118.02, -118.07, -121.13, -121.19. LCMS purity 99%, MS ESI calcd. for C24H21F2N4O [M+H]+ 419.2, found 419.2.

*Example 84. Synthesis of 1-([2, 4'-bipyridine]-3-carbonyl)-4-(3-(trifluoromethyl)benzyl)piperidine-4-carbonitrile (A279)*

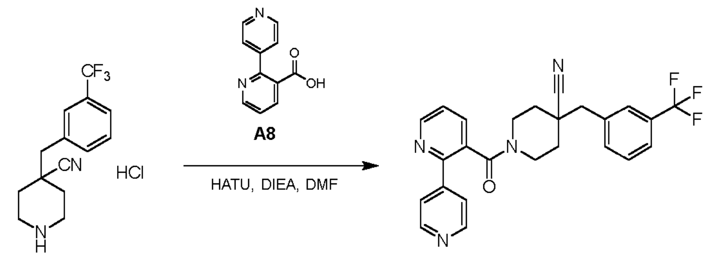


A338 A279

A solution of [2, 4'-bipyridine]-3-carboxylic acid (130 mg, 0.65 mmol), HATU (336 mg, 0.89 mmol), DIPEA (381 mg, 2.95 mmol) and 4-{[3-(trifluoromethyl) phenyl] methyl}piperidine-4-carbonitrile hydrochloride (180 mg, 0.59 mmol) in DMF (5 mL) was stirred at 0oC for 2 hours. The mixture was concentrated, and the residue was purified by HPLC (Phenomenex Gemini-NX 80*40mm*3μm); Condition: water (water (0.05%NH3H2O+10mM NH4HCO3)-ACN; Begin B: 34%; End B:54 %) to afford 1-([2,4'-bipyridine]-3-carbonyl)-4-(3-(trifluoromethyl)benzyl)piperidine-4-carbonitrile (10.8 mg, 4%). 1H NMR (400 MHz, CDCl3) δH 8.95-8.65 (m, 3 H), 7.85-7.75 (m, 2 H), 7.65-7.55 (m, 1 H), 7.54-7.28 (m, 5 H), 4.91-4.76 (m, 1 H), 3.14-2.88 (m, 3 H), 2.75-2.66 (m, 1 H), 2.50-2.42 (m, 1 H), 1.95-1.85 (m, 1 H), 1.33-1.16 (m, 2 H), 0.17--0.30 (m, 1 H). 19F NMR (376.5 MHz, CDCl3) δF -62.670. LCMS purity > 99%, calcd. for C25H21F3N4O [M+H]+ 451.2, found 451.3.

*Example 85. Synthesis of (S)-1-(2-(pyrimidin-4-yl)nicotinoyl)-4-(1-(2,4,6-trifluorophenyl)ethyl)piperidine-4-carbonitrile (A270) and (R)-1-(2-(pyrimidin-4-yl)nicotinoyl)-4-(1-(2,4,6-trifluorophenyl)ethyl)piperidine-4-carbonitrile (A271)*

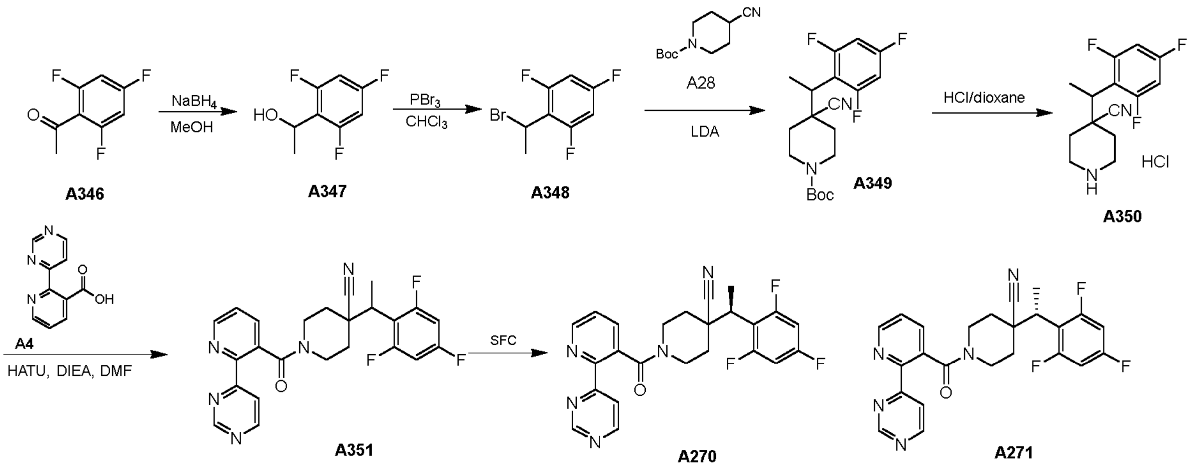

A346 A347 A348 A349 A350 A4 A351 A270 A271

Synthesis of A347

To a solution of 1-(2,4,6-trifluorophenyl)ethan-1-one (2 g, 11.4 mmol) in MeOH (20 mL) was added $NaBH_4$ (862 mg, 22.8 mmol) at 0°C under nitrogen. The reaction mixture was stirred at 25°C for 2 hours. The mixture was poured into ice-water (20 mL) and stirred for 20 minutes. The mixture was extracted with EtOAc (3 x 20 mL). The combined organic layers were washed with brine (2 x 20 mL), dried over anhydrous $Na_2SO_4$, filtered and concentrated to give 1-(2,4,6-trifluorophenyl)ethan-1-ol (700 mg).

Synthesis of A348

To a solution of 1-(2,4,6-trifluorophenyl)ethan-1-ol (700 mg, 3.97 mmol) in $CHCl_3$ (10 mL) was added tribromophosphane (3.22 g, 11.9 mmol) in one portion at 25°C under nitrogen. The mixture was stirred at 25°C for 2 hours. The mixture was poured slowly into saturated ice-water (15 mL) and extracted with EtOAc (3 x 10 mL). The combined organic layers were washed with brine (2 x 10 mL), dried over anhydrous $Na_2SO_4$, filtered, and concentrated. The residue was purified by flash chromatography (0~30% of EtOAc in PE) to give to give 2-(1-bromoethyl)-1,3,5-trifluorobenzene (400 mg). $^1H$ NMR (400 MHz, $CDCl_3$) $\delta_H$ 6.69-6.64 (m, 3 H), 5.46-5.44 (m, 1 H), 2.10-2.05 (m, 3 H). $^{19}F$ NMR (376.5 MHz, $CDCl_3$) $\delta_F$ -107.148, -110.185.

Synthesis of A349

LDA solution (2.50 mL, 5.01 mmol) was added to a solution of tert-butyl 4-cyanopiperidine-1-carboxylate (0.525 g, 2.50 mmol) in THF (5 mL) at 0°C. After stirring for 30 minutes, 2-(1-bromoethyl)-1,3,5-trifluorobenzene (0.4 g, 1.67 mmol) was added. The mixture was stirred at 25°C for 2 hours. The mixture was poured into water (10 mL) and extracted with EtOAc (3 x 10 mL). The combined organic layers were washed with brine (2 x 10 mL), dried over $Na_2SO_4$, filtered, and concentrated to give tert-butyl 4-cyano-4-(1-(2,4,6-trifluorophenyl)ethyl)piperidine-1-carboxylate (0.2 g). $^1H$ NMR (400 MHz, $CDCl_3$) $\delta_H$ 6.69-6.63 (m, 2 H), 5.24-5.17 (m, 1 H), 4.29-4.10 (m, 1 H), 3.04-3.00 (m, 2 H), 2.13-2.08 (m, 1 H), 1.70-1.58 (m, 5 H), 1.441 (s, 9 H), 1.30-1.24 (m, 1 H), 0.99-0.86 (m, 1 H). $^{19}F$ NMR (376.5 MHz, $CDCl_3$) $\delta_F$ -101.39,-108.31,-112.76.

Synthesis of A350

To tert-butyl 4-cyano-4-(1-(2,4,6-trifluorophenyl)ethyl)piperidine-1-carboxylate (200 mg, 0.543 mmol) in dioxane (2 mL) was added dioxane/HCl (1.35 mL, 4 M in dioxane) at 25°C, and the mixture was stirred for 16 hours. The mixture was concentrated to give 4-(1-(2,4,6-trifluorophenyl)ethyl)piperidine-4-carbonitrile hydrochloride (120 mg). $^1H$ NMR (400 MHz, $CDCl_3$) $\delta_H$ 6.69-6.65 (m, 2 H), 5.92-5.81 (m, 2 H), 1.76-1.74 (m, 3 H), 1.66-1.61 (m, 1 H), 1.29-1.25 (m, 3 H), 1.06-0.83 (m, 2 H). $^{19}F$ NMR (376.5 MHz, $CDCl_3$) $\delta_F$ -106.62, - 110.16.

Synthesis of A351 and SFC Separation

A mixture of 2-(pyrimidin-4-yl)pyridine-3-carboxylic acid (107 mg, 0.537 mmol), HATU (255 mg, 0.671 mmol), DIPEA (288 mg, 2.23 mmol) and 4-(1-(2,4,6-trifluorophenyl)ethyl)piperidine-4-carbonitrile hydrochloride (120 mg, 0.447 mmol) in DMF (5 mL) was stirred at 25°C for 2 hours. The mixture was poured into water (10 mL) and extracted with EtOAc (3 x 10 mL). The combined organic layers were washed with brine (2 x 10 mL), dried over $Na_2SO_4$, filtered, and

concentrated to give (150 mg). The residue was purified by prep-HPLC (Column: Phenomenex Gemini-NX C18 75*30mm*3um; Condition: water (0.225%FA)-ACN; Begin B: 30; End B: 60) to afford racemic-1-(2-(pyrimidin-4-yl) nicotinoyl)-4-(1-(2,4,6-trifluorophenyl)ethyl)piperidine-4-carbonitrile (100 mg, 49.7%), which was further purified by SFC Column: DAICEL CHIRALCEL OD-H (250mm*30mm, 5um); Condition: 0.1%NH3H2O ETOH; Begin B: 25; End B: 25) to give:

(S)-1-(2-(pyrimidin-4-yl)nicotinoyl)-4-(1-(2,4,6-trifluorophenyl)ethyl)piperidine-4-carbonitrile (25.6 mg, 25.6%). $^1$H NMR (400 MHz, $CDCl_3$) $\delta_H$ 8.89-8.74 (m, 3 H), 8.27-8.23 (m, 1 H), 7.67-7.65 (m, 1 H), 7.47-7.44 (m, 1 H), 6.68-6.66 (m, 2 H), 4.95-4.80 (m, 1 H), 3.67-3.06 (m, 4 H), 1.93-1.84 (m, 2 H), 1.65-1.59 (m, 4 H),1.48-1.47 (m, 1 H). $^{19}$F NMR (376.5 MHz, $CDCl_3$) $\delta_F$ -101.49, -101.56,-107.69,-107.86. LCMS purity 99%, calcd. for $C_{24}H_{21}F_3N_5O$ $[M+H]^+$ 452.2, found 452.2.

(R)-1-(2-(pyrimidin-4-yl)nicotinoyl)-4-(1-(2,4,6-trifluorophenyl)ethyl)piperidine-4-carbonitrile (12.6 mg, 12.6% ).$^1$H NMR (400 MHz, $CDCl_3$) $\delta_H$ 8.89-8.74 (m, 3 H), 8.27-8.22 (m, 1 H), 7.67-7.65 (m, 1 H), 7.48-7.26 (m, 1 H), 6.69-6.68 (m, 2 H), 4.95-4.87 (m, 1 H), 3.67-3.06 (m, 4 H), 1.92-1.79 (m, 3 H), 1.65-1.59 (m, 4 H). $^{19}$F NMR (376.5 MHz, $CDCl_3$) $\delta_F$ -101.49, 101.56,-107.53,-107.86. LCMS purity 99%, calcd. for $C_{24}H_{21}F_3N_5O$ $[M+H]^+$ 452.2, found 452.2.

*Example 86. Synthesis of 1-([2,4'-bipyridine]-3-carbonyl)-4-(2,4, 6-trifluorobenzyl)piperidine-4-carbonitrile (A272)*

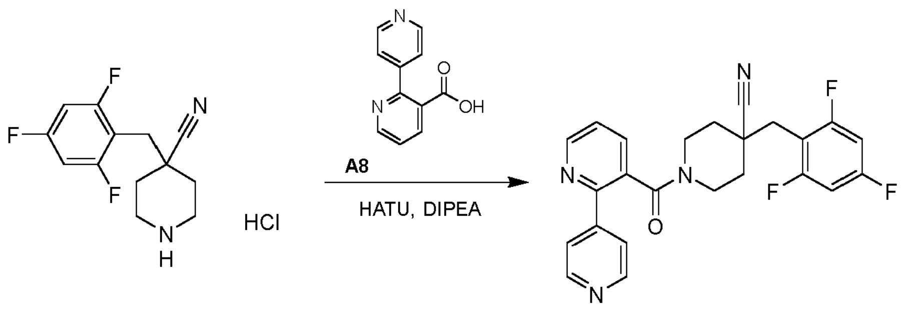


A335 A272

A mixture of [2,4'-bipyridine]-3-carboxylic acid (113 mg, 150 mmol), HATU (264 mg, 1.03 mmol), DIPEA (0.461 mL, 2.57mmol) and (4-(2,4,6-trifluorobenzyl)piperidine-4-carbonitrile hydrochloride (150 mg, 0.5159 mmol) in DMF (2 mL) was stirred at 15°C for 16 hours. The mixture was filtered, concentrated and the residue was purified by prep-HPLC (Column: Phenomenex Gemini-NX 80*40 mm*3 um; Condition: water (0.05%$NH_3H_2O$+10 mM $NH_4HCO_3$)-ACN; Begin B: 25; End B: 55; Gradient Time (min):8; 100%B Hold Time (min): 2) to give 1-([2,4'-bipyridine]-3-carbonyl)-4-(2,4,6-trifluorobenzyl)piperidine-4-carbonitrile (10.2 mg, 4.53%). $^1$H NMR (400MHz, $CDCl_3$) $\delta_H$ 8.89-8.61 (m, 3H), 7.83-7.68 (m, 2H), 7.60 (s, 1H), 7.45 (dd, 1H), 6.70 (t, 2H), 4.83 (d, 1H), 3.30-2.83 (m, 3H), 2.79-2.49 (m, 2H), 2.05-1.87 (m, 1H), 1.47-1.31 (m, 2H), 0.86 (s, 1H), 0.02 (s, 1H). LCMS purity 99%, MS ESI calcd. For $C_{24}H_{20}F_3N_4O$ $[M+H]^+$ 437. found 437.

*Example 87. Synthesis of 4-(2-fluoro-5-(trifluoromethyl)benzyl)-1-(2-(pyrimidin-4-yl)nicotinoyl)piperidine-4-carbonitrile (A273)*

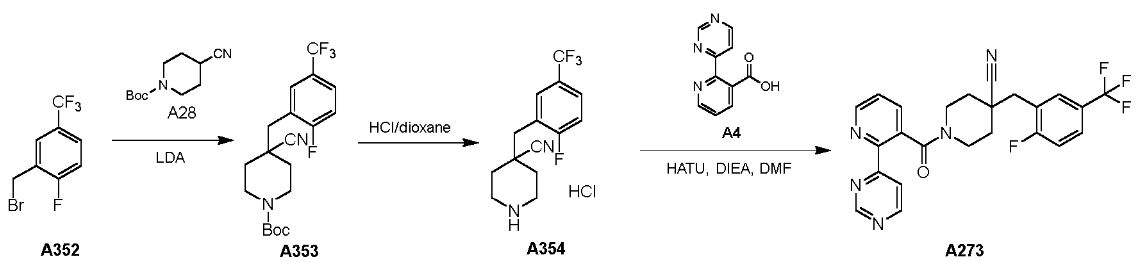


A352 A353 A354 A273

Synthesis of A353

To a solution of tert-butyl 4-cyanopiperidine-1-carboxylate (897 mg, 4.27 mmol) in THF (10 mL) was added LDA solution (3.89 mL, 2 M in hexane, 7.78 mmol) at -70°C. After stirring for 30 minutes, 2-(bromomethyl)-1-fluoro-4-(trifluoromethyl)benzene (1 g, 3.89 mmol) in THF (5 mL) was added at -70°C and stirring was continued for 2 hours. Saturated $NH_4Cl$ solution (50 mL) was added, and the mixture was extracted with EtOAc (2 x 30 mL). The combined organic layers were dried over $Na_2SO_4$, filtered, and concentrated. The residue was purified by silica gel chromatography (PE/EtOAc = 10/1 to 5/1) to give tert-butyl 4-cyano-4-(2-fluoro-5-(trifluoromethyl)benzyl)piperidine-1-carboxylate (1 g, 66.6%). $^1H$ NMR (400MHz, $CDCl_3$) $\delta_H$ 7.72-7.53 (m, 2H), 7.25-7.16 (m, 1H), 4.32-4.05 (m, 2H), 2.98 (s, 4H), 1.87 (d, 2H), 1.63-1.57 (m, 1H), 1.46 (s, 9H).

Synthesis of A354

To tert-butyl 4-cyano-4-(2-fluoro-5-(trifluoromethyl)benzyl)piperidine-1-carboxylate (1 g, 2.58 mmol) was added HCl/dioxane (10 mL, 4 M, 40.0 mmol) at 25°C and the mixture was stirred for 16 hours. The mixture was concentrated to give 4-(2-fluoro-5-(trifluoromethyl)benzyl)piperidine-4-carbonitrile hydrochloride (800 mg). The residue was used directly in the next step.

Synthesis of A273

A mixture of 2-(pyrimidin-4-yl)nicotinic acid (77.2 mg,0.384 mmol), HATU (265 mg, 0.698 mmol), DIPEA (0.301 mL, 1.74 mmol) and 4-(2-fluoro-5-(trifluoromethyl)benzyl)piperidine-4-carbonitrile hydrochloride (100 mg, 0.349 mmol) in DMF (4 mL) was stirred at 15°C for 16 hours. The mixture was filtered, concentrated, and purified by prep-HPLC (Column: Phenomenex Gemini-NX 80*30 mm*3 um; Condition: water (10mM $NH_4HCO_3$)-ACN; Begin B: 33; End B: 63; Gradient Time (min):9; 100%B Hold Time (min): 2) to give 4-(2-fluoro-5-(trifluoromethyl)benzyl)-1-(2-(pyrimidin-4-yl)nicotinoyl) piperidine-4-carbonitrile (18.8 mg, 11.5%). $^1H$ NMR (400MHz, $CDCl_3$) $\delta_H$ 9.21 (s, 0.5H), 8.93-8.83 (m, 1.5H), 8.76 (dd, 1H), 8.31-8.19 (m, 1H), 7.75-7.55 (m, 3H), 7.47 (dd, 1H), 7.22 (t, 1H), 4.98-4.73 (m, 1H), 3.64 (d, 1H), 3.43 (t, 1H), 3.30-2.98 (m, 3H), 2.17-1.63 (m, 4H). LCMS purity 99%, MS ESI calcd. For $C_{24}H_{20}F_4N_5O$ $[M+H]^+$ 470. found 470.

*Example 88. Synthesis of 1-([2, 4'-bipyridine]-3-carbonyl)-4-(2-fluoro-5-(trifluoromethyl)benzyl)piperidine-4-carbonitrile (A283)*

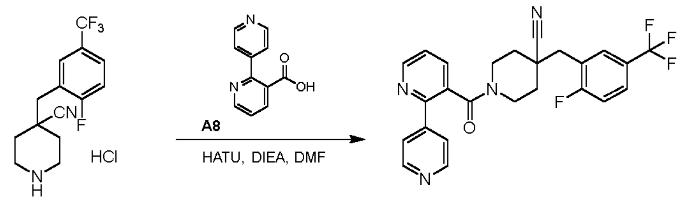

A354
A283

A mixture of [2,4'-bipyridine]-3-carboxylic acid (116 mg, 0.58 mmol), HATU (367 mg, 0.97 mmol), DIPEA (419 mL, 2.41 mmol) and 4-(2-fluoro-5-(trifluoromethyl)benzyl)piperidine-4-carbonitrile hydrochloride (90 mg, 0.483 mmol) in DMF (2 mL) was stirred at 15°C for 16 hours. The mixture was filtered, concentrated, and purified by prep-HPLC (Column: Phenomenex Gemini-NX 80*30 mm*3 um; Condition: water (10 mM $NH_4HCO_3$)-ACN; Begin B: 31; End B: 61; Gradient Time (min):9; 100%B Hold Time (min): 2) to give 1-([2,4'-bipyridine]-3-carbonyl)-4-(2-fluoro-5-(trifluoromethyl)benzyl) piperidine-4-carbonitrile (29.4 mg, 13%). $^1H$ NMR (400MHz, $CDCl_3$) $\delta_H$ 8.90-8.63 (m, 3H), 7.76 (s, 2H), 7.66-7.53 (m, 2H), 7.51-7.40 (m, 2H), 7.19 (t, 1H), 4.93-4.71 (m, 1H), 3.31-2.83 (m, 3H), 2.80-2.47 (m, 2H), 1.99-1.80 (m, 1H), 1.45-1.18 (m, 2H), 0.03--0.14 (m, 1H). LCMS purity 99%, MS ESI calcd. For $C_{25}H_{21}F_4N_4O$ $[M+H]^+$ 469. found 469.

*Example 89. Synthesis of 1-([2, 4'-bipyridine]-3-carbonyl)-4-(2, 6-difluorobenzyl)piperidine-4-carbonitrile (A274)*

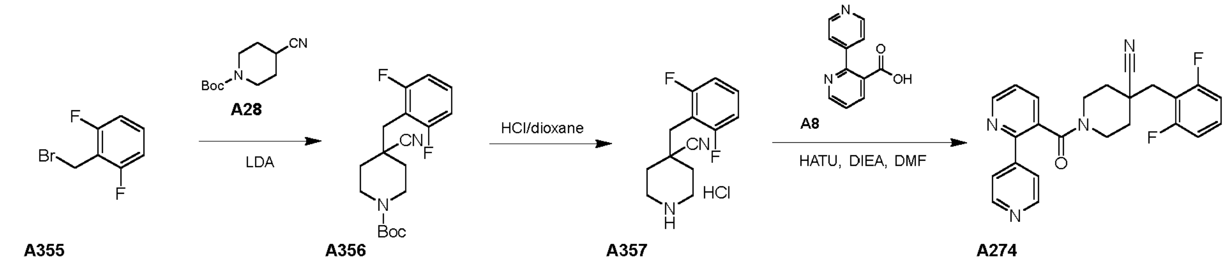


A355 A356 A357 A274

Synthesis of A356

To a solution of tert-butyl 4-cyanopiperidine-1-carboxylate (912 mg, 4.34 mmol) in THF (10 mL) was added LDA solution (4.83 mL, 2 M in hexane, 9.66 mmol) at -70°C. After stirring for 30 minutes, 2-(bromomethyl)-1,3-difluorobenzene (1 g, 4.83 mmol) was added at -70°C, and the mixture was stirred for 1 hour. The mixture was warmed to 25°C and stirred for 16 hours. To the mixture was added $NH_4Cl$ (50 mL, sat. aq.), and the mixture was extracted with EtOAc (2 x 30 mL). The combined organic layers were dried over $Na_2SO_4$, filtered, and concentrated to give tert-butyl 4-cyano-4-(2,6-difluorobenzyl)piperidine-1-carboxylate (1 g). $^1H$ NMR (400MHz, $CDCl_3$) $\delta_H$ 7.26-7.17 (m, 1H), 6.94 (t, 2H), 4.31-3.96 (m, 2H), 3.01 (s, 4H), 1.98-1.82 (m, 2H), 1.60-1.52 (m, 2H), 1.45 (s, 9H).

Synthesis of A357

To tert-butyl 4-cyano-4-(2,6-difluorobenzyl)piperidine-1-carboxylate (200 mg, 0.70 mmol) was added HCl/dioxane (10 mL, 4M, 40.0 mmol) at 25°C. The mixture was stirred at 25°C for 16 hours under nitrogen. The mixture was concentrated to give 4-(2,6-difluorobenzyl)piperidine-4-carbonitrile hydrochloride (800 mg). The residue was used directly in the next step.

Synthesis of A274

A mixture of [2,4'-bipyridine]-3-carboxylic acid (276 mg, 1.38 mmol), HATU (957 mg, 2.52 mmol), DIPEA (1.09 mL, 6.30 mmol) and 4-(2,6-difluorobenzyl)piperidine-4-carbonitrile hydrochloride (300 mg, 1.26 mmol) in DMF (8 mL) was stirred at 15°C for 16 hours. The mixture was filtered, concentrated, and purified by prep-HPLC (Column: Phenomenex Gemini-NX 80*40 mm*3 um; Condition: water (0.05%$NH_3H_2O$+10mM $NH_4HCO_3$)-ACN; Begin B: 26; End B: 52; Gradient Time (min):8; 100%B Hold Time (min): 2) to give 1-([2,4'-bipyridine]-3-carbonyl)-4-(2,6-difluorobenzyl)piperidine-4-carbonitrile (108.3 mg, 20.4%). $^1H$ NMR (400MHz, DMSO-d6) $\delta_H$ 8.85-8.77 (m, 1H), 8.69 (s, 2H), 8.06-7.84 (m, 1H), 7.72-7.52 (m, 3H), 7.50-7.34 (m, 1H), 7.21-7.07 (m, 2H), 4.69-4.48 (m, 1H), 3.18-2.95 (m, 2H), 2.89-2.58 (m, 3H), 1.94-1.50 (m, 3H), 1.28-1.15 (m, 0.5H), 0.33-0.16 (m, 0.5H). LCMS purity 96%, MS ESI calcd. For $C_{24}H_{21}F_2N_4O$ $[M+H]^+$ 419. found 419.

*Example 90. Synthesis of 4-(4-fluoro-2-(trifluoromethyl)benzyl)-1-(2-(pyrimidin-4-yl)nicotinoyl)piperidine-4-carbonitrile (A276)*

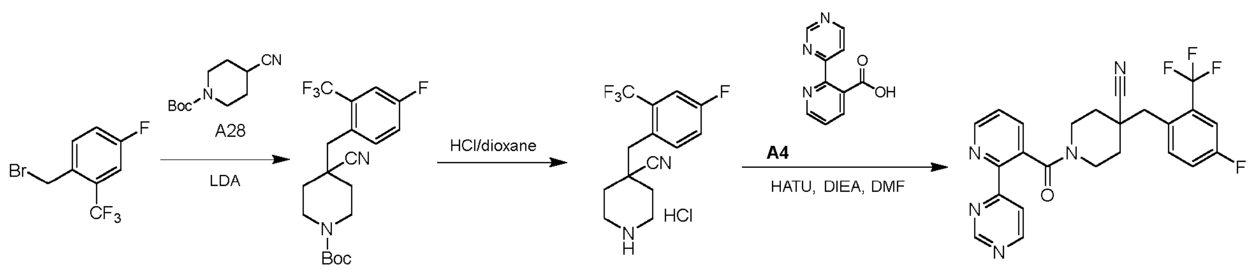


A358 A359 A360 A276

Synthesis of A359

To a solution of tert-butyl 4-cyanopiperidine-1-carboxylate (1.96 g, 9.33 mmol) in THF (40 mL) was added LDA solution (8.0 mL, 2 M in hexane, 15.5 mmol) at -60°C. After stirring for 30 minutes, 1-(bromomethyl)-4-fluoro-2-(trifluoromethyl)benzene (2 g, 7.78 mmol) in THF (10 mL) was added at -60°C. After stirring for 30 minutes, the mixture was poured into ice-water and EtOAc (50 mL) was added. The mixture was extracted with EtOAc (2 x 30 mL). The combined organic layers were dried over $Na_2SO_4$, filtered, and concentrated. The residue was purified by silica gel chromatography (PE/EtOAc = 0 to 20%) to give tert-butyl 4-cyano-4-(4-fluoro-2-(trifluoromethyl)benzyl)piperidine-1-carboxylate (2.06 g, 68.6%). $^1H$ NMR (400 MHz, $CDCl_3$) $\delta_H$ 7.76 (m, 1 H), 7.40 (m, 1 H), 7.26-7.31 (m, 1 H), 4.09-4.27 (m, 2 H), 2.91-3.09 (m, 4 H), 1.81 (d, 2 H), 1.54 (m, 2 H), 1.45 (s, 9 H).

Synthesis of A360

To a solution of tert-butyl 4-cyano-4-(4-fluoro-2-(trifluoromethyl)benzyl)piperidine-1-carboxylate (2.06 g, 5.33mmol) in dioxane (10 mL) was added HCl/dioxane (8 mL, 4M, 32.0 mmol ) at 25°C. The mixture was concentrated to give 4-(4-fluoro-2-(trifluoromethyl)benzyl)piperidine-4-carbonitrile hydrochloride (1.69 g). The reaction was mixture was used directly in the next reaction.

Synthesis of A276

A mixture of 2-(pyrimidin-4-yl)pyridine-3-carboxylic acid (105 mg, 0.523 mmol), HATU (297 mg, 0.784 mmol) in DMF (2 mL) was added DIPEA (1 mL) and 4-(4-fluoro-2-(trifluoromethyl)benzyl)piperidine-4-carbonitrile hydrochloride (150 mg, 0.523 mmol). The mixture was stirred at 20°C for 2 hours. The mixture was concentrated, and the residue was purified by prep-HPLC (Phenomenex Gemini-NX 80*30mm*3um, water (10mM $NH_4HCO_3$)-ACN Begin B 35 End B 65) to afford 4-(4-fluoro-2-(trifluoromethyl)benzyl)-1-(2-(pyrimidin-4-yl)nicotinoyl)piperidine-4-carbonitrile (52.4 mg, 21.3%). $^1H$ NMR (400 MHz, $CDCl_3$) $\delta_H$ 9.22 (s, 0.4 H), 9.09 (s, 0.6 H), 8.85-8.94 (m, 1 H), 8.76 (m, 1 H), 8.21-8.31 (m, 1 H), 7.79 (m, 1 H), 7.62-7.73 (m, 1 H), 7.37-7.51 (m, 2 H), 7.27-7.33 (m, 1 H), 4.71-5.00 (m, 1 H), 3.65 (d, 0.6 H), 3.35-3.51 (m, 1 H), 3.20-3.32 (m, 0.4 H), 3.02-3.19 (m, 3 H), 1.81-2.11 (m, 2 H), 1.62-1.79 (m, 2 H). $^{19}F$ NMR (376.5 MHz, $CDCl_3$) $\delta_F$ -57.324, -57.472, - 111.927. LCMS purity 99%, MS ESI calcd. For $C_{24}H_{19}F_4N_5O$ $[M+H]^+$ 470.1 found 470.2.

*Example 91. Synthesis of 4-(2, 6-difluoro-4-(trifluoromethyl)benzyl)-1-(2-(pyrimidin-4-yl)nicotinoyl)piperidine-4-carbonitrile (A281)*

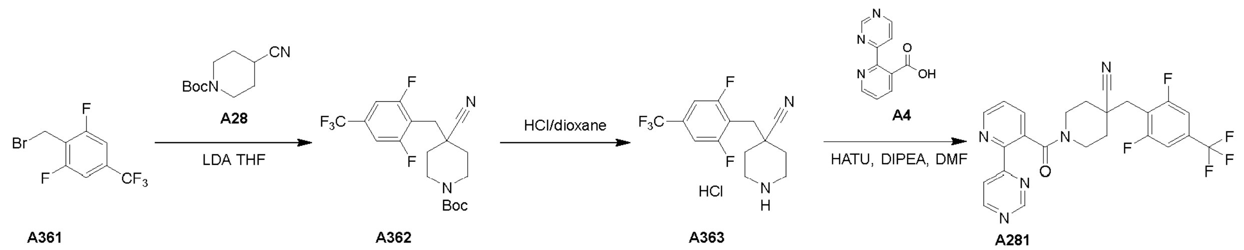


Synthesis of A362

To a mixture of tert-butyl 4-cyanopiperidine-1-carboxylate (456 mg, 2.17 mmol) in THF (10 mL) was added dropwise LDA solution (1.08 mL, 2 M, 2.17 mmol) at -78°C. The mixture was stirred at -78°C for 1 hour, and 2-(bromomethyl)-1,3-difluoro-5-(trifluoromethyl)benzene (400 mg, 1.45 mmol) was added. The mixture was warmed to 20°C stirred for 16 hours. The mixture was poured into water (100 mL) and extracted with EtOAc (3x100 mL). The combined organic layers were washed with brine (2 x 50 mL), dried over $Na_2SO_4$, filtered, and concentrated to give tert-butyl 4-cyano-4-(2,6-difluoro-4-(trifluoromethyl)benzyl)piperidine-1-carboxylate (0.5 g).

Synthesis of A363

To a solution of tert-butyl 4-cyano-4-(2,6-difluoro-4-(trifluoromethyl)benzyl)piperidine-1-carboxylate (0.5 g, 1.23 mmol) in dioxane (5 mL) was added HCl/dioxane (1.53 mL, 4 M in dioxane, 6.15 mmol). The mixture was stirred at 25°C for 4 hours. The mixture was concentrated to give 4-(2,6-difluoro-4-(trifluoromethyl)benzyl)piperidine-4-carbonitrile hydrochloride (400 mg). The product was used directly in the next step.

Synthesis of A281

A solution of 2-(pyrimidin-4-yl)pyridine-3-carboxylic acid (118 mg, 0.59 mmol), HATU (334 mg, 0.88 mmol), DIPEA (378 mg, 2.93 mmol) and 4-(2,6-difluoro-4-(trifluoromethyl)benzyl)piperidine-4-carbonitrile hydrochloride (200 mg, 0.59 mmol) in DMF (5 mL) was stirred at 25°C for 2 hours. The mixture was poured into water (20 mL) and extracted with EtOAc (3 x 10 mL). The combined organic layers were washed with brine (2 x 20 mL), dried over anhydrous $Na_2SO_4$, filtered, and concentrated. The residue was purified by prep-HPLC (Column: Phenomenex Gemini-NX 80*30mm*3μm); Condition: water (10mM $NH_4HCO_3$)-ACN; Begin B: 33%; End B:63 %) to afford 4-(2,6-difluoro-4-(trifluoromethyl) benzyl)-1-(2-(pyrimidin-4-yl)nicotinoyl)piperidine-4-carbonitrile (68.5 mg, 24%). $^1H$ NMR (400 MHz, $CDCl_3$) $\delta_H$ 9.31-8.82 (m, 2 H), 8.80-8.74 (m, 1 H), 8.30-8.22 (m, 1 H), 7.71-7.64 (m, 1 H), 7.50-7.44 (m, 1 H), 7.26-7.20 (m, 2 H), 5.01-4.71 (m, 1 H), 3.69-3.60 (m, 1 H), 3.52-3.36 (m, 1 H), 3.18-2.96 (m, 3 H), 2.27-1.70 (m, 4 H). $^{19}F$ NMR (376.5 MHz, $CDCl_3$). $\delta_F$ -63.188, -107.296. LCMS purity 99%, calcd. for $C_{24}H_{18}F_5N_5O$ $[M+H]^+$ 488.2, found 488.2.

*Example 92. Synthesis of 1-([2, 4'-bipyridine]-3-carbonyl)-4-(2, 6-difluoro-4-(trifluoromethyl)benzyl)piperidine-4-carbonitrile (A280)*

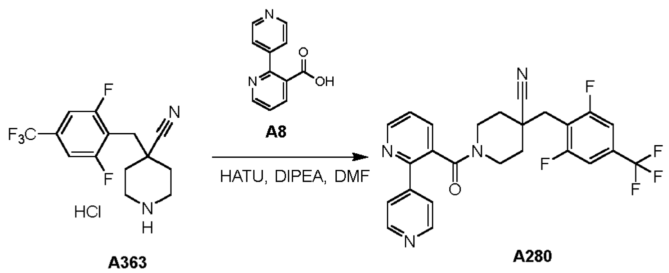


A363 A280

A mixture of [2,4'-bipyridine]-3-carboxylic acid (118 mg, 0.59 mmol), HATU (334 mg, 0.88 mmol), DIPEA (378 mg, 2.93 mmol) and 4-(2,6-difluoro-4-(trifluoromethyl)benzyl)piperidine-4-carbonitrile hydrochloride (200 mg, 0.59 mmol) in DMF (5 mL) was stirred at 25°C for 2 hours. The mixture was poured into water (20 mL) and extracted with EtOAc (3x10 mL). The combined organic layers were washed with brine (2 x 20 mL), dried over anhydrous $Na_2SO_4$, filtered, concentrated. The residue was purified by prep-HPLC (Column: Phenomenex Gemini-NX 80*30mm*3μm); Condition: water(10mM $NH_4HCO_3$)-ACN; Begin B: 33%; End B:63 %) to afford 1-([2,4'-bipyridine]-3-carbonyl)-4-(2,6-difluoro-4-(trifluoromethyl)benzyl)piperidine-4-carbonitrile (12.2 mg, 4%). $^1H$ NMR (400 MHz, $CDCl_3$) $\delta_H$ 8.88-8.62 (m, 3 H), 7.82-7.54 (m, 3 H), 7.50-7.42 (m, 1 H), 7.24-7.18 (m, 2 H), 4.90-4.80 (m, 1 H), 3.15-2.58 (m, 5 H), 2.04-1.91 (m, 1 H), 1.51-1.37 (m, 1 H), 1.35-1.20 (m, 1 H), 0.09-0.07 (m, 1 H). $^{19}F$ NMR (376.5 MHz, $CDCl_3$) $\delta_F$ -63.247 -107.349. LCMS purity 99%, calcd. for $C_{25}H_{19}F_5N_4O$ $[M+H]^+$ 487.2, found 487.4.

*Example 93. Synthesis of 1-([2,4'-bipyridine]-3-carbonyl)-4-(2,4-difluorobenzyl)piperidine-4-carbonitrile (A284)*

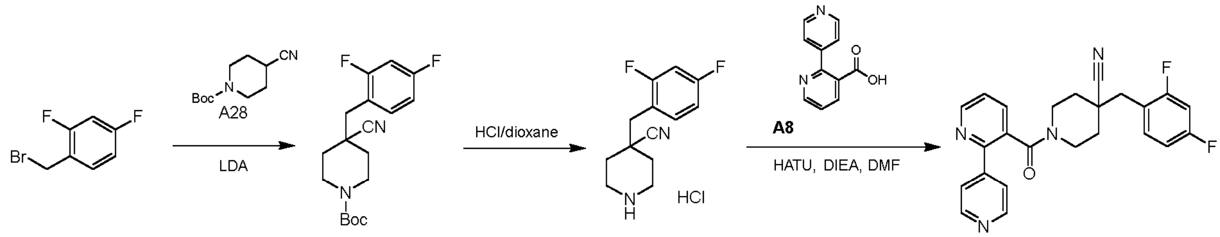


A364 A365 A366 A284

Synthesis of A365

LDA solution (3.55 mL, 7.10 mmol) was added to tert-butyl 4-cyanopiperidine-1-carboxylate (500 mg, 2.37 mmol) in THF (5 mL) at 0°C. After stirring for 30 minutes, 1-(bromomethyl)-2,4-difluorobenzene (587 mg, 2.84mmol) was added the mixture. The mixture was stirred at 25°C for 2 hours. The mixture was poured into water (10 mL) and extracted with EtOAc (3 x 10 mL). The combined organic layers were washed with brine (2 x 10 mL), dried over $Na_2SO_4$, filtered, and

concentrated to give tert-butyl 4-cyano-4-(2,4-difluorobenzyl)piperidine-1-carboxylate (500 mg).

Synthesis of A366

To a solution of tert-butyl 4-cyano-4-(2,4-difluorobenzyl)piperidine-1-carboxylate (500 mg, 1.48 mmol) in dioxane (2.5 mL) was added dioxane/HCl (5.0 mL) at 25°C for 16 hours. The reaction mixture was concentrated to give 4-(2,4-difluorobenzyl)piperidine-4-carbonitrile hydrochloride (700 mg).

Synthesis of A284

A mixture of [2,4'-bipyridine]-3-carboxylic acid (220 mg, 1.10 mmol), HATU (479 mg, 1.26 mmol), DIPEA (546 mg, 4.23 mmol) and 4-(2,4-difluorobenzyl)piperidine-4-carbonitrile hydrochloride (200 mg, 0.846 mmol) in DMF (5 mL) was stirred at 25°C for 2 hours. The mixture was poured into water (10 mL) and extracted with EtOAc (3 x 10 mL). The combined organic layers were washed with brine (2 x 10 mL), dried over $Na_2SO_4$, filtered, and concentrated to give 200 mg. The residue was purified by prep-HPLC (Column: Phenomenex Gemini-NX 80*30mm*3um; Condition: water(10mM $NH_4HCO_3$)-ACN; Begin B: 33; End B: 63) to afford 1-([2,4'-bipyridine]-3-carbonyl)-4-(2,4-difluorobenzyl)piperidine-4-carbonitrile (129.4 mg, 36.4%). $^1H$ NMR (400 MHz, $CDCl_3$) $\delta_H$ 8.81-8.77 (m, 3 H), 7.78-7.58 (m, 3 H), 7.46-7.43 (m, 1 H), 7.22-7.20 (m, 1 H), 6.89-6.79 (m, 2 H), 4.83-4.80 (m, 1 H), 3.20-3.00 (m, 2 H), 2.95-2.85 (m, 1 H), 2.60-2.48 (m, 2 H), 1.94-1.84 (m, 1 H), 1.36-1.22 (m, 2 H), 0.08-0 (m, 1H). $^{19}F$ NMR (376.5 MHz, $CDCl_3$) $\delta_F$ -109.85, -109.86, -110.79, - 110.81. LCMS purity 99%, calcd. for $C_{24}H_{21}F_2N_4O$ $[M+H]^+$ 419.2, found 419.2.

*Example 94. Synthesis of 1-([2,4'-bipyridine]-3-carbonyl)-4-(4-(trifluoromethyl)benzyl)piperidine-4-carbonitrile (A285)*

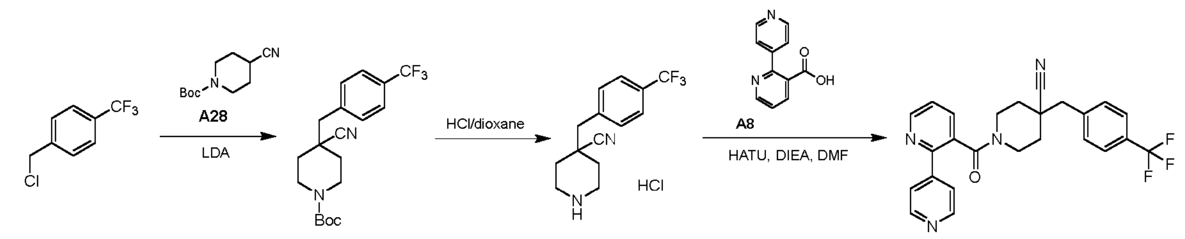


A367 A368 A369 A285

Synthesis of A368

To LDA (2.56 mL, 5.13 mmol) solution was added tert-butyl 4-cyanopiperidine -1-carboxylate (897 mg, 4.27 mmol) in THF (15 mL) at 0°C. After stirring for 30 minutes, 1-(chloromethyl)-4-(trifluoromethyl) benzene (1 g, 5.13 mmol) was added to the mixture. The mixture was stirred at 25°C for 2 hours. The mixture was poured into water (10 mL) and extracted with EtOAc (3 x 10 mL). The combined organic layers were washed with brine (2 x 10 mL), dried over $Na_2SO_4$, filtered, and concentrated to give tert-butyl 4-cyano-4-(4-(trifluoromethyl)benzyl)piperidine-1-carboxylate (1.5 g).

Synthesis of A369

To a solution of tert-butyl 4-cyano-4-(4-(trifluoromethyl)benzyl)piperidine-1-carboxylate (200 mg, 0.543 mmol) in dioxane (2 mL) was added dioxane/HCl (4.0 mL) at 25°C for 2 hours. The solution was concentrated to give 4-(4-(trifluoromethyl)benzyl)piperidine-4-carbonitrile hydrochloride (150 mg).

Synthesis of A285

A mixture of [2,4'-bipyridine]-3-carboxylic acid (134 mg, 0.671 mmol), HATU (318 mg, 0.839 mmol), DIPEA (360 mg, 2.79 mmol) and 4-(4-(trifluoromethyl)benzyl)piperidine-4-carbonitrile hydrochloride (150 mg, 0.559 mmol) in DMF (5 mL) was stirred at 25°C for 2 hours. The mixture was poured into water (10 mL) and extracted with EtOAc (3 x 10 mL). The combined organic layers were washed with brine (2 x 10 mL), dried over $Na_2SO_4$, filtered, and concentrated to give 200 mg. The residue was purified by prep-HPLC (Column: Phenomenex Gemini-NX 80*30mm*3um; Condition: water (10mM $NH_4HCO_3$)-ACN; Begin B: 37; End B: 67) to afford 1-([2,4'-bipyridine]-3-carbonyl)-4-(4-(trifluoromethyl)benzyl)piperidine-4-carbonitrile (66 mg, 26.2%). $^1H$ NMR (400 MHz, $CDCl_3$) $\delta_H$ 8.90-8.69 (m, 3 H), 7.80-7.78 (m, 2 H), 7.64-7.52 (m, 3

H), 7.49-7.33 (m, 2 H), 7.25-7.23 (m, 1 H), 4.83-4.79 (m, 1 H), 3.12-2.87 (m, 3 H), 2.68-2.61 (m, 1 H), 2.52-2.40 (m, 1 H), 1.95-1.84 (m, 1 H), 1.31-1.13 (m, 2 H), 0.14-0.27 (m, 1 H). $^{19}$F NMR (376.5 MHz, $CDCl_3$) $\delta_F$ -62.667. LCMS purity 99%, calcd. for $C_{25}H_{22}F_3N_4O$ $[M+H]^+$ 451.2, found 451.2.

*Example 95. Synthesis of (R)-4-(1-(2-fluoro-4-(trifluoromethyl)phenyl)ethyl)-1-(2-(pyrimidin-4-yl)nicotinoyl)piperidine-4-carbonitrile (A286) and (S)-4-(1-(2-fluoro-4-(trifluoromethyl)phenyl)ethyl)-1-(2-(pyrimidin-4-yl)nicotinoyl)piperidine-4-carbonitrile (A287)*

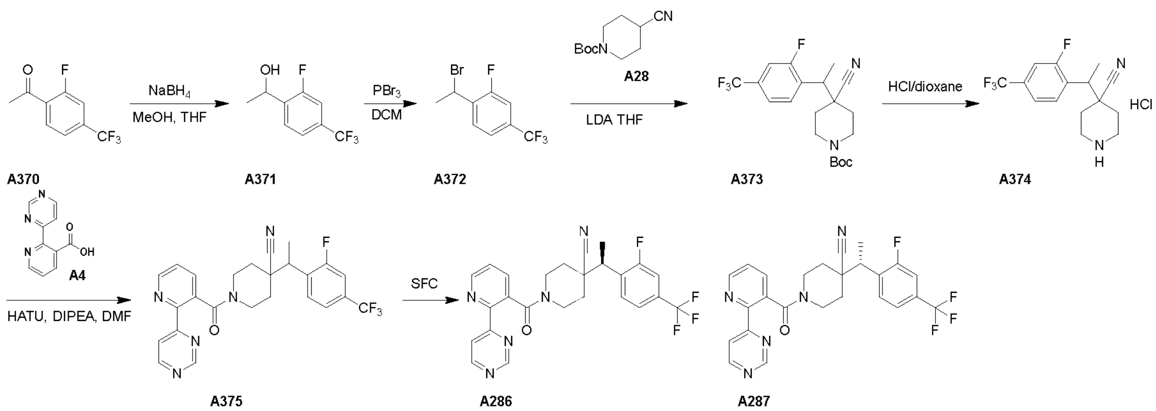


Synthesis of A371

To a solution of 1-(2-fluoro-4-(trifluoromethyl)phenyl)ethan-1-one (2.0 g, 9.70 mmol) in MeoH (10 mL) was added $NaBH_4$ (0.773 g, 19.4 mmol) portion-wise at 0°C over a period of 2 hours under nitrogen and then was allowed warm to 20°C. The mixture was poured into ice-water (80 mL) and stirred for 20 minutes and extracted with EtOAc (3 x 30 mL). The combined organic layers were dried over anhydrous $Na_2SO_4$, filtered, and concentrated to give 1-(2-fluoro-4-(trifluoromethyl)phenyl)ethan-1-ol (1.9 g). $^1$H NMR (400 MHz, $CDCl_3$) $\delta_H$ 7.68-7.64 (m, 1 H), 7.45-7.43 (m, 1 H), 7.30-7.26 (m, 1 H), 5.27-5.26 (m, 1H), 2.01-1.99 (m, 1H), 1.53-1.51 (m, 3H). $^{19}$F NMR (376.5 MHz, $CDCl_3$) $\delta_F$ -62.658, -117.684.

Synthesis of A372

To a solution of 1-(2-fluoro-4-(trifluoromethyl)phenyl)ethan-1-ol (0.4 g, 1.92 mmol) in $CHCl_3$ (10 mL) was added tribromophosphane (1.29 g, 4.80 mmol) at 0°C. The reaction mixture was stirred at 20°C for 2 hours. Saturated aqueous $NaHCO_3$ solution (50 mL) was added, and the mixture was extracted with EtOAc (3 x 50 mL). The combined organic layers were dried over $Na_2SO_4$, filtered, and concentrated to give 1-(1-bromoethyl)-2-fluoro-4-(trifluoromethyl)benzene (140 mg). $^1$H NMR (400 MHz, $CDCl_3$) $\delta_H$ 7.71-7.65 (m, 1 H), 7.52-7.40 (m, 1 H), 7.36-7.30 (m, 1 H), 5.51-5.41 (m, 1 H), 2.08-2.03 (m, 3 H). $^{19}$F NMR (376.5 MHz, CDCl3) $\delta_F$ -62.915,-114.755.

Synthesis of A373

To a solution of n-BuLi (0.616 mL, 1.54 mmol) was added i-$Pr_2NH$ (155 mg, 1.54 mmol) in THF (2 mL) drop-wise at -70°C under $N_2$. After stirring for 30 minutes, tert-butyl 4-cyanopiperidine-1-carboxylate (162 mg, 0.775 mmol) in THF (8 mL) was added at -78°C. After stirring for 30 minutes, 1-(1-bromoethyl)-2-fluoro-4-(trifluoromethyl)benzene (0.14 g, 0.516 mmol) was added into one portion. The reaction mixture was allowed warm to 25°C and stirred for 2 hours. The resulting mixture was quenched with saturated aqueous $NH_4C1$ solution (100 mL) at 0°C and extracted with EtOAc (3 x 20 mL). The combined organic layers were washed with brine (2 x 10 mL), dried over $Na_2SO_4$, filtered, and concentrated to give tert-butyl 4-cyano-4-(1-(2-fluoro-4-(trifluoromethyl)phenyl)ethyl)piperidine-1-carboxylate (0.2 g). $^1$H NMR (400 MHz, $CDCl_3$) $\delta_H$ 7.77-7.64 (m, 1 H), 7.51-7.44 (m, 1 H), 7.38-7.24 (m, 1 H), 4.27-4.01 (m, 2 H), 3.30-3.22 (m, 1 H), 3.09-2.87 (m, 2 H), 2.22-2.15 (m, 1 H), 1.91-1.72 (m, 1 H), 1.68-1.61 (m, 2 H), 1.47-1.46 (m, 3 H), 1.45 (s, 9 H). $^{19}$F NMR (376.5 MHz, $CDCl_3$) $\delta_F$ -62.850.

Synthesis of A374

To a solution of tert-butyl 4-cyano-4-(1-(2-fluoro-4-(trifluoromethyl)phenyl)ethyl)piperidine-1-carboxylate (200

mg, 0.4994mmol) in dioxane (2 mL) was added dioxane/HCl (1.24 mL, 4 M in dioxane) at 25°C and stirring was continued for 2 hours. The reaction mixture was concentrated to give 4-(1-(2-fluoro-4-(trifluoromethyl)phenyl)ethyl)piperidine-4-carbonitrile hydrochloride (160 mg).

Synthesis of A375 and SFC Separation

A mixture of 2-(pyrimidin-4-yl)pyridine-3 (124 mg, 0.618 mmol), HATU (270 mg, 0.713 mmol), DIPEA (306 mg, 2.37 mmol) and 4-(1-(2-fluoro-4-(trifluoromethyl)phenyl)ethyl)piperidine-4-carbonitrile hydrochloride (160 mg, 0.475 mmol) in DMF (5 mL) was stirred at 25°C for 2 hours. The mixture was poured into water (10 mL) and extracted with EtOAc (2 x 10 mL). The combined organic layers were washed with brine (2 x 10 mL), dried over $Na_2SO_4$, filtered, and concentrated to give 250 mg. The residue was purified by prep-HPLC (Column: Phenomenex Gemini-NX 80*30mm*3um; Condition: water (10mM $NH_4HCO_3$)-ACN; Begin B: 40; End B: 70) to afford racemic-4-(1-(2-fluoro-4-(trifluoromethyl) phenyl)ethyl)-1-(2-(pyrimidin-4-yl)nicotinoyl)piperidine-4-carbonitrile (120 mg, 52.4%), which was further purified.

SFC (Column: DAICEL CHIRALCEL OD-H (250mm*30mm, 5um); Condition: 0.1%$NH_3H_2O$ ETOH; Begin B: 20%; End B: 20%) to give:

(R)-4-(1-(2-fluoro-4-(trifluoromethyl)phenyl)ethyl)-1-(2-(pyrimidin-4-yl)nicotinoyl)piperidine-4-carbonitrile (39.1 mg, 32.8%).

$^1$H NMR (400 MHz, $CDCl_3$) $\delta_H$ 9.28-9.07 (m, 1 H), 8.98-8.87 (m, 1 H), 8.82-8.72 (m, 1 H), 8.35-8.21 (m, 1 H), 7.73-7.62 (m, 2 H), 7.57-7.34 (m, 3 H), 5.02-4.66 (m, 1 H), 3.57-3.43 (m, 1 H), 3.36-3.25 (m, 2 H),3.20-2.95 (m, 3 H), 2.17-1.65 (m, 3 H), 1.53-1.44 (m, 2 H), 1.26-1.17 (m, 1 H). $^{19}$F NMR (376.5 MHz, CDCl3) δF -62.906. LCMS purity 99%, calcd. for $C_{25}H_{22}F_4N_5O$ $[M+H]^+$ 484.1, found 484.1.

(S)-4-(1-(2-fluoro-4-(trifluoromethyl)phenyl)ethyl)-1-(2-(pyrimidin-4-yl)nicotinoyl)piperidine-4-carbonitrile (45.3 mg, 38.0%).

$^1$H NMR (400 MHz, $CDCl_3$) $\delta_H$ 9.26-9.08 (m, 1 H), 8.94-8.82 (m, 1 H), 8.78-8.71 (m, 1 H), 8.33-8.20 (m, 1 H), 7.75-7.62 (m, 2 H), 7.55-7.30 (m, 3 H), 5.00-4.67 (m, 1 H), 3.57-3.43 (m, 1 H), 3.36-3.25 (m, 2 H),3.20-2.95 (m, 3 H), 2.17-1.65 (m, 3 H), 1.53-1.44 (m, 2 H), 1.27-1.16 (m, 1 H). $^{19}$F NMR (376.5 MHz, $CDCl_3$) $\delta_F$ -62.906. LCMS purity 99%, calcd. for $C_{25}H_{22}F_4N_5O$ $[M+H]^+$ 484.1, found 484.1.

*Example 96. Synthesis of (1R,3r,5S)-3-(4-fluorobenzyl)-8-(2-(pyrimidin-4-yl)nicotinoyl)-8-azabicyclo[3.2.1]octane-3-carbonitrile (A252)*

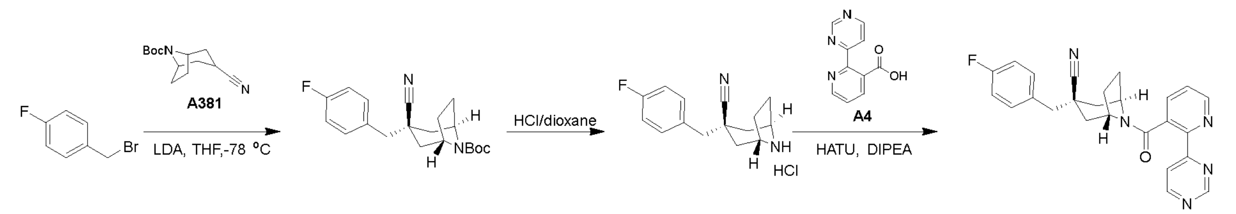


A376 A377 A378 A252

Synthesis of A377

To a solution of tert-butyl 8-cyano-3-azabicyclo[3.2.1]octane-3-carboxylate (200mg, 846 μmol) in THF (10 mL) was added LDA solution (1.05 mL, 2 M in hexane, 2.10 mmol) at -60°C. After stirring for 30 minutes, 1-(bromomethyl)-4-fluorobenzene (200 mg, 1.05 mmol) and stirring was continued for 1 hour. The mixture was warmed to 25°C and stirred for 16 hours. The mixture was poured into ice-water and saturated aqueous $NH_4Cl$ solution (50 mL) was added. The mixture was extracted with EtOAc (2 x 30 mL). The combined organic layers were dried over $Na_2SO_4$, filtered, and concentrated. The residue was purified by silica gel chromatography (PE/EtOAc = 0 to 40%) to give tert-butyl (1R,3r,5S)-3-cyano-8-azabicyclo[3.2.1]octane-8-carboxylate (345 mg, 95.5%). $^1$H NMR (400 MHz, $CDCl_3$) $\delta_H$ 7.21-7.25 (m, 2 H), 7.02 (m, 2 H), 4.28 (d, 2 H), 2.75 (s, 2 H), 2.19 (m, 2 H), 2.02 (m, 2 H), 1.92 (s, 2 H), 1.46 (s, 9 H), 1.22-1.32 (m, 2 H).

Synthesis of A378

To a solution of tert-butyl (1R,3r,5S)-3-cyano-8-azabicyclo[3.2.1]octane-8-carboxylate (345 mg,1.00 mmol) in

dioxane (2 mL) was added HCl/Dioxane (8 mL, 4M, 32.0 mmol ) was stirred at 25°C for 16 hours. The mixture was concentrated under reduce pressure to give (1R,3r,5S)-3-(4-fluorobenzyl)-8-azabicyclo[3.2.1]octane-3-carbonitrile hydrochloride (254 mg). The reaction was mixture was used directly for the next step without monitor.

Synthesis of A252

To a mixture of 2-(pyrimidin-4-yl) pyridine-3-carboxylic acid (71.4 mg, 355 μmol), HATU (202 mg, 533 μmol) in DMF (15 mL) was added DIPEA (0.8 mL) and (1R,3r,5S)-3-(4-fluorobenzyl)-8-azabicyclo[3.2.1]octane-3-carbonitrile hydrochloride (120 mg, 427 μmmol). The mixture was stirred at 20°C for 16 hours. The mixture was concentrated, and the residue was purified by prep-HPLC (water (10mM $NH_4HCO_3$)-ACN Begin B 29 End B 59 Gradient Time (min): 9; 100% Hold Time (min): 2) to afford (1R,3r,5S)-3-(4-fluorobenzyl)-8-(2-(pyrimidin-4-yl)nicotinoyl)-8-azabicyclo[3.2.1]octane-3-carbonitrile (63.6 mg, 42.3%). $^1$H NMR (400 MHz, $CDCl_3$) $\delta_H$ 9.24 (s, 1 H), 8.93 (d, 1 H), 8.77-8.83 (m, 1 H), 8.22-8.26 (m, 1 H), 7.66-7.71 (m, 1 H), 7.49 (dd, 1 H), 7.22-7.26 (m, 2 H), 7.03 (t, 2 H), 4.96-5.04 (m, 1 H), 3.89-4.01 (m, 1 H), 2.83-2.90 (m, 2 H), 2.35-2.45 (m, 1 H), 2.08-2.33 (m, 6 H), 1.84-1.97 (m, 1 H). LC-ELSD/MS purity 95%, MS ESI calcd. For $C_{25}H_{22}FN_5O$ [M+H]+ 428.1 found 428.3.

*Example 97. Synthesis of (1R,5S,8r)-8-(4-fluorobenzyl)-3-(2-(pyrimidin-4-yl)nicotinoyl)-3-azabicyclo[3.2.1]octane-8-carbonitrile (A253)*

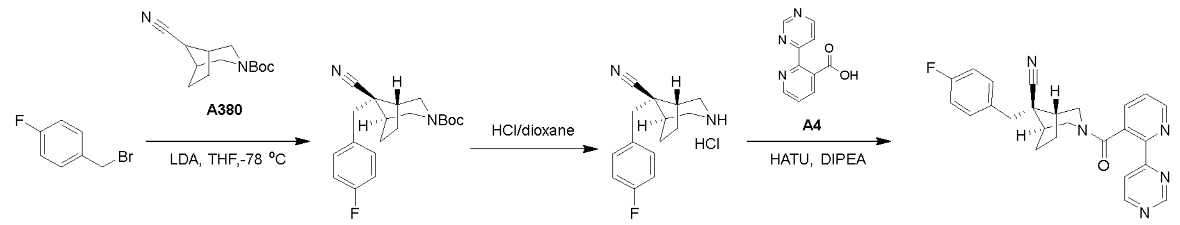


A379 A382 A383 A253

Synthesis of A382

To a solution of tert-butyl 8-cyano-3-azabicyclo[3.2.1]octane-3-carboxylate (200mg, 0.846 mol) in THF (10 mL) was added LDA solution (845 μL, 2 M in hexane, 1.69 mmol) at -70°C. After stirring for 30 minutes, 1-(bromomethyl)-4-fluorobenzene (190 mg, 1.01 mmol) was added at -70°C and stirred for 1 hour. The mixture was warmed to 25°C and stirred for 16 hours. The mixture was poured into ice-water and saturated aqueous $NH_4Cl$ solution (50 mL) was added. The mixture was extracted with EtOAc (2 x 30 mL). The combined organic layers were dried over $Na_2SO_4$, filtered, and concentrated. The residue was purified by HPLC (water (10mM $NH_4HCO_3$)-ACN Begin B 60 End B 90 ) to afford tert-butyl (1R,5S,8r)-8-cyano-8-(4-fluorobenzyl)-3-azabicyclo[3.2.1]octane-3-carboxylate (58 mg, 19.9%). $^1$H NMR (400 MHz, $CDCl_3$) $\delta_H$ 7.27-7.32 (m, 2 H), 7.05 (m, 2 H), 3.72-4.01 (m, 2 H), 3.07-3.38 (m, 2 H), 2.75 (s, 2 H), 2.11-2.24 (m, 2 H), 1.90-2.01 (m, 2 H), 1.67-1.83 (m, 2 H), 1.45 (s, 9 H).

Synthesis of A383

To a solution of tert-butyl (1R,5S,8r)-8-cyano-8-(4-fluorobenzyl)-3-azabicyclo[3.2.1]octane-3-carboxylate (105 mg, 304 μmol) in dioxane (2 mL) was added HCl/Dioxane (4 mL, 4M, 16.0 mmol ) at 25°C. The mixture was concentrated to give (1R,5S,8r)-8-(4-fluorobenzyl)-3-azabicyclo[3.2.1]octane-8-carbonitrile hydrochloride (120 mg), which was used directly in the next reaction.

Synthesis of A253

To a mixture of 2-(pyrimidin-4-yl)pyridine-3-carboxylic acid (68.4 mg, 340 μmol), HATU (194 mg, 511 μmol) in DMF (15 mL) was added DIPEA (0.8 mL) and (1R,5S,8r)-8-(4-fluorobenzyl)-3-azabicyclo[3.2.1]octane-8-carbonitrile hydrochloride (100mg, 409 μmol). The mixture was stirred at 20°C for 16 hours. The mixture solution was concentrated under reduce pressure, and the residue was purified by HPLC (water (10mM $NH_4HCO_3$)-ACN Begin B 10 End B 80) to afford (1R,5S,8r)-8-(4-fluorobenzyl)-3-(2-(pyrimidin-4-yl)nicotinoyl)-3-azabicyclo[3.2.1]octane-8-carbonitrile (66 mg, 45.5%). $^1$H NMR (400 MHz, $CDCl_3$) $\delta_H$ 9.16-9.27 (m, 1 H), 8.83-8.93 (m, 1 H), 8.72-8.76 (m, 1 H), 8.21-8.28 (m, 1 H), 7.61-7.73 (m, 1 H), 7.40-7.50 (m, 1 H), 7.26-7.32 (m, 2 H), 7.04 (q, 2 H), 4.33-4.69 (m, 1 H), 3.50-3.67 (m, 1 H), 3.30-3.46 (m,

1.5 H), 3.15 (d, 0.5 H), 2.71-2.86 (m, 2 H), 2.41 (s, 1 H), 1.90-2.19 (m, 4.5 H), 1.73 (d, 0.5 H). LC-ELSD/MS purity 99%, MS ESI calcd. For $C_{25}H_{22}FN_5O$ [M+H]+ 428.1 found 428.3.

*Example 98. Synthesis of 4-(4-fluorobenzyl)-1-(2-(pyrimidin-5-yl)nicotinoyl)piperidine-4-carbonitrile (A66)*

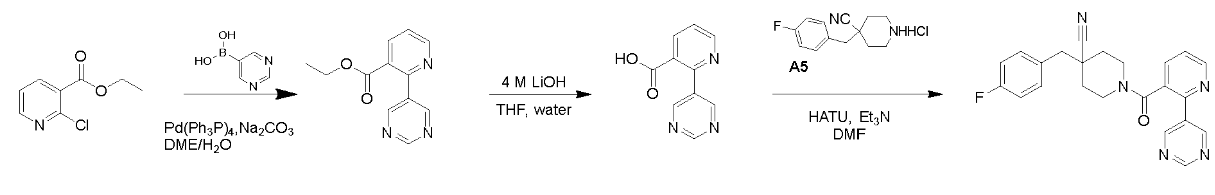


A384 A385 A386 A66

Synthesis of A385

A mixture of ethyl 2-chloronicotinate (0.5 g, 2.69 mmol), pyrimidin-5-ylboronic acid (398 mg, 3.22 mmol), tetrakis(triphenylphosphane) palladium (310 mg, 0.269 mmol), and $Na_2CO_3$ (570 mg, 5.37 mmol) in DME (10 mL) and water (2 mL) was stirred at 80°C for 16 hours. The mixture was filtered, concentrated, and the residue was purified by silica gel chromatography (PE/EtOAc = 2/1 to 1/2) to afford ethyl 2-(pyrimidin-5-yl)nicotinate (0.5 g, 1.74 mmol).$^1$H NMR (400 MHz, $CDCl_3$) $\delta_H$ 9.28 (s, 1H), 8.91 (s, 2H), 8.87 (dd, 1H), 8.35 (dd, 1H), 7.56 - 7.54 (m, 1H), 4.27 (q, 2H), 1.19 (t, 3H).

Synthesis of A386

A mixture of ethyl 2-(pyrimidin-5-yl)nicotinate (0.5 g, 2.18 mmol) and LiOH·$H_2O$ (178 mg, 4.36 mmol) in THF (10 mL) and water (10 mL) was stirred at 15°C for 2 hours. The mixture concentrated to give 2-(pyrimidin-5-yl)nicotinic acid (0.6 g). $^1$H NMR (400 MHz, DMSO-$d_6$) $\delta_H$ 9.12 (s, 1H), 9.04 (s, 2H), 8.53 (dd, 1H), 7.84 (dd, 1H), 7.35 (dd, 1H).

Synthesis of A66

A mixture of 2-(pyrimidin-5-yl)nicotinic acid (50 mg, 0.248 mmol), HOBt (67.1 mg, 0.497 mmol), EDCI (95.2 mg, 0.497 mmol), DIPEA (96.3 mg, 0.745 mmol) and 4-[(4-fluorophenyl)methyl]piperidine-4-carbonitrile hydrochloride (75.9 mg, 0.298 mmol) in DCM (2 mL) was stirred at 0 °C for 2h. The mixture was filtered, concentrated and the resiue was purified by prep-HPLC (Column: Welch Xtimate C18 150 * 25mm * 5 um; Condition: water(0.04%$NH_3H_2O$)-ACN; Begin B: 30; End B: 50; Gradient Time (min):8.5; 100%B Hold Time (min): 2 ) to give the product, which was triturated from n-hexane (0.5 mL) to afford 4-(4-fluorobenzyl)-1-(2-(pyrimidin-5-yl)nicotinoyl)piperidine-4-carbonitrile (4.8 mg, 4.76%). $^1$H NMR (400 MHz, $CDCl_3$) $\delta_H$ 9.32 (s, 1H), 9.22 - 8.99 (m, 2H), 8.83 (dd, 1H), 7.79 (dd, 1H), 7.46 (dd, 1H), 7.24 - 7.06 (m, 2H), 7.06 - 6.97 (m, 2H), 4.81 (d, 1H), 3.37 - 3.02 (m, 2H), 3.01 - 2.49 (m, 3.4H), 1.92 (s, 1H), 1.25 (s, 2H), 0.07 (s, 0.6H). LC/MS purity>=99%, MS ESI calcd. For $C_{23}H_{20}FN_5O$ $[M+H]^+$ 402.2, found 402.2.

*Example 99. Synthesis of 1-([2,4'-bipyridine]-3-carbonyl)-4-(3,5-difluorobenzyl)piperidine-4-carbonitrile (A70)*

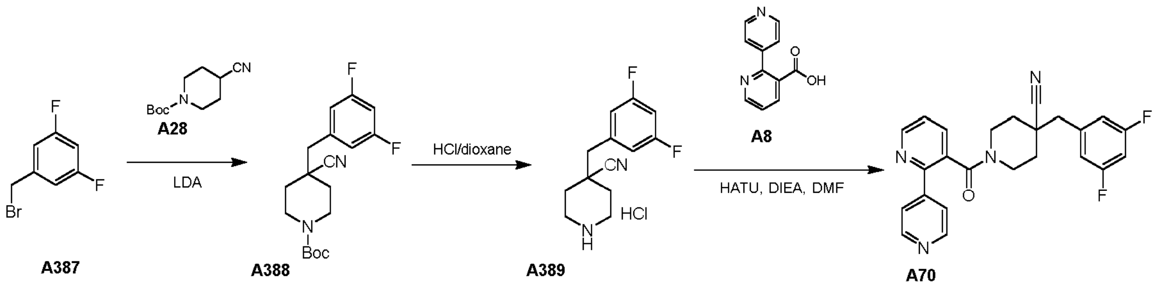


A387 A388 A389 A70

Synthesis of A388

To a mixture of tert-butyl 4-cyanopiperidine-1-carboxylate (607 mg, 2.89 mmol) in THF (10 mL) was added dropwise LDA solution (1.80 mL, 2 M, 3.61 mmol) at -78°C. After stirring at -78°C for 1 hour, 1-(bromomethyl)-3,5-difluorobenzene (500 mg, 2.41 mmol) was added, and the mixture was warmed to 20°C and stirred for 3 h. The mixture was poured into water (25 mL) and extracted with EtOAc (3 x 20 mL). The combined organic layers were washed with brine (2 x 50 mL), dried over $Na_2SO_4$, filtered and concentrated to give tert-butyl 4-cyano-4-(3,5-difluorobenzyl)piperidine-1-carboxylate (800 mg). $^1H$ NMR (400MHz, $CDCl_3$) $\delta_H$ 6.87-6.73 (m, 3H), 4.27-4.06 (m, 2H), 3.00 (s, 2H), 1.85 (m, 2H), 1.55-1.49 (m, 1H), 1.46 (s, 9H).

Synthesis of A389

To tert-butyl 4-cyano-4-(3,5-difluorobenzyl)piperidine-1-carboxylate (0.8 g, 2.37 mmol) in dioxane (5 mL) was added HCl/dioxane (4.52 mL, 4 M in dioxane, 18.1mmol). The mixture was stirred at 25°C for 4 hours. The mixture was concentrated to give 4-(3,5-difluorobenzyl)piperidine-4-carbonitrile hydrochloride (600 mg). $^1H$ NMR (400MHz, $CDCl_3$) $\delta_H$ 6.91-6.77 (m, 3H), 3.72 (s, 3H), 3.58 (m, 2H), 3.19 (s, 2H), 2.24-2.11 (m, 2H), 1.29-1.23 (m, 2H).

Synthesis of A70

To a mixture of [2,4'-bipyridine]-3-carboxylic acid (252 mg, 1.26 mmol), 4-(3,5-difluorobenzyl)piperidine-4-carbonitrile hydrochloride (300 mg, 1.26 mmol) and HATU (718 mg, 1.89 mmol) in DMF (5 mL) was added DIPEA (0.659 mL, 3.78 mmol). The mixture was stirred at 20°C for 12 hours. The reaction mixture was poured into water (50 mL) and stirred for 20 minutes. The aqueous phase was extracted with EtOAc (3 x 20 mL). The combined organic layers were washed with brine (2 x 20 mL), dried over anhydrous $Na_2SO_4$, filtered and concentrated to afford 70 mg. The residue was purified by prep-HPLC (Column Phenomenex Gemini-NX 80*30mm*3um Condition water (10mM $NH_4HCO_3$)-ACN Begin B 34 End B 64 Gradient Time(min) 9 100%B Hold Time(min) 1.5 FlowRate(ml/min) 30 Injections 6) to afford 1-([2,4'-bipyridine]-3-carbonyl)-4-(3,5-difluorobenzyl)piperidine-4-carbonitrile (70.7 mg, 13.4%). $^1H$ NMR (400MHz, $CDCl_3$) $\delta_H$ 8.87-8.65 (m, 3H), 7.78 (m, 2H), 7.61 (s, 1H), 7.46 (m, 1H), 6.87-6.60 (m, 3H), 4.85 (m, 1H), 3.17-2.78 (m, 3H), 2.61 (m, 1H), 2.39 (m, 1H), 2.02-1.84 (m, 1H), 1.32-1.13 (m, 2H), -0.17--0.31 (m, 1H). $^{19}F$ NMR (376.5 MHz, $CDCl_3$) $\delta_F$ -108.898. LCMS purity 99%, MS ESI calcd. For $C_{24}H_{20}F_2N_4O$ $[M+H]^+$ 419.2 found 419.2.

*Example 100. Synthesis of 1-(2-(1H-1,2,4-triazol-1-yl)nicotinoyl)-4-(4-fluorobenzyl)piperidine-4-carbonitrile (A74)*

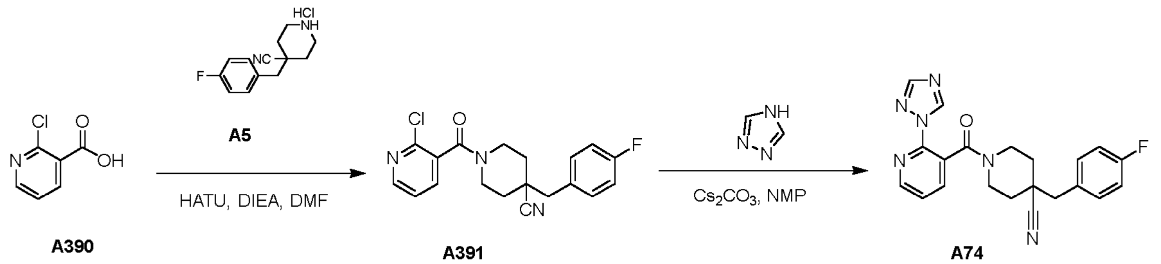


Synthesis of A391

To a mixture of 2-chloropyridine-3-carboxylic acid (500 mg, 3.17 mmol) and HATU (1.80 g, 4.75 mmol) in DMF (10 mL) was added DIPEA (2.03 g, 15.8 mmol). The mixture was stirred at 15°C for 10 min and then 4-[(4-fluorophenyl)methyl] piperidine-4-carbonitrile hydrochloride (807 mg, 3.17 mmol) was added. The mixture was stirred at 15°C for 16 hours. Water (50 mL) was added and the aqueous layer was extracted with EtOAc (2 x 50 mL). The combined organic layers were washed with brine (50 mL), dried over anhydrous $Na_2SO_4$, and concentrated. The residue was purified by flash chromatography (0~80% of EtOAc in PE) to give 1-(2-chloronicotinoyl)-4-(4-fluorobenzyl)piperidine-4-carbonitrile (800 mg). $^1H$ NMR (400MHz, $CDCl_3$) 9.30-9.29 (m, 1H),8.63-8.61 (m, 1H), 8.27-8.19 (m, 1H), 8.00-8.19 (m,1H) 7.61-7.57 (m,1H), 7.33-7.30 (m, 2H), 7.21-7.18 (m, 2H), 4.58-4.48 (m, 1H), 3.51-3.48 (m, 1H), 3.32-3.30 (m, 1H), 3.19 (s, 2H), 2.92-2.88 (m, 2H), 2.50-2.44 (m, 2H), 1.66-1.60 (m, 1H), 1.56-1.44 (m, 3H).

Synthesis of A74

To a suspension of 1-(2-chloronicotinoyl)-4-(4-fluorobenzyl)piperidine-4-carbonitrile (100 mg, 0.279 mmol) and $Cs_2CO_3$ (183 mg, 0.559 mmol) in NMP (2 mL) was added 4H-1,2,4-triazole (96.0 mg, 1.39 mmol). The mixture was stirred at 120°C for 16 hours. The reaction mixture was poured into water (30 mL) and extracted with EtOAc (2 x 50 mL). The combined organic layers were washed with brine (50 mL), dried over $Na_2SO_4$, filtered, and concentrated. The residue was purified by prep-HPLC (Column:Phenomenex Gemini-NX 150*30mm*5um; Condition:water (0.04%$NH_3H_2O$+10mM $NH_4HCO_3$)-ACN; Begin B: 30%; End 60%) to give 1-(2-(1H-1,2,4-triazol-1-yl)nicotinoyl)-4-(4-fluorobenzyl)piperidine-4-carbonitrile (22.1 mg). $^1H$ NMR (400MHz, DMSO-d6) $\delta_H$ 8.47-8.45 (m, 1H), 7.66-7.62 m(m, 1H) 7.34-7.30 (m, 1H) 7.27-7.24 (m, 2H) 7.07-7.03 (m,2H) 4.89-4.81(m,1H), 3.51-3.25 (m,2H), 3.15-2.90 (m, 2H), 2.85-2.70(m,3H), 2.08 (s, 2H), 1.80-1.75 (m, 2H), 1.65-1.55 (m,3H), 1.30-1.20 (m,3H). $^{19}F$ NMR (400 MHz, $CDCl_3$) $\delta_F$ -115.637. LCMS purity 99%, MS ESI calcd. For $C_{21}H_{19}FN_6O$ $[M+H]^+$ 391.2 found 391.2.

*Example 101. Synthesis of 1-([2,4' bipyridine]-3-carbonyl)-4-(4-phenylbutyl)piperidine-4-carbonitrile (A67)*

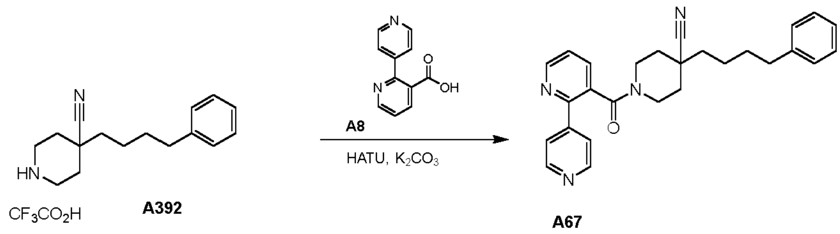


To a suspension of 4-(4-phenylbutyl)piperidine-4-carbonitrile trifluoroacetate (98 mg, 0.27 mmol) and lithio [2,4'-bipyridine]-3-carboxylate (59.5 mg,0.29 mmol) in a mixture of THF (0.3 mL) and DMF (2.7 mL), anhydrous potassium carbonate (113 mg, 0.82 mmol) was added in one portion and the mixture stirred 1 hour at room temperature. HATU (109 mg, 0.29 mmol) was added and the reaction mixture stirred 1 hour at room temperature. The mixture was diluted with EtOAc (30 mL). The suspension was filtered, and the filtrate was concentrated. The residue was purified by flash chromatography (0-50% MeOH/DCM gradient. $NH_4OH$ (10%v/v) was added to the methanol solvent) to give 1-([2,4' bipyridine]-3-carbonyl)-4-(4-phenylbutyl)piperidine-4-carbonitrile (40.9 mg, 35%). $^1H$ NMR (400 MHz, CDCl3): Mixture of rotamers; 8.79 (dd; 1 H); 8.71 (s; 2 H); 7.75 (d; 1 H); 7.71 (d; 1.4 H); 7.60 (s; 0.7 H); 7.41-7.45 (m; 1 H); 7.26-7.30 (m; 2H); 7.14-7.20 (m; 3 H); 4.71-4.78 (m; 1H); 3.19 (d; 0.3 H); 3.07 (d; 1.5 H); 2.89-2.96 (m; 0.8 H); 2.67-2.77 (m; 0.5 H); 2.59 (t; 2.2 H); 1.98 (d; 0.3 H); 1.87 (d; 0.9H); 1.48-1.70 (m; 3.6 H); 1.30-1.41 (m; 2.5 H); 1.18-1.26 (m; 1.7 H); 0.97-1.04 (m; 0.7 H); -0.20--0.12 (m; 0.5 H). LCMS (ESI) calcd. For $C_{27}H_{28}N_4O$ [M+H]+ 425.2, found 425.3.

*Example 102. Synthesis of 1-([2,4'-bipyridine]-3-carbonyl)-4-phenethylpiperidine-4-carbonitrile (A68)*

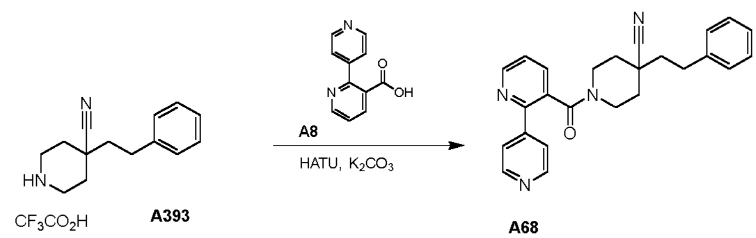


To a suspension of 4-phenethylpiperidine-4-carbonitrile trifluoroacetate (103 mg, 0.31 mmol) and lithio [2,4'-bipyridine]-3-carboxylate (67.8 mg, 0.33 mmol) in a mixture of THF (0.3 mL) and DMF (2.7 mL), anhydrous potassium carbonate (130 mg, 0.94 mmol) was added in one portion and the mixture stirred 1 hour at room temperature. HATU (125 mg, 0.33 mmol) was added and the reaction mixture stirred 1 hour at room temperature. The mixture was diluted with EtOAc (30 mL). The suspension was filtered, and the filtrate was concentrated under reduced pressure. The residue was purified by flash chromatography (0-70% MeOH/DCM gradient. $NH_4OH$ (10%v/v) was added to the methanol solvent) to give 1-([2,4'-bipyridine]-3-carbonyl)-4-phenethylpiperidine-4-carbonitrile (110 mg, 87%). $^1H$ NMR (400 MHz, CDCl3): Mixture of rotamers; 8.80 (dd; 1 H); 8.73 (s; 2 H); 7.77 (d; 1 H); 7.72 (d; 1.4 H); 7.61 (s; 0.6 H); 7.43-7.46 (m; 1 H); 7.27-7.31

(m; 2 H); 7.19-7.23 (m; 1 H); 7.12-7.14 (m; 2H); 4.75-4.82 (m; 1 H); 3.23 (d; 0.3 H); 3.11 (d; 1.5 H); 2.96 (t; 0.8 H); 2.60-2.77 (m; 2.9 H); 2.07 (d; 0.3 H); 1.94 (d; 0.8 H); 1.74-1.85 (m; 1.1 H); 1.52-1.55 (m; 1.7H); 1.43-1.47 (m; 0.8H); 1.23-1.27 (m; 0.3H); 1.04-1.11 (m; 0.7 H); -0.17--0.09 (m; 0.5 H). LCMS (ESI) calcd. For $C_{25}H_{24}N_4O$ [M+H]+ 397.2, found 397.2.

*Example 103. Synthesis of 1-([2,4'-bipyridine]-3-carbonyl)-4-(3,4-difluorobenzyl)piperidine-4-carbonitrile (A69)*

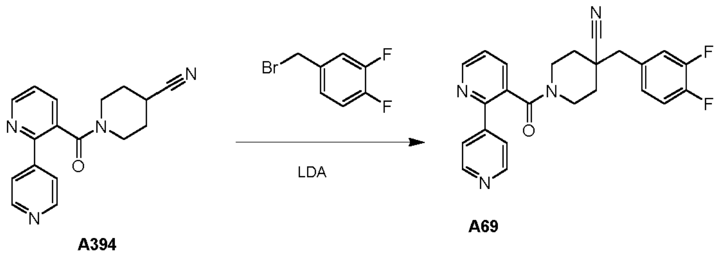


A394

A69

1-([2,4'-bipyridine]-3-carbonyl)piperidine-4-carbonitrile (130 mg, 0.44 mmol) was dissolved in dry THF (4.4 mL). The resulting solution was cooled to 0°C and LDA (2M, 0.24 mL, 0.49 mmol) was added dropwise. After stirring for 1 hour at 0 °C, 4-(bromomethyl)-1,2-difluorobenzene (54 μL, 0.42 mmol) was added and the reaction stirred overnight at room temperature. The reaction was diluted with EtOAc (25 mL), and the organic layer was washed 1N NaOH (2 x 5 mL). The combined aqueous washes were extracted with EtOAc (15 mL). The combined organic layers were washed with brine (15 mL) and dried over $MgSO_4$ and concentrated. The residue was purified by flash chromatography (0-70% MeOH/DCM gradient. $NH_4OH$ (10%v/v) was added to the methanol solvent) to give 1-([2,4'-bipyridine]-3-carbonyl)-4-(3,4-difluorobenzyl)piperidine-4-carbonitrile (60 mg, 0.14 mmol, 31%). $^1H$ NMR (400 MHz, DMSO-d6): Mixture of rotamers; 8.79 (d; 1 H); 8.69 (d; 2 H); 7.90 (s; 1 H); 7.53-7.60 (m; 3 H); 7.39-7.42 (m; 1 H); 7.21-7.30 (m; 1 H); 6.99-7.09 (m; 1 H); 4.51 (t; 1 H); 3.17-3.21 (m; 0.7 H); 2.79-3.03 (m; 2.5 H); 2.63-2.69 (m; 2.3 H); 1.84 (d; 1.1 H); 1.42-1.66 (m; 2.1 H); 1.21-1.27 (m; 0.5 H); 0.18-0.21 (m; 0.3 H). LCMS (ESI) calcd. For $C_{24}H_{20}F_2N_4O$ [M+H]+ 419.2, found 419.2.

*Example 104. Synthesis of 1-{[2,4'-bipyridine]-3-carbonyl}-4-(3-phenylpropyl)piperidine-4-carbonitrile (A71)*

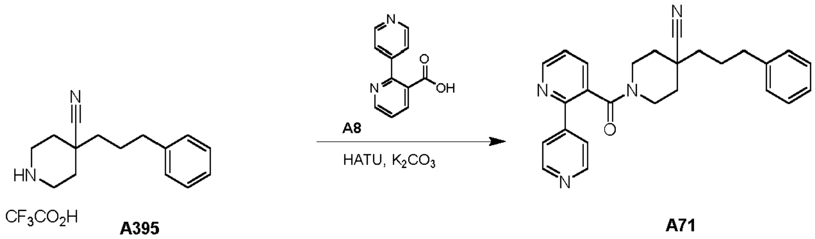


A395

A71

To a suspension of 4-(3-phenylpropyl)piperidine-4-carbonitrile; trifluoroacetic acid (2.37 g, 10.6 mmol) and lithio [2,4'-bipyridine]-3-carboxylate (120 mg, 582 μmol) in a mixture of THF (582 μL) and DMF (5.81 mL), anhydrous potassium carbonate (240 mg, 1.74 mmol) was added in one portion and the mixture stirred 1 hour at room temperature. HATU (232 mg, 611 μmol) was added and the reaction mixture stirred 2 hours at room temperature. The mixture was diluted with EtOAc (25 mL). The suspension filtered, and the filtrate was concentrated. The residue was purified by reverse phase chromatography (ISCO 2x60 g C-18 silica, solvents: solvent A: 0.1% ammonium carbonate in water, solvent B (acetonitrile), 10% MeCN (3 CV), 10% to 50 % MeCN (5 CV), 50 % MeCN (3 CV)) to give 1-{[2,4'-bipyridine]-3-carbonyl}-4-(3-phenylpropyl)piperidine-4-carbonitrile (91.2 mg, 36.7%). $^1H$ NMR (400 MHz, rotamers, $CD_3OD$): $\delta_H$ 8.79 (dd; 1.71 Hz; 1 H); 8.68 (m; 2 H); 7.92 (br s; 1 H); 7.74 (s; 1 H); 7.67 (br s; 1 H); 7.53-7.60 (m; 1 H); 7.26 (m; 2 H); 7.17 (m; 3 H); 4.62 (br s; 1.1 H); 3.20-3.1 (m; 1.2 H); 2.97 (br s; 0.6 H); 2.77 (d; 1 H); 2.63 (m; 2.2 H); 1.94 (m; 1 H); 1.78 (m; 1.1 H); 1.69 (br s; 1.9 H); 1.58 (br s; 1.8 H); 1.43 (s; 0.3 H); 1.32 (s; 0.7 H); 1.09 (s; 0.5 H); 0.07 (s; 0.5 H). LCMS (ESI) calcd. For $C_{26}H_{26}N_4O$ [M+H]+ 411.2, found 411.3

*Example 105. Synthesis of 1-{[2,4'-bipyridine]-3-carbonyl}-4-[(pyridin-3-yl)methyl]piperidine-4-carbonitrile (A72)*

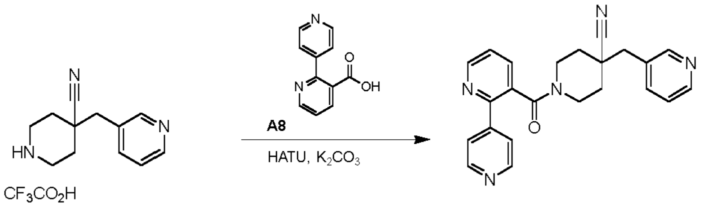


A396

A72

To a suspension of 4-[(pyridin-3-yl)methyl]piperidine-4-carbonitrile; bis(trifluoroacetic acid) (249 mg, 582 μmol) and lithio [2,4'-bipyridine]-3-carboxylate (120 mg, 582 μmol) in a mixture of THF (582 μL) and DMF (5.81 mL), anhydrous potassium carbonate (240 mg, 1.74 mmol) was added in one portion and the mixture stirred 1 hour at room temperature. HATU (221 mg, 582 μmol) was added and the reaction mixture stirred 2 hours at room temperature. The mixture was diluted with EtOAc (25 mL). The suspension was filtered, and the filtrate was concentrated. The residue was purified by reverse phase chromatography (ISCO 2x60 g C-18 silica, solvents: solvent A: 0.1% ammonium carbonate in water, solvent B (acetonitrile), 10% MeCN (3 CV), 10% to 50 % MeCN (5 CV), 50 % MeCN (3 CV)) to give 1-{[2,4'-bipyridine]-3-carbonyl}-4-[(pyridin-3-yl)methyl]piperidine-4-carbonitrile (223 mg, 27%). $^1$H-NMR (400 MHz, rotamers, $CD_3OD$): $\delta_H$ 8.76 (m; 2 H); 8.66 (s; 1 H); 8.47 (s; 1.5 H); 8.38 (s; 0.5 H); 7.97 (s; 0.5 H); 7.91 (s; 0.5 H); 7.78 (s; 2 H); 7.68 (m; 1 H); 7.60 (s; 1 H); 7.42 (dd; 1 H); 4.69 (m; 1.1 H); 3.40 (br s; 0.5 H); 3.12 (s; 0.6 H); 2.97 (m; 1.6 H); 2.75 (m; 1.5 H); 2.65 (m; 0.6 H); 1.93 (d; 1 H); 1.64 (m; 1.3 H); 1.47 (m; 0.5 H); 1.32 (m; 0.6 H); 0.10 (m; 0.5 H). LCMS (ESI) calcd. For $C_{23}H_{21}N_5O$ [M+H]+ 384.2, found 384.2.

*Example 106. Synthesis of 1-{[2,4'-bipyridine]-3-carbonyl}-4-[(pyridin-3-yl)methyl]piperidine-4-carbonitrile (A73)*

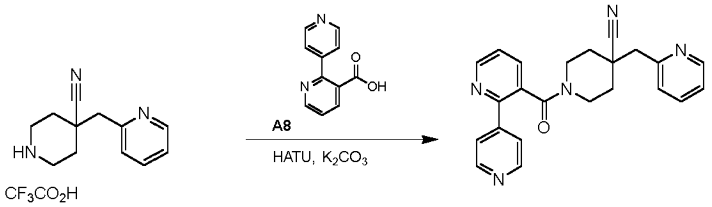


A397

A73

A73 was synthesized by established methods. LCMS (ESI) calcd. For $C_{23}H_{21}N_5O$ [M+H]+ 384.12, found 384.2 .

*Example 107. Synthesis of lithium [2,4'-bipyridine]-3-carboxylate (A8)*

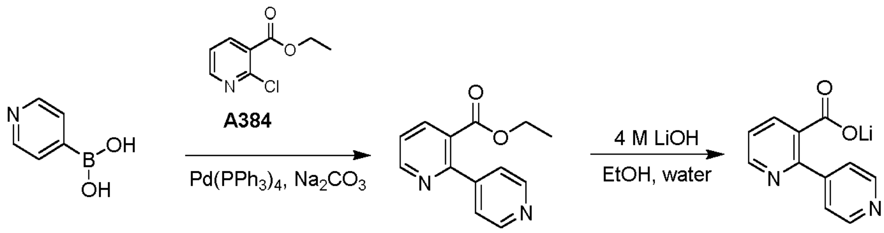


A398

A399

A8

Synthesis of A399

To the mixture of pyridin-4-ylboronic acid (5 g, 0.0406 mmol), ethyl 2-chloropyridine-3-carboxylateethyl (15 g, 81.2 mmol) and $Na_2CO_3$ (12.8 g, 121 mmol) in THF (20 mL) under nitrogen was added Pd($PPh_3$)$_4$ (21.4 mg, 0.0186 mmol). The reaction mixture was stirred at 130°C for 15 hours, cooled, and made acidic (pH=2) with HCl solution (100 mL, 2 M). The mixture was washed with EtOAc (2 x 100 mL). The pH of the aqueous phase was adjusted to pH=7 and extracted with EtOAc (2 x 200 mL). The combined organic layers were dried over anhydrous $Na_2SO_4$, filtered, and concentrated to give

ethyl [2,4'-bipyridine]-3-carboxylate (7 g, 75.6%). $^1$H NMR (400 MHz, $CDCl_3$) $\delta_H$ 8.85-8.79 (m, 1H), 8.72-8.66 (m, 2H), 8.21 (d, 1H), 7.49-7.42 (m, 3H), 4.25-4.15 (m, 2H), 1.12-1.07 (m, 3H).

Synthesis of A8

To a solution of ethyl [2,4'-bipyridine]-3-carboxylate (1 g, 4.38 mmol) in THF (10 mL), MeOH (5 mL) and water (1 mL) was added $LiOH{\cdot}H_2O$ (275 mg, 8.57 mmol) at 25°C. The reaction mixture was stirred at 50°C for 3 hours and concentrated to give lithium [2,4'-bipyridine]-3-carboxylate (1.1g). $^1$H NMR (400 MHz, DMSO-$d_6$) $\delta_H$ 8.57-8.51 (m, 2H), 8.50-8.45 (m,1H), 7.82-7.76 (m, 2H), 7.69-7.61 (m, 1H), 7.32-7.22 (m ,1H).

*Example 108. Synthesis of 1-([2,4'-bipyridine]-3-carbonyl)-4-(4-fluoro-2-(trifluoromethyl)benzyl)piperidine-4-carbonitrile (A277)*

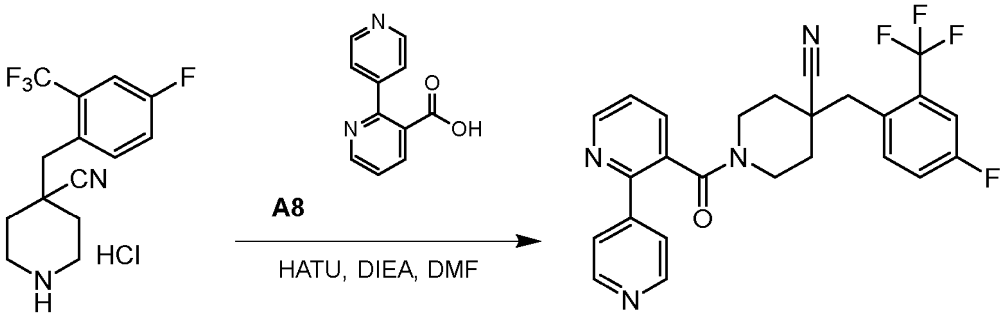


A360 A277

A solution of [2,4'-bipyridine]-3-carboxylic acid (104 mg, 0.523 mmol), HATU (297 mg, 0.784 mmol) in DMF (2 mL) was added DIPEA (1 mL) and 4-(4-fluoro-2-(trifluoromethyl)benzyl)piperidine-4-carbonitrile hydrochloride (150 mg, 0.523 mmol). The mixture was stirred at 20°C for 2 hours. The mixture solution was concentrated, and the residue was purified by prep-HPLC (Phenomenex Gemini-NX 80*30mm*3um, water (10mM $NH_4HCO_3$)-ACN Begin B 35 End B 65 ) to afford 1-([2,4'-bipyridine]-3-carbonyl)-4-(4-fluoro-2-(trifluoromethyl)benzyl)piperidine-4-carbonitrile (68.3 mg, 27.8%). $^1$H NMR (400 MHz, $CDCl_3$) $\delta_H$ 8.68-8.85 (m, 3 H), 7.72-7.81 (m, 2 H), 7.49-7.71 (m, 2 H), 7.44 (m, 1 H), 7.38 (m, 1 H), 7.21-7.26 (m, 1 H), 4.82 (d, 1 H), 3.01-3.21 (m, 2 H), 2.93 (t, 0.7 H), 2.59-2.84 (m, 1.8 H), 1.19-1.94 (m, 5 H), 0.08 (d). $^{19}$F NMR (376.5 MHz, $CDCl_3$) $\delta_F$ -57.389,-57.472, - 111.835. LCMS purity 95%, MS ESI calcd. For $C_{25}H_{20}F_4N_4O$ $[M+H]^+$ 469.1 found 469.2.

*Reference Example 109. Synthesis of 4-phenyl-1-(2-(pyrimidin-4-yl)nicotinoyl)piperidine-4-carbonitrile (A256)*

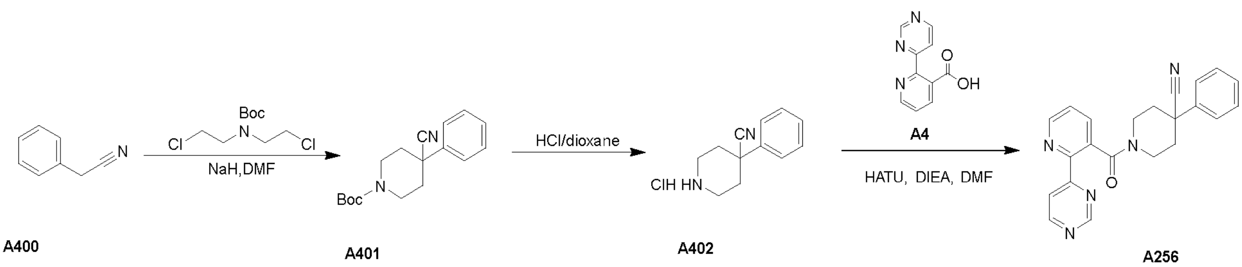


A400 A401 A402 A256

Synthesis of A401

To a mixture of NaH (102 mg, 2.56 mmol, 60% in oil) in DMF (5 mL) was added 2-phenylacetonitrile (150 mg, 1.28 mmol) in DMF (2 mL) slowly at 0°C. After stirring for 1 hour, tert-butyl N,N-bis(2-chloroethyl)carbamate (309 mg, 1.28 mmol) in DMF (3 mL) was added slowly. The mixture was stirred at 75°C for 5 hours. The reaction was cooled, and brine (20 mL) was added slowly. The mixture was extracted with EtOAc (2 x 20 mL). The combined organic layers were washed with brine (20 mL), dried over anhydrous $Na_2SO_4$, filtered, and concentrated. The residue was purified by silica gel

chromatography (PE/EtOAc =5/1 to 3/1) to give tert-butyl 4-cyano-4-phenylpiperidine-1-carboxylate (200 mg, 54.6 %). $^1H$ NMR (400MHz, $CDCl_3$) $\delta_H$ 7.50-7.45 (m, 2H), 7.44-7.39 (m, 2H), 7.38-7.32 (m, 1H), 4.41-4.13 (m, 2H), 3.33-3.08 (m, 2H), 2.17-2.04 (m, 2H), 2.01-1.87 (m, 2H), 1.49 (s, 9H).

Synthesis of A402

To a solution of tert-butyl 4-cyano-4-phenylpiperidine-1-carboxylate (200 mg, 0.70 mmol) was added HCl/dioxane (10 mL, 4M, 40.0 mmol) at 25°C. The mixture was stirred at 15°C for 16 hours under nitrogen. The mixture was concentrated to give 4-phenylpiperidine-4-carbonitrile hydrochloride (180 mg).

Synthesis of A256

A mixture of 2-(pyrimidin-4-yl)nicotinic acid (116 mg, 0.5798 mmol), HATU (367 mg, 0.97 mmol), DIPEA (0.419 mL, 2.41 mmol) and 4-phenylpiperidine-4-carbonitrile hydrochloride (90 mg, 0.4832 mmol) in DMF (2 mL) was stirred at 15°C for 16 hours. The mixture was filtered and concentrated. The residue was purified by Prep-HPLC (Column: Phenomenex Gemini-NX 80*30 mm*3 um; Condition: water (10mM $NH_4HCO_3$)-ACN; Begin B: 25; End B: 55; Gradient Time (min):9; 100%B Hold Time (min): 2) to afford 4-phenyl-1-(2-(pyrimidin-4-yl)nicotinoyl)piperidine-4-carbonitrile (118.6 mg, 66.2%). $^1H$ NMR (400MHz, $CDCl_3$) $\delta_H$ 9.29 (s, 0.3H), 9.13 (s, 0.7H), 8.96-8.86 (m, 1H), 8.83-8.74 (m, 1H), 8.40-8.16 (m, 1H), 7.78-7.69 (m, 1H), 7.60-7.33 (m, 6H), 5.16-4.80 (m, 1H), 3.86-3.19 (m, 3H), 2.50-1.85 (m, 4H). LCMS purity 99%, MS ESI calcd. For $C_{22}H_{20}N_5O$ $[M+H]^+$ 370. found 370.

*Example 110. Synthesis of 1-(5-fluoro-2-(pyrimidin-4-yl)benzoyl)-4-(4-fluorobenzyl)piperidine-4-carbonitrile (A219)*

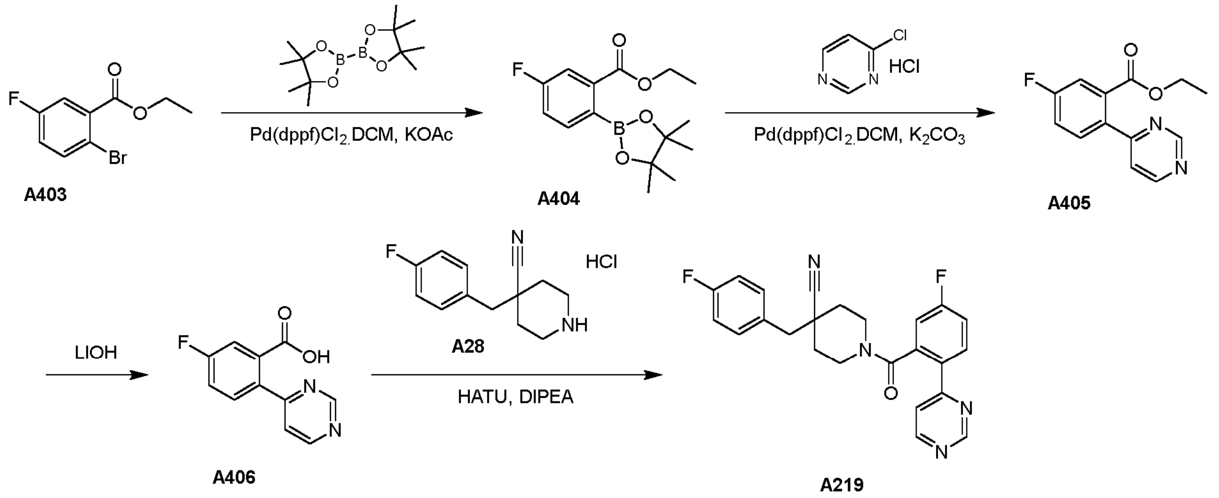


Synthesis of A404

A mixture of ethyl 2-bromo-5-fluorobenzoate (1 g, 4.04 mmol), 4,4,5,5-tetramethyl-2-(4,4,5,5-tetramethyl-1,3,2-dioxaborolan-2-yl)-1,3,2-dioxaborolane (1.22 g, 4.84 mmol), Pd(dppf)$Cl_2$ (164 mg, 0.2020 mmol) and AcOK (399 mg, 4.04 mmol) in dioxane (15 mL) was stirred at 100°C for 16 hours under nitrogen. The reaction mixture poured into water (50 mL), the aqueous layer was extracted with EtOAc (2 x 20 mL). The combined organic layers were washed with brine (30 mL), dried over anhydrous $Na_2SO_4$, and concentrated. The residue was purified by flash chromatography (0~10% of EA in PE) to give ethyl 5-fluoro-2-(4,4,5,5-tetramethyl-1,3,2-dioxaborolan-2-yl)benzoate (600 mg, 50.8% yield). $^1H$ NMR (400 MHz, $CDCl_3$) $\delta_H$ 7.65-7.55 (m, 1H), 7.53-7.43 (m, 1H), 7.23-7.12 (m, 1H), 4.45-4.43 (m, 2H), 1.50-1.25 (m, 12H), 1.28-1.18 (m, 3H).

Synthesis of A405

A mixture of ethyl 5-fluoro-2-(4,4,5,5-tetramethyl-1,3,2-dioxaborolan-2-yl)benzoate (400 mg, 1.35 mmol), 4-chloropyrimidine hydrochloride (182 mg, 1.21 mmol), Pd(dppf)$Cl_2$ (99.6mg, 0.135 mmol) and $K_2CO_3$ (932mg, 6.75 mmol) in dioxane (15 mL)/$H_2O$ (2 mL) was stirred at 100°C for 3 hours under nitrogen. The reaction mixture was cooled, poured

into water (50 mL), and the aqueous layer was extracted with EtOAc (2 x 50 mL). The combined organic layers were washed with brine (100 mL), dried over anhydrous $Na_2SO_4$, and concentrated. The residue was purified by flash chromatography (0~30% of EA in PE) to give ethyl 5-fluoro-2-(pyrimidin-4-yl)benzoate (400 mg). $^1H$ NMR (400 MHz, $CDCl_3$) $\delta_H$ 9.23 (s, 1H), 8.80-8.71 (m, 1H), 7.65-7.55 (m, 2H), 7.49-7.41 (m, 1H), 7.30-7.27 (m, 1H), 4.25-4.10 (m, 2H), 1.27-1.17 (m, 3H).

Synthesis of A406

To a mixture of ethyl 5-fluoro-2-(pyrimidin-4-yl)benzoate (400 mg, 1.62 mmol) in MeOH (10 mL)/water (1 mL) was added $LiOH{\cdot}H_2O$ (74.7 mg, 1.78 mmol), the mixture was stirred at 60°C for 16 hours. The reaction mixture was concentrated to give 5-fluoro-2-(pyrimidin-4-yl)benzoic acid (300 mg). $^1H$ NMR (400 MHz, $CD_3OD$) $\delta_H$ 9.23 (s, 1H), 8.80-8.71 (m, 1H), 7.65-7.55 (m, 2H), 7.49-7.41 (m, 1H), 7.30-7.27 (m, 1H).

Synthesis of A219

To a mixture of 5-fluoro-2-(pyrimidin-4-yl)benzoic acid (150 mg, 0.6874 mmol) , 4-[(4-fluorophenyl)methyl] piperidine-4-carbonitrile hydrochloride (157 mg, 0.6186 mmol) and HATU (391 mg, 1.03 mmol) in DMF (5 mL) was added DIPEA (442 mg, 3.43 mmol) . The mixture was stirred at 20°C for 2 hours. The reaction mixture was poured into water (50 mL), and the aqueous layer was extracted with EtOAc (2 x 100 mL). The combined organic layers were washed with brine (100 mL), dried over anhydrous $Na_2SO_4$, and concentrated. The residue was purified by prep-HPLC (column : Phenomenex Gemini-NX 150*30mm*5um; Condition:water (0.04%NH3H2O+10mM NH4HCO3)-ACN; Begin B: 43%; End 73%) to give 1-[5-fluoro-2-(pyrimidin-4-yl)benzoyl]-4-[(4-fluorophenyl)methyl]piperidine-4-carbonitrile (54.7 mg, 18.8% yield). $^1H$ NMR (400 MHz, $CDCl_3$) $\delta_H$ 9.21 (s, 0.33H), 8.97 (s, 0.52H), 8.96-8.86 (m, 1H), 7.80-7.51 (m, 2H), 7.26-7.16 (m, 3H), 7.13-7.58 (m, 3H), 4.81-4.65 (m, 1H), 3.65-3.55 (m, 0.61H), 3.50-3.40 (m, 0.39H), 3.36-3.21 (m, 0.60H), 3.11-2.99 (m, 1H), 2.08-1.98 (m, 1H), 1.78-1.68 (m, 0.4H), 1.65-1.60 (m, 0.60H), 1.55-1.45 (m, 2H). $^{19}F$ NMR (376.5 MHz, $CDCl_3$) $\delta_F$ -108.527, $\delta_F$ -108.744 & $\delta_F$ -114.425, $\delta_F$ -114.515. LC-ELSD/MS purity 99%, MS ESI calcd. for $C_{24}H_{21}F_2N_4O$ $[M+H]^+$ 419.3, found 419.3.

*Example 111. Synthesis of (S)-3-(4-fluorobenzyl)-1-(2-(pyrimidin-4-yl)nicotinoyl)pyrrolidine-3-carbonitrile (A236) and (R)-3-(4-fluorobenzyl)-1-(2-(pyrimidin-4-yl)nicotinoyl)pyrrolidine-3-carbonitrile (A235)*

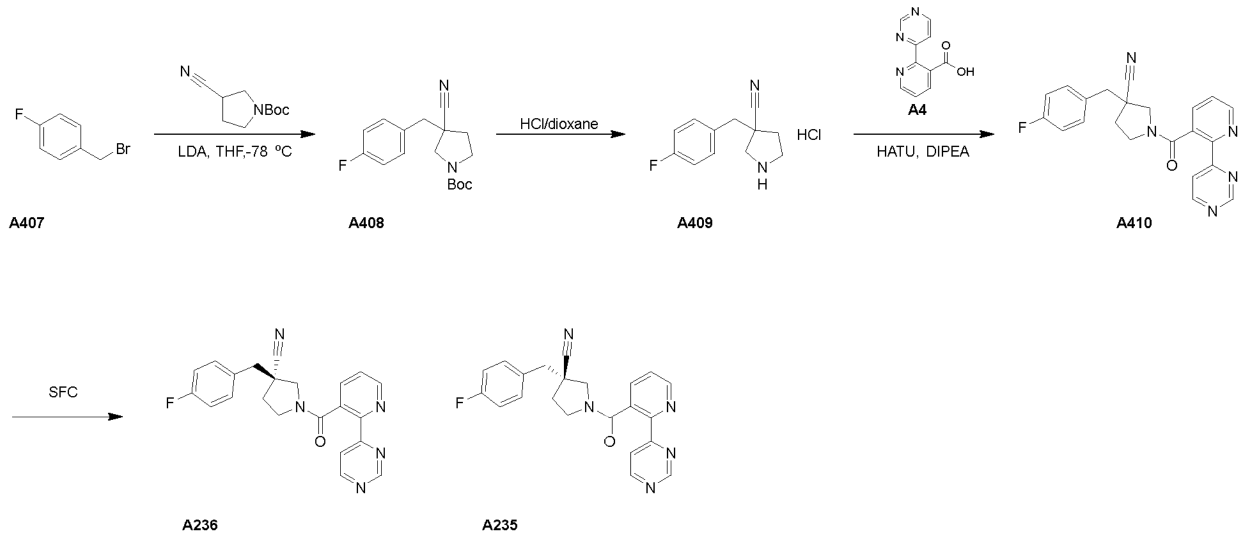


Synthesis of A408

To a mixture of tert-butyl 3-cyanopyrrolidine-1-carboxylate (1.03 g, 5.29 mmol) in THF (10 mL) was added dropwise LDA (3.96 mL, 2 M,7.93 mmol) at -78°C. After stirring at -78°C for 1 hour, 1-(bromomethyl)-4-fluorobenzene (307 mg, 1.57 mmol) was added. The mixture was warmed to 20°C and stirred for 16 hours. The mixture was poured into water (25 mL) and extracted with EtOAc (3 x 20 mL). The combined organic layers were washed with brine (2 x 50 mL), dried over

$Na_2SO_4$, filtered. and concentrated. The residue was purified by flash chromatography (10~30% of EtOAc in PE) to give tert-butyl 3-cyano-3-(4-fluorobenzyl)pyrrolidine-1-carboxylate (570 mg, 35.4%). $^1$H NMR (400 MHz, $CDCl_3$) $\delta_H$ 7.34-7.28 (m, 2H), 7.08 (s, 2H), 3.83-3.52 (m, 3H), 3.42 (m, 1H), 2.95 (s, 2H), 2.27 (m, 1H), 2.11-1.97 (m, 1H), 1.49 (s, 9H).

Synthesis of A409

To a mixture of tert-butyl 3-cyano-3-(4-fluorobenzyl)pyrrolidine-1-carboxylate (100 mg, 0.279 mmol) in dioxane (5 mL) was added HCl/dioxane (5.0 mL, 4M in dioxane, 20 mmol). After stirring at 25°C for 4 hours, the mixture concentrated to give 3-(4-fluorobenzyl)pyrrolidine-3-carbonitrile hydrochloride (66 mg), which was used directly in the next reaction.

Synthesis of A410 and SFC Separation

To a mixture of 2-(pyrimidin-4-yl)pyridine-3-carboxylic acid (374 mg, 1.86 mmol), 3-(4-fluorobenzyl)pyrrolidine-3-carbonitrile hydrochloride (450 mg, 1.86 mmol) and HATU (1.06 g, 2.79 mmol) in DMF (15 mL) was added DIPEA (1.19 g, 9.30 mmol) . After stirring at 20°C for 12 hours. The reaction mixture poured into water (50 mL) and extracted with EtOAc (3 x 20 mL). The combined organic layers were washed with brine (2 x 20 mL), dried over anhydrous $Na_2SO_4$, filtered and concentrated. The residue was purified by HPLC (Column Xtimate C18 100*30mm*3um Condition water(0.225%FA)-ACN Begin B 25 End B 55 Gradient Time(min) 8 100%B Hold Time(min) 2.5 FlowRate(ml/min) 20 Injections 6) to afford racemic-3-(4-fluorobenzyl)-1-(2-(pyrimidin-4-yl)nicotinoyl)pyrrolidine-3-carbonitrile (270 mg, 37.5%). $^1$H NMR (400 MHz, $CDCl_3$) $\delta_H$ 7.34-7.28 (m, 2H), 7.08 (s, 2H), 3.83-3.52 (m, 3H), 3.42 (m, 1H), 2.95 (s, 2H), 2.27 (m, 1H), 2.11-1.97 (m, 1H), 1.49 (s, 9H).

Racemic-3-(4-fluorobenzyl)-1-(2-(pyrimidin-4-yl)nicotinoyl)pyrrolidine-3-carbonitrile (270 mg, 0.697 mmol) was purified by SFC (Column: Chiralpak AD-3, 50 x 4.6mm I.D., 3um Mobile phase: A: $CO_2$ B:ethanol (0.05% DEA) Gradient: from 5% to 40% of B in 2 min and hold 40% for 1.2 min, then 5% of B for 0.8 min Flow rate: 4mL/min Column temp.: 35°C ABPR: 1500psi) to give (R)-3-(4-fluorobenzyl)-1-(2-(pyrimidin-4-yl)nicotinoyl)pyrrolidine-3-carbonitrile (100 mg, 37.1%) as a solid and (S)-3-(4-fluorobenzyl)-1-(2-(pyrimidin-4-yl)nicotinoyl)pyrrolidine-3-carbonitrile (80 mg, 29.7%).

(R)-3-(4-fluorobenzyl)-1-(2-(pyrimidin-4-yl)nicotinoyl)pyrrolidine-3-carbonitrile (100 mg, 0.2581 mmol) was further purified by SFC (Column: Chiralpak AD-3, 50 x 4.6mm I.D., 3um Mobile phase: A: $CO_2$ B:ethanol (0.05% DEA) Gradient: from 5% to 40% of B in 2 min and hold 40% for 1.2 min, then 5% of B for 0.8 min Flow rate: 4mL/min Column temp.: 35°C ABPR: 1500psi) to give (R)-3-(4-fluorobenzyl)-1-(2-(pyrimidin-4-yl)nicotinoyl)pyrrolidine-3-carbonitrile (63.4 mg, 63%). $^1$H NMR (400 MHz, $CDCl_3$) $\delta_H$ 9.21 (d, 1H), 8.94-8.86 (m, 1H), 8.83-8.75 (m, 1H), 8.30-8.22 (m, 1H), 7.79-7.70 (m, 1H), 7.49 (m, 1H), 7.33 (m, 1H), 7.20 (m, 1H), 7.13-6.96 (m, 2H), 4.20-3.78 (m, 2H), 3.71-3.40 (m, 2H), 3.17-3.06 (m, 1H), 2.95 (s, 1H), 2.47-2.06 (m, 2H). $^{19}$F NMR (376.5 MHz, $CDCl_3$) $\delta_F$ -114.055, -114.119.

LC-ELSD/MS purity 98%, de 99%, MS ESI calcd. for $C_{22}H_{18}FN_5O$ [M+H] 388.2 found 388.2.

(S)-3-(4-fluorobenzyl)-1-(2-(pyrimidin-4-yl)nicotinoyl)pyrrolidine-3-carbonitrile (80 mg, 0.206 mmol) was further purified by SFC (Column: Chiralpak AD-3, 50 x 4.6mm I.D., 3um Mobile phase: A: $CO_2$ B:ethanol (0.05% DEA) Gradient: from 5% to 40% of B in 2 min and hold 40% for 1.2 min, then 5% of B for 0.8 min Flow rate: 4mL/min Column temp.: 35°C ABPR: 1500psi) to give (S)-3-(4-fluorobenzyl)-1-(2-(pyrimidin-4-yl)nicotinoyl)pyrrolidine-3-carbonitrile (55.8 mg, 55.8%). $^1$H NMR (400 MHz, $CDCl_3$) $\delta_H$ 9.21 (m, 1H), 8.93-8.87 (m, 1H), 8.79 (m, 1H), 8.31-8.22 (m, 1H), 7.81-7.71 (m, 1H), 7.51-7.44 (m, 1H), 7.33 (m, 1H), 7.20 (m, 1H), 7.13-6.97 (m, 2H), 4.21-3.79 (m, 2H), 3.70-3.32 (m, 2H), 3.16-3.08 (m, 1H), 2.95 (s, 1H), 2.48-2.29 (m, 1H), 2.22-2.05 (m, 1H), 1.26 (s, 2H). $^{19}$F NMR (376.5 MHz, $CDCl_3$) $\delta_F$ -114.055, -114.119. LCMS purity 99%, de 99%, MS ESI calcd. for $C_{22}H_{18}FN_5O$ [M+H] 388.2 found 388.2.

*Example 112. Exemplary CYP46A1 enzyme assay 1*

CYP46A1 inhibition of 4-(4-Fluorobenzyl)-1-(2-(pyrimidin-4-yl)nicotinoyl)piperidine-4-carbonitrile (A6) was measured and a dose response curve was generated. See **FIG. 1.**

Briefly, in 96-well plate format, 88.5 μL of CYP46A1R (50 pM/mL final concentration) in potassium phosphate buffer with $MgCl_2$ and 1 μL of varying half-log concentrations of inhibitor were added to designated wells and incubated at 37 °C for 2 min. The substrate testosterone (0.5 μL of a 3000 μM solution) and 10 μL of the NADPH generating system were added to all wells and incubated for 10 minutes at 37 °C. The reaction was stopped by adding 100 μL of methanol containing 1 μM of the internal standards bucetin and diclofenac. The plates were sealed and placed in a -20 °C freezer for 10 minutes, centrifuged at 5700 rpm for 20 min at 4 °C and the supernatants transferred for LC-MS/MS analysis.

The NADPH generating system was prepared by combining 1500 μL of 200 mM glucose-6-phospate, 3000 μL of 20 mM NADH, 60 μL of 1000 U/mL glucose-6-phosphate dehydrogenase and 1440 μL of 100 mM potassium phosphate at pH 7.4.

A standard curve of 16β-hydroxytestosterone was prepared in 96-well plate format by combining 88.5 μL of

CYP46A1R (50 pM/mL final concentration) in potassium phosphate buffer with $MgCl_2$, 0.5 μL of a 3000 μM solution of testosterone, 0.5 μL of varying concentrations of 16β-hydroxytestosterone and incubated for 10 minutes at 37 °C. Then 10 μL of the NADPH generating system was added to all wells immediately followed by adding 100 μL of methanol containing 1 μM of the internal standards bucetin and diclofenac. The plates were sealed and placed in a -20 °C freezer for 10 minutes, centrifuged at 5700 rpm for 20 min at 4 °C and the supernatants transferred for LC-MS/MS analysis.

For LC-MS Analysis, samples were injected onto a Waters Acquity UPLC with an Acquity UPLC HSS T3 1.8 μm, 2.1x50 mm column and triple quadrupole Xevo TQ MS detector using a gradient elution using mobile phase A of water with 0.1% formic acid and mobile phase B of acetonitrile with 0.1% formic acid. The gradient was maintained at 2% B for 0.05 min, then ramped linearly to 95% B in 0.95 min, then immediately returned linearly to 2% B in 0.50 min with a total runtime of 1.8 min. 16β-hydroxytestosterone was monitored (parent m/z 305.1844 and daughter m/z 96.9690) and quantified. Dose response curves were generated by graphing 16β-hydroxytestosterone levels versus inhibitor concentrations.

*Example 113. Exemplary CYP46A1 enzyme assay 2*

Briefly, in a 384-well plate format, 10 μL per well of an enzyme-substrate mixture of CYP46A1 (5 μM final concentration) and Testosterone (10 mM final concentration) were dispensed to wells containing test compound. For CYP46A1 titration, 5 μL per well of serially diluted CYP46A1 (concentrations including 10 μM, 5 μM, and 2.5 μM) and Testosterone (concentration of 10 mM) were dispensed. For Testosterone titration, 5 μL per well of serially diluted Testosterone (concentrations including 10 μM, 5 μM, and 2.5 μM) and CYP46A1 (concentration of 5 μM) were dispensed. The plates were centrifuged at 1000 rpm for 30 seconds, and then sealed and incubated at 37 °C for 30 minutes. The plate was removed from the incubator, and then 10 μL per well of NADPH-generating system were dispensed to initiate the reaction. The plates were incubated at 37 °C for 15 minutes, added with 80 μL per well of 100% methanol consisting 1ng/ml diclofenac as internal standard, and then transferred for HPLC-MS.

$IC_{50}$ values for exemplaray compounds and reference example A256 were also obtained with the assay described above, as shown in **Table 3.**

*Example 114. Compound stability in cryopreserved human hepatocytes*

Pooled cryopreserved human hepatocytes from 10 donors were obtained from BioreclamationIVT (Cat No. X008001, Lot MMN). Test compounds (20 μL of 1 mM stock solution in DMSO) and control compounds 7-ethoxycoumarin and 7-hydroxycoumarin (20 μL of 3 mM stock solutions in DMSO) were diluted with 380 μL of 45% $MeOH/H_2O$ to obtain 50 μM solutions of test compound and 150 μM solutions of control compounds. Then 50 μL of these respective solutions were mixed with 450 μL of pre-warmed Williams' Medium E to obtain 5 μM test compound and 15 μM control compound solutions; and in duplicate 10 μL of these solutions were added to appropriate wells in a 96-well plate corresponding to incubation times T0, T15, T30, T60 and T90 and kept at 37 °C. Cryopreserved human hepatocytes were thawed, isolated and suspended in Williams' Medium E, then diluted with pre-warmed William' Medium E to a concentration of 0.625 X 10^6 cells/mL. The reaction was started by aliquoting 40 μL of the hepatocytes into the 96-well plates containing 10 μL of the test and control compounds and incubated at 37 °C with 5% $CO_2$. Stop solution (acetonitrile containing 200 ng/mL tolbutamide and labetalol as internal standards) was added at the requisite time (15, 30, 60, or 90 minutes). Hepatocytes were not added to the T0 samples, which were rather directly treated with stop solution. Medium control (MC) sample plates (T0-MC and T90-MC) were incubated in identical fashion as for the test samples, but without addition of hepatocytes and stop solution was added at the requisite times. The plates were vortexed immediately after addition of stop solution and placed on a plate shaker at 500 rpm for 10 min, then centrifuged at 3220 x g for 20 min. The supernatants were transferred to 96-deep-well plates, sealed and stored at 4 °C until LC-MS-MS analysis. The concentration of compound remaining was determined by LC-MS-MS and plotted versus time. The equation of first order kinetics was used to calculate $T_{1/2}$ and $Cl_{int}$, thus $C_t = C_0 \cdot e^{-kt}$ when $C_t = 1/2C_0$, $t_{1/2} = \ln2/k = 0.693/k$. $Cl_{int\ (hep)}$ = k/million cells per mL; $Cl_{int(liver)} = Cl_{int(hep)}$*liver weight (g/kg bodyweight)*hepatocellularity. References: (1) Sohlenius-Sternbeck, Anna-Karin. Determination of the hepatocellularity number for human, dog, rabbit, rat and mouse livers from protein concentration measurements. Toxicology in Vitro, 2006, 20, 1582-1586. (2) Davies, B. and Morris, T., Physiological Parameters in Laboratory Animals and Human. Pharmaceutical Research, 1993, 10, 1093-1095. (3) Obach R S, Baxter J G, Liston T E, et al. The prediction of human pharmacokinetic parameters from preclinical and in vitro metabolism data. Journal of Pharmacology and Experimental Therapeutics, 1997, 283(1): 46-58.

Cutoff values determined by the hepatocyte stability assay are in **Table 2** below:

**Table 2.** Cutoff values in hepatocyte stability assay.

| **Cell Conc. (×10$^6$ cells/mL )** | **$T_{max}$ (min )** | **Remaining (%) at the maximum incubation time (CV=25%)** | **$T_{1/2}$ Cut Off (min ) (>) *** | **k** | ***In vitro* CLint (µL/min/10$^6$cells) (<)** | ***In vivo* CLint (mL/min/kg) (<)** | | |
|---|---|---|---|---|---|---|---|---|
| | | | | | | **Mouse** | **Human** | |
| 0.5 | 90 | 75% | 216.8 | 0.003196 | 6.4 | 75.9 | 17.8 | |

* Based on the first-order reaction, $T_{1/2}$ cut-off = ln2 /((1/Tmax) ln(1/remaining)). $T_{1/2}$ cut-off is a maximum measurable value at the maximum incubation time (Tmax) at which the reaction remaining is within an acceptable experimental variation.

*Example 115. Compound permeability and efflux across a MDCK-MDR1 cellular membrane*

MDR1-MDCKII cells (obtained from Piet Borst at the Netherlands Cancer Institute) were seeded onto polyethylene membranes (PET) in 96-well BD insert systems at 2.5 x 10$^5$ cells/ mL and grown for 4-7 days until confluent cell monolayer formation.

Transport buffer for test compound was HBSS with 10 mM Hepes (pH 7.4±0.05) with or without 1% BSA and with or without GF120918. Transport buffer for reference compound was HBSS with 10 mM Hepes (pH 7.4±0.05) with or without GF120918. Test and reference compounds (digoxin, nadolol and metoprolol) were diluted with transport buffer from stock solution to a concentration of 2 µM (DMSO<1%) and applied to the apical or basolateral side of the cell monolayer. Permeation of the test compounds from A to B direction or B to A direction was determined in triplicate with and without P-gp inhibitor (GF120918, 10 µM). Digoxin was tested at 10 µM in the presence or absence of 10 µM GF120918 bidirectionally as well, while nadolol and metoprolol were tested at 2 µM in the absence of GF120918 in A to B direction in duplicate. The plate was incubated for 2.5 h in a $CO_2$ incubator at 37±1 °C, with 5% $CO_2$ at saturated humidity without shaking. Test and reference compounds were quantified by LC/MS/MS analysis based on the peak area ratio of analyte/IS.

Six wells per 96-well plate were selected for the lucifer yellow rejection assay to determine the cell monolayer integrity. Thus, 75 µL of 100 µM lucifer yellow in transport buffer and 250 µL transport buffer were added to apical and basolateral chambers, respectively. The plate was incubated for 150 min at 37 °C with 5% $CO_2$ and 95% relative humidity without shaking. After 150 minutes incubation, 20 µL of lucifer yellow samples were taken from the apical sides, followed by the addition of 60 µL of Transport Buffer, and then 80 µL of lucifer yellow samples were taken from the basolateral sides. The relative fluorescence unit (RFU) of lucifer yellow was measured at 425/528 nm (excitation/emission) with a Envision plate reader.

The apparent permeability coefficient $P_{app}$ (cm/s) was calculated using the equation:

$$P_{app} = (dC_r/dt) \times V_r / (A \times C_0)$$

wherein $dC_r/dt$ is the cumulative concentration of compound in the receiver chamber as a function of time (µM/s); $V_r$ is the solution volume in the receiver chamber (0.075 mL on the apical side, 0.25 mL on the basolateral side); A is the surface area for the transport, i.e. 0.0804 cm$^2$ for the area of the monolayer; $C_0$ is the initial concentration in the donor chamber (µM).

The efflux ratio was calculated using the equation:

$$\text{Efflux Ratio} = P_{app}\,(BA) / P_{app}\,(AB)$$

Percent recovery was calculated using the equation:

$$\%\ \text{Recovery} = 100 \times [(V_r \times C_r) + (V_d \times C_d)] / (V_d \times C_0)$$

$$\%\text{Total recovery} = 100 \times [(V_r \times C_r) + (V_d \times C_d) + (V_c \times C_c)] / (V_d \times C_0)$$

wherin Vd is the volume in the donor chambers (0.075 mL on the apical side, 0.25 mL on the basolateral side); Cd and Cr are the final concentrations of transport compound in donor and receiver chambers, respectively. Cc is the compound concentration in the cell lysate solution (µM). Vc is the volume of insert well (0.075 mL in this assay).

Percent of lucifer yellow in basolateral well was calculated using the equation:

$$\%LuciferYellow = \frac{V_{Basolateral} \times RFU_{Basolateral}}{V_{Apical} \times RFU_{Apical} + V_{Basolateral} \times RFU_{Basolateral}} \times 100$$

wherein RFUApical and RFUBasolateral are the relative fluorescence unit values of lucifer yellow in the apical and basolateral wells, respectively; VApical and VBasolateral are the volume of apical and basolateral wells (0.075 mL and 0.25 mL), respectively. The %Lucifer Yellow should be less than 2.

In **Table 3** below, A indicates a CYP46A1 $IC_{50}$ < 0.1 μM, B indicates a CYP46A1 $IC_{50}$ (μM) of 0.1 μM to < 1.0 μM, and C indicates a CYP46A1 $IC_{50}$ (μM) of ≥ 1.0 μM.

**Table 3.** CYP46A1 inhibitory activity data for exemplary compounds and reference example A256.

| Compound No. | Structure | $IC_{50}$ |
|---|---|---|
| A6 | 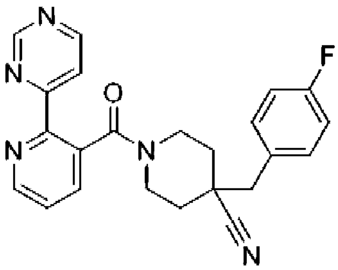 | A |
| A10 | 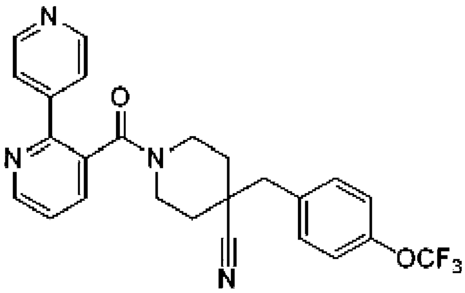 | A |
| A11 | 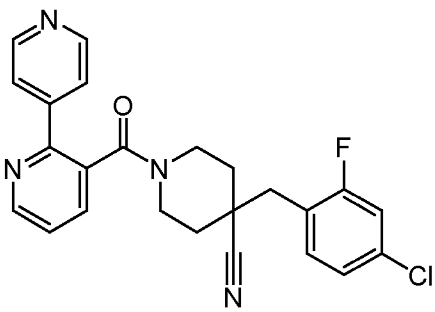 | A |
| A12 | 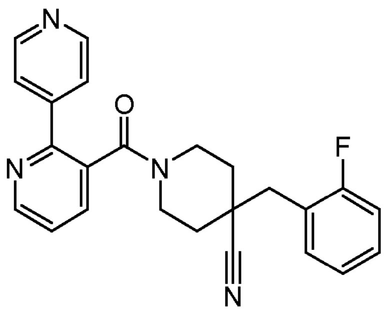 | A |

(continued)

| Compound No. | Structure | IC50 |
|---|---|---|
| A13 | 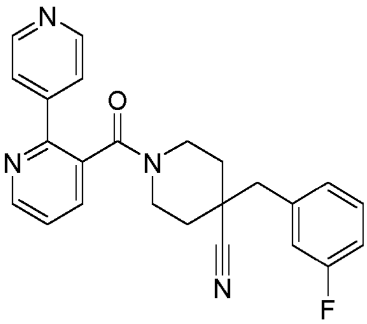 | A |
| A14 | 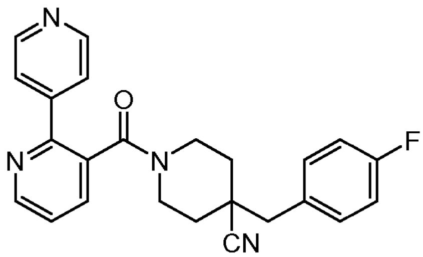 | A |
| A16 | 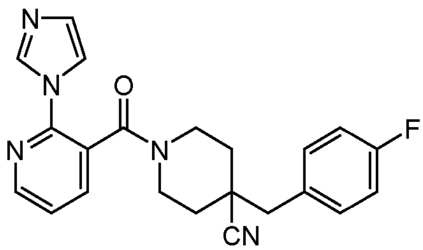 | C |
| A18 | 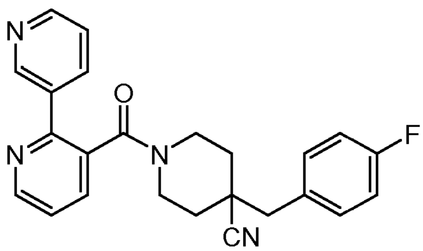 | C |
| A19 | 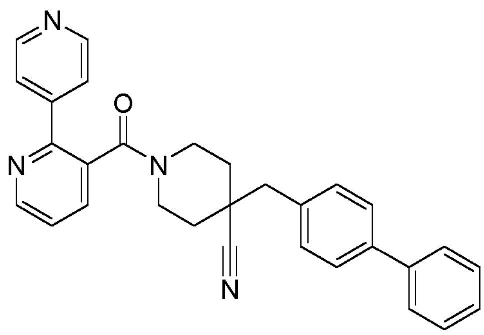 | A |

(continued)

| Compound No. | Structure | IC50 |
|---|---|---|
| A21 | 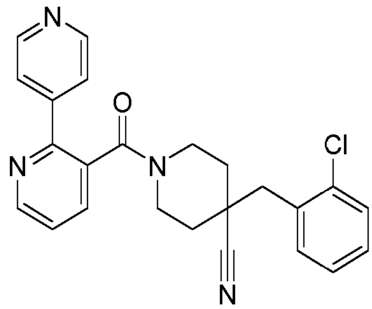 | A |
| A22 | 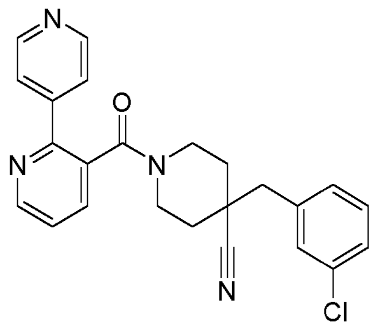 | A |
| A25 | 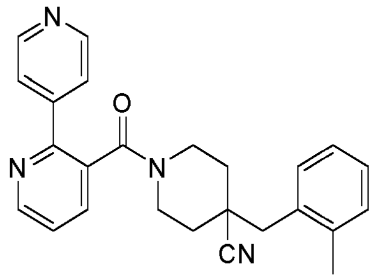 | A |
| A27 | 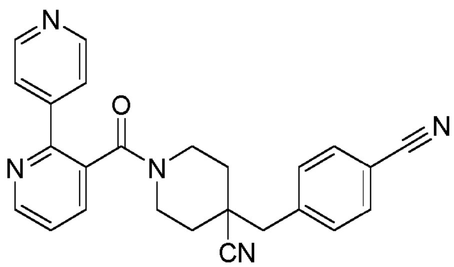 | A |
| A31 | 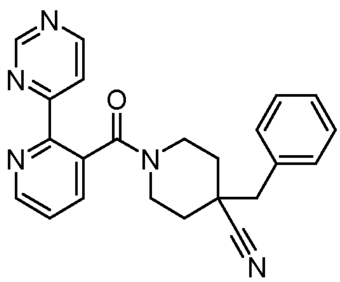 | A |

(continued)

| Compound No. | Structure | $IC_{50}$ |
|---|---|---|
| A32 | 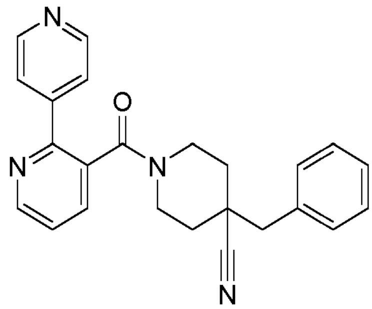 | A |
| A35 | 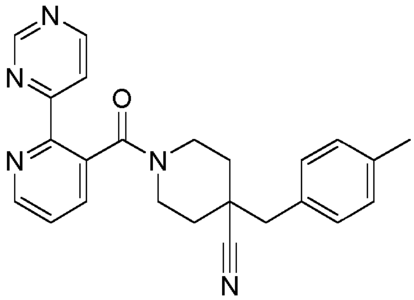 | A |
| A37 | 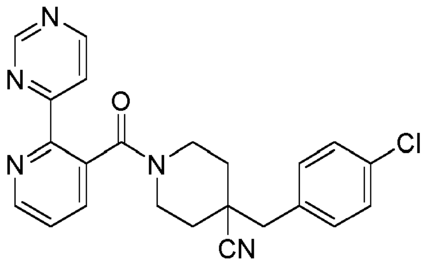 | A |
| A40 | 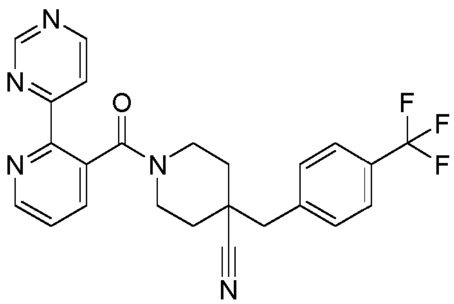 | A |
| A44 | 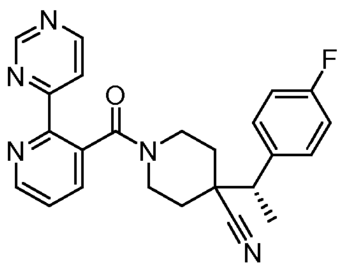 | A |

(continued)

| Compound No. | Structure | IC50 |
|---|---|---|
| A45 | 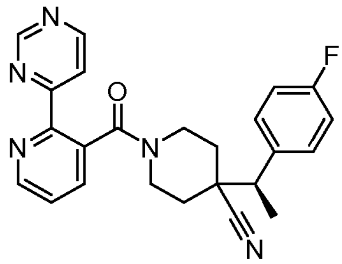 | A |
| A48 | 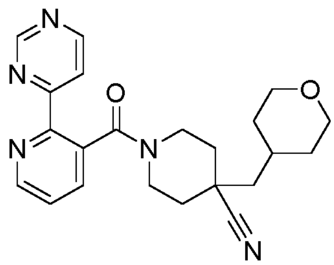 | B |
| A51 | 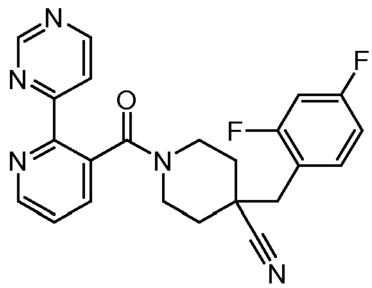 | A |
| A54 | 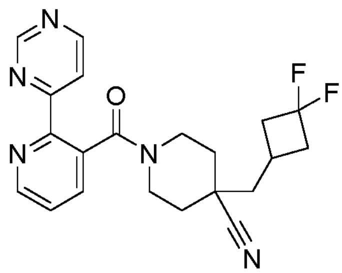 | B |
| A56 | 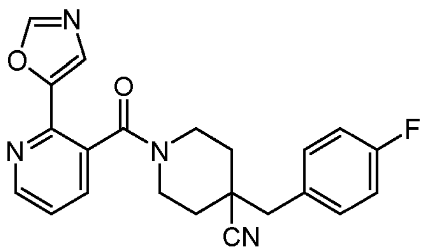 | B |
| A58 | 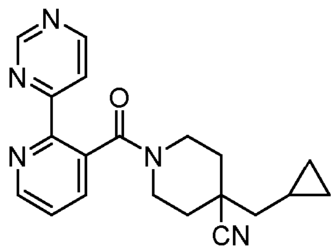 | B |

(continued)

| Compound No. | Structure | $IC_{50}$ |
|---|---|---|
| A63 | 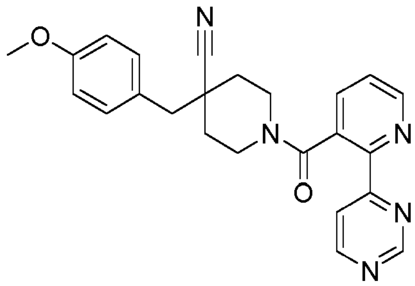 | A |
| A65 | 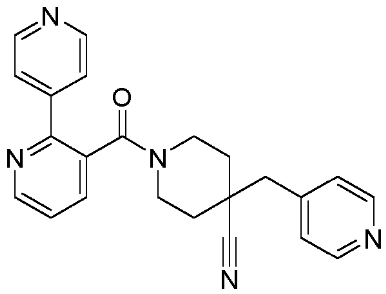 | B |
| A66 | 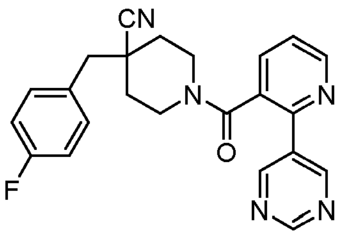 | B |
| A67 | 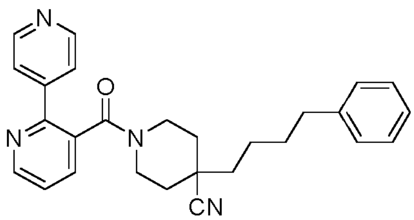 | A |
| A68 | 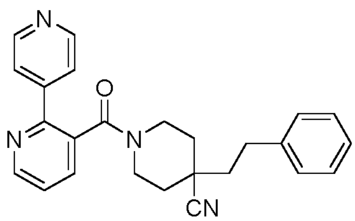 | B |
| A69 | 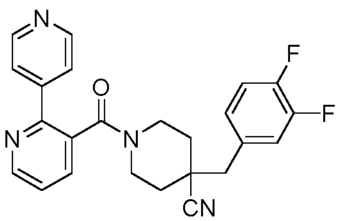 | A |

(continued)

| Compound No. | Structure | $IC_{50}$ |
|---|---|---|
| A70 | 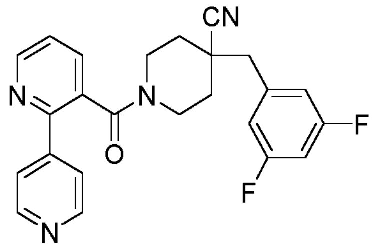 | A |
| A71 | 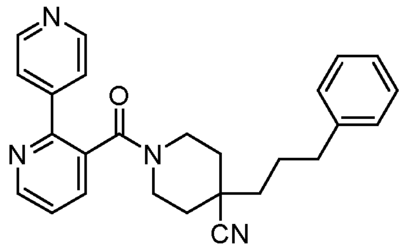 | A |
| A72 | 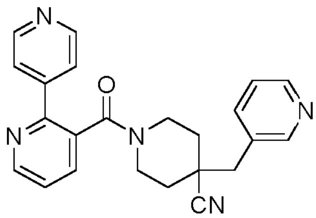 | B |
| A73 | 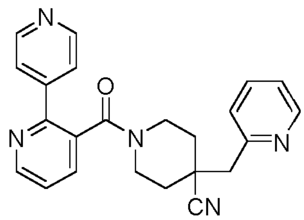 | B |
| A74 | 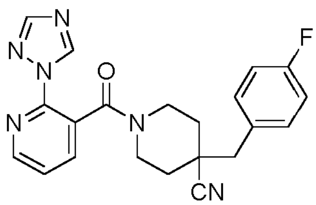 | C |
| A200 | 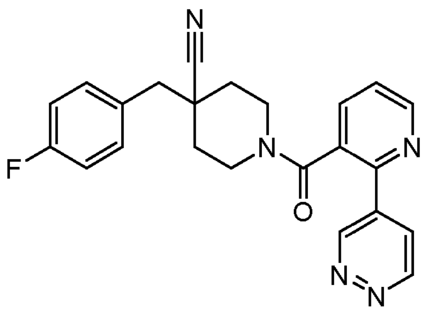 | C |

(continued)

| Compound No. | Structure | IC50 |
|---|---|---|
| A201 | 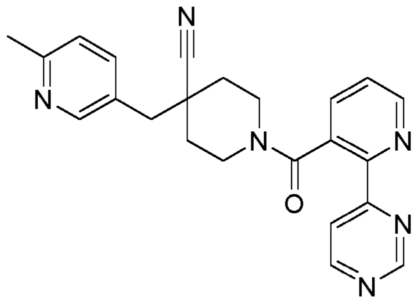 | A |
| A202 | 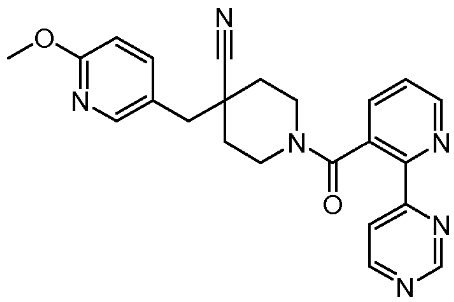 | A |
| A203 | 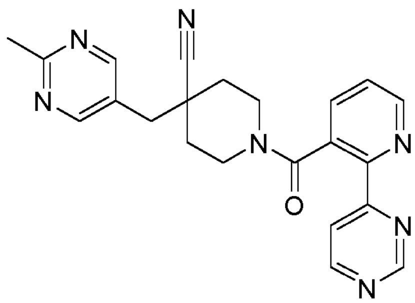 | C |
| A204 | 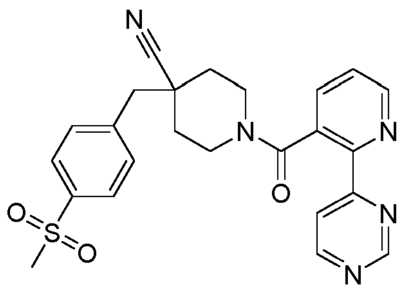 | B |
| A205 | 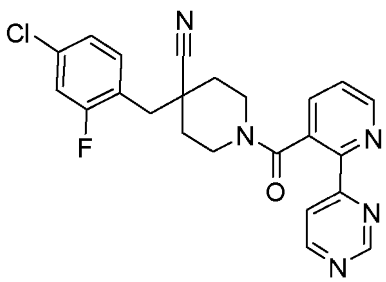 | A |

(continued)

| Compound No. | Structure | IC50 |
|---|---|---|
| A206 | 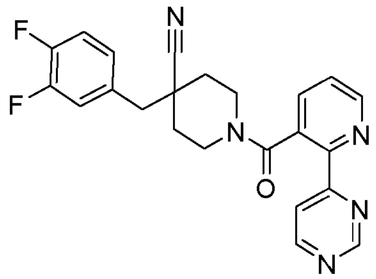 | A |
| A207 | 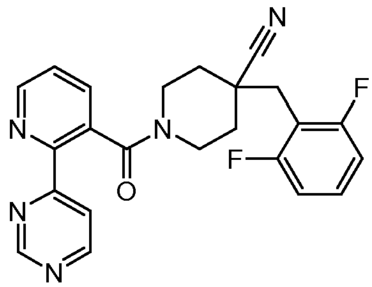 | A |
| A208 | 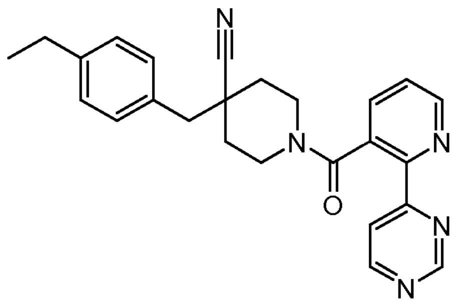 | A |
| A209 | 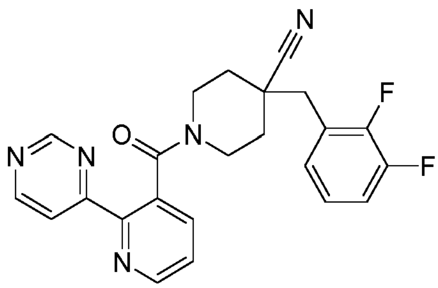 | A |
| A210 | 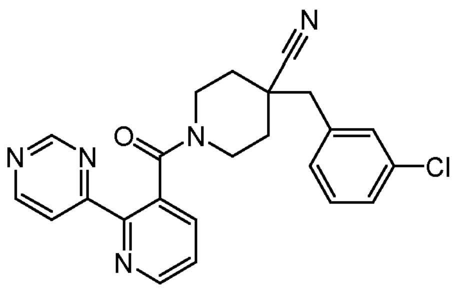 | A |

(continued)

| Compound No. | Structure | $IC_{50}$ |
|---|---|---|
| A211 | 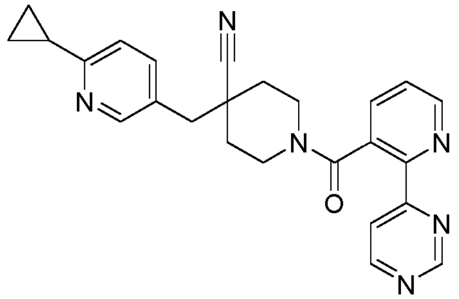 | A |
| A212 | 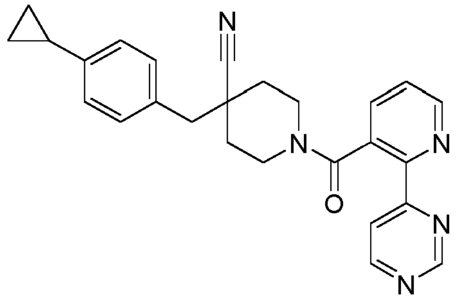 | A |
| A213 | 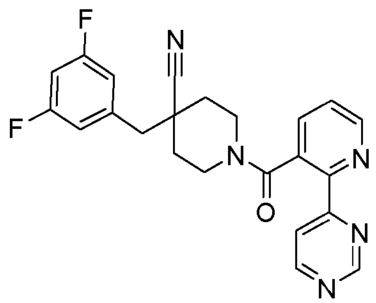 | A |
| A214 | 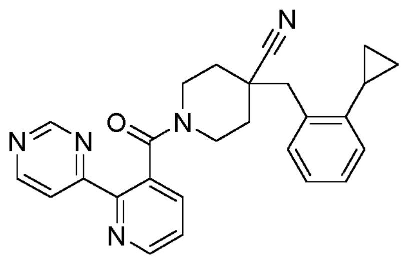 | A |
| A215 | 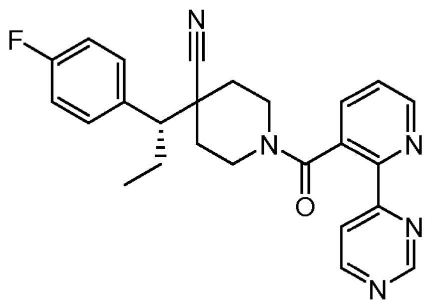 | A |

(continued)

| Compound No. | Structure | IC50 |
|---|---|---|
| A216 | 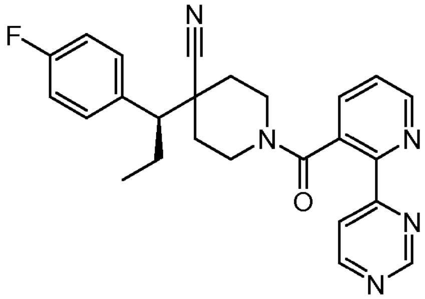 | A |
| A217 | 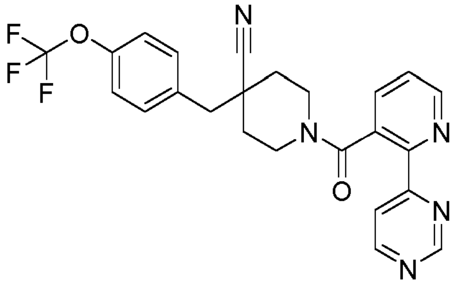 | A |
| A218 | 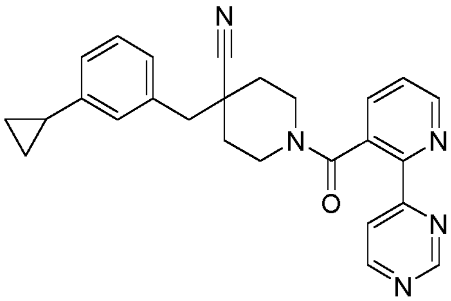 | A |
| A219 | 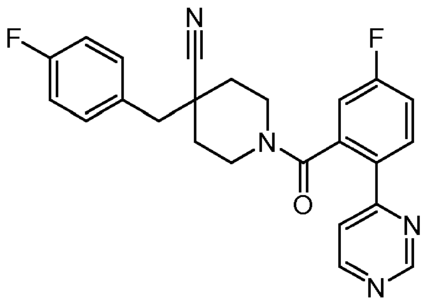 | A |
| A220 | 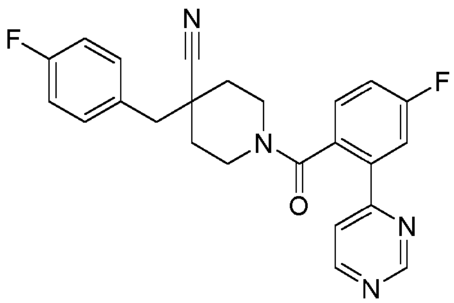 | A |

(continued)

| Compound No. | Structure | $IC_{50}$ |
|---|---|---|
| A221 | 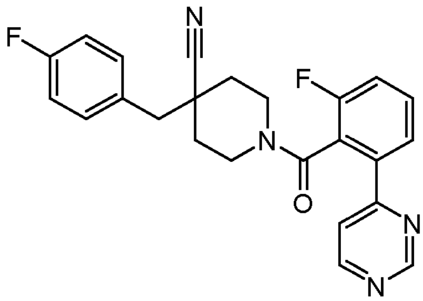 | A |
| A222 | 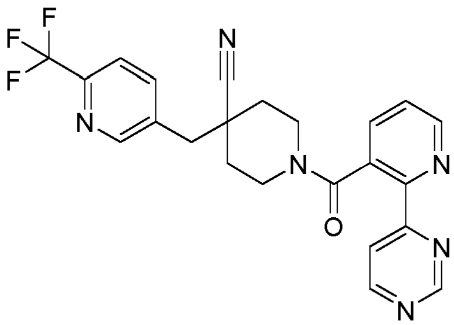 | B |
| A223 | 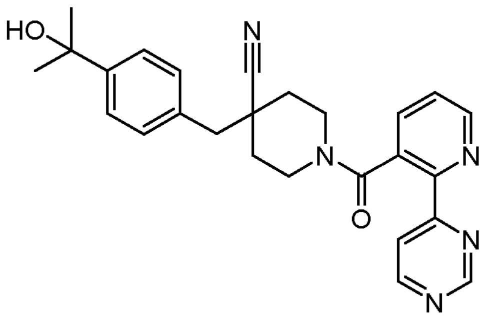 | B |
| A224 | 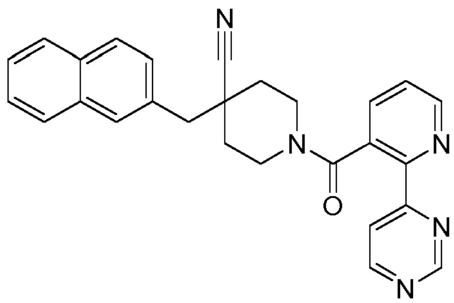 | A |
| A225 | 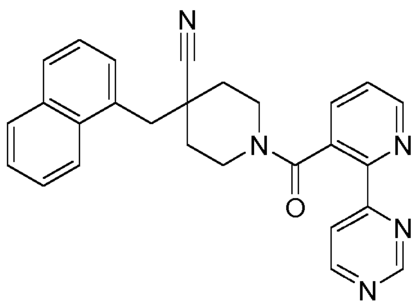 | A |

(continued)

| Compound No. | Structure | IC50 |
|---|---|---|
| A226 | 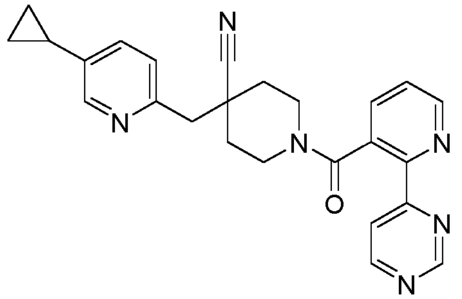 | B |
| A227 | 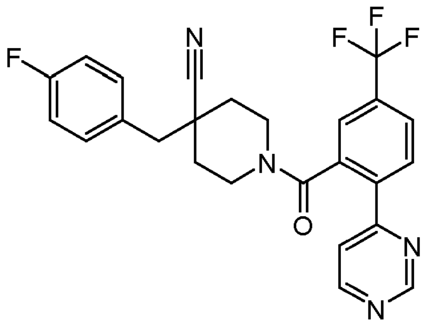 | A |
| A228 | 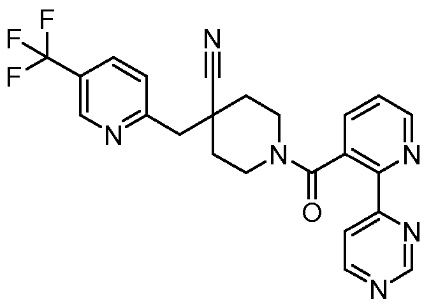 | B |
| A229 | 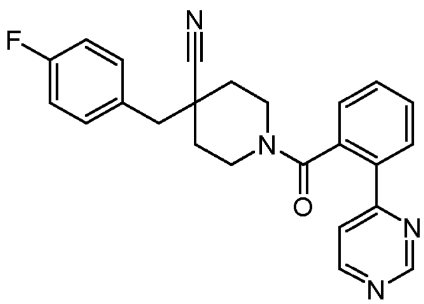 | A |
| A230 | 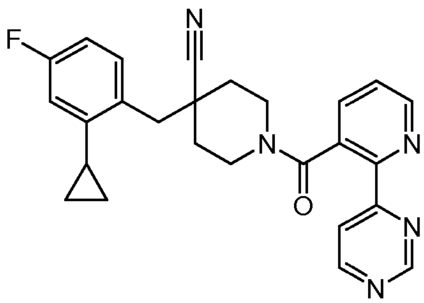 | A |

(continued)

| Compound No. | Structure | $IC_{50}$ |
|---|---|---|
| A231 | 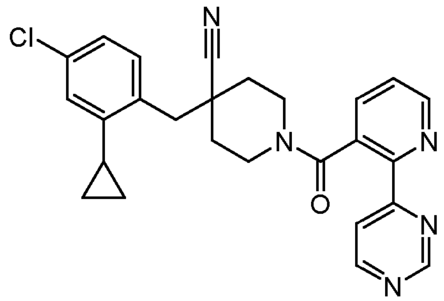 | A |
| A232 | 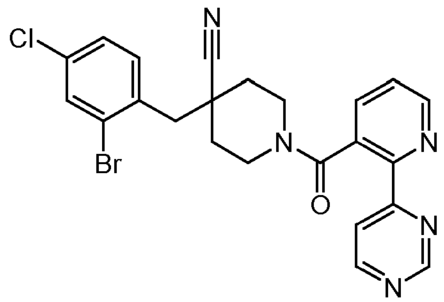 | A |
| A233 | 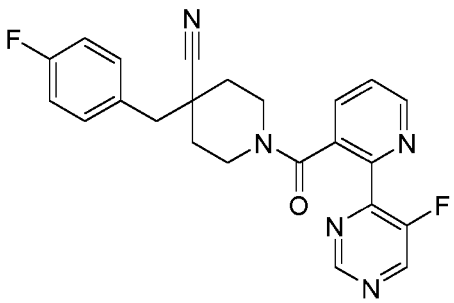 | C |
| A234 | 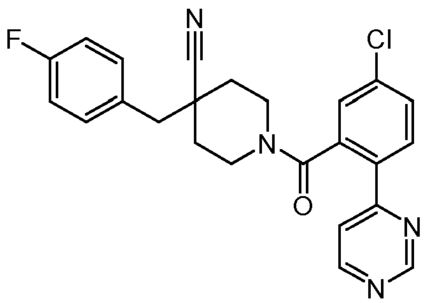 | A |
| A235 | 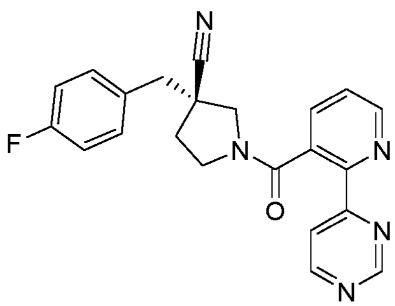 | C |

(continued)

| Compound No. | Structure | $IC_{50}$ |
|---|---|---|
| A236 | 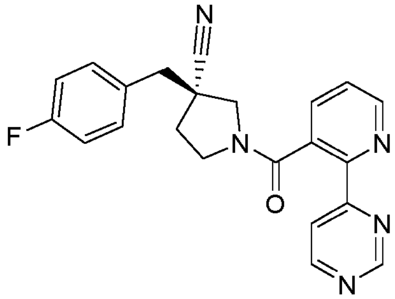 | C |
| A237 | 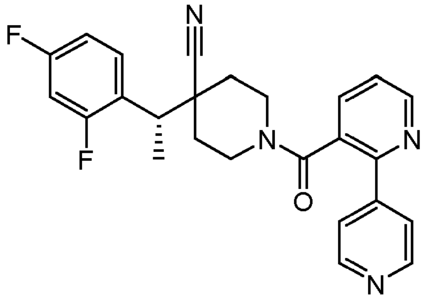 | A |
| A238 | 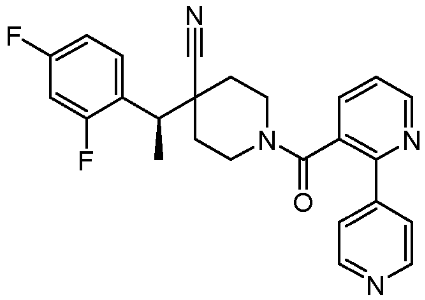 | A |
| A239 | 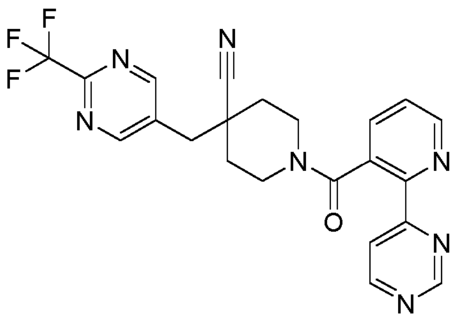 | C |
| A240 | 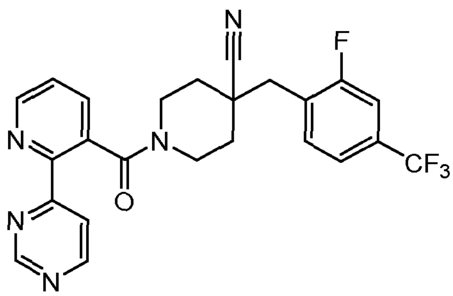 | A |

(continued)

| Compound No. | Structure | IC50 |
|---|---|---|
| A241 | 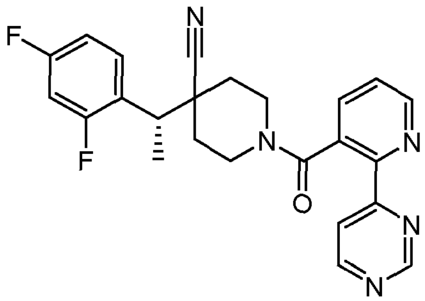 | A |
| A242 | 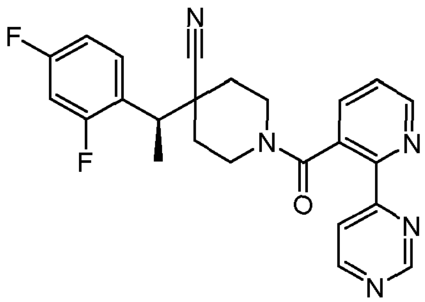 | A |
| A243 | 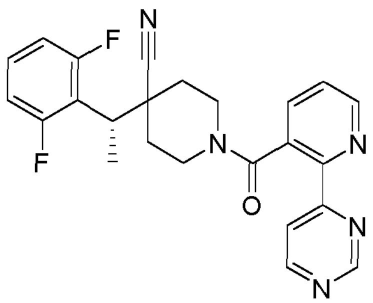 | A |
| A244 | 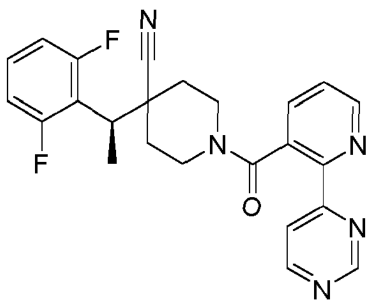 | A |
| A245 | 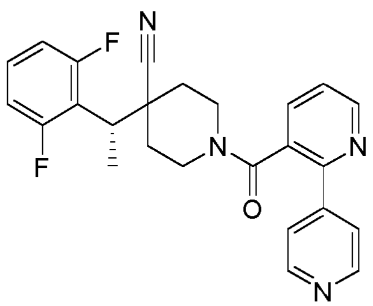 | A |

(continued)

| Compound No. | Structure | IC50 |
|---|---|---|
| A246 | 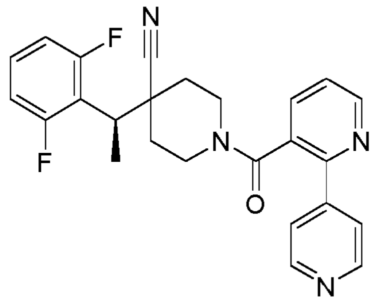 | A |
| A247 | 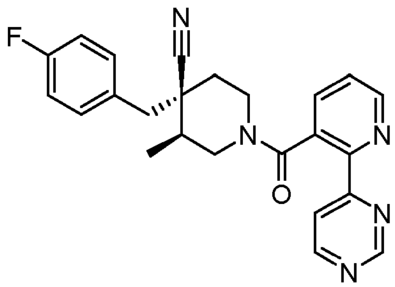 | A |
| A248 | 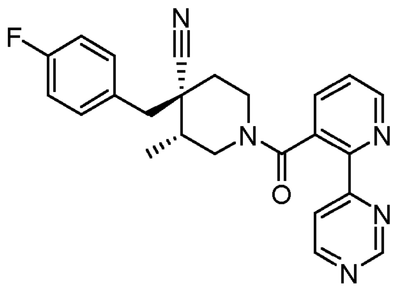 | A |
| A249 | 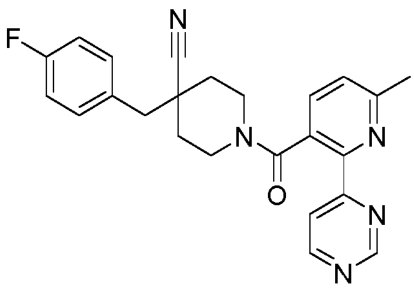 | C |
| A250 | 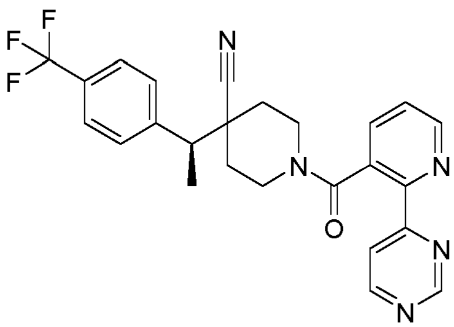 | A |

(continued)

| Compound No. | Structure | $IC_{50}$ |
|---|---|---|
| A251 | 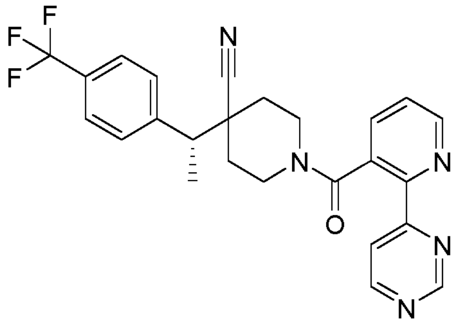 | A |
| A252 | 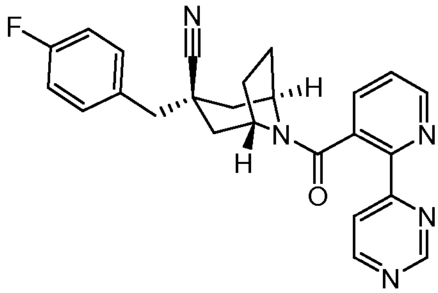 | B |
| A253 | 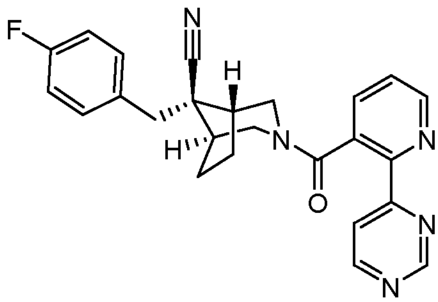 | C |
| A254 | 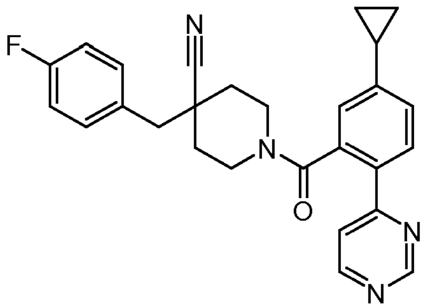 | A |
| A255 | 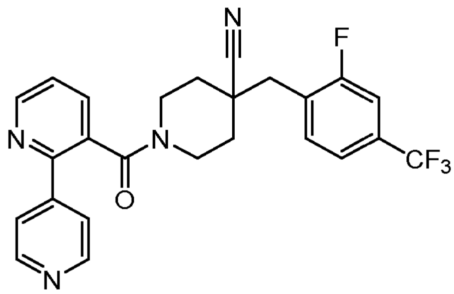 | A |

(continued)

| Compound No. | Structure | $IC_{50}$ |
|---|---|---|
| A256* | 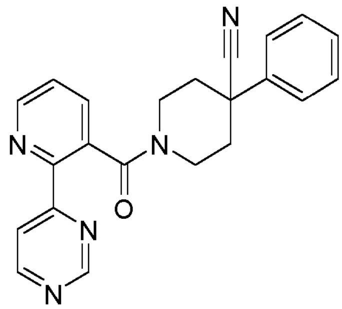 | C |
| A257 | 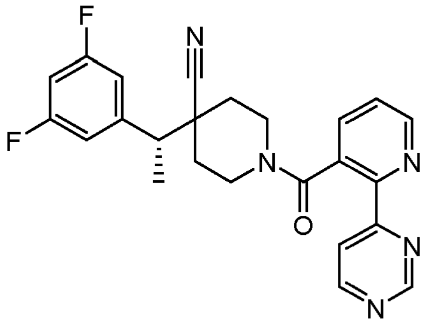 | A |
| A258 | 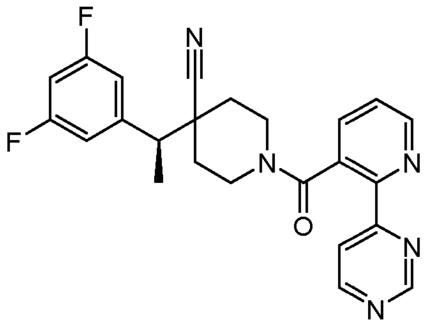 | A |
| A259 | 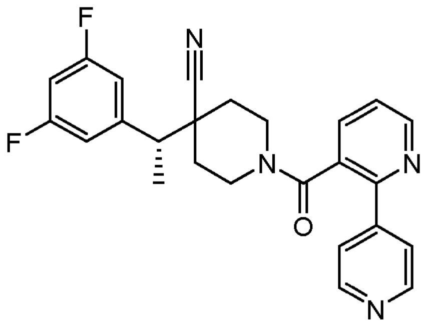 | A |
| A260 | 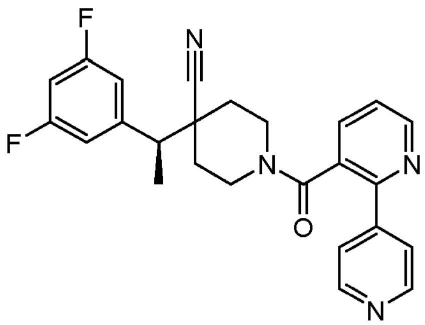 | A |

(continued)

| Compound No. | Structure | IC$_{50}$ |
|---|---|---|
| A261 | 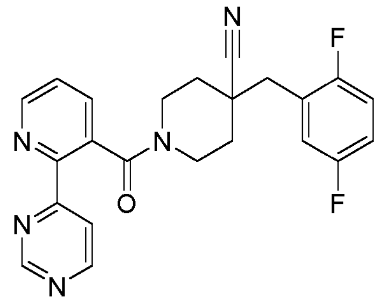 | A |
| A262 | 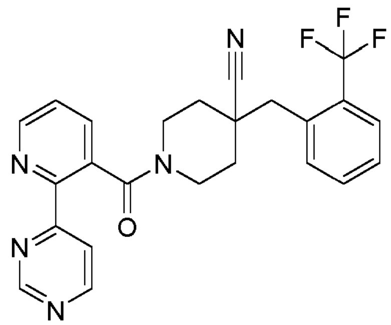 | A |
| A263 | 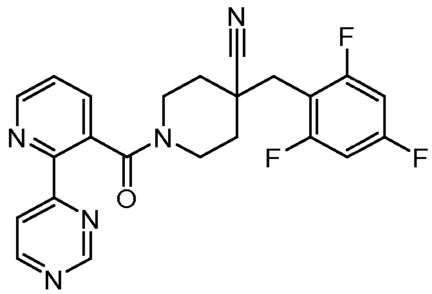 | A |
| A264 | 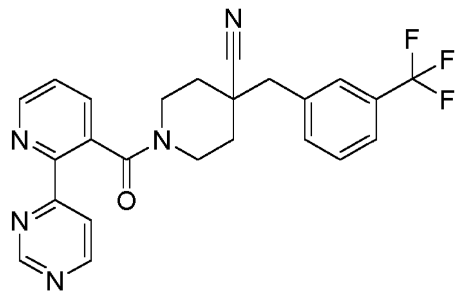 | A |
| A265 | 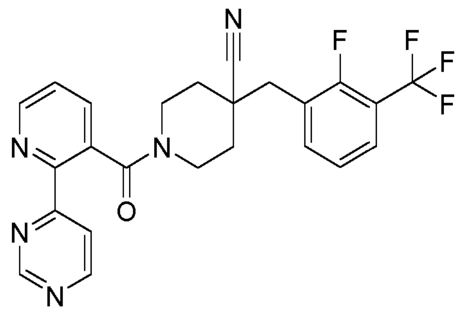 | A |

(continued)

| Compound No. | Structure | $IC_{50}$ |
|---|---|---|
| A266 | 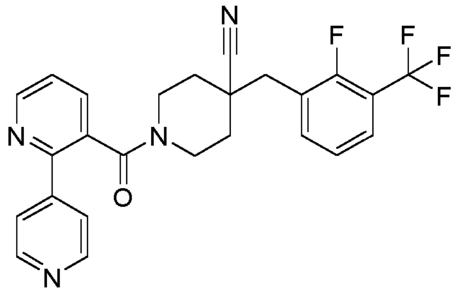 | A |
| A267 | 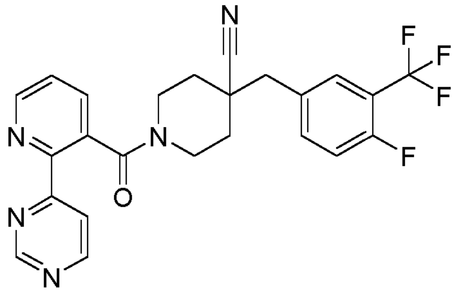 | A |
| A268 | 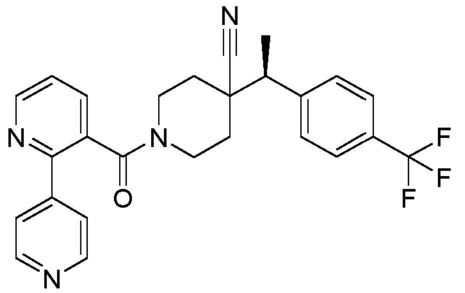 | A |
| A269 | 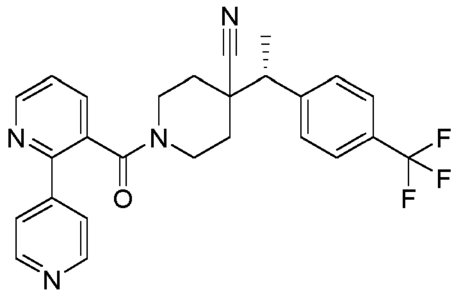 | A |
| A270 | 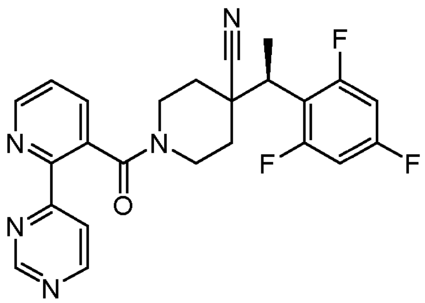 | A |

(continued)

| Compound No. | Structure | $IC_{50}$ |
|---|---|---|
| A271 | 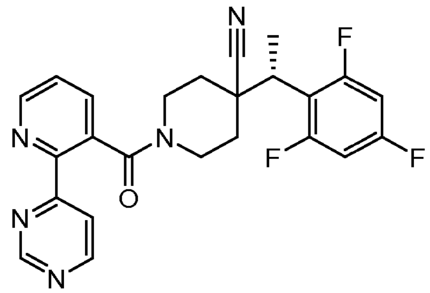 | A |
| A272 | 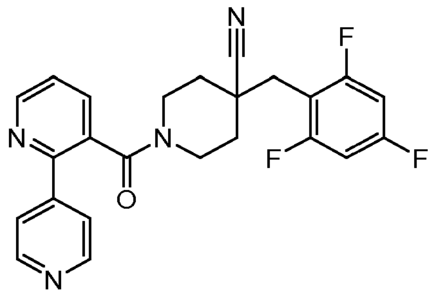 | A |
| A273 | 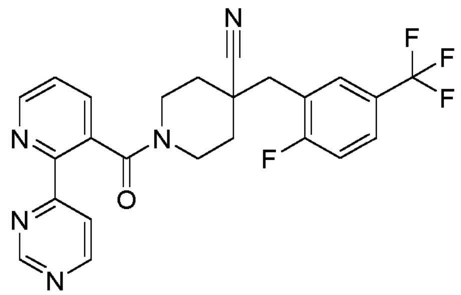 | A |
| A274 | 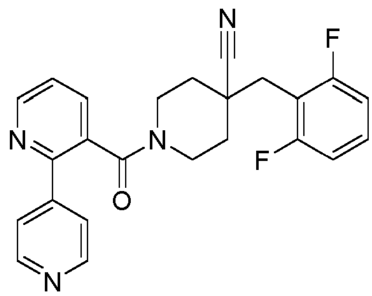 | A |
| A275 | 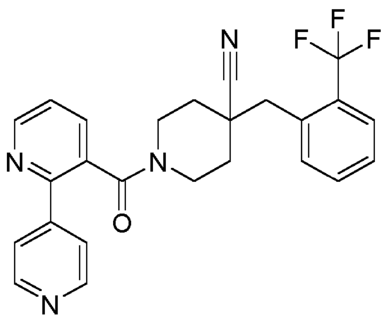 | A |

(continued)

| Compound No. | Structure | IC<sub>50</sub> |
|---|---|---|
| A276 | 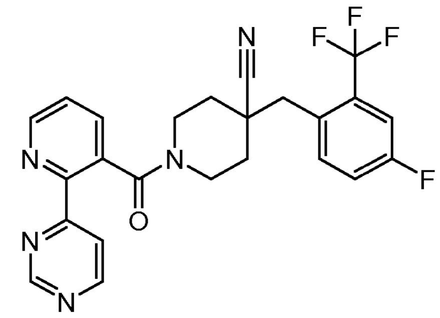 | B |
| A277 | 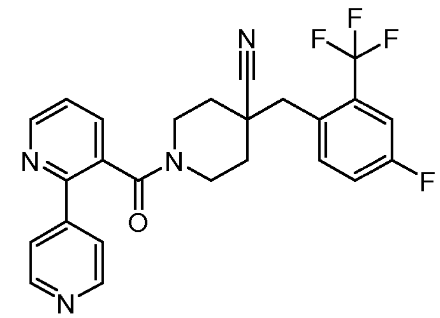 | A |
| A278 | 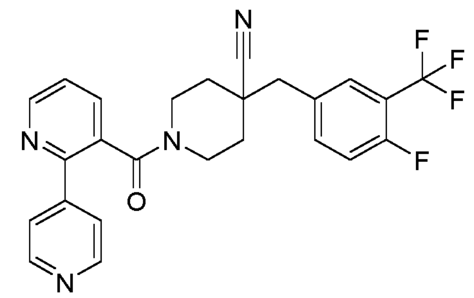 | A |
| A279 | 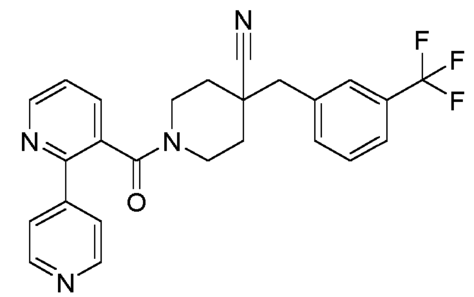 | A |
| A280 | 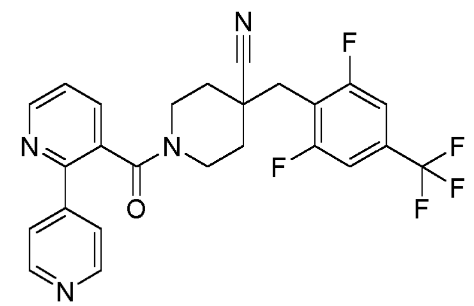 | A |

(continued)

| Compound No. | Structure | $IC_{50}$ |
|---|---|---|
| A281 | 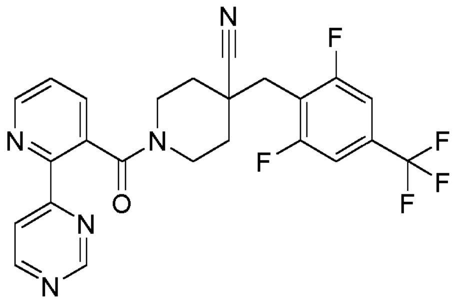 | A |
| A282 | 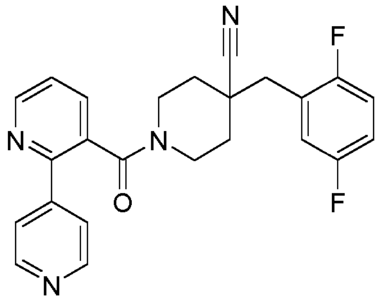 | A |
| A283 | 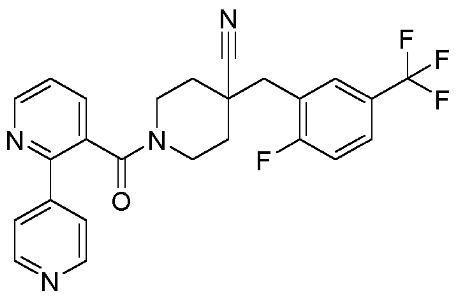 | A |
| A284 | 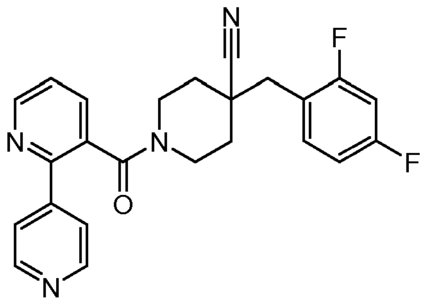 | A |
| A285 | 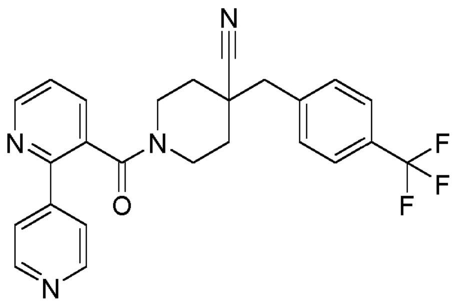 | A |

(continued)

| Compound No. | Structure | $IC_{50}$ |
|---|---|---|
| A286 | 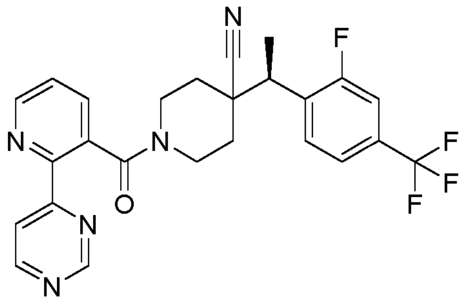 | A |
| A287 | 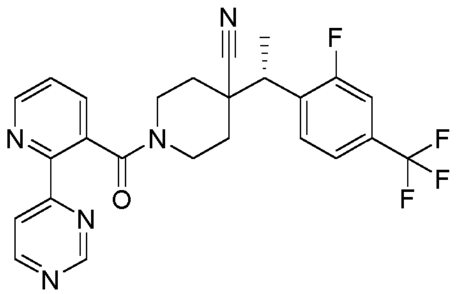 | A |
| *Reference example not falling within the scope of the present invention. | | |

Stability in human hepatocytes and permeability across MDCK-MDR1 cell monolayer data for selected compounds are in **Table 4** below:

**Table 4.** Human hepatocyte stability and MDCK-MDR1 cell monolayer permeability data for exemplary compounds.

| Intermediate | Structure | Human Hep Clint (mL/min/kg) | MDCK Mean Papp A:B ($10^{-6}$ cm/s) | MDCK Effux Ratio (B:A/A:B) |
|---|---|---|---|---|
| A32 | 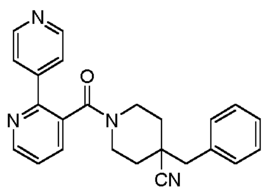 | <18 | 28 | 1.2 |
| A14 | 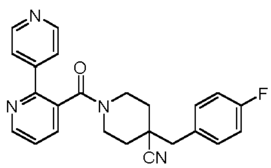 | <18 | 21 | 1.2 |
| A6 | 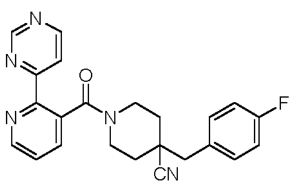 | <18 | 22 | 1.4 |

*Example 116. Mouse pharmacokinetics*

Compound A6 was evaluated in a mouse pharmacokinetics (PK) study. The compound was dosed at 30 mg/kg PO to 3 male CD-1 mice, formulated as a solution in 30% hydroxypropyl beta-cyclodextran, with a dosing volume of 5 ml/kg. Plasma samples were collected at 0.25, 0.50, 1.0, 2.0, 4.0, 8.0 and 24 h post-dose. In 2 separate groups with 3

mice/group, Compound A6 was dosed at 30 mg/kg, PO and plasma and brain samples were collected at 0.5 and 2.0 h post-dose. The compound had a $T_{1/2}$ of 0.96 h, Cmax of 10,700 ng/mL and an AUC of 14,400 ng*h/mL. The brain to plasma ratio at 0.25 and 2.0 h was 0.39 and 0.41, respectively.

## EQUIVALENTS AND SCOPE

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

It should it be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps.

## Claims

**1.** A compound of Formula I:

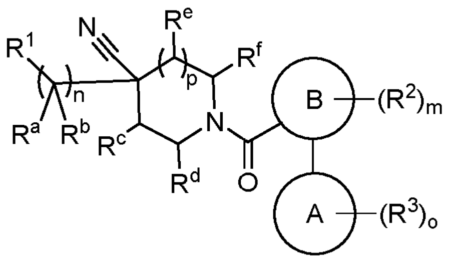

(I),

or a pharmaceutically acceptable salt thereof;
wherein:

$R^1$ is selected from the group consisting of $C_6$-$C_{10}$ aryl, $C_3$-$C_7$ cycloalkyl, 3-7 membered heterocyclyl, and 5-10 membered heteroaryl, wherein $R^1$ is optionally substituted with one, two, three, or four instances of $R^4$;
each of $R^a$ and $R^b$ is independently selected from the group consisting of H, halo, - CN, -OH, $-NO_2$, $-N(R^5)_2$, $C_1$-$C_6$ alkyl, $C_1$-$C_6$ haloalkyl, $C_1$-$C_6$ alkoxy, and $C_1$-$C_6$ haloalkoxy;
each of $R^{\circ}$, $R^d$, $R^e$, and $R^f$ is independently selected from the group consisting of H, $C_1$-$C_6$ alkyl, $C_1$-$C_6$ haloalkyl, $C_1$-$C_6$ alkoxy, and $C_1$-$C_6$ haloalkoxy; or $R^c$ and $R^e$ may form, together with the carbons to which they are attached, a $C_1$-$C_3$ alkylene bridge; or $R^d$ and $R^f$ may form, together with the carbons to which they are attached, a $C_1$-$C_3$ alkylene bridge;
each $R^4$ is independently selected from the group consisting of halo, -CN, -OH, $-NO_2$, $-N(R^5)_2$, $-S(O)_2R^5$, $C_1$-$C_6$ alkyl, $C_1$-$C_6$ haloalkyl, $C_1$-$C_6$ alkoxy, $C_1$-$C_6$ haloalkoxy, $C_6$-$C_{10}$ aryl, $C_3$-$C_7$ cycloalkyl, and 3-7 membered heterocyclyl;
each $R^5$ is independently selected from H and $C_1$-$C_6$ alkyl;
each $R^2$ is independently selected from the group consisting of halo, -CN, -OH, $-NO_2$, $-N(R^5)_2$, $C_1$-$C_6$ alkyl, $C_3$-$C_7$ cycloalkyl, $C_1$-$C_6$ haloalkyl, $C_1$-$C_6$ alkoxy, and $C_1$-$C_6$ haloalkoxy;
each $R^3$ is independently selected from the group consisting of halo, -CN, -OH, $-NO_2$, $-N(R^5)_2$, $C_1$-$C_6$ alkyl, $C_1$-$C_6$ haloalkyl, $C_1$-$C_6$ alkoxy, $C_3$-$C_7$ cycloalkyl, and $C_1$-$C_6$ haloalkoxy;
A is a 5-6 membered nitrogen-containing heteroaryl;
B is selected from $C_6$-$C_{10}$ aryl and 5-6 membered heteroaryl;
m is 0, 1, 2, or 3;
n is 1, 2, 3, or 4;
o is 0, 1, 2, or 3; and

p is 0, 1, or 2.

**2.** The compound of claim 1, or a pharmaceutically acceptable salt thereof, wherein:

(a) the compound of Formula I is a compound of Formula I-a:

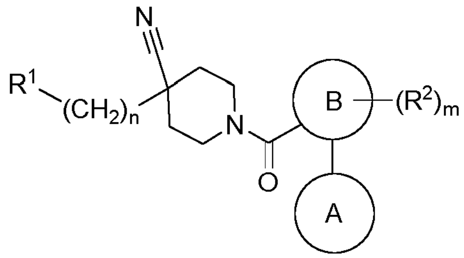

(I-a);

(b) the compound of Formula I is a compound of Formula I-b:

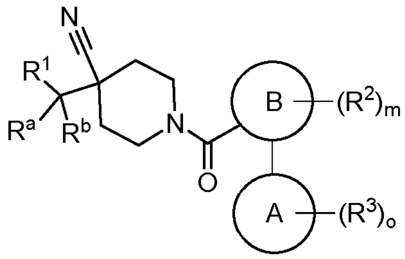

(I-b);

(c) the compound of Formula I is a compound of Formula I-c:

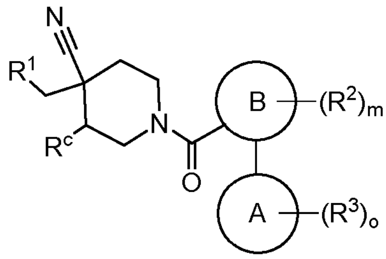

(I-c);

(d) the compound of Formula I is a compound of Formula I-d:

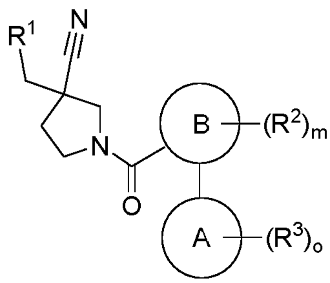

(I-d);

(e) the compound of Formula I is a compound of Formula I-e:

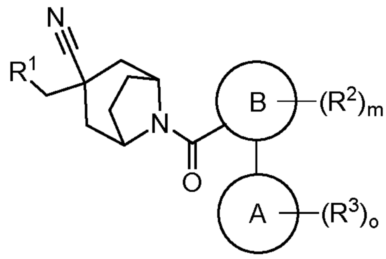

(I-e); or

(f) the compound of Formula I is a compound of Formula I-f:

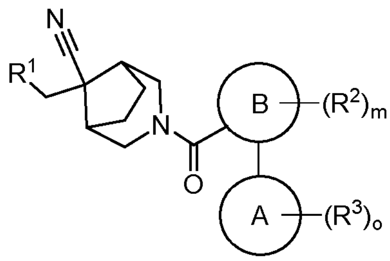

(I-f).

**3.** The compound of claim 1 or 2, or a pharmaceutically acceptable salt thereof, wherein:

(a) $R^1$ is $C_6$-$C_{10}$ aryl substituted with one, two, three, or four instances of $R^4$;
(b) $R^1$ is unsubstituted $C_6$-$C_{10}$ aryl;
(c) $R^1$ is 5-10 membered heteroaryl substituted with one, two, three, or four instances of $R^4$;
(d) $R^1$ is unsubstituted 5-10 membered heteroaryl;
(e) $R^1$ is $C_3$-$C_7$ cycloalkyl substituted with one, two, three, or four instances of $R^4$;
(f) $R^1$ is unsubstituted $C_3$-$C_7$ cycloalkyl;
(g) $R^1$ is 3-7 membered heterocyclyl substituted with one, two, three, or four instances of $R^4$; or
(h) $R^1$ is unsubstituted 3-7 membered heterocyclyl.

**4.** The compound of claim 1 or 2, or a pharmaceutically acceptable salt thereof, wherein:

(a) $R^1$ is

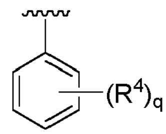

,

wherein each $R^4$ is independently halo, -CN, -OH, $-NO_2$, $-N(R^5)_2$, - $S(O)_2R^5$, $C_1$-$C_6$ alkyl, $C_1$-$C_6$ haloalkyl, $C_1$-$C_6$ alkoxy, $C_1$-$C_6$ haloalkoxy, $C_6$-$C_{10}$ aryl, $C_3$-$C_7$ cycloalkyl, or 3-7 membered heterocyclyl; wherein each $R^5$ is independently H or $C_1$-$C_6$ alkyl; and q is 0, 1, 2, or 3;
(b) $R^1$ is

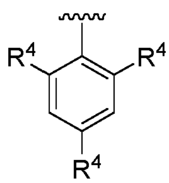

;

(c) $R^1$ is

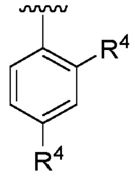, 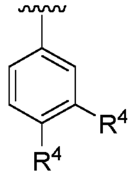, 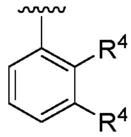,

or

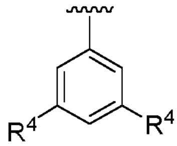;

(d) $R^1$ is

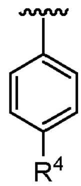;

(e) $R^1$ is

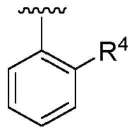;

(f) $R^1$ is

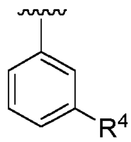;

or

(g) $R^1$ is

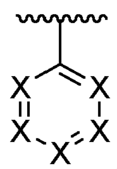,

wherein each X is independently CH or N, wherein the H of CH may be substituted with one or more instances of $R^4$; wherein each $R^4$ is independently halo, -CN, - OH, $-NO_2$, $-N(R^5)_2$, $C_1$-$C_6$ alkyl, $C_1$-$C_6$ haloalkyl, $C_1$-$C_6$ alkoxy, $C_1$-$C_6$ haloalkoxy, $C_6$-$C_{10}$ aryl, $C_3$-$C_7$ cycloalkyl, or 3-7 membered heterocyclyl; and each $R^5$ is independently H or $C_1$-$C_6$ alkyl.

**5.** The compound of claim 1 or 2, or a pharmaceutically acceptable salt thereof, wherein:

(a) $R^1$ is pyrrolyl, furanyl, thiophenyl, imidazolyl, pyrazolyl, oxazolyl, isoxazolyl, thiazolyl, isothiazolyl, triazolyl, oxadiazolyl, thiadiazolyl, pyridyl, 2-pyridyl, 3-pyridyl, 4-pyridyl, pyridinyl, pyridazinyl, pyrimidinyl, 2-pyrimidinyl, 4-pyrimidinyl, pyrazinyl, triazinyl, tetrazinyl, tetrazolyl, azocinyl, dithiazinyl, or oxazinyl;
(b) $R^1$ is pyridyl, 2-pyridyl, 3-pyridyl, 4-pyridyl, pyridinyl, pyridazinyl, pyrimidinyl, 2-pyrimidinyl, 4-pyrimidinyl, pyridazinyl, or pyrazinyl;
(c) $R^1$ is cyclopropyl or cyclobutyl;
(d) $R^1$ is tetrahydrofuran, tetrahydropyran, pyrrolidine, piperidine, piperazine, dioxolane, dioxane, thiomorpholine, or dithiane; or
(e) $R^1$ is tetrahydrofuran or tetrahydropyran.

**6.** The compound of claim 1 or 2, or a pharmaceutically acceptable salt thereof, wherein:

(a) $R^1$ is

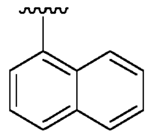

or

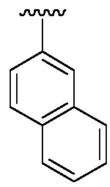
;

(b) $R^1$ is

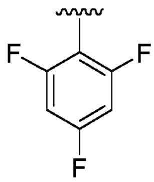

or

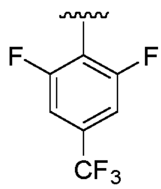
;

(c) $R^1$ is

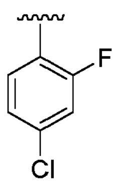 , 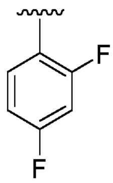 , 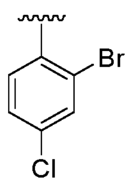 , 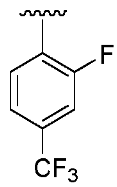 , 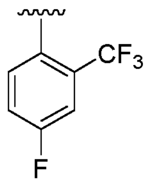 , 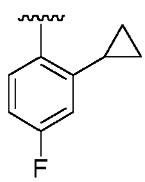 ,

or

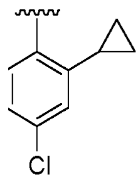;

(d) $R^1$ is

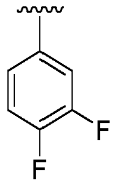, 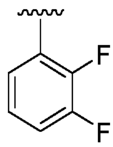, 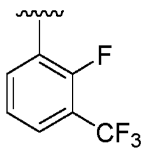,

or

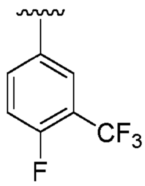;

(e) $R^1$ is

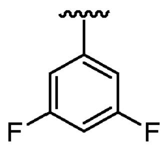

or

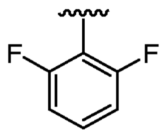;

(f) $R^1$ is

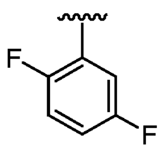

or

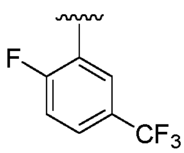;

(g) $R^1$ is

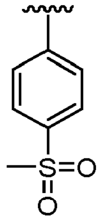 , 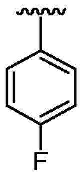 , 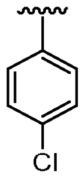 , 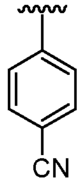 , 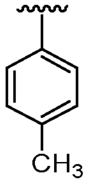 , 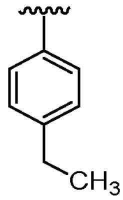 , 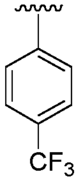 , 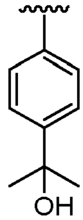 , 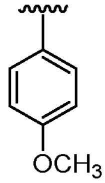 , 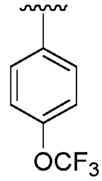 ,

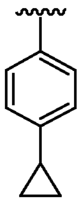 , 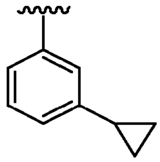 , 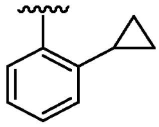 , 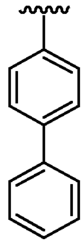 , 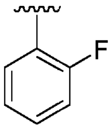 , 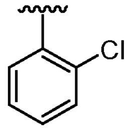 , 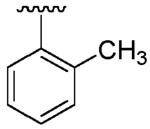 , 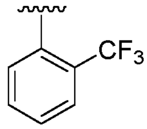 ,

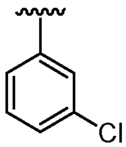 , 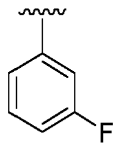 ,

or

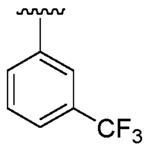 ;

(h) $R^1$ is

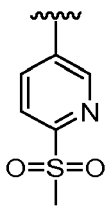 , 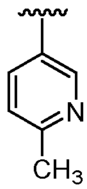 , 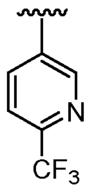 , 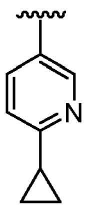 , 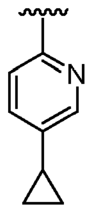 , 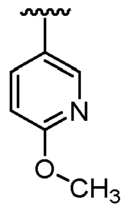 , 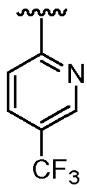 , 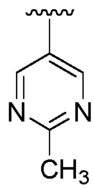 ,

or

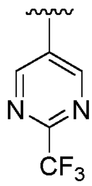 ;

(i) $R^1$ is

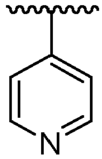,
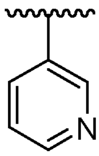,

or

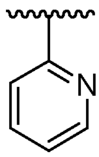;

(j) $R^1$ is

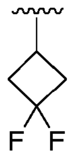;

(k) $R^1$ is

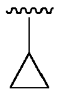;

or
(l) $R^1$ is

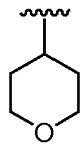.

**7.** The compound of claim 3, or a pharmaceutically acceptable salt thereof, wherein $R^1$ is $C_6$-$C_{10}$ aryl optionally substituted with one, two, three, or four instances of $R^4$.

**8.** The compound of claim 3, or a pharmaceutically acceptable salt thereof, wherein $R^1$ is 5-10 membered heteroaryl optionally substituted with one, two, three, or four instances of $R^4$.

**9.** The compound of claim 3, or a pharmaceutically acceptable salt thereof, wherein $R^1$ is $C_3$-$C_7$ cycloalkyl optionally substituted with one, two, three, or four instances of $R^4$.

**10.** The compound of claim 3, or a pharmaceutically acceptable salt thereof, wherein $R^1$ is 3-7 membered heterocyclyl optionally substituted with one, two, three, or four instances of $R^4$.

**11.** The compound of any one of claims 1-10, or a pharmaceutically acceptable salt thereof, wherein:

(a) each $R^4$ is independently substituted $C_1$-$C_6$ alkyl, substituted $C_1$-$C_6$ alkoxy, or substituted $C_3$-$C_7$ cycloalkyl;
(b) each $R^4$ is independently unsubstituted $C_1$-$C_6$ alkyl, unsubstituted $C_1$-$C_6$ alkoxy, or unsubstituted $C_3$-$C_7$ cycloalkyl;
(c) each $R^4$ is independently halo, $-NH_2$, $-NH(C_1$-$C_6$ alkyl), $-N(C_1$-$C_6$ alkyl$)_2$, $C_1$-$C_6$ alkyl, $C_1$-$C_6$ haloalkyl, $C_1$-$C_6$ alkoxy, $C_1$-$C_6$ haloalkoxy, or $C_6$-$C_{10}$ aryl; or

(d) each $R^4$ is independently halo, $C_1$-$C_6$ alkyl, $C_1$-$C_6$ haloalkyl, $C_1$-$C_6$ alkoxy, $C_1$-$C_6$ - haloalkoxy, or $C_6$-$C_{10}$ aryl.

**12.** The compound of any one of claims 1-10, or a pharmaceutically acceptable salt thereof, wherein:

(a) each $R^4$ is independently halo, -CN, $-CH_3$, $-CH_2CH_3$, $-CF_3$, $-OCH_3$, $-OCF_3$, $-C(CH_3)_2OH$, or $-C_6H_5$; or
(b) each $R^4$ is independently Cl, F, Br, or I.

**13.** The compound of any one of claims 1-12, or a pharmaceutically acceptable salt thereof, wherein:

(a) n is 4;
(b) n is 3;
(c) n is 2; or
(d) n is 1.

**14.** The compound of claim 13, or a pharmaceutically acceptable salt thereof, wherein n is 1.

**15.** The compound of any one of claims 1-14, or a pharmaceutically acceptable salt thereof, wherein:

(a) n is 1, and $R^a$ is $C_1$-$C_6$ alkyl and $R^b$ is H;
(b) n is 1, and $R^a$ is ethyl and $R^b$ is H; or
(c) n is 1, and $R^a$ is methyl and $R^b$ is H.

**16.** The compound of any one of claims 1-15, or a pharmaceutically acceptable salt thereof, wherein:

(a) p is 2;
(b) p is 1; or
(c) p is 0.

**17.** The compound of claim 16, or a pharmaceutically acceptable salt thereof, wherein:

(a) p is 1, and $R^c$, $R^d$, $R^e$, and $R^f$ are H;
(b) p is 1, $R^c$ is methyl, and $R^d$, $R^e$, and $R^f$ are H;
(c) p is 1, $R^c$ and $R^e$ are H, and $R^d$ and $R^f$ , together with the carbons to which they are attached, form a $C_1$-$C_3$ alkylene bridge;
(d) p is 1, $R^d$ and $R^f$ are H, and $R^c$ and $R^e$, together with the carbons to which they are attached, form a $C_1$-$C_3$ alkylene bridge; or
(e) p is 0, and $R^c$, $R^d$, and $R^f$ are H.

**18.** The compound of any one of claims 1-17, or a pharmaceutically acceptable salt thereof, wherein:

(a) B is

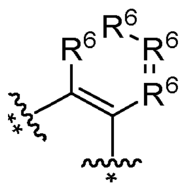
;

wherein each $R^6$ is independently N or $CR^{6a}$, wherein $R^{6a}$ is H or $R^2$; and ** is the point of attachment to the carbonyl, and * is the point of attachment to A; or
(b) B is

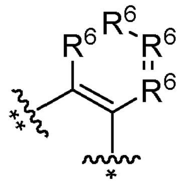
;

wherein up to two $R^6$ may be N and the other occurrences of $R^6$ are CH; and ** is the point of attachment to the carbonyl, and * is the point of attachment to A.

**19.** The compound of any one of claims 1-17, or a pharmaceutically acceptable salt thereof, wherein:

(a) B is

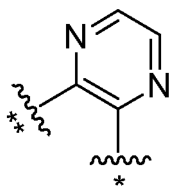
,
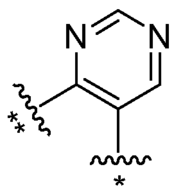
,
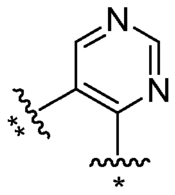
,
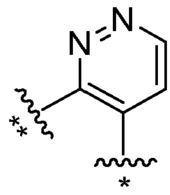
,
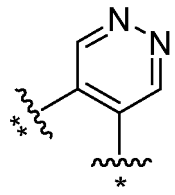
,
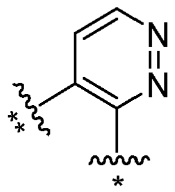
,
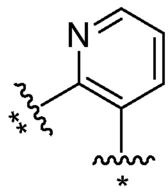
,
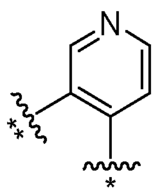
,
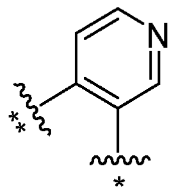
,
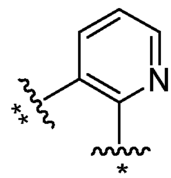
,

or

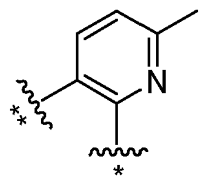
;

wherein ** is the point of attachment to the carbonyl, and * is the point of attachment to A;
(b) B is

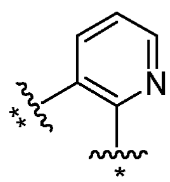
;

wherein ** is the point of attachment to the carbonyl, and * is the point of attachment to A; or
(c) B is

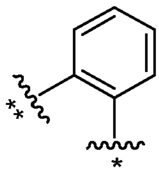 , 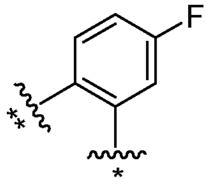 , 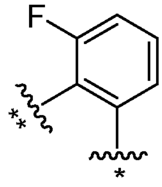 , 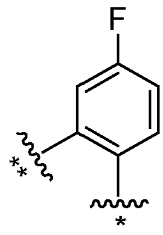 , 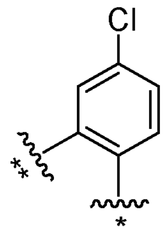 ,

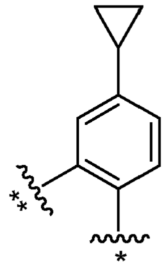 ,

or

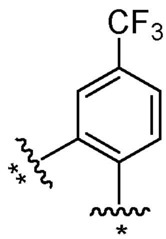 ;

wherein ** is the point of attachment to the carbonyl, and * is the point of attachment to A.

**20.** The compound of claim 19, or a pharmaceutically acceptable salt thereof, wherein B is

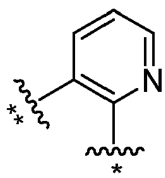 ;

wherein ** is the point of attachment to the carbonyl, and * is the point of attachment to A.

**21.** The compound of claim 19, or a pharmaceutically acceptable salt thereof, wherein B is

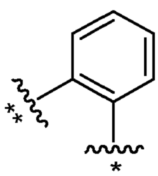 , 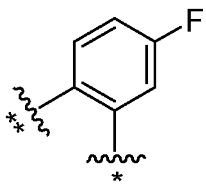 , 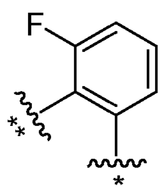 , 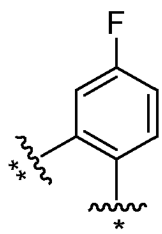 , 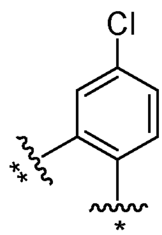 ,

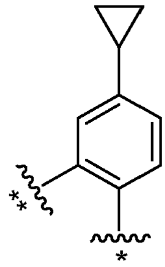

,

or

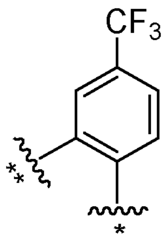

;

wherein ** is the point of attachment to the carbonyl, and * is the point of attachment to A.

**22.** The compound of any one of claims 1-21, or a pharmaceutically acceptable salt thereof, wherein:

(a) m is 1; or
(b) m is 0.

**23.** The compound any one of claims 1-22, or a pharmaceutically acceptable salt thereof, wherein $R^2$ is halo, -CN, -OH, $-NO_2$, $-CH_3$, $-CH_2CH_3$, cyclopropyl, $-CHF_2$, $-CF_3$, $-OCF_3$, - $OCH_3$, $-OCH_2CH_3$, or $-OCH_2CF_3$.

**24.** The compound of any one of claims 1-23, or a pharmaceutically acceptable salt thereof, wherein:

(a) A is pyridinyl, pyrrolyl, imidazolyl, pyrazolyl, pyridazinyl, pyrimidinyl, pyrazinyl, triazolyl, triazinyl, tetrazinyl, tetrazolyl, oxazolyl, isoxazolyl, or thiozolyl; or
(b) A is pyridinyl, oxazolyl, imidazolyl, triazolyl, pyridinyl, pyridazinyl, pyrimidinyl, or triazinyl.

**25.** The compound of any one of claims 1-23, or a pharmaceutically acceptable salt thereof, wherein A is

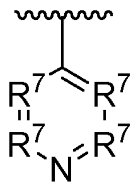

;

wherein each $R^7$ is independently N or CH, wherein up to two $R^7$ may be N and the other occurrences of $R^7$ are CH, wherein the hydrogen of CH may be substituted with $R^3$.

**26.** The compound of any one of claims 1-23, or a pharmaceutically acceptable salt thereof, wherein:

(a) A is

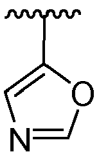, 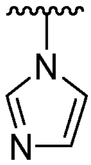, 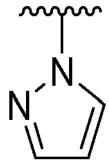, 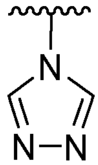, 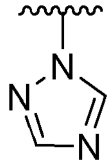, 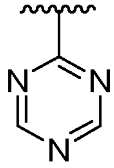, 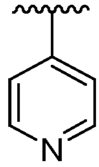, 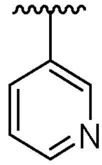, 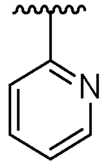,

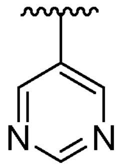, 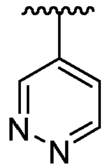, 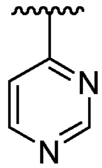,

or

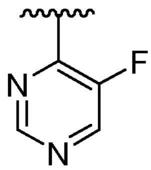;

(b) A is

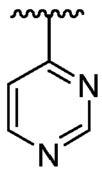, 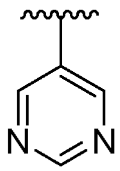, 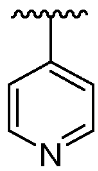, 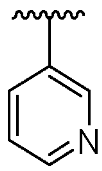, 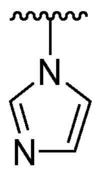, 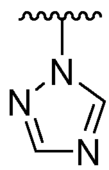, 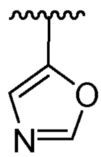,

or

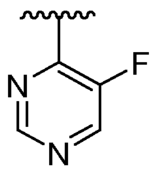.

**27.** The compound of any one of claims 1-26, or a pharmaceutically acceptable salt thereof, wherein the compound is selected from the group consisting of:

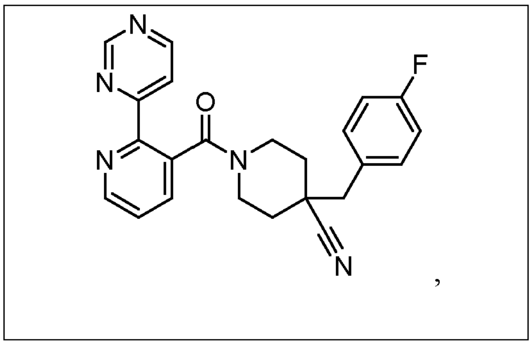,

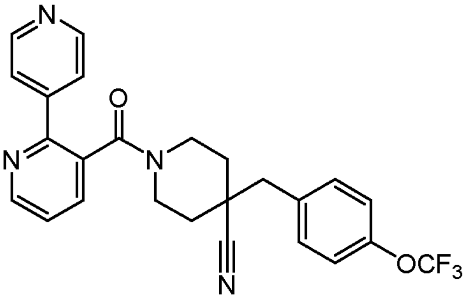

,

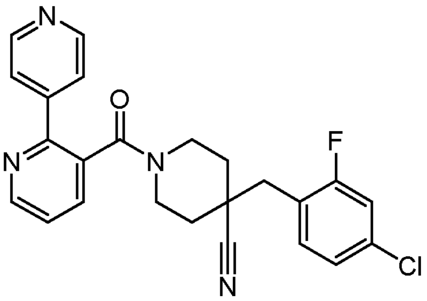

,

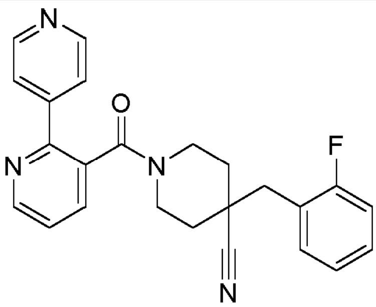

,

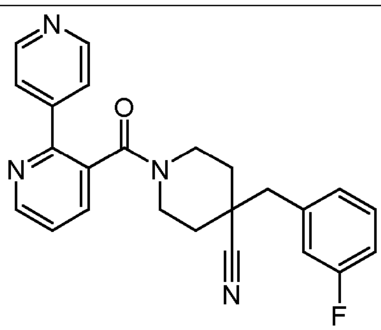

,

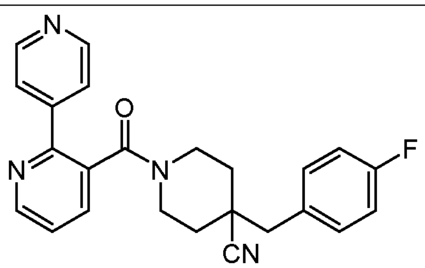

,

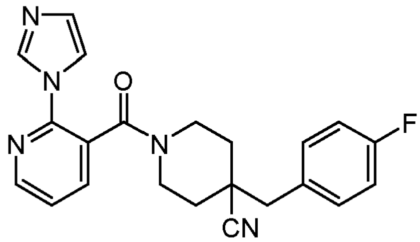

,

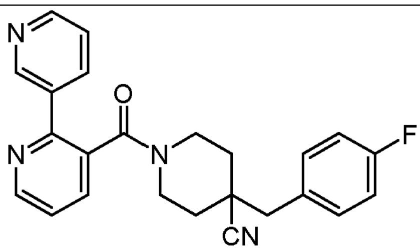

,

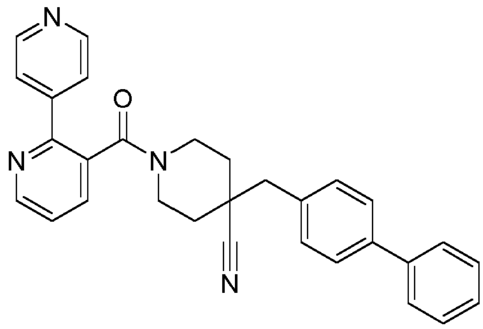

,

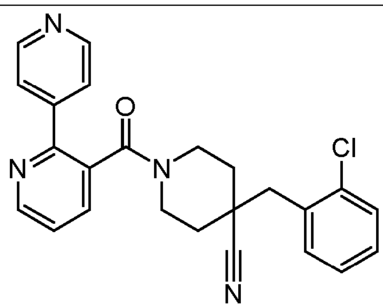

,

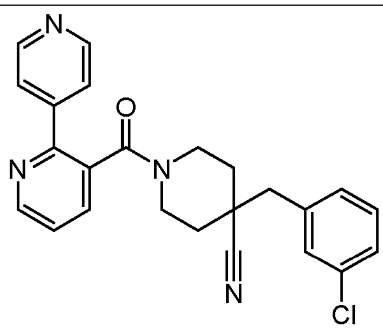

,

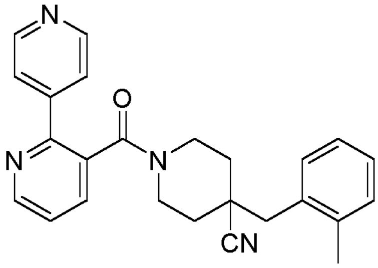
,

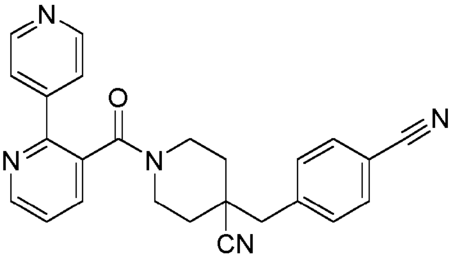
,

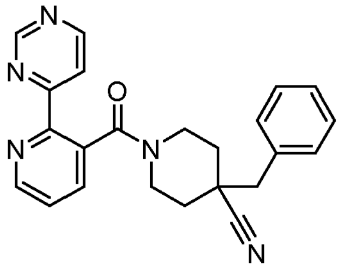
,

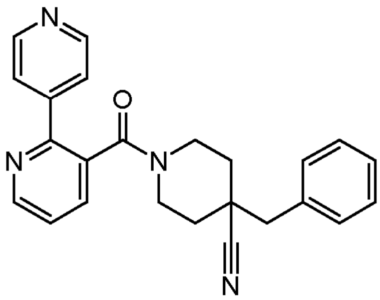
,

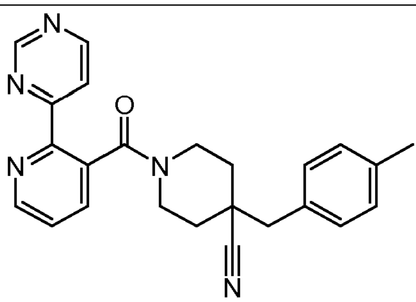
,

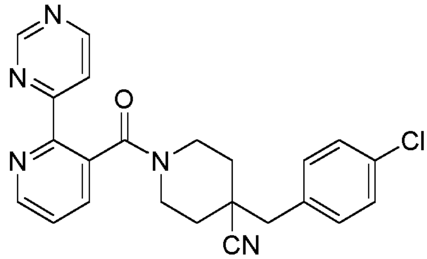
,

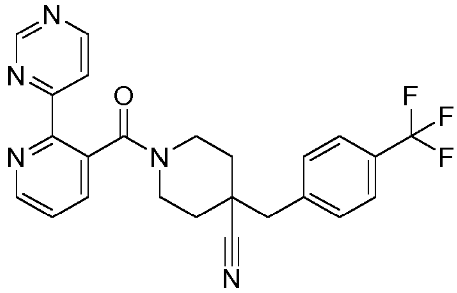
,

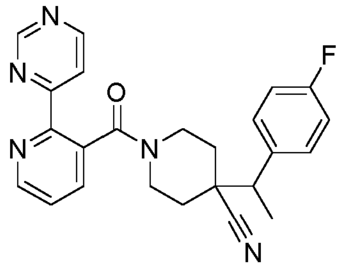
,

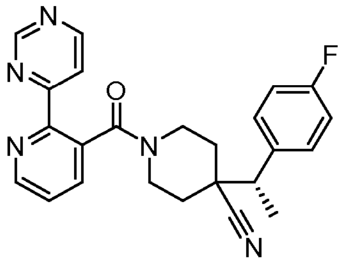
,

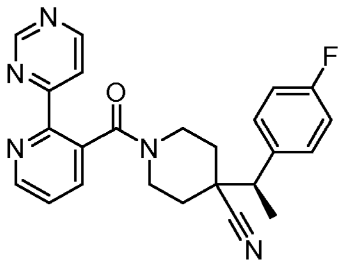
,

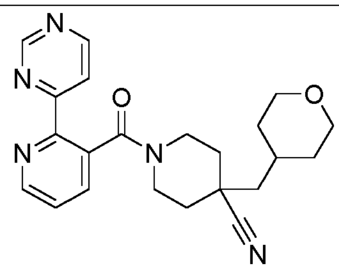
,

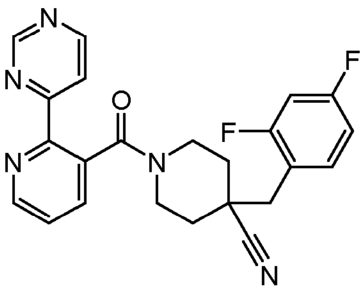
,

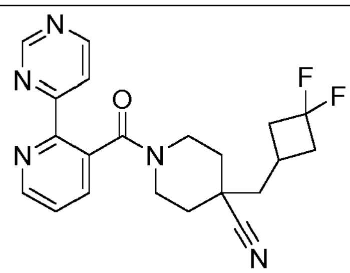
,

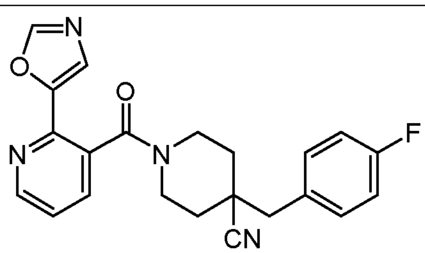
,

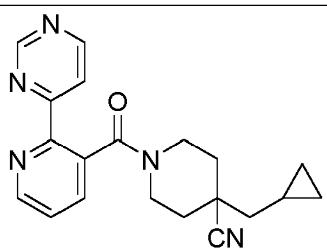
,

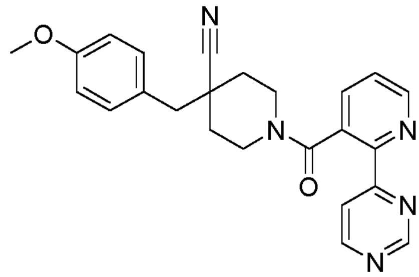
,

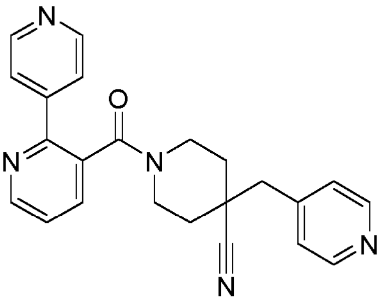
,

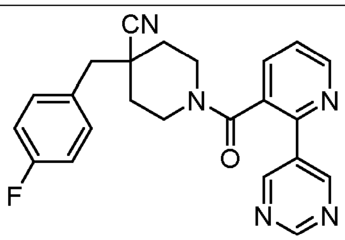
,

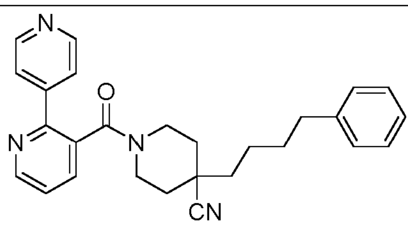
,

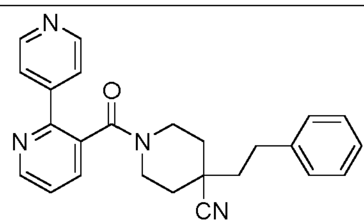
,

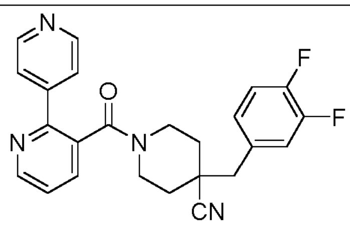
,

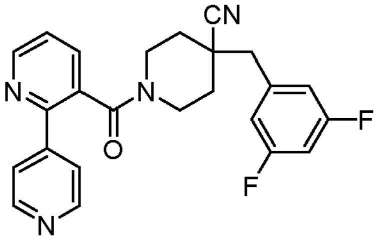
,

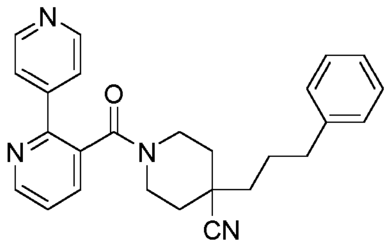
,

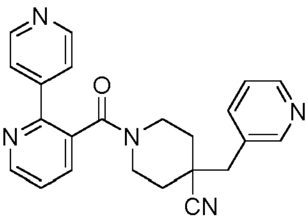
,

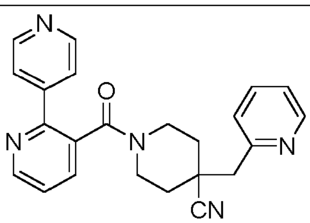
,

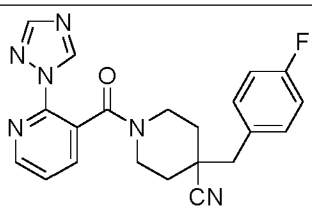
,

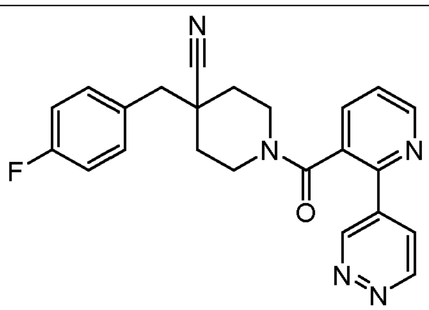
,

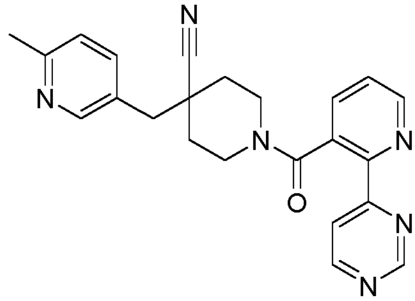
,

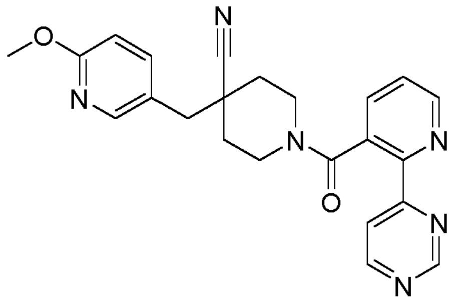
,

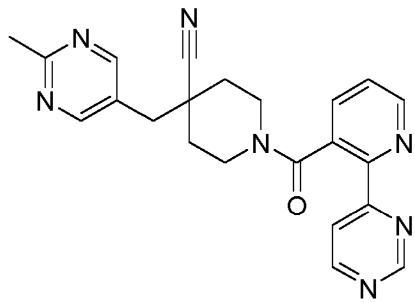
,

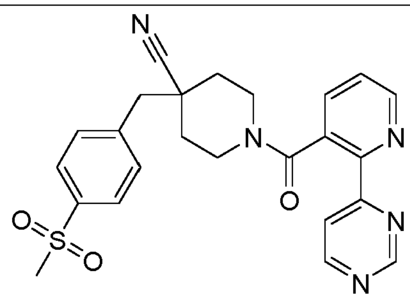
,

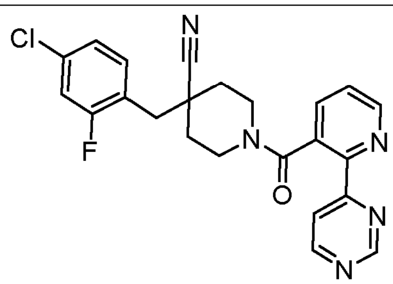
,

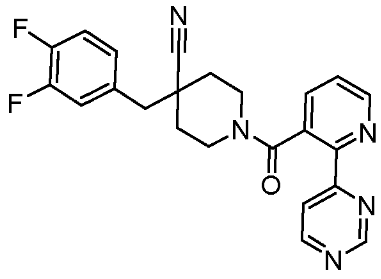
,

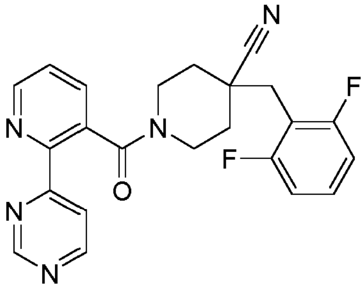
,

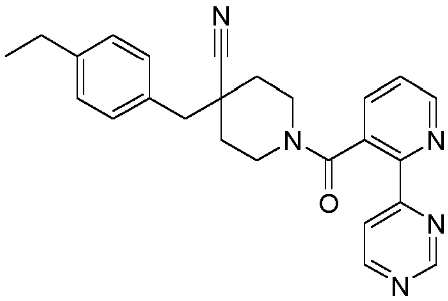
,

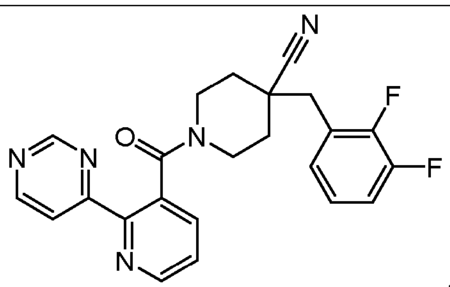
,

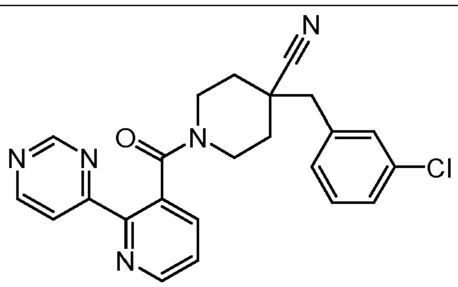
,

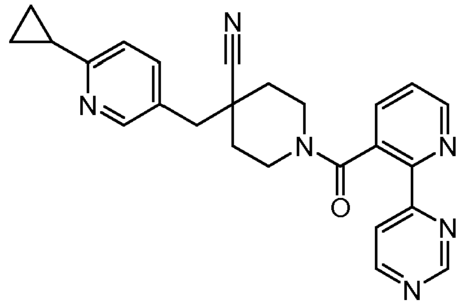
,

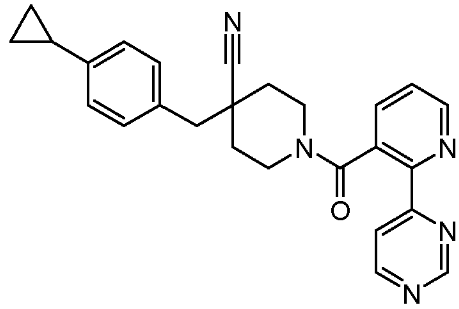
,

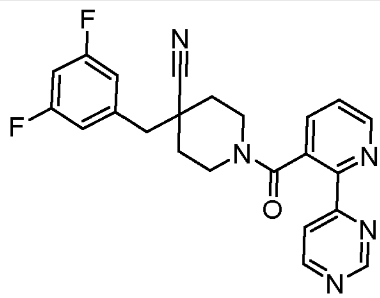
,

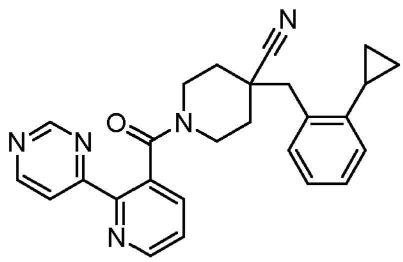
,

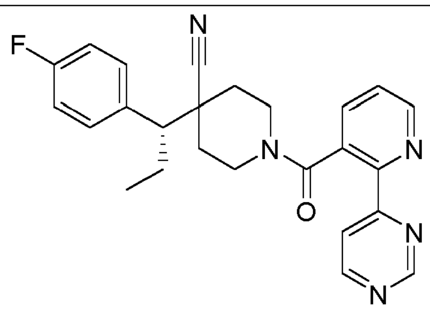
,

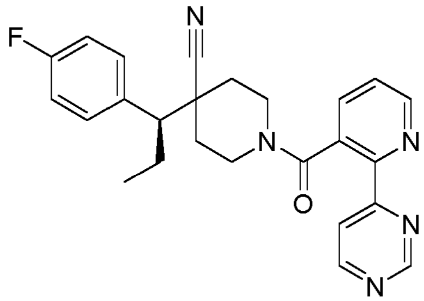
,

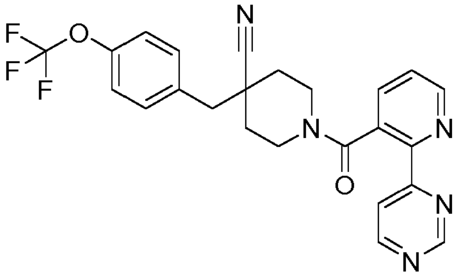
,

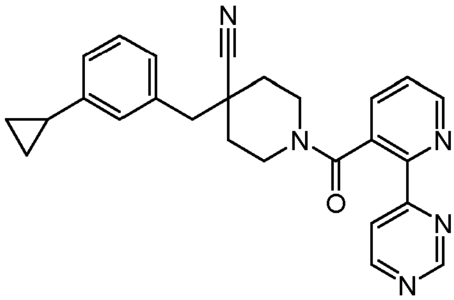
,

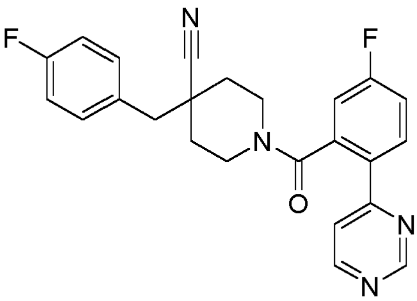
,

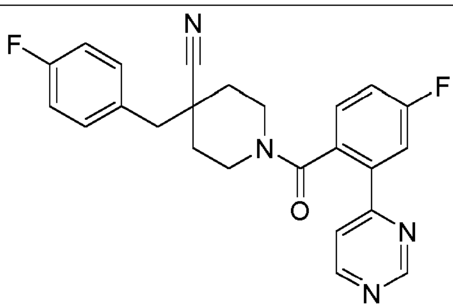
,

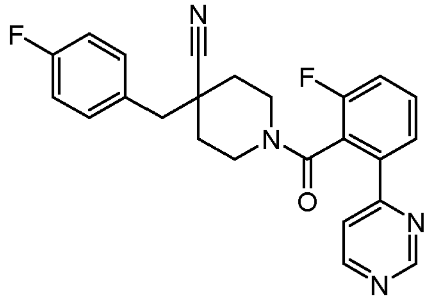
,

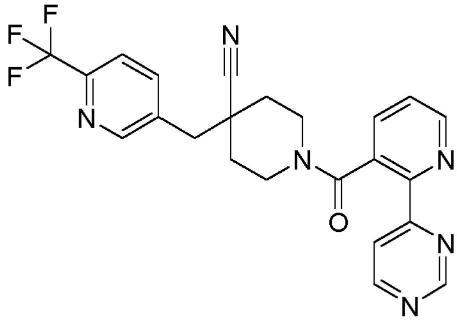
,

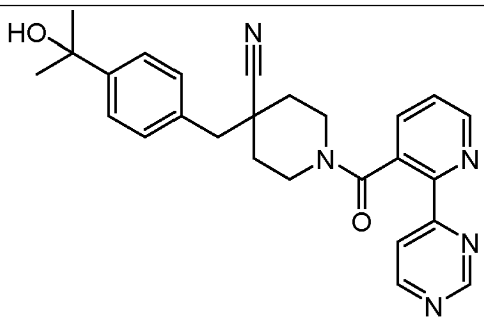
,

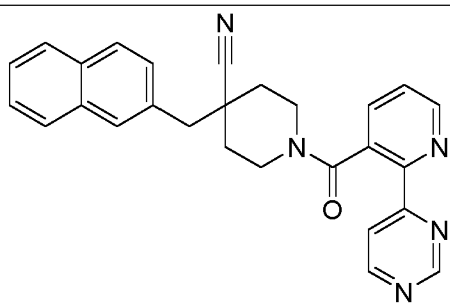
,

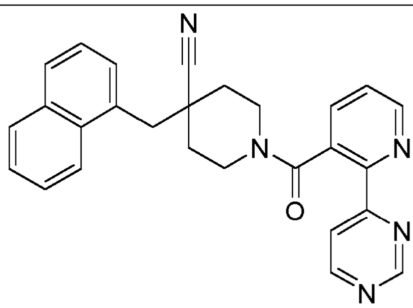
,

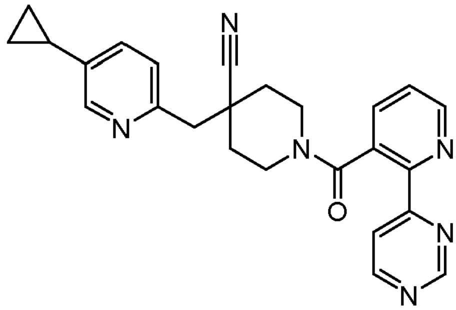 ,

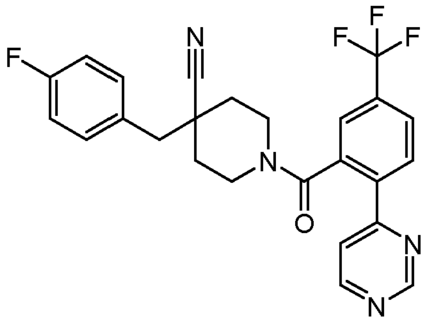 ,

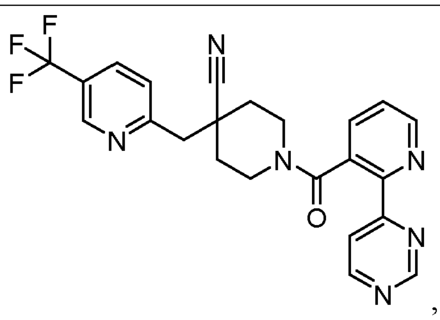 ,

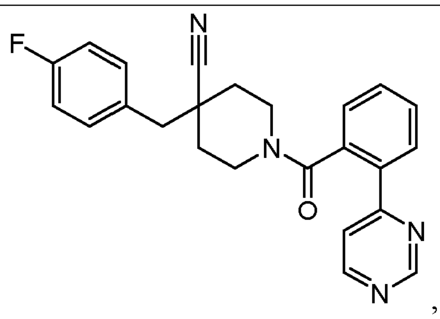 ,

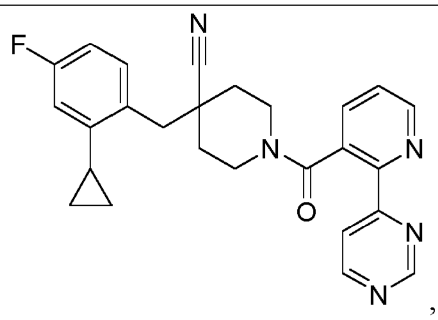 ,

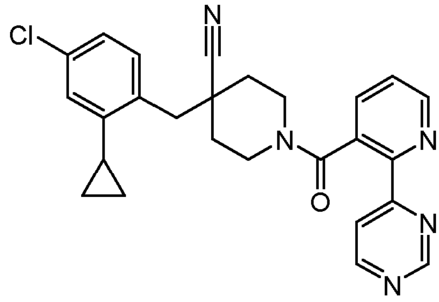 ,

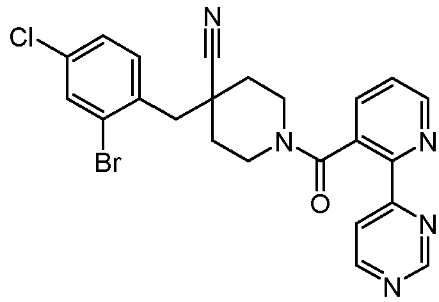 ,

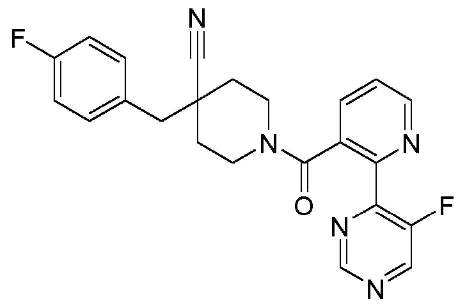 ,

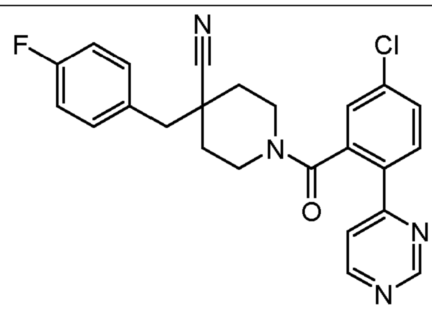 ,

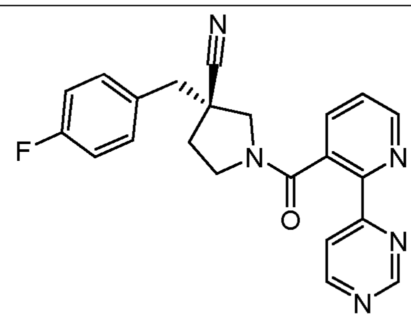 ,

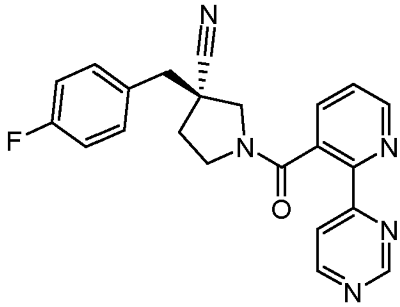
,

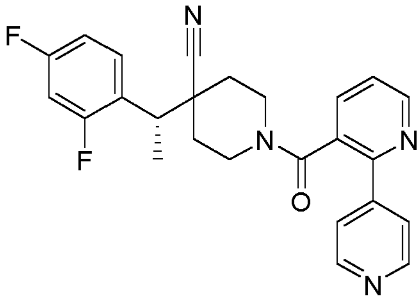
,

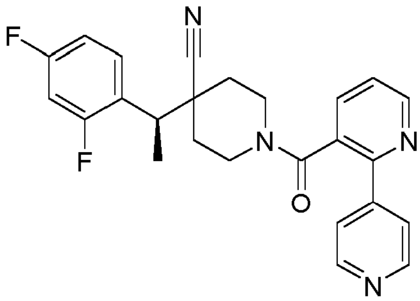
,

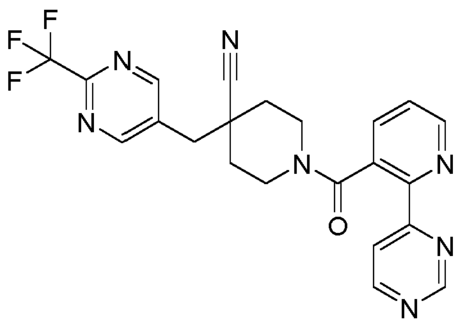
,

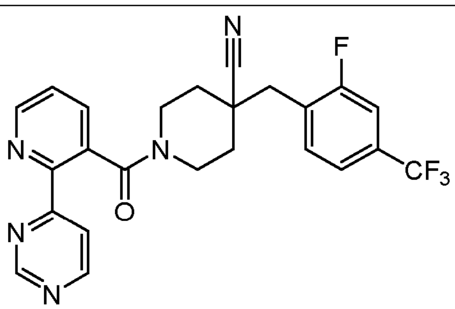
,

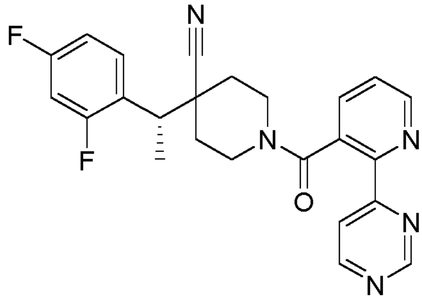
,

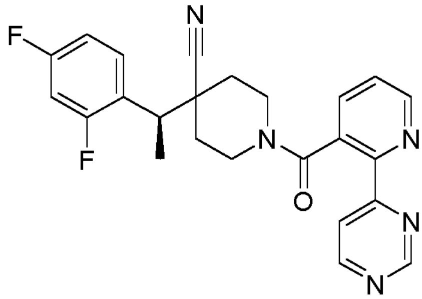
,

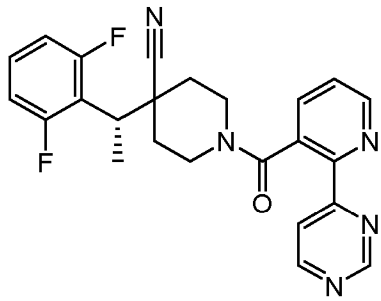
,

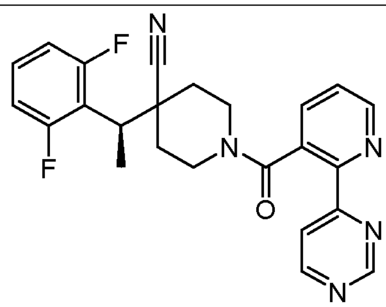
,

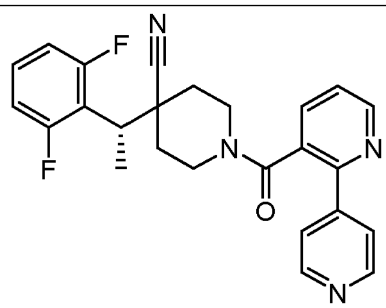
,

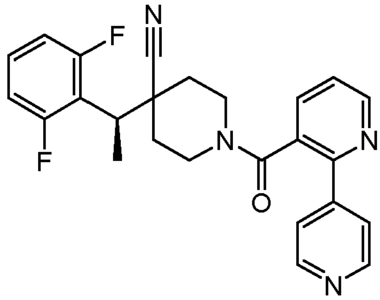
,

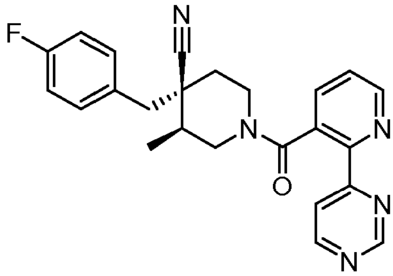
,

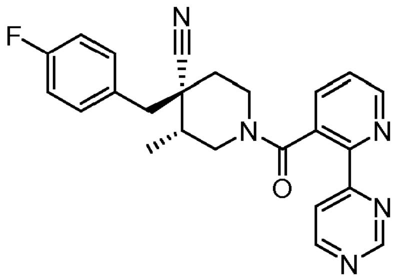
,

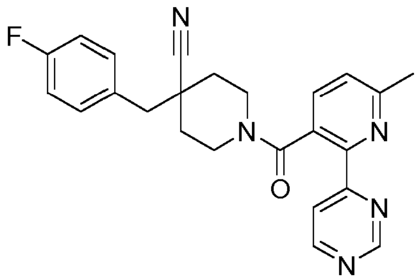
,

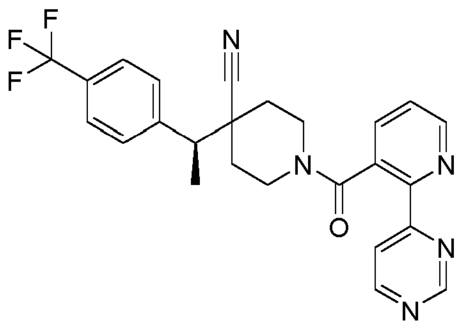
,

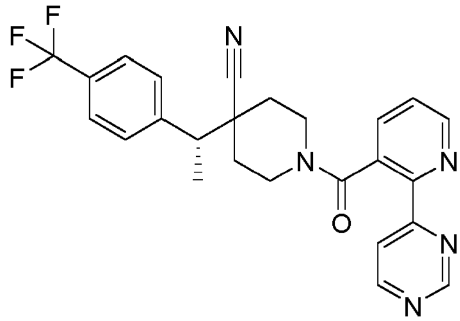
,

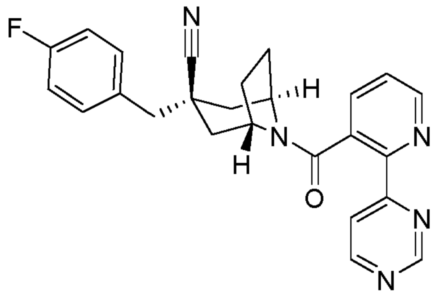
,

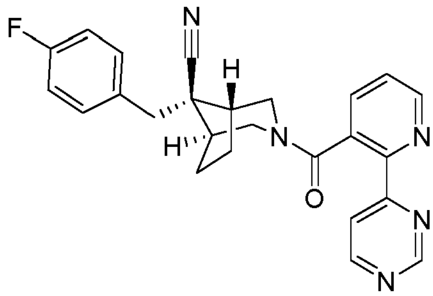
,

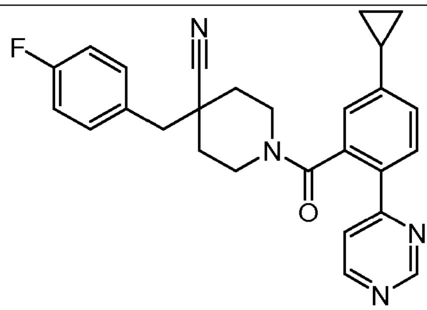
,

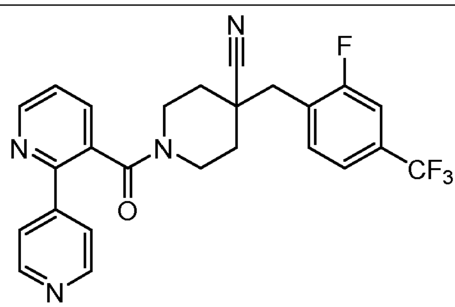
,

| | |
|---|---|
| 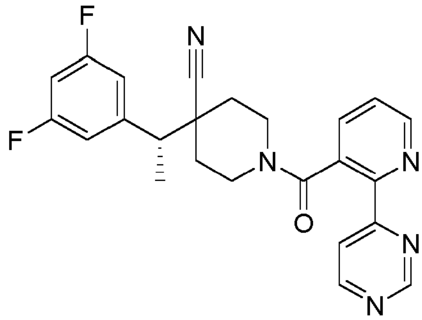 , | 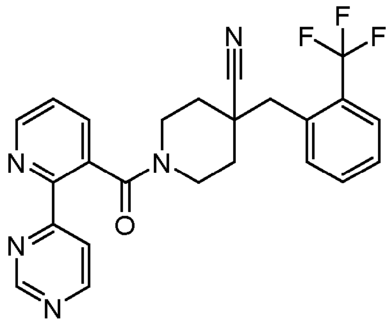 , |
| 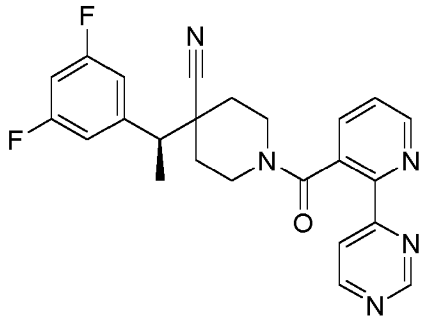 , | 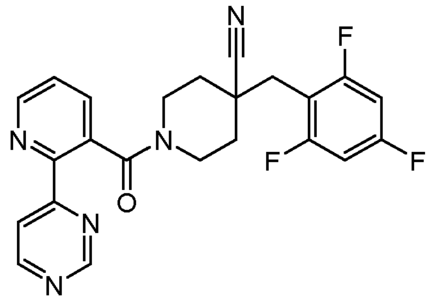 , |
| 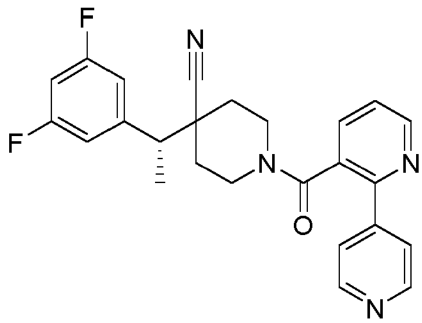 , | 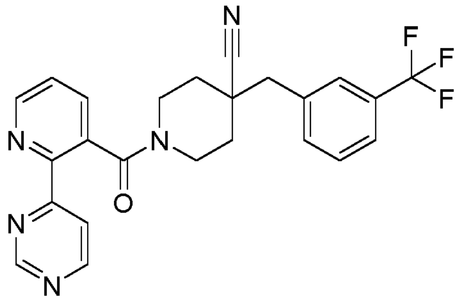 , |
| 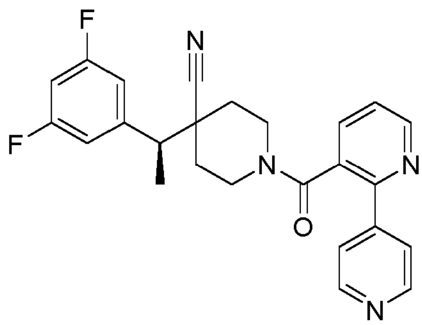 , | 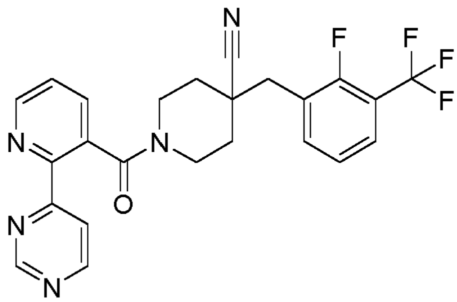 , |
| 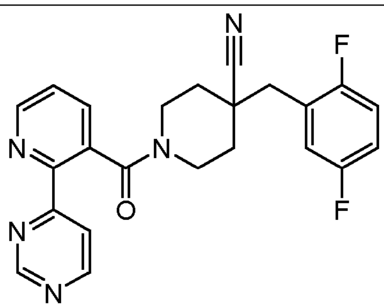 , | 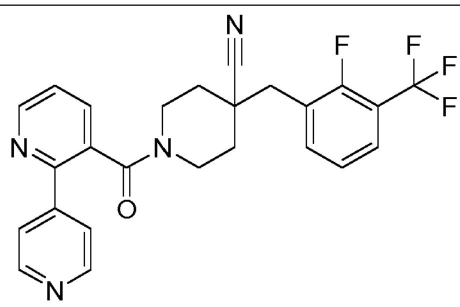 , |

| | |
|---|---|
| 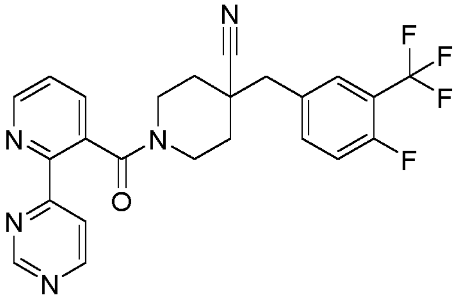 , | 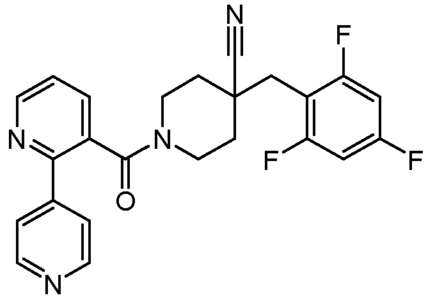 , |
| 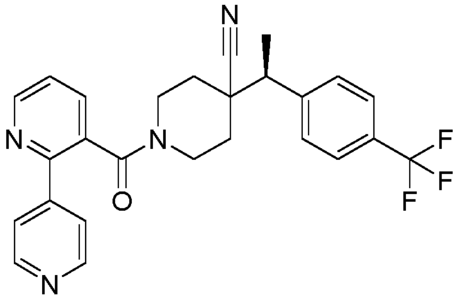 , | 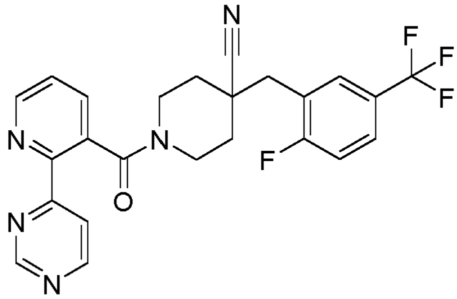 , |
| 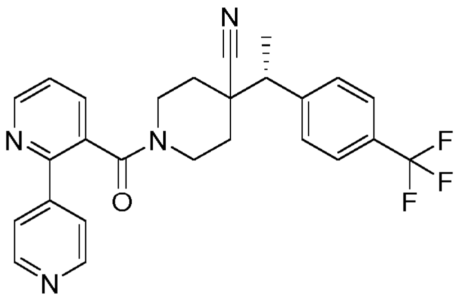 , | 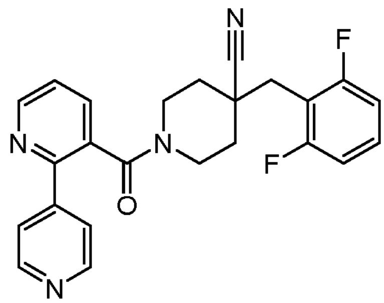 , |
| 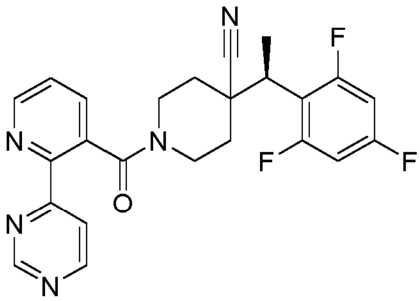 , | 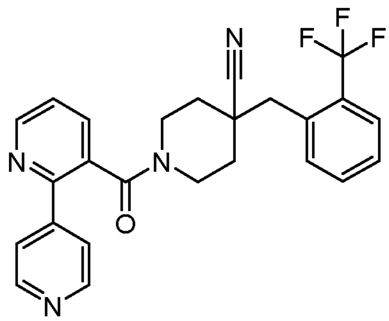 , |
| 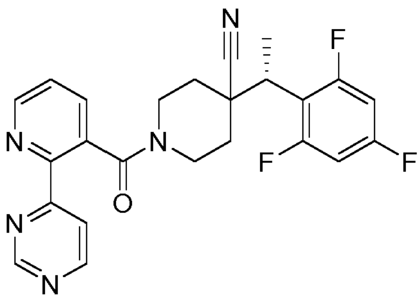 , | 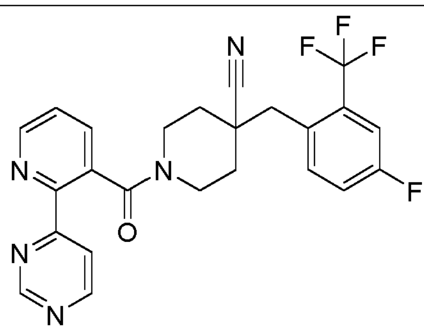 , |

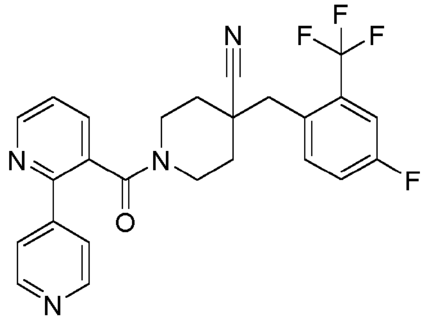
,

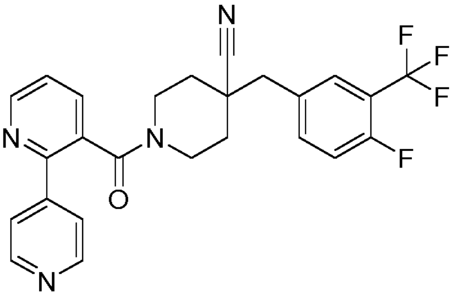
,

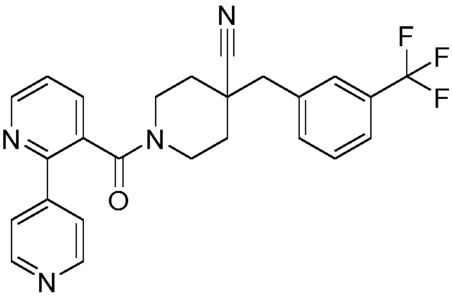
,

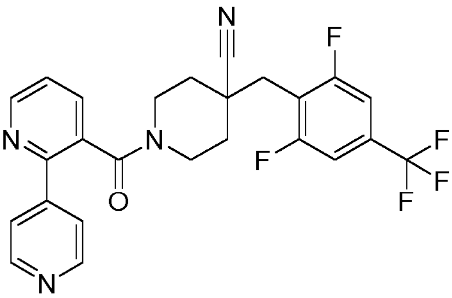
,

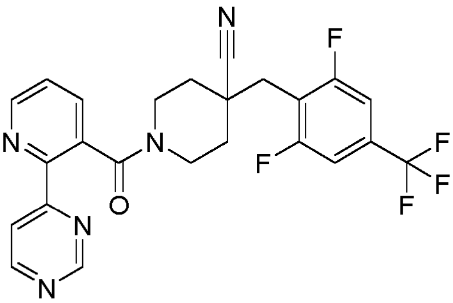
,

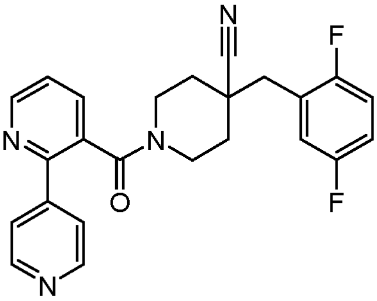
,

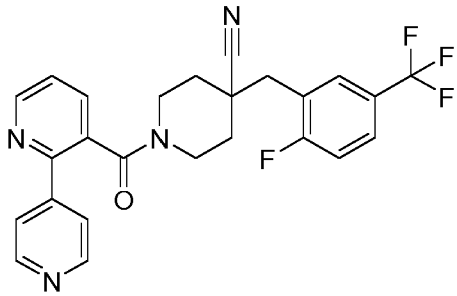
,

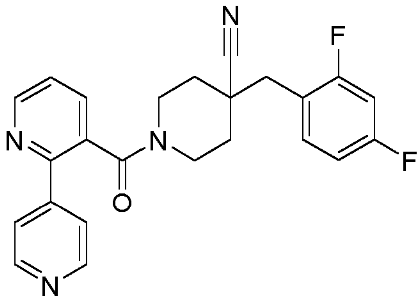
,

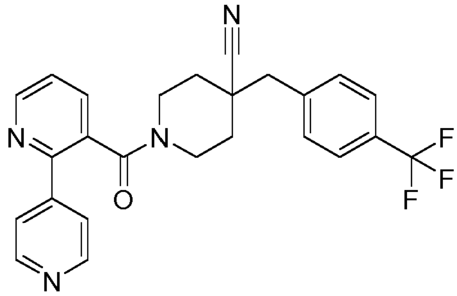
,

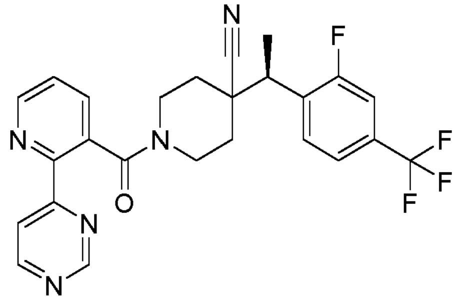
,

and

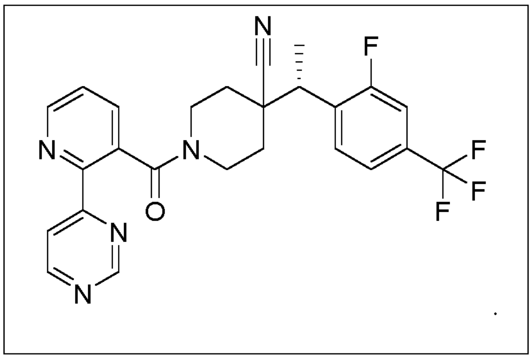

**28.** A pharmaceutical composition comprising a compound of any one of claims 1-27, or a pharmaceutically acceptable salt thereof, and a pharmaceutically acceptable carrier.

**29.** A compound of any one of claims 1-27, or a pharmaceutically acceptable salt thereof, or a pharmaceutical composition of claim 28, for use in treating a disease or disorder selected from the group consisting of a neurodegenerative disorder, epilepsy, developmental and epileptic encephalopathies, a psychiatric disorder, and spasm.

**30.** The compound, pharmaceutically acceptable salt, or pharmaceutical composition for use of claim 29, wherein the disease or disorder is a neurodegenerative disorder.

**31.** The compound, pharmaceutically acceptable salt, or pharmaceutical composition for use of claim 30, wherein the neurodegenerative disorder is selected from the group consisting of Alzheimer's disease, mild cognitive impairment, Huntington's disease, Parkinson's disease, amyotrophic lateral sclerosis, traumatic brain injury, cerebral infarction, glaucoma, and multiple sclerosis.

**32.** The compound, pharmaceutically acceptable salt, or pharmaceutical composition for use of claim 29, wherein the disease or disorder is epilepsy.

**33.** The compound, pharmaceutically acceptable salt, or pharmaceutical composition for use of claim 29, wherein the disease or disorder is developmental and epileptic encephalopathies.

**34.** The compound, pharmaceutically acceptable salt, or pharmaceutical composition for use of claim 29, wherein the disease or disorder is a psychiatric disorder.

**35.** The compound, pharmaceutically acceptable salt, or pharmaceutical composition for use of claim 34, wherein the psychiatric disorder is selected from the group consisting of schizophrenia, an autism spectrum disorder, a delusional disorder, a schizoaffective disorder, and depression.

**36.** The compound, pharmaceutically acceptable salt, or pharmaceutical composition for use of claim 29, wherein the disease or disorder is spasm.

**Patentansprüche**

**1.** Verbindung der Formel I:

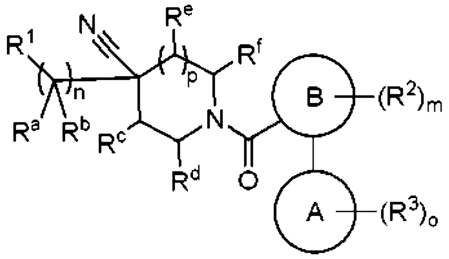

(I),

oder ein pharmazeutisch unbedenkliches Salz davon; wobei:

$R^1$ aus der Gruppe bestehend aus $C_6$-$C_{10}$-Aryl, $C_3$-$C_7$-Cycloalkyl, 3-7-gliedrigem Heterocyclyl und 5-10-gliedrigem Heteroaryl ausgewählt ist, wobei $R^1$ gegebenenfalls durch ein, zwei, drei oder vier Vorkommen von $R^4$ substituiert ist;
$R^a$ und $R^b$ jeweils unabhängig aus der Gruppe bestehend aus H, Halogen, -CN, -OH, $-NO_2$, $-N(R^5)_2$, $C_1$-$C_6$-Alkyl, $C_1$-$C_6$-Halogenalkyl, $C_1$-$C_6$-Alkoxy und $C_1$-$C_6$-Halogenalkoxy ausgewählt ist;
$R^c$, $R^d$, $R^e$ und $R^f$ jeweils unabhängig aus der Gruppe bestehend aus H, $C_1$-$C_6$-Alkyl, $C_1$-$C_6$-Halogenalkyl, $C_1$-$C_6$-Alkoxy und $C_1$-$C_6$-Halogenalkoxy ausgewählt sind; oder $R^c$ und $R^e$ zusammen mit den Kohlenstoffatomen, an die sie gebunden sind, eine $C_1$-$C_3$-Alkylenbrücke bilden können;
oder $R^d$ und $R^f$ zusammen mit den Kohlenstoffatomen, an die sie gebunden sind, eine $C_1$-$C_3$-Alkylenbrücke bilden können;
$R^4$ jeweils unabhängig aus der Gruppe bestehend aus Halogen, -CN, -OH, $-NO_2$, $-N(R^5)_2$, $-S(O)_2R^5$, $C_1$-$C_6$-Alkyl, $C_1$-$C_6$-Halogenalkyl, $C_1$-$C_6$-Alkoxy, $C_1$-$C_6$-Halogenalkoxy, $C_6$-$C_{10}$-Aryl, $C_3$-$C_7$-Cycloalkyl und 3-7-gliedrigem Heterocyclyl ausgewählt ist;
$R^5$ jeweils unabhängig aus H und $C_1$-$C_6$-Alkyl ausgewählt ist;
$R^2$ jeweils unabhängig aus der Gruppe bestehend aus Halogen, -CN, -OH, $-NO_2$, $-N(R^5)_2$, $C_1$-$C_6$-Alkyl, $C_3$-$C_7$-Cycloalkyl, $C_1$-$C_6$-Halogenalkyl, $C_1$-$C_6$-Alkoxy und $C_1$-$C_6$-Halogenalkoxy ausgewählt ist;
$R^3$ jeweils unabhängig aus der Gruppe bestehend aus Halogen, -CN, -OH, $-NO_2$, $-N(R^5)_2$, $C_1$-$C_6$-Alkyl, $C_1$-$C_6$-Halogenalkyl, $C_1$-$C_6$-Alkoxy, $C_3$-$C_7$-Cycloalkyl und $C_1$-$C_6$-Halogenalkoxy ausgewählt ist;
A für ein 5-6-gliedriges stickstoffhaltiges Heteroaryl steht;
B aus $C_6$-$C_{10}$-Aryl und 5-6-gliedrigem Heteroaryl ausgewählt ist;
m für 0, 1, 2 oder 3 steht;
n für 1, 2, 3 oder 4 steht;
o für 0, 1, 2 oder 3 steht; und
p für 0, 1 oder 2 steht.

**2.** Verbindung nach Anspruch 1 oder ein pharmazeutisch unbedenkliches Salz davon, wobei:

(a) es sich bei der Verbindung der Formel I um eine Verbindung der Formel I-a handelt:

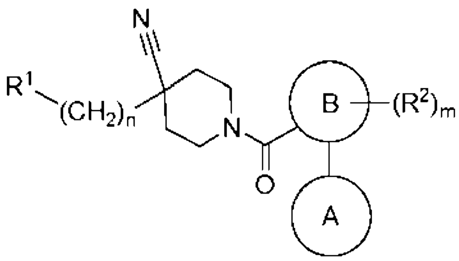

(I-a);

(b) es sich bei der Verbindung der Formel I um eine Verbindung der Formel I-b handelt:

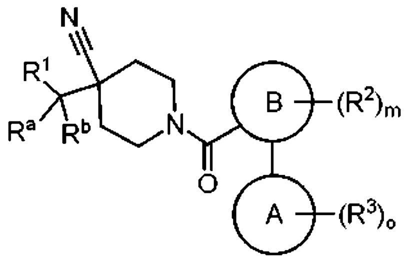

(I-b);

(c) es sich bei der Verbindung der Formel I um eine Verbindung der Formel I-c handelt:

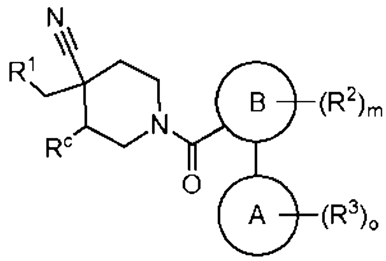

(I-c);

(d) es sich bei der Verbindung der Formel I um eine Verbindung der Formel I-d handelt:

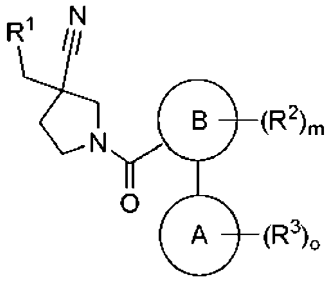

(I-d);

(e) es sich bei der Verbindung der Formel I um eine Verbindung der Formel I-e handelt:

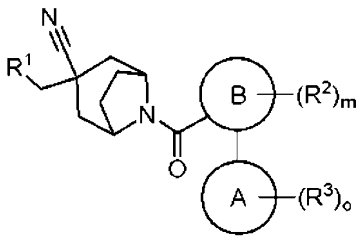

(I-e);

oder

(f) es sich bei der Verbindung der Formel I um eine Verbindung der Formel I-f handelt:

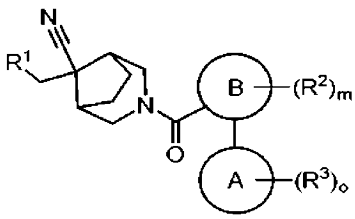

(I-f).

**3.** Verbindung nach Anspruch 1 oder 2 oder ein pharmazeutisch unbedenkliches Salz davon, wobei:

(a) $R^1$ für $C_6$-$C_{10}$-Aryl steht, das durch ein, zwei, drei oder vier Vorkommen von $R^4$ substituiert ist;
(b) $R^1$ für unsubstituiertes $C_6$-$C_{10}$-Aryl steht;
(c) $R^1$ für 5-10-gliedriges Heteroaryl steht, das durch ein, zwei, drei oder vier Vorkommen von $R^4$ substituiert ist;
(d) $R^1$ für unsubstituiertes 5-10-gliedriges Heteroaryl steht;
(e) $R^1$ für $C_3$-$C_7$-Cycloalkyl steht, das durch ein, zwei, drei oder vier Vorkommen von $R^4$ substituiert ist;
(f) $R^1$ für unsubstituiertes $C_3$-$C_7$-Cycloalkyl steht;
(g) $R^1$ für 3-7-gliedriges Heterocyclyl steht, das durch ein, zwei, drei oder vier Vorkommen von $R^4$ substituiert ist; oder
(h) $R^1$ für unsubstituiertes 3-7-gliedriges Heterocyclyl steht.

**4.** Verbindung nach Anspruch 1 oder 2 oder ein pharmazeutisch unbedenkliches Salz davon, wobei:

(a) $R^1$ für

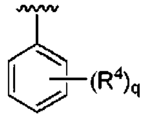

steht, wobei $R^4$ jeweils unabhängig für Halogen, -CN, -OH, $-NO_2$, $-N(R^5)_2$, $-S(O)_2R^5$, $C_1$-$C_6$-Alkyl, $C_1$-$C_6$-Halogenalkyl, $C_1$-$C_6$-Alkoxy, $C_1$-$C_6$-Halogenalkoxy, $C_6$-$C_{10}$-Aryl, $C_3$-$C_7$-Cycloalkyl oder 3-7-gliedriges Heterocyclyl steht; wobei $R^5$ jeweils unabhängig für H oder $C_1$-$C_6$-Alkyl steht; und q für 0, 1, 2 oder 3 steht;

(b) $R^1$ für

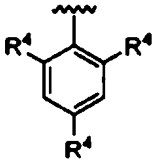

steht;

(c) $R^1$ für

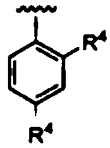 , 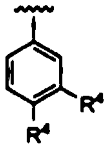 , 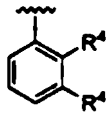

oder

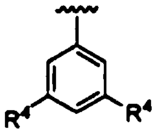

steht;

(d) $R^1$ für

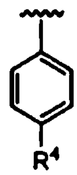

steht;

(e) $R^1$ für

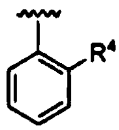

steht;

(f) $R^1$ für

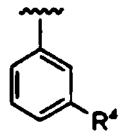

steht; oder

(g) $R^1$ für

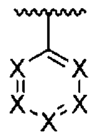

steht, wobei X jeweils unabhängig für CH oder N steht, wobei das H von CH durch ein oder mehrere Vorkommen von $R^4$ ersetzt sein kann; wobei $R^4$ jeweils unabhängig für Halogen, -CN, -OH, $-NO_2$, $-N(R^5)_2$, $C_1$-$C_6$-Alkyl, $C_1$-$C_6$-Halogenalkyl, $C_1$-$C_6$-Alkoxy, $C_1$-$C_6$-Halogenalkoxy, $C_6$-$C_{10}$-Aryl, $C_3$-$C_7$-Cycloalkyl oder 3-7-gliedriges Heterocyclyl steht und $R^5$ jeweils unabhängig für H oder $C_1$-$C_6$-Alkyl steht.

**5.** Verbindung nach Anspruch 1 oder 2 oder ein pharmazeutisch unbedenkliches Salz davon, wobei:

(a) $R^1$ für Pyrrolyl, Furanyl, Thiophenyl, Imidazolyl, Pyrazolyl, Oxazolyl, Isoxazolyl, Thiazolyl, Isothiazolyl, Triazolyl, Oxadiazolyl, Thiadiazolyl, Pyridyl, 2-Pyridyl, 3-Pyridyl, 4-Pyridyl, Pyridinyl, Pyridazinyl, Pyrimidinyl, 2-Pyrimidinyl, 4-Pyrimidinyl, Pyrazinyl, Triazinyl, Tetrazinyl, Tetrazolyl, Azocinyl, Dithiazinyl oder Oxazinyl steht;

(b) $R^1$ für Pyridyl, 2-Pyridyl, 3-Pyridyl, 4-Pyridyl, Pyridinyl, Pyridazinyl, Pyrimidinyl, 2-Pyrimidinyl, 4-Pyrimidinyl, Pyridazinyl, oder Pyrazinyl steht;

(c) $R^1$ für Cyclopropyl oder Cyclobutyl steht;

(d) $R^1$ für Tetrahydrofuran, Tetrahydropyran, Pyrrolidin, Piperidin, Piperazin, Dioxolan, Dioxan, Thiomorpholin oder Dithian steht; oder

(e) $R^1$ für Tetrahydrofuran oder Tetrahydropyran steht.

**6.** Verbindung nach Anspruch 1 oder 2 oder ein pharmazeutisch unbedenkliches Salz davon, wobei:

(a) $R^1$ für

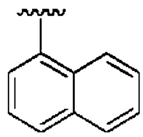

oder

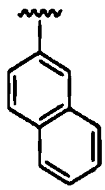

steht;

(b) $R^1$ für

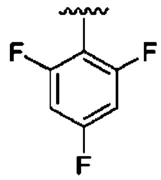

oder

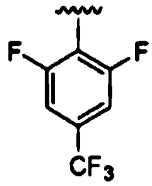

steht;
(c) $R^1$ für

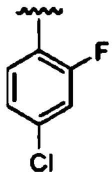, 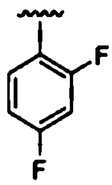, 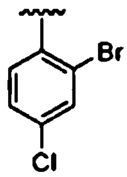, 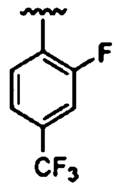, 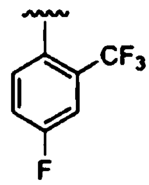, 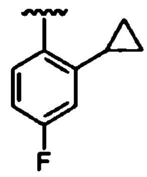

oder

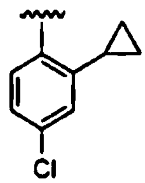

steht;
(d) $R^1$ für

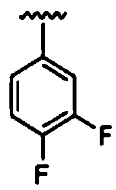, 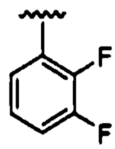, 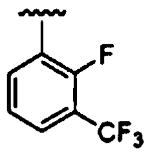

oder

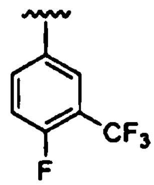

steht;
(e) $R^1$ für

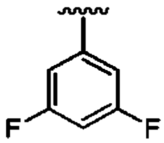

oder

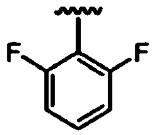

steht;

(f) $R^1$ für

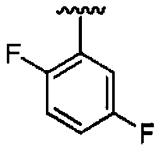

oder

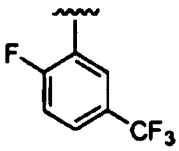

steht;

(g) $R^1$ für

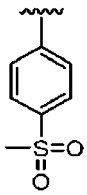, 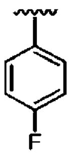, 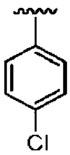, 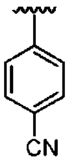, 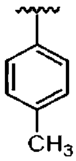, 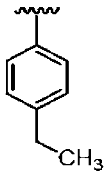, 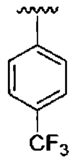, 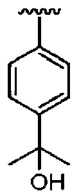, 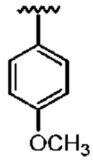, 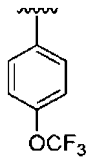,

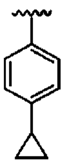, 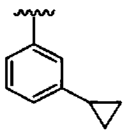, 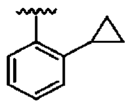, 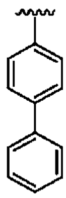, 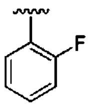, 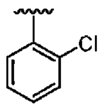, 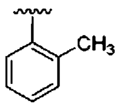, 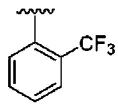,

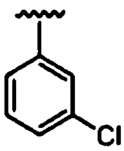, 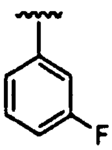

oder

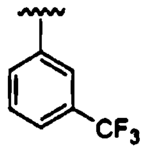

steht;
(h) $R^1$ für

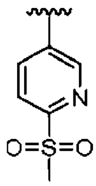 , 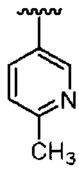 , 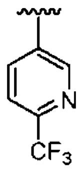 , 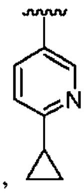 , 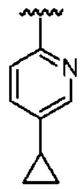 , 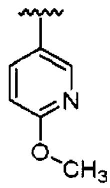 , 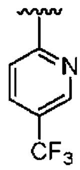 , 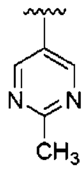 ,

oder

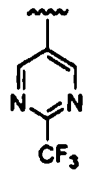

steht;
(i) $R^1$ für

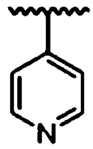 , 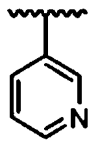

oder

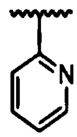

steht;
(j) $R^1$ für

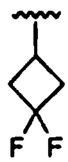

steht;
(k) $R^1$ für

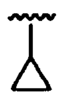

steht; oder

(l) $R^1$ für

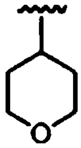

steht.

7. Verbindung nach Anspruch 3 oder ein pharmazeutisch unbedenkliches Salz davon, wobei $R^1$ für $C_6$-$C_{10}$-Aryl steht, das gegebenenfalls durch ein, zwei, drei oder vier Vorkommen von $R^4$ substituiert ist.

8. Verbindung nach Anspruch 3 oder ein pharmazeutisch unbedenkliches Salz davon, wobei $R^1$ für 5-10-gliedriges Heteroaryl steht, das gegebenenfalls durch ein, zwei, drei oder vier Vorkommen von $R^4$ substituiert ist.

9. Verbindung nach Anspruch 3 oder ein pharmazeutisch unbedenkliches Salz davon, wobei $R^1$ für $C_3$-$C_7$-Cycloalkyl steht, das gegebenenfalls durch ein, zwei, drei oder vier Vorkommen von $R^4$ substituiert ist.

10. Verbindung nach Anspruch 3 oder ein pharmazeutisch unbedenkliches Salz davon, wobei $R^1$ für 3-7-gliedriges Heterocyclyl steht, das gegebenenfalls durch ein, zwei, drei oder vier Vorkommen von $R^4$ substituiert ist.

11. Verbindung nach einem der Ansprüche 1-10 oder ein pharmazeutisch unbedenkliches Salz davon, wobei:

(a) $R^4$ jeweils unabhängig für substituiertes $C_1$-$C_6$-Alkyl, substituiertes $C_1$-$C_6$-Alkoxy oder substituiertes $C_3$-$C_7$-Cycloalkyl steht;
(b) $R^4$ jeweils unabhängig für unsubstituiertes $C_1$-$C_6$-Alkyl, unsubstituiertes $C_1$-$C_6$-Alkoxy oder unsubstituiertes $C_3$-$C_7$-Cycloalkyl steht;
(c) $R^4$ jeweils unabhängig für Halogen, $-NH_2$, $-NH(C_1$-$C_6$-Alkyl), $-N(C_1$-$C_6$-Alkyl$)_2$, $C_1$-$C_6$-Alkyl, $C_1$-$C_6$-Halogenalkyl, $C_1$-$C_6$-Alkoxy, $C_1$-$C_6$-Halogenalkoxy oder $C_6$-$C_{10}$-Aryl steht; oder
(d) $R^4$ jeweils unabhängig für Halogen, $C_1$-$C_6$-Alkyl, $C_1$-$C_6$-Halogenalkyl, $C_1$-$C_6$-Alkoxy, $C_1$-$C_6$-Halogenalkoxy oder $C_6$-$C_{10}$-Aryl steht.

12. Verbindung nach einem der Ansprüche 1-10 oder ein pharmazeutisch unbedenkliches Salz davon, wobei:

(a) $R^4$ jeweils unabhängig für Halogen, -CN, $-CH_3$, - $CH_2CH_3$, $-CF_3$, $-OCH_3$, $-OCF_3$, $-C(CH_3)_2OH$ oder $-C_6H_5$ steht; oder
(b) $R^4$ jeweils unabhängig für Cl, F, Br oder I steht.

13. Verbindung nach einem der Ansprüche 1-12 oder ein pharmazeutisch unbedenkliches Salz davon, wobei:

(a) n für 4 steht;
(b) n für 3 steht;
(c) n für 2 steht; oder
(d) n für 1 steht.

14. Verbindung nach Anspruch 13 oder ein pharmazeutisch unbedenkliches Salz davon, wobei n für 1 steht.

15. Verbindung nach einem der Ansprüche 1-14 oder ein pharmazeutisch unbedenkliches Salz davon, wobei:

(a) n für 1 steht und $R^a$ für $C_1$-$C_6$-Alkyl steht und $R^b$ für H steht;
(b) n für 1 steht und $R^a$ für Ethyl steht und $R^b$ für H steht; oder
(c) n für 1 steht und $R^a$ für Methyl steht und $R^b$ für H steht.

16. Verbindung nach einem der Ansprüche 1-15 oder ein pharmazeutisch unbedenkliches Salz davon, wobei:

(a) p für 2 steht;
(b) p für 1 steht; oder

(c) p für 0 steht.

**17.** Verbindung nach Anspruch 16 oder ein pharmazeutisch unbedenkliches Salz davon, wobei:

(a) p für 1 steht und $R^c$, $R^d$, $R^e$ und $R^f$ für H stehen;
(b) p für 1 steht, $R^c$ für Methyl steht und $R^d$, $R^e$ und $R^f$ für H stehen;
(c) p für 1 steht, $R^c$ und $R^e$ für H stehen und $R^d$ und $R^f$ zusammen mit den Kohlenstoffatomen, an die sie gebunden sind, eine $C_1$-$C_3$-Alkylenbrücke bilden;
(d) p für 1 steht, $R^d$ und $R^f$ für H stehen und $R^c$ und $R^e$ zusammen mit den Kohlenstoffatomen, an die sie gebunden sind, eine C1-C3-Alkylenbrücke bilden; oder
(e) p für 0 steht und $R^c$, $R^d$ und $R^f$ für H stehen.

**18.** Verbindung nach einem der Ansprüche 1-17 oder ein pharmazeutisch unbedenkliches Salz davon, wobei:

(a) B für

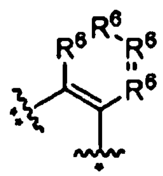

steht, wobei $R^6$ jeweils unabhängig für N oder $CR^{6a}$ steht, wobei $R^{6a}$ für H oder $R^2$ steht und ** für den Punkt der Verknüpfung mit dem Carbonyl steht und * für den Punkt der Verknüpfung mit A steht; oder
(b) B für

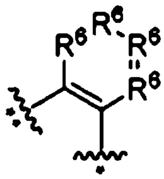

steht, wobei bis zu zwei $R^6$ für N stehen können und die anderen Vorkommen von $R^6$ für CH stehen und ** für den Punkt der Verknüpfung mit dem Carbonyl steht und * für den Punkt der Verknüpfung mit A steht.

**19.** Verbindung nach einem der Ansprüche 1-17 oder ein pharmazeutisch unbedenkliches Salz davon, wobei:

(a) B für

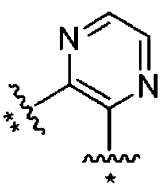 , 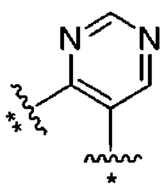 , 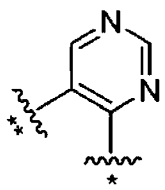 , 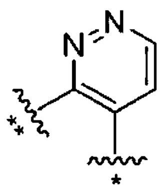 , 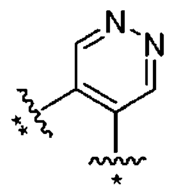 ,

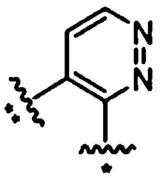 , 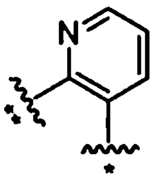 , 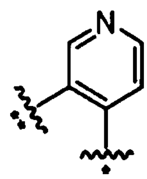 , 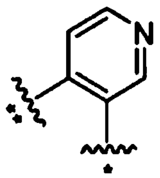 , 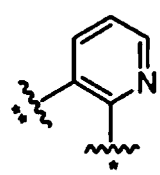

oder

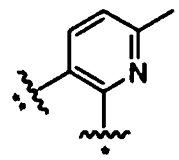

steht, wobei ** für den Punkt der Verknüpfung mit dem Carbonyl steht und * für den Punkt der Verknüpfung mit A steht;

(b) B für

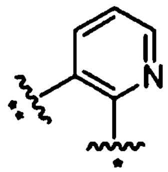

steht, wobei ** für den Punkt der Verknüpfung mit dem Carbonyl steht und * für den Punkt der Verknüpfung mit A steht; oder

(c) B für

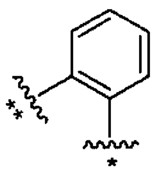 , 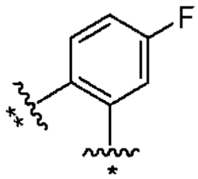 , 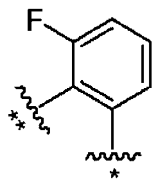 , 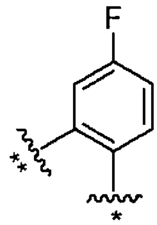 , 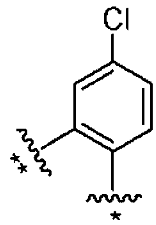 ,

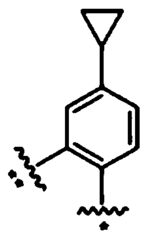

oder

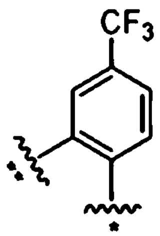

steht, wobei ** für den Punkt der Verknüpfung mit dem Carbonyl steht und * für den Punkt der Verknüpfung mit A steht.

**20.** Verbindung nach Anspruch 19 oder ein pharmazeutisch unbedenkliches Salz davon, wobei B für

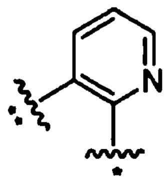

steht, wobei ** für den Punkt der Verknüpfung mit dem Carbonyl steht und * für den Punkt der Verknüpfung mit A steht.

**21.** Verbindung nach Anspruch 19 oder ein pharmazeutisch unbedenkliches Salz davon, wobei B für

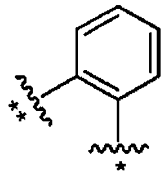 , 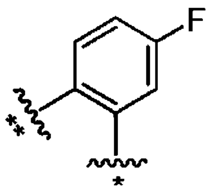 , 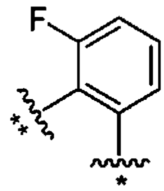 , 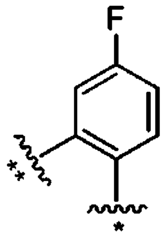 , 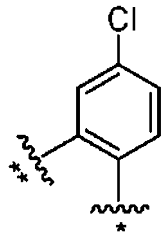 ,

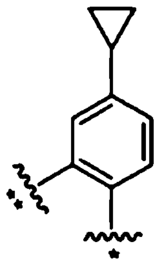

oder

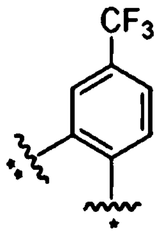

steht, wobei ** für den Punkt der Verknüpfung mit dem Carbonyl steht und * für den Punkt der Verknüpfung mit A steht.

**22.** Verbindung nach einem der Ansprüche 1-21 oder ein pharmazeutisch unbedenkliches Salz davon, wobei:

(a) m für 1 steht; oder
(b) m für 0 steht.

**23.** Verbindung nach einem der Ansprüche 1-22 oder ein pharmazeutisch unbedenkliches Salz davon, wobei $R^2$ für Halogen, -CN, -OH, $-NO_2$, $-CH_3$, $-CH_2CH_3$, Cyclopropyl, - $CHF_2$, $-CF_3$, $-OCF_3$, $-OCH_3$, $-OCH_2CH_3$ oder $-OCH_2CF_3$ steht.

**24.** Verbindung nach einem der Ansprüche 1-23 oder ein pharmazeutisch unbedenkliches Salz davon, wobei:

(a) A für Pyridinyl, Pyrrolyl, Imidazolyl, Pyrazolyl, Pyridazinyl, Pyrimidinyl, Pyrazinyl, Triazolyl, Triazinyl, Tetrazinyl, Tetrazolyl, Oxazolyl, Isoxazolyl oder Thiozolyl steht; oder
(b) A für Pyridinyl, Oxazolyl, Imidazolyl, Triazolyl, Pyridinyl, Pyridazinyl, Pyrimidinyl oder Triazinyl steht.

**25.** Verbindung nach einem der Ansprüche 1-23 oder ein pharmazeutisch unbedenkliches Salz davon, wobei A für

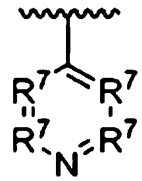

steht, wobei $R^7$ jeweils unabhängig für N oder CH steht, wobei bis zu zwei $R^7$ für N stehen können und die anderen Vorkommen von $R^7$ für CH stehen, wobei der Wasserstoff von CH durch $R^3$ ersetzt sein kann.

**26.** Verbindung nach einem der Ansprüche 1-23 oder ein pharmazeutisch unbedenkliches Salz davon, wobei:

(a) A für

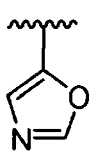, 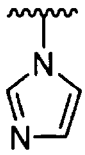, 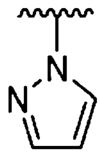, 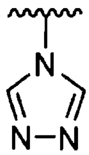, 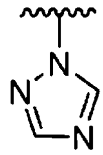, 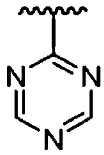, 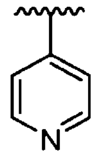, 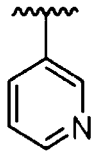, 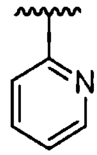,

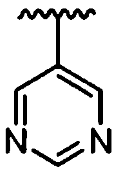, 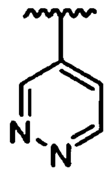, 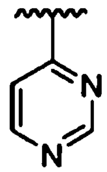

oder

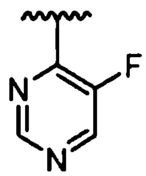

steht;

(b) A für

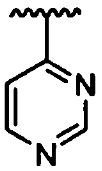, 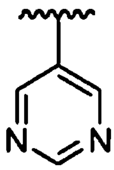, 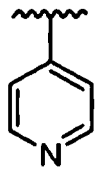, 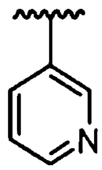, 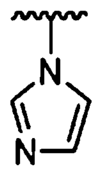, 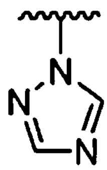, 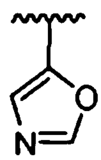

oder

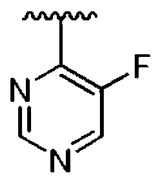

steht.

**27.** Verbindung nach einem der Ansprüche 1-26 oder ein pharmazeutisch unbedenkliches Salz davon, wobei die Verbindung ausgewählt ist aus der Gruppe bestehend aus:

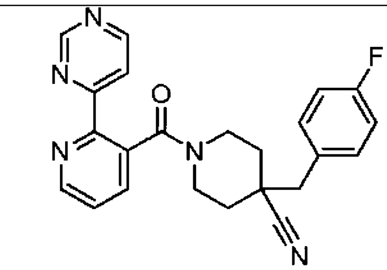
,

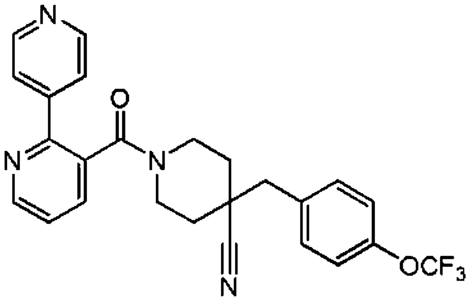
,

| |
|---|
| 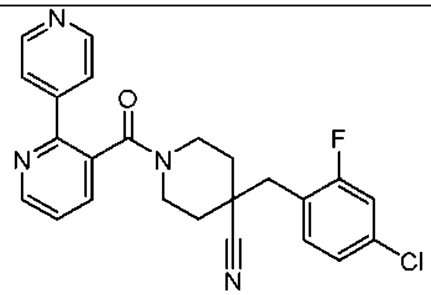 , |
| 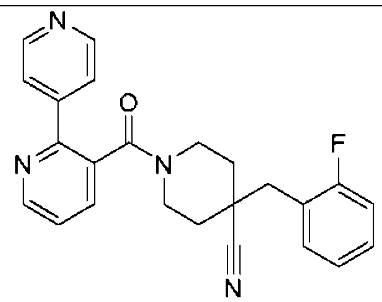 , |
| 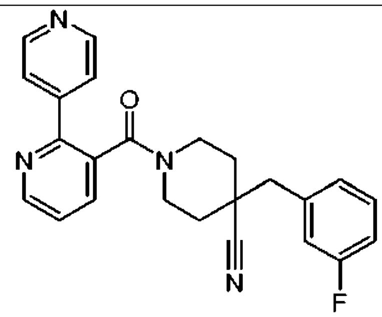 , |
| 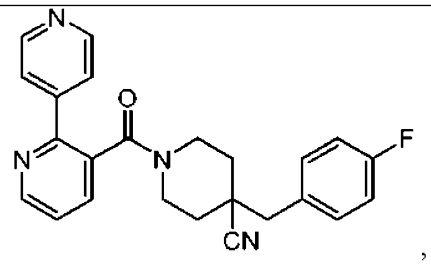 , |
| 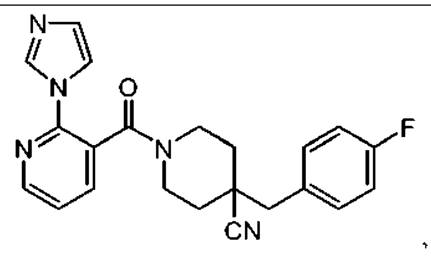 , |
| 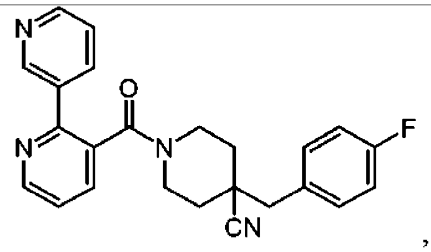 , |

| |
|---|
| 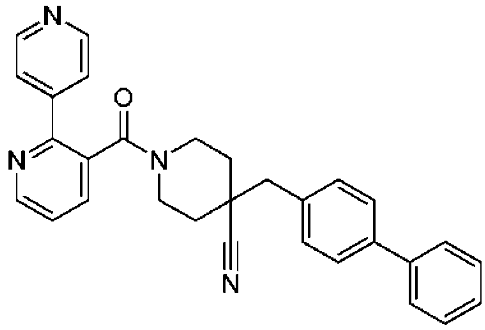 , |
| 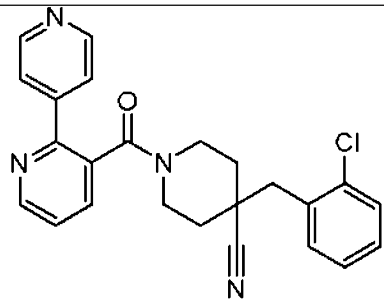 , |
| 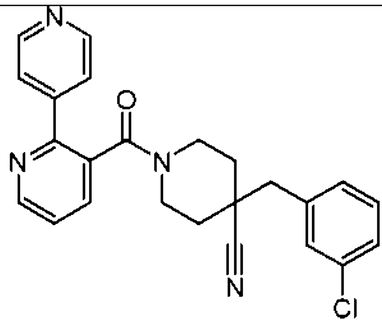 , |
| 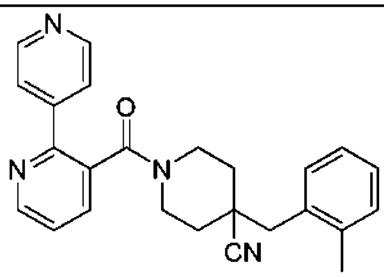 , |
| 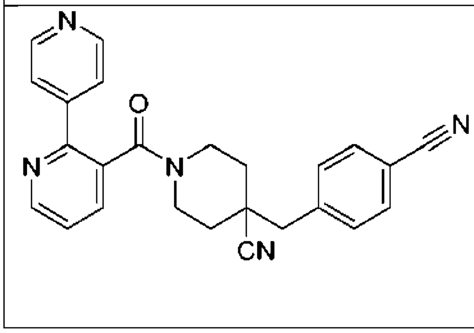 , |

| |
|---|
| 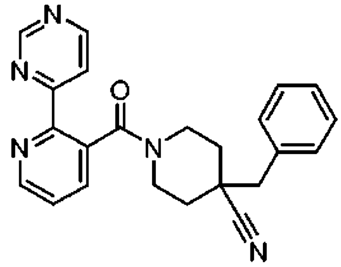 , |
| 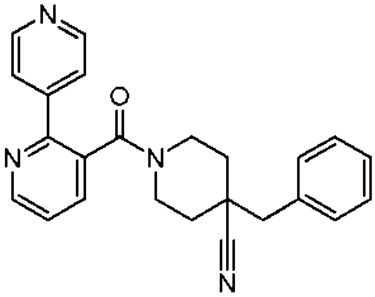 , |
| 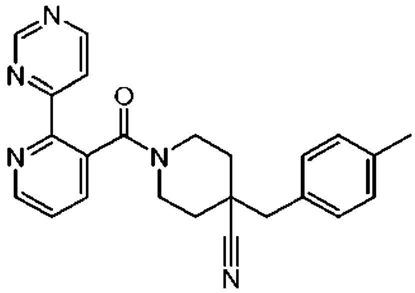 , |
| 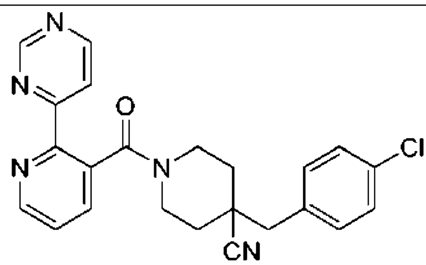 , |
| 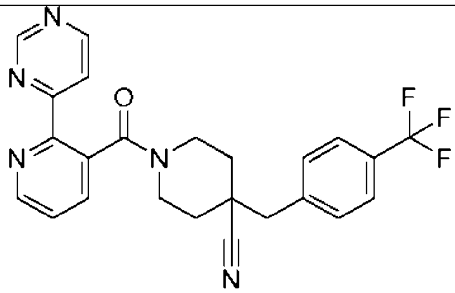 , |
| 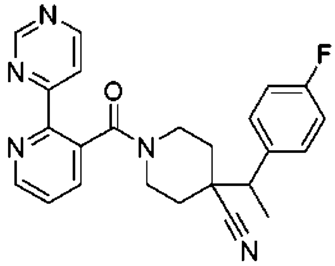 , |

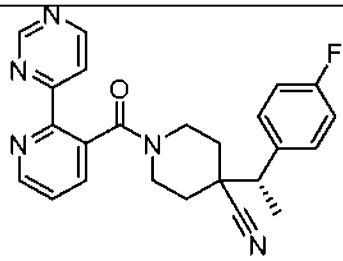
,

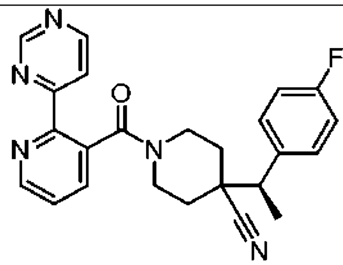
,

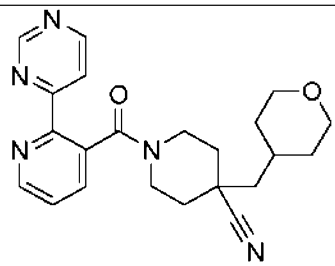
,

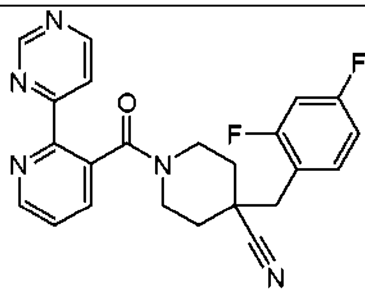
,

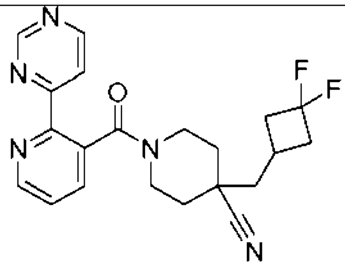
,

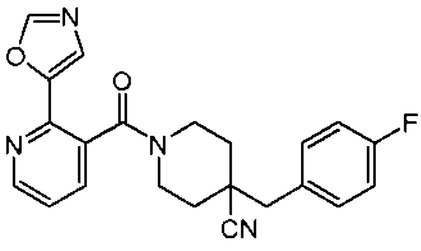
,

| |
|---|
| 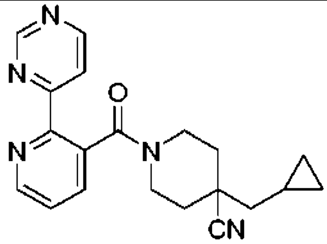 , |
| 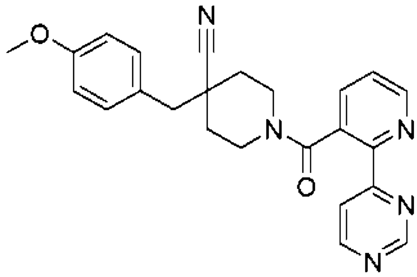 , |
| 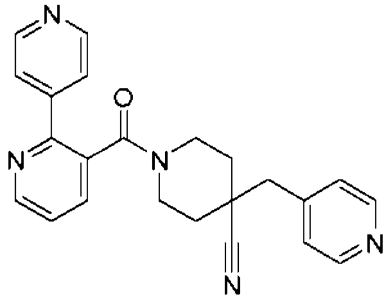 , |
| 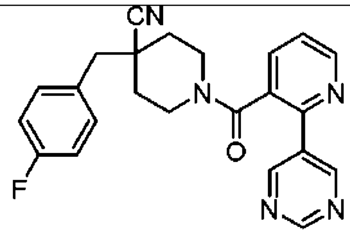 , |
| 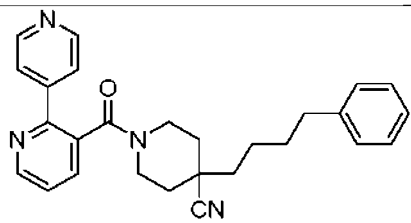 , |
| 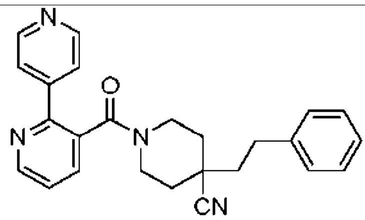 , |

| |
|---|
| 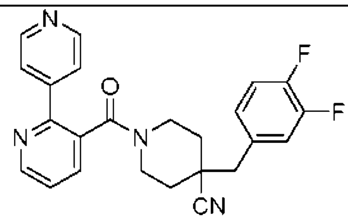 , |
| 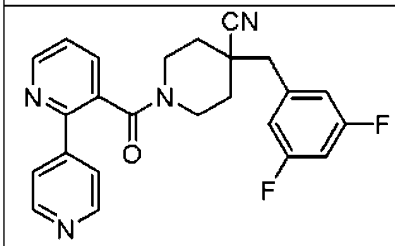 , |
| 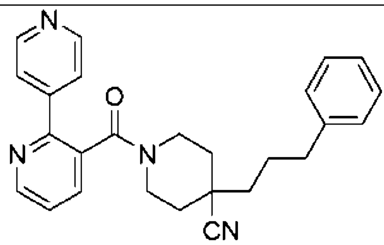 , |
| 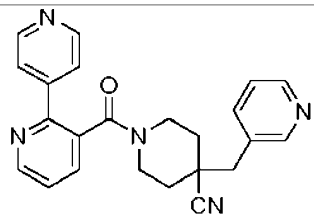 , |
| 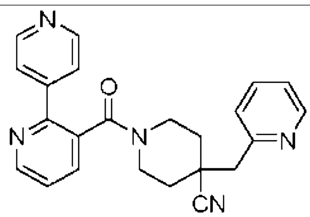 , |
| 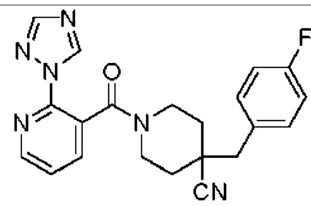 , |
| 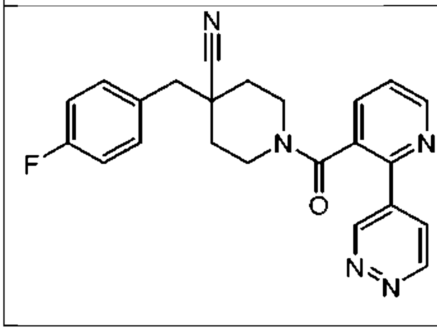 , |

| |
|---|
| 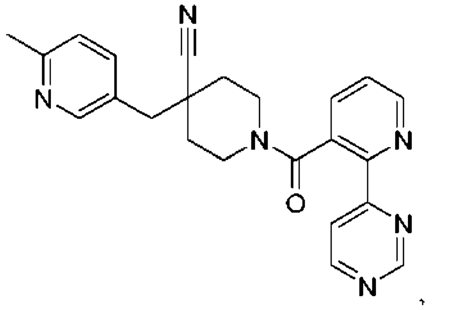 , |
| 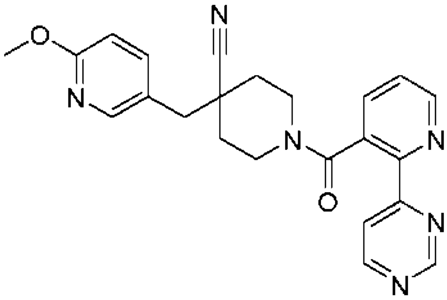 , |
| 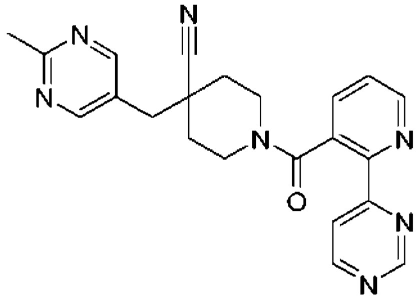 , |
| 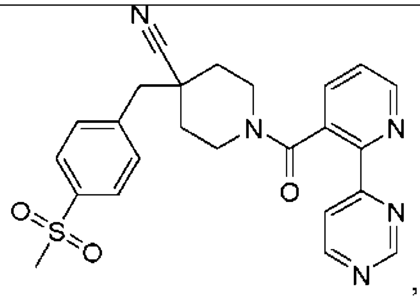 , |
| 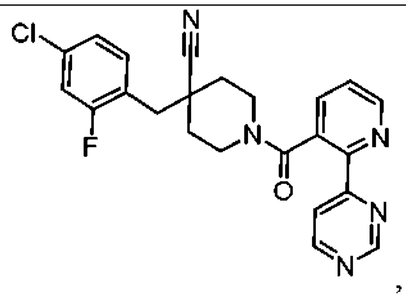 , |

| |
|---|
| 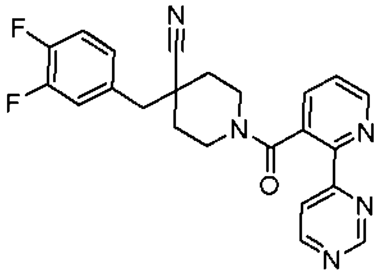 , |
| 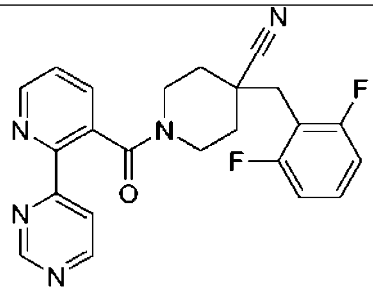 , |
| 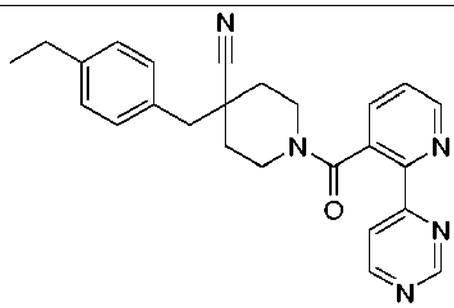 , |
| 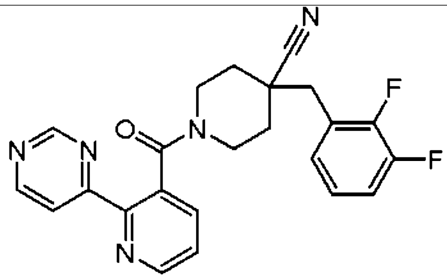 , |
| 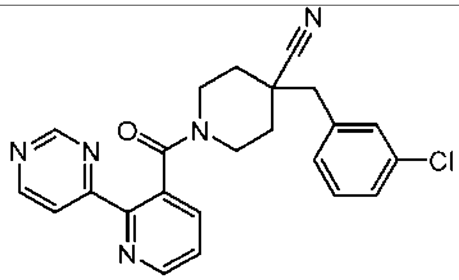 , |

| |
|---|
| 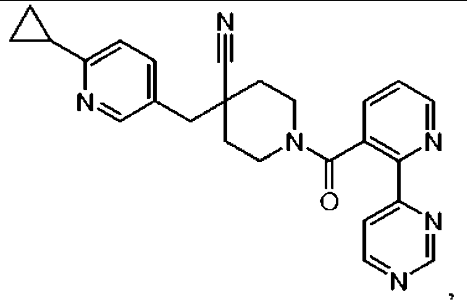 , |
| 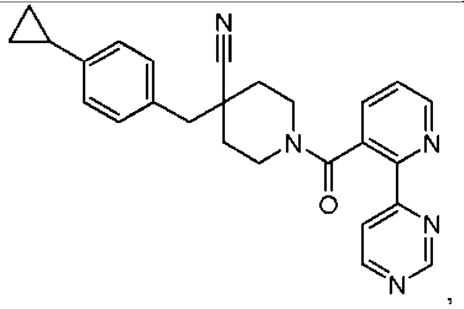 , |
| 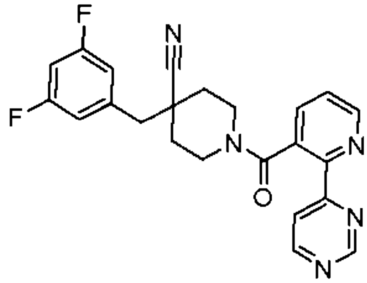 , |
| 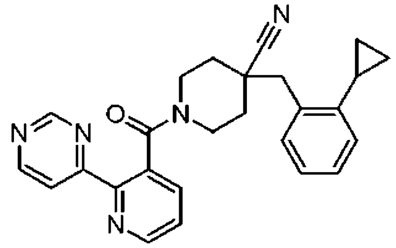 , |
| 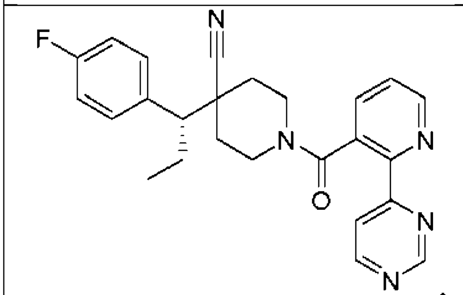 , |
| 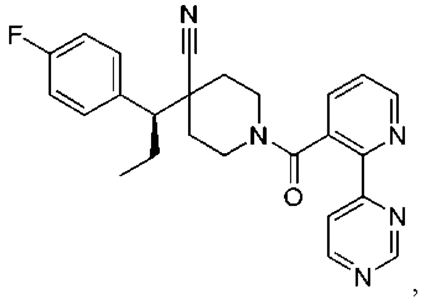 , |

| |
|---|
| 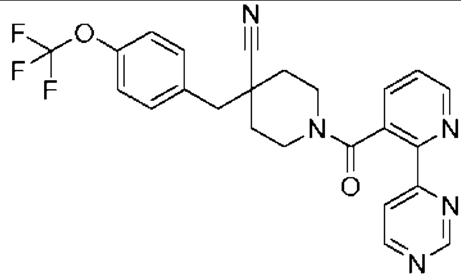 , |
| 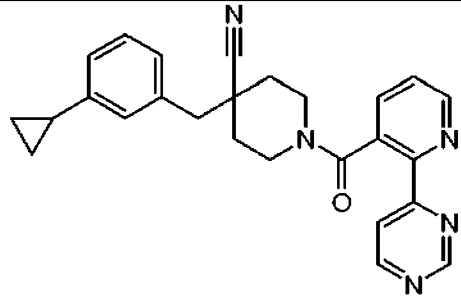 , |
| 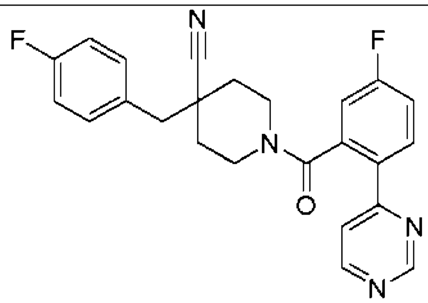 , |
| 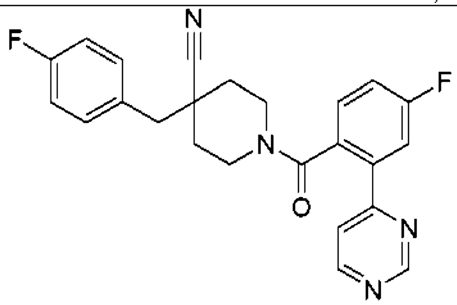 , |
| 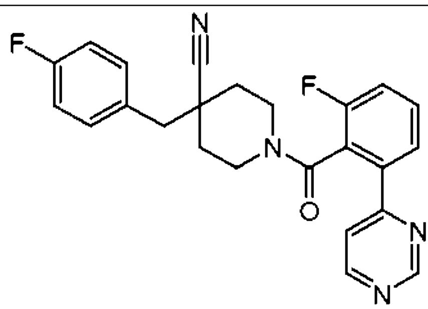 , |

| |
|---|
| 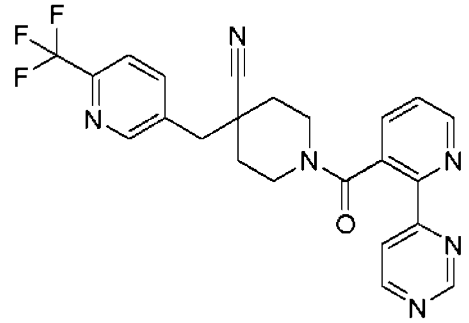 , |
| 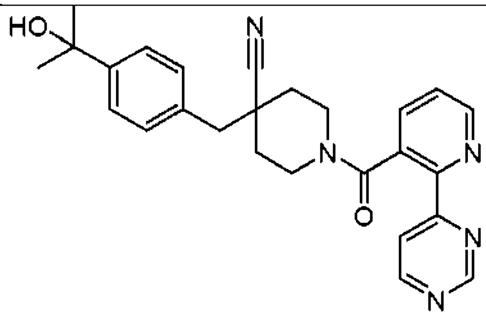 , |
| 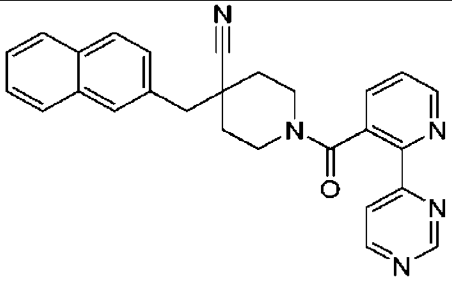 , |
| 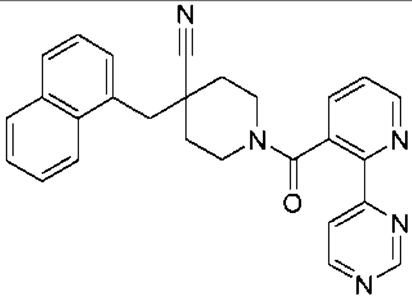 , |
| 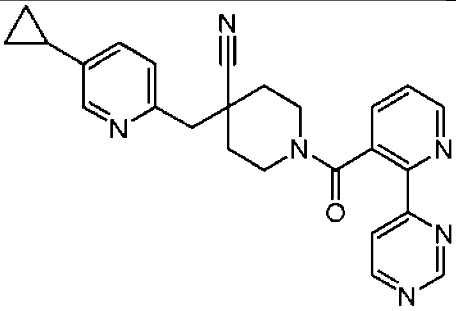 , |

| |
|---|
| 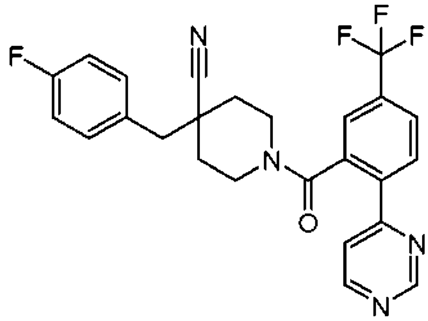 , |
| 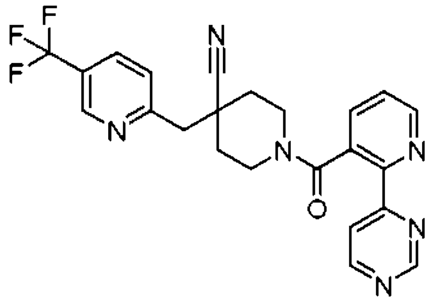 , |
| 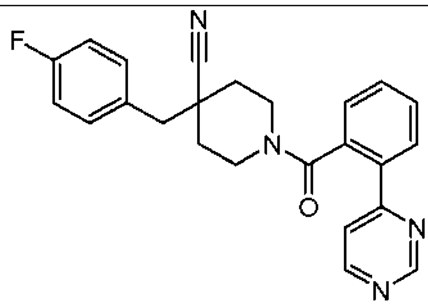 , |
| 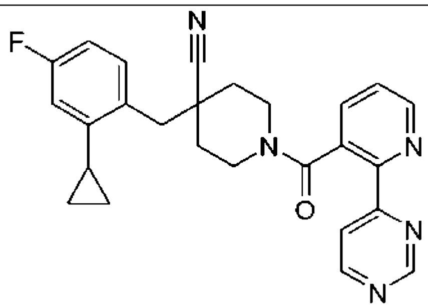 , |
| 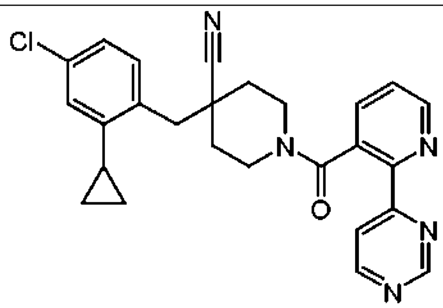 , |

| |
|---|
| 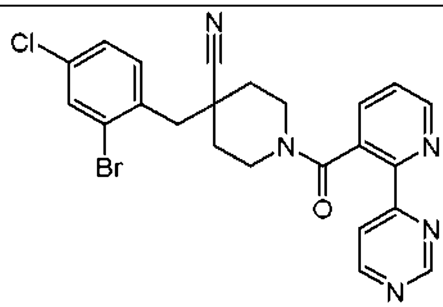 , |
| 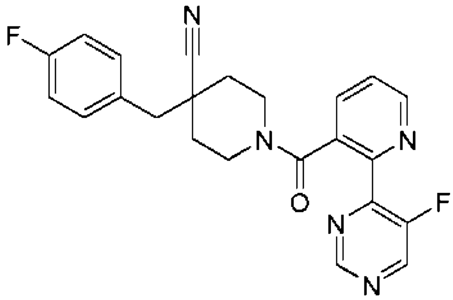 , |
| 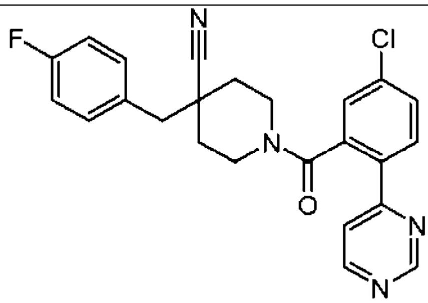 , |
| 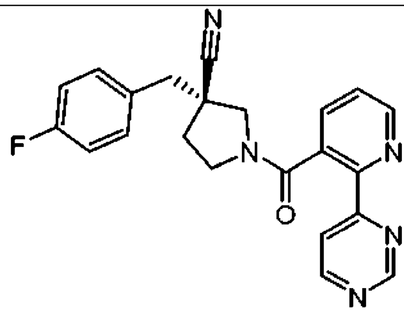 , |
| 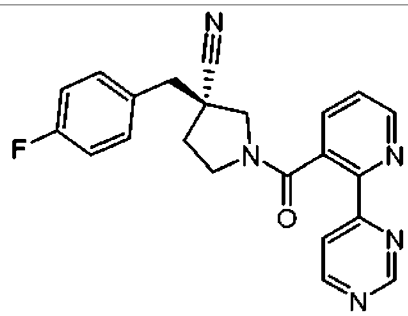 , |

| |
|---|
| 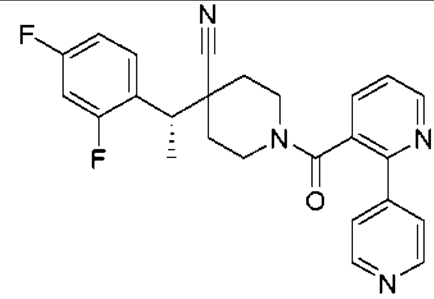 , |
| 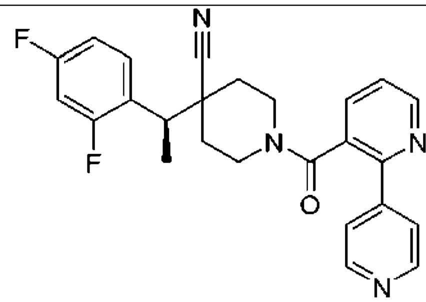 , |
| 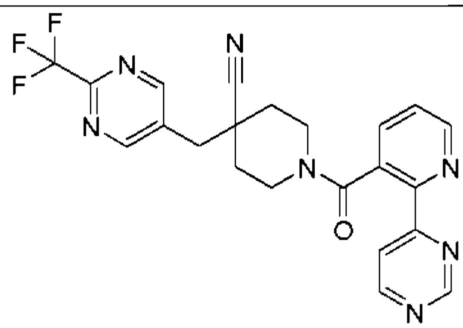 , |
| 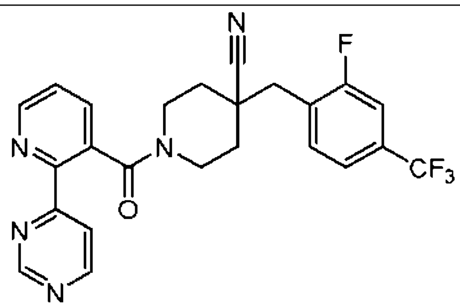 , |
| 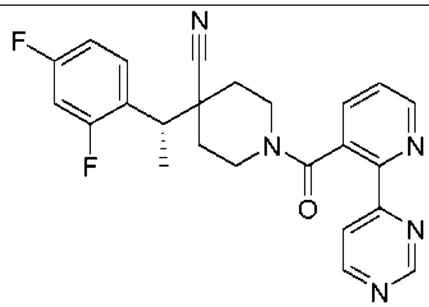 , |

| |
|---|
| 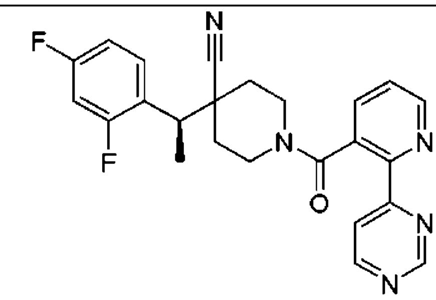 , |
| 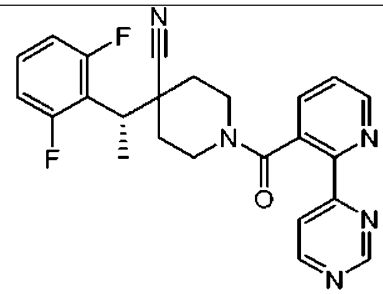 , |
| 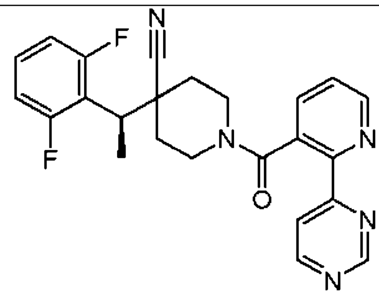 , |
| 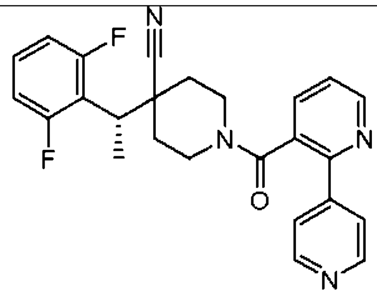 , |
| 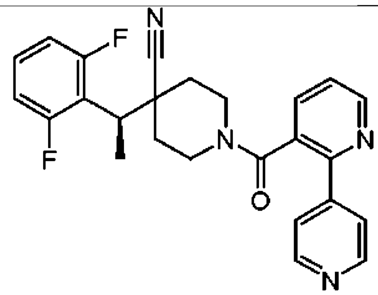 , |
| 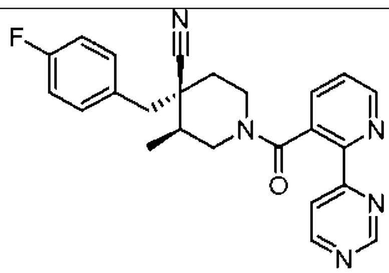 , |

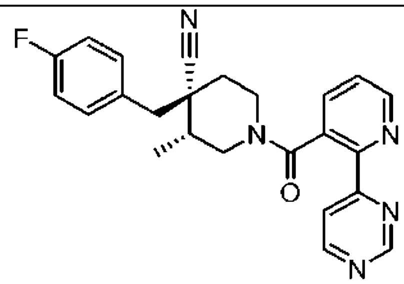
,

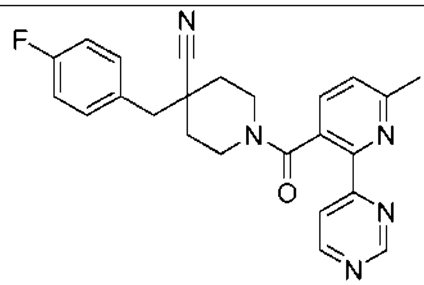
,

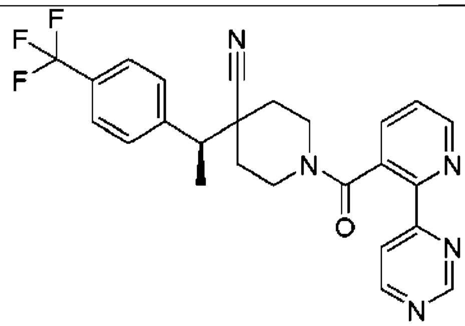
,

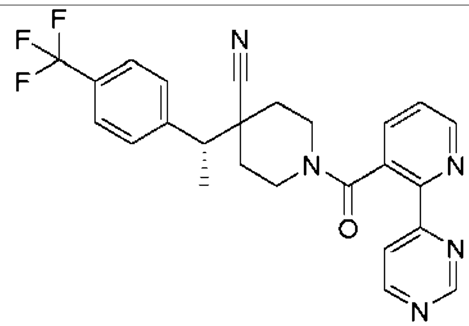
,

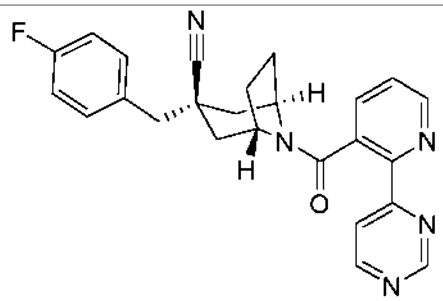
,

| |
|---|
| 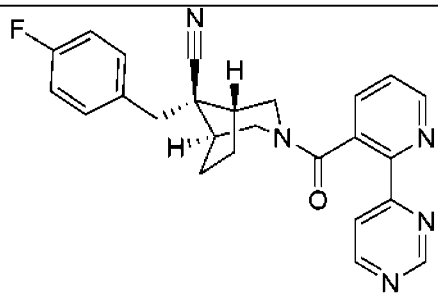 , |
| 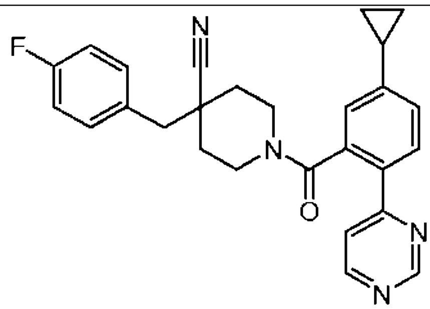 , |
| 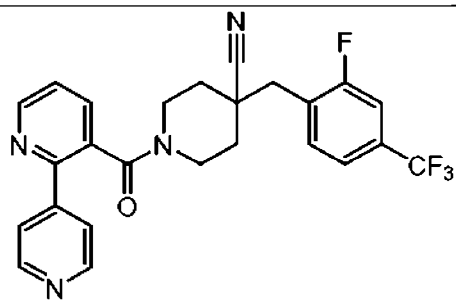 , |
| 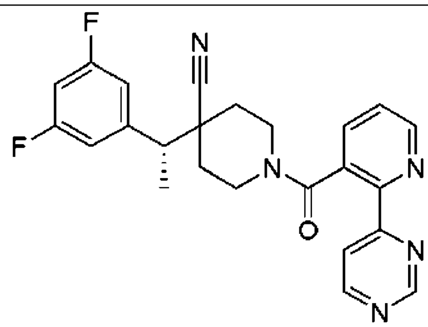 , |
| 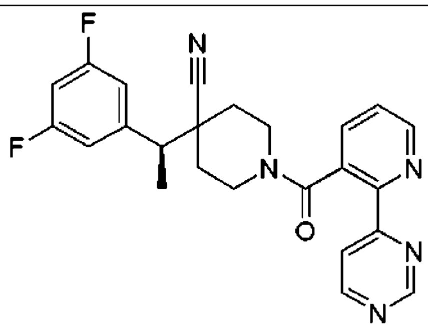 , |

| |
|---|
| 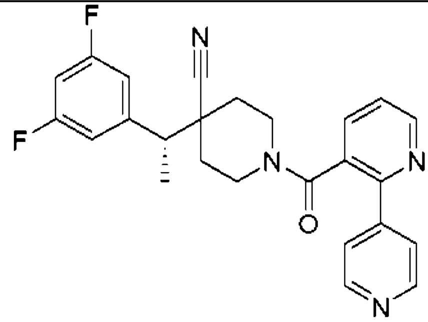 , |
| 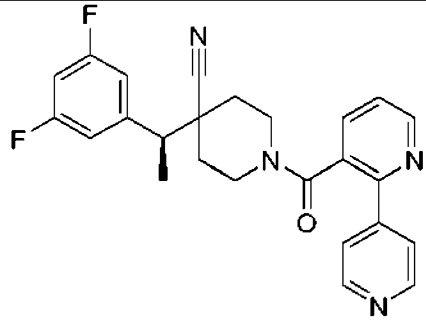 , |
| 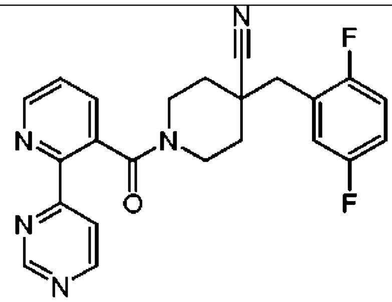 , |
| 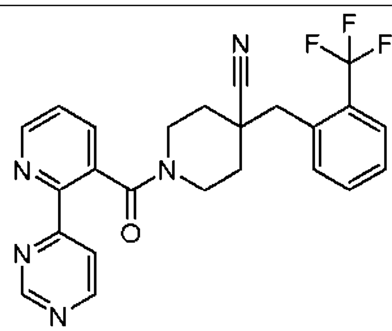 , |
| 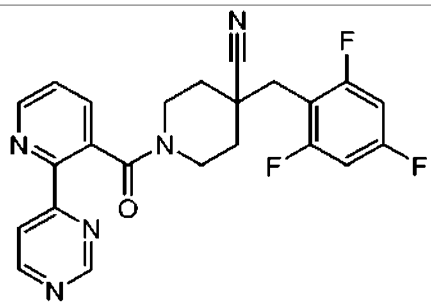 , |

| |
|---|
| 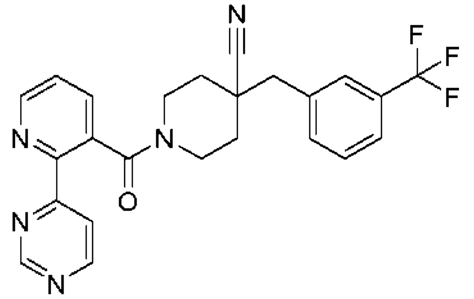 , |
| 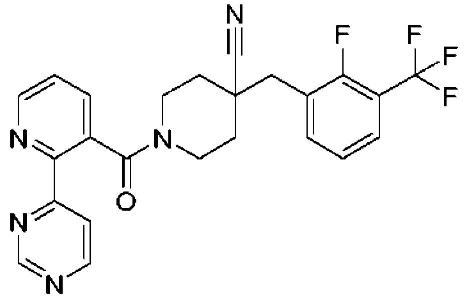 , |
| 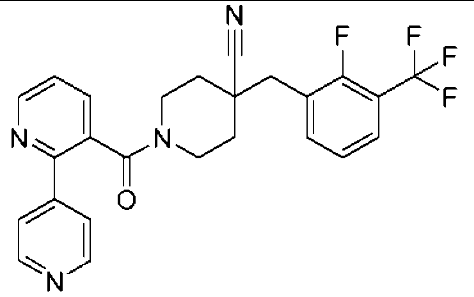 , |
| 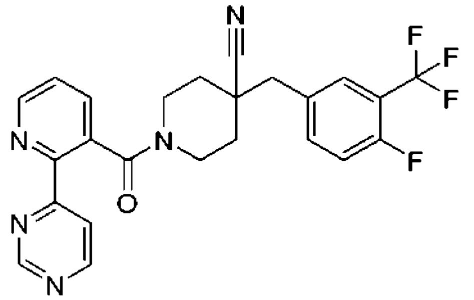 , |
| 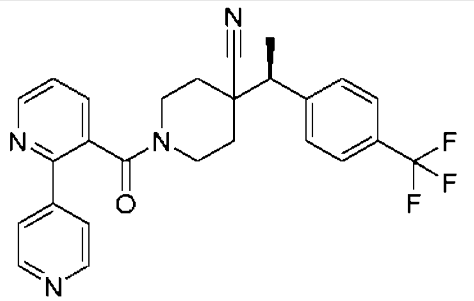 , |

| |
|---|
| 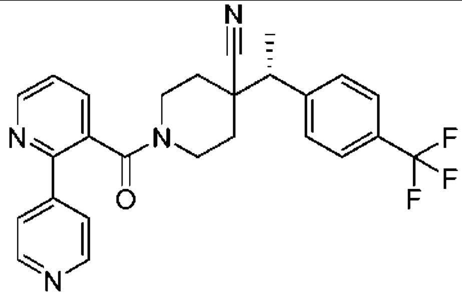 , |
| 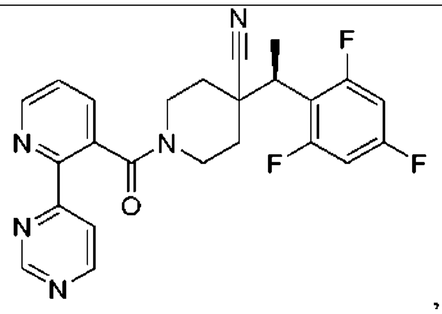 , |
| 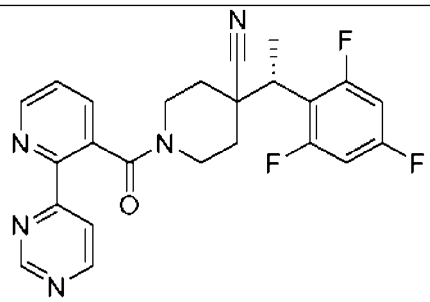 , |
| 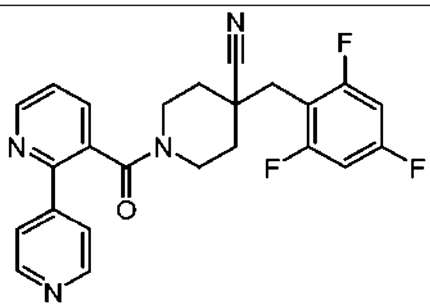 , |
| 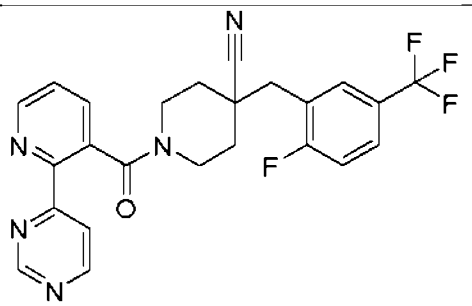 , |

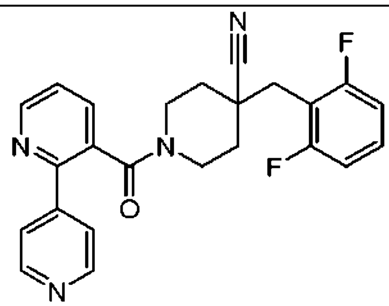
,

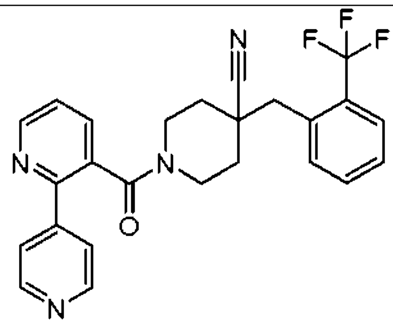
,

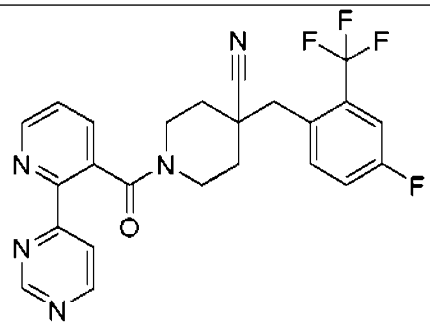
,

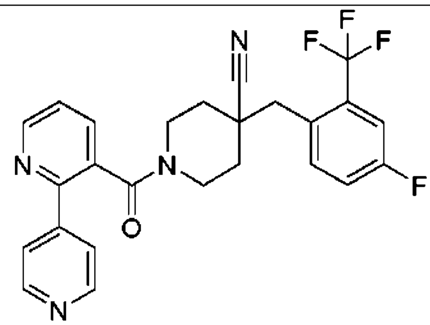
,

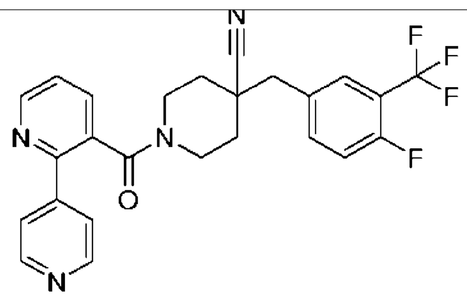
,

| |
|---|
| 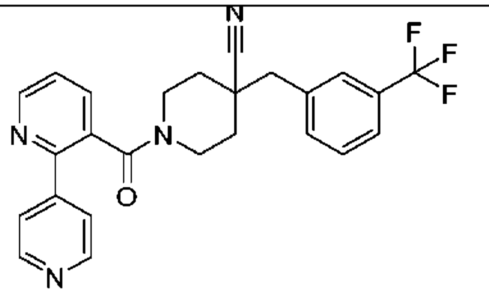 , |
| 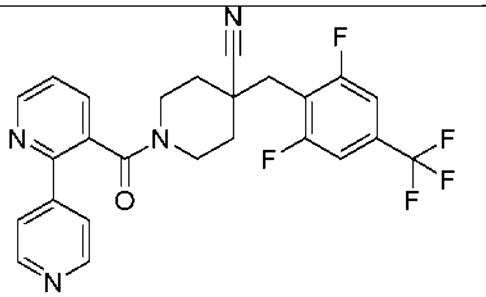 , |
| 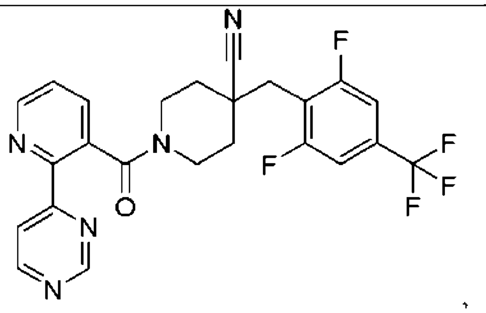 , |
| 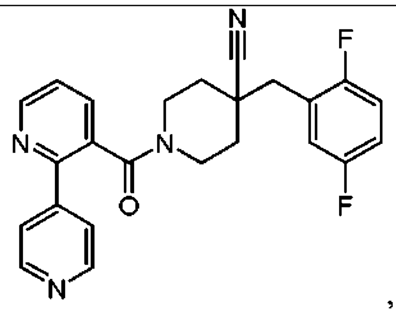 , |
| 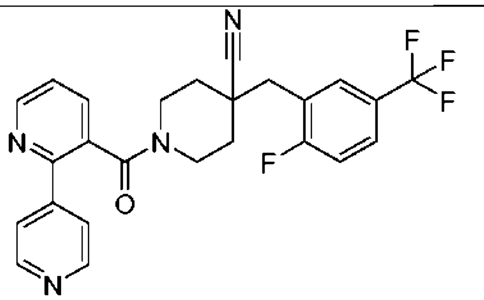 , |

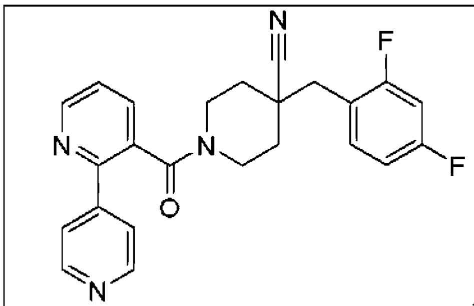,

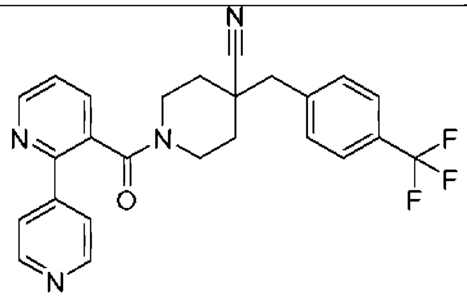,

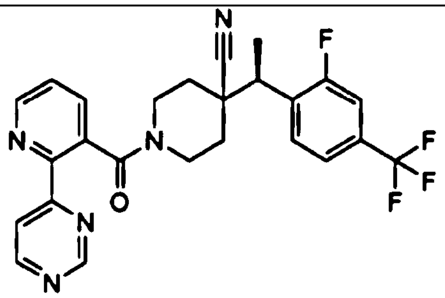

und

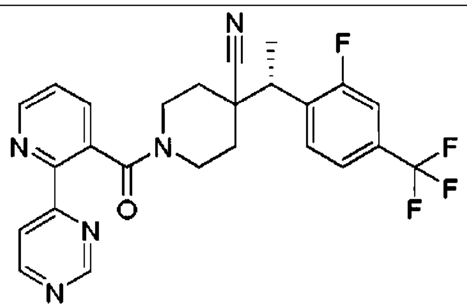.

**28.** Pharmazeutische Zusammensetzung, umfassend eine Verbindung nach einem der Ansprüche 1-27 oder ein pharmazeutisch unbedenkliches Salz davon und einen pharmazeutisch unbedenklichen Träger.

**29.** Verbindung nach einem der Ansprüche 1-27 oder ein pharmazeutisch unbedenkliches Salz davon oder pharmazeutische Zusammensetzung nach Anspruch 28 zur Verwendung bei der Behandlung einer Erkrankung oder Störung, die aus der Gruppe bestehend aus einer neurodegenerativen Störung, Epilepsie, entwicklungsbedingten und epileptischen Enzephalopathien, einer psychiatrischen Störung und Spasmus ausgewählt ist.

**30.** Verbindung, pharmazeutisch unbedenkliches Salz oder pharmazeutische Zusammensetzung zur Verwendung nach Anspruch 29, wobei es sich bei der Erkrankung oder Störung um eine neurodegenerative Störung handelt.

**31.** Verbindung, pharmazeutisch unbedenkliches Salz oder pharmazeutische Zusammensetzung zur Verwendung nach Anspruch 30, wobei die neurodegenerative Störung aus der Gruppe bestehend aus Alzheimer-Krankheit, leichter kognitiver Störung, Chorea Huntington, Parkinson-Krankheit, amyotropher Lateralsklerose, traumatischer Hirnverletzung, Hirninfarkt, Glaukom und multipler Sklerose ausgewählt ist.

**32.** Verbindung, pharmazeutisch unbedenkliches Salz oder pharmazeutische Zusammensetzung zur Verwendung nach Anspruch 29, wobei es sich bei der Erkrankung oder Störung um Epilepsie handelt.

**33.** Verbindung, pharmazeutisch unbedenkliches Salz oder pharmazeutische Zusammensetzung zur Verwendung nach Anspruch 29, wobei es sich bei der Erkrankung oder Störung um entwicklungsbedingte und epileptische Enzephalopathien handelt.

**34.** Verbindung, pharmazeutisch unbedenkliches Salz oder pharmazeutische Zusammensetzung zur Verwendung nach Anspruch 29, wobei es sich bei der Erkrankung oder Störung um eine psychiatrische Störung handelt.

**35.** Verbindung, pharmazeutisch unbedenkliches Salz oder pharmazeutische Zusammensetzung zur Verwendung nach Anspruch 34, wobei die psychiatrische Störung aus der Gruppe bestehend aus Schizophrenie, einer Störung des Autismus-Spektrums, einer wahnhaften Störung, einer schizoaffektiven Störung und Depression ausgewählt ist.

**36.** Verbindung, pharmazeutisch unbedenkliches Salz oder pharmazeutische Zusammensetzung zur Verwendung nach Anspruch 29, wobei es sich bei der Erkrankung oder Störung um Spasmus handelt.

## Revendications

**1.** Composé de formule I :

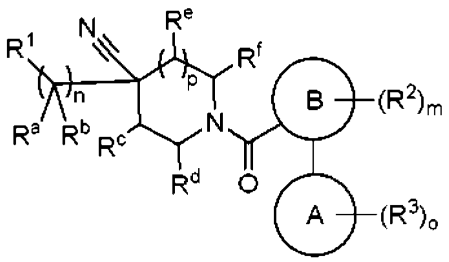

(I),

ou sel pharmaceutiquement acceptable de celui-ci ;

$R^1$ étant choisi dans le groupe constitué par $C_6$-$C_{10}$ aryle, $C_3$-$C_7$ cycloalkyle, hétérocyclyle à 3 à 7 chaînons, et hétéroaryle à 5 à 10 chaînons, $R^1$ étant éventuellement substitué par une, deux, trois ou quatre instances de $R^4$ ;

chacun parmi $R^a$ et $R^b$ étant indépendamment choisi dans le groupe constitué par H, halogéno, -CN, -OH, $-NO_2$, $-N(R^5)_2$, $C_1$-$C_6$ alkyle, $C_1$-$C_6$ halogénoalkyle, $C_1$-$C_6$ alcoxy, et $C_1$-$C_6$ halogénoalcoxy ;

chacun parmi $R^c$, $R^d$, $R^e$, et $R^f$ étant indépendamment choisi dans le groupe constitué par H, $C_1$-$C_6$ alkyle, $C_1$-$C_6$ halogénoalkyle, $C_1$-$C_6$ alcoxy, et $C_1$-$C_6$ halogénoalcoxy ; ou $R^c$ et $R^e$ pouvant former, conjointement avec les carbones auxquels ils sont fixés, un pont $C_1$-$C_3$ alkylène ; ou $R^d$ et $R^f$ pouvant former, conjointement avec les carbones auxquels ils sont fixés, un pont $C_1$-$C_3$ alkylène ;

chaque $R^4$ étant indépendamment choisi dans le groupe constitué par halogéno, -CN, -OH, $-NO_2$, $-N(R^5)_2$, $-S(O)_2R^5$, $C_1$-$C_6$ alkyle, $C_1$-$C_6$ halogénoalkyle, $C_1$-$C_6$ alcoxy, $C_1$-$C_6$ halogénoalcoxy, $C_6$-$C_{10}$ aryle, $C_3$-$C_7$ cycloalkyle, et hétérocyclyle à 3 à 7 chaînons ;

chaque $R^5$ étant indépendamment choisi parmi H et $C_1$-$C_6$ alkyle ;

chaque $R^2$ étant indépendamment choisi dans le groupe constitué par halogéno, -CN, -OH, $-NO_2$, $-N(R^5)_2$, $C_1$-$C_6$ alkyle, $C_3$-$C_7$ cycloalkyle, $C_1$-$C_6$ halogénoalkyle, $C_1$-$C_6$ alcoxy, et $C_1$-$C_6$ halogénoalcoxy ;

chaque $R^3$ étant indépendamment choisi dans le groupe constitué par halogéno, -CN, -OH, $-NO_2$, $-N(R^5)_2$, $C_1$-$C_6$ alkyle, $C_1$-$C_6$ halogénoalkyle, $C_1$-$C_6$ alcoxy, $C_3$-$C_7$ cycloalkyle, et $C_1$-$C_6$ halogénoalcoxy ;

A étant un hétéroaryle contenant azote à 5 à 6 chaînons ; B étant choisi parmi $C_6$-$C_{10}$ aryle et hétéroaryle à 5 à 6 chaînons ;

m étant 0, 1, 2, ou 3 ;

n étant 1, 2, 3, ou 4 ;

o étant 0, 1, 2, ou 3 ; et

p étant 0, 1, ou 2.

**2.** Composé selon la revendication 1, ou sel pharmaceutiquement acceptable de celui-ci,

(a) le composé de formule I étant un composé de formule I-a :

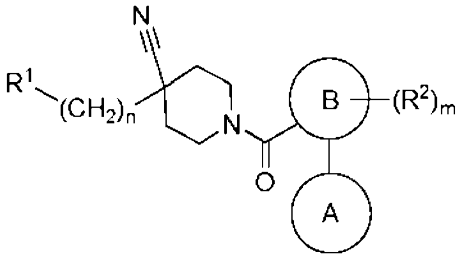

(I-a);

(b) le composé de formule I étant un composé de formule I-b :

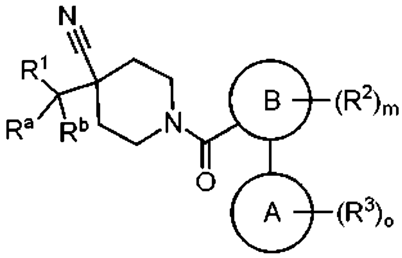

(I-b);

(c) le composé de formule I étant un composé de formule I-c :

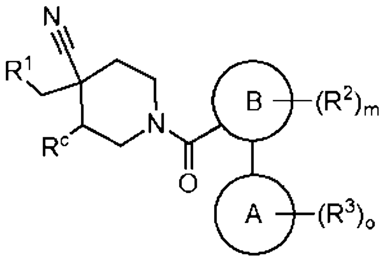

(I-c);

(d) le composé de formule I étant un composé de formule I-d :

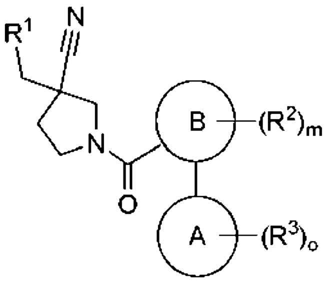

(I-d);

(e) le composé de formule I étant un composé de formule I-e :

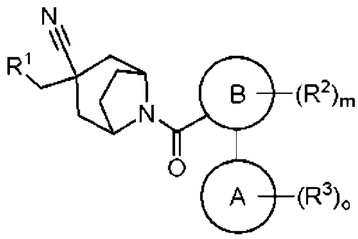

(I-e);

ou

(f) le composé de formule I étant un composé de formule I-f :

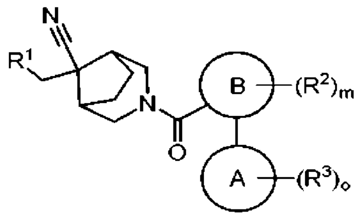

(I-f).

**3.** Composé selon la revendication 1 ou 2, ou sel pharmaceutiquement acceptable de celui-ci,

(a) $R^1$ étant $C_6$-$C_{10}$ aryle substitué par une, deux, trois ou quatre instances de $R^4$ ;
(b) $R^1$ étant $C_6$-$C_{10}$ aryle non substitué ;
(c) $R^1$ étant hétéroaryle à 5 à 10 chaînons substitué par une, deux, trois ou quatre instances de $R^4$ ;
(d) $R^1$ étant hétéroaryle non substitué à 5 à 10 chaînons ;
(e) $R^1$ étant $C_3$-$C_7$ cycloalkyle substitué par une, deux, trois ou quatre instances de $R^4$ ;
(f) $R^1$ étant $C_3$-$C_7$ cycloalkyle non substitué ;
(g) $R^1$ étant hétérocyclyle à 3 à 7 chaînons substitué par une, deux, trois ou quatre instances de $R^4$ ;
ou
(h) $R^1$ étant hétérocyclyle non substitué à 3 à 7 chaînons.

**4.** Composé selon la revendication 1 ou 2, ou sel pharmaceutiquement acceptable de celui-ci,

(a) $R^1$ étant

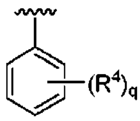

,

chaque $R^4$ étant indépendamment halogéno, -CN, -OH, $-NO_2$, $-N(R^5)_2$, $-S(O)_2R^5$, $C_1$-$C_6$ alkyle, $C_1$-$C_6$ halogénoalkyle, $C_1$-$C_6$ alcoxy, $C_1$-$C_6$ halogénoalcoxy, $C_6$-$C_{10}$ aryle, $C_3$-$C_7$ cycloalkyle, ou hétérocyclyle à 3 à 7 chaînons ; chaque $R^5$ étant indépendamment H ou $C_1$-$C_6$ alkyle ; et q étant 0,1, 2, ou 3 ;
(b) $R^1$ étant

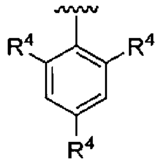

;

(c) $R^1$ étant

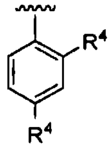, 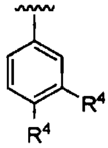, 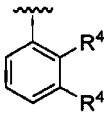,

ou

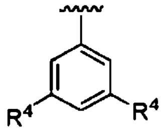;

(d) $R^1$ étant

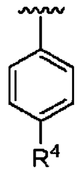;

(e) $R^1$ étant

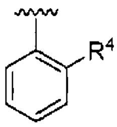;

(f) $R^1$ étant

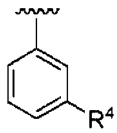;

ou
(g) $R^1$ étant

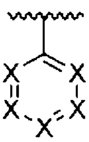,

chaque X étant indépendamment CH ou N, le H de CH pouvant être substitué par une ou plusieurs instances de $R^4$ ; chaque $R^4$ étant indépendamment halogéno, -CN, -OH, $-NO_2$, $-N(R^5)_2$, $C_1$-$C_6$ alkyle, $C_1$-$C_6$ halogénoalkyle, $C_1$-$C_6$ alcoxy, $C_1$-$C_6$ halogénoalcoxy, $C_6$-$C_{10}$ aryle, $C_3$-$C_7$ cycloalkyle, ou hétérocyclyle à 3 à 7 chaînons ; et chaque $R^5$ étant indépendamment H ou $C_1$-$C_6$ alkyle.

**5.** Composé selon la revendication 1 ou 2, ou sel pharmaceutiquement acceptable de celui-ci,

(a) $R^1$ étant pyrrolyle, furanyle, thiophenyle, imidazolyle, pyrazolyle, oxazolyle, isoxazolyle, thiazolyle, isothiazolyle, triazolyle, oxadiazolyle, thiadiazolyle, pyridinyle, 2-pyridinyle, 3-pyridinyle, 4-pyridinyle, pyridinyle, pyridazinyle, pyrimidinyle, 2-pyrimidinyle, 4-pyrimidinyle, pyrazinyle, triazinyle, tétrazinyle, tétrazolyle, azocinyle, dithiazinyle, ou oxazinyle ;
(b) $R^1$ étant pyridinyle, 2-pyridinyle, 3-pyridinyle, 4-pyridinyle, pyridinyle, pyridazinyle, pyrimidinyle, 2-pyrimidinyle, 4-pyrimidinyle, pyridazinyle, ou pyrazinyle ;
(c) $R^1$ étant cyclopropyle ou cyclobutyle ;
(d) $R^1$ étant tétrahydrofurane, tétrahydropyrane, pyrrolidine, pipéridine, pipérazine, dioxolane, dioxane, thiomorpholine, ou dithiane ; ou
(e) $R^1$ étant tétrahydrofurane ou tétrahydropyrane.

**6.** Composé selon la revendication 1 ou 2, ou sel pharmaceutiquement acceptable de celui-ci,

(a) $R^1$ étant

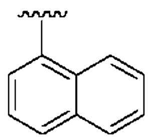

ou

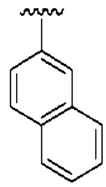

;

(b) $R^1$ étant

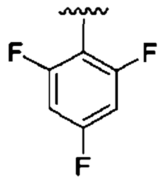

ou

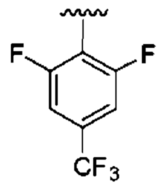

;

(c) $R^1$ étant

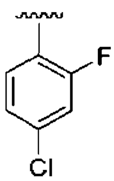 , 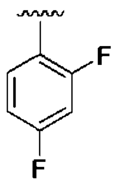 , 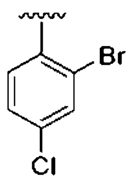 , 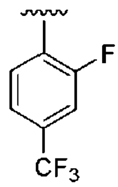 , 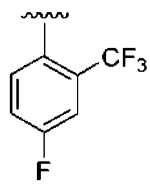 , 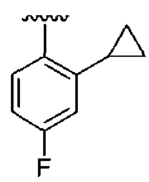 ,

ou

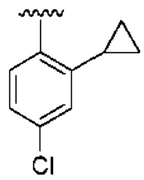

;

(d) $R^1$ étant

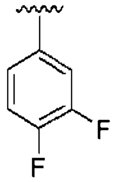

,

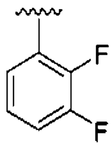

,

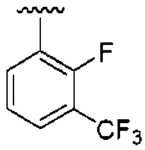

,

ou

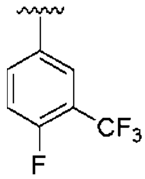

;

(e) $R^1$ étant

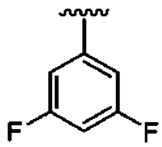

ou

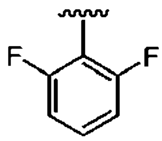

;

(f) $R^1$ étant

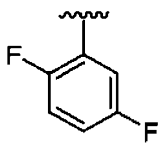

ou

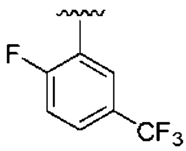

;

(g) $R^1$ étant

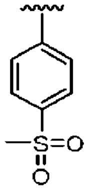, 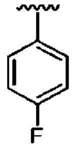, 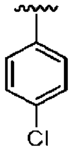, 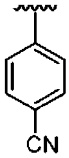, 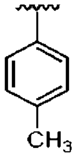, 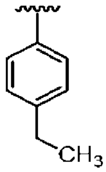, 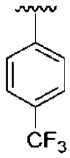, 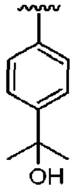, 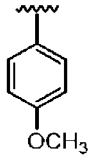, 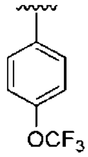,

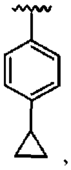, 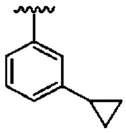, 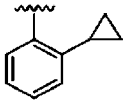, 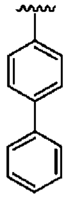, 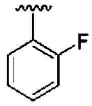, 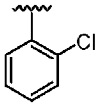, 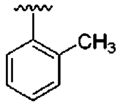, 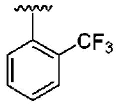,

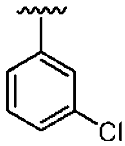, 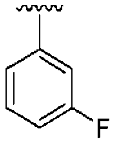,

ou

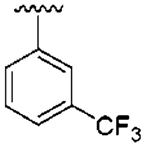;

(h) $R^1$ étant

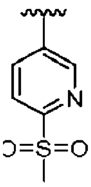, 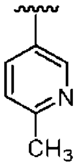, 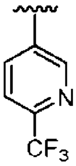, 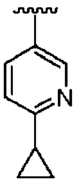, 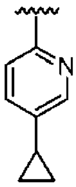, 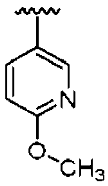, 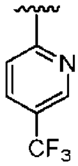, 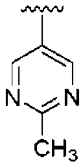,

ou

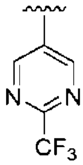;

(i) $R^1$ étant

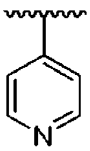, 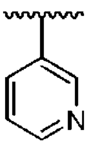,

ou

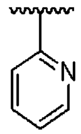

;

(j) $R^1$ étant

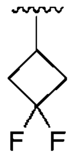

;

(k) $R^1$ étant

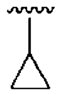

;

ou

(l) $R^1$ étant

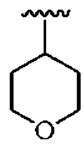

.

7. Composé selon la revendication 3, ou sel pharmaceutiquement acceptable de celui-ci, $R^1$ étant $C_6$-$C_{10}$ aryle éventuellement substitué par une, deux, trois ou quatre instances de $R^4$.

8. Composé selon la revendication 3, ou sel pharmaceutiquement acceptable de celui-ci, $R^1$ étant hétéroaryle à 5 à 10 chaînons éventuellement substitué par une, deux, trois ou quatre instances de $R^4$.

9. Composé selon la revendication 3, ou sel pharmaceutiquement acceptable de celui-ci, $R^1$ étant $C_3$-$C_7$ cycloalkyle éventuellement substitué par une, deux, trois ou quatre instances de $R^4$.

10. Composé selon la revendication 3, ou sel pharmaceutiquement acceptable de celui-ci, $R^1$ étant hétérocyclyle à 3 à 7 chaînons éventuellement substitué par une, deux, trois ou quatre instances de $R^4$.

11. Composé selon l'une quelconque des revendications 1-10, ou sel pharmaceutiquement acceptable de celui-ci,

(a) chaque $R^4$ étant indépendamment $C_1$-$C_6$ alkyle substitué, $C_1$-$C_6$ alcoxy substitué, ou $C_3$-$C_7$ cycloalkyle substitué ;
(b) chaque $R^4$ étant indépendamment $C_1$-$C_6$ alkyle non substitué, $C_1$-$C_6$ alcoxy non substitué, ou $C_3$-$C_7$ cycloalkyle non substitué ;
(c) chaque $R^4$ étant indépendamment halogéno, $-NH_2$, - NH($C_1$-$C_6$ alkyl), -N($C_1$-$C_6$ alkyle)$_2$, $C_1$-$C_6$ alkyle, $C_1$-$C_6$ halogénoalkyle, $C_1$-$C_6$ alcoxy, $C_1$-$C_6$ halogénoalcoxy, ou $C_6$-$C_{10}$ aryle ; ou
(d) chaque $R^4$ étant indépendamment halogéno, $C_1$-$C_6$ alkyle, $C_1$-$C_6$ halogénoalkyle, $C_1$-$C_6$ alcoxy, $C_1$-$C_6$-halogénoalcoxy, ou $C_6$-$C_{10}$ aryle.

12. Composé selon l'une quelconque des revendications 1-10, ou sel pharmaceutiquement acceptable de celui-ci,

(a) chaque $R^4$ étant indépendamment halogéno, -CN, $-CH_3$, $-CH_2CH_3$, $-CF_3$, $-OCH_3$, $-OCF_3$, $-C(CH_3)_2OH$, ou $-C_6H_5$ ; ou
(b) chaque $R^4$ étant indépendamment Cl, F, Br, ou I.

**13.** Composé selon l'une quelconque des revendications 1-12, ou sel pharmaceutiquement acceptable de celui-ci,

(a) n étant 4 ;
(b) n étant 3 ;
(c) n étant 2 ; ou
(d) n étant 1.

**14.** Composé selon la revendication 13, ou sel pharmaceutiquement acceptable de celui-ci, n étant 1.

**15.** Composé selon l'une quelconque des revendications 1-14, ou sel pharmaceutiquement acceptable de celui-ci,

(a) n étant 1, et $R^a$ étant $C_1$-$C_6$ alkyle et $R^b$ étant H ;
(b) n étant 1, et $R^a$ étant éthyle et $R^b$ étant H ; ou
(c) n étant 1, et $R^a$ étant méthyle et $R^b$ étant H.

**16.** Composé selon l'une quelconque des revendications 1-15, ou sel pharmaceutiquement acceptable de celui-ci,

(a) p étant 2 ;
(b) p étant 1 ; ou
(c) p étant 0.

**17.** Composé selon la revendication 16, ou sel pharmaceutiquement acceptable de celui-ci,

(a) p étant 1, et $R^c$, $R^d$, $R^e$, et $R^f$ étant H ;
(b) p étant 1, $R^c$ étant méthyle, et $R^d$ $R^e$, et $R^f$ étant H ;
(c) p étant 1, $R^c$ et $R^e$ étant H, et $R^d$ et $R^f$, conjointement avec les carbones auxquels ils sont fixés, formant un pont $C_1$-$C_3$ alkylène ;
(d) p étant 1, $R^d$ et $R^f$ étant H, et $R^c$ et $R^e$, conjointement avec les carbones auxquels ils sont fixés, formant un pont C1-C3 alkylène ; ou
(e) p étant 0, et $R^c$, $R^d$ et $R^f$ étant H.

**18.** Composé selon l'une quelconque des revendications 1-17, ou sel pharmaceutiquement acceptable de celui-ci,

(a) B étant

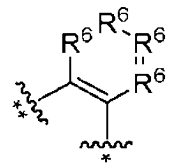

,

chaque $R^6$ étant indépendamment N ou $CR^{6a}$, $R^{6a}$ étant H ou $R^2$ ; et ** étant le point de fixation au carbonyle, et * étant le point de fixation à A ; ou
(b) B étant

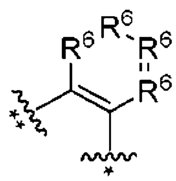

,

jusqu'à deux $R^6$ pouvant être N et les autres occurrences de $R^6$ étant CH ; et ** étant le point de fixation au carbonyle, et * étant le point de fixation à A.

**19.** Composé selon l'une quelconque des revendications 1-17, ou sel pharmaceutiquement acceptable de celui-ci,

(a) B étant

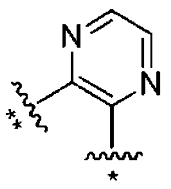,
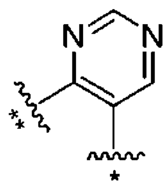,
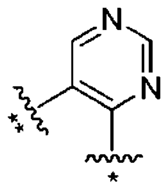,
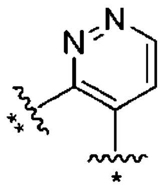,
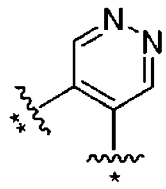,

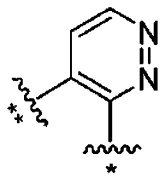,
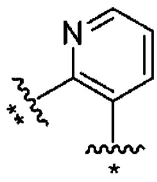,
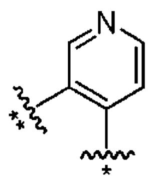,
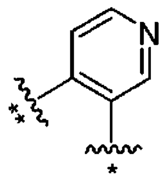,
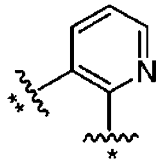;

ou

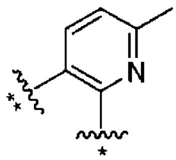;

** étant le point de fixation au carbonyle, et * étant le point de fixation à A ;

(b) B étant

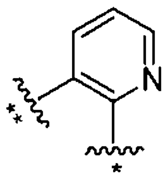;

** étant le point de fixation au carbonyle, et * étant le point de fixation à A ; ou

(c) B étant

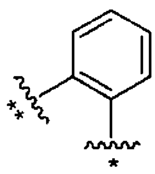,
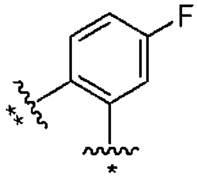,
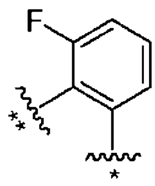,
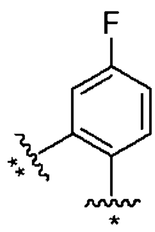,
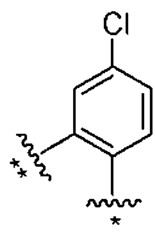,

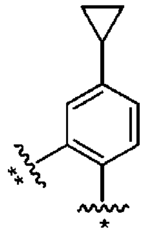

ou

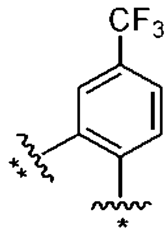

;

** étant le point de fixation au carbonyle, et * étant le point de fixation à A.

**20.** Composé selon la revendication 19, ou sel pharmaceutiquement acceptable de celui-ci, B étant

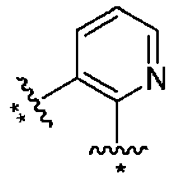

;

** étant le point de fixation au carbonyle, et * étant le point de fixation à A.

**21.** Composé selon la revendication 19, ou sel pharmaceutiquement acceptable de celui-ci, B étant

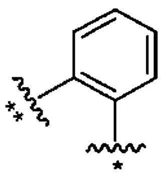 , 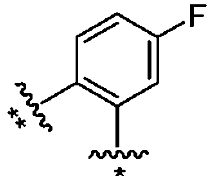 , 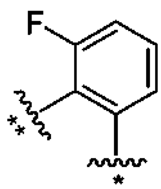 , 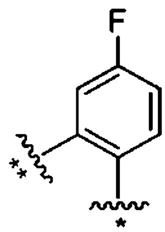 , 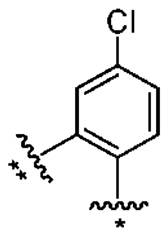 ,

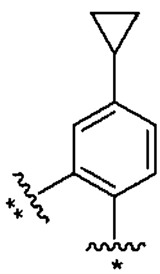

,

ou

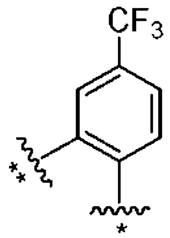

;

** étant le point de fixation au carbonyle, et * étant le point de fixation à A.

**22.** Composé selon l'une quelconque des revendications 1-21, ou sel pharmaceutiquement acceptable de celui-ci,

(a) m étant 1 ; ou
(b) m étant 0.

**23.** Composé selon l'une quelconque des revendications 1-22, ou sel pharmaceutiquement acceptable de celui-ci, $R^2$ étant halogéno, -CN, -OH, $-NO_2$, $-CH_3$, $-CH_2CH_3$, cyclopropyle, $-CHF_2$, $-CF_3$, $-OCF_3$, $-OCH_3$, $-OCH_2CH_3$, ou $-OCH_2CF_3$.

**24.** Composé selon l'une quelconque des revendications 1-23, ou sel pharmaceutiquement acceptable de celui-ci,

(a) A étant pyridinyle, pyrrolyle, imidazolyle, pyrazolyle, pyridazinyle, pyrimidinyle, pyrazinyle, triazolyle, triazinyle, tétrazinyle, tétrazolyle, oxazolyle, isoxazolyle, ou thiozolyle ; ou
(b) A étant pyridinyle, oxazolyle, imidazolyle, triazolyle, pyridinyle, pyridazinyle, pyrimidinyle, ou triazinyle.

**25.** Composé selon l'une quelconque des revendications 1-23, ou sel pharmaceutiquement acceptable de celui-ci, A étant

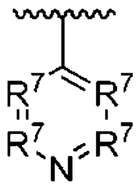

;

chaque $R^7$ étant indépendamment N ou CH, jusqu'à deux $R^7$ pouvant être N et les autres occurrences de $R^7$ étant CH, l'hydrogène de CH pouvant être substitué par $R^3$.

**26.** Composé selon l'une quelconque des revendications 1-23, ou sel pharmaceutiquement acceptable de celui-ci,

(a) A étant

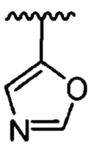 , 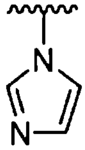 , 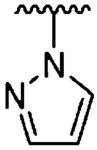 , 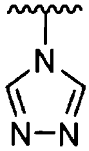 , 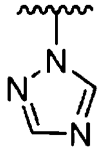 , 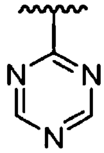 , 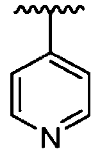 , 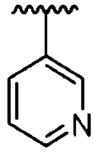 , 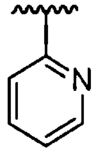 ,

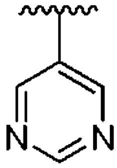, 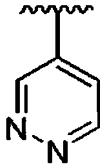, 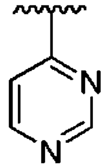,

ou

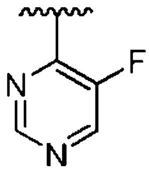 ;

(b) A étant

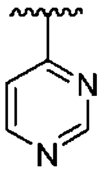, 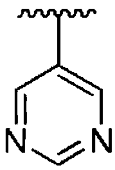, 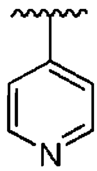, 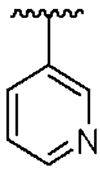, 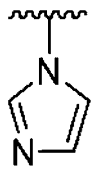, 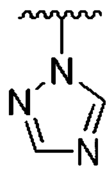, 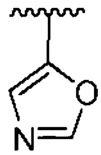,

ou

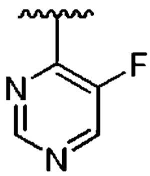.

**27.** Composé selon l'une quelconque des revendications 1-26, ou sel pharmaceutiquement acceptable de celui-ci, le composé étant choisi dans le groupe constitué par :

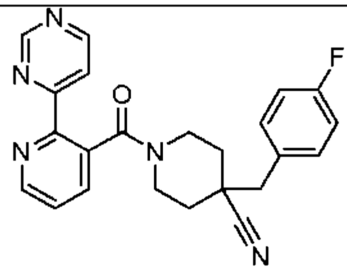

,

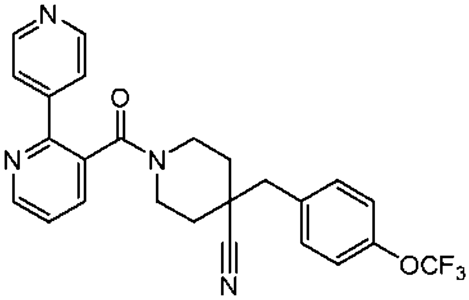

,

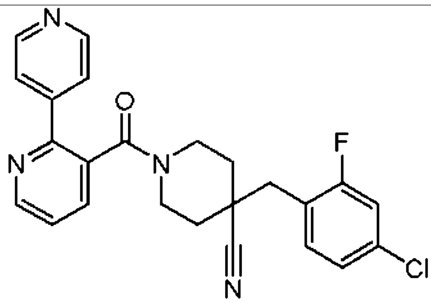

,

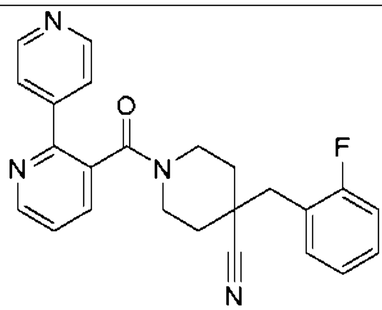

,

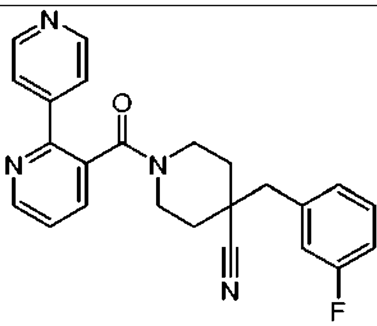

,

| |
|---|
| 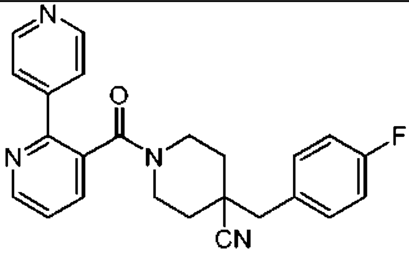 , |
| 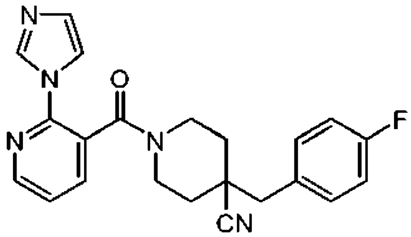 , |
| 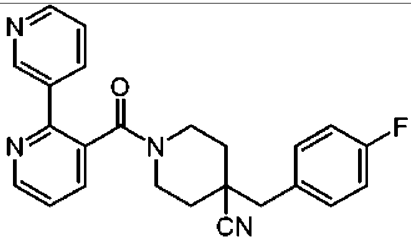 , |
| 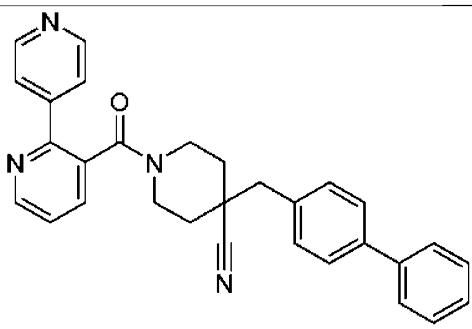 , |
| 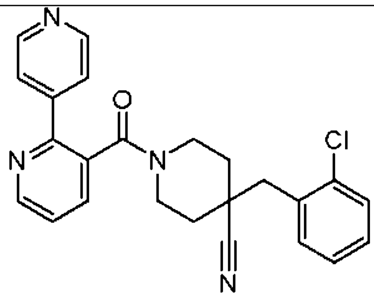 , |
| 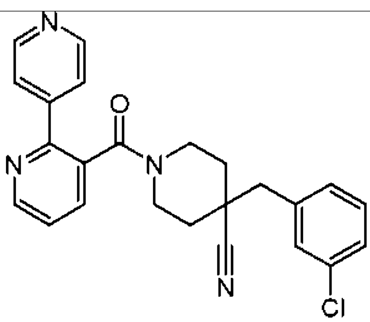 , |

| |
|---|
| 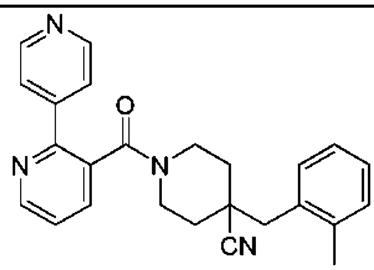 , |
| 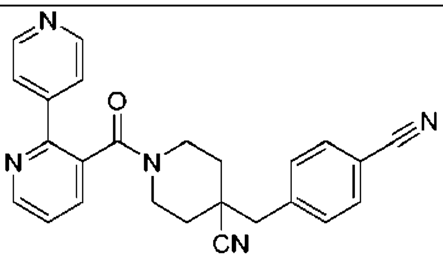 , |
| 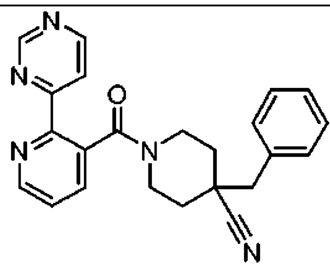 , |
| 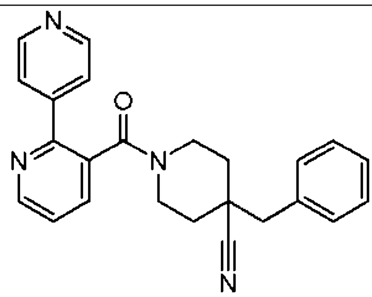 , |
| 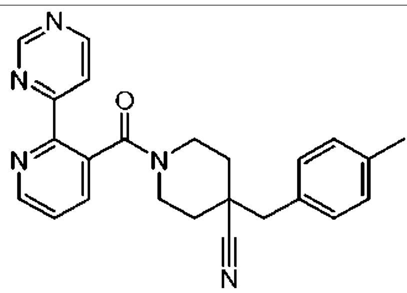 , |
| 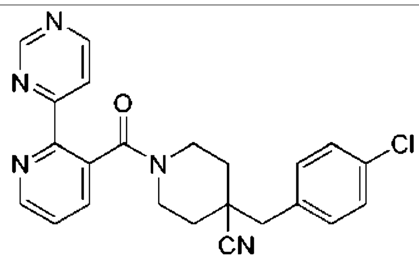 , |

| |
|---|
| 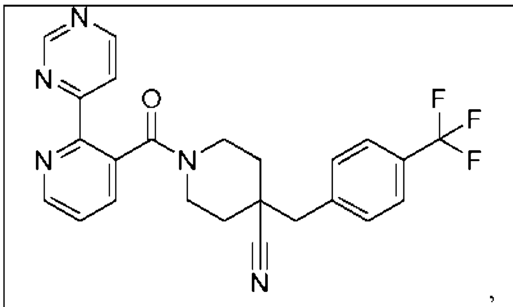 , |
| 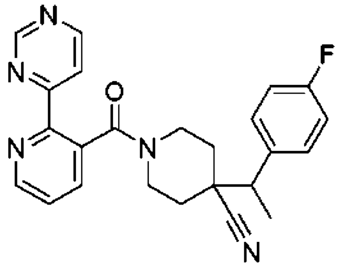 , |
| 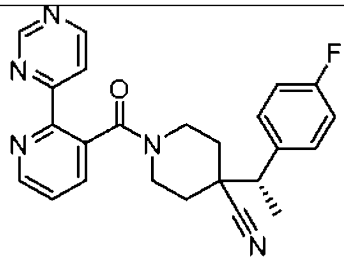 , |
| 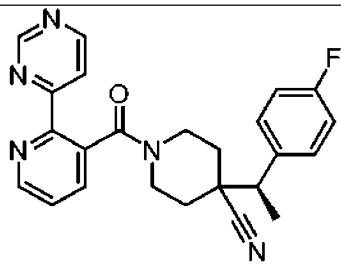 , |
| 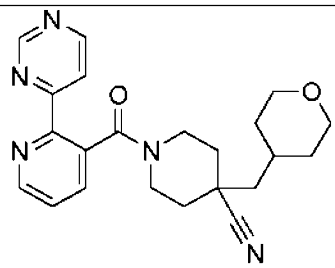 , |
| 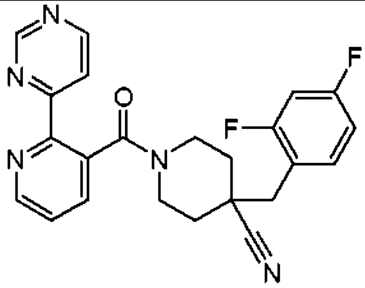 , |

| |
|---|
| 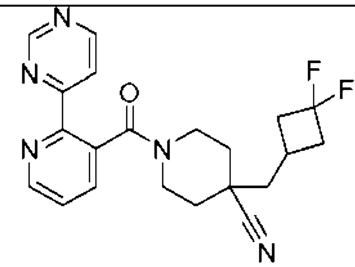 , |
| 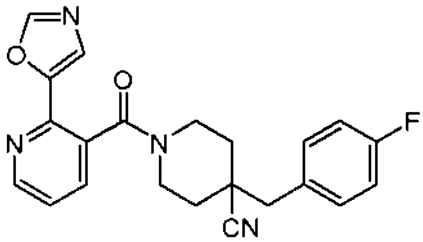 , |
| 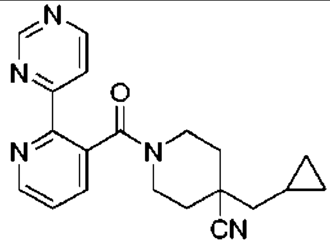 , |
| 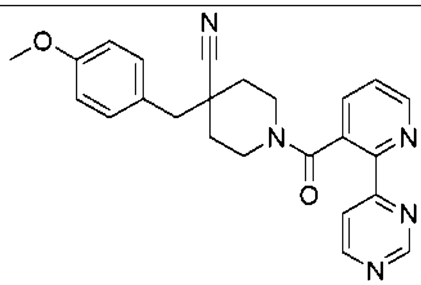 , |
| 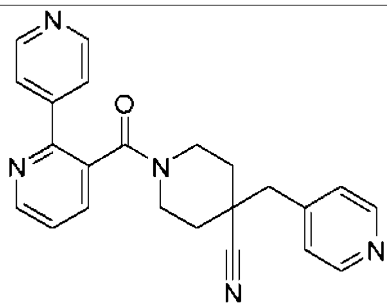 , |
| 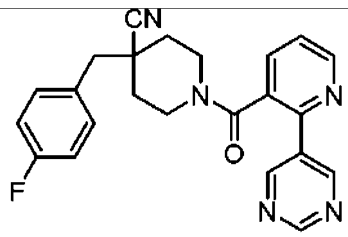 , |

| |
|---|
| 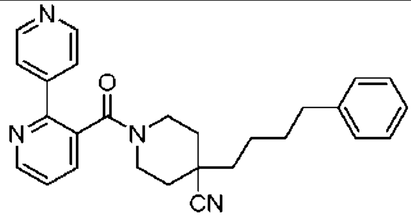 , |
| 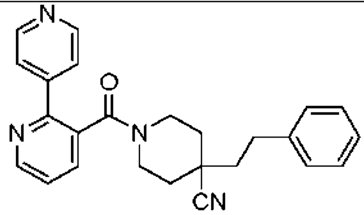 , |
| 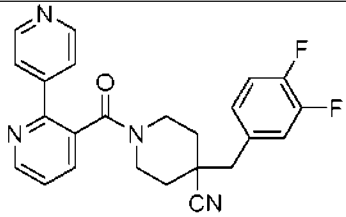 , |
| 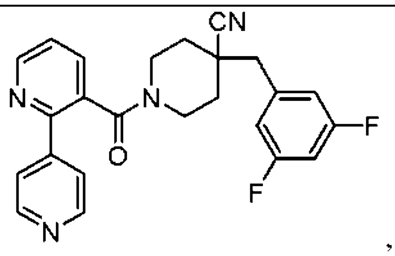 , |
| 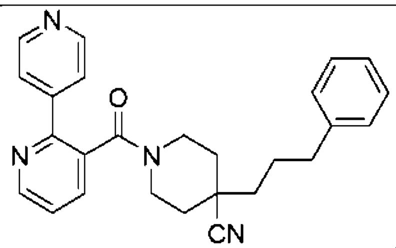 , |
| 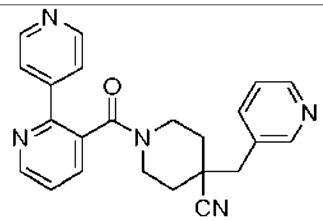 , |
| 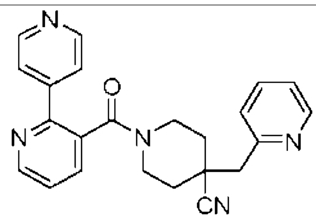 , |

| |
|---|
| 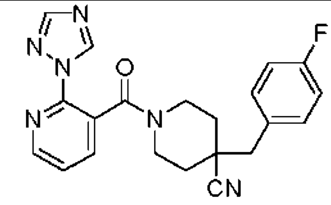 , |
| 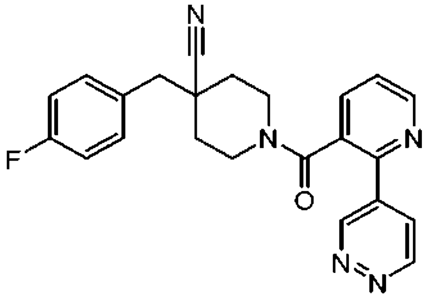 , |
| 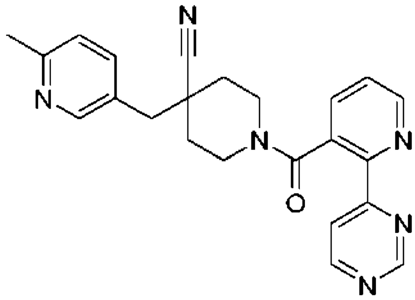 , |
| 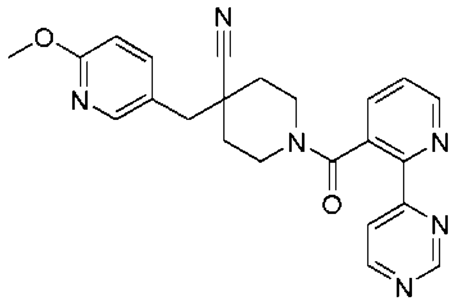 , |
| 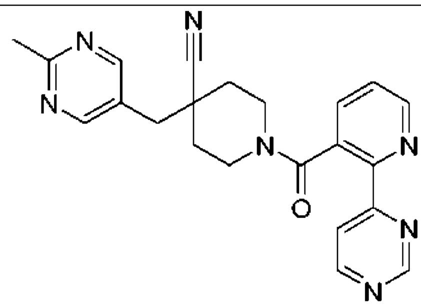 , |
| 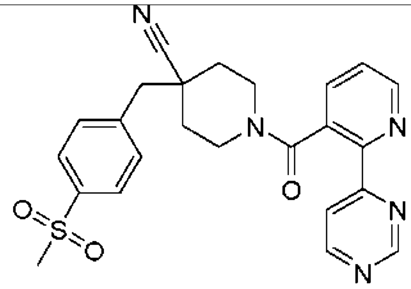 , |

| |
|---|
| 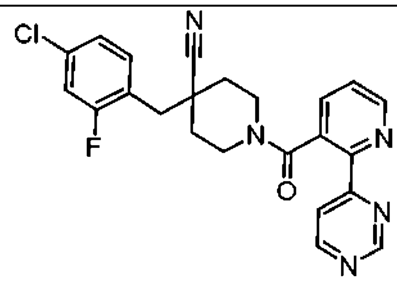 , |
| 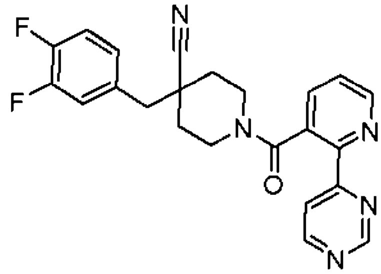 , |
| 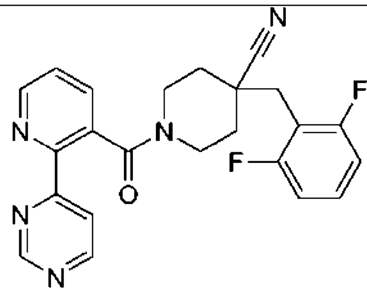 , |
| 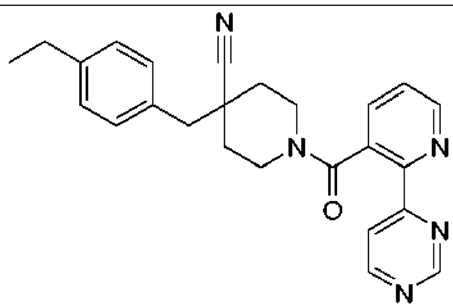 , |
| 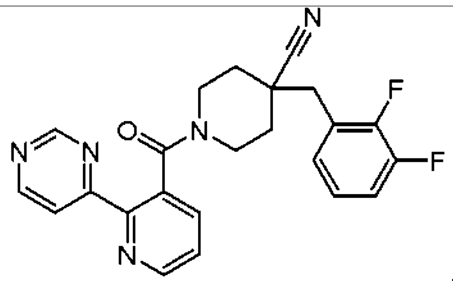 , |

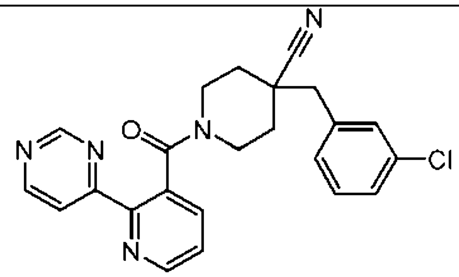

,

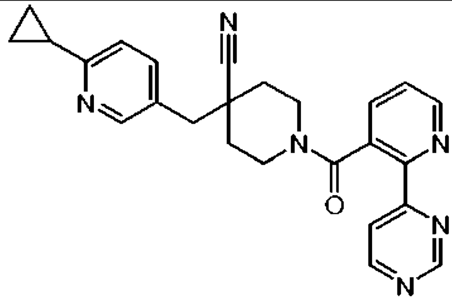

,

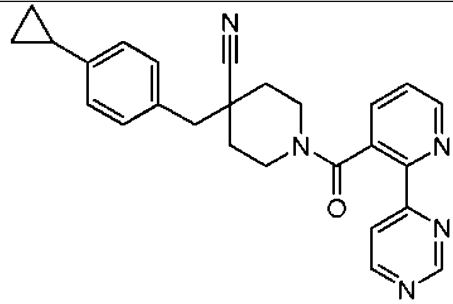

,

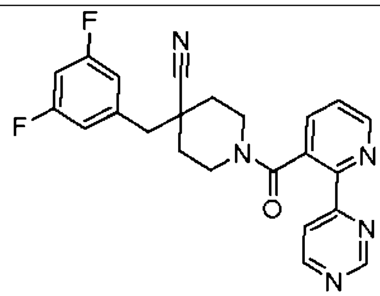

,

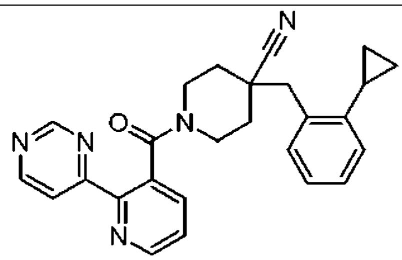

,

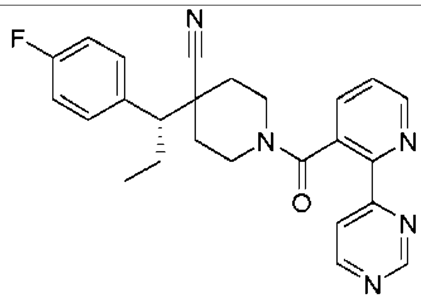

,

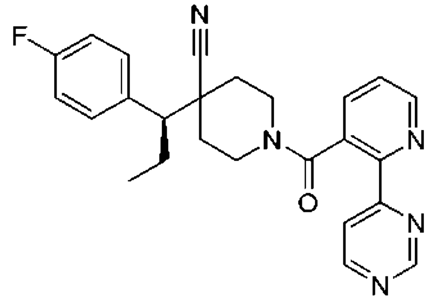
,

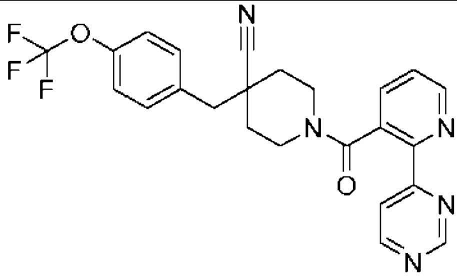
,

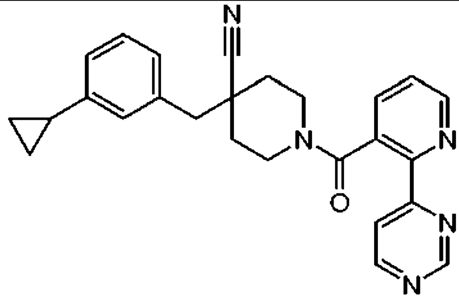
,

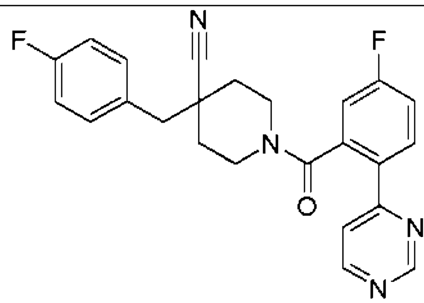
,

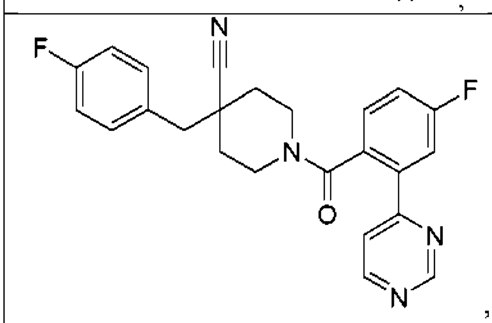
,

| |
|---|
| 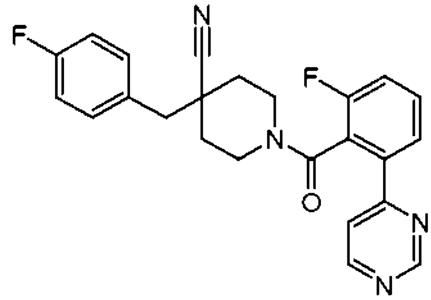 , |
| 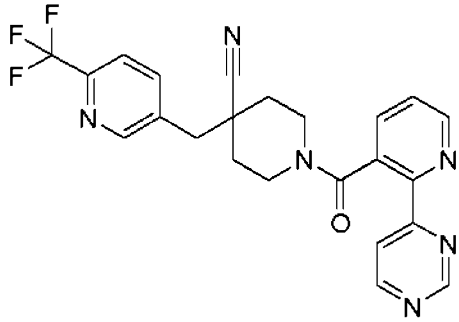 , |
| 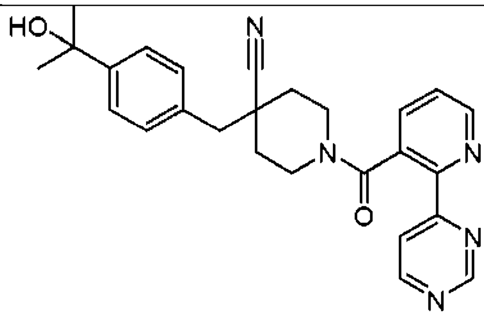 , |
| 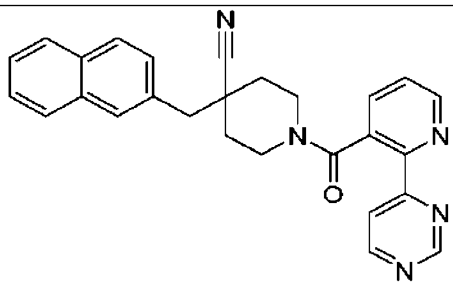 , |
| 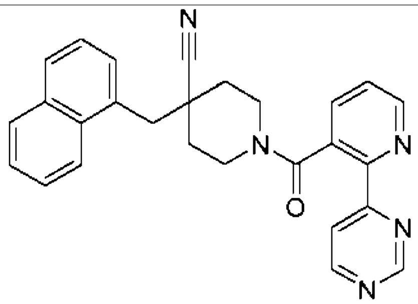 , |

| |
|---|
| 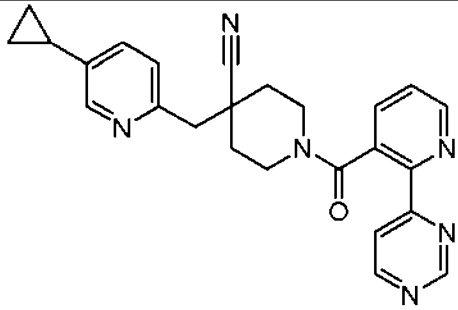 , |
| 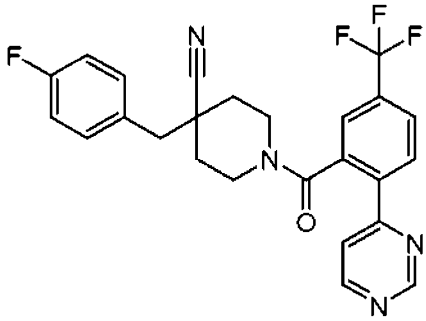 , |
| 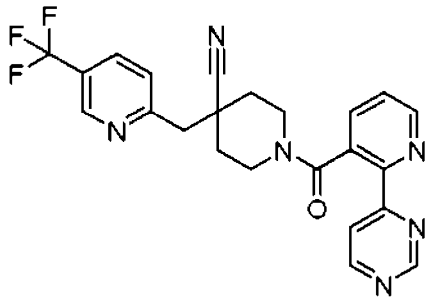 , |
| 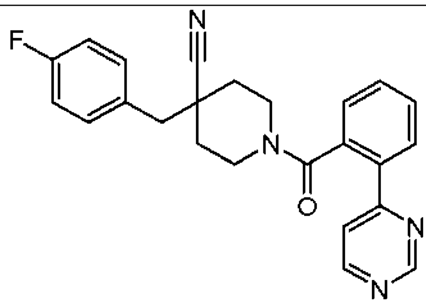 , |
| 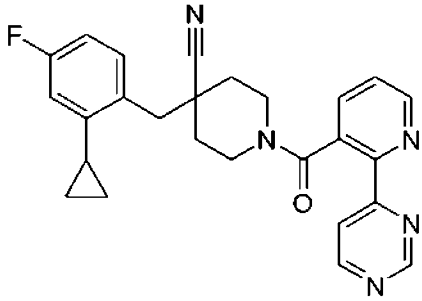 , |

| |
|---|
| 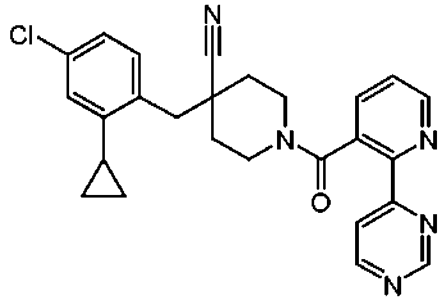 , |
| 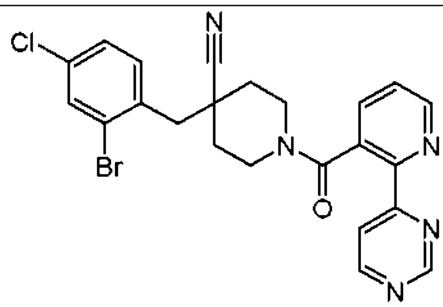 , |
| 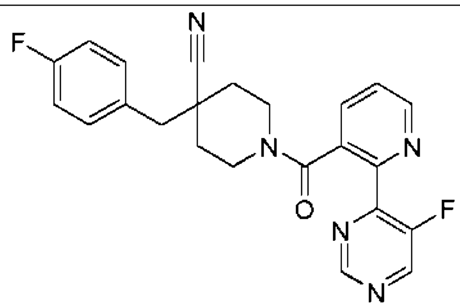 , |
| 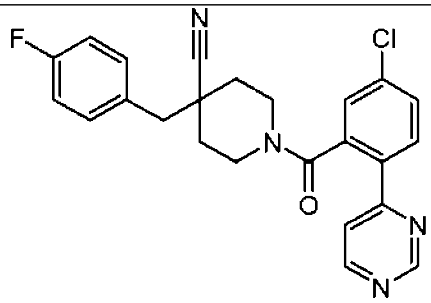 , |
| 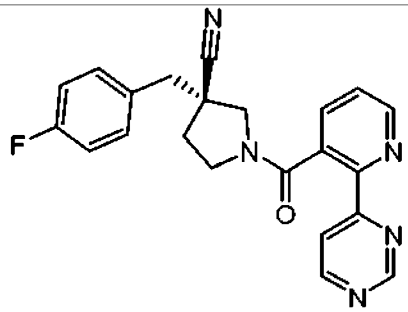 , |

| |
|---|
| 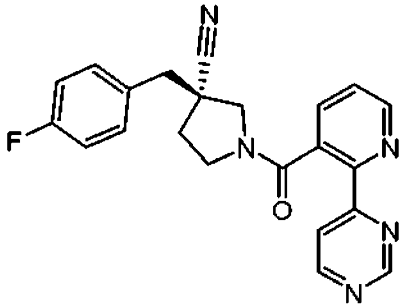 , |
| 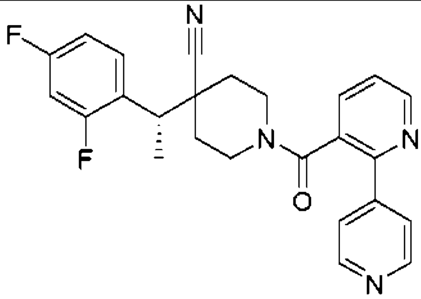 , |
| 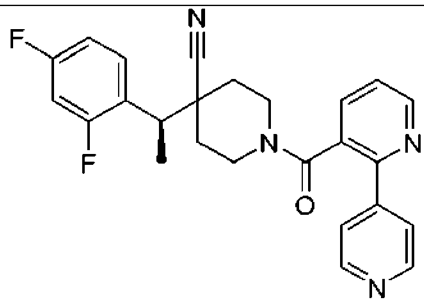 , |
| 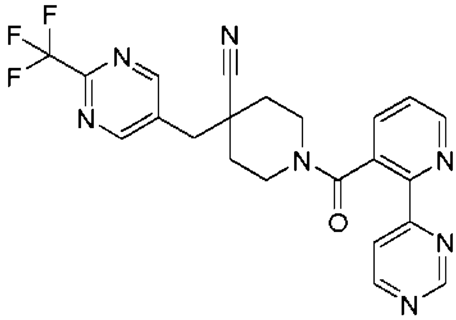 , |
| 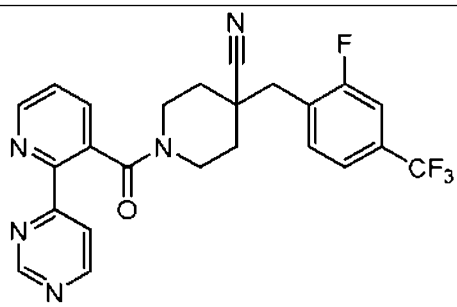 , |

| |
|---|
| 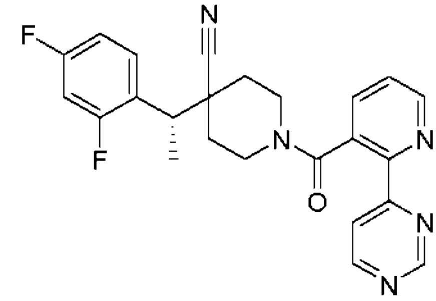 , |
| 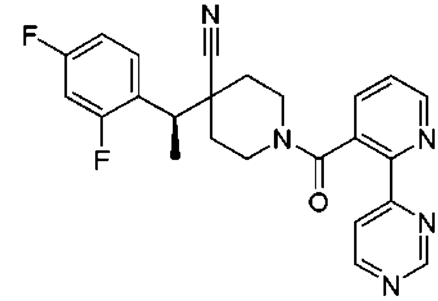 , |
| 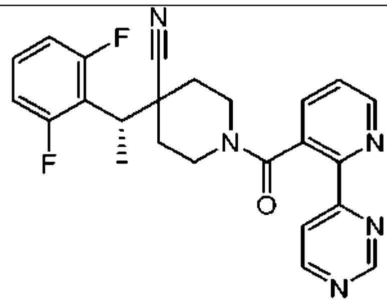 , |
| 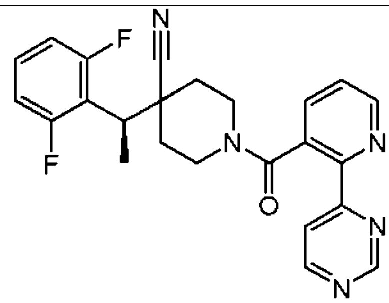 , |
| 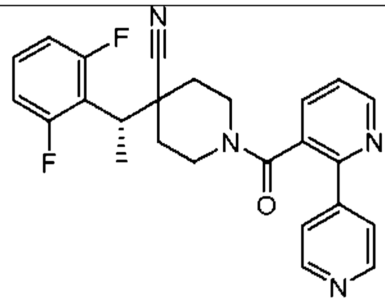 , |

| |
|---|
| 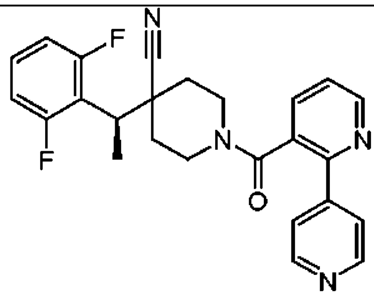 , |
| 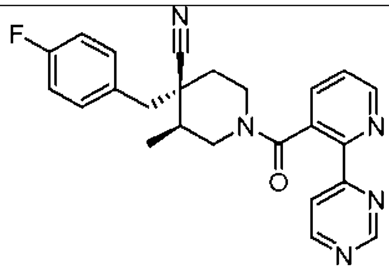 , |
| 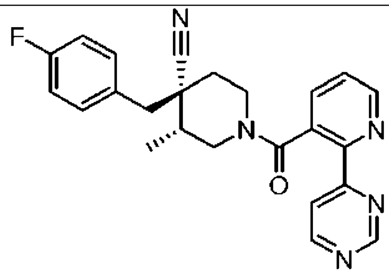 , |
| 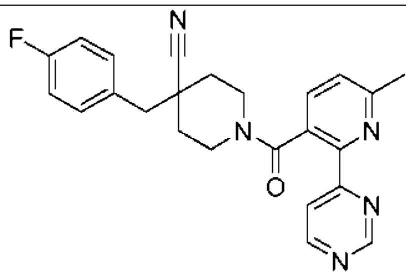 , |
| 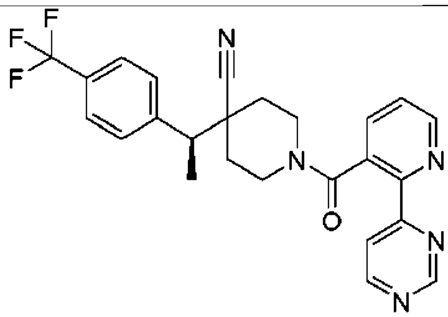 , |
| 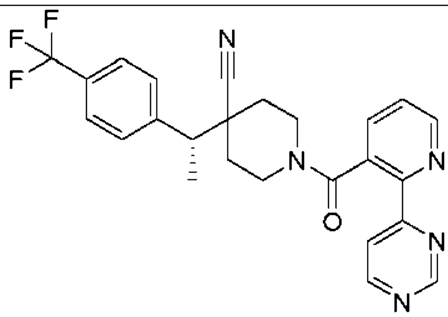 , |

| |
|---|
| 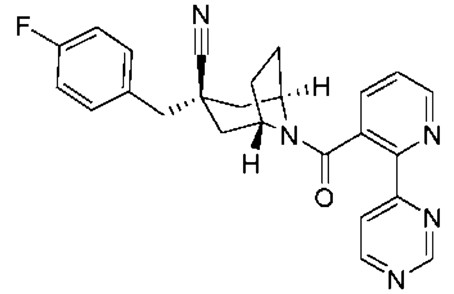 , |
| 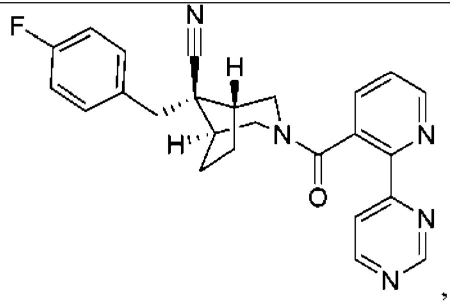 , |
| 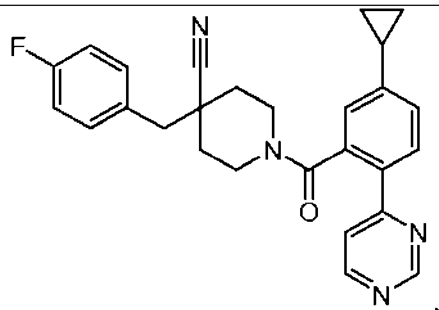 , |
| 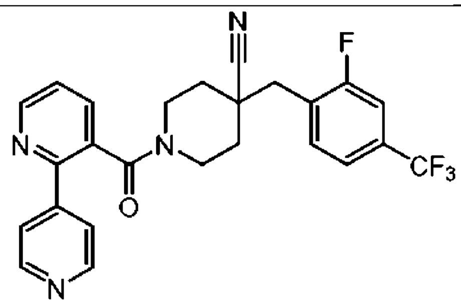 , |
| 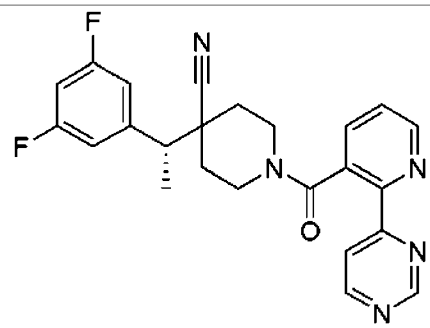 , |

| |
|---|
| 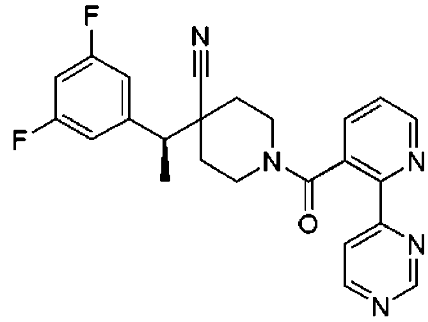 , |
| 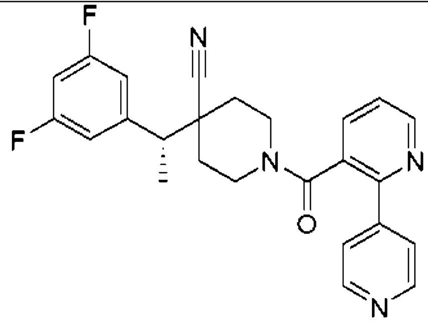 , |
| 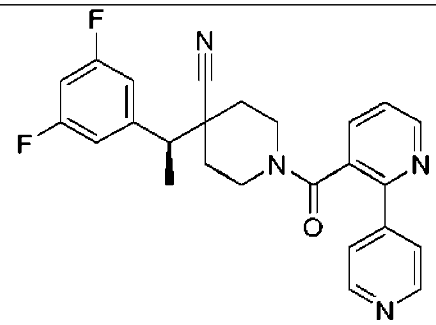 , |
| 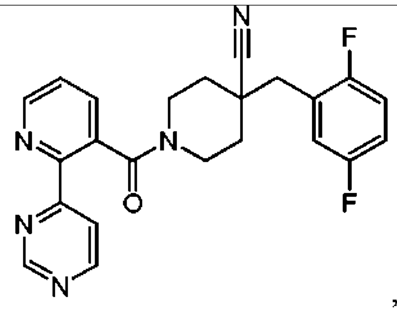 , |
| 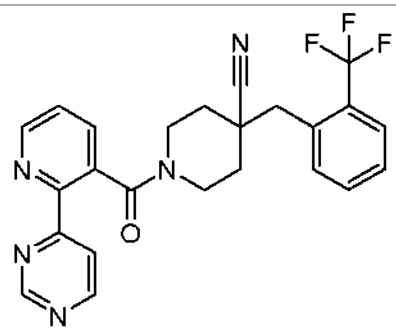 , |

| |
|---|
| 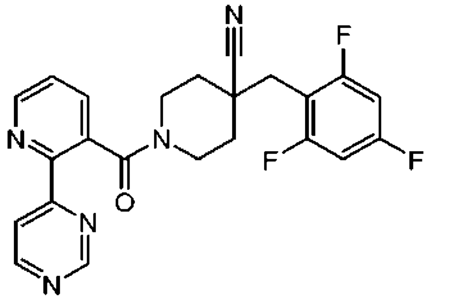 , |
| 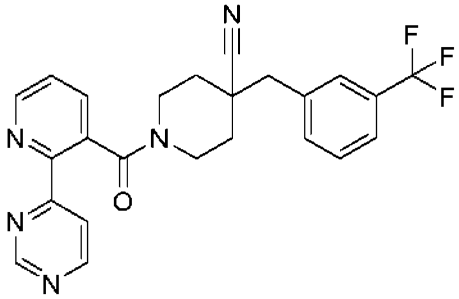 , |
| 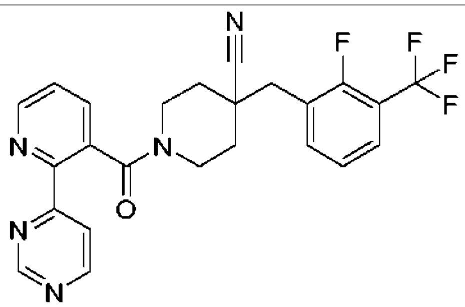 , |
| 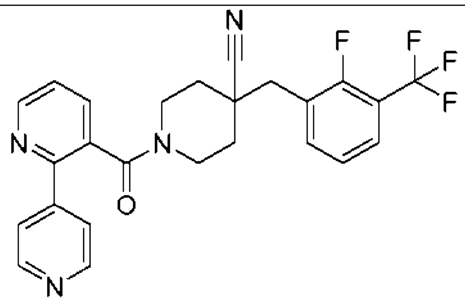 , |
| 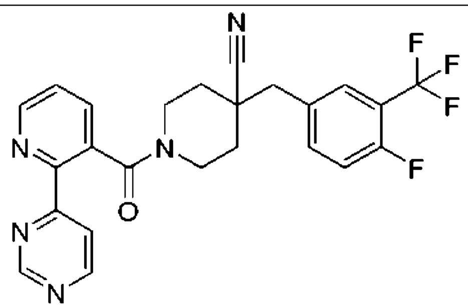 , |

| |
|---|
| 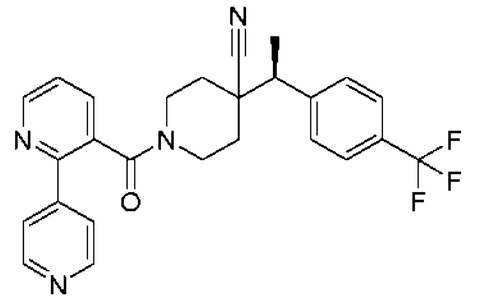 , |
| 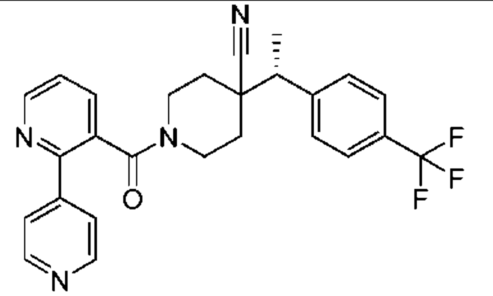 , |
| 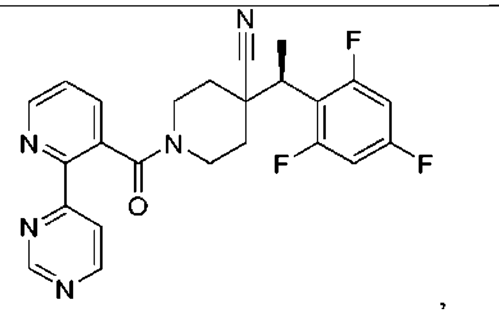 , |
| 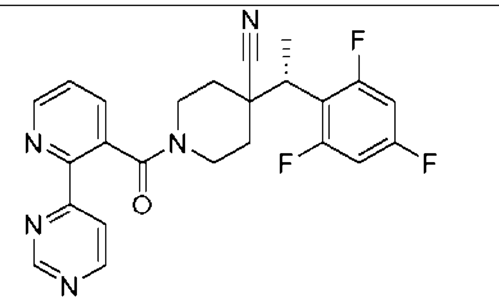 , |
| 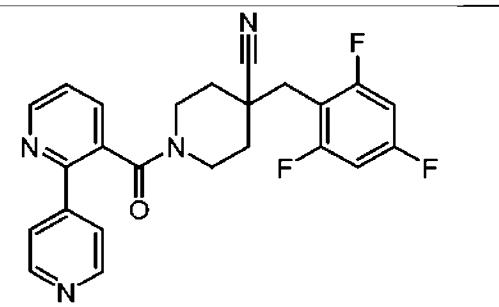 , |

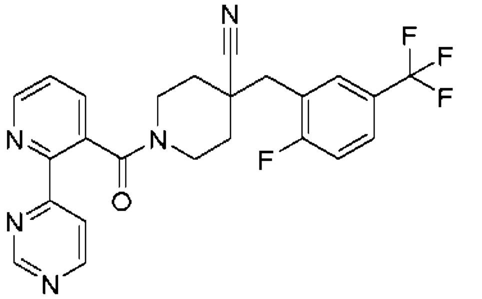
,

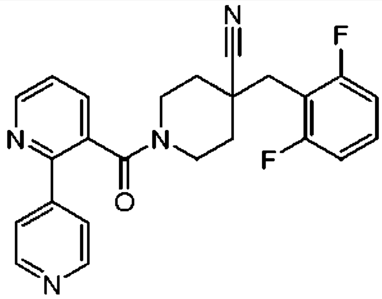
,

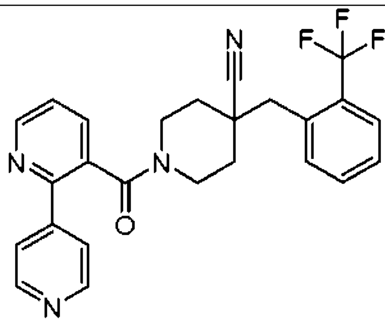
,

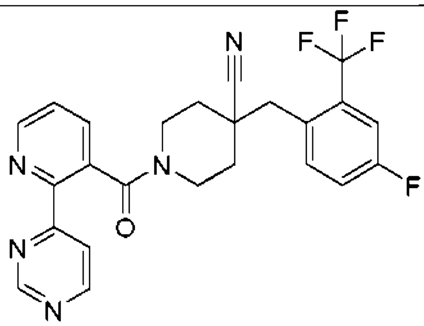
,

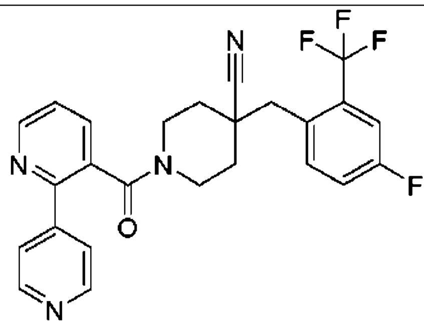
,

| |
|---|
| 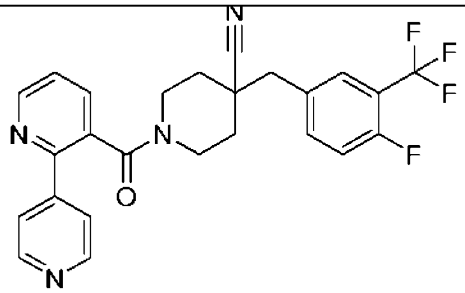 , |
| 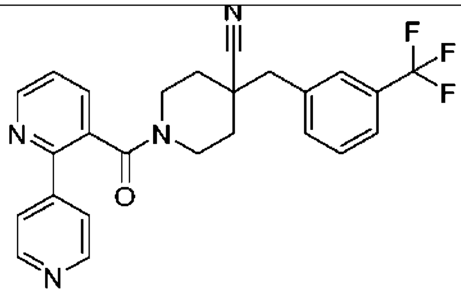 , |
| 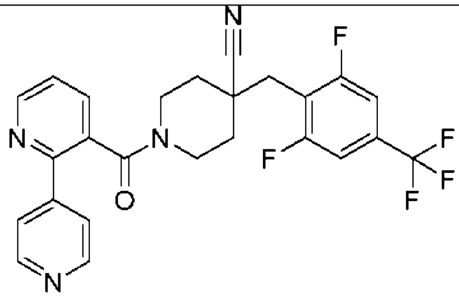 , |
| 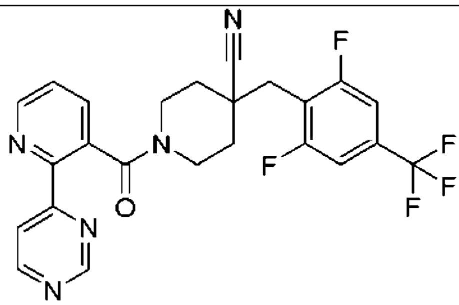 , |
| 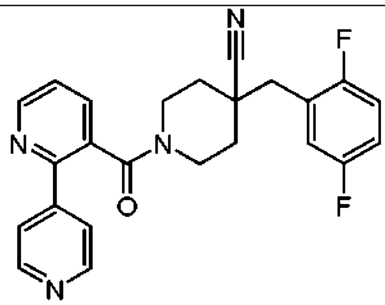 , |

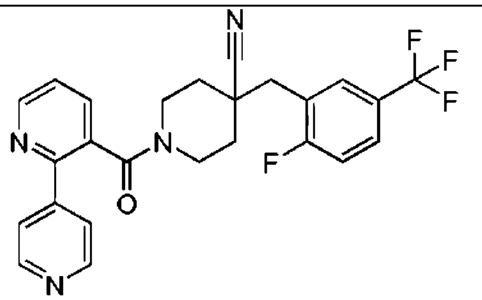

,

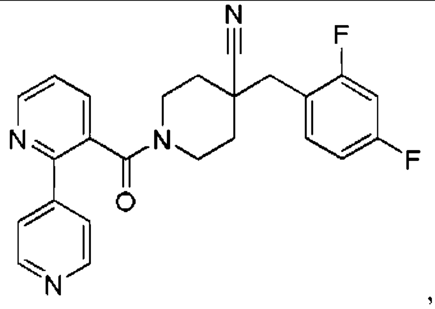

,

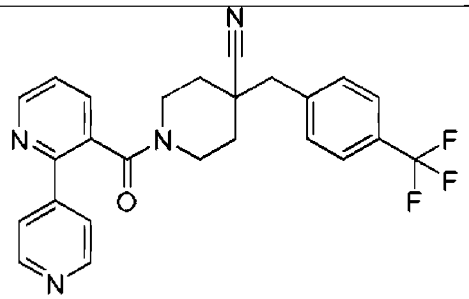

,

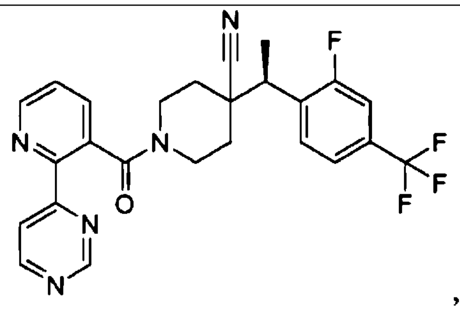

,

et

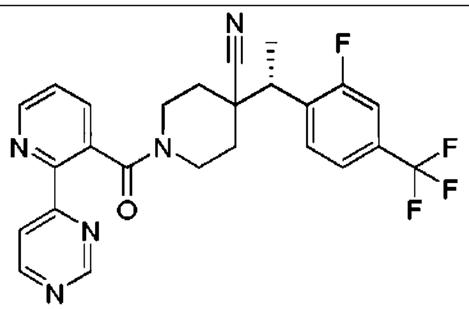

.

**28.** Composition pharmaceutique comprenant un composé selon l'une quelconque des revendications 1-27 ou un sel pharmaceutiquement acceptable de celui-ci, et un support pharmaceutiquement acceptable.

**29.** Composé selon l'une quelconque des revendications 1-27, ou sel pharmaceutiquement acceptable de celui-ci, ou

composition pharmaceutique selon la revendication 28, pour une utilisation dans le traitement d'une maladie ou d'un trouble choisi dans le groupe constitué par un trouble neurodégénératif, l'épilepsie, des encéphalopathies développementales et épileptiques, un trouble psychiatrique et un spasme.

**30.** Composé, sel pharmaceutiquement acceptable, ou composition pharmaceutique pour utilisation selon la revendication 29, dans lequel la maladie ou le trouble est un trouble neurodégénératif.

**31.** Composé, sel pharmaceutiquement acceptable, ou composition pharmaceutique pour utilisation selon la revendication 30, dans lequel le trouble neurodégénératif est choisi dans le groupe constitué par la maladie d'Alzheimer, une déficience cognitive légère, la maladie de Huntington, la maladie de Parkinson, la sclérose latérale amyotrophique, une lésion cérébrale traumatique, un infarctus cérébral, un glaucome, et la sclérose en plaques.

**32.** Composé, sel pharmaceutiquement acceptable, ou composition pharmaceutique pour utilisation selon la revendication 29, dans lequel la maladie ou le trouble est l'épilepsie.

**33.** Composé, sel pharmaceutiquement acceptable, ou composition pharmaceutique pour utilisation selon la revendication 29, dans lequel la maladie ou le trouble est une encéphalopathie développementale et épileptique.

**34.** Composé, sel pharmaceutiquement acceptable, ou composition pharmaceutique pour utilisation selon la revendication 29, dans lequel la maladie ou le trouble est un trouble psychiatrique.

**35.** Composé, sel pharmaceutiquement acceptable, ou composition pharmaceutique pour utilisation selon la revendication 34, dans lequel le trouble psychiatrique est choisi dans le groupe constitué par la schizophrénie, un trouble du spectre autistique, un trouble délirant, un trouble schizoaffectif et la dépression.

**36.** Composé, sel pharmaceutiquement acceptable, ou composition pharmaceutique pour utilisation selon la revendication 29, dans lequel la maladie ou le trouble est un spasme.

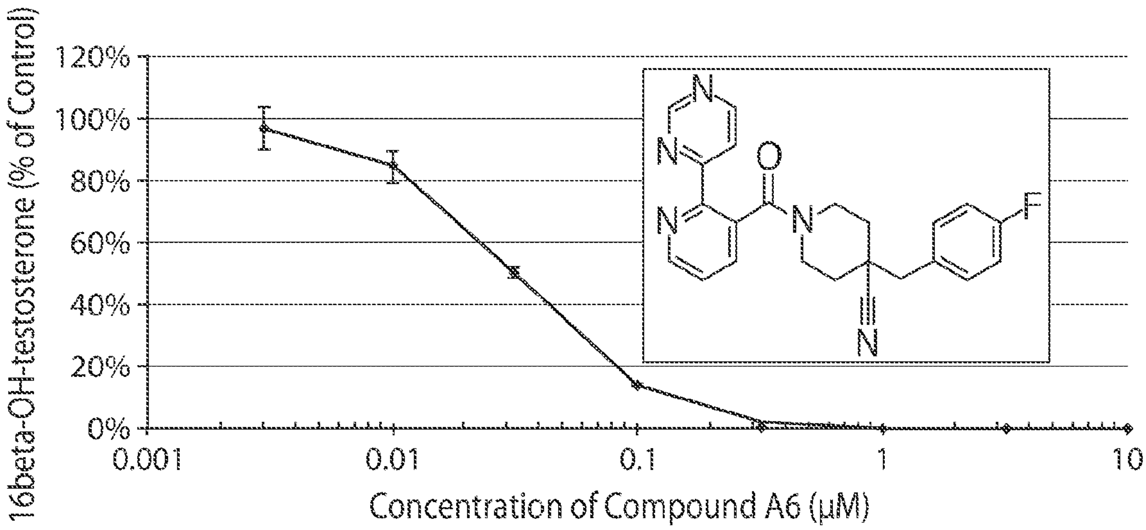


| A6 Concentration (µM) | % Activity 1 | % Activity 2 | % Activity Mean |
|---|---|---|---|
| **10** | 0.0% | 0.0% | 0.0% |
| **3.2** | 0.0% | 0.0% | 0.0% |
| **1** | 0.0% | 0.0% | 0.0% |
| **0.32** | 0.0% | 0.0% | 0.0% |
| **0.1** | 13.7% | 13.1% | 13.4% |
| **0.032** | 50.8% | 48.6% | 49.7% |
| **0.01** | 80.2% | 87.2% | 83.7% |
| **0.003** | 101% | 91.5% | 96.4% |

**FIG. 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2933247 A1
- EP 2982666 A1
- WO 2013054822 A1
- JP 11514333 A
- JP 2001500852 A
- WO 0114372 A


### Non-patent literature cited in the description


- Remington's Pharmaceutical Sciences. Mack Publishing Company, 1985
- **E. W. MARTIN**. Remington's Pharmaceutical Sciences. Mack Publishing Co., 1980
- Remington: The Science and Practice of Pharmacy. Lippincott Williams & Wilkins, 2005
- *Cancer Research*, 1989, vol. 49, 5935-5939
- *Journal of Clinical Oncology*, 1994, vol. 12, 213-225
- *British Journal of Cancer*, 1993, vol. 68, 314-318
- Periodic Table of the Elements, CAS version. *Handbook of Chemistry and Physics*
- **THOMAS SORRELL**. Organic Chemistry. University Science Books, 1999
- **SMITH** ; **MARCH**. March's Advanced Organic Chemistry. John Wiley & Sons, Inc, 2001
- **LAROCK**. Comprehensive Organic Transformations. VCH Publishers, Inc., 1989
- **CARRUTHERS**. Some Modern Methods of Organic Synthesis. Cambridge University Press, 1987
- **JACQUES et al.** Enantiomers, Racemates and Resolutions. Wiley Interscience, 1981
- **WILEN et al.** *Tetrahedron*, 1977, vol. 33, 2725
- **ELIEL**. Stereochemistry of Carbon Compounds. McGraw-Hill, 1962
- **WILEN**. Tables of Resolving Agents and Optical Resolutions. Univ. of Notre Dame Press, 1972, 268
- **BERGE et al.** *J. Pharm. Sci.*, 1977, vol. 66 (1), 1-79
- **T. W. GREENE** ; **P. G. M. WUTS**. Protecting Groups in Organic Synthesis. John Wiley & Sons, 1999
- **T. W. GREENE** ; **P. G. M. WUTS**. Protecting Groups in Organic Synthesis. Wiley, 1991
- **SOHLENIUS-STERNBECK** ; **ANNA-KARIN**. Determination of the hepatocellularity number for human, dog, rabbit, rat and mouse livers from protein concentration measurements. *Toxicology in Vitro*, 2006, vol. 20, 1582-1586
- **DAVIES, B.** ; **MORRIS, T.** Physiological Parameters in Laboratory Animals and Human. *Pharmaceutical Research*, 1993, vol. 10, 1093-1095
- **OBACH R S** ; **BAXTER J G** ; **LISTON T E et al.** The prediction of human pharmacokinetic parameters from preclinical and in vitro metabolism data.. *Journal of Pharmacology and Experimental Therapeutics*, 1997, vol. 283 (1), 46-58